(19) Europäisches Patentamt
European Patent Office
Office européen des brevets

(11) **EP 1 930 320 A1**

(12) **EUROPEAN PATENT APPLICATION**
published in accordance with Art. 153(4) EPC

(43) Date of publication:
**11.06.2008 Bulletin 2008/24**

(21) Application number: **06810832.3**

(22) Date of filing: **29.09.2006**

(51) Int Cl.:
***C07D 209/12*** (2006.01) ***A61K 31/404*** (2006.01)
***A61K 31/4045*** (2006.01) ***A61K 31/405*** (2006.01)
***A61K 31/4155*** (2006.01) ***A61K 31/427*** (2006.01)
***A61K 31/454*** (2006.01) ***A61K 31/496*** (2006.01)
***A61K 31/5377*** (2006.01) ***A61K 31/5386*** (2006.01)
***A61K 31/541*** (2006.01) ***A61K 31/551*** (2006.01)
***A61P 3/10*** (2006.01) ***A61P 5/44*** (2006.01)
***A61P 5/46*** (2006.01) ***A61P 29/00*** (2006.01)
***C07D 209/14*** (2006.01) ***C07D 209/22*** (2006.01)
***C07D 209/24*** (2006.01) ***C07D 401/06*** (2006.01)
***C07D 401/14*** (2006.01)

(86) International application number:
**PCT/JP2006/319426**

(87) International publication number:
**WO 2007/040166 (12.04.2007 Gazette 2007/15)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **30.09.2005 JP 2005286576**

(71) Applicant: **Dainippon Sumitomo Pharma Co., Ltd.**
**Osaka 541-8524 (JP)**

(72) Inventors:
- **SONE, Toshihiko**
  **Suita-shi, Osaka 564-0053 (JP)**
- **SAWAKI, Rieko**
  **Osaka-shi**
  **Osaka 554-0022 (JP)**
- **NAKAJIMA, Tomoko**
  **Suita-shi, Osaka 564-0053 (JP)**

(74) Representative: **Vossius & Partner**
**Siebertstrasse 4**
**81675 München (DE)**

(54) **NOVEL FUSED PYRROLE DERIVATIVE**

(57) The present invention relates to a compound represented by the formula (1) which is useful as a glucocorticoid receptor function regulating agent, an anti-inflammatory agent or an antidiabetic agent, or a pharmaceutically acceptable salt thereof:

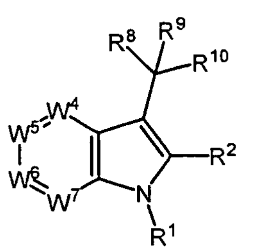

(1)

wherein $R^1$ is aralkyl, etc.; $R^2$ is H, etc.; $-W^4=W^5-W^6=W^7-$ is a group of the formula: $-CR^4=CR^5-CR^6=CR^7-$ (in which $R^4$, $R^5$, $R^6$ and $R^7$ are independently a group of the formula: -E-A (E is a single bond, etc., A is H or nitro, etc.)), etc.; $R^8$ is a group of the formula: $-OR^{11}$ ($R^{11}$ is H, etc.), etc.; $R^9$ is trifluoromethyl, etc.; $R^{10}$ is a group of the formula: $-[C(R^{13})(R^{14})]_n-R^{15}$ ($R^{13}$ and $R^{14}$ are independently H, etc., n is an integer of 0 to 10, $R^{15}$ is a group of the formula: $N(R^{18})R^{19}$ ($R^{18}$ and $R^{19}$ form a nitrogen-containing heteromonocycle together with a nitrogen atom to which they are bonded), etc.).

EP 1 930 320 A1

## Description

### TECHNICAL FIELD

The present invention relates to a novel fused pyrrole derivative being useful as a medicament. More particularly, the present invention relates to a novel fused pyrrole derivative being useful as glucocorticoid receptor regulating agent (modulator), i.e., as an anti-inflammatory agent or an antidiabetic agent.

### BACKGROUND ART

Steroidal anti-inflammatory agent exhibits a potent anti-inflammatory activity and immunosuppressive activity, and is a medicament to be used in the treatment of inflammatory diseases or autoimmune diseases caused by excessive immune response. These steroidal anti-inflammatory agents have been known to exhibit the anti-inflammatory activity and immunoregluating activity by binding to glucocorticoid receptor (hereinafter, referred to as GR), which is known as a nuclear receptor. In old times, it has been considered that steroids show significant effects on rheumatoid arthritis which is one of inveterate inflammatory diseases, but despite of the potent main medical effects thereof, it has become apparent that steroids have potent side effects such as disorder of glucose metabolism, adrenal hypofunction, disorder of bone metabolism, easy infectious, etc. Consequently, recently, it has been desired to develop a novel GR-binding compound (GR ligand) as a novel anti-inflammatory agent where the conventional side effects are reduced but the anti-inflammatory activity thereof is retained as the same as that of steroids.

With regard to the anti-inflammatory action and the metabolic action such as glucose metabolism or bone metabolism by steroidal anti-inflammatory agents, the following molecular mechanism has become apparent by the recent development in the study.
GR is usually staying within the cytoplasm, but when a steroid, a GR ligand, binds to GR, then GR translocates into the nucleus from the cytoplasm and regulates the transcription, during which GR acts on inflammatory transcription factors such as AP1 or NF-κB, etc. or enzymes participating in the inflammatory response and suppresses the activity thereof, and finally exhibits anti-inflammatory activity. On the other hand, in the action mechanism of GR exhibiting metabolic actions such as glucose metabolism, bone metabolism, etc., GR is considered to form a homodimer and directly bind to the gene sequence called as glucocorticoid responsive element: GRE adjacent to the target gene, to exhibit transcriptional regulation.

Some trials have been reported to find compounds having a lowered metabolic activity while keeping anti-inflammatory activity, based on the above findings. Especially, when focusing on the reduction of the side effects of steroidal anti-inflammatory agents, the conventional steroid anti-inflammatory agents show a potent agonistic activity to the transcriptional regulation through GRE, and said agonistic activity has been considered as a principal body exhibiting a metabolic action. Accordingly, it has been thought that compounds having fewer side effects can be found by searching compound having a weaker agonistic activity than the conventional steroidal anti-inflammatory agent, namely, by searching GR partial agonists, and as a result, some non-steroidal anti-inflammatory agents have been reported (cf., Patent Document 1).

On the other hand, in the conditions where the cortisol blood level is high such as Cushing's syndrome, etc., or GR is over-activated, it is useful to suppress the overactivation of GR. In addition, in the conditions where immunity is lowered, or where depression or glucose metabolism is increased, compounds suppressing glucocorticoid action via GR have been considered to normalize such abnormal conditions, and GR antagonists have been reported (cf., Patent Document 2). Especially, gluconeogenesis is increased in diabetes mellitus, and the over-production of glucose by the liver is observed. It is well known that hyperglycemia is caused by increasing the expression of various transaminase, glucose-6-phosphatase, phosphoenolpyruvate carboxykinase (PEPCK), etc. which transform amino acids into glucose precursor, by GR, and a GR antagonist has been expected to be a remedy for treatment of diabetes mellitus.

Patent Document 1: WO 2004/058733 pamphlet
Patent Document 2: US Patent Publication 2004/0235810
Patent Document 3: WO 2004/067529 pamphlet

### DISCLOSURE OF INVENTION

### PROBLEMS TO BE SOLVED BY INVENTION

An object of the present invention is to provide an agent for treatment and/or prophylaxis of diseases involved with GR, concretely, diseases such as inflammation. More particularly, an object of the present invention is to provide

a compound showing a partial agonistic property to GR as a non-steroidal anti-inflammatory agent having a fewer side effects than steroidal anti-inflammatory agents.

MEANS FOR SOLVING THE PROBLEMS

The present inventors have intensively studied, and found that compounds having the following fused pyrrole nucleus, or a prodrug thereof, or a pharmaceutically acceptable salt thereof can function as a GR modulator, and finally accomplished the present invention.
Namely, the present invention provides the following features:
[1] A compound of the formula (1):
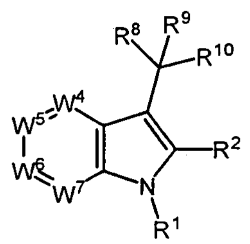

(1)

wherein $R^1$ is a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkoxy group, an optionally substituted alkanoyl group, an optionally substituted alkoxycarbonyl group, an optionally substituted alkylsulfonyl group, an optionally substituted cycloalkyloxy group, an optionally substituted cycloalkylcarbonyl group, an optionally substituted cycloalkyloxycarbonyl group, an optionally substituted cycloalkylsulfonyl group, an optionally substituted aryloxy group, an optionally substituted aroyl group, an optionally substituted aryloxycarbonyl group, an optionally substituted arylsulfonyl group, an optionally substituted heteroaryloxy group, an optionally substituted heteroarylcarbonyl group, an optionally substituted heteroaryloxycarbonyl group, an optionally substituted heteroarylsulfonyl group, an optionally substituted aralkyloxy group, an optionally substituted aralkylcarbonyl group, an optionally substituted aralkyloxycarbonyl group, an optionally substituted aralkylsulfonyl group, an optionally substituted heteroaralkyloxy group, an optionally substituted heteroaralkylcarbonyl group, an optionally substituted heteroaralkyloxycarbonyl group, an optionally substituted heteroaralkylsulfonyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, an optionally substituted carbamoyl group, or an optionally substituted sulfamoyl group;
$R^2$ is a hydrogen atom, a halogen atom, a carboxyl group, an optionally substituted alkyl group, an optionally substituted cycloalkyl group, an optionally substituted aryl group, an optionally substituted heteroaryl group, an optionally substituted aralkyl group, an optionally substituted heteroaralkyl group, an optionally substituted alkanoyl group, an optionally substituted cycloalkylcarbonyl group, an optionally substituted alkoxycarbonyl group, an optionally substituted, saturated or unsaturated aliphatic heterocyclic group, or a carbamoyl group optionally substituted with an optionally substituted alkyl group;

- $W^4=W^5-W^6=W^7-$ is a group selected from the following formulae (a) to (h):

  (a) $-CR^4=CR^5-CR^6=CR^7-$;
  (b) $-N=CR^5-CR^6=CR^7-$;
  (c) $-CR^4=N-CR^6=CR^7-$;
  (d) $-CR^4=CR^5-N=CR^7-$;
  (e) $-CR^4=CR^5-CR^6=N-$;
  (f) $-N=CR^5-N=CR^7-$;
  (g) $-CR^4=N-CR6=N-$;
  (h) $-CR^4=N-N=CR^7-$

  [in which $R^4$, $R^5$, $R^6$ and $R^7$ are independently the same or different, and each is a group of the formula: -E-A, and in the formula, E is a single bond, or a group selected from the following formulae 1) to 14):

1) $-C(R^{16})R^{17}-$,
2) -O-,
3) $-S(=O)_m-$,
4) $-S(=O)_2NR^{16}-$,
5) -C(=O)-,
6) -C(=O)O-, .
7) $-C(=O)NR^{16}-$,
8) $-C(=NR^{16})NR^{17}-$,
9) $-NR^{16}-$,
10) $-N(R^{16})C(=O)-$,
11) $-N(R^{16})S(=O)_2-$,
12) $-N(R^{16})C(=O)N(R^{17})-$,
13) $-N(R^{16})S(=O)_2N(R^{17})-$,
14) $-P(=O)(OR^{16})_2-$

(in which $R^{16}$ and $R^{17}$ are independently a hydrogen atom, a $C_{1-3}$ alkyl group, or a $C_{1-3}$ alkoxy group, or in the formulae 8), 12) and 13), $R^{16}$ and $R^{17}$ may combine each other to form a $C_{2-4}$ alkylene group, and m is 0, 1 or 2),
when E is a single bond, then A is a hydrogen atom, a halogen atom, a cyano group, a nitro group, a hydroxy group, a carboxyl group, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, or an optionally substituted, saturated or unsaturated aliphatic heterocyclic group,
When E is a group selected from the above formulae 1) to 14), then A is a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, or an optionally substituted, saturated or unsaturated aliphatic heterocyclic group];
$R^8$ is a group of the formula $-OR^{11}$, $-SR^{11}$, or $-N(R^{11})R^{12}$ (in which $R^{11}$ and $R^{12}$ are independently a hydrogen atom, or an optionally substituted $C_{1-5}$ alkyl group);
$R^9$ is an alkyl group substituted by one or more halogen atoms, or a cycloalkyl group substituted by one or more halogen atoms;
$R^{10}$ is a group of the formula: $-[C(R^{13})R^{14})_n-R^{15}$ (in which $R^{13}$ and $R^{14}$ are independently a hydrogen atom, an alkyl group or a halogen atom, or $R^{13}$ and $R^{14}$ may combine each other to form an oxo group, or $R^{13}$ and $R^{14}$ may combine each other together with a carbon atom to which they are bonded to form a cycloalkane (one or two $-CH_2-$ groups in said cycloalkane may be replaced by the same or different group(s) selected from -NH-, -S-, -S(=O)-, $-S(=O)_2-$, -C (=O)- and -O-); n is an integer of 0 to 10, and when n is an integer of 2 to 10, then $C(R^{13})R^{14}$ may be either the same or different, $R^{15}$ is a hydroxy group, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted alkoxy group, an optionally substituted alkylthio group, an optionally substituted alkylsulfinyl group, an optionally substituted alkylsulfonyl group, an optionally substituted cycloalkyloxy group, an optionally substituted cycloalkylthio group, an optionally substituted cycloalkylsulfinyl group, an optionally substituted cycloalkylsulfonyl group, an optionally substituted aryloxy group, an optionally substituted arylthio group, an optionally substituted arylsulfinyl group, an optionally substituted arylsulfonyl group, an optionally substituted heteroaryloxy group, an optionally substituted heteroarylthio group, an optionally substituted heteroarylsulfinyl group, an optionally substituted heteroarylsulfonyl group, an optionally substituted carbamoyl group, an optionally substituted sulfamoyl group, an optionally substituted thiocarbamoyl group, an optionally substituted, saturated or unsaturated aliphatic heterocyclic group, an optionally substituted, saturated or unsaturated aliphatic heterocyclic oxy group, an optionally substituted, saturated or unsaturated aliphatic heterocyclic thio group, an optionally substituted, saturated or unsaturated aliphatic heterocyclic sulfinyl group, an optionally substituted, saturated or unsaturated aliphatic heterocyclic sulfonyl group, or a group of the formula: $N(R^{18})R^{19}$ (in which $R^{18}$ and $R^{19}$ are independently a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkanoyl group, an optionally substituted alkoxycarbonyl group, an optionally substituted alkylsulfonyl group, an optionally substituted cycloalkylcarbonyl group, an optionally substituted cycloalkyloxycarbonyl group, an optionally substituted cycloalkylsulfonyl group, an optionally substituted aroyl group, an optionally substituted aryloxycarbonyl group, an optionally substituted arylsulfonyl group, an optionally substituted heteroarylcarbonyl group, an optionally substituted

heteroaryloxycarbonyl group, an optionally substituted heteroarylsulfonyl group, an optionally substituted aralkylcarbonyl group, an optionally substituted aralkyloxycarbonyl group, an optionally substituted aralkylsulfonyl group, an optionally substituted heteroaralkylcarbonyl group, an optionally substituted heteroaralkyloxycarbonyl group, an optionally substituted heteroaralkylsulfonyl group, an optionally substituted carbamoyl group, an optionally substituted sulfamoyl group, an optionally substituted thiocarbamoyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, or $R^{18}$ and $R^{19}$ may combine each other together with a nitrogen atom to which they are bonded to form an optionally substituted, saturated or unsaturated monocyclic, bicyclic or tricyclic nitrogen-containing heterocyclic group containing 1 to 4 heteroatoms selected from 0 to 2 oxygen atoms, 0 to 2 sulfur atoms, and 1 to 4 nitrogen atoms);
provided that when $R^{10}$ is methyl, trifluoromethyl, hydroxymethyl, acetoxymethyl, ethoxycarbonylmethyl, methoxycarbonyl, ethoxycarbonyl, N-butylcarbamoyl, N-(4-methylbenzenesulfonylmethyl)carbamoyl, piperidinocarbonyl, 1-allyl-1H-imidazol-2-yl, 1-methyl-1H-1,2,4-triazol-5-yl or 1,3-benzoxazol-2-yl, then $R^1$ is not a hydrogen atom nor methyl, or a prodrug thereof, or a pharmaceutically acceptable salt thereof.

[2] The compound according to the above [1], or a prodrug thereof, or a pharmaceutically acceptable salt thereof, wherein $R^1$ is an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkoxy group, an optionally substituted alkanoyl group, an optionally substituted alkoxycarbonyl group, an optionally substituted alkylsulfonyl group, an optionally substituted cycloalkyloxy group, an optionally substituted cycloalkylcarbonyl group, an optionally substituted cycloalkyloxycarbonyl group, an optionally substituted cycloalkylsulfonyl group, an optionally substituted aryloxy group, an optionally substituted aroyl group, an optionally substituted aryloxycarbonyl group, an optionally substituted arylsulfonyl group, an optionally substituted heteroaryloxy group, an optionally substituted heteroarylcarbonyl group, an optionally substituted heteroaryloxycarbonyl group, an optionally substituted heteroarylsulfonyl group, an optionally substituted aralkyloxy group, an optionally substituted aralkylcarbonyl group, an optionally substituted aralkyloxycarbonyl group, an optionally substituted aralkylsulfonyl group, an optionally substituted heteroaralkyloxy group, an optionally substituted heteroaralkylcarbonyl group, an optionally substituted heteroaralkyloxycarbonyl group, an optionally substituted heteroaralkylsulfonyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, an optionally substituted carbamoyl group, or an optionally substituted sulfamoyl group.
[3] The compound according to the above [1] or [2], or a prodrug thereof, or a pharmaceutically acceptable salt thereof, wherein $-W^4=W^5-W^6=W^7-$ is a group of the formula (a): $-CR^4=CR^5-CR^6=CR^7-$ (in which $R^4$, $R^5$, $R^6$ and $R^7$ are the same as defined in the above [1]).
[4] The compound according to the above [1] or [2], or a prodrug thereof, or a pharmaceutically acceptable salt thereof, wherein $-W^4=W^5-W^6=W^7-$ is a group of the formula (d): $-CR^4=CR^5-N=CR^7-$ (in which $R^4$, $R^5$ and $R^7$ are the same as defined in the above [1]).
[5] The compound according to any one of the above [1] to [4], or a prodrug thereof, or a pharmaceutically acceptable salt thereof, wherein $R^1$ is an optionally substituted aralkyl group, an optionally substituted heteroaralkyl group, an optionally substituted aryl group, an optionally substituted heteroaryl group, an optionally substituted cycloalkyl group, an alkyl group substituted by an optionally substituted cycloalkyl group, or an alkyl group substituted by an optionally substituted, saturated or unsaturated aliphatic heterocyclic group.
[6] The compound according to any one of the above [1] to [5], or a prodrug thereof, or a pharmaceutically acceptable salt thereof, wherein $R^2$ is a hydrogen atom, or an optionally substituted $C_{1-6}$ alkyl group.
[7] The compound according to any one of the above [1] to [6], or a prodrug thereof, or a pharmaceutically acceptable salt thereof, wherein $R^8$ is a group of the formula: $-OR^{11}$ (in which $R^{11}$ is the same as defined in the above [1]).
[8] The compound according to the above [7], or a prodrug thereof, or a pharmaceutically acceptable salt thereof, wherein $R^{11}$ is a hydrogen atom.
[9] The compound according to any one of the above [1] to [6], or a prodrug thereof, or a pharmaceutically acceptable salt thereof, wherein $R^8$ is a group of the formula: $-N(R^{11})R^{12}$ (in which $R^{11}$ and $R^{12}$ are the same as defined in the above [1]).
[10] The compound according to the above [9], or a prodrug thereof, or a pharmaceutically acceptable salt thereof, wherein $R^{11}$ and $R^{12}$ are a hydrogen atom.
[11] The compound according to any one of the above [1] to [10], or a prodrug thereof, or a pharmaceutically acceptable salt thereof, wherein $R^9$ is a $C_{1-6}$ alkyl group substituted by 1 to 7 fluorine or chlorine atoms.
[12] The compound according to the above [11], or a prodrug thereof, or a pharmaceutically acceptable salt thereof,

wherein $R^9$ is a trifluoromethyl group.

[13] The compound according to any one of the above [1] to [12], or a prodrug thereof, or a pharmaceutically acceptable salt thereof, wherein in the formula: $-[C(R^{13})R^{14}]_n-R^{15}$ for $R^{10}$, $R^{13}$ and $R^{14}$ are a hydrogen atom, n is 1 or 2, and $R^{15}$ is a group of the formula: $N(R^{18})R^{19}$ (in which $R^{18}$ and $R^{19}$ are the same as defined in the above [1]).

[14] The compound according to the above [13], or a prodrug thereof, or a pharmaceutically acceptable salt thereof, wherein $R^{18}$ and $R^{19}$ may combine each other together with a nitrogen atom to which they are bonded to form a substituted saturated or unsaturated nitrogen-containing heteromonocyclic or heterobicyclic group.

[15] A glucocorticoid receptor function regulating agent, which comprises as the active ingredient the compound as set forth in any one of the above [1]-[14], or a prodrug thereof, or a pharmaceutically acceptable salt thereof.

[16] An agent for treatment or prophylaxis of inflammatory diseases or diabetes mellitus, which comprises as the active ingredient the compound as set forth in any one of the above [1]-[14], or a prodrug thereof, or a pharmaceutically acceptable salt thereof.

EFFECTS OF INVENTION

By the present invention, it becomes possible to provide a compound having a novel fused pyrrole nucleus being useful as a glucocorticoid function regulating agent (modulator), i.e., an agent for treatment or prophylaxis of inflammatory diseases or diabetes mellitus, or a prodrug thereof, or a pharmaceutically acceptable salt thereof.

BEST MODE FOR CARRYING OUT THE INVENTION

The present invention will be more concretely illustrated below.

Each group in the present invention is explained below. Unless specified otherwise, the definition for each group should be applied to cases wherein said group is a part of another substituent.

In the present specification, the number of the substituents are not necessarily specified, and it may be one or more, preferably 1 to 5.

In the present specification, the halogen atom is fluorine atom, chlorine atom, bromine atom, or iodine atom.

The alkyl group includes, for example, a straight chain or branched chain alkyl group having 1 to 10 carbon atoms, such as methyl, ethyl, propyl, isopropyl, butyl, isobutyl, sec-butyl, tert-butyl, pentyl, isopentyl, neopentyl, tert-pentyl, 1-methylbutyl, hexyl, heptyl, octyl, nonyl, decyl, etc. Among them, alkyl groups having 1 to 6 carbon atoms are preferable.

The alkenyl group includes, for example, a straight chain or branched chain alkenyl group having 2 to 6 carbon atoms, such as vinyl, 1-propenyl, allyl (2-propenyl), isopropenyl (1-methylvinyl), 1-butenyl, 2-butenyl, 3-butenyl, 1-methyl-1-propenyl, 1-methyl-2-propenyl, 2-methylallyl, 1-ethylvinyl, 1-pentenyl, or 1-hexenyl, etc. Among them, alkenyl group having 2 to 4 carbon atoms are preferable.

The alkynyl group includes, for example, a straight chain or branched chain alkynyl group having 2 to 6 carbon atoms, such as ethynyl, 1-propynyl, 2-propynyl, 1-butynyl, 1-methyl-2-propynyl, 2-butynyl, 3-butynyl, 1-pentynyl, or 1-hexynyl, etc. Among them, alkynyl groups having 2 to 4 carbon atoms are preferable.

The cycloalkyl group includes, for example, a 3- to 10-membered, saturated monocyclic to tricyclic aliphatic hydrocarbon rings, such as cyclopropyl, cyclobutyl, cyclopentyl, cyclohexyl, cycloheptyl, cyclooctyl, bicyclo[3,2,1]octyl, bicyclo[2,2,2]octyl, or adamantyl, etc. Among them, 4- to 6-membered saturated cycloalkyl groups are preferable.

The cycloalkenyl group includes, for example, a 4- to 10-membered monocyclic to tricyclic aliphatic hydrocarbon ring having 1 or 2 double bonds, such as cyclobutenyl, cyclopentenyl, cyclohexenyl, cycloheptenyl, or cyclooctenyl, where the binding position of these groups can be any possible position. Among them, 4- to 6-membered cycloalkenyl groups are preferable.

The aryl group includes, for example, an aryl group having 6 to 10 carbon atoms, such as phenyl, 1-naphthyl, 2-naphthyl, etc.

The heteroaryl group includes, for example, a 5- or 6-membered monocyclic heteroaryl group or a 9- or 10-membered bicyclic heteroaryl group, said heteroaryl having 1 to 4 heteroatoms selected from 0 to 4 nitrogen atoms, 0 to 2 oxygen atoms, and 0 to 2 sulfur atoms, and the binding position thereof may be any possible position as long as it is chemically stable, such as furyl, thienyl, pyrrolyl, oxazolyl, isoxazolyl, thiazolyl, imidazolyl, pyrazolyl, furazanyl, triazolyl, pyridyl, pyrimidinyl, pyrazinyl, tetrazolyl, indolyl, benzoxazolyl, benzisoxazolyl, benzothiazolyl, benzoimidazolyl, quinolyl, isoquinolyl, quinazolinyl, imidazopyridinyl, purynyl, etc.

The aralkyl group is also called as an arylalkyl group, and includes a straight chain or branched chain alkyl group having 1 to 6 carbon atoms which is substituted by an aryl group, and said aryl group and said alkyl group are the same as mentioned above. The aralkyl group includes, for example, benzyl (phenylmethyl), phenethyl (2-phenylethyl), 3-phenylpropyl, 2-phenyl-2-methylethyl, 1-phenylethyl, 1-naphthylmethyl, 2-naphthylmethyl, etc.

The heteroaralkyl group is also called as a heteroarylalkyl group, and includes a straight chain or branched chain alkyl group having 1 to 6 carbon atoms which is substituted by a heteroaryl group, and said heteroaryl group and said alkyl

group are the same as mentioned above, and the binding position of these groups may be any position as long as it is chemically stable. The heteroaralkyl group includes, for example, 2-pyridylmethyl, 3-pyridylmethyl, 4-pyridylmethyl, 2-furylmethyl (furfuryl), 3-furylmethyl, 2-thienylmethyl, 3-thienylmethyl, 2-pyridylethyl, 3-pyridylethyl, 4-pyridylethyl, etc.

The alkoxy group includes a straight chain or branched chain alkoxy group having 1 to 10 carbon atoms, for example, methoxy, ethoxy, propoxy, isopropoxy, butoxy, isobutoxy, sec-butoxy, tert-butoxy, pentyloxy, isopentyloxy, neopentyloxy, tert-pentyloxy, 1-methylbutoxy, hexyloxy, heptyloxy, octyloxy, nonyloxy, decyloxy, etc., and among them, alkoxy groups having 1 to 6 carbon atoms are preferable.

The alkanoyl group is also called as an acyl group, and includes a straight chain or branched chain alkanoyl group having 2 to 10 carbon atoms, for example, acetyl, propionyl, butyryl, isobutyryl, valeryl, isovaleryl, pivaloyl, hexanoyl, heptanoyl, octanoyl, nonanoyl, decanoyl, etc., and among them, alkanoyl groups having 2 to 6 carbon atoms are preferable.

The alkanoyl moiety of the alkanoyloxy group is the same as defined in the above alkanoyl group.

The alkoxycarbonyl group includes a straight chain or branched chain alkoxycarbonyl group having 2 to 11 carbon atoms, for example, methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, tert-pentyloxycarbonyl, 1-methylbutoxycarbonyl, hexyloxycarbonyl, heptyloxycarbonyl, octyloxycarbonyl, nonyloxycarbonyl, decyloxycarbonyl, etc., and among them, alkoxycarbonyl groups having 2 to 7 carbon atoms are preferable.

The alkylthio group includes a straight chain or branched chain alkylthio group having 1 to 10 carbon atoms, for example, methylthio, ethylthio, propylthio, isopropylthio, butylthio, isobutylthio, sec-butylthio, tert-butylthio, pentylthio, isopentylthio, neopentylthio, tert-pentylthio, 1-methylbutylthio, hexylthio, heptylthio, octylthio, nonylthio, decylthio group, etc. Among them, alkylthio groups having 1 to 6 carbon atoms are preferable.

The alkylsulfinyl group includes a straight chain or branched chain alkylsulfinyl group having 1 to 10 carbon atoms, for example, methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, isopentylsulfinyl, neopentylsulfinyl, tert-pentylsulfinyl, 1-methylbutylsulfinyl, hexylsulfinyl, heptylsulfinyl, octylsulfinyl, nonylsulfinyl, decylsulfinyl, etc. Among them, alkylsulfinyl groups having 1 to 6 carbon atoms are preferable.

The alkylsulfonyl group includes a straight chain or branched chain alkylsulfonyl group having 1 to 10 carbon atoms, for example, methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, isopentylsulfonyl, neopentylsulfonyl, tert-pentylsulfonyl, 1-methylbutylsulfonyl, hexylsulfonyl, heptylsulfonyl, octylsulfonyl, nonylsulfonyl, or decylsulfonyl, and among them, alkylsulfonyl groups having 1 to 6 carbon atoms are preferable.

The alkylene group includes a straight chain or branched chain alkylene group having 1 to 10 carbon atoms, for example, methylene, ethylene, trimethylene, 1-methylmethylene, 1-methylethylene, tetramethylene, octamethylene, decamethylene, etc., and among them, alkylene groups having 1 to 6 carbon atoms are preferable.

The saturated aliphatic heterocyclic group includes a 4- to 10-membered monocyclic or bicyclic saturated aliphatic heterocyclic group containing 1 to 4 heteroatoms selected from 0 to 4 nitrogen atoms, 0 to 2 oxygen atoms and 0 to 2 sulfur atoms, wherein the binding position thereof may be any position as long as it is chemically stable, for example, azetidinyl, pyrrolidinyl, piperidinyl, piperidino, piperazinyl, azepanyl, azocanyl, tetrahydrofuryl, tetrahydrothienyl, tetrahydropyranyl, morpholinyl, morpholino, thiomorpholinyl, 1-oxothiomorpholinyl, 1,1-dioxothiomorpholinyl, thiomorpholino, 1-oxothiomorpholino, 1,1-dioxothiomorpholino, 1,3-dioxolanyl, 1,4-dioxanyl, decahydroquinolino, decahydroquinoxalino, 1,4-diazabicyclo[4.4.0]decano, or decahydroisoquinolino.

The unsaturated aliphatic heterocyclic group includes a 5- to 10-membered non-aromatic monocyclic or bicyclic unsaturated aliphatic heterocyclic group having 1 or 2 double bonds and containing 1 to 4 heteroatoms selected from 0 to 4 nitrogen atoms, 0 to 2 oxygen atoms and 0 to 2 sulfur atoms, wherein the binding position may be any position as long as it is chemically stable, for example, 2-pyrrolinyl, 3-pyrrolinyl, 2-imidazolinyl, 3-imidazolinyl, 2-pyrazolinyl, 3-pyrazolinyl, octahydroquinolinyl, etc.

The saturated or unsaturated aliphatic heterocyclic oxy group includes a group being formed by binding an oxygen atom to any carbon atom of the above mentioned saturated aliphatic heterocyclic group or the unsaturated aliphatic heterocyclic group.

The saturated or unsaturated aliphatic heterocyclic carbonyl group includes a group being formed by binding a carbonyl group to any carbon atom of the above mentioned saturated aliphatic heterocyclic group or the unsaturated aliphatic heterocyclic group.

The saturated or unsaturated aliphatic heterocyclic sulfonyl group includes a group being formed by binding a sulfonyl ($SO_2$) to any carbon atom of the above mentioned saturated aliphatic heterocyclic group or the unsaturated aliphatic heterocyclic group.

The saturated or unsaturated aliphatic heterocyclic sulfinyl group includes a group being formed by binding a sulfinyl group (SO) to any carbon atom of the above mentioned saturated aliphatic heterocyclic group or the unsaturated aliphatic

heterocyclic group.
The saturated or unsaturated aliphatic heterocyclic thio group includes a group being formed by binding a thio group (S) to any carbon atom of the above mentioned saturated aliphatic heterocyclic group or the unsaturated aliphatic heterocyclic group.
The saturated or unsaturated aliphatic heterocyclic oxycarbonyl group includes a group, which is formed by binding a carbonyl group to the oxygen atom other than the ring-forming ones of the above mentioned saturated aliphatic heterocyclic oxy group or the unsaturated aliphatic heterocyclic oxy group.

The arolyl group is also called as an arylcarbonyl group, and includes a carbonyl group having a $C_{6-10}$ aryl group, such as benzoyl, 1-naphthoyl, 2-naphthoyl, etc.
The cycloalkyl moiety of the cycloalkyloxy group, the cycloalkylcarbonyl group, the cycloalkyloxycarbonyl group, the cycloalkylthio group, the cycloalkylsulfinyl group, and the cycloalkylsulfonyl group is the same as defined above.
The cycloalkenyl moiety of the cycloalkenyloxy group, the cycloalkenylcarbonyl group, the cycloalkenyloxycarbonyl group, the cycloalkenylthio group, the cycloalkenylsulfinyl group, and the cycloalkenylsulfonyl group is the same as defined above.
The aryl moiety of the aryloxy group, the aryloxycarbonyl group, the arylthio group, the arylsulfinyl group, and the arylsulfonyl group is the same as defined above.
The heteroaryl moiety of the heteroaryloxy group, the heteroarylcarbonyl group, the heteroaryloxycarbonyl group, the heteroarylthio group, the heteroarylsulfinyl group, and the heteroarylsulfonyl group is the same as defined above.
The aralkyl moiety of the aralkyloxy group, the aralkylcarbonyl group, the aralkyloxycarbonyl group, the aralkylthio group, the aralkyl sulfinyl group, and the aralkyl sulfonyl group is the same as defined above.
The heteroaralkyl moiety of the heteroaralkyloxy group, the heteroaralkylcarbonyl group, the heteroaralkyloxycarbonyl group, the heteroaralkylthio group, the heteroaralkylsulfinyl group and the heteroaralkylsulfonyl group is the same as defined above.

When the alkyl, alkenyl, alkynyl, alkoxy, alkanoyl, alkoxycarbonyl, alkylthio, alkylsulfinyl, and alkylsulfonyl groups are substituted, these groups may have the same or different 1 to 6 substituents selected from the following groups (i) to (v).

(i) a halogen atom, a hydroxy group, a carboxyl group, a cyano group, formyl group, oxo group, a sulfo group (-$SO_2OH$), phosphono group (-$PO(OH)_2$);
(ii) an optionally substituted amino group, an optionally substituted carbamoyl group, an optionally substituted sulfamoyl group, an optionally substituted thiocarbamoyl group;
(iii) an alkoxy group, an alkanoyl group, an alkanoyloxy group, an alkoxycarbonyl group, an alkylthio group, an alkylsulfinyl group, and alkylsulfonyl group (these groups being optionally substituted by a group selected from a halogen atom, a hydroxy group, a carboxyl group and an optionally substituted amino group, an optionally substituted carbamoyl group, an optionally substituted sulfamoyl group, and an optionally substituted thiocarbamoyl group);
(iv) an optionally substituted cycloalkyl group, an optionally substituted cycloalkyloxy group, an optionally substituted cycloalkylcarbonyl group, an optionally substituted cycloalkyloxycarbonyl group, an optionally substituted cycloalkylthio group, an optionally substituted cycloalkylsulfonyl group, an optionally substituted cycloalkylsulfinyl group, an optionally substituted cycloalkenyl group, an optionally substituted, saturated or unsaturated aliphatic heterocyclic group, an optionally substituted, saturated or unsaturated aliphatic heterocyclic oxy group, an optionally substituted, saturated or unsaturated aliphatic heterocyclic sulfonyl group, an optionally substituted, saturated or unsaturated aliphatic heterocyclic carbonyl group, an optionally substituted, saturated or unsaturated aliphatic heterocyclic thio group, an optionally substituted, saturated or unsaturated aliphatic heterocyclic sulfinyl group, an optionally substituted, saturated or unsaturated aliphatic heterocyclic oxycarbonyl group;
(v) an optionally substituted aryl group, an optionally substituted heteroaryl group, an optionally substituted aroyl group, an optionally substituted heteroarylcarbonyl group, an optionally substituted aryloxy group, an optionally substituted heteroaryloxy group, an optionally substituted aryloxycarbonyl group, an optionally substituted heteroaryloxycarbonyl group, an optionally substituted arylthio group, an optionally substituted heteroarylthio group, an optionally substituted arylsulfonyl group, an optionally substituted heteroarylsulfonyl group, an optionally substituted arylsulfinyl group, an optionally substituted heteroarylsulfinyl group, an optionally substituted aralkyloxy group, an optionally substituted aralkylcarbonyl group, an optionally substituted aralkyloxycarbonyl group, an optionally substituted aralkylthio group, an optionally substituted aralkylsulfinyl group, an optionally substituted aralkylsulfonyl group, an optionally substituted heteroaralkyloxy group, an optionally substituted heteroaralkylcarbonyl group, an optionally substituted heteroaralkyloxycarbonyl group, an optionally substituted heteroaralkylthio group, an optionally substituted heteroaralkylsulfinyl group, an optionally substituted heteroaralkylsulfonyl group.

When the cycloalkyl, cycloalkenyl, cycloalkyloxy, cycloalkylcarbonyl, cycloalkyloxycarbonyl, cycloalkylthio, cycloalkylsulfinyl, cycloalkylsulfonyl, a saturated or unsaturated aliphatic heterocycle, saturated or unsaturated aliphatic

heterocyclic oxy, saturated or unsaturated aliphatic heterocyclic carbonyl, saturated or unsaturated aliphatic heterocyclic oxycarbonyl, saturated or unsaturated aliphatic heterocyclic sulfonyl, saturated or unsaturated aliphatic heterocyclic sulfinyl, and saturated or unsaturated aliphatic heterocyclic thio groups are substituted, these groups may have the same or different 1 to 5 substituents selected from the following groups (vi) to (ix):

(vi) a halogen atom, a hydroxy group, a carboxyl group, a cyano group, a nitro group, an oxo group, a thioxo group, a formyl group, a sulfo group (-$SO_2OH$), a phosphono group (-$PO(OH)_2$);
(vii) an optionally substituted amino group, an optionally substituted carbamoyl group, an optionally substituted sulfamoyl group, an optionally substituted thiocarbamoyl group, an optionally substituted aryl group, an optionally substituted heteroaryl group, an optionally substituted aryloxy group, an optionally substituted heteroaryloxy group, an optionally substituted aroyl group, an optionally substituted heteroarylcarbonyl group, an optionally substituted aryloxycarbonyl group, an optionally substituted heteroaryloxycarbonyl group, an optionally substituted arylthio group, an optionally substituted heteroarylthio group, an optionally substituted arylsulfonyl group, an optionally substituted heteroarylsulfonyl group, an optionally substituted arylsulfinyl group, an optionally substituted heteroarylsulfinyl group, an optionally substituted aralkyl group, an optionally substituted aralkyloxy group, an optionally substituted aralkylcarbonyl group, an optionally substituted aralkyloxycarbonyl group, an optionally substituted aralkylthio group, an aralkyl sulfinyl group, an optionally substituted aralkylsulfonyl group, an optionally substituted heteroaralkyl group, an optionally substituted heteroaralkyloxy group, an optionally substituted heteroaralkylcarbonyl group, an optionally substituted heteroaralkyloxycarbonyl group, an optionally substituted heteroaralkylthio group, an optionally substituted heteroaralkylsulfinyl group, an optionally substituted heteroaralkylsulfonyl group;
(viii) an alkyl group, an alkenyl group, an alkynyl group, an alkoxy group, an alkanoyl group, an alkanoyloxy group, an alkoxycarbonyl group, an alkylthio group, an alkylsulfinyl group, an alkylsulfonyl group (these groups may be substituted by the same or different 1 to 5 substituents selected from the following (1) to (3):

(1) a halogen atom, a hydroxy group, a carboxyl group, a formyl group, a sulfo group (-$SO_2OH$), a phosphono group (-$PO(OH)_2$), an optionally substituted amino group, an optionally substituted carbamoyl group, an optionally substituted sulfamoyl group, an optionally substituted thiocarbamoyl group;
(2) an alkoxy group, an alkanoyl group, an alkoxycarbonyl group, an alkanoyloxy group, an alkylthio group, an alkylsulfinyl group, an alkylsulfonyl group (these groups may be substituted by a halogen atom, a hydroxy group, a carboxyl group, an alkoxy group, an optionally substituted amino group, an optionally substituted carbamoyl group, or an optionally substituted sulfamoyl group);
(3) a cycloalkyl group, a cycloalkyloxy group, a cycloalkylsulfonyl group, a cycloalkylcarbonyl group, a cycloalkyloxycarbonyl group, a cycloalkylthio group, a cycloalkyl sulfinyl group, a cycloalkenyl group, a cycloalkenyloxy group, a cycloalkenylsulfonyl group, a cycloalkenylthio group, a cycloalkenylcarbonyl, a cycloalkenyloxycarbonyl group, a cycloalkenylsulfinyl group, a saturated or unsaturated aliphatic heterocyclic group, a saturated or unsaturated aliphatic heterocyclic oxy group, a saturated or unsaturated aliphatic heterocyclic sulfonyl group, a saturated or unsaturated aliphatic heterocyclic thio group, a saturated or unsaturated aliphatic heterocyclic carbonyl group, a saturated or unsaturated aliphatic heterocyclic oxycarbonyl group, a saturated or unsaturated aliphatic heterocyclic sulfinyl group, an aryl group, an aryloxy group, an arylthio group, an arylsulfonyl group, an aroyl group, an aryloxycarbonyl group, an arylsulfinyl group, a heteroaryl group, a heteroaryloxy group, a heteroarylthio group, a heteroarylsulfonyl group, a heteroarylcarbonyl group, a heteroaryloxycarbonyl group, a heteroarylsulfinyl group (these groups may be substituted by a halogen atom, a hydroxy group, a carboxyl group, a formyl group, a cyano group, a sulfo group (-$SO_2OH$), a phosphono group (-$PO(OH)_2$), an alkyl group being optionally substituted by a carboxyl group or an alkoxycarbonyl group, an alkenyl group being optionally substituted by a carboxyl group or an alkoxycarbonyl group, an alkoxy group, an alkanoyl group, an alkoxycarbonyl group, an optionally substituted amino group, an optionally substituted carbamoyl group, or an optionally substituted sulfamoyl group));

(ix) a cycloalkyl group, a cycloalkenyl group, a cycloalkoxy group, a cycloalkylcarbonyl group, a cycloalkoxycarbonyl group, a cycloalkylthio group, a cycloalkylsulfonyl group, a cycloalkylsulfinyl group, a saturated or unsaturated aliphatic heterocyclic group, a saturated or unsaturated aliphatic heterocyclic oxy group, a saturated or unsaturated aliphatic heterocyclic carbonyl group, a saturated or unsaturated aliphatic heterocyclic sulfonyl group, a saturated or unsaturated aliphatic heterocyclic thio group, a saturated or unsaturated aliphatic heterocyclic oxycarbonyl group, a saturated or unsaturated aliphatic heterocyclic sulfinyl group [these groups may be substituted by the same or different 1 to 5 groups selected from the following (1) to (3):

(1) a halogen atom, a hydroxy group, a carboxyl group, a formyl group, a cyano group, a nitro group, a sulfo group (-$SO_2OH$), a phosphono group (-$PO(OH)_2$), an optionally substituted amino group, an optionally substituted carbamoyl group, an optionally substituted sulfamoyl group, an optionally substituted thiocarbamoyl group;

(2) an alkyl group, an alkoxy group, an alkanoyl group, an alkoxycarbonyl group, an alkanoyloxy group, an alkylthio group, an alkylsulfinyl group, an alkylsulfonyl group (thee groups may be substituted by a halogen atom, a hydroxy group, a carboxyl group, an alkoxy group, an optionally substituted amino group, an optionally substituted carbamoyl group, or an optionally substituted sulfamoyl group);

(3) a cycloalkyl group, a cycloalkyloxy group, a cycloalkylsulfonyl group, a cycloalkylcarbonyl group, a cycloalkyloxycarbonyl group, a cycloalkylthio group, a cycloalkylsulfinyl group, a cycloalkenyl group, a saturated or unsaturated aliphatic heterocyclic group, a saturated or unsaturated aliphatic heterocyclic oxy group, a saturated or unsaturated aliphatic heterocyclic sulfonyl group, a saturated or unsaturated aliphatic heterocyclic thio group, a saturated or unsaturated aliphatic heterocyclic carbonyl group, a saturated or unsaturated aliphatic heterocyclic oxycarbonyl group, a saturated or unsaturated aliphatic heterocyclic sulfinyl group, an aryl group, an aryloxy group, an arylthio group, an arylsulfonyl group, an aroyl group, an aryloxycarbonyl group, an arylsulfinyl group, a heteroaryl group, a heteroaryloxy group, a heteroarylthio group, a heteroarylsulfonyl group, a heteroarylcarbonyl group, a heteroaryloxycarbonyl group, a heteroarylsulfinyl group, an aralkyl group, an aralkyloxy group, an aralkylcarbonyl group, an aralkyloxycarbonyl group, an aralkylthio group, an aralkylsulfinyl group, an aralkylsulfonyl group, a heteroaralkyl group, a heteroaralkyloxy group, a heteroaralkylcarbonyl group, a heteroaralkyloxycarbonyl group, a heteroaralkylthio group, a heteroaralkylsulfinyl group, a heteroaralkylsulfonyl group (these groups may be substituted by a halogen atom, a hydroxy group, a carboxyl group, a formyl group, a cyano group, a sulfo group ($-SO_2OH$), a phosphono group ($-PO(OH)_2$), an alkyl group being optionally substituted by a carboxyl group or an alkoxycarbonyl group, an alkenyl group being optionally substituted by a carboxyl group or an alkoxycarbonyl group, an alkoxy group, an alkanoyl group, an alkoxycarbonyl group, an optionally substituted amino group, an optionally substituted carbamoyl group, or an optionally substituted sulfamoyl group)].

When the aryl, aryloxy, aroyl, aryloxycarbonyl, arylthio, arylsulfinyl, arylsulfonyl, heteroaryl, heteroaryloxy, heteroarylcarbonyl, heteroaryloxycarbonyl, heteroarylthio, heteroarylsulfinyl, heteroarylsulfonyl, aralkyl, aralkyloxy, aralkylcarbonyl, aralkyloxycarbonyl, aralkylthio, aralkylsulfinyl, aralkylsulfonyl, heteroaralkyl, heteroaralkyloxy, heteroaralkylcarbonyl, heteroaralkyloxycarbonyl, heteroaralkylthio, heteroaralkylsulfinyl and heteroaralkylsulfonyl groups are substituted, these groups may have the same or different 1 to 5 substituents selected from the following (x) to (xiv):

(x) a halogen atom, a hydroxy group, a carboxyl group, a cyano group, a nitro group, a formyl group, a sulfo group ($-SO_2OH$), a phosphono group ($-PO(OH)_2$);

(xi) an optionally substituted amino group, an optionally substituted carbamoyl group, an optionally substituted sulfamoyl group, an optionally substituted thiocarbamoyl group;

(xii) an alkyl group, an alkenyl group, an alkynyl group, an alkoxy group, an alkanoyl group, an alkanoyloxy group, an alkoxycarbonyl group, an alkylthio group, an alkylsulfinyl group, an alkylsulfonyl group [these groups may be substituted by 1 to 5 groups selected from the following (1) to (4):

(1) a halogen atom, a hydroxy group, a carboxyl group, a formyl group, an oxo group, a cyano group, a sulfo group ($-SO_2OH$), a phosphono group ($-PO(OH)_2$);

(2) an optionally substituted amino group, an optionally substituted carbamoyl group, an optionally substituted sulfamoyl group, an optionally substituted thiocarbamoyl group;

(3) an alkoxy group, an alkoxycarbonyl group, an alkylthio group, an alkylsulfinyl group, an alkylsulfonyl group [these groups may be substituted by (a) or (b):

(a) a halogen atom, a hydroxy group, a carboxyl group, alkoxy group, an optionally substituted amino group, an optionally substituted carbamoyl group, an optionally substituted sulfamoyl group, an optionally substituted thiocarbamoyl group;

(b) a cycloalkyl group, a cycloalkyloxy group, a cycloalkylthio group, a cycloalkylsulfinyl group, a cycloalkylsulfonyl group, a cycloalkenyl group, a cycloalkenyloxy group, a saturated or unsaturated aliphatic heterocyclic group, a saturated or unsaturated aliphatic heterocyclic oxy group, a saturated or unsaturated aliphatic heterocyclic thio group, a saturated or unsaturated aliphatic heterocyclic sulfinyl group, a saturated or unsaturated aliphatic heterocyclic sulfonyl group, an aryl group, an aryloxy group, an arylthio group, an arylsulfonyl group, an arylsulfinyl group, a heteroaryl group, a heteroaryloxy group, a heteroarylthio group, a heteroarylsulfonyl group, a heteroarylsulfinyl group (these groups may be substituted by a group selected from a halogen atom, a hydroxy group, a carboxyl group, a formyl group, a cyano group, a sulfo group ($-SO_2OH$), a phosphono group ($-PO(OH)_2$), an alkyl group being optionally substituted by a carboxyl group, an alkenyl group being optionally substituted by a carboxyl group, an alkoxy group being optionally substituted by a carboxyl group, an alkanoyl group, an alkoxycarbonyl group, an alkylsulfonyl group being optionally substituted by a carboxyl group, an optionally substituted amino group, an optionally substituted carbamoyl group, or an optionally substituted sulfamoyl group)];

(4) a cycloalkyl group, a cycloalkyloxy group, a cycloalkylthio group, a cycloalkylsulfinyl group, a cycloalkylsulfonyl group, a cycloalkenyl group, a cycloalkenyloxy group, a saturated or unsaturated aliphatic heterocyclic group, a saturated or unsaturated aliphatic heterocyclic oxy group, a saturated or unsaturated aliphatic heterocyclic thio group, a saturated or unsaturated aliphatic heterocyclic sulfinyl group, a saturated or unsaturated aliphatic heterocyclic sulfonyl group, an aryl group, an aryloxy group, an arylthio group, an arylsulfonyl group, an arylsulfinyl group, a heteroaryl group, a heteroaryloxy group, a heteroarylthio group, a heteroarylsulfonyl group, a heteroarylsulfinyl group (these groups may optionally be substituted by a halogen atom, a hydroxy group, a carboxyl group, a formyl group, a cyano group, a sulfo group ($-SO_2OH$), a phosphono group ($-PO(OH)_2$), an alkyl group being optionally substituted by a carboxyl group, an alkenyl group being optionally substituted by a carboxyl group, an alkoxy group, an alkanoyl group, an alkoxycarbonyl group, an optionally substituted amino group, an optionally substituted carbamoyl group, or an optionally substituted sulfamoyl group)];

(xiii) a cycloalkyl group, a cycloalkenyl group, a cycloalkyloxy group, a cycloalkylcarbonyl group, a cycloalkylthio group, a cycloalkylsulfinyl group, a cycloalkylsulfonyl group, a cycloalkenyl group, a cycloalkenyloxy group, a saturated or unsaturated aliphatic heterocyclic group, a saturated or unsaturated aliphatic heterocyclic oxy group, a saturated or unsaturated aliphatic heterocyclic carbonyl group, a saturated or unsaturated aliphatic heterocyclic thio group, a saturated or unsaturated aliphatic heterocyclic sulfinyl group, a saturated or unsaturated aliphatic heterocyclic sulfonyl group [these groups may be substituted by the same or different 1 to 5 groups selected from the following (1) to (3):

(1) a halogen atom, a hydroxy group, a carboxyl group, a formyl group, an oxo group, a thioxo group, a sulfo group ($-SO_2OH$), a phosphono group ($-PO(OH)_2$);
(2) an alkoxy group, an alkoxycarbonyl group, an alkylthio group, an alkylsulfinyl group, an alkylsulfonyl group, an amino group being optionally substituted by the same or different 1 or 2 alkyl groups, a carbamoyl group being optionally substituted by the same or different 1 or 2 alkyl groups, or a sulfamoyl group being optionally substituted by the same or different 1 or 2 alkyl groups (these groups may be substituted by a halogen atom, a hydroxy group, a carboxyl group, an alkoxy group, an alkoxycarbonyl group, an amino group being optionally substituted by the same or different 1 or 2 alkyl groups, or a carbamoyl group being optionally substituted by the same or different 1 or 2 alkyl groups);
(3) a cycloalkyl group, a cycloalkyloxy group, a cycloalkenyl group, a cycloalkenyloxy group, a saturated or unsaturated aliphatic heterocyclic group, a saturated or unsaturated aliphatic heterocyclic oxy group, an aryl group, an aryloxy group, an arylthio group, an arylsulfonyl group, a heteroaryl group, a heteroaryloxy group, a heteroarylthio group, a heteroarylsulfonyl group (these groups may be substituted by a halogen atom, a hydroxy group, a carboxyl group, an alkyl group, an alkoxy group, an amino group being optionally substituted by the same or different 1 or 2 alkyl groups, a carbamoyl group being optionally substituted by the same or different 1 or 2 alkyl groups, or a sulfamoyl group being optionally substituted by the same or different 1 or 2 alkyl groups)];

(xiv) an aryl group, an aryloxy group, an arylthio group, an arylsulfonyl group, an aroyl group, an aryloxycarbonyl group, an arylsulfinyl group, a heteroaryl group, a heteroaryloxy group, a heteroarylthio group, a heteroarylsulfonyl group, a heteroarylcarbonyl group, a heteroaryloxycarbonyl group, a heteroarylsulfinyl group [these groups may be substituted by the same or different 1 to 5 groups selected from the following (1) to (3):

(1) a halogen atom, a hydroxy group, a carboxyl group, a cyano group, a nitro group, a formyl group, a sulfo group ($-SO_2OH$), a phosphono group ($-PO(OH)_2$);
(2) an alkyl group, an alkoxy group, an alkylthio group, an alkylsulfonyl group, an alkylsulfinyl group, an alkoxycarbonyl group, an amino group being optionally substituted by the same or different 1 or 2 alkyl groups, a carbamoyl group being optionally substituted by the same or different 1 or 2 alkyl groups, or a sulfamoyl group being optionally substituted by the same or different 1 or 2 alkyl groups (these groups may be substituted by a halogen atom, a hydroxy group, a carboxyl group, an alkoxy group, an alkoxycarbonyl group, an amino group being optionally substituted by the same or different 1 or 2 alkyl groups, or a carbamoyl group being optionally substituted by the same or different 1 or 2 alkyl groups);
(3) a cycloalkyl group, a cycloalkenyl group, a cycloalkenyloxy group, a saturated or unsaturated aliphatic heterocyclic group, a saturated or unsaturated aliphatic heterocyclic oxy group, an aryl group, an aryloxy group, an arylthio group, an arylsulfonyl group, a heteroaryl group, a heteroaryloxy group, a heteroarylthio group, a

heteroarylsulfonyl group (these groups may be substituted by a halogen atom, a hydroxy group, a carboxyl group, a carbamoyl group, an alkyl group, an alkoxy group, an alkylsulfonyl group, an amino group being optionally substituted by the same or different 1 or 2 alkyl groups, a carbamoyl group being optionally substituted by the same or different 1 or 2 alkyl groups, or a sulfamoyl group being optionally substituted by the same or different 1 or 2 alkyl groups)].

In addition, the adjacent two substituents of the aryl, heteroaryl, aralkyl, heteroaralkyl, aryloxy, aroyl, aryloxycarbonyl, arylthio, arylsulfinyl, arylsulfonyl, heteroaryloxy, heteroarylcarbonyl, heteroaryloxycarbonyl, heteroarylthio, heteroarylsulfinyl, heteroarylsulfonyl, aralkyloxy, aralkylcarbonyl, aralkyloxycarbonyl, aralkylsulfonyl, heteroaralkyloxy, heteroaralkylcarbonyl, heteroaralkyloxycarbonyl, and heteroaralkylsulfonyl groups may combine each other to form a divalent group of the following formulae (T1) to (T18):

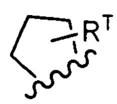
(T1)

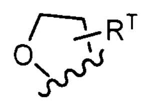
(T2)

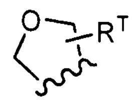
(T3)

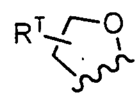
(T4)

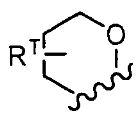
(T5)

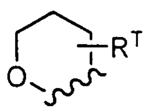
(T6)

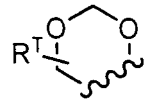
(T7)

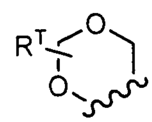
(T8)

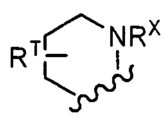
(T9)

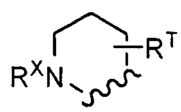
(T10)

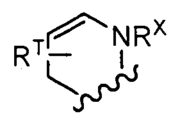
(T11)

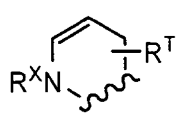
(T12)

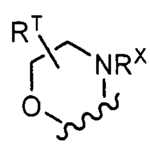
(T13)

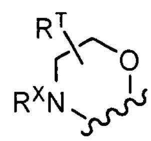
(T14)

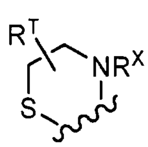
(T15)

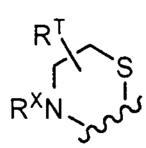
(T16)

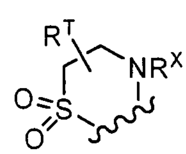
(T17)

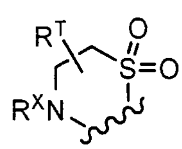
(T18)

[wherein $R^T$ does not exist or the number of $R^T$ is 1 or more, and each is independently the following (1) to (3):

(1) a halogen atom, a hydroxy group, an oxo group, a carboxyl group, a carbamoyl group being optionally substituted by the same or different 1 or 2 substituents, a saturated aliphatic heterocyclic oxycarbonyl group;
(2) an alkyl group, an alkoxycarbonyl group, an alkoxy group (these groups may be substituted by a halogen atom, a carboxyl group or an alkoxy group);
(3) two of $R^T$ combine each other to form methylene, ethylene, trimethylene, tetramethylene or butenylene, and further combine with 1 or 2 ring-forming carbon atoms to additionally form another ring; and $R^x$ is a hydrogen atom

or an alkyl group].

In the present invention, the saturated aliphatic heterocyclic group of the above-mentioned saturated aliphatic heterocyclic oxycarbonyl group includes, for example, a 5- or 6-membered saturated aliphatic heterocyclic containing 1 or 2 atoms selected from oxygen atom, nitrogen atom and/or sulfur atom. The saturated aliphatic heterocyclic oxycarbonyl group includes, for example, tetrahydrofuranyloxycarbonyl, tetrahydropyranyloxycarbonyl, dihydrofuranyloxycarbonyl, tetrahydrothiopyranyloxycarbonyl, tetrahydrodioxothiopyranyloxycarbonyl, pyrrolidinyloxycarbonyl, piperidyloxycarbonyl, piperazyloxycarbonyl, imidazolidinyloxycarbonyl, oxazolidinyloxycarbonyl, and thiazolidinyloxycarbonyl.

The substituent of the substituted amino, substituted carbamoyl, substituted thiocarbamoyl, and substituted sulfamoyl groups is the same or different 1 or 2 groups selected from the following groups (1) to (3):

(1) an alkyl group, an alkylcarbonyl group, an alkoxycarbonyl group, an alkylsulfonyl group, an alkenyl group [these groups may be substituted by a group selected from the following groups (a) or (b):

(a) a halogen atom, a hydroxy group, a carboxyl group, an oxo group, an alkoxy group, an alkoxycarbonyl group, an alkylthio group, an alkylsulfinyl group, an alkylsulfonyl group, a sulfo group ($-SO_2OH$), a phosphono group ($-PO(OH)_2$), an amino group being optionally substituted by the same or different 1 or 2 alkyl groups, a carbamoyl group being optionally substituted by the same or different 1 or 2 alkyl groups;
(b) a cycloalkyl group, a cycloalkyloxy group, a cycloalkylthio group, a cycloalkylsulfonyl group, a cycloalkenyl group, a cycloalkenyloxy group, a saturated or unsaturated aliphatic heterocyclic group, a saturated or unsaturated aliphatic heterocyclic oxy group, a saturated or unsaturated aliphatic heterocyclic thio group, a saturated or unsaturated aliphatic heterocyclic sulfonyl group, an aryl group, an aryloxy group, an arylthio group, an arylsulfonyl group, an aralkyl group, a heteroaryl group, a heteroaryloxy group, a heteroarylthio group, a heteroarylsulfonyl group, a heteroaralkyl group (these groups may optionally be substituted by a group selected from a halogen atom, a hydroxy group, a carboxyl group, a cyano group, an alkyl group being optionally substituted by a carboxyl group, an alkoxy group being optionally substituted by a carboxyl group, an amino group being optionally substituted by the same or different 1 or 2 alkyl groups, a carbamoyl group being optionally substituted by the same or different 1 or 2 alkyl groups and a sulfamoyl group being optionally substituted by the same or different 1 or 2 alkyl groups)];

(2) an aryl group, a heteroaryl group, an aralkyl group, a heteroaralkyl group, a cycloalkyl group, an aroyl group, a heteroarylcarbonyl group, an aralkylcarbonyl group, a heteroaralkylcarbonyl group, a cycloalkylcarbonyl group, an aryloxycarbonyl group, a heteroaryloxycarbonyl group, an aralkyloxycarbonyl group, a heteroaralkyloxycarbonyl group, a cycloalkyloxycarbonyl group, an arylsulfonyl group, a heteroarylsulfonyl group, an aralkylsulfonyl group, a heterbaralkylsulfonyl group, a cycloalkylsulfonyl group, a saturated or unsaturated aliphatic heterocyclic group, a saturated or unsaturated aliphatic heterocyclic carbonyl group, a saturated or unsaturated aliphatic heterocyclic oxycarbonyl group, a saturated or unsaturated aliphatic heterocyclic sulfonyl group [these groups may be substituted by the same or different 1 to 5 groups selected from the following (a) and (b):

(a) a halogen atom, a hydroxy group, a carboxyl group, a cyano group, a nitro group;
(b) an alkyl group, an alkoxy group, an alkoxycarbonyl group, an alkylsulfonyl group, an amino group being optionally substituted by the same or different 1 or 2 alkyl groups, a carbamoyl group being optionally substituted by the same or different 1 or 2 alkyl groups and a sulfamoyl group being optionally substituted by the same or different 1 or 2 alkyl groups (these group may be substituted by a group selected from a halogen atom, a hydroxy group, a carboxyl group, an alkoxy group, an alkoxycarbonyl group, an alkylsulfonyl group, an aryl group, an amino group being optionally substituted by the same or different 1 or 2 alkyl groups, and a carbamoyl group being optionally substituted by the same or different 1 or 2 alkyl groups)];

(3) two substituents may combine each other together with an adjacent nitrogen atom to form a $C_{2-6}$ alkylene group which may optionally be substituted by a carboxyl group, a hydroxy group, an alkyl group, an alkoxy group or an alkoxycarbonyl group, and any $-CH_2-$ of said alkylene may optionally be replaced by a divalent group such as -O-, $-NR^{20}-$, -SO-, $-SO_2-$, -S- and -CO [in which $R^{20}$ is the following (a) or (b):

(a) a hydrogen atom,
(b) an alkyl group, an alkoxycarbonyl group, an alkylcarbonyl group and an alkylsulfonyl group (these groups may optionally be substituted by a hydroxy group, an alkoxy group, a carboxyl group, an amino group being optionally substituted by the same or different 1 or 2 alkyl groups, or a carbamoyl group being optionally substituted by the same or different 1 or 2 alkyl groups)].

The saturated or unsaturated, monocyclic, bicyclic or tricyclic nitrogen-containing heterocyclic group formed by combining $R^{18}$ and $R^{19}$ together with a nitrogen atom to which they are bonded includes, for example, an optionally substituted 4- to 14-membered saturated or unsaturated nitrogen-containing heterocyclic group containing 1 to 4 heteroatoms selected from 0 to 2 oxygen atoms, 0 to 2 sulfur atoms and 1 to 4 nitrogen atoms. The unsaturated nitrogen-containing heterocyclic group includes a non-aromatic unsaturated nitrogen-containing heterocyclic group having 1 or 2 double bonds within the ring. For example, azetidine, pyrrolidine, piperidine, piperazine, azepane, morpholine, thiazolidine, isothiazolidine, thiomorpholine, tetrahydropyrimidine, tetrahydroquinoline, tetrahydroisoquinoline, tetrahydrothienopyridine, tetrahydrothiazolopyridine, tetrahydropyrazolopyridine, tetrahydroimidazopyrazine, tetrahydrotriazopyrazine, tetrahydropyrazolopyrazine, dihydropyrrolopyrimidine, dihydropyrrolopyridazine, dihydropyrrolopyridine, tetrahydropyrrolopyridine, tetrahydrofuropyridine, tetrahydroimidazopyridine, tetrahydrooxazolopyridine, tetrahydroisothiazolopyridine, tetrahydroisoxazolopyridine, tetrahydroimidazopyrimidine, tetrahydrotriazopyrimidine, tetrahydropyrazolopyrimidine, tetrahydronaphthyridine, tetrahydropyridopyrimidine, tetrahydropyridopyrazine, tetrahydropyridopyridazine, tetrahydroimidazodiazepine, tetrahydrotriazolodiazepine, tetrahydropyrazolodiazepine, tetrahydrotriazolodiazepine, tetrahydrothienoazepine, hexahydropyrroloazepine, tetrahydrofuroazepine, tetrahydrothiazoloazepine, hexahydroimidazoazepine, tetrahydrooxazoloazepine, tetrahydroisothiazoloazepine, hexahydropyrazoloazepine, tetrahydroisoxazoloazepine, hexahydrotriazoloazepine, dihydroimidazoimidazole, dihydroimidazotriazole, dihydroimidazopyrazole, dihydroimidazotriazole, dihydrothienopyrrole, tetrahydropyrrolopyrrole, dihydrofuropyrrole, dihydropyrrolothiazole, tetrahydropyrroloimidazole, dihydropyrrolooxazole, dihydropyrroloisothiazole, tetrahydropyrrolopyrazole, dihydropyrroloisoxazole, tetrahydropyrrolotriazole, isoindoline, azabicyclohexane, azabicyclooctane, azabicycloheptane, azabicyclononane, and tetrahydrocarboline can be exemplified. Moreover, such nitrogen-containing heterocyclic group may form together with a saturated or unsaturated aliphatic heterocyclic group wherein an aryl group or a heteroaryl group is fused to form a spiro ring, for example, groups of the following formulae (S1) to (S7):

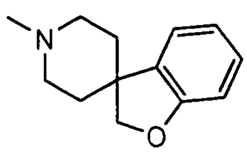
(S1)

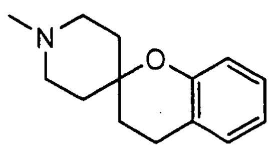
(S2)

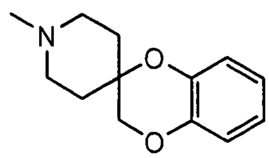
(S3)

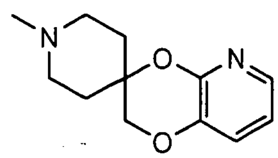
(S4)

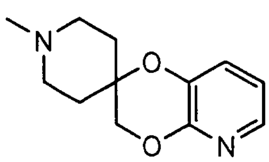
(S5)

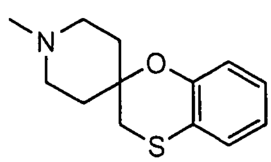
(S6)

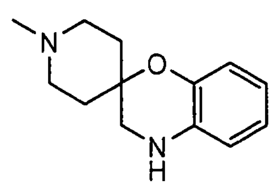
(S7)

When the saturated or unsaturated, monocyclic, bicyclic or tricyclic nitrogen-containing heterocyclic group formed by combining $R^{18}$ and $R^{19}$ together with a nitrogen atom to which they are bonded is substituted, then these groups may have the same or different 1 to 5 substituents, which is the same ones as disclosed for the above-mentioned saturated or unsaturated aliphatic heterocyclic group. The substitution position of these substituents is not necessarily defined, and these substituents can bond any carbon atom or nitrogen atom as long as that position is chemically stable.
When such a substituent bonds to a carbon atom, the substituent is preferably ones in the following groups (1) to (4):

(1) a halogen atom, a hydroxy group, a carboxyl group, a cyano group, a nitro group;
(2) an alkyl group, an alkenyl group, an alkynyl group, an alkoxy group, an alkanoyl group, an alkanoyloxy group, an alkoxycarbonyl group, an alkylthio group, an alkylsulfinyl group, an alkylsulfonyl group, an amino group being optionally substituted by the same or different 1 or 2 alkyl groups, a carbamoyl group being optionally substituted by the same or different 1 or 2 alkyl groups, a sulfamoyl group being optionally substituted by the same or different 1 or 2 alkyl groups (these groups may be substituted by a group selected from a halogen atom, a hydroxy group, a carboxyl group, an alkoxy group, an alkoxycarbonyl group, an alkylthio group, an alkylsulfinyl group, an alkylsulfonyl group, an amino group being optionally substituted by the same or different 1 or 2 alkyl groups, a carbamoyl group

being optionally substituted by the same or different 1 or 2 alkyl groups and a sulfamoyl group being optionally substituted by the same or different 1 or 2 alkyl groups);

(3) an optionally substituted aryl group, an optionally substituted aralkyl group, an optionally substituted aryloxy group, an optionally substituted aroyl group, an optionally substituted arylthio group, an optionally substituted arylsulfinyl group, an optionally substituted arylsulfonyl group, an optionally substituted heteroaryl group, an optionally substituted heteroaralkyl group, an optionally substituted heteroaryloxy group, an optionally substituted heteroarylcarbonyl group, an optionally substituted heteroarylthio group, an optionally substituted heteroarylsulfinyl group, an optionally substituted heteroarylsulfonyl group;

(4) a cycloalkyloxy group, a cycloalkyloxycarbonyl group, a cycloalkylthio group, a cycloalkylsulfinyl group, a cycloalkylsulfonyl group, a saturated or unsaturated aliphatic heterocyclic group, a saturated or unsaturated aliphatic heterocyclic oxy group, a saturated or unsaturated aliphatic heterocyclic oxycarbonyl group, a saturated or unsaturated aliphatic heterocyclic thio group, a saturated or unsaturated aliphatic heterocyclic sulfinyl group, a saturated or unsaturated aliphatic heterocyclic sulfonyl group (these groups may be substituted by a group selected from a halogen atom, a hydroxy group, a carboxyl group, a formyl group, a cyano group, a sulfo group ($-SO_2OH$), a phosphono group ($-PO(OH)_2$), an alkyl group being optionally substituted by a carboxyl group or an alkoxycarbonyl group, an alkenyl group being optionally substituted by a carboxyl group or an alkoxycarbonyl group, an alkoxy group, an alkanoyl group, an alkoxycarbonyl group, an optionally substituted amino group, an optionally substituted carbamoyl group, and an optionally substituted sulfamoyl group).

When these substituents bond to a nitrogen atom, then the substituent may be ones as disclosed in the following groups (5) and (6):

(5) an alkyl group, an alkoxycarbonyl group, an alkylcarbonyl group or an alkylsulfonyl group and a carbamoyl group being optionally substituted by the same or different 1 or 2 alkyl groups (these groups may be substituted by a group selected from a hydroxy group, an alkoxy group, a carboxyl group, an amino group being optionally substituted by the same or different 1 or 2 alkyl groups, and a carbamoyl group being optionally substituted by the same or different 1 or 2 alkyl groups);

(6) any groups as disclosed in the above (3) and (4).

Preferable $R^1$ of the formula (1) is, for example, an optionally substituted aryl group, an optionally substituted heteroaryl group, an optionally substituted aralkyl group, an optionally substituted heteroaralkyl group, an optionally substituted cycloalkyl group, an optionally substituted cycloalkenyl group, an alkyl group substituted by an optionally substituted cycloalkyl group, an alkyl group substituted by an optionally substituted cycloalkenyl group, and among them, an optionally substituted benzyl group, and an alkyl group having a $C_1$ carbon chain being substituted by an optionally substituted cycloalkyl group, i.e., a methyl group substituted by an optionally substituted cycloalkyl group are more preferable. In addition, the substituent of said substituted aryl group, the substituted heteroaryl group, the substituted aralkyl group, the substituted heteroaralkyl group, the substituted cycloalkyl group, the substituted cycloalkenyl group, the alkyl group substituted by a substituted cycloalkyl group and the alkyl group substituted by a substituted cycloalkenyl group is preferably a halogen atom, an alkyl group, and an alkoxy group.

Preferable alkyl group for $R^2$ of the formula (1) includes, for example, a straight chain or branched chain $C_{1-4}$ alkyl group. For example, methyl, ethyl, propyl, isopropyl, butyl, isobutyl, sec-butyl, and tert-butyl can be exemplified.

Preferable cycloalkyl group for $R^2$ includes, for example, a 3- to 6-membered cycloalkyl group. For example, cyclopropyl, cyclobutyl, cyclopentyl group, and cyclohexyl can be exemplified.

Preferable aralkyl group for $R^2$ includes, for example, a straight chain or branched chain $C_{1-3}$ alkyl group which is substituted by a phenyl group. For example, benzyl (phenylmethyl), phenethyl (2-phenylethyl), and 3-phenylpropyl can be exemplified.

Preferable heteroaralkyl group for $R^2$ includes, for example, a straight chain or branched chain $C_{1-3}$ alkyl group which is substituted by a heteroaryl group, and the binding position between the heteroaryl group and the alkyl group is not necessarily specified, and can be any position as long as it is chemically stable. For example, 2-pyridylmethyl, 3-pyridylmethyl, 4-pyridylmethyl, 2-furylmethyl (furfuryl), 3-furylmethyl, 2-thienylmethyl, 3-thienylmethyl, 2-pyridylethyl, 3-pyridylethyl, and 4-pyridylethyl, etc. can be exemplified.

The alkanoyl group for $R^2$ includes a straight chain or branched chain $C_{2-4}$ alkanoyl group, for example, acetyl, propionyl, butyryl, and isobutyryl.

The alkoxycarbonyl group for $R^2$ includes a straight chain or branched chain $C_{2-4}$ alkoxycarbonyl group, for example, methoxycarbonyl, ethoxycarbonyl, propoxycarbonyl, and isopropoxycarbonyl.

The carbamoyl group being optionally substituted by an alkyl group for $R^2$ includes a carbamoyl group being optionally substituted by the same or different 1 or 2 $C_{1-3}$ alkyl groups (methyl, ethyl, propyl or isopropyl).

Preferable $R^2$ is a hydrogen atom, an optionally substituted $C_{1-4}$ alkyl group, an optionally substituted $C_{2-4}$ alkoxycarbonyl group and an optionally substituted aryl group. Especially preferable $R^2$ is a hydrogen atom.

In the formula (1), preferable $-W^4=W^5-W^6=W^7-$ is a group of the formula (a): $-CR^4=CR^5-CR^6=CR^7-$ (in which

$R^4$, $R^5$, $R^6$ and $R^7$ are independently the same or different, and each is a group of the formula:-E-A), and a group of the formula (d): $-CR^4=CR^5-N=CR^7-$ (in which $R^4$, $R^5$ and $R^7$ are independently the same or different, and each is a group of the formula: -E-A).
In the formula: -E-A, when E is a group of the following formulae 1) to 14):

1) $-C(R^{16})R^{17}-$,
2) -O-,
3) $-S(=O)_m-$,
4) $-S(=O)_2NR^{16}-$,
5) -C(=O)-,
6) -C(=O)O-,
7) $-C(=O)NR^{16}-$,
8) $-C(=NR^{16})NR^{17}-$,
9) $-NR^{16}-$,
10) $-N(R^{16})C(=O)-$,
11) $-N(R^{16})S(=O)_2-$,
12) $-N(R^{16})C(=O)N(R^{17})-$,
13) $-N(R^{16})S(=O)_2N(R^{17})-$,
14) $-P(=O)(OR^{16})_2-$

(in which $R^{16}$ and $R^{17}$ are independently a hydrogen atom, or a $C_{1-3}$ alkyl group, or in the formulae 8), 12) and 13), $R^{16}$ and $R^{17}$ may combine each other to form a $C_{2-4}$ alkylene group, and m is 0, 1, or 2), then A is bonded to the right side of the divalent group of the above formulae 1) to 14).
In the present description, when a different compound is obtained in cases where the binding direction of the divalent group is varied, then unless indicated specifically, the divalent group is intended to bond in a direction as indicated by the chemical structure.
The $C_{1-3}$ alkyl group for $R^{16}$ and $R^{17}$ of the formulae 1), 4), and 7) to 14) includes a straight chain or branched chain $C_{1-3}$ alkyl group, for example, methyl, ethyl, propyl, and isopropyl.
In the formulae 8), 12) and 13), when $R^{16}$ and $R^{17}$ combine each other to form a $C_{2-4}$ alkylene group, the divalent group of the formulae 8), 12) and 13) may be groups of the formulae (R1) to (R9):
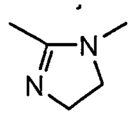
(R1)

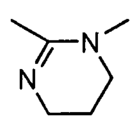
(R2)

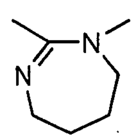
(R3)

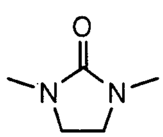
(R4)

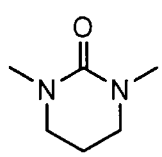
(R5)

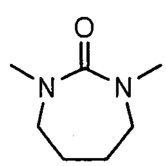
(R6)

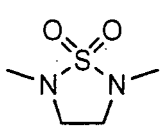
(R7)

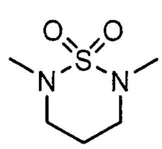
(R8)

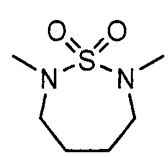
(R9)

The group of the formula (a): $-CR^4=CR^5-CR^6=CR^7-$ for $-W^4=W^5-W^6=W^7-$ is preferably a group of the formula:

-CH=CH-C(-E-A)=CH-, and among them, a group of the formula: -CH=CH-C($NO_2$)=CH-, and a group of the formula: -CH=CH-C(CN)=CH- are especially preferable. The group of the formula (d): -$CR^4$=$CR^5$-N=$CR^7$- of -$W^4$=$W^5$-$W^6$=$W^7$- is preferably a group of the formula: -CH=$CR^5$-N=$CR^7$-. Namely, preferable compound of the formula (1) is a compound of the formulae (2), (3) and (4).

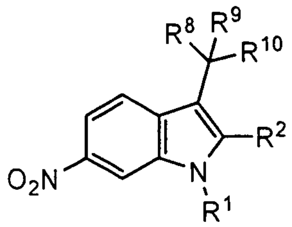

(2)

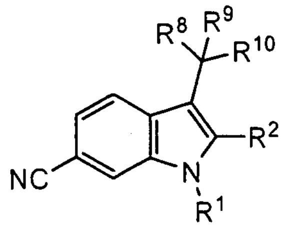

(3)

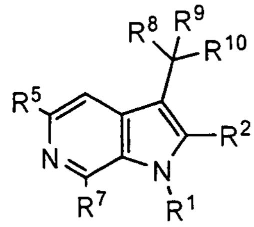

(4)

In the formula (4), especially preferable group for $R^5$ and $R^7$ is a halogen atom, which is independently the same or different.

In the formula (1), preferable group for $R^8$ is a group of the formula: -$OR^{11}$, and among them, a hydroxy group is especially preferable.

The $C_{1-5}$ alkyl group for $R^{11}$ and $R^{12}$ is a straight chain or branched chain $C_{1-5}$ alkyl group, such as methyl, ethyl, propyl, isopropyl, butyl, and pentyl. The substituent of said alkyl group is a halogen atom, a hydroxy group, or a $C_{1-3}$ alkoxy group.

The $C_{1-6}$ alkyl group substituted by one or more halogen atoms for $R^9$ includes a straight chain or branched chain $C_{1-6}$ alkyl group being substituted by the same or different 1 to 7 halogen atoms (for example, methyl, ethyl, propyl, isopropyl, butyl, isobutyl, sec-butyl, tert-butyl, pentyl, isopentyl, neopentyl, tert-pentyl, 1-methylbutyl, or hexyl, etc.). The above-mentioned halogen atom is preferably a fluorine atom or a chlorine atom.

The $C_{3-6}$ cycloalkyl group substituted by one or more halogen atoms for $R^9$ includes a saturated 3- to 6-membered cycloalkyl group being substituted by the same or different 1 to 5 halogen atoms (for example, cyclopropyl, cyclobutyl, cyclopentyl, or cyclohexyl). The above-mentioned halogen atom is preferably a fluorine atom or a chlorine atom.

Preferable group for $R^9$ is a $C_{1-3}$ alkyl group being substituted by the same or different 1 to 5 halogen atoms, and trifluoromethyl is especially preferable.

The alkyl group for $R^{13}$ and $R^{14}$ includes a straight chain or branched chain $C_{1-3}$ alkyl group. For example, methyl, ethyl, propyl, and isopropyl can be exemplified.

The cycloalkane group formed by combining $R^{13}$ and $R^{14}$ together with a carbon atom to which they are bonded includes a 3- to 7-membered cycloalkane, for example, cyclopropane, cyclobutane, cyclopentane, cyclohexane or cycloheptane. Cyclobutane, cyclopentane or cyclohexane are preferable.

Preferable n of the formula: -[C($R^{13}$)$R^{14}$]$_n$-$R^{15}$ for $R^{10}$ of the formula (1) is an integer of 0 to 6, and an integer of 1 to 2 is especially preferable.

The formula: -[C($R^{13}$)$R^{14}$]n-$R^{15}$ for $R^{10}$ of the formula (1) is preferably the formula: -$CH_2$-$R^{15}$, the formula: -$(CH_2)_2$-$R^{15}$, the formula: -$(CH_2)_3$-$R^{15}$, the formula: -$CH_2C(=O)$-$R^{15}$, or the formula: -C(=O)-$R^{15}$, and among them, the formula: -$CH_2$-$R^{15}$ is especially preferable.

Preferable group for $R^{15}$ is an optionally substituted alkenyl group, an optionally substituted aryl group, an optionally substituted heteroaryl group, an optionally substituted, saturated or unsaturated aliphatic heterocyclic group, an optionally substituted alkylthio group, an optionally substituted alkylsulfonyl group, an optionally substituted arylthio group, an optionally substituted heteroarylthio group, an optionally substituted, saturated or unsaturated aliphatic heterocyclic thio group, an optionally substituted arylsulfonyl group, an optionally substituted heteroarylsulfonyl group, an optionally substituted, saturated or unsaturated aliphatic heterocyclic sulfonyl group, or a group of the formula: N($R^{18}$)$R^{19}$. Among them, an optionally substituted heteroaryl group, or a group of the formula: N($R^{18}$)$R^{19}$ is especially preferable.

$R^{18}$ and $R^{19}$ preferably combine each other together with a nitrogen atom to which they are bonded to form a substituted saturated or unsaturated monocyclic or bicyclic nitrogen-containing heterocyclic group. Among them, the group of the formula: N($R^{18}$)$R^{19}$ is a substituted piperidino group, tetrahydroquinolinyl, tetrahydroisoquinolinyl, tetrahydrothienopyridyl, tetrahydrothiazoropyridyl, tetrahydropyrazolopyridyl, a substituted piperazinyl group, or tetrahydroimidazopyrazinyl. Said tetrahydrothienopyridyl is preferably tetrahydrothieno[3,2-c]pyridyl. The tetrahydrothiazolopyridyl group is preferably tetrahydro[1,3]thiazolo[5,4-c]pyridyl. The tetrahydropyrazolopyridyl is preferably 4,5,6,7-tetrahydro-2H-pyrazolo [4,3-c]pyridyl. The substituent of the above-mentioned piperidino and piperazinyl groups is preferably an optionally substituted aryl group, an optionally substituted aralkyl group, an optionally substituted aryloxy group, an optionally

substituted aroyl group, an optionally substituted arylthio group, an optionally substituted arylsulfinyl group, an optionally substituted arylsulfonyl group, an optionally substituted heteroaryl group, an optionally substituted heteroaralkyl group, an optionally substituted heteroaryloxy group, an optionally substituted heteroarylcarbonyl group, an optionally substituted heteroarylthio group, an optionally substituted heteroarylsulfinyl group, an optionally substituted heteroarylsulfonyl group, an optionally substituted cycloalkyloxy group, an optionally substituted cycloalkyloxycarbonyl group, an optionally substituted cycloalkylthio group, an optionally substituted cycloalkylsulfinyl group, an optionally substituted cycloalkylsulfonyl group, an optionally substituted, saturated or unsaturated aliphatic heterocyclic group, an optionally substituted, saturated or unsaturated aliphatic heterocyclic oxy group, an optionally substituted, saturated or unsaturated aliphatic heterocyclic oxycarbonyl group, an optionally substituted, saturated or unsaturated aliphatic heterocyclic thio group, an optionally substituted, saturated or unsaturated aliphatic heterocyclic sulfinyl group, an optionally substituted, saturated or unsaturated aliphatic heterocyclic sulfonyl group.

The compound of the present invention of the formula (1) may be prepared from well-known compounds by Methods 1 to 11 as mentioned below, or a similar method of the following Methods, or by combining conventional synthetic methods well known to a person skilled in this art. In addition, in Tables showing chemical structures of Examples, • contained in partial structures indicates a binding position to a common nucleus. Further, in the present specification, the following abbreviations may be occasionally used in order to simplify the description.

Boc: tert-butoxycarbonyl group:
Cbz: benzyloxycarbonyl group
TMS: trimethylsilyl group
TBS: tert-butyldimethylsilyl group
SEM: 2-[(trimethylsilyl)ethoxy)methyl group
Ac: acetyl group
Me: methyl group
Et: ethyl group
Pr: propyl group
i-Pr: isopropyl group
Bu: butyl group
i-Bu: isobutyl group
t-Bu: tert-butyl group
Ph: phenyl group
Bn: benzyl group
Ms: methanesulfonyl group
TFA: trifluoroacetic acid
Alloc: allyloxycarbonyl group

[Method 1]

Among the compounds of the formula (1), the compound of the formula (1-8) or a salt thereof is prepared, for example, by the following method.

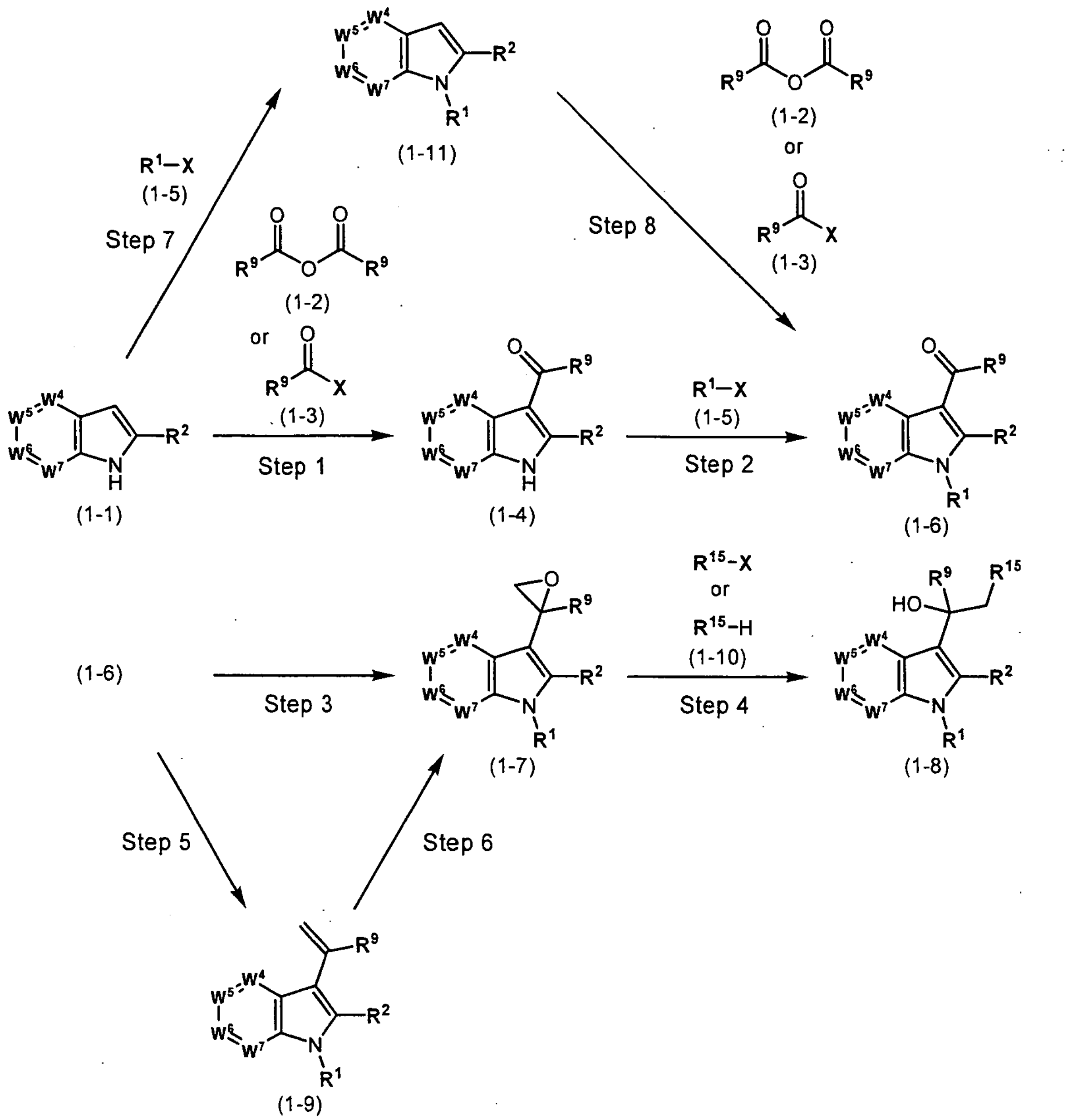


[wherein $R^1$, $R^2$, $-W^4=W^5-W^6=W^7-$, $R^9$ and $R^{15}$ are as defined above; X is a leaving group (e.g., iodine atom, bromine atom, chlorine atom, methanesulfonyloxy, trifluoromethanesulfonyloxy or p-toluenesulfonyloxy, etc.)]

1) Step 1

The compound (1-4) is prepared by reacting an indole (1-1), which may be conventional one or may be prepared by a conventional method, with an acid anhydride (1-2) or an acid halide (1-3) in an inert solvent in the presence or absence of either one of a Lewis acid or a base. The Lewis acid includes a metal halide or a metal triflate such as aluminum chloride, titanium tetrachloride, tin tetrachloride, zinc chloride, scandium trifluoromethanesulfonate, etc. The base includes, for example, an organic base (1-hydroxybenztriazole, N-methylmorpholine, triethylamine, diisopropylethylamine, tributylamine, 1,8-diazabicyclo[5.4.0]undeca-7-ene, 1,5-diazabicyclo[4.3.0]-nona-5-ene, 1,4-diazabicyclo[5.4.0]undeca-7-ene, pyridine, dimethylaminopyridine, picoline, etc.), an alkali metal (n-butyllithium, methyllithium, isopropylmagnesium bromide, etc.), an inorganic base (sodium ethoxide, sodium methoxide, potassium tert-butoxide, sodium hydride, etc.). The base is usually used in an amount of 1 to 5 equivalents to one equivalent of the indole (1-1). The acid anhydride (1-2) and the acid halide (1-3) are usually used in an amount of 1 to 5 equivalents to 1 equivalent of the indole (1-1). The inert solvent includes, for example, ether solvents (tetrahydrofuran, diethyl ether, 1,2-dimethoxyethane, 1,4-dioxane, etc.), halogenated hydrocarbons (dichloromethane, chloroform, 1,2-dichloroethane, chlorobenzene, etc.), nitro compounds (nitromethane, nitroethane, nitrobenzene, etc.), or a mixture of these solvent. The reaction is carried out at a temperature of from about -78˚C to about 150˚C.

2) Step 2

The compound (1-6) is prepared by reacting the compound (1-4) obtained in Step 1 and the compound (1-5) with a base in an inert solvent. The compound (1-5) is usually used in an amount of 1 equivalent to an excess amount to 1 equivalent of the compound (1-4). The inert solvent includes, for example, ether solvents (tetrahydrofuran, diethyl ether, 1,2-dimethoxyethane, 1,4-dioxane, etc.), aprotic polar solvents (N,N-dimethylformamide, N,N-dimethylacetamide, dimethylsulfoxide, acetonitrile, etc.), ketones (acetone, etc.), or a mixture of these solvents.
The base includes, for example, alkali metal carbonates (potassium carbonate, sodium carbonate, potassium hydrogen carbonate or sodium hydrogen carbonate, etc.), alkali metal hydrides (sodium hydride, potassium hydride, etc.) or alkali metal hydroxides (potassium hydroxide or sodium hydroxide, etc.), alkali metal alkoxides (sodium ethoxide, sodium tert-butoxide, potassium tert-butoxide, etc.), and potassium carbonate, potassium tert-butoxide, etc. are preferable. The base is usually used in an amount of 1 to 5 equivalents to 1 equivalent of the compound (1-4). The reaction is carried out at a temperature of from about 0˚C to about 150˚C, but it is usually carried out under reflux.

3) Step 3

The compound (1-7) is prepared by reacting the compound (1-6) obtained in Step 2 with trimethylsulfonyl iodide or trimethylsulphoxonium iodide, etc. in a solvent in the presence of a base (cf., Bull. Chem. Soc. Jpn., 68, 3591 (1995), and J. Am. Chem. Soc. 86, 1899 (1964), etc.). Trimethylsulfonyl iodide or trimethylsulphoxonium iodide is usually used in an amount of 1 to 5 equivalents to 1 equivalent of the compound (1-7). The base includes, for example, alkali metal carbonates (potassium carbonate, sodium carbonate, potassium hydrogen carbonate, sodium hydrogen carbonate, etc.), alkali metal hydrides (sodium hydride, potassium hydride, etc.) and alkali metal hydroxides (potassium hydroxide, sodium hydroxide, etc.), etc., and potassium carbonate, etc. is more preferable. The base is usually used in an amount of 1 to 3 equivalents to 1 equivalent of the compound (1-6). The inert solvent includes aprotic solvents (N,N-dimethylformamide, dimethylsulfoxide, etc.), ether solvents (diethyl ether, tetrahydrofuran, 1,4-dioxane, etc.), halogenated hydrocarbons (dichloromethane, chloroform, 1,2-dichloroethane, chlorobenzene, etc.), ketones (acetone, etc.), hydrocarbons (toluene, benzene, etc.), or a mixture of these solvents, or a mixture of water and these solvents, and preferable one is dimethylsulfoxide, and a mixture of dichloromethane and water. The reaction is carried out at a temperature of from about -78˚C to about 50˚C.

4) Step 4

The compound (1-8) is prepared by reacting the compound (1-7) with the compound (1-10) in the presence or absence of either one of a Lewis acid or a base in an inert solvent.
When the reaction of Step 4 is carbon-heteroatom (oxygen atom, sulfur atom or nitrogen atom) bond forming reaction, that is, when the compound (1-10) is amines, alcohols, thiols, phenols, thiophenols, or anilines, then the base includes, for example, organic bases (1-hydroxybenztriazole N-methylmorpholine, triethylamine, diisopropylethylamine, tributylamine, 1,8-diazabicyclo[5.4.0]undeca-7-ene, 1,5-diazabicyclo[4.3.0]nona-5-ene, 1,4-diazabicyclo[5.4.0]undeca-7-ene, pyridine, dimethylaminopyridine, picolic acid, etc.), inorganic bases such as alkali metal carbonates (potassium carbonate, sodium carbonate, potassium hydrogen carbonate, sodium hydrogen carbonate, etc.), alkali metal hydrides (sodium hydride, potassium hydride, etc.), alkali metal hydroxides (potassium hydroxide, sodium hydroxide, etc.), etc. Preferable one is triethylamine, potassium carbonate, etc. The base is usually used in an amount of 1 to 3 equivalents to 1 equivalent of the compound (1-10). The inert solvent includes, for example, N,N-dimethylformamide, ether solvents (diethyl ether, tetrahydrofuran, 1,4-dioxane, etc.), alcohols (ethanol, methanol, 2-propanol, hexafluoro-2-propanol, etc.), or a mixture of these solvents. Preferable inert solvent is 2-propanol, etc. The reaction is carried out at temperature of from about -78˚C to about 180˚C.
When the reaction of Step 4 is carbon-carbon bond forming reaction, the reaction is usually carried out by reacting the compound (1-10) with a base in an inert solvent, followed by subjecting the resultant to condensation reaction with the compound (1-7). The base is preferably alkali metals, organic copper reagents, etc., which are obtained by a method disclosed in the literatures (cf., Comprehensive Organic transformation, written by R. C. Larock, VCH publisher Inc., 1989, Jikken-Kagaku-Koza (edited by Chemical Society of Japan, published by MARUZEN, etc.)).

5) Step 5

The compound (1-9) is prepared by a method disclosed in the literatures (cf., Comprehensive Organic transformation, written by R. C. Larock, VCH publisher Inc., 1989, etc.), for example, by a Wittig reaction, Tebbe reaction or Peterson olefination reaction, etc. in an inert solvent. Preferable method is Wittig reaction, and the base is preferably a strong base, such as alkali metal hydrides (sodium hydride, potassium hydride, etc.), alkali metal alkoxides (sodium ethoxide, sodium tert-butoxide, potassium tert-butoxide, etc.), alkali metals (n-butyllithium, methyllithium, isopropylmagnesium bromide, etc.), and preferable base is potassium tert-butoxide, n-butyllithium, etc. The base is usually used in an amount of 1 to 3 equivalents to 1 equivalent of phosphonium halide such as methyltriphenylphosphonium bromide, etc. The inert solvent includes, for example, ether solvents (diethyl ether, tetrahydrofuran, 1,4-dioxane, etc.), aprotic solvents (N,N-dimethylformamide, N,N-dimethylacetamide), or a mixture of these solvents. The reaction is carried out at a temperature of from about -78˚C to about 100˚C.

6) Step 6

The compound (1-7) is prepared by epoxidation reaction of the compound (1-9) in a solvent according to the disclosure of the literatures (cf., J. Am. Chem. Soc., 112, 2801 (1990), J. Am. Chem. Soc., 119, 6189 (1997), J. Am. Chem. Soc., 122, 3220 (2000), J. Am. Chem. Soc., 123, 2933 (2001), etc.).

7) Step 7

The reaction of Step 7 is carried out in a similar manner to Step 2 of Method 1.

8) Step 8

The reaction of Step 8 is carried out in a similar manner to Step 1 of Method 1.

Method 2

Among the compounds of the formula (1), the compound (2-4) or a salt thereof is prepared, for example, by the following method.

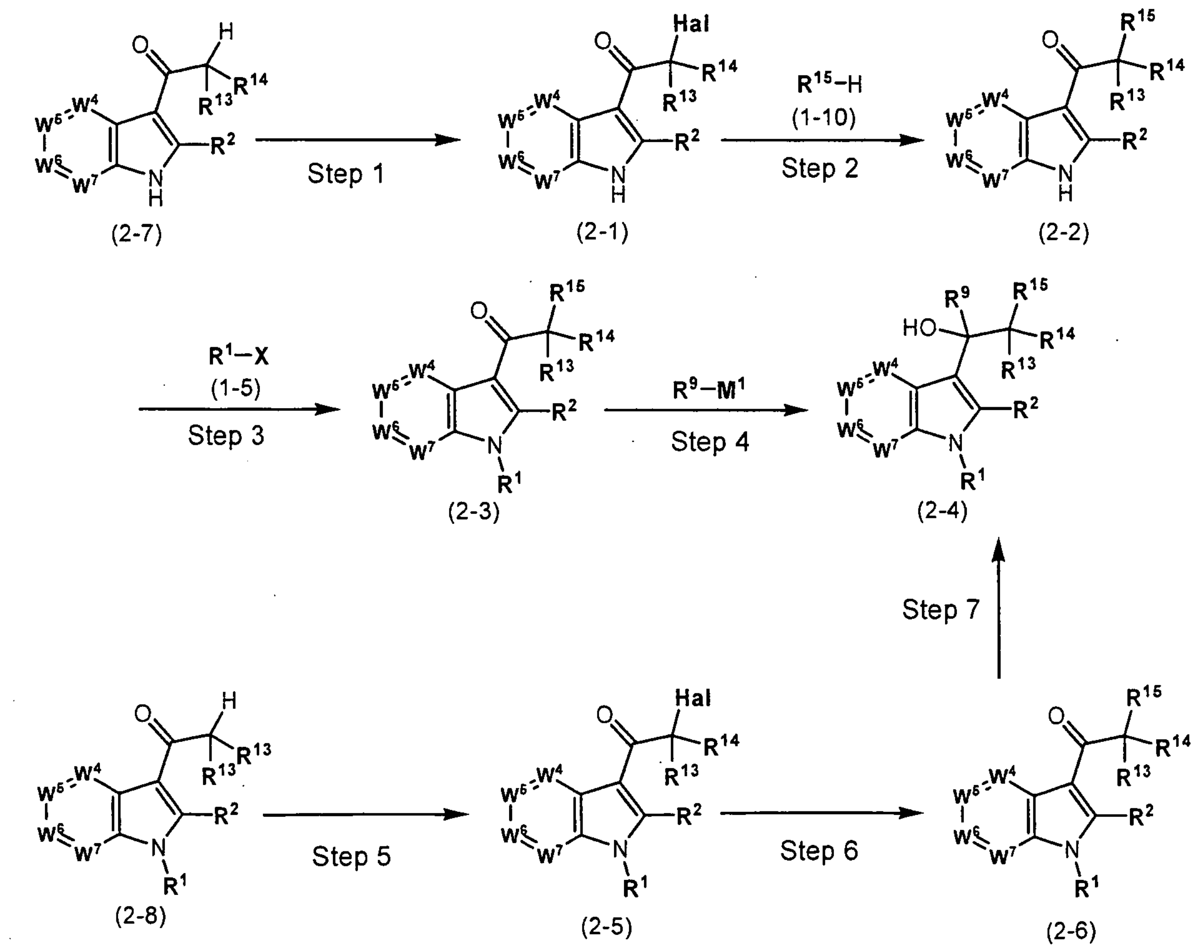


(2-7) (2-1) (2-2) (2-3) (2-4) (2-8) (2-5) (2-6)

[wherein $R^1$, $R^2$, $-W^4=W^5-W^6=W^7-$, $R^9$, $R^{13}$, $R^{14}$, $R^{15}$ are as defined above; Hal is a halogen atom (e.g., iodine atom, bromine atom, chlorine atom); $M^1$ is a hetero element (preferably alkali metals, alkaline earth metals, main group metals, silicone atom, etc.)].

1) Step 1

The compound (2-1) is prepared by reacting the compound (2-7), which is obtained by a similar method to Step 1 of Method 1, with a halogenating reagent (cf., Heterocycles, 55, 569 (2001), Heterocycles, 42, 83 (1996), etc.). The halogenating reagent includes, for example, halogen molecules (chlorine, bromine, iodine, etc.), thionyl halides (thionyl chloride, thionyl bromide, thionyl iodide, etc.), N-halogenated imides (N-chlorosuccinyl imide, N-bromosuccinyl imide, etc.). The halogenating reagent is usually used in an amount of 0.8 to 10 equivalents to 1 equivalent of the compound (2-7). The reaction is carried out in the presence or absence of either one of an acid or a base. The acid includes hydrochloric acid, hydrobromic acid, sulfuric acid, perchloric acid, phosphoric acid, acetic acid, formic acid, methanesulfonic acid, trifluoroacetic acid. The acid is usually used in an amount of 1 equivalent to an excess amount to 1 equivalent of the compound (2-7). The base includes, for example, organic bases (1-hydroxybenztriazole, N-methylmorpholine, triethylamine, diisopropylethylamine, tributylamine, 1,8-diazabicyclo[5.4.0]undeca-7-ene, 1,5-diazabicyclo[4.3.0]nona-5-ene, 1,4-diazabicyclo[5.4.0]undeca-7-ene, pyridine, dimethylaminopyridine, picoline, etc.). The base is usually used in an amount of 1 to 5 equivalents to 1 equivalent of the compound (1-4). The solvent includes, for example, ether solvents (tetrahydrofuran, 1,4-dioxane, etc.), halogenated hydrocarbons (dichloromethane, chloroform, 1,2-dichloroethane, chlorobenzene, etc.), organic acids (formic acid, acetic acid, etc.), or a mixture of these solvents. The reaction is carried out at a temperature of from about -78°C to about 180°C.

2) Step 2

The reaction of Step 2 is carried out in a similar manner to Step 4 of Method 1.

3) Step 3

The reaction of Step 3 is carried out in a similar manner to Step 2 of Method 1.

4) Step 4

The compound (2-4) is prepared by reacting the compound (2-3) obtained in Step 3 with $R^9$-$M^1$ in an inert solvent in the presence or absence of a base (cf., Tetrahedron. 56, 7613 (2000), Chemistry Letters. 34, 88 (2005), etc.). $R^9$-$M^1$ is usually used in an amount of 1 to 10 equivalents to 1 equivalent of the compound (2-3). The base includes, for example, fluorides (potassium fluoride, sodium fluoride, cesium fluoride, tetrabutylammonium fluoride, etc.), alkali metal acetates (lithium acetate, sodium acetate, potassium acetate, etc.), etc. Preferable one is lithium acetate, etc. The base is usually used in an amount of 0.1 to 3 equivalents to 1 equivalent of the compound (2-3). The inert solvent includes, for example, aprotic solvents (N,N-dimethylformamide, dimethylsulfoxide, etc.), ether solvents (diethyl ether, tetrahydrofuran, 1,4-dioxane, etc.), halogenated hydrocarbons (dichloromethane, chloroform, 1,2-dichloroethane, chlorobenzene, etc.), hydrocarbons (toluene, benzene, etc.), or a mixture of these solvents. Preferable one is dimethylsulfoxide, tetrahydrofuran, etc. The reaction is carried out at a temperature of from about -78˚C to about 150˚C.

5) Step 5

The reaction of Step 5 is carried out in a similar manner to Step 1 of Method 2, using the compound (2-8) obtained in Step 1 of Method 1.

6) Step6

The reaction of Step 6 is carried out in a similar manner to Step 2 of Method 2.

7) Step 7

The reaction of Step 7 is carried out in a similar manner to Step4 of Method 2.

Method 3

Among the compounds of the formula (1), the compound (1-8), the compound (3-8) or a salt thereof are prepared by the following Method.

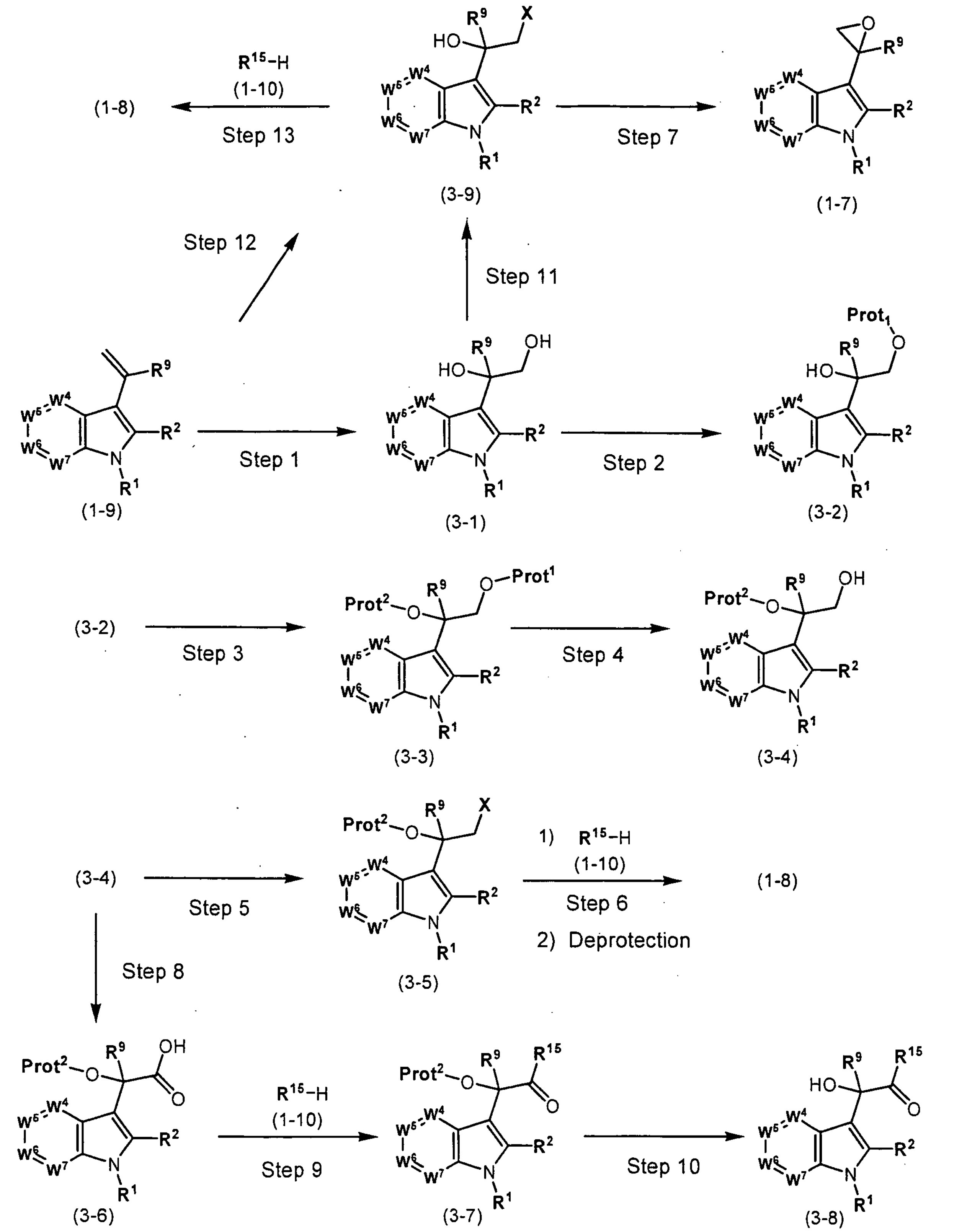


[wherein $R^1$, $R^2$, $-W^4=W^5-W^6=W^7-$, $R^9$, $R^{15}$ are as defined above; X is a leaving group (e.g., iodine atom, bromine atom, chlorine atom, methanesulfonyloxy, trifluoromethanesulfonyloxy, p-toluenesulfonyloxy, etc.); $Prot^1$ and $Prot^2$ are a protecting group for hydroxy group (Protective Groups in Organic Synthesis 3rd Edition (John Wiley & Sons, Inc.) or

$R^{11}$]

1) Step 1

The compound (3-1) is prepared by reacting the compound (1-9) obtained in Step 1 of Method 1 as a starting compound with osmium tetraoxide, etc. in a solvent in the presence of an oxidizing agent (cf., J. Org. Chem. 56, 4585 (1991), Tetrahedron: Asymmetry. 14, 503 (2003), etc.).

2) Step 2

The compound (3-2) is prepared by reacting the compound (3-1) in an inert solvent in the presence of a base., according to a method disclosed in the literatures (Protective Groups in Organic Synthesis 3rd Edition (John Wiley & Sons, Inc.), Tetrahedron: Asymmetry. 14, 503 (2003), etc.).

3) Step 3

The compound (3-3) is prepared from the compound (3-2) in a similar manner to ones disclosed in the literatures (Protective Groups in Organic Synthesis 3rd Edition (John Wiley & Sons, Inc.), Tetrahedron: Asymmetry. 14, 503 (2003), etc.). The protecting group used in this reaction is ones, which can be removed by a different method from the method to be applied for removing the protecting groups used in Step 2 of Method 3.

4) Step 4

The compound (3-4) is prepared from the compound (3-3) by selectively removing the protecting group for a primary hydroxy group ($Prot^1$) according to a method disclosed in the literatures (Protective Groups in Organic Synthesis 3rd Edition (John Wiley & Sons, Inc.)).

5) Step 5

The compound (3-5) is prepared from the compound (3-4) according to a method disclosed in the literature (cf., Comprehensive Organic transformation, written by R. C. Larock, VCH publisher Inc., 1989, etc.).

6) Step6

The condensation reaction 1) is carried out in a similar manner to Step 4 of Method 1. The protecting group for hydroxy group of the obtained compound is removed by a method disclosed in the literature (Protective Groups in Organic Synthesis 3rd Edition (John Wiley & Sons, Inc.)) to give the compound (1-8).

7) Step7

The compound (1-7) is prepared from the compound (3-9) by a method disclosed in the literatures (cf., Comprehensive Organic transformation, R. C. Larock, VCH publisher Inc., 1989, Tetrahedron Lett., 40, 7879 (1999), etc.).

8) Step 8

The compound (3-6) is prepared from the compound (3-4) in a similar manner to a method disclosed in the literature (cf., Tetrahedron: Asymmetry. 14, 503 (2003), Tetrahedron Lett., 40, 7879 (1999), etc.).

9) Step 9

The compound (3-7) is prepared from the compound (3-6) in a similar manner to a method disclosed in the literature (cf., Comprehensive Organic transformation, R. C. Larock, VCH publisher Inc., 1989, etc.).

10) Step 10

The compound (3-8) is prepared from the compound (3-7) in a similar manner to a method disclosed in the literature (cf., Protective Groups in Organic Synthesis 3rd Edition (John Wiley & Sons, Inc.)).

11) Step 11

The reaction of Step 11 is carried out in a similar manner to Step 5 of Method 3.

12) Step 12

The compound (3-5) and the compound (3-9) are prepared from the compound (1-9) in a similar manner to a method disclosed in the literatures (cf., Tetrahedron. 52, 12761 (1996), Synthesis. 11, 1584 (1998), Synthesis. 1, 45 (2003), Comprehensive Organic transformation, written by R. C. Larock, VCH publisher Inc., 1989, etc.).

13) Step 13

The reaction of Step 13 is carried out in a similar manner to Step 4 of Method 1.

Method 4

Among the compounds of the formula (1), the compound of the formula (4-3) or a salt thereof is prepared, for example, by the following method.

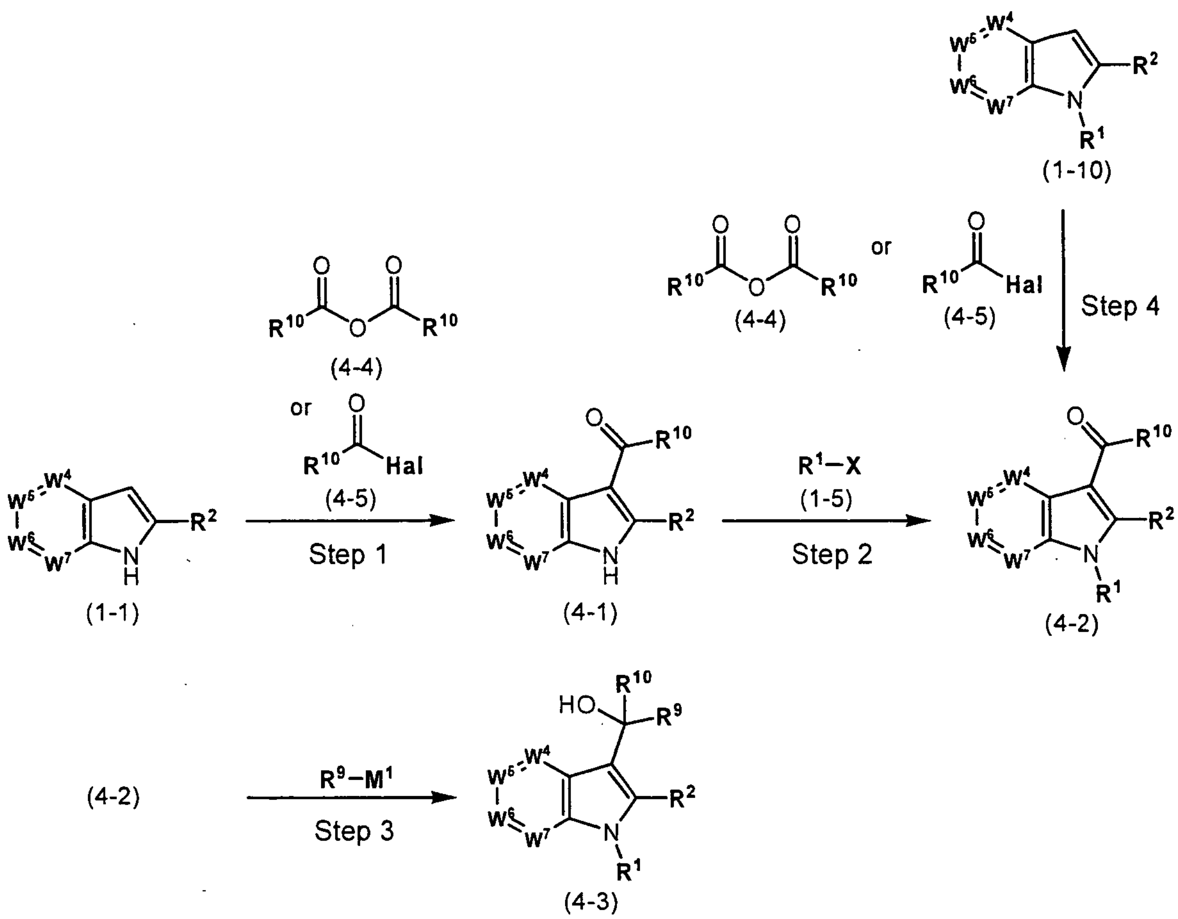


[wherein $R^1$, $R^2$, $-W^4{=}W^5\text{-}W^6{=}W^7-$, $R^9$, $R^{10}$, $R^{15}$ are as defined above; Hal is a halogen atom (e.g., iodine atom, bromine atom, chlorine atom); $M^1$ is a hetero element (preferably alkali metals, alkaline earth metals, main group metals, silicone atom, etc.)]

1) Step 1

The reaction of Step 1 is carried out in a similar manner to Step 1 of Method 1, using the compound (1-1) as a starting compound.

2) Step 2

The reaction of Step 2 is carried out in a similar manner to Step 2 of Method 1.

3) Step 3

The compound (4-3) is prepared by reacting the compound (4-2) obtained in Step 2 with $R^9$-$M^1$ in an inert solvent in the presence or absence of a base (cf., Comprehensive Organic transformation, written by R. C. Larock, VCH publisher Inc., 1989, Tetrahedron. 56, 7613 (2000), Chemistry Letters. 34, 88 (2005), etc.). $R^9$-$M^1$ is usually used in an amount of 1 to 10 equivalents to 1 equivalent of the compound (4-2). The base includes, for example, fluorides (potassium fluoride, sodium fluoride, cesium fluoride, tetrabutylammonium fluoride, etc.), alkali metal acetates (lithium acetate, sodium acetate, potassium acetate, etc.), etc., and preferable one is lithium acetate, etc. The base is usually used in an amount of 0.1 to 3 equivalents to 1 equivalent of the compound (1-10). The inert solvent includes, for example, aprotic solvents (N,N-dimethylformamide, dimethylsulfoxide, etc.), ether solvents (diethyl ether, tetrahydrofuran; 1,4-dioxane, etc.), halogenated hydrocarbons (dichloromethane, chloroform, 1,2-dichloroethane, chlorobenzene, etc.), hydrocarbons (toluene, benzene, etc.), or a mixture of these solvents. Preferable one is dimethylsulfoxide, tetrahydrofuran. The reaction is carried out at a temperature of from about -78˚C to about 100˚C.

4) Step4

The reaction of Step 4 is carried out in a similar manner to Step 1 of Method 1, using the compound (1-10) obtained in Step 7 of Method 1 as a starting compound.

Method 5

Among the compounds of the formula (1), the compounds of the formulae (5-1), (5-2) and (5-3) or a salt thereof are prepared, for example, by the following method.

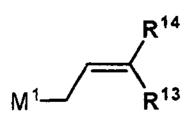

or

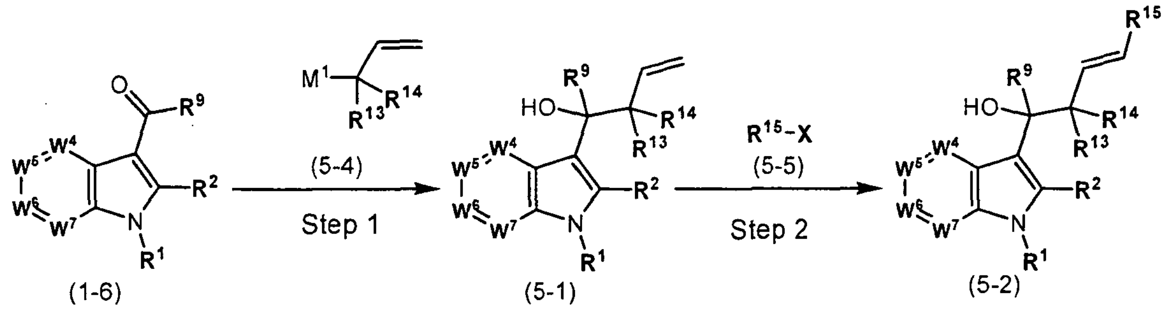


(1-6) (5-1) (5-2)

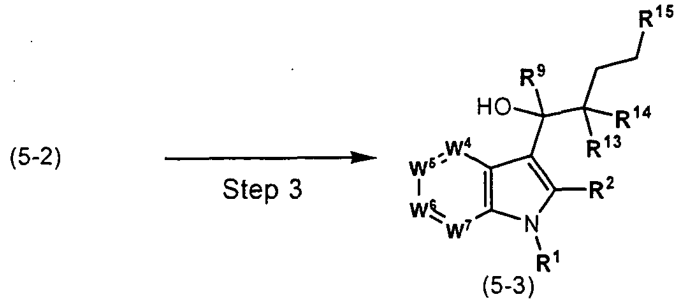


(5-3)

[wherein $R^1$, $R^2$, $-W^4=W^5-W^6=W^7-$, $R^9$, $R^{13}$, $R^{14}$, $R^{15}$ are as defined above; $M^1$ is metal elements (preferably alkali metals, alkaline earth metals, main group metals, etc.); X is a leaving group (e.g., iodine atom, bromine atom, chlorine atom, methanesulfonyloxy, trifluoromethanesulfonyloxy, p-toluenesulfonyloxy, etc.)]

1) Step 1

The compound (5-1) is prepared by reacting the compound (1-6) obtained in Method 1 with the compound (5-4) in a solvent (cf., Jikken-Kagaku-Koza (edited by Chemical Society of Japan, published by MARUZEN), Tetrahedron Lett., 40, 9333 (1999), etc.). The compound (5-4) is usually used in an amount of 1 to 10 equivalents to 1 equivalent of the compound (1-6). The solvent includes aprotic solvents (N,N-dimethylformamide, dimethylsulfoxide, etc.), ether solvents (diethyl ether, tetrahydrofuran, 1,4-dioxane, etc.), halogenated hydrocarbons (dichloromethane, chloroform, 1,2-dichloroethane, chlorobenzene, etc.), hydrocarbons (toluene, benzene, etc.), or a mixture of these solvent, or a mixture of water and these solvents. Preferable solvent is ether solvents. The reaction is carried out at a temperature of from about -78˚C to about 150˚C.

2) Step 2

The compound (5-2) is prepared by condensation of the compound (5-1) with the compound (5-5) by Heck reaction in a solvent in the presence of a catalyst (cf., Palladium Reagents and Catalysts, written by Jiro Tsuji, John Wiley & Sons Ltd, 2004, J. Am. Chem. Soc. 123, 6989 (2001), etc.). In addition, $R^{15}$ of the compound (5-5) is preferably an aryl group or a heteroaryl group.

3) Step 3

The compound (5-3) is prepared from the compound (5-2) in a similar manner to a method disclosed in the literature (cf., Comprehensive Organic transformation, written by R. C. Larock, VCH publisher Inc., 1989, etc.).

Method 6

Among the compounds of the formula (1), the compound of the formula (6-1) or a salt thereof is prepared, for example, by the following method.

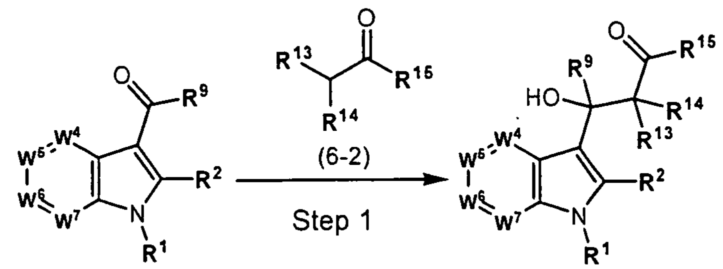


(1-6) (6-1)

[wherein $R^1$, $R^2$, $-W^4=W^5-W^6=W^7-$, $R^9$, $R^{13}$, $R^{14}$, $R^{15}$ are as defined above]

1) Step 1

The compound (6-1) is prepared by reacting the compound (1-6) obtained in Method 1 with the compound (6-2) by Aldol reaction in a solvent (cf., Modem aldol reactions, written by Rainer Mahrwald, John Wiley & Sons linc., 2004, Tetrahedron. 58, 8269 (2002), etc.).

Method 7

Among the compounds of the formula (1), the compound of the formula (3-6) or a salt thereof is prepared, for example, by the following method.
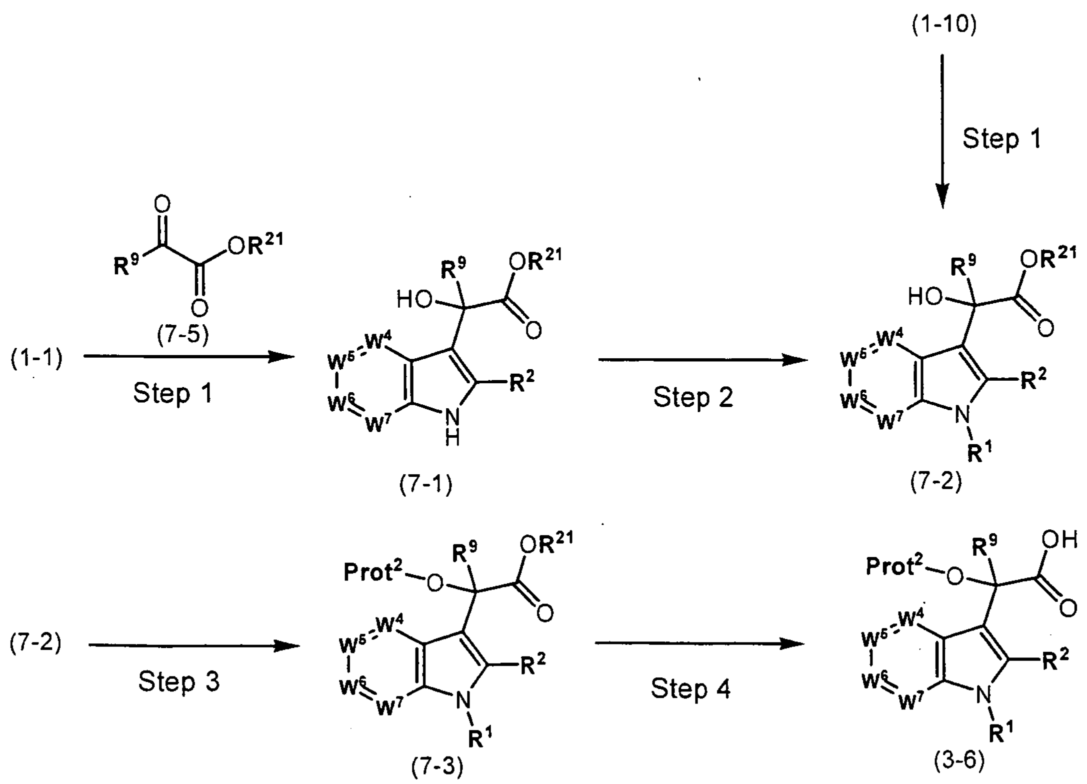


[wherein $R^1$, $R^2$, $-W^4=W^5-W^6=W^7-$, $R^9$, $R^{21}$ are as defined above; $Prot^2$ is a protecting group for hydroxy group (cf., Protective Groups in Organic Synthesis 3rd Edition (John Wiley & Sons, Inc.)), or $R^{11}$]

1) Step 1

The compound (7-1) is prepared by reacting the indole (the compound (1-1)) with a keto ester (the compound

(7-5)) in an inert solvent in the presence of absence of an acid. The acid includes metal halides or metal triflates such as aluminum chloride, titanium tetrachloride, tin tetrachloride, zinc chloride, scandium trifluoromethanesulfonate, etc. and organic acids such as trifluoromethanesulfonic acid, sulfonic acid, etc. The acid is usually used in an amount of 0.1 to 3 equivalents to 1 equivalent of the compound (1-1). The compound (7-5) is usually used in an amount of 1 to 5 equivalents to 1 equivalent of the compound (1-1). The inert solvent includes ether solvents (tetrahydrofuran, diethyl ether, 1,2-dimethoxyethane, 1,4-dioxane, etc.), halogenated hydrocarbons (dichloromethane, chloroform, 1,2-dichloroethane, chlorobenzene, etc.), nitro compounds (nitromethane, nitroethane, nitrobenzene, etc.), or a mixture of these solvents. The reaction is carried out at a temperature of from about -78°C to about 150°C.

2) Step 2

The reaction of Step 2 is carried out in a similar manner to Step 2 of Method 1.

3) Step 3

The compound (7-3) is prepared from the compound (7-2) in a similar manner to a method disclosed in the literature (Protective Groups in Organic Synthesis 3rd Edition (John Wiley & Sons, Inc.)).

4) Step 4

The compound (3-6) is prepared from the compound (7-3) in a similar manner to a method disclosed in the literature (Protective Groups in Organic Synthesis 3rd Edition (John Wiley & Sons, Inc.)). The obtained compound (3-6) is converted into the compound (1) of the present invention by removing the protecting group for hydroxy group.

Method 8

Among the compounds of the formula (1), the compound (8-1), the compound (8-2), and the compound (8-3) or a salt thereof are prepared, for example, by the following method.

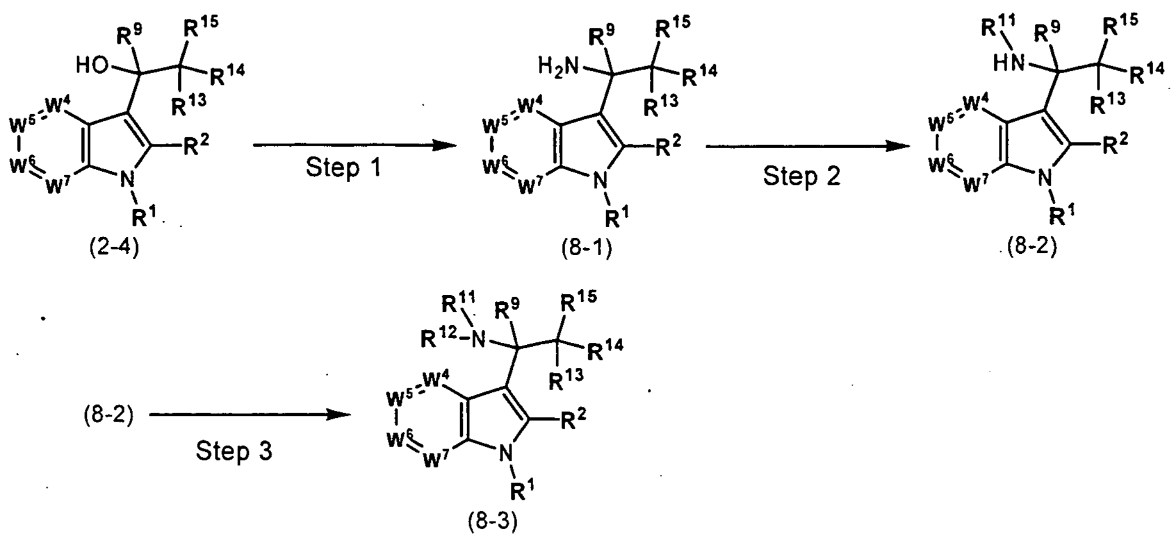


(2-4) (8-1) (8-2)

(8-2) (8-3)

[wherein $R^1$, $R^2$, $-W^4=W^5-W^6=W^7-$, $R^9$, $R^{11}$, $R^{12}$, $R^{13}$, $R^{14}$, $R^{15}$ are as defined above]

1) Step 1

The compound (8-1) is prepared from the compound (2-4) in a similar manner to a method disclosed in the literatures (cf., Synth Commun. 24, 2419 (1999), Tetrahedron Lett., 28, 6513 (1987), etc.).

2) Step 2

The compound (8-2) is prepared from the compound (8-1) in a similar manner to a method disclosed in the literature (Protective Groups in Organic Synthesis 3rd Edition (John Wiley & Sons, Inc.)).

3) Step 3

The compound (8-3) is prepared from the compound (8-2) in a similar manner to a method disclosed in the literatures (Protective Groups in Organic Synthesis 3rd Edition (John Wiley & Sons, Inc.); Comprehensive Organic transformation, written by R. C. Larock, VCH publisher Inc., 1989).

Method 9

Among the compounds of the formula (1), the compound (9-4), the compound (9-5), and the compound (9-6) or a salt thereof are prepared, for example, by the following method.

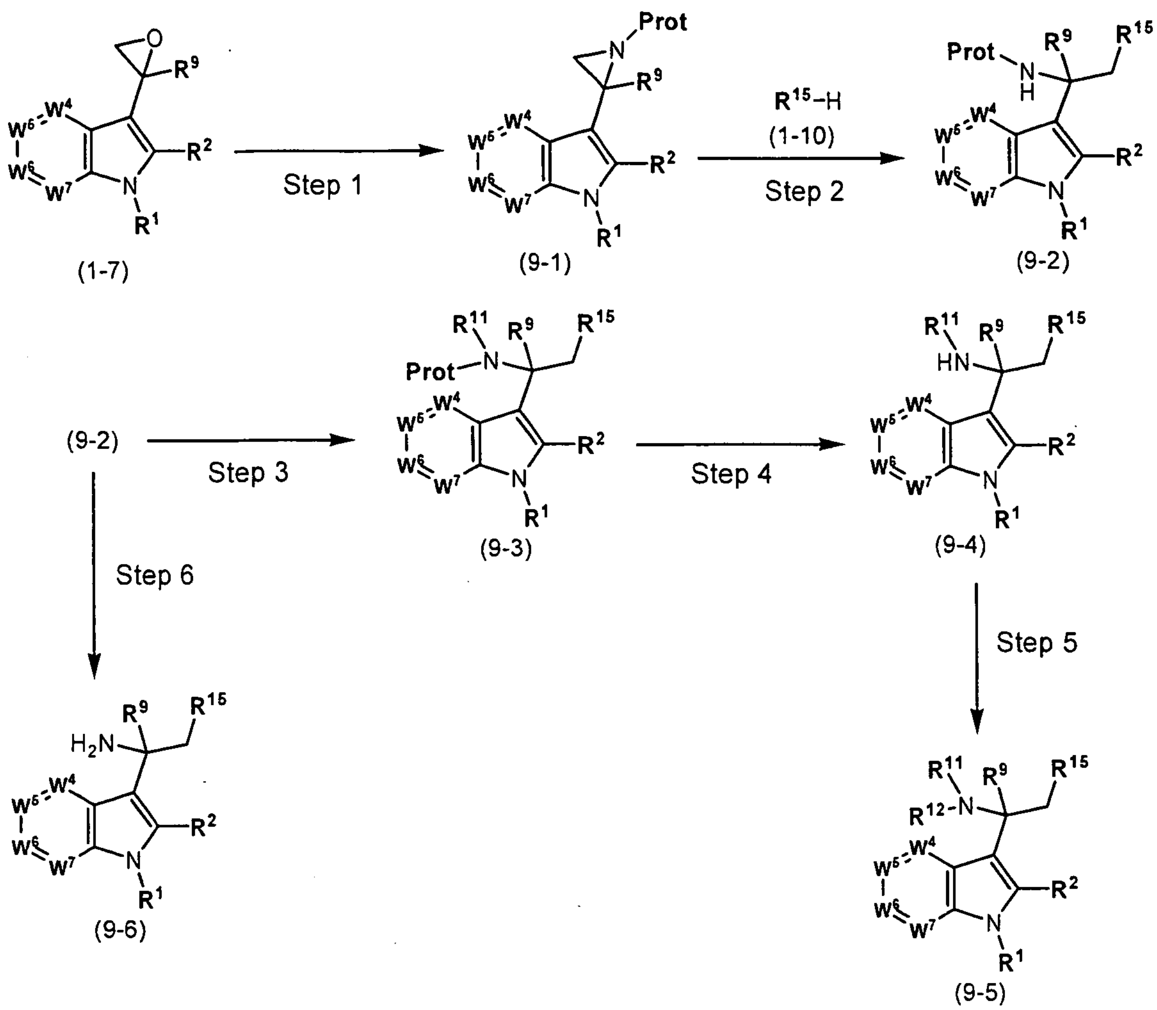


[wherein $R^1$, $R^2$, $-W^4=W^5-W^6=W^7-$, $R^9$, $R^{11}$, $R^{12}$, $R^{15}$ are as defined above; Prot is a protecting group for amino group (Protective Groups in Organic Synthesis 3rd Edition (John Wiley & Sons, Inc.)), or $R^{11}$]

1) Step 1

The compound (9-1) is prepared from the compound (1-7) obtained in Method 1 in a similar manner to a method disclosed in the literatures (cf., J. Org. Chem. 43, 4271 (1978), Tetrahedron: Asymmetry. 8, 903 (1997), J. Heterocycl.

Chem. 28, 473 (1991), etc.).

2) Step 2

The compound (9-2) is prepared from the compound (9-1) in a similar manner to a method disclosed in the literatures (cf., Tetrahedron. 51, 11515 (1995), Synthesis. 15, 2254 (2002), etc.).

3) Step 3

The compound (9-3) is prepared from the compound (9-2) in a similar manner to a method disclosed in the literatures (cf., Comprehensive Organic transformation, written by R. C. Larock, VCH publisher Inc., 1989, etc.).

4) Step 4

The compound (9-4) is prepared from the compound (9-3) in a similar manner to a method disclosed in the literature (Protective Groups in Organic Synthesis 3rd Edition (John Wiley & Sons, Inc.)).

5) Step 5

The compound (9-5) is prepared from the compound (9-4) in a similar manner to a method disclosed in the literature (cf., Comprehensive Organic transformation, written by R. C. Larock, VCH publisher Inc., 1989, etc.).

6) Step 6

The compound (9-6) is prepared from the compound (9-2) in a similar manner to a method disclosed in the literature (cf., Protective Groups in Organic Synthesis 3rd Edition (John Wiley & Sons, Inc.)).

Method 10

Among the compounds of the formula (1), the compound (10-2) and the compound (10-4) or a salt thereof are prepared, for example, by the following method.

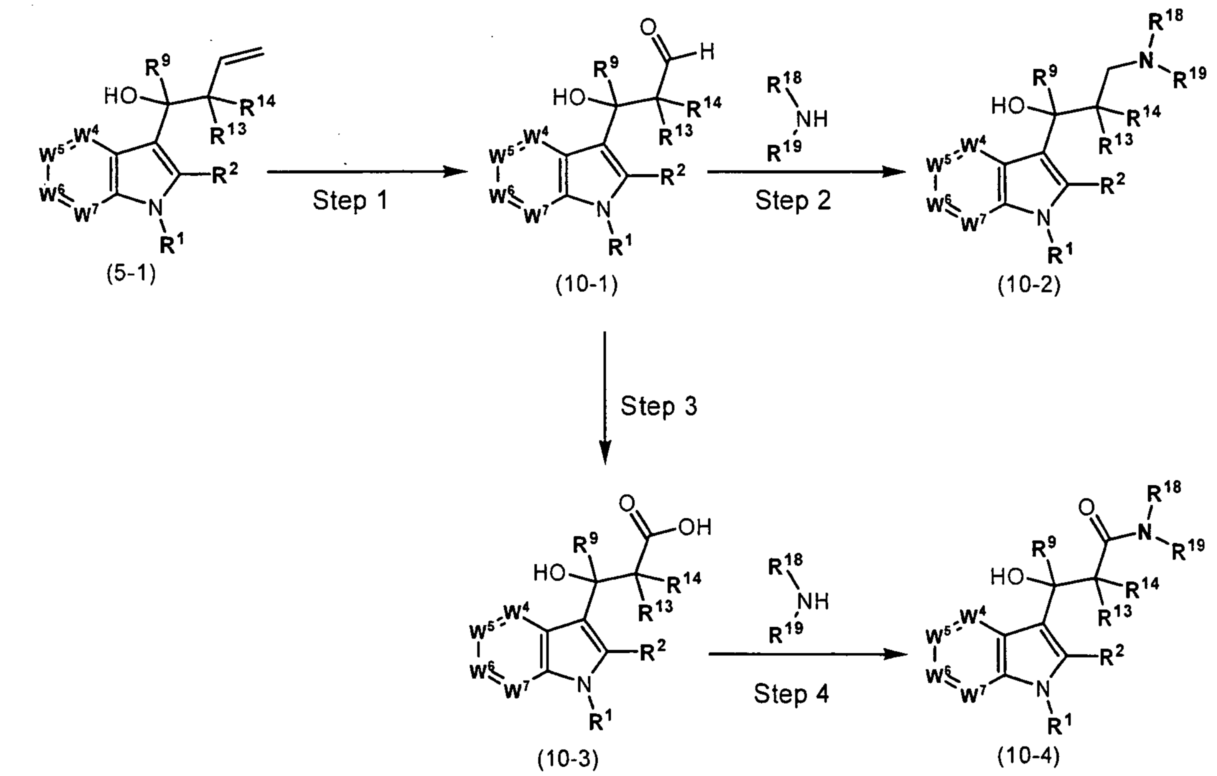


(5-1) (10-1) (10-2) (10-3) (10-4)

[wherein $R^1$, $R^2$, $-W^4=W^5-W^6=W^7-$, $R^9$, $R^{13}$, $R^{14}$, $R^{18}$, $R^{19}$ are as defined above]

1) Step 1

The compound (10-1) is prepared from the compound (5-1) obtained in Method 5 in a similar manner to a method disclosed in the literature (cf., Comprehensive Organic transformation, written by R. C. Larock, VCH publisher Inc., 1989, etc.), preferably by a method using ozonolysis.

2) Step 2

The compound (10-2) is prepared from the compound (10-1) in a similar manner to a method disclosed in the literature (cf., J. Org. Chem. 61, 3849 (1996), etc.).

3) Step 3

The compound (10-3) is prepared from the compound (10-1) in a similar manner to a method disclosed in the literatures (cf., J. Am. Chem. Soc. 119, 12386 (1997), Tetrahedron Lett., 40, 7879 (1999), etc.).

4) Step 4

The compound (10-4) is prepared from the compound (10-3) in a similar manner to a method disclosed in the literature (cf., Comprehensive Organic transformation, written by R. C. Larock, VCH publisher Inc., 1989, etc.).

Method 11

Among the compounds of the formula (1), the compound (11-3) or a salt thereof is prepared, for example, by the following method.

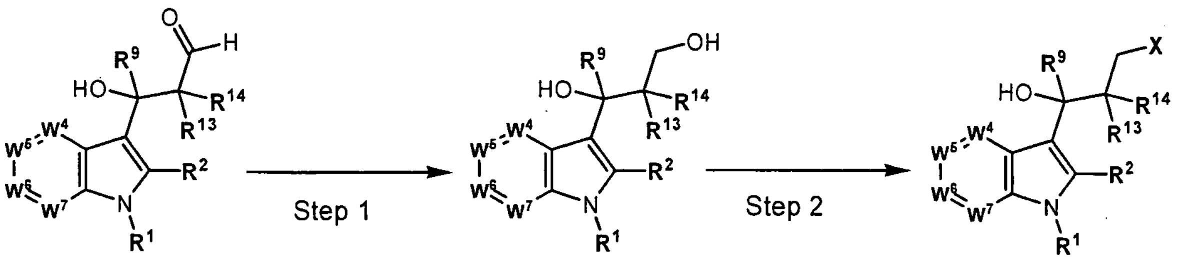


(10-1) (11-1) (11-2)

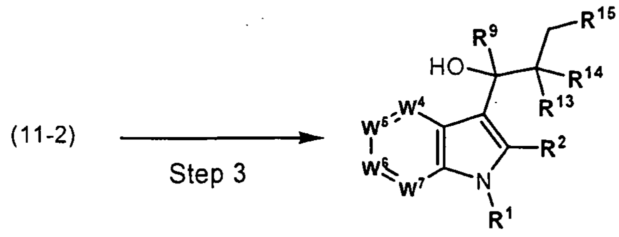


(11-3)

[wherein $R^1$, $R^2$, $-W^4=W^5-W^6=W^7-$, $R^9$, $R^{13}$, $R^{14}$, $R^{15}$ are as defined above; X is a leaving group (e.g., iodine atom, bromine atom, chlorine atom, methanesulfonyloxy, trifluoromethanesulfonyloxy, p-toluenesulfonyloxy, etc.)]

1) Step 1

The compound (11-1) is prepared from the compound (10-1) obtained in Method 10 in a similar manner to a method disclosed in the literature (cf., Comprehensive Organic transformation, written by R. C. Larock, VCH publisher Inc., 1989, etc.).

2) Step 2

The compound (11-2) is prepared from the compound (11-1) in a similar manner to a method disclosed in the literature (cf., Comprehensive Organic transformation, written by R. C. Larock, VCH publisher Inc., 1989, etc.).

3) Step 3

The compound (11-3) is prepared from the compound (11-2) in a similar manner to a method disclosed in the literature (cf., Comprehensive Organic transformation, written by R. C. Larock, VCH publisher Inc., 1989, etc.).

Method 12

Among the compounds of the formula (1), the compound (12-2) or a salt thereof is prepared, for example, by the following method.

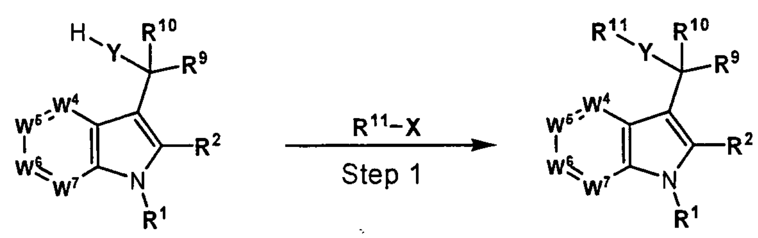


(12-1) (12-2)

[wherein $R^1$, $R^2$, $-W^4=W^5-W^6=W^7-$, $R^9$, $R^{10}$, $R^{11}$ are as defined above; Y is an oxygen atom or a sulfur atom; X is a

leaving group (cf., iodine atom, bromine atom, chlorine atom, methanesulfonyloxy, trifluoromethanesulfonyloxy, p-toluenesulfonyloxy, etc.)]

1) Step 1

The compound (12-2) is prepared from the compound (12-1) in a similar manner to a method disclosed in the literatures (cf., Protective Groups in Organic Synthesis 3rd Edition (John Wiley & Sons, Inc.); Comprehensive Organic transformation, written by R. C. Larock, VCH publisher Inc., 1989), for example, by reacting the compound (12-1) with an alkyl halide, etc. in the presence of a base.

In the above Methods, the starting compounds or the reagents to be used therein are commercially available ones or can be prepared from the well-known compounds by a conventional well-known method, unless specified otherwise.

In each Step of the above Methods, when the starting compound in each reaction has a reactive group being active to such a reaction, for example, a hydroxy group, an amino group or a carboxyl group, then these reactive groups other than ones at the reaction site are previously protected with a suitable protecting group, if necessary, and after said reaction(s) are completed, the protecting groups are removed to give the desired compounds. The protecting groups for hydroxy, amino or carboxyl group are conventional ones which are widely used in the organic chemistry, and the introduction and the removal of these protecting groups can be carried out by a conventional method (cf., the methods disclosed in Protective Groups in Organic Synthesis, T. W. Greene, co-written by P. G. M. Wuts, 3rd edition, John Wiley & Sons, Inc. (1999)).

For example, the protecting group for hydroxy group includes tert-butyldimethylsilyl, methoxymethyl, tetrahydropyranyl, etc., and the protecting group for amino group includes tert-butyloxycarbonyl, benzyloxycarbonyl, etc. These protecting groups for hydroxy group can be removed by reacting in the presence of an acid such hydrochloric acid, sulfuric acid, acetic acid, etc. in a solvent such as aqueous methanol, aqueous ethanol, aqueous tetrahydrofuran, etc. When tert-butyldimethylsilyl is used, the removal thereof is carried out, for example, in the presence of tetrabutylammonium fluoride in a solvent such as tetrahydrofuran, etc. With respect to the removal of the protecting group for amino group, for example, when tert-butyloxycarbonyl is used, the removal thereof is carried out by reacting in the presence of an acid such as hydrochloric acid, trifluoroacetic acid, etc. in a solvent such as aqueous tetrahydrofuran, methylene chloride, chloroform, aqueous methanol, etc., and when benzyloxycarbonyl is used, the removal thereof is carried out, for example, by reacting in the presence of an acid such as hydrobromic acid in a solvent such as acetic acid, etc.

As a protecting group for carboxyl group, tert-butyl esters, ortho-ester, acid amides, etc. are exemplified. With respect to the removal of these protecting groups, when a tert-butyl ester is used, the removal thereof is carried out by reacting in the presence of hydrochloric acid in an aqueous solvent.

When an ortho ester is used, the removal thereof is carried out by treating with an acid in a solvent such as aqueous methanol, aqueous tetrahydrofuran, aqueous 1,2-dimethoxyethane, etc., followed by treating the resultant with a base such as sodium hydroxide. When an acid amide is used, the removal thereof is carried out by reacting in the presence of an acid such as hydrochloric acid, sulfuric acid, etc. in a solvent such as water, aqueous methanol, aqueous tetrahydrofuran, etc.

The compound of the formula (1) may have an optically active center, by which the compound of the formula (1) may exist in the form of racemic mixture, or in the form of an optically active compound when it is prepared from an optically active starting compound. If necessary, the obtained racemic compound may be physically or chemically resolved into an optical enantiomer by a conventional method, or may be synthesized by a conventional asymmetric reaction. Preferably, the racemic compound is converted into a diastereomer by a reaction using an optically active resolving agent. Diastereomers in a different form can be separated by a conventional method such fractional crystallization.

In addition, the compound of the formula (1) may exist in the form of a tautomer. Examples thereof are shown in the following formulae (13-1) and (13-2).

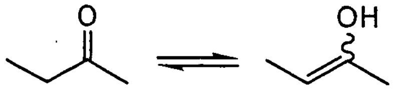 (13-1) 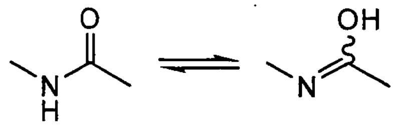 (13-2)

The compound of the present invention or a prodrug thereof may be converted into a salt thereof by mixing with a pharmaceutically acceptable acid or base in a solvent such as water, methanol, ethanol, acetone, etc. The

pharmaceutically acceptable acid includes, for example, inorganic acids such as hydrochloric acid, hydrobromic acid, sulfuric acid, phosphoric acid, nitric acid, etc., organic acids such as acetic acid, propionic acid, oxalic acid, succinic acid, lactic acid, malic acid, tartaric acid, citric acid, maleic acid, fumaric acid, methanesulfonic acid, p-toluenesulfonic acid, ascorbic acid, etc. The pharmaceutically acceptable alkali addition salt includes ammonium salt, lithium salt, sodium salt, potassium salt, calcium salt, magnesium salt, etc.
In addition, the present invention further includes hydrates and solvates such as ethanoates of the compound of the formula (1) or a pharmaceutically acceptable salt thereof.

In the present invention, the prodrug, i.e., compound which can be metabolized by an enzyme in the living body and converted into the active compound of the present invention, includes compounds wherein a carboxyl group is esterified, etc. Examples of the ester are methyl ester, ethyl ester, benzyl ester, pivaloyloxymethyl ester, cilexetil, medoxomil, pivoxyl, proxetil, mofetil (cf., J. Med. Chem. 47, 2393 (2004), etc.), etc. These compounds may be prepared by a conventional method (cf., J. Med. Chem. 35, 4727 (1992), WO 01/40180, etc.). Further, prodrugs may usually be administered in the form of an oral preparation. Namely, when the compound of the present invention has a carboxyl group or a hydroxy group, the corresponding compounds wherein these groups are converted into an ester may be included in the present invention.
In addition, when the compound of the present invention is metabolized by an intravital enzyme to be converted into a less bioactive metabolite, said compound can be used as an antedrug. Further, such antedrugs may usually be administered in the dosage form for local administration.

The novel fused pyrrole derivative of the present invention, or a prodrug thereof or a pharmaceutically acceptable salt thereof acts as a glucocorticoid function regulating agent (GR modulator), and can be used as an agent for treatment and/or prophylaxis of diseases in which GR is involved.
The glucocorticoid function regulating agent of the present invention means a substance which activates or suppresses the function of glucocorticoid receptor (GR), and includes, for example, GR agonists (including partial agonists), GR antagonists (including partial antagonists), etc. Concretely, GR modulator is a substance which inhibits the binding between GR and a well-known steroid compound (including both natural and synthesized steroids) such as dexamethasone, etc. in vitro, and simultaneously as exhibits inhibitory activity /increasing activity of the effects of dexamethanzone, etc. as well.
It can be possible to apply the glucocorticoid function regulating agents to the following various diseases where existing steroidal anti-inflammatory agents have been clinically used, and the present compounds are useful as an agent for treatment or prophylaxis of the following diseases.

1) Diseases which can be ameliorated by physiological hormone action, such as hormone deficiencies showing an abnormal low blood level of cortisol due to pituitary deficiency/adrenalism, adrenal enzymatic defect due to high ACTH level, dexamethasone-suppressive hypertension, etc.;
2) Connective tissue disorders associated diseases such as rheumatoid arthritis, juvenile rheumatoid arthritis, systemic erythematosus, diffuse scleroderma, multiple myositis, dermatomyositis, mixed connective tissue disease, vasculitis syndrome, Behcet's disease, adult onset still's disease, Sjogren's syndrome, etc.;
3) Blood diseases such as idiopathic thrombocytopenic purpura, autoimune hemolytic anemia, anaplastic anemia, pure red cell aplasia, hemophagocyctic syndrome, Hodgkin's disease, non-Hodgkin's lymphoma, multiple myeloma, acute lymphatic leukemia, chronic lymphatic leukemia, paroxysmal nocturnal hemoglobinuria, thrombotic thrombocytopenic purpura, rhetinoid syndrome, cytarabine syndrome, etc.;
4) Renal diseases such as minimal lesion nephrotic syndrome, membranous nephropathy, focal glomerular sclerosis, IgA nephropathy, membranoproliferative glomerulonephritis, rapidly progressive glomerulonephritis syndrome, renal disease associated multiple myeloma, kidney disoder in cryoglobulinemia, acute interstitial nephritis, drug-induced renal disorder;
5) Respiratory diseases such as infections (carinii pneumonia, mycoplasma pneumonia), chronic obstructive pulmonary disease, interstitial lung disease (idiopathic interstitial pneumonia, acute interstitial pneumonia, adult respiratory distress syndrome, nonspecific interstitial pneumonia, bronchiolitis obliterans with orgazing pneumonia), bronchial asthma, plumonary eosinophilia syndrome (allergic bronchopulmonary aspergillosis, allergic glanulomatous angiitis, chronic eosinophilic pneumonia, acute eosinophilic pneumonia), hypersensitive pneumonia, sarcoidosis, Goodpasture's syndrome, drug-induced pneumonia, radiation-induced pneumonia, collagen diseases of lung, etc.;
6) Brain nerve diseases such as multiple sclerosis, acute disseminated encephalomyelitis, myasthenia gravis, chronic inflammatory demyelinating polyneuropathy, Lambert-Eaton myasthenic syndrome, HTLV-1 associated myelopathy, meningitis tuberculosa, central nervous system lupus, vasculitis-associated neuropathy, Sjogren's syndrome assocaited nervous symptoms, Tolosa-Hunt syndrome, nerve sarcoidosis, nerve Behcet's disease, idiopathic peripheral facial palsy, brain edema associated with head injury and brain cancer, cord injury, etc.;
7) Thyroid deficiencies such as thyroid crisis, Grave's ophthalmopathy, subacute thyroiditis, drug-induced hyperthyroidism, myxedematoid coma, etc,;

8) Digestive disorders such as Crohn's disease, ulcerative colitis, primary malignant lymphoma of the bowel, intestinal Behcet's disease, etc.;

9) Hepatic diseases such as acute hepatitis, fulminant hepatitis, autoimmune hepatitis, etc.;

10) Ophthalmic disorders such as allergic conjunctivitis, hey fever, spring catarrh, rejection after cornea grafting, inflammation of the iris, iridocyclitis, Vogt-Koyanagi-Harada disease, neuritis optica, etc.;

11) Skin diseases such as autoimmune hydroa (pemphigus, pemphigoid, linear IgA blister, epidermolysis bullosa acquisita), skin diseases assocaited with connective tissue disease, neutrophil dermatosis (Sweet's disease, pyoderma gangrenosum), Weber-Christian disease, Stevens-Johnson syndrome, toxic epidermal necrosis, cutaneous vasculitis, veeping eczema, drug eruption, measles, erythema exsudativum multiforme, erythema nodosum, scleroderma, discoid lupus erythematosus, lupus erythematosus profundus, cutaneous malignant lymphoma, cutaneous sarcoidosis, alopecia areata, strawberry birthmark, prurigo, pustulosis palmoplantaris, psoriasis, insect bites, toxic eruption, pityriasis rosea Gibert, lichen red planus, erythroderma, hyperplastic scar, keloid, granulomatosis, amyloid lichen, alopecia areata, etc.;

12) Orthopedic surgery related diseases such as osteoarthritis, gout, pseudogout, inflammation in the tendon or around the tendon, entrapment syndrome, disc hernia, etc.

In addition, the glucocorticoid function regulating agent can be applied to the treatment of the following diseases, where the action of glucocorticoid is expected to be suppressed.

Namely, 1) diseases wherein the blood cortisol level is high or GR is overactivated, such as Cushing's syndrome; 2) conditions wherein the immunity is lowered due to HIV-infection; 3) depression; 4) changes accompanying to the overstressed condition; 5) diabetes mellitus, etc.

In addition, the glucocorticoid function regulating agent of the present invention can be used as a GR partial agonist in the treatment or prophylaxis of inflammatory diseases.

Further, the present compounds can also be used together with another drug so that the effect of such drug can be enhanced, for example, immune suppressor, anti-inflammatory agent, antirheumatic agent, antithrombotic agent, antihistamine agent, antiallergic agent, β2 stimulant, ST combination drug, antidiabetic agent, remedy for diabetes complications, antilipidemic agents, hypotensive agent, antiobesity agent (hereinafter, referred to as a coadministered drug).

The immune suppressor includes cyclophosphamide, methotrexate, cyclosporine A, etc. The anti-inflammatory agent includes indometacin, bucillamine, etc. The antirheumatic agent includes leflunomide, sulfasalazine, rimacalib, etc. The antithrombotic agent includes warfarin, etc. The antihistamine agent includes olopatadine hydrochloride, fexofenadine hydrochloride, etc. The antiallergic agent includes tranilast, etc. The β2 stimulant includes salbutamol sulfate, procaterol hydrochloride, etc. The ST combination agent includes co-trimoxazole, etc. The antidiabetic agent includes insulin sensitizer (pioglitazone or a hydrochloride thereof, etc.), etc.

The remedy for diabetes complications, antilipidemic agent, hypotensive agent, antiobesity agent include central antiobesity agent (phentermine, etc.) or pancreatic lipase inhibitor (orlistat, etc.).

When anti-inflammatory activity or immune regulation activity is desired, then the coadministered drug is preferably methotrexate, indometacin, fexofenadine hydrochloride, etc., and the antidiabetic agent may be preferably insulin sensitizer, etc.

When the present compound is used together with a coadministered drug, the dosage of these coadministered drug can be reduced within the safe range from viewpoint of the side effects of these drugs.

When the present compound is clinically used, it can be administered either orally or parenterally in the form of a pharmaceutical composition (e.g., intravenously, subcutaneously, intramuscularly, locally, rectrally, percutaneously, or transnasally, transpulmonaryly). The dosage form for oral administration includes, for example, tablets, capsules, pils, granules, powders, liquids, suspension, etc. The dosage form for parenteral administration includes, for example, aqueous injections, oily injections, ointments, creams, lotions, aerosols, suppositories, patches, etc. These formulations may be prepared by a conventional technique, and can contain a conventional nontoxic and inactive carrier or excipient, which is conventionally used in the pharmaceutical field.

The dosage of the present compound may vary according to each compound, or diseases, ages, body weight, sex, condition of patients, or administration routes, but the present fused pyrrole derivative, or a prodrug thereof, or a pharmaceutically acceptable salt thereof is usually administered in at a dose of 0.1 to 1000 mg/day in adult (body weight: 50 kg), preferably 1 to 300 mg/day in an adult, which is administered once a day or divided into 2 to 3 dosage units, or alternatively which is administered once a day for a period of several days to several weeks.

Further, the present compounds can also be used together with another drug with the aim of enhancing the effects of such drugs, for example, together with immune suppressor, anti-inflammatory agent, antirheumatic agent, antithrombotic agent, antihistamine agent, antiallergic agent, β2 stimulant, ST combination drug, antidiabetic agent, remedy for diabetes complications, antilipidemic agents, hypotensive agent, antiobesity agent (coadministered drug).

The timing of administration of the present compound and a coadministered drug is not necessarily specified, and both can be administered simultaneously or time-differently to a subject, or the present compound and a coadministered drug may be administered in the form of a combined drug. The dosage of a coadministered drug can be determined suitably based on the conventional clinically-used dosage range thereof. In addition, the combination ratio of the present compound and a coadministered drug may be determined suitably according to the subjects to be administered, the administration routes, the diseases to be treated, or the combination of two drugs. For example, when the administration subject is a human, then a coadministered drug is administered in an amount of 0.01 to 100 parts by weight to 1 part by weight of the present compound.

Examples

The present invention is illustrated in more detail by the following Examples and Experiments, but should not be construed to be limited thereto. In addition, the compound names used in Reference Examples and Examples are not ones as prescribed by IUPAC nomenclature system.

Reference Example 1

2,2,2-Trifluoro-1-(6-nitro-1H-indol-3-yl)ethanone

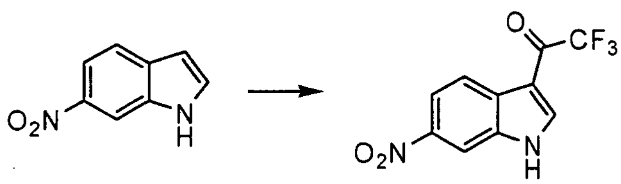

To a solution of 6-nitroindole (5.0g) in tetrahydrofuran (25 ml) was added trifluroacetic anhydride (6.55 ml), and the mixture was stirred at 25˚C for 30 hours. Water was added to the reaction solution, and the mixture was extracted with ethyl acetate, and the organic layer was washed with a saturated saline solution, dried over sodium sulfate, and filtered. The filtrate is concentrated under reduced pressure, and the obtained residue was purified by silica gel column chromatography to give the title compound (6.07 g).
$^1$H-NMR (400 MHz, $CDCl_3$) δ 8.54 (d, J= 8.9 Hz, 1H), 8.46 (d, J= 1.9 Hz, 1H), 8.32-8.26 (m, 2H).
MS (ESI+) 259, 2.11($M^+$+1, detection time)

Reference Example 2

1-(1-Benzyl-6-nitro-1H-indol-3-yl)-2,2,2-trifluoroethanone

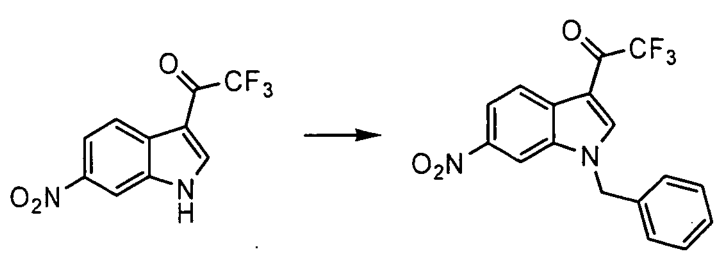

To a solution of 2,2,2-trifluoro-1-(6-nitro-1H-indol-3-yl)ethanone (5.16 g) obtained in Reference Example 1 in N,N-dimethylformamide (50 ml) were added potassium carbonate (8.30 g) and benzyl bromide (5.13g), and the mixture was stirred at 80˚C for 4 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to

give the title compound (6.57 g).
$^1$H-NMR (400 MHz, $CDCl_3$) δ 8.53 (d, J= 8.8 Hz, 1H), 8.33 (d, J= 1.8 Hz, 1H), 8.26 (dd, J= 8.8, 1.5 Hz, 1H), 8.20-8.15 (m, 1H), 7.45-7.36 (m, 3H), 7.25-7.19 (m, 2H), 5.50 (s, 2H).
MS (ESI+) 349, 2.50($M^+$+1, detection time)

Reference Example 3

1-Benzyl-6-nitro-3-[2-(trifluoromethyl)oxiran-2-yl]-1H-indole

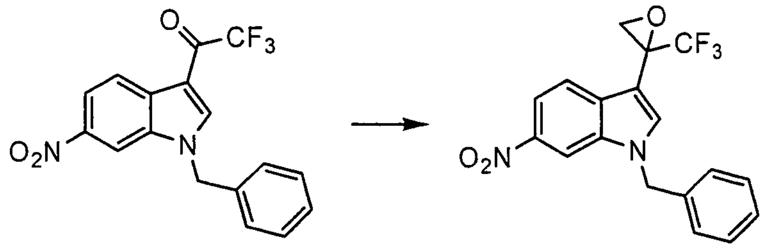

To a mixture of dimethylsulfoxide (20 ml) and tetrahydrofuran (30 ml) was added sodium hydride (55 %, 367 mg), and the mixture was stirred at 25˚C for 30 minutes. The reaction solution was cooled to -10˚C, and thereto was added trimethylsulfonium iodide (1.71 g), and the mixture was further stirred at -10˚C for 30 minutes. To the mixture was added 1-(1-benzyl-6-nitro-1H-indol-3-yl)-2,2,2-trifluoroethanone (2.43 g) obtained in Reference Example 2 at the same temperature, and the mixture was stirred for 5 hours while it was warmed to 25˚C. Then, water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (1.88 g).
$^1$H-NMR (400 MHz, $CDCl_3$) δ 8.30 (d, J= 1.9 Hz, 1H), 8.08 (dd, J= 8.9, 1.9 Hz, 1H), 7.82 (d, J= 8.9 Hz, 1H), 7.59 (s, 1H), 7.40-7.30 (m, 3H), 7.19-7.13 (m, 2H), 5.41 (s, 2H), 3.50 (d, J= 5.3 Hz, 1H), 3.16-3.10 (m, 1H).
MS (ESI+) 363, 2.54($M^+$+1, detection time)

Example 1

2-(1-Benzyl-6-nitro-1H-indol-3-yl)-1,1,1-trifluoro-3-piperidin-1-ylpropan-2-ol

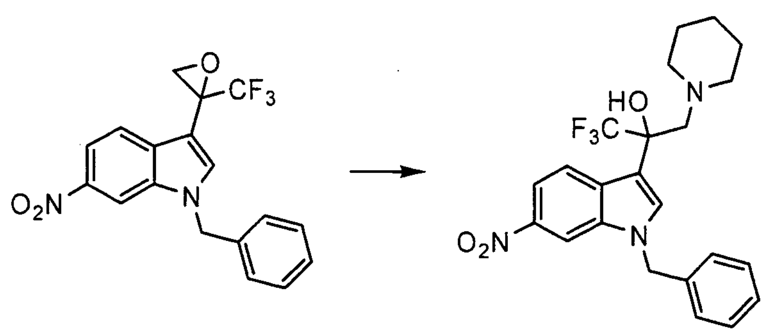

To a solution of 1-benzyl-6-nitro-3-[2-(trifluoromethyl)oxiran-2-yl]-1H-indole (72 mg) obtained in Reference Example 3 in isopropyl alcohol (3 ml) was added piperidine (26 mg), and the mixture was stirred at 90˚C for 10 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (56 mg). $^1$H-NMR (400 MHz, $CDCl_3$) δ 8.25 (d, J= 2.0Hz, 1H), 8.0 (dd, J= 9.0, 2.0 Hz, 1H), 7.90 (d, J= 9.0 Hz, 1H), 7.58 (s, 1H), 7.33-7.31

(m, 3H), 7.09 (d, J= 7.8 Hz, 2H), 6.06 (bs, 1H), 5.40 (s, 2H), 3.18 (d, J= 13.6 Hz, 1H), 3.07 (d, J= 13.6 Hz, 1H), 2.49-2.46 (m, 4H), 1.57-1.40 (m, 6H).
MS (ESI+) 448, 1.95($M^+$+1, detection time)

Reference Example 4

1-Benzyl-6-nitro-1 H-indole

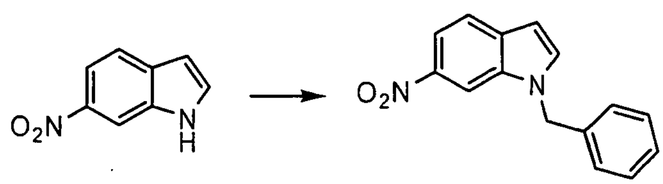

To a solution of 6-nitroindole (1.01 g) in N,N-dimethylformamide (25 ml) were added potassium carbonate (2.58 g) and benzyl bromide (1.17g), and the mixture was stirred at 70°C for 8 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (1.01 g).
$^1$H-NMR (400 MHz, $CDCl_3$) $\delta$ 8.29 (d, J= 1.9 Hz, 1H), 8.02 (dd, J= 8.8, 1.9 Hz, 1H), 7.68 (d, J= 8.8 Hz, 1H), 7.41 (d, J= 3.1 Hz, 1H), 7.39-7.27 (m, 3H), 7.16-7.10 (m, 2H), 6.65 (dd, J= 3.1, 0.9 Hz, 1H), 5.41 (s, 2H).
MS (ESI+) 253, 2.35($M^+$+1, detection time)

Example 2

Ethyl 2-(1-benzyl-6-nitro-1H-indol-3-yl)-3,3,3-trifluoro-2-hydroxypropanoate
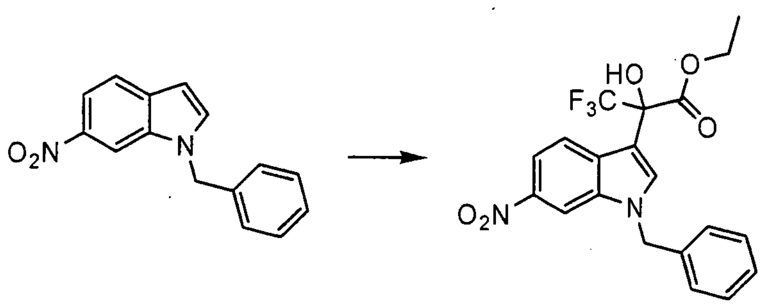

To a solution of 1-benzyl-6-nitro-1H-indole (1.01 g) obtained in Reference Example 4 in dichloromethane (7.5ml) were added ethyl 3,3,3-trifluoropyruvate (905 mg) and trifluoromethanesulfonic acid (70 ul), and the mixture was stirred at 25°C for 8 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (410 mg).
MS (ESI+) 423, 2.45($M^+$+1, detection time)

Example 3

Ethyl 2-(1-benzyl-6-nitro-1H-indol-3-yl)-3,3,3-trifluoro-2-(methoxymethoxy)-propanoate
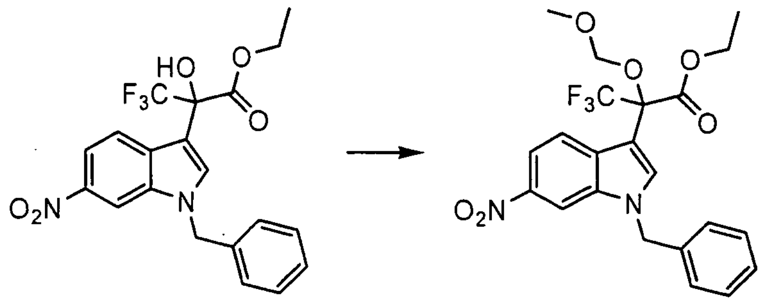

To a solution of ethyl 2-(1-benzyl-6-nitro-1H-indol-3-yl)-3,3,3-trifluoro-2-hydroxypropanoate (422 mg) obtained in Example 2 in N,N-dimethylformamide (5 ml) was added sodium hydride (55 %, 52 mg), and the mixture was stirred at 25˚C for 10 minutes. Further, chloromethyl methyl ether (121 mg) was added to the reaction solution, and the mixture was stirred at 80˚C for 3 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (444 mg).

$^1$H-NMR (400 MHz, $CDCl_3$) δ 8.27 (d, J= 2.0 Hz, 1H), 8.04 (dd, J= 9.0, 2.0 Hz, 1H), 7.81 (d, J= 9.0 Hz, 1H), 7.74 (s, 1H), 7.38-7.32 (m, 3H), 7.15-7.13 (m, 2H), 5.42 (s, 2H), 4.90 (d, J= 6.5 Hz, 1H), 4.82 (d, J= 6.5 Hz, 1H), 4.37 (q, J= 7.2 Hz, 2H), 3.48 (s, 3H), 1.30 (t, J= 7.2 Hz, 3H).

MS (ESI+) 467, 2.60($M^+$+1, detection time)

Example 4

2-(1-Benzyl-6-nitro-1H-indol-3-yl)-3,3,3-trifluoro-2-(methoxymethoxy)propanoic acid

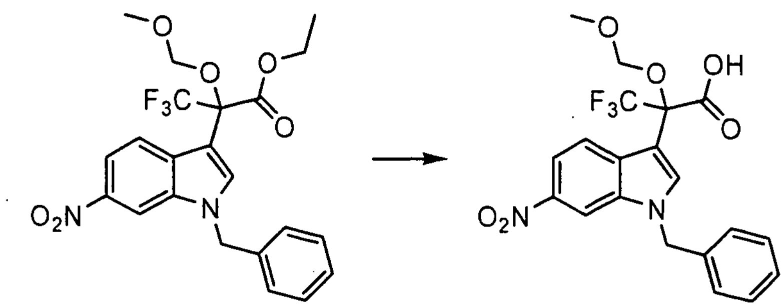

To a solution of ethyl 2-(1-benzyl-6-nitro-1H-indol-3-yl)-3,3,3-trifluoro-2-(methoxymethoxy)propanoate (444 mg) obtained in Example 3 in a mixture of tetrahydrofuran (10ml) and methanol (10 ml) was added 1N aqueous sodium hydride solution (5 ml), and the mixture was stirred at 25˚C for 10 hours. Water was added to the reaction solution, and the mixture was extracted with ethyl acetate. The organic layer was washed with a saturated saline solution, dried over sodium sulfate, and filtered. The filtrate was concentrated under reduced pressure to give the title compound (444 mg) as a residue.

MS (ESI+) 439, 2.31($M^+$+1, detection time)

Example 5

Ethyl [4-({1-[2-(1-benzyl-6-nitro-1H-indol-3-yl)-3,3,3-trifluoro-2-(methoxymethoxy)propanoyl]piperidin-4-yl}oxy)-3-methoxyphenyl]acetate

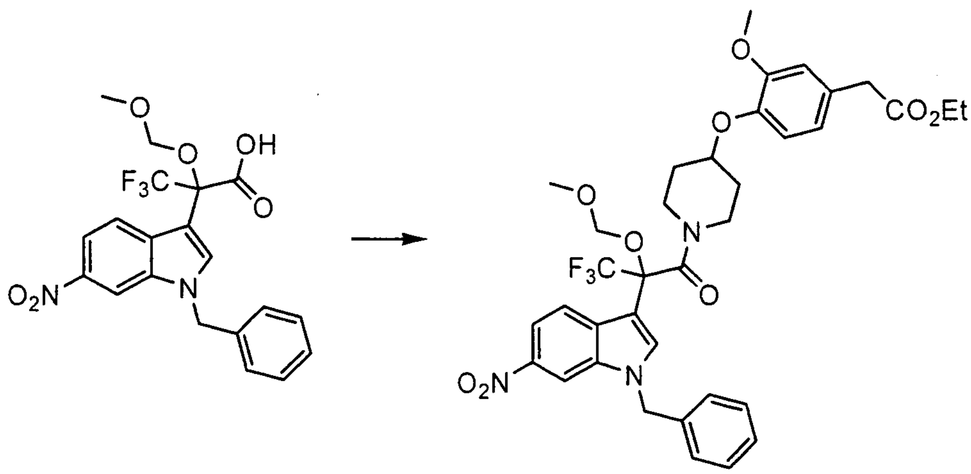

To a solution of 2-(1-benzyl-6-nitro-1H-indol-3-yl)-3,3,3-trifluoro-2-(methoxymethoxy)propanoic acid (444 mg) obtained in Example 4 in N,N-dimethylformamide (25 ml) were added TBTU {O-(benzotriazol-1-yl)-1,1,3,3-tetramethyl uronium tetrafluoroborate} (385 mg), triethylamine (303 mg), and ethyl [3-methoxy-4-(piperidin-4-yloxy)phenyl]acetate hydrochloride (396 mg), and the mixture was stirred at 25˚C for 3 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (460 mg).
$^1$H-NMR (400 MHz, $CDCl_3$) δ 8.30 (bdd, J= 2.5, 2.1 Hz, 1H), 8.06-8.00 (m, 1H), 7.86 (dd, J= 9.1, 9.0 Hz, 1H), 7.61+7.53 (s, 1H), 7.34-7.28 (m, 3H), 7.13-7.08 (m, 2H), 6.79-6.76 (m, 1H), 7.73-6.66 (m, 2H), 5.41+5.40 (s, 2H), 5.05+5.04 (s, 2H), 4.27-4.01 (m, 3H), 4.12 (q, J= 7.0 Hz, 2H), 3.79+3.68 (s, 2H), 3.62-3.35 (m, 2H), 3.57+3.56 (s, 3H), 3.51 (s, 3H), 1.98-1.32 (m, 2H), 1.25 (t, J= 7.0 Hz, 3H).
MS (ESI+) 714, 2.65($M^+$+1, detection time)

Example 6

[4-({1-[2-(1-Benzyl-6-nitro-1H-indol-3-yl)-3,3,3-trifluoro-2-(methoxymethoxy)-propanoyl]piperidin-4-yl}oxy)-3-methoxyphenyl]acetic acid

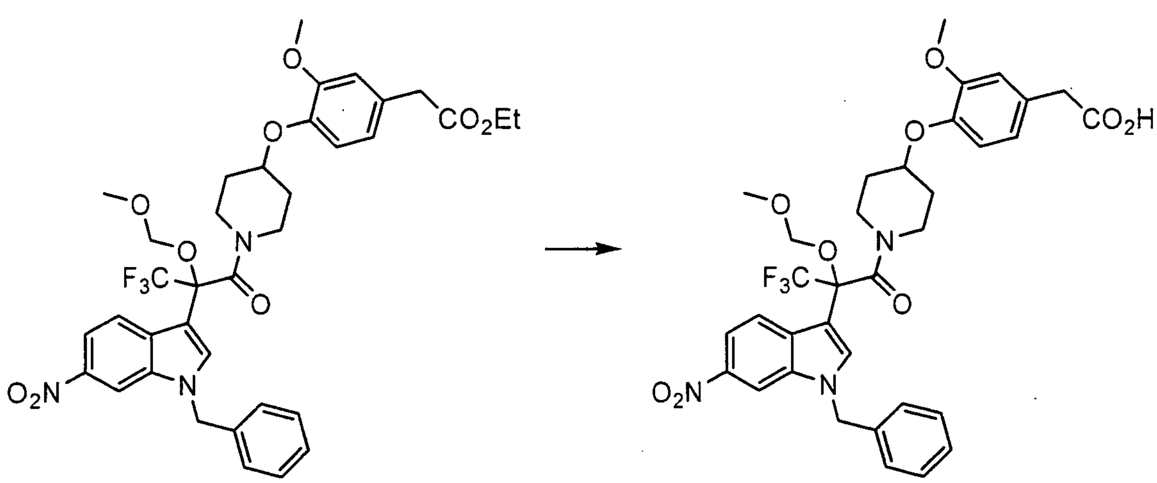

To a solution of ethyl [4-({1-[2-(1-benzyl-6-nitro-1H-indol-3-yl)-3,3,3-trifluoro-2-(methoxymethoxy)propanoyl]piperidin-4-yl}oxy)-3-methoxyphenyl]-acetate (460 mg) obtained in Example 5 in a mixed solvent of tetrahydrofuran (10 ml) and methanol (10 ml) was added 1 N aqueous sodium hydride solution (5 ml), and the mixture was stirred at 25˚C for 3 hours. Water was added to the reaction solution, and the mixture was extracted with ethyl acetate. The organic layer

was washed with a saturated saline solution, dried over sodium sulfate, and filtered. The filtrate was concentrated under reduced pressure to give the title compound (300 mg) in the form of a residue. MS (ESI+) 686, 2.46($M^{+}$+1, detection time)

Example 7

[4-({1-[2-(1-Benzyl-6-nitro-1H-indol-3-yl)-3,3,3-trifluoro-2-hydroxypropanoyl]-piperidin-4-yl}oxy)-3-methoxyphenyl]acetic acid

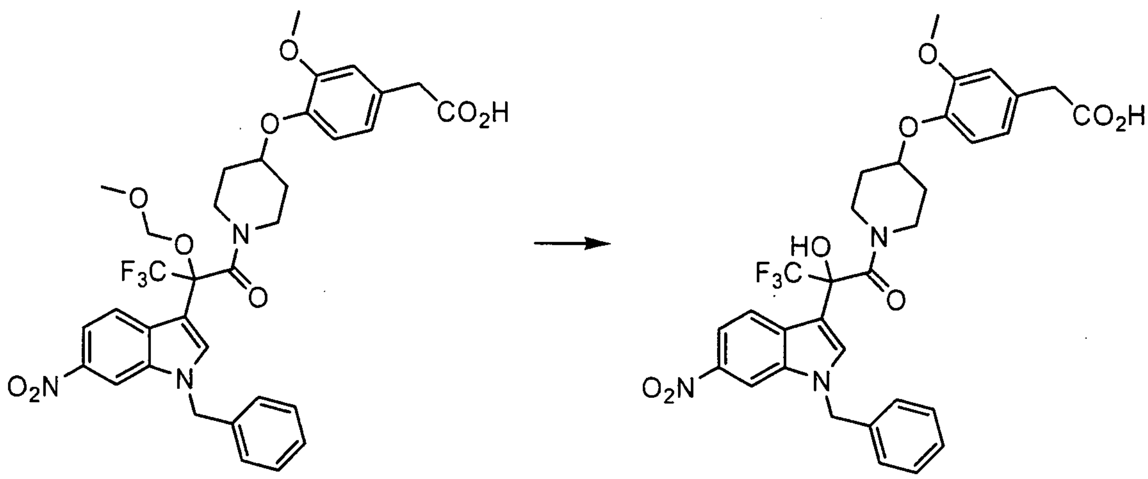

To a solution of [4-({1-[2-(1-benzyl-6-nitro-1H-indol-3-yl)-3,3,3-trifluoro-2-(methoxymethoxy)propanoyl]piperidin-4-yl}oxy)-3-methoxyphenyl]acetic acid (240 mg) obtained in Example 6 in dichloromethane (2 ml) was added trifluoroacetic acid (2 ml), and the mixture was stirred at 25˚C for 10 minutes. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (110 mg).
$^{1}$H-NMR (400 MHz, $CDCl_3$) δ 8.29 (d, J= 1.7 Hz, 1H), 8.01 (bd, J= 7.7 Hz, 1H), 7.59 (bs, 1H), 7.53 (d, J= 8.9 Hz, 1H), 7.31-7.28 (m, 3H), 7.10-7.08 (m, 2H), 6.77 (bs, 1H), 6.74-6.69 (m, 2H), 5.43 (d, J= 5.0 Hz, 2H), 4.26-4.19 (m, 1H), 3.87-3.69 (m, 2H), 3.77 (bs, 3H), 3.56 (s, 2H), 3.49-3.30 (m, 2H), 1.87-1.60 (m, 2H).
MS (ESI+) 642, 2.33($M^{+}$+1, detection time)

Example 8

2-(1-Benzyl-6-nitro-1H-indol-3-yl)-1,1,1-trifluoropent-4-en-2-ol

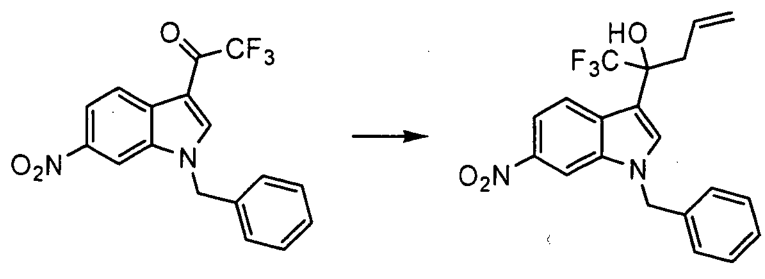

A mixture of indium powder (230 mg) and allyl bromide (726 mg) in tetrahydrofuran (10 ml) was stirred at 50˚C for one hour. The reaction solution was cooled to 0˚C, and thereto was added the compound obtained in Reference Example 2 (348 mg), and the mixture was stirred at 0˚C for one hour. To the reaction solution was added a saturated aqueous

ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (371 mg).
$^1$H-NMR (400 MHz, $CDCl_3$) δ 8.27 (d, J= 2.0 Hz, 1H), 8.04 (dd, J= 9.0, 2.0 Hz, 1H), 7.96 (d, J= 9.0 Hz, 1H), 7.49 (s, 1H), 7.40-7.28 (m, 3H), 7.11 (d, J= 8.6 Hz, 2H), 5.72-7.59 (m, 1H), 5.40 (s, 2H), 5.27 (dd, J= 12.7, 1.4 Hz, 1H), 5.24 (dd, J= 7.3, 1.4 Hz, 1H), 3.09 (dd, J= 14.3, 6.8 Hz, 1H), 2.94 (dd, J= 14.3, 6.8 Hz, 1H), 2.72 (s, 1H).
MS (ESI+) 391, 2.40($M^+$+1, detection time)

Example 9

(4E)-2-(1-Benzyl-6-nitro-1H-indol-3-yl)-1,1,1-trifluoro-5-(4-fluorophenyl)pent-4-en-2-ol

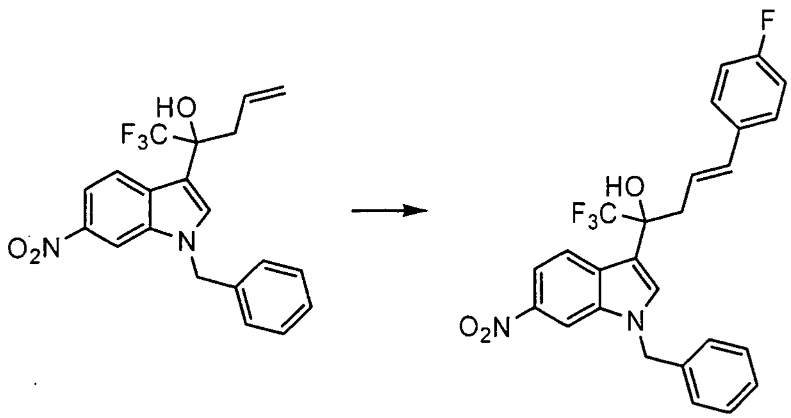

To a solution of 2-(1-benzyl-6-nitro-1H-indol-3-yl)-1,1,1-trifluoropent-4-en-2-ol (98 mg) obtained in Example 8 in acetonitrile (4 ml) were added palladium (II) acetate (5.6 mg), dicyclohexylmethylamine (98 mg), 1-fluoro-4-iodobenzene (56 mg), benzyltriethylammonium chloride (46 mg), and the mixture was stirred at 70°C for 6 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (32 mg).
$^1$H-NMR (400 MHz, $CDCl_3$) δ 8.28 (d, J= 2.0 Hz, 1H), 8.05 (dd, J=9.0, 2.0 Hz, 1H), 7.99 (d, J= 9.0 Hz, 1H), 7.52 (s, 1H), 7.32-7.24 (m, 3H), 7.19 (dd, J= 8.5, 5.4 Hz, 2H), 7.08 (d, J= 7.4 Hz, 2H), 6.96 (dd, J= 8.5, 8.5 Hz, 2H), 6.50 (d, J= 15.8 Hz, 1H), 5.97-5.85 (m, 1H), 5.41 (s, 2H), 3.21 (dd, J= 14.3, 7.1 Hz, 1H), 3.07 (dd, J= 14.3, 7.1 Hz, 1H), 2.74 (s, 1H).
MS (ESI+) 485, 2.54($M^+$+1, detection time)

Example 10

3-(1-Benzyl-6-nitro-1H-indol-3-yl)-4, 4, 4-trifluoro-3-hydroxybutanal

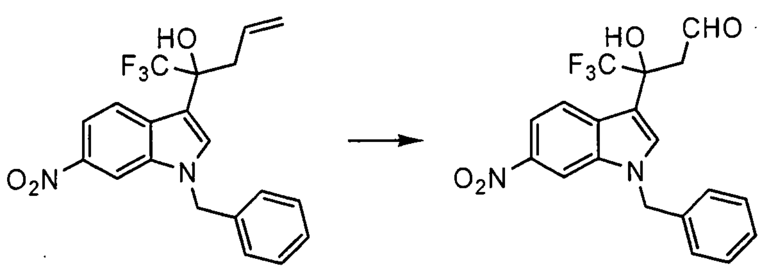

A solution of 2-(1-benzyl-6-nitro-1H-indol-3-yl)-1,1,1-trifluoropent-4-en-2-ol (2.20 g) obtained in Example 8 in methanol (30 ml) was cooled to -78°C, and ozone was blowed into the mixture with bubbling for 30 minutes. Nitrogen gas was

further blowed thereto with bubbling for 30 minutes, and thereto was added dimethylsulfide (621 ul). The reaction solution was warmed to 25˚C with stirring over a period of 3 hours, and the reaction solution was concentrated under reduced pressure. Water and ethyl acetate were added, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (900 mg).

$^1$H-NMR (400 MHz, $CDCl_3$) δ 9.78 (s, 1H), 8.27 (d, J= 2.0 Hz, 1H), 8.06 (dd, J= 9.0, 2.0 Hz, 1H), 7.96 (d, J= 9.0 Hz, 1H), 7.48 (s, 1H), 7.40-7.30 (m, 3H), 7.10 (dd, J= 7.7, 1.7 Hz, 2H), 5.40 (s, 2H), 4.25 (s, 1H), 3.43 (dd, J= 17.3, 1.4 Hz, 1H), 3.27 (dd, J= 17.3, 1.4 Hz, 1H).

MS (ESI+) 393, 2.37($M^+$+1, detection time)

Example 11

2-(1-Benzyl-6-nitro-1H-indol-3-yl)-1,1,1-trifluoro-4-morpholin-4-ylbutan-2-ol

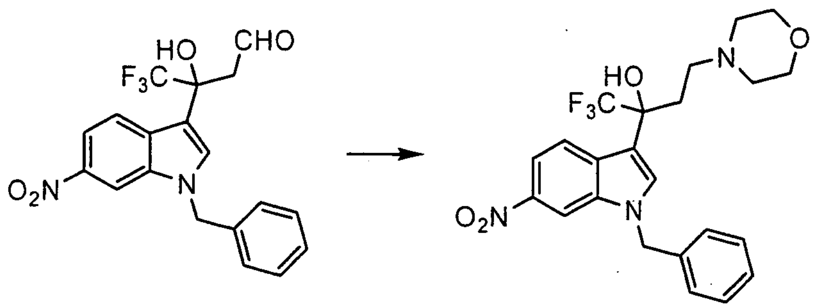

A solution of 3-(1-benzyl-6-nitro-1H-indol-3-yl)-4,4,4-trifluoro-3-hydroxybutanol (39 mg) obtained in Example 10, morpholine (9.6 mg) and acetic acid (6.3 ul) in tetrahydrofuran (10 ml) was stirred at 25˚C for 30 minutes. To the reaction solution was added sodium triacetoxyborohydride (34 mg), and the mixture was stirred at 25˚C for 10 hours. To the reaction solution was added aqueous potassium carbonate solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (20 mg).

$^1$H-NMR (400 MHz, $CDCl_3$) δ 8.30 (d, 2.0 Hz, 1H), 8.02 (dd, J= 9.0, 2.0 Hz, 1H), 7.86 (d, J= 9.0 Hz, 1H), 7.58 (s, 1H), 7.39-7.29 (m, 3H), 7,12 (dd, J= 7.7, 2.0 Hz, 2H), 5.44 (d, J= 15.8 Hz, 1H), 5.37 (d, J= 15.8 Hz, 1H), 3.68 (bs, 4H), 2.76-2.55 (m, 2H), 2.55-2.40 (m, 2H), 2.34-2.20 (m, 2H), 1.72-1.44 (m, 2H).

MS (ESI+) 464, 1.93($M^+$+1, detection time)

Example 12

3-(1-Benzyl-6-nitro-1H-indol-3-yl)-4,4,4-trifluoro-3-hydroxybutanoic acid

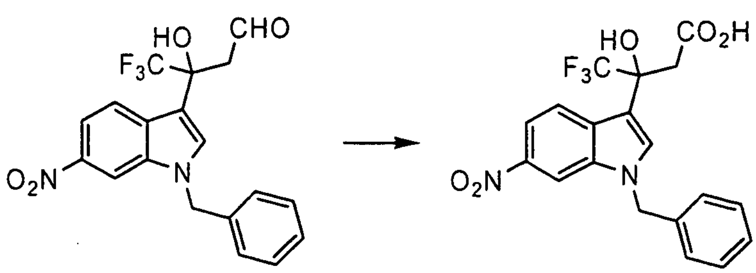

A solution of 3-(1-benzyl-6-nitro-1H-indol-3-yl)-4,4,4-trifluoro-3-hydroxybutanal (392 mg) obtained in Example 10, sodium chlorite (137 mg), sodium dihydrogenphosphate dihydrate (281 mg), 2-methyl-2-butene (1.0 ml) in a mixed solvent of acetonitrile (20 ml) and water (10 ml) was stirred at 25°C for 10 hours. To the reaction solution was added sodium thiosulfate (500 mg), and the mixture was stirred at 25°C for one hour. The reaction solution was concentrated under reduced pressure, and thereto was added 1N hydrochloric acid, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (232 mg).
MS (ESI+) 409, 2.24($M^{+}$+1, detection time)

Example 13

2-(1-Benzyl-6-nitro-1H-indol-3-yl)-1,1,1-trifluoro-4-morpholin-4-yl-4-oxobutan-2-ol

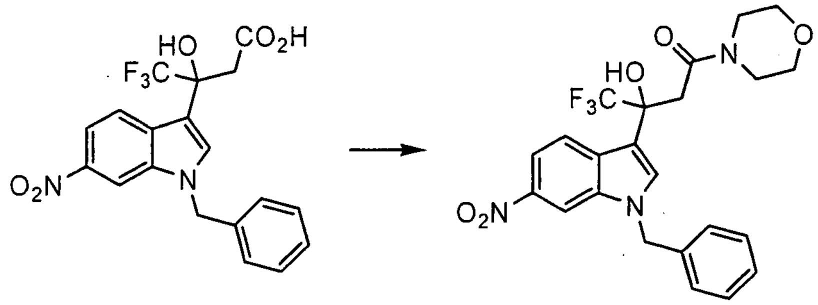

To a solution of 3-(1-benzyl-6-nitro-1H-indol-3-yl)-4,4,4-trifluoro-3-hydroxybutanoic acid (49 mg) obtained in Example 12, morpholine (11 mg), 4-dimethylaminopyridine (15 mg) in N,N-dimethylformamide (1.5 ml) was added thionyl chloride (8.8 ul) at 0°C, and the mixture was stirred at 25°C for 6 hours. To the reaction solution was added sodium thiosulfate (500 mg), and the mixture was stirred at 25°C for one hour. Saturated aqueous ammonium chloride solution was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (7.4 mg).
MS (ESI+) 477, 2.34($M^{+}$+1, detection time)

Example 14

3-(1-Benzyl-6-nitro-1H-indol-3-yl)-4,4,4-trifluoro-3-hydroxy-1-phenylbutan-1-one

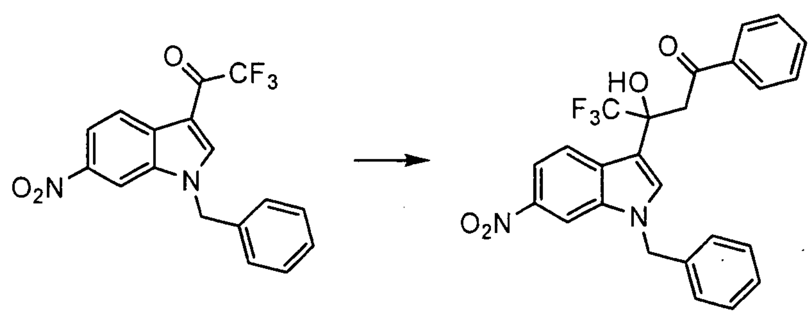

To a solution of the compound (700 mg) obtained in Reference Example 2, 1-phenyl-1-(trimethylsiloxy)ethylene (800 mg) in dichloromethane (100 ml) was added titanium tetrachloride (440 ul) at -78°C. The mixture was stirred for 3 hours while the temperature was raised from -78°C to 0°C. Saturated aqueous ammonium chloride solution was added to the reaction solution, and the mixture was extracted with ethyl acetate. The organic layer was washed with a saturated

saline solution, dried over sodium sulfate, and filtered. The filtrate was concentrated under reduced pressure, and the obtained residue was purified by silica gel column chromatography to give the title compound (256 mg).
$^1$H-NMR (400 MHz, $CDCl_3$) δ 8.22 (d, J= 1.7 Hz, 1H), 8.09-8.00 (m, 2H), 7.90 (dd, J= 8.2, 1.7 Hz, 2H), 7.64 (dd, J= 8.1, 8.1 Hz, 1H), 7.49 (dd, J= 8.1, 8.1 Hz, 2H), 7.40 (s, 1H), 7.32-7.24 (m, 3H), 7.05-6.96 (m, 2H), 6.00 (s, 1H), 5.33 (s, 2H), 4.05 (d, J= 7.1 Hz, 1H), 3.66 (d, J= 17.1 Hz, 1H).
MS (ESI+) 469, 2.48($M^+$+1, detection time)

Example 15

1-Benzyl-6-nitro-3-[1-(trifluoromethyl)vinyl]-1H-indole

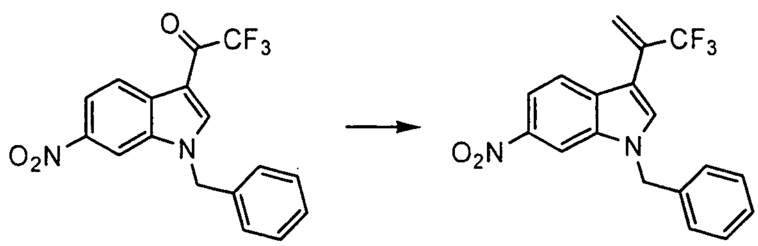

A solution of methyltriphenylphosphonium bromide (5.78 g) in tetrahydrofuran (50 ml) was cooled to -78°C, and thereto was added a solution of butyllithium in hexane (2.71 M, 5.98 ml). The mixture was stirred at 25°C for one hour, and thereto was added the compound of Reference Example 2 (2.8 g), and the mixture was further stirred at 25°C for 3 hours. To the reaction solution was added a saturated saline solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (1.89 g).
$^1$H-NMR (400 MHz, $CDCl_3$) δ 8.30 (d, J= 1.9 Hz, 1H), 8.10 (dd, J= 8.9, 2.0 Hz, 1H), 7.84 (d, J= 8.9 Hz, 1H), 7.57 (bs, 1H), 7.38-7.31 (m, 3H), 7.17-7.15 (m, 2H), 6.07 (d, J= 4.0 Hz, 1H), 5.95 (d, J= 4.0 Hz, 1H), 5.42 (s, 2H).
MS (ESI+) 347, 2.59($M^+$+1, detection time)

Example 16

2-(1-Benzyl-6-nitro-1H-indol-3-yl)-3,3,3-trifluoropropane-1,2-diol

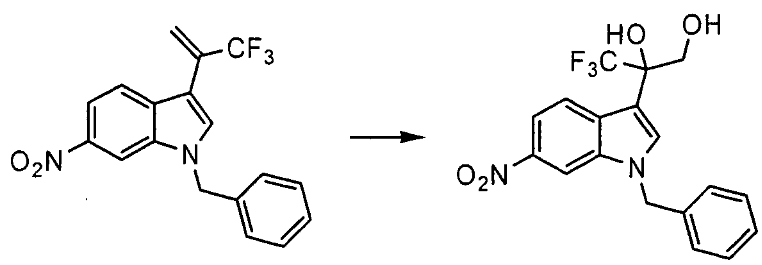

A solution of 1-benzyl-6-nitro-3-[1-(trifluoromethyl)vinyl]-1H-indole (346 mg) obtained in Example 15, AD-mix α (1.4g) in a mixed solvent of tert-butanol (5 ml) and water (5 ml) was stirred at 25°C for 20 hours. To the reaction solution was added sodium thiosulfate, and the mixture was stirred at 25°C for one hour. Then, a saturated saline solution was added to the reaction solution, and extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (383 mg).
MS (ESI+) 381, 1.96($M^+$+1, detection time)

Example 17

Ethyl 2-(1-benzyl-5-nitro-1H-indol-3-yl)-3,3,3-trifluoro-2-hydroxypropanoate

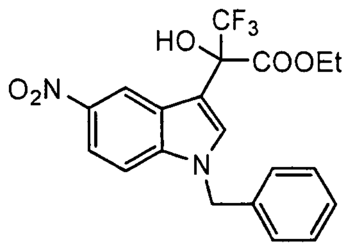

The title compound was obtained from 1-benzyl-5-nitro-indole as a starting compound in a similar manner to the preparation of the compound of Example 2.
$^1$H-NMR (400 MHz, $CDCl_3$) δ 8.98 (d, J= 2.1 Hz, 1H), 8.10 (dd, J= 9.1, 2.4 Hz, 1H), 7.61 (s, 1H), 7.41-7.30 (m, 4H), 7.13-7.11 (m, 2H), 5.36 (s, 2H), 4.52 (s, 1H), 4.52-4.47 (m, 1H), 4.43-4.38 (m, 1H), 1.39 (t, J= 7.2 Hz, 3H).
MS (ESI+) 423, 2.39($M^+$+1, detection time)

Example 18

Ethyl 2-(5-amino-1-benzyl-1H-indol-3-yl)-3,3,3-trifluoro-2-hydroxypropanoate

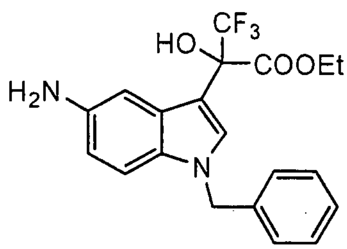

To the compound of Example 17 (1.63 g), iron (1.72 g), ammonium chloride (205 mg) was added a mixed solvent of tetrahydrofuran (15 ml), ethanol (10 ml), and water (5 ml), and the mixture was stirred at 70°C for 6 hours. The reaction solution was filtered through celite with ethyl acetate. The filtrate was washed with a saturated saline solution, dried over sodium sulfate, and filtered.
The filtrate was concentrated under reduced pressure to give the title compound (1.47 g).
MS (ESI+) 393, 1.84($M^+$+1, detection time)

Example 19

Ethyl 2-(1-benzyl-5-{[(4-methylphenyl)sulfonyl]amino}-1H-indol-3-yl)-3,3,3-trifluoro-2-hydroxypropanoate

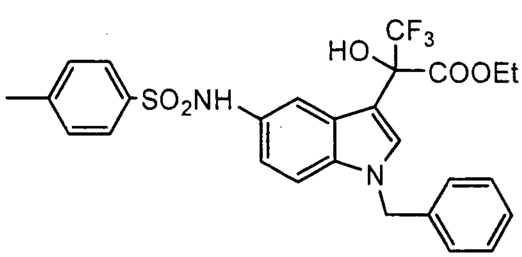

To a solution of the compound of Example 18 (1.47 g) in pyridine (20 ml) was added p-toluenesulfonyl chloride (858 mg), and the mixture was stirred at 70˚C for 8 hours. The reaction solution was concentrated under reduced pressure, and to the residue was added 1N hydrochloric acid, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (1.20 g). H-NMR (400 MHz, $CDCl_3$) δ 7.58 (d, J= 8.19 Hz, 2H), 7,52 (s, 1H), 7.38 (s, 1H), 7.32-7.30 (m, 3H), 7.19 (d, J= 8.2 Hz, 2H), 7.14 (d, J= 8.8 Hz, 1H), 7.10-7.08 (m, 2H), 4.34-4.30 (m, 1H), 4.30 (s, 1H), 2.37 (s, 3H), 1.30 (t, J= 7.13 Hz, 3H)
MS (ESI+) 547, 2.34($M^+$+1, detection time)

Example 20

2-(1-Benzyl-5-{[(4-methylphenyl)sulfonyl]amino}-1H-indol-3-yl)-3,3,3-trifluoro-2-hydroxypropanoic acid

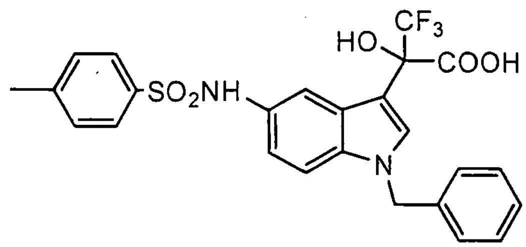

The title compound was obtained from the compound of Example 19 (109 mg) as a starting compound in a simillar manner to the preparation of the compound of Example 4 (Yield: 100 mg).
MS (ESI+) 519, 2.21($M^+$+1, detection time)

Example 21

Ethyl 2-(1-benzyl-7-nitro-1H-indol-3-yl)-3,3,3-trifluoro-2-hydroxypropanoate
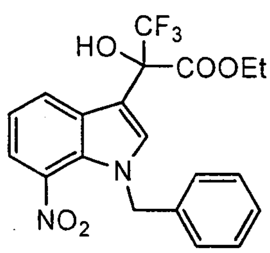

The title compound was obtained from 1-benzyl-7-nitro-1H-indole as a staring compound in a similar manner to the preparation of the compound of Example 2 (Yield: 2.35g)
$^1$H-NMR (400 MHz, $CDCl_3$) δ 8.27 (d, J= 8.1Hz, 1H), 7.70(d, J= 7.9 Hz, 1H), 7.57 (s, 1H), 7.26-7.15 (m, 4H), 6.90-6.87 (m, 2H), 5.46 (s, 2H), 4.51 (s, 1H), 4.48-4.35 (m, 2H), 1.32 (t, J=7.1 Hz, 3H).
MS (ESI+) 423, 2.38($M^+$+1, detection time)

Example 22

Ethyl 2-(7-amino-1-benzyl-1H-indol-3-yl)-3,3,3-trifluoro-2-hydroxypropanoate
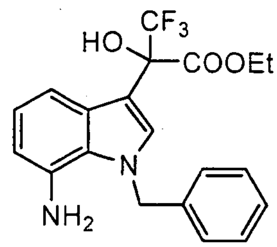

The title compound was obtained from the compound of Example 21 as a starting compound in a similar manner to the preparation of the compound of Example 18 (Yield: 400mg).
MS (ESI+) 393, 2.17($M^+$+1, detection time)

Example 23

2-(1-Benzyl-7-nitro-1H-indol-3-yl)-3,3,3-trifluoropropane-1,2-diol

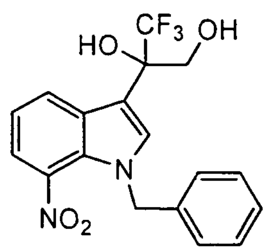

To a solution of the compound of Example 21 (1.91 g) in tetrahydrofuran (50 ml) was added a 0.95M solution of DIBAL (diisobutylaluminium hydride) in hexane (10.5ml) at -78˚C. The mixture was stirred for 5 hours while the temperature was raised to -78˚C to 25˚C. Then, to the mixture was added a 0.95M solution of DIBAL (diisobutylaluminium hydride) in hexane (21 ml) at 25˚C, and the mixture was stirred at the same temperature for one hour. To the reaction solution were added ethyl acetate, water and 1N aqueous sodium hydroxide solution, and the mixture was filtered through celite. The filtrate was extracted with ethyl acetate, and the organic layer was washed with a saturated saline solution, dried over sodium sulfate, and filtered. The filtrate was concentrated under reduced pressure, and the obtained residue was purified by silica gel column chromatography to give the title compound (274 mg).
$^1$H-NMR (400 MHz, $CDCl_3$) δ 8.24 (d, J= 8.1 Hz, 1H), 7.69 (d, J= 7.9 Hz, 1H), 7.40 (s, 1H), 7.23-7.21 (m, 3H), 7.16 (dd, J= 8.0, 8.0 Hz, 1H), 6.87-6.85 (m, 2H), 5.45 (s, 2H), 4.33 (d, J= 11.9 Hz, 1H), 4.08 (d, J= 11.9 Hz, 1H), 3.99 (s, 1H), 2.40 (bs, 1H).
MS (ESI+) 381, 2.21($M^+$+1, detection time)

Example 24

Ethyl 2-(1-benzyl-7-{[(4-methylphenyl)sulfonyl]amino}-1H-indol-3-yl)-3,3,3-trifluoro-2-hydroxypropanoate

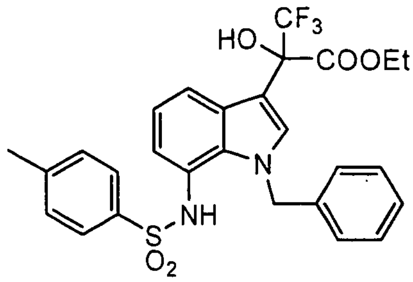

The title compound was obtained from the compound of Example 21 as a starting compound in a similar manner to the preparation of the compound of Example 19.
MS (ESI+) 547, 2.39($M^+$+1, detection time)

Example 25

Ethyl 2-{1-benzyl-7-[(methylsulfonyl)amino]-1H-indol-3-yl}-3,3,3-trifluoro-2-hydroxypropanoate

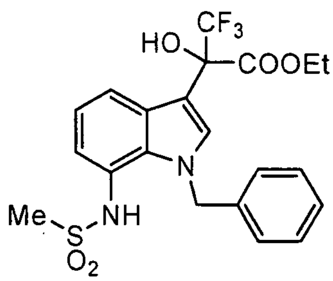

The title compound was obtained from the compound of Example 21 as a starting compound in a similar manner to the preparation of the compound of Example 19.
MS (ESI+) 471, 2.25($M^+$+1, detection time)

Example 26

2-(1-Benzyl-7-nitro-1H-indol-3-yl)-1,1,1-trifluoro-3-phenoxypropan-2-ol

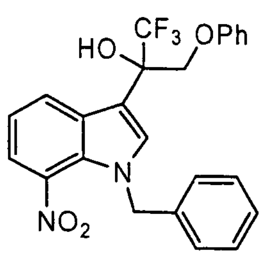

To a solution of the compound of Example 23 (137 mg) in dichloromethane (3 ml) was added methanesulfonyl chloride (49 mg), and the mixture was stirred at 25°C for 30 minutes. Then, a solution of potassium carbonate (207 mg) in methanol (10 ml) was cooled to 0°C, and thereto was added dropwise the dichloromethane solution. The mixture was stirred at the same temperature for 3 hours. Water was added to the reaction solution, and the mixture was extracted with ethyl acetate. The organic layer was washed with a saturated saline solution, dried over sodium sulfate, and filtered.

The filtrate was concentrated under reduced pressure to give the residue (130 mg). A solution of the residue (130 mg), phenol (97 mg) and potassium carbonate (138 mg) in N,N-dimethylformamide (5 ml) was stirred at 80˚C for 6 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (12 mg).
MS (ESI+) 457, 2.61($M^{+}$+1, detection time)

Example 27

Benzyl 1-benzyl-3-[1-(ethoxycarbonyl)-2,2,2-trifluoro-1-hydroxyethyl]-1H-indole-5-carboxylate

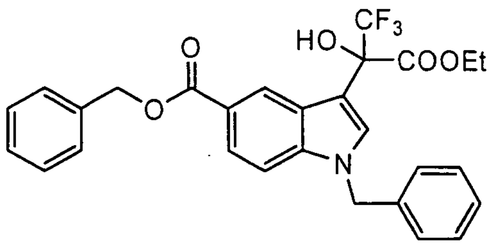

The title compound was obtained from benzyl 1-benzyl-1H-indole-5-carboxylate as a starting compound in a similar manner to the preparation of the compound of Example 2.
MS (ESI+) 512, 2.55($M^{+}$+1, detection time)

Example 28

1-Benzyl-3-[1-(ethoxycarbonyl)-2,2,2-trifluoro-1-hydroxyethyl]-1H-indole-5-carboxylic acid

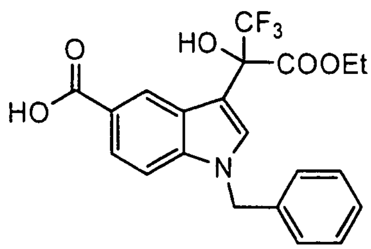

A solution of the compound of Example 27 (130 mg) and 5 % Pd-C (130 mg) in tetrahydrofuran (10 ml) was stirred under hydrogen atmosphere at 25˚C for 8 hours. The reaction solution was filtered through celite with ethyl acetate. Water was added to the filtrate, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (80 mg).
MS (ESI+) 422, 2.15($M^{+}$+1, detection time)

Example 29

Ethyl 2-(1-benzyl-1H-indol-3-yl)-3,3,3-trifluoro-2-hydroxypropanoate

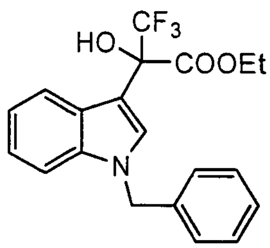

The title compound was obtained from N-benzyl-indole as a starting compound in a similar manner to the preparation of the compound of Example 2 (Yield: 640 mg).
$^{1}$H-NMR (400 MHz, $CDCl_3$) δ 7.93 (d, J= 8.0 Hz, 1H), 7.40 (s, 1H), 7.31-7.05 (m, 8H), 5.31 (s, 2H), 4.49-4.39 (m, 1H), 4.40 (s, 1H), 4.39-4.30 (m, 1H), 1.31 (t, J= 7.1 Hz, 3H).

Reference Example 9

Ethyl 3,3,3-trifluoro-2-hydroxy-2-(1H-indol-3-yl)propanoate

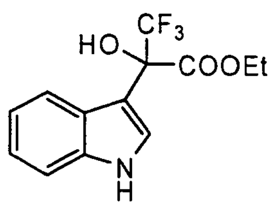

The title compound was obtained from indole as a starting compound in a similar manner to the preparation of the compound of Example 2 (Yield: 470 mg).
$^{1}$H-NMR (400 MHz, $CDCl_3$) δ 8.29 (bs, 1H), 7.90 (d, J= 8.1 Hz, 1H), 7.45 (d, J= 2.7 Hz, 1H), 7.36 (d, J= 8.1 Hz, 1H), 7.22 (dd, J= 8.2, 7.1 Hz, 1H), 7.16 (dd, J= 8.1, 7.1 Hz, 1H), 4.48-4.42 (m, 1H), 4.42 (s, 1H), 4.39-4.31 (m, 1H), 1.34 (t, J= 7.1 Hz, 3H).

Example 31

Ethyl 2-[1-(3-tert-butoxy-3-oxopropyl)-1H-indol-3-yl]-3,3,3-trifluoro-2-hydroxypropanoate

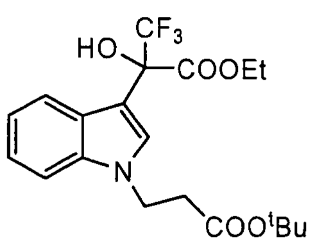

The title compound was obtained from tert-butyl 3-(1H-indol-1-yl)propanoate as a starting compound in a similar manner to the preparation of the compound of Example 2 (Yield: 572 mg).
MS (ESI+) 416, 2.37($M^{+}$+1, detection time)

Example 32

Ethyl 2-[1-(2-tert-butoxy-2-oxoethyl)-1H-indol-3-yl]-3,3,3-trifluoro-2-hydroxypropanoate

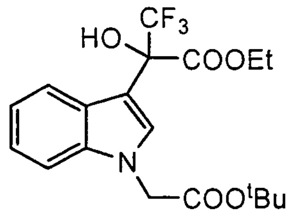

The title compound was obtained from tert-butyl 1H-indol-1-ylacetate as a starting compound in a similar manner to the preparation of the compound of Example 2 (Yield: 602 mg).
MS (ESI+) 402, 2.33 ($M^{+}$+1, detection time)

Example 33

{3-[1-(Ethoxycarbonyl)-2,2,2-trifluoro-1-hydroxyethyl]-1H-indol-1-yl}acetic acid

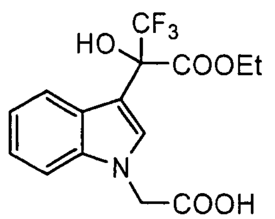

To the compound of Example 32 (602 mg) was added a solution of 4N hydrochloric acid in 1,4-dioxane (20 ml), and the mixture was stirred at 25°C for 20 hours. The reaction solution was concentrated under reduced pressure to give the title compound (508 mg).
MS (ESI+) 346, 2.02($M^{+}$+1, detection time)

Example 34

3-{3-[1-(Ethoxycarbonyl)-2,2,2-trifluoro-1-hydroxyethyl]-1H-indol-1-yl}propanoic acid

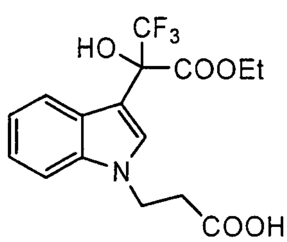

The title compound was obtained from the compound of Example 31 as a starting compound in a similar manner to the preparation of the compound of Example 33 (Yield: 500 mg).

MS (ESI+) 360, 2.08($M^+$+1, detection time)

Example 35

1-(1-Benzyl-1H-indol-3-yl)-2,2,2-trifluoro-1-phenylethanol

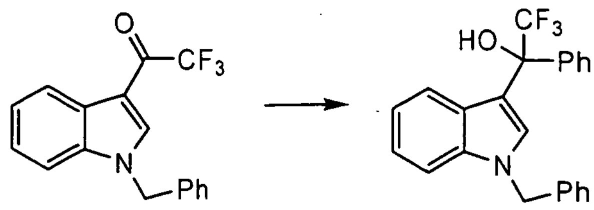

To a solution of 1-(1-benzyl-1H-indol-3-yl)-2,2,2-trifluoroethanone (91 mg) in diethyl ether (5 ml) was added dropwise a 0.94M solution of phenylmagnesium bromide in tetrahydrofuran (1.0 ml) at 0˚C. The reaction solution was stirred at the same temperature for one hour, and further stirred at 25˚C for 4 hours. To the reaction solution was added an aqueous ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (13 mg).
MS (ESI+) 382, 2.57($M^+$+1, detection time)

Reference Example 10

Ethyl 3-[1-(ethoxycarbonyl)-2,2,2-trifluoro-1-hydroxyethyl]-5-methoxy-1H-indole-2-carboxylate

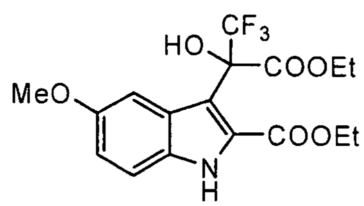

The title compound was obtained from ethyl 5-methoxy-1H-indole-2-carboxylate as a starting compound in a similar manner to the preparation of the compound of Example 2.
$^1$H-NMR (400 MHz, $CDCl_3$) δ 9.07 (bs, 1H), 8.92 (s, 1H), 7.32 (d, J= 8.9 Hz, 1H), 7.06 (d, J= 8.9 Hz, 1H), 7.04 (d, J= 8.9 Hz, 1H), 4.50 (q, J= 7.1 Hz, 2H), 4.33 (q, J= 7.1 Hz, 2H), 3.80 (s, 3H), 1.47 (t, J= 7.1 Hz, 3H), 1.22 (t, J= 7.1 Hz, 3H).
MS (ESI+) 390, 2.22($M^+$+1, detection time)

Example 37

Ethyl 3,3,3-trifluoro-2-hydroxy-2-(1-(phenylsulfonyl)-1H-indol-3-yl]propanoate

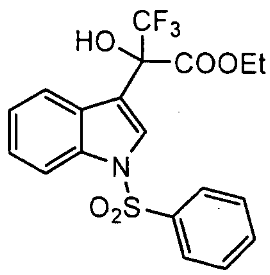

The title compound was obtained from 1-(phenylsulfonyl)indole (257 mg) as a starting compound in a similar manner to the preparation of the compound of Example 2 (Yield: 328 mg).
MS (ESI+) 428, 2.32($M^+$+1, detection time)

Reference Example 11

Ethyl 3,3,3-trifluoro-2-hydroxy-2-(1-methyl-2-phenyl-1H-indol-3-yl)propanoate

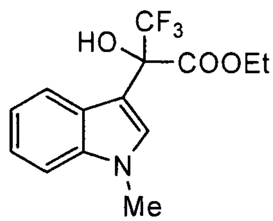

The title compound was obtained from N-methyl-indole as a starting compound in a similar manner to the preparation of the compound of Example 2.
$^1$H-NMR (400 MHz, $CDCl_3$) δ 8.80 (d, J= 8.4 Hz, 1H), 7.36-7.18 (m, 5H), 3.92-3.87 (m, 1H), 3.69 (s, 1H), 3.44-3.37 (m, 1H), 3.35 (s, 3H), 1.10 (t, J= 7.2 Hz, 3H). MS (ESI+) 378, 2.35(M++1, detection time)

Reference Example 12

Ethyl 3,3,3-trifluoro-2-hydroxy-2-(7-{[(4-methylphenyl)sulfonyl]amino}-1H-indol-3-yl)propanoate

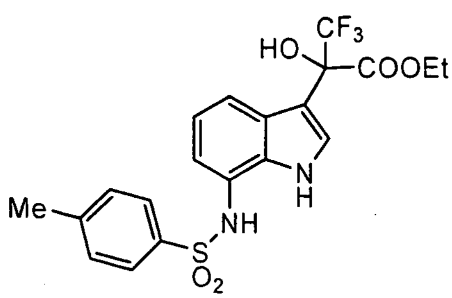

The title compound was obtained from N-1H-indol-7-yl-4-methylbenzene sulfonamide (286 mg) as a starting compound in a similar manner to the preparation of the compound of Example 2 (Yield: 280mg).
H-NMR (400 MHz, $CDCl_3$) δ 9.44 (bs, 1H), 7.80 (d, J= 7.8 Hz, 1H), 7.55 (d, J= 2.6 Hz, 1H), 7.51 (d, J=8.2 Hz, 2H), 7.20 (d, J= 8.2 Hz, 2H), 6.88 (dd, J= 7.8, 7.8 Hz, 1H), 6.50 (bs, 1H), 6.37 (d, J= 7.8 Hz, 1H), 4.50-4.36 (m, 3H), 2.38 (s, 3H),

1.36 (t, J= 7.1 Hz, 3H).

Reference Example 13

Ethyl 3,3,3-trifluoro-2-hydroxy-2-{7-[(methylsulfonyl)amino]-1H-indol-3-yl}propanoate

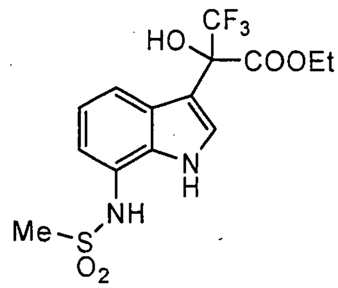

The title compound was obtained from N-1H-indol-7-ylmethane-sulfonamide (210 mg) as a starting compound in a similar manner to the preparation of the compound of Example 2 (Yield: 198 mg).
H-NMR (400 MHz, $CDCl_3$) δ 9.45 (bs, 1H), 7.94 (d, J= 7.8 Hz, 1H), 7.53 (d, J= 2.7 Hz, 1H), 7.11 (dd, J= 7.79, 7.79 Hz, 1H), 7.00 (d, J= 7.8 Hz, 1H), 6.91 (s, 1H), 4.50-4.43 (m, 1H), 4.47 (s, 3H), 4.41-4.35 (m, 1H), 3.01 (s, 3H), 1.26 (t, J=7.2 Hz, 3H).

Example 41

Ethyl 2-(1-ethyl-1H-indol-3-yl)-3,3,3-trifluoro-2-hydroxypropanoate

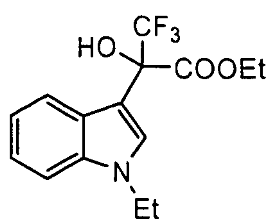

The title compound was obtained from N-ethyl-indole (184 mg) as a starting compound in a similar manner to the preparation of the compound of Example 2 (Yield: 216 mg).
$^1$H-NMR (400 MHz, $CDCl_3$) δ 7.88 (d, J= 8.1 Hz, 1H), 7.39 (s, 1H), 7.35 (d, J= 8.2 Hz, 1H), 7.25 (dd, J= 8.2, 8.0 Hz, 1H), 7.14 (dd, J= 8.1, 8.0 Hz, 1H), 4.46-4.44 (m, 1H), 4.39 (s, 1H), 4.39-4.32 (m, 1H), 4.20-4.14 (m, 2H), 1.48 (t, J= 7.3 Hz, 3H), 1.35 (t, J= 7.2 Hz, 3H),

Reference Example 14

Ethyl 3,3,3-trifluoro-2-hydroxy-2-(1-methyl-7-nitro-1H-indol-3-yl)propanoate

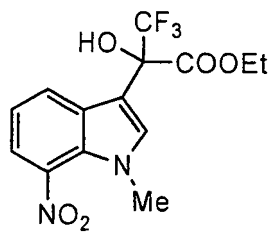

The title compound was obtained from 1-methyl-7-nitro-1 H-indole as a starting compound in a similar manner to the preparation of the compound of Example 2 (Yield: 182 mg).
$^1$H-NMR (400 MHz, $CDCl_3$) δ 8.21 (d, J= 8.0 Hz, 1H), 7.81 (d, J= 8.0, 0.9 Hz, 1H), 7.42 (s, 1H), 7.18 (dd, J= 8.0, 8.0 Hz, 1H), 4.51-4.43 (m, 1H), 4.50 (s, 1H), 4.41-4.36 (m, 1H), 3.84 (s, 3H), 1.36 (t, J= 7.1 Hz, 3H).

Reference Example 15

Ethyl 3,3,3-trifluoro-2-hydroxy-2-(1-methyl-7-{[(4-methylphenyl)sulfonyl]amino}-1H-indol-3-yl)propanoate

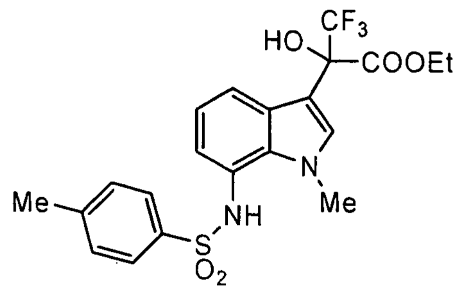

The title compound was obtained from 4-methyl-N-(1-methyl-1H-indol-7-yl)benzenesulfonamide (300 mg) as a starting compound in a similar manner to the preparation of the compound of Example 2 (Yield: 320 mg).
H-NMR (400 MHz, $CDCl_3$) δ 7.84 (d, J= 7.8 Hz, 1H), 7.57 (d, J= 8.3 Hz, 2H), 7.34-7.26 (m, 3H), 6.81 (dd, J= 7.8, 7.8 Hz, 1H), 6.18 (d, J= 7.8 Hz, 1H), 6.11 (bs, 1H), 4.50-4.44 (m, 1H), 4.40-4.32 (m, 1H), 4.38 (s, 1H), 4.19 (s, 3H), 2.46 (s, 3H), 1.36 (t, J= 7.2 Hz, 3H).

Reference Example 16

Ethyl 3,3,3-trifluoro-2-hydroxy-2-{1-methyl-7-[(methylsulfonyl)amino]-1H-indol-3-yl}propanoate

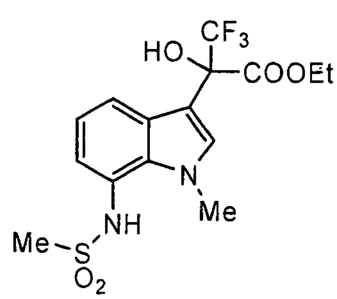

The title compound was obtained from N-(1-methyl-1H-indol-7-yl)-methanesulfonamide (224 mg) as a starting compound in a similar manner to the preparation of the compound of Example 2 (Yield: 232 mg).
H-NMR (400 MHz, $CDCl_3$) δ 7.81 (d, J= 7.7 Hz, 1H), 7.19 (s, 1H), 6.98 (dd, J= 7.7, 7.7 Hz, 1H), 6.81 (d, J= .7 Hz, 1H), 6.75 (bs, 1H), 4.72 (bs, 1H), 4.47-4.42 (m, 1H), 4.32-4.28 (m, 1H), 4.00 (s, 3H), 3.00 (s, 3H), 1.31 (t, J= 7.1 Hz, 3H).

Example 45

Ethyl 2-{1-benzyl-6-[(methylsulfonyl)amino]-1H-indol-3-yl}-3,3,3-trifluoro-2-hydroxypropanoate

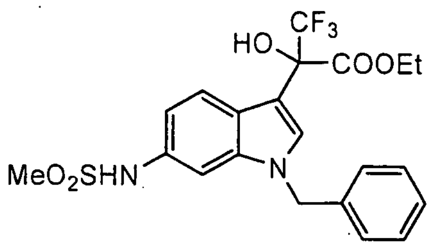

The title compound was obtained from ethyl 2-(6-amino-1-benzyl-1H-indol-3-yl)-3,3,3-trifluoro-2-hydroxypropanoate as a starting compound in a similar manner to the preparation of the compound of Example 19 (Yield: 21 mg).
H-NMR (400 MHz, $CDCl_3$) δ 7.89 (d, J= 8.6 Hz, 1H), 7.41 (s, 1H), 7.32-7.26 (m, 4H), 7.12 (d, J= 7.8 Hz, 2H), 6.92 (d, J= 8.6 Hz, 1H), 6.75 (bs, 1H), 5.26 (s, 2H), 4.49-4.32 (m, 3H), 2.87 (s, 3H), 1.30 (t, J= 6.5 Hz, 3H).
MS (ESI+) 471, 2.25($M^+$+1, detection time)

Example 46

Ethyl 2-(1-benzyl-6-{[(4-methylphenyl)sulfonylJamino}-1H-indol-3-yl)-3,3,3-trifluoro-2-hydroxypropanoate

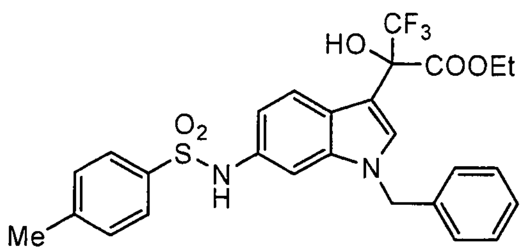

The title compound was obtained from ethyl 2-(6-amino-1-benzyl-1H-indol-3-yl)-3,3,3-trifluoro-2-hydroxypropanoate as a starting compound in a similar manner to the preparation of the compound of Example 19 (Yield: 32 mg).
H-NMR (400 MHz, $CDCl_3$) δ 7.70 (d, J=8.6 Hz, 1H), 7.51 (d, J= 8.2 Hz, 2H), 7.35 (s, 1H), 7.30-7.29 (m, 3H), 7.21 (s, 1H), 7.09-7.05 (m, 4H), 6.95 (bs, 1H), 6.66 (d, J= 8.6 Hz, 1H), 5.20 (s, 2H), 4.43-4.30 (m, 3H), 2.32 (s, 3H), 1.27 (t, J= 7.1 Hz, 3H).
MS (ESI+) 547, 2.39($M^+$+1, detection time)

Example 47

Ethyl 3,3,3-trifluoro-2-hydroxy-2-(1-propyl-1H-indol-3-yl)propanoate
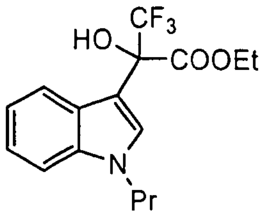

The title compound was obtained from N-propyl-indole as a starting compound in a similar manner to the preparation of the compound of Example 2.

$^1$H-NMR (400 MHz, $CDCl_3$) δ 7.89 (d, J= 8.1 Hz, 1H), 7.37 (s, 1H), 7.34 (d, J= 8.3 Hz, 1H), 7.23 (dd, J= 8.5, 7.2 Hz, 1H), 7.15 (t, J= 8.1, 7.2 Hz, 2H), 4.48-4.44 (m, 1H), 4.38-4.43 (m, 1H), 4.37 (s, 1H), 4.08 (t, J= 7.1 Hz, 2H), 1.90-1.80 (m, 2H), 1.34 (t, J= 7.1Hz, 3H), 0.94 (t, J= 7.4 Hz, 3H).

Reference Example 17

Ethyl 3,3,3-trifluoro-2-hydroxy-2-(1-methyl-6-nitro-1H-indol-3-yl)propanoate

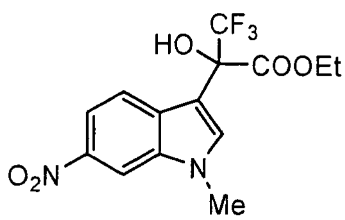

The title compound was obtained from 1-methyl-6-nitro-1H-indole as a starting compound in a similar manner to the preparation of the compound of Example 2.

$^1$H-NMR (400 MHz, $CDCl_3$) δ 8.31 (d, J= 1.8 Hz, 1H), 8.06-8.00 (m, 2H), 7.64 (s, 1H), 4.52-4.37 (m, 2H), 4.47 (s, 1H), 3.92 (s, 3H), 1.37 (t, J= 7.1 Hz, 3H).

Example 49

Ethyl 3,3,3-trifluoro-2-hydroxy-2-(1-isopropyl-1H-indol-3-yl)propanoate

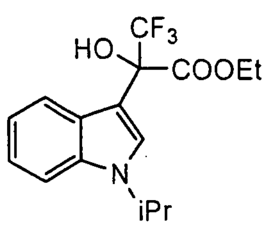

The title compound was obtained from N-isopropylindole as a starting compound in a similar manner to the preparation of the compound of Example 2.

$^1$H-NMR (400 MHz, $CDCl_3$) δ 7.90 (d, J= 7.9 Hz, 1H), 7.48 (s, 1H), 7.38 (d, J= 8.0 Hz, 1H), 7.23 (dd, J= 8.0, 7.3 Hz, 1H), 7.14 (dd, J= 7.9, 7.3 Hz, 1H), 4.69-4.63 (m, 1H), 4.48-4.42 (m, 1H), 4.40 (s, 1H), 4.40-4.33 (m, 1H), 1.54 (d, J= 6.7 Hz, 3H), 1.53 (d, J= 6.7 Hz, 3H), 1.35 (t, J= 7.1 Hz, 3H).

Example 50

Ethyl 1-benzyl-3-[1-(ethoxycarbonyl)-2,2,2-trifluoro-1-hydroxyethyl]-5-methoxy-1H-indole-2-carboxylate

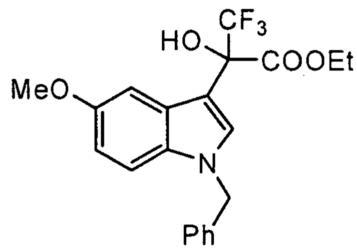

The title compound was obtained from N-benzyl-5-methoxyindole as a starting compound in a similar manner to the preparation of the compound of Example 2.
$^1$H-NMR (400 MHz, $CDCl_3$) δ 8.02 (s, 1H), 7.31-7.21 (m 3H), 7.19 (d, J= 9.1 Hz, 1H), 7.00-6.96 (m, 3H), 6.82 (s, 1H), 5.53 (s, 2H), 4.37 (q, J= 7.2Hz, 2H), 4.36-4.26 (m, 2H), 3.82 (s, 3H), 1.30 (t, J= 7.2 Hz, 3H), 1.18 (t, J= 7.2 Hz, 3H).
MS (ESI+) 480, 2.44($M^+$+1, detection time)

Reference Example 18

Ethyl 3-[1-(ethoxycarbonyl)-2,2,2-trifluoro-1-hydroxyethyl]-5-methoxy-1-methyl-1H-indole-2-carboxylate

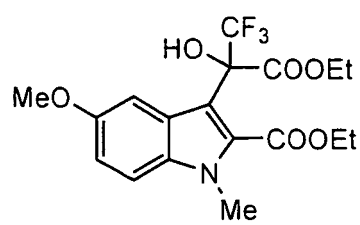

The title compound was obtained from ethyl 5-methoxy-1-methyl-1H-indole-2-carboxylate as a starting compound in a similar manner to the preparation of the compound of Example 2.
$^1$H-NMR (400 MHz, $CDCl_3$) δ 7.31-7.21 (m, 1H), 7.17 (s, 1H), 7.05 (d, J= 9.2 Hz, 1H), 7.02 (s, 1H), 4.54-4.45 (m, 2H), 4.34 (q, J= 7.2 Hz, 2H), 3.87 (s, 3H), 3.83 (s, 3H), 1.45 (t, J= 7.2 Hz, 3H), 1.27 (t, J= 7.2 Hz, 3H).
MS (ESI+) 404, 2.26($M^+$+1, detection time)

Reference Example 19

Ethyl 2-(1,2-dimethyl-1H-indol-3-yl)-3,3,3-trifluoro-2-hydroxypropanoate

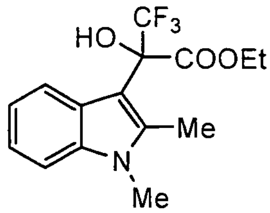

The title compound was obtained from 1,2-dimethyl-1H-indole as a starting compound in a similar manner to the preparation of the compound of Example 2.
$^1$H-NMR (400 MHz, $CDCl_3$) δ 7.79 (d, J= 7.9 Hz, 1H), 7.28 (d, J= 8.0 Hz, 1H), 7.20 (dd, J= 8.0, 7.3 Hz, 1H), 7.09 (dd, J= 7.9, 7.3 Hz, 1H), 4.44-4.34 (m, 2H), 3.82 (s, 1H), 3.65 (s, 3H), 2.53 (s, 3H), 1.34 (t, J= 7.1 Hz, 3H).
MS (ESI+) 316, 2.15(M++1, detection time)

Reference Example 20

Ethyl 3,3,3-trifluoro-2-hydroxy-2-[2-(hydroxymethyl)-1-methyl-1H-indol-3-yl]propanoate

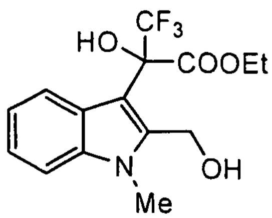

The title compound was obtained from (1-methyl-1H-indol-2-yl)methanol (1.48 mg) as a starting compound in a similar manner to the preparation of the compound of Example 2 (Yield: 790 mg).
$^1$H-NMR (400 MHz, $CDCl_3$) δ 7.84 (d, J= 8.3 Hz, 1H), 7.35 (d, J= 8.3 Hz, 1H), 7.27 (dd, J= 8.3, 8.3 Hz, 1H), 7.16 (dd, J= 8.3, 8.3 Hz, 1H), 5.02 (d, J=, 13.3Hz, 1H), 4.94 (d, J= 13.3 Hz, 1H), 4.60 (s, 1H), 4.46-4.35 (m, 2H), 3.82 (sw, 3H), 1.35 (t, J= 7.2 Hz, 3H).

Example 54

3,3,3-Trifluoro-2-hydroxy-2-[2-(hydroxymethyl)-1-methyl-1H-indol-3-yl]propanoic acid

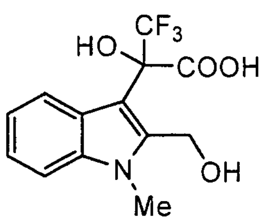

To a solution of the compound of Reference Example 20 (100 mg) in ethanol (5 ml) was added sodium hydride (55 %, 52 mg) at 25˚C, and the mixture was stirred at the same temperature for 3 hours. To the reaction solution was added 1N hydrochloric acid, and the mixture was extracted with ethyl acetate. The organic layer was washed with a saturated saline solution, dried over sodium sulfate, and filtered. The filtrate was concentrated under reduced pressure, and the

obtained residue was purified by silica gel column chromatography to give the title compound (23 mg).

Reference Example 21

Ethyl 3-[1-(ethoxycarbonyl)-2,2,2-trifluoro-1-hydroxyethyl]-1-methyl-1H-indole-2-carboxylate

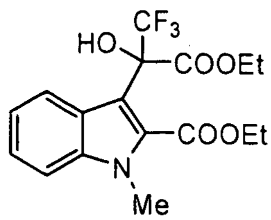

The title compound was obtained from ethyl 1-methyl-1H-indol-2-carboxylate (1.75 g) as a starting compound in a similar manner to the preparation of the compound of Example 2 (Yield: 2.59 g).
MS (ESI+) 374, 2.33($M^+$+1, detection time)

Reference Example 22

Ethyl 3,3,3-trifluoro-2-hydroxy-2-(1-methyl-6-{[(4-methylphenyl)sulfonyl]amino}-1H-indol-3-yl)propanoate

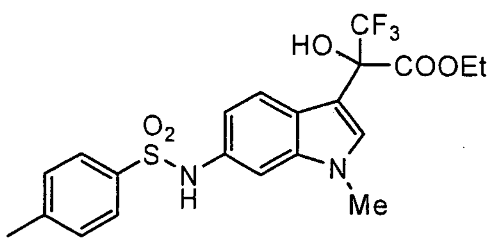

The title compound was obtained from 4-methyl-N-(1-methyl-1H-indol-6-yl)benzenesulfonamide as a starting compound in a similar manner to the preparation of the compound of Example 2 (Yield: 120 mg).
$^1$H-NMR (400 MHz, $CDCl_3$) δ 7.67-7.63 (m, 3H), 7.29 (s, 1H), 7.24 (s, 1H), 7.17 (d, J= 8.1 Hz, 2H), 7.01-6.91 (m, 1H), 6.67 (d, J= 8.5 Hz, 1H), 4.47-4.28 (m, 2H), 4.36 (s, 1H), 3.70 (s, 1H), 2.35 (s, 1H), 1.31 (t, J= 7.1 Hz, 3H).

Reference Example 23

Ethyl 3,3,3-trifluoro-2-hydroxy-2-{1-methyl-6-[(methylsulfonyl)amino]-1H-indol-3-yl}propanoate

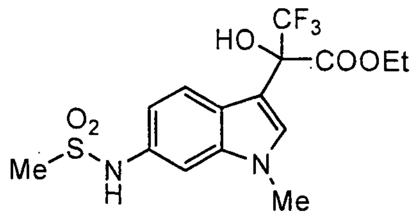

The title compound was obtained from N-(1-methyl-1H-indol-6-yl)-methanesulfonamide as a starting compound in a similar manner to the preparation of the compound of Example 2 (Yield: 85 mg).
MS (ESI+) 395, 1.99($M^{+}$+1, detection time)

Reference Example 24

Ethyl 2-[6-(benzyloxy)-1-methyl-1H-indol-3-yl]-3,3,3-trifluoro-2-hydroxy-propanoate

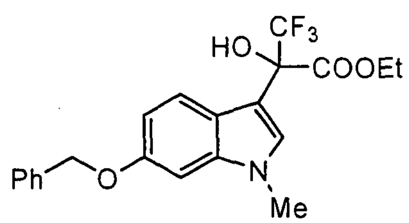

The title compound was obtained from 6-(benzyloxy)-1-methyl-indole (1.25 g) as a starting compound in a similar manner to the preparation of the compound of Example 2 (Yield: 1.13 g).
MS (ESI+) 408, 2.47($M^{+}$+1, detection time)

Reference Example 25

Ethyl 3,3,3-trifluoro-2-hydroxy-2-(6-hydroxy-1-methyl-1H-indol-3-yl)propanoate

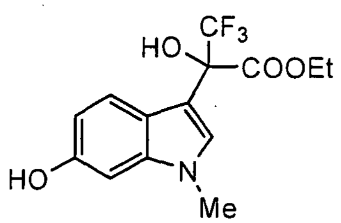

The title compound was obtained from the compound of Reference Example 24 (200 mg) as a starting compound in a similar manner to the preparation of the compound of Example 28 (Yield: 85 mg).
$^{1}$H-NMR (400 MHz, $CDCl_3$) δ 7.63 (d, J= 8.7 HZ, 1H), 7.13 (s, 1H), 6.67 (d, J= 2.2 HZ, 1H), 6.63 (dd, J= 8.7, 2.2 Hz, 1H), 4.76 (bs, 1H), 4.40-4.34(m, 1H), 4.31-4.25 (m, 1H), 3.62 (s, 3H), 1.27 (t, J= 7.2 Hz, 3H).
MS (ESI+) 300, 1.92($M^{+}$+1, detection time)

Reference Example 26

Ethyl 3,3,3-trifluoro-2-hydroxy-2-(2-{[(4-methoxybenzyl)amino]methyl}-1-methyl-1H-indol-3-yl)propanoate

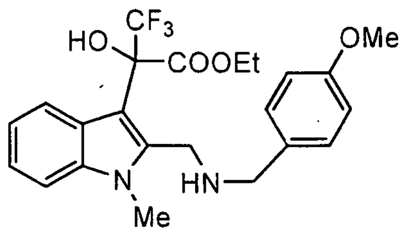

The title compound was obtained from 1-(4-methoxyphenyl)-N-[(1-methyl-1H-indol-2-yl)methyl]methanamine (673 mg) as a starting compound in a similar manner to the preparation of the compound of Example 2 (Yield: 874 mg) .
MS (ESI+) 451, 2.34($M^{+}$+1, detection time)

Example 61

2-(1-Benzyl-6-nitro-1H-indol-3-yl)-3,3,3-trifluoro-2-hydroxypropanoic acid [0206].

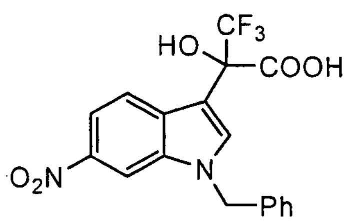

The title compound was obtained from the compound of Example 2 (422 mg) as a starting compound in a similar manner to the preparation of the compound of Example 4 (Yield: 280 mg).
MS (ESI+) 395, 1.78($M^{+}$+1, detection time)

Example 62

Benzyl 1-benzyl-3-[1-(ethoxycarbonyl)-2,2,2-trifluoro-1-hydroxyethyl]-1H-indole-6-carboxylate

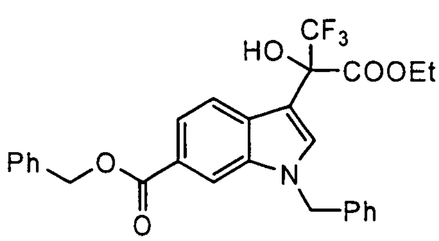

The title compound was obtained from benzyl 1-benzyl-1H-indole-6-carboxylate (775 mg) as a starting compound in a similar manner to the preparation of the compound of Example 2 (Yield: 820 mg).
MS (ESI+) 512, 3.03($M^{+}$+1, detection time)

Example 63

1-Benzyl-3-[1-(ethoxycarbonyl)-2,2,2-trifluoro-1-hydroxyethyl]-1H-indole-6-carboxylic acid

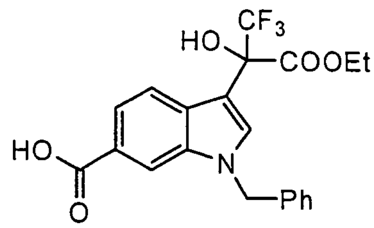

The title compound was obtained from the compound of Example 62 (600 mg) as a starting compound in a similar manner to the preparation of the compound of Example 28 (Yield: 480 mg).
MS (ESI+) 422, 2.57($M^+$+1, detection time)

Example 64

(4E)-2-(1-Benzyl-6-nitro-1H-indol-3-yl)-1,1,1-trifluoro-5-pyridin-3-ylpent-4-en-2-ol

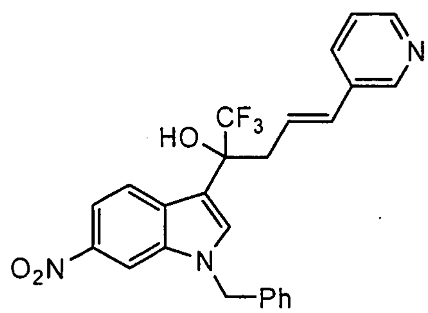

The title compound was obtained in a similar manner to the preparation of the compound of Example 9 (Yield: 48 mg).
$^1$H-NMR (400 MHz, $CDCl_3$) δ 8.43-8.33 (m, 2H), 8.29 (d, J= 2.0 Hz, 1H), 8.05 (dd, J= 9.0, 2.0 Hz, 1H), 7.99 (d, J= 9.0 Hz, 1H), 7.53 (s, 1H), 7.50-7.45 (m, 1H), 7.32-7.23 (m, 3H), 7.19-7.12 (m, 1H), 7.12-7.03 (m, 2H), 6.43 (d, J= 15.9 Hz, 1H), 6.12-6.01 (m, 1H), 5.41 (s, 2H), 3.56 (bs, 1H), 3.25 (dd, J= 14.8, 6.8 Hz, 1H), 3.09 (dd, J= 14.8, 6.8 Hz, 1H).
MS (ESI+) 468, 2.11($M^+$+1, detection time)

Example 65

2-(1-Benzyl-6-nitro-1H-indol-3-yl)-1,1,1-trifluoro-3-morpholin-4-ylpropan-2-ol

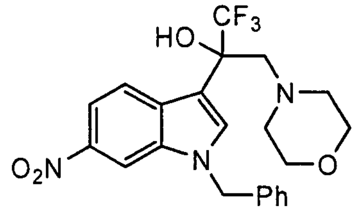

The title compound was obtained in a similar manner to the preparation of the compound of Example 1 (Yield: 48 mg). H-NMR (400 MHz, $CDCl_3$) δ 8.21 (d, J= 2.0 Hz, 1H), 7.97 (dd, J= 9.0, 2.0 Hz, 1H), 7.85 (d, J= 9.0 Hz, 1H), 7.49 (s, 1H), 7.28-7.25 (m, 3H), 7.04-7.01 (m, 1H), 5.32 (s, 2H), 3.59-3.56 (m, 4H), 3.16 (d, J= 13.7 Hz, 1H), 3.06 (d, J= 13.7 Hz, 1H), 2.50-2.46 (m, 4H).
MS (ESI+) 450, 2.13($M^+$+1, detection time)

Example 66

Ethyl 2-[1-benzyl-6-(morpholin-4-ylsulfonyl)-1H-indol-3-yl]-3,3,3-trifluoro-2-hydroxypropanoate

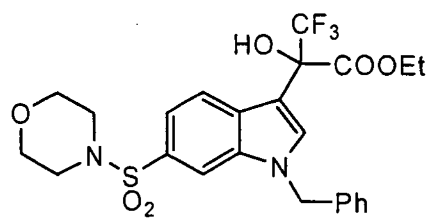

The title compound was obtained from 1-benzyl-6-(morpholin-4-ylsulfonyl)-1H-indole as a starting compound in a similar manner to the preparation of the compound of Example 2 (Yield: 104 mg).
MS (ESI+) 527, 2.28($M^+$+ 1, detection time)

Example 67

{1-[2-(1-Benzyl-6-nitro-1H-indol-3-yl)-3,3,3-trifluoro-2-hydroxypropyl]piperidin-4-yl}(phenyl)methanone

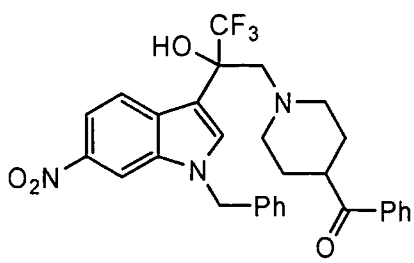

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 7.0, 2.0 Hz, 1H), 7.89 (dd, J= 7.0 Hz, 1H), 7.89 (d, J= 8.2 Hz, 2H), 7.60 (s, 1H), 7.58-7.54 (m, 1H), 7.48-7.46 (m, 2H), 7.32-7.29 (m, 3H), 7.11-7.09 (m, 2H), 5.69 (bs, 1H), 5.39 (s, 2H), 3.26-3.16 (m, 1H), 3,24 (d, J= 13.6 Hz, 1H), 3.18 (d, J= 13.6 Hz, 1H), 3.02-2.99 (m, 1H), 2.68-2.61 (m, 2H), 2.33-2.28 (m, 1H), 1.89-1.70 (m, 4H). MS (ESI+) 524, 1.71 ($M^+$+1, detection time)

Example 68

3-(1-Benzyl-6-nitro-1H-indol-3-yl)-4,4,4-trifluoro-3-hydroxy-1-(2-methylphenyl)-butan-1-one

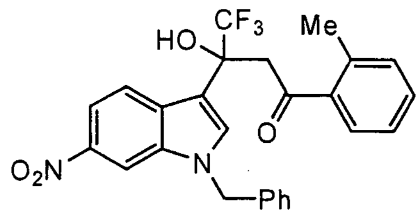

The title compound was obtained in a similar manner to the preparation of the compound of Example 14 (Yield: 455 mg). $^1$H-NMR (400 MHz, $CDCl_3$) δ 8.24 (s, 1H), 8.00 (d, J= 1.8 Hz, 2H), 7.61 (d, J= 8.0 Hz, 1H), 7.46-7.39 (m, 2H), 7.34-7.28 (m, 4H), 7.21 (d, J= 8.0 Hz, 1H), 7.08-7.00 (m, 2H), 6.07 (s, 1H), 5.37 (d, J= 15.8 Hz, 1H), 5.31 (d, J= 15.8 Hz, 1H), 3.93 (d, J= 16.8 Hz, 1H), 3.66 (d, J= 16.8 Hz, 1H), 2.24 (s, 3H).
MS (ESI+) 483, 2.54($M^+$+1, detection time)

Example 69

3-(1-Benzyl-6-nitro-1H-indol-3-yl)-4,4,4-trifluoro-3-hydroxy-1-(2-methoxyphenyl)butan-1-one

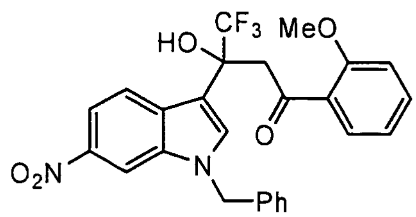

The title compound was obtained in a similar manner to the preparation of the compound of Example 14.
MS (ESI+) 499, 2.95($M^+$+1, detection time)

Example 70

2-(1-Benzyl-6-nitro-1H-indol-3-yl)-1,1,1-trifluoro-4-piperidin-1-ylbutan-2-ol

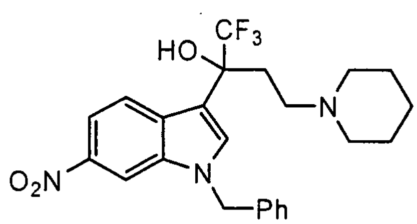

The title compound was obtained in a similar manner to the preparation of the compound of Example 11 (Yield: 15 mg). $^1$H-NMR (400 MHz, $CDCl_3$) δ 8.29 (d, J= 2.0 Hz, 1H), 8.01 (dd, J= 9.0, 2.0 Hz, 1H), 7.85 (d, J= 9.0 Hz, 1H), 7.61 (s, 1H), 7.39-7.27 (m, 3H), 7.13 (dd, J= 7.8, 2.0 Hz, 2H), 5.44 (d, J= 15.8 Hz, 1H), 5.37 (d, J= 15.8 Hz, 1H), 2.68-2.52 (m, 2H), 2.52-2.32 (m, 3H), 2.32-2.05 (m, 3H), 1.65-1.46 (m, 5H), 1.46-1.32 (m, 1H). MS (ESI+) 462, 1.95($M^+$+1, detection time)

Example 71

{1-[2-(1-Benzyl-6-nitro-1H-indol-3-yl)-3,3,3-trifluoro-2-hydroxypropyl]piperidin-4-yl}(4-fluorophenyl)methanone

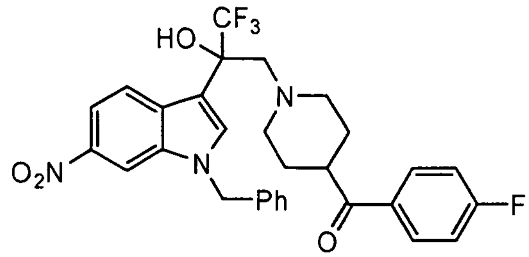

The title compound was obtained in a similar manner to the preparation of the compound of Example 1.
$^{1}$H-NMR (400 MHz, $CDCl_3$) δ 8.26 (d, J= 2.0 Hz, 1H), 8.02 (d, J= 9.0, 2.0 Hz, 1H), 7.95-7.90 (m, 3H), 7.60 (s, 1H), 7.32-7.30 (m, 3H), 7.15-7.09 (m, 4H), 5.39 (s, 2H), 3.25 (d, J= 13.6 Hz, 1H), 3.25-3.16 (m, 1H), 3.17 (d, J= 13.6 Hz, 1H), 3.02-2.99 (m, 1H), 2.65-2.62 (m, 2H), 2.32-2.23 (m, 1H), 1.87-1.68 (m, 4H).
MS (ESI+) 570, 2.17($M^{+}$+1, detection time)

Example 72

{1-[2-(1-Benzyl-6-nitro-1H-indol-3-yl)-3,3,3-trifluoro-2-hydroxypropyl]piperidin-4-yl}(4-chlorophenyl)methanone

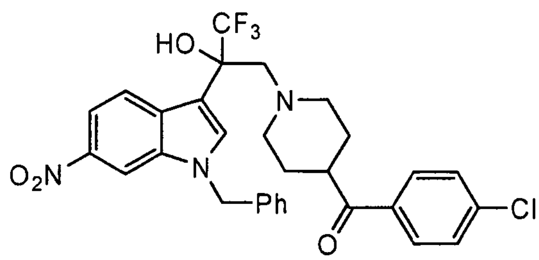

The title compound was obtained in a similar manner to the preparation of the compound of Example 1.
$^{1}$H-NMR (400 MHz, $CDCl_3$) δ 8.26 (d, J= 2.0 Hz, 1H), 8.03 (dd, J= 9.0, 2.0 Hz, 1H), 7.89 (d, J= 9.0 Hz, 1H), 7.83 (d, J= 8.6 Hz, 2H), 7.60 (s, 1H), 7.43 (d, J= 8.6 Hz, 2H), 7.32-7.30 (m, 3H), 7.10 (d, J= 7.8 Hz, 2H), 5.39 (s, 2H), 3.25 (d, J= 13.6 Hz, 1H), 3.25-3.15 (m, 1H), 3.17 (d, J= 13.6 Hz, 1H), 3.02-2.99 (m, 1H), 2.65-2.62 (m, 2H), 2.32-2.23 (m, 1H), 1.87-1.68 (m, 4H).
MS (ESI+) 586, 2.25($M^{+}$+1, detection time)

Example 73

{1-[2-(1-Benzyl-6-nitro-1H-indol-3-yl)-3,3,3-trifluoro-2-hydroxypropyl]piperidin-4-yl}(4-methoxyphenyl)methanone

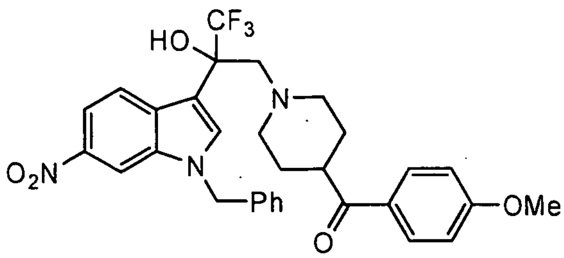

The title compound was obtained in a similar manner to the preparation of the compound of Example 1.
$^1$H-NMR (400 MHz, $CDCl_3$) δ 8.26 (d, J= 2.0 Hz, 1H), 8.03 (dd, J= 9.0, 2.0 Hz, 1H), 7.89 (d, J= 9.0 Hz, 3H), 7.61 (s, 1H), 7.32-7.29 (m, 3H), 7.10 (d, J= 7.9 Hz, 2H), 6.93 (d, J= 9.0 Hz, 2H), 5.39 (s, 2H), 3.86 (s, 3H), 3.24 (d, J= 13.6 Hz, 1H), 3.26-3.16 (m, 1H), 3.18 (d, J= 13.6 Hz, 1H), 3.02-2.99 (m, 1H), 2.65-2.61 (m, 2H), 2.31-2.27 (m, 1H), 1.88-1.64 (m, 4H).
MS (ESI+) 582, 2.13($M^+$+1, detection time)

.

Example 74

2-(1-Benzyl-6-nitro-1H-indol-3-yl)-3-(4-benzylpiperidin-1-yl)-1,1,1-trifluoropropan-2-ol

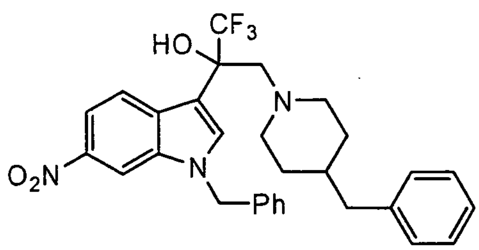

The title compound was obtained in a similar manner to the preparation of the compound of Example 1.
MS (ESI+) 538, 2.16($M^+$+1, detection time)

Example 75

2-(1-Benzyl-6-nitro-1H-indol-3-yl)-1,1,1-trifluoro-3-(4-phenylpiperazin-1-yl)propan-2-ol

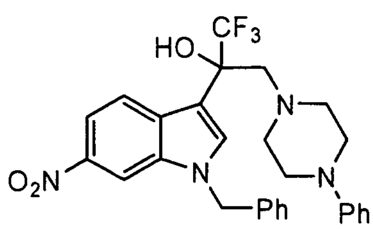

The title compound was obtained in a similar manner to the preparation of the compound of Example 1.
H-NMR (400 MHz, $CDCl_3$) δ 8.28 (d, J= 2.0 Hz, 1H), 8.04 (dd, J= 9.0, 2.0 Hz, 1H), 7.94 (d, J= 9.0 Hz, 1H), 7.58 (s, 1H), 7.34-7.23 (m, 5H), 7.12-7.10 (m, 2H), 6.88-6.86 (m, 3H), 5.40 (s, 2H), 3.31 (d, J= 13.7 Hz, 1H), 3.19 (d, J= 13.7 Hz, 1H), 3.13-3.09 (m, 4H), 2.74-2.70 (m, 4H).
MS (ESI+) 525, 2.40($M^+$+1, detection time)

Example 76

4-Benzyl-1-[2-(1-benzyl-6-nitro-1H-indol-3-yl)-3,3,3-trifluoro-2-hydroxypropyl]-piperidin-4-ol

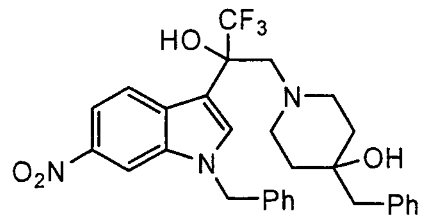

The title compound was obtained in a similar manner to the preparation of the compound of Example 1.
MS (ESI+) 554, 2.04($M^{+}$+1, detection time)

Example 77

2-(1-Benzyl-6-nitro-1H-indol-3-yl)-1,1,1-trifluoro-3-[4-(4-fluorophenoxy)-piperidin-1-yl]propan-2-ol

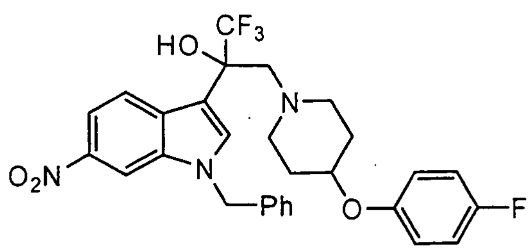

The title compound was obtained in a similar manner to the preparation of the compound of Example 1.
MS (ESI+) 558, 2.23($M^{+}$+1, detection time)

Example 78

1-[2-(1-Benzyl-6-nitro-1H-indol-3-yl)-3,3,3-trifluoro-2-hydroxypropyl]piperidin-4-ol

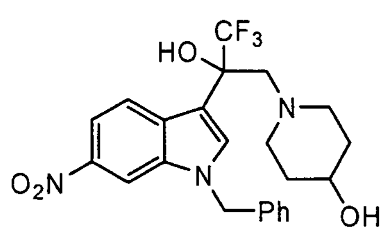

The title compound was obtained in a similar manner to the preparation of the compound of Example 1.
H-NMR (400 MHz, $CDCl_3$) δ 8.26 (d, J= 2.0 Hz, 1H), 8.02 (dd, J= 9.0, 2.0 Hz, 1H), 7.90 (d, J= 9.0 Hz, 1H), 7.58 (s, 1H), 7.35-7.28 (m, 3H), 7.09 (d, J= 7.7 Hz, 2H), 5.39 (s, 2H), 3.74-3.71 (m, 1H), 3.23 (d, J= 13.6 Hz, 1H), 3.11 (d, J= 13.6 Hz, 1H), 2.81-2.80 (m, 1H), 2.67-2.64 (m, 1H), 2.48-2.45 (m, 1H), 2.35-2.33 (m, 1H), 1.87-1.76 (m, 2H), 1.58-1.50 (m, 2H), 1.30 (bs, 1H).

MS (ESI+) 464, 1.88($M^+$+1, detection time)

Example 79

2-(1-Benzyl-6-nitro-1H-indol-3-yl)-1,1,1-trifluoro-3-[4-(hydroxymethyl)piperidin-1-yl]propan-2-ol

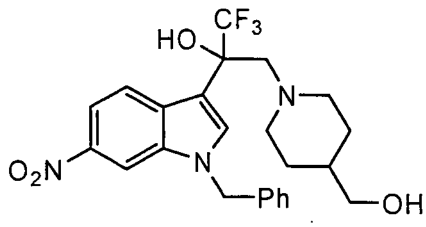

The title compound was obtained in a similar manner to the preparation of the compound of Example 1.
H-NMR (400 MHz, $CDCl_3$) δ 8.26 (d, J= 2.0 Hz, 1H), 8.02 (dd, J= 9.0, 2.0 Hz, 1H), 7.90 (d, J= 9.0 Hz, 1H), 7.57 (s, 1H), 7.35-7.30 (m, 3H), 7.09 (d, J= 7.8 Hz, 2H), 5.39 (s, 2H), 3.48 (d, J= 6.2 Hz, 2H), 3.23 (d, J= 13.6 Hz, 1H), 3.10 (d, J= 13.6 Hz, 1H), 2.97-2.94 (m, 1H), 2.57-2.49 (m, 1H), 2.17-2.16 (m, 1H), 1.77-1.74 (m, 1H), 1.58-1.16 (m, 5H).
MS (ESI+) 478, 1.85($M^+$+1, detection time)

Example 80

2-(1-Benzyl-6-nitro-1H-indol-3-yl)-1,1,1-trifluoro-3-(4-methylpiperidin-1-yl)-propan-2-ol

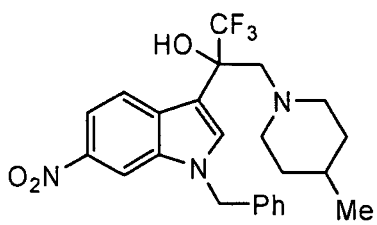

The title compound was obtained in a similar manner to the preparation of the compound of Example 1.
H-NMR (400 MHz, $CDCl_3$) δ 8.25 (d, J= 2.0 Hz, 1H), 8.02 (dd, J= 9.0, 2.0 Hz, 1H), 7.90 (d, J= 9.0 Hz, 1H), 7.,58 (s, 1H), 7.35-7.30 (m, 3H), 7.09 (d, J= 7.7 Hz, 1H), 5.39 (s, 2H), 3.20 (d, J= 13.5 Hz, 1H), 3.09 (d, J= 13.5 Hz, 1H), 2.89-2.86 (m, 1H), 2.52-2.46 (m, 2H), 2.16-2.13 (m, 1H), 1.65-1.62 (m, 1H), 1.46-1.10 (m, 5H), 0.89 (d, J= 17.7 Hz, 3H).
MS (ESI+) 462, 1.96($M^+$+1, detection time)

Example 81

Ethyl 1-[2-(1-benzyl-6-nitro-1H-indol-3-yl)-3,3,3-trifluoro-2-hydroxypropyl]-piperidine-4-carboxylate

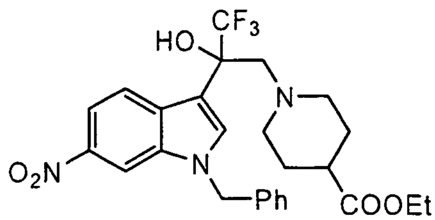

The title compound was obtained in a similar manner to the preparation of the compound of Example 1.
H-NMR (400 MHz, $CDCl_3$) δ 8.26 (d, J= 2.0 Hz, 1H), 8.03 (dd, J= 9.0, 2.0 Hz, 1H), 7.88 (d, J=9.0 Hz, 1H), 7.57 (s, 1H), 7.34-7.32 (m, 3H), 7.09 (d, J= 7.9 Hz, 1H), 5.40 (s, 2H), 4.12 (q, J= 7.1 Hz, 2H), 3.21 (d, J= 13.6 Hz, 1H), 3.11 (d, J=13.6 Hz, 1H), 2.91-2.88 (m, 1H), 2.59-2.50 (m, 2H), 2.28-2.23 (m, 2H), 1.92-1.89 (m, 1H), 1.79-1.65 (m, 3H), 1.24 (t, J= 7.1 Hz, 3H).

Example 82

1-[2-(1-Benzyl-6-nitro-1H-indol-3-yl)-3,3,3-trifluoro-2-hydroxypropyl]piperidine-4-carboxylic acid

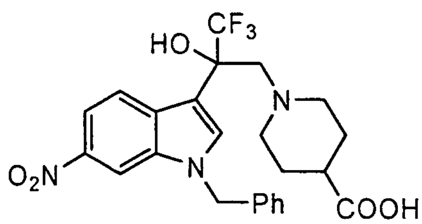

The title compound was obtained from the compound of Example 81 (486 mg) as a starting compound in a similar manner to the preparation of the compound of Example 4 (Yield: 439 mg).
MS (ESI+) 492, 1.99($M^+$+1, detection time)

Example 83

Ethyl 2-[1-benzyl-6-(methylsulfonyl)-1H-indol-3-yl]-3,3,3-trifluoro-2-hydroxypropanoate

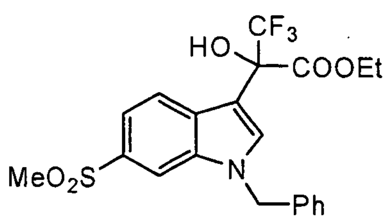

The title compound was obtained in a similar manner to the preparation of the compound of Example 2.
MS (ESI+) 456, 2.69($M^+$+1, detection time)

Example 84

2-(1-Benzyl-6-nitro-1H-indol-3-yl)-1,1,1-trifluoro-3-(4-phenylpiperidin-1-yl)-propan-2-ol

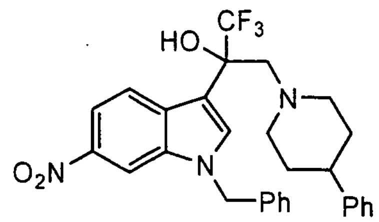

The title compound was obtained in a similar manner to the preparation of the compound of Example 1. MS (ESI+) 524, 2.16($M^{+}$+1, detection time)

Example 85

2-(1-Benzyl-6-nitro-1H-indol-3-yl)-1,1,1-trifluoro-3-(4-pyrrolidin-1-ylpiperidin-1-yl)propan-2-ol

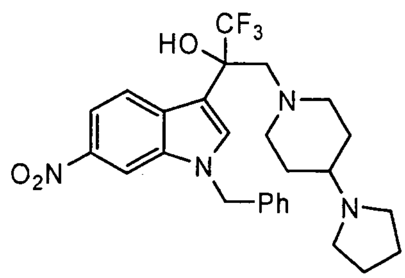

The title compound was obtained in a similar manner to the preparation of the compound of Example 1. MS (ESI+) 517, 1.90($M^{+}$+1, detection time)

Example 86

1-{1-[2-(1-Benzyl-6-nitro-1H-indol-3-yl)-3,3,3-trifluoro-2-hydroxypropyl]-4-phenylpiperidin-4-yl}ethanone

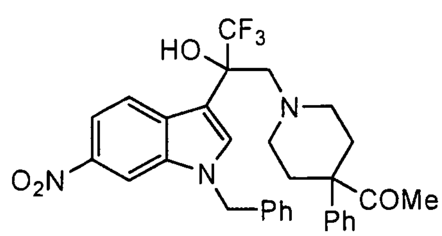

The title compound was obtained in a similar manner to the preparation of the compound of Example 1. MS (ESI+) 566, 2.23($M^{+}$+1, detection time)

Example 87

{1-[2-(1-Benzyl-1H-indol-3-yl)-3,3,3-trifluoro-2-hydroxypropyl]piperidin-4-yl}(phenyl)methanone

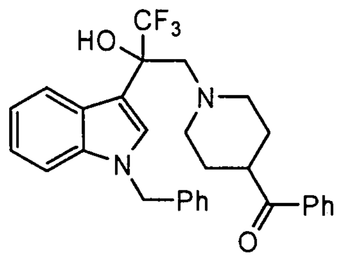

The title compound was obtained in a similar manner to the preparation of the compound of Example 1.
MS (ESI+) 507, 2.39($M^+$+1, detection time)

Example 88

Ethyl 2-(1-benzyl-6-cyano-1H-indol-3-yl)-3,3,3-trifluoro-2-hydroxypropanoate
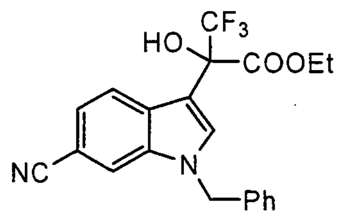

The title compound was obtained in a similar manner to the preparation of the compound of Example 2.
$^1$H-NMR (400 MHz, $CDCl_3$) δ 8.04 (d, J= 8.5 Hz, 1H), 7.62 (s, 1H), 7.59 (s, 1H), 7.39-7.33 (m, 4H), 7.11 (dd, J= 7.7, 1.4 Hz, 1H), 5.34 (s, 2H), 4.49-4.36 (m, 2H), 4.44(s, 1H), 1.33 (t, J= 7.1 Hz, 3H).

Example 89

1-[2-(1-Benzyl-6-nitro-1H-indol-3-yl)-3,3,3-trifluoro-2-hydroxypropyl]piperidin-4-one

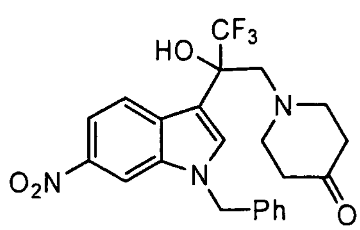

The title compound was obtained in a similar manner to the preparation of the compound of Example 1.
MS (ESI+) 462, 2.27($M^+$+1, detection time)

Example 90

4-[2-(1-Benzyl-6-nitro-1H-indol-3-yl)-3,3,3-trifluoro-2-hydroxypropyl]piperazin-2-one

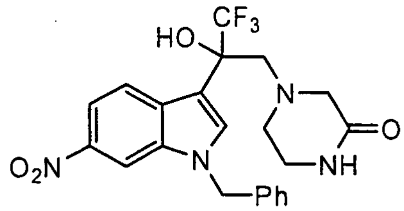

The title compound was obtained in a similar manner to the preparation of the compound of Example 1.
MS (ESI+) 463, 2.17($M^{+}$+1, detection time)

Example 91

2-(1-Benzyl-6-nitro-1H-indol-3-yl)-1,1,1-trifluoro-3-(4-methylpiperazin-1-yl)propan-2-ol

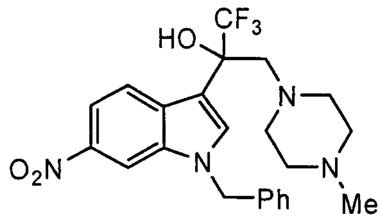

The title compound was obtained in a similar manner to the preparation of the compound of Example 1.
H-NMR (400 MHz, $CDCl_3$) δ 8.27 (d, J= 2.0 Hz, 1H), 8.03 (dd, J= 9.0, 2.0 Hz, 1H), 7.93 (d, J= 9.0 Hz, 1H), 7.55 (s, 1H), 7.34-7.32 (m, 3H), 7.09 (d, J= 7.8 Hz, 2H), 5.72 (bs, 1H), 5.39 (s, 2H), 3.24 (d, J= 13.7 Hz, 1H), 3.11 (d, J= 13.7 Hz, 1H), 2.58 (bs, 4H), 2.42 (bs, 4H), 2.25 (s, 3H).
MS (ESI+) 463, 1.96($M^{+}$+1, detection time)

Example 92

2-(1-Benzyl-6-nitro-1H-indol-3-yl)-1,1,1-trifluoro-3-[4-(methylsulfonyl)piperazin-1-yl]propan-2-ol

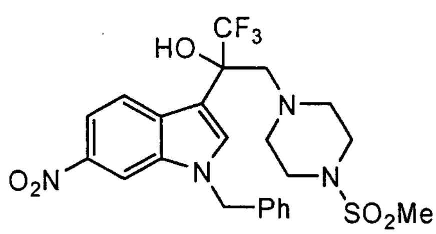

The title compound was obtained in a similar manner to the preparation of the compound of Example 1.
MS (ESI+) 527, 2.38($M^{+}$+1, detection time)

Example 93

2-(1-Benzyl-6-nitro-1H-indol-3-yl)-1,1,1-trifluoro-3-[4-(morpholin-4-ylcarbonyl)piperidin-1-yl]propan-2-ol

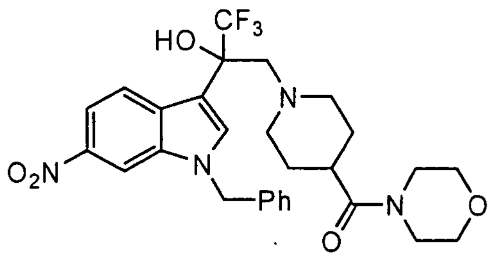

A solution of the compound of Example 82 (58 mg), triethylamine (48 mg), morpholine (14 mg), 1-hydroxybenzotriazole (22 mg) and 1-ethyl-3-(3-dimethylaminopropyl)carbodiimide hydrochloride (32 mg) in dichloromethane (5 ml) was stirred at 25°C overnight. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (43 mg).
$^1$H-NMR (400 MHz, $CDCl_3$) δ 8.26 (d, J= 1.8 Hz, 1H), 8.02 (dd, J= 9.0, 1.8 Hz, 1H), 7.87 (d, J= 9.0 Hz, 1H), 7.60 (s, 1H), 7.41-7.29 (m, 3H), 7.10 (d, J= 7.5 Hz, 2H), 5.39 (s, 2H), 3.65 (bs, 4H), 3.61 (bs, 2H), 3.45 (bs, 2H), 3.21 (d, J= 13.6 Hz, 1H), 3.14 (d, J= 13.6 Hz, 1H), 3.03-2.92 (m, 1H), 2.66-2.48 (m, 2H), 2.48-2.35 (m, 1H), 2.27-2.12 (m, 1H), 1.98-1.75 (m, 2H), 1.75-1.64 (m, 1H), 1.56-1.45 (m, 1H).
MS (ESI+) 561, 1.93($M^+$+1, detection time)

Example 94

1-[2-(1-Benzyl-6-nitro-1H-indol-3-yl)-3,3,3-trifluoro-2-hydroxypropyl]-N,N-dimethylpiperidine-4-carboxamide

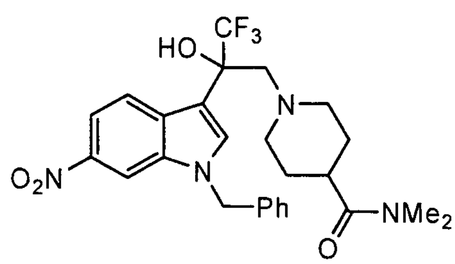

The title compound was obtained in a similar manner to the preparation of the compound of Example 93 (Yield: 36 mg).
$^1$H-NMR (400 MHz, $CDCl_3$) δ 8.26 (d, J= 2.0 Hz, 1H), 8.02 (dd, J= 9.0, 2.0 Hz, 1H), 7.87 (d, J= 9.0 Hz, 1H), 7.61 (s, 1H), 7.36-7.29 (m, 3H), 7.10 (d, J= 7.9 Hz, 2H), 5.40 (s, 2H), 3.21 (d, J= 13.6 Hz, 1H), 3.14 (d, J= 13.6 Hz, 1H), 3.01 (s, 3H), 2.91 (s, 3H), 2.62-2.42 (m, 3H), 2.25-2.14 (m, 1H), 1.94-1.65 (m, 4H), 1.56-1.46 (m, 1H).
MS (ESI+) 519, 1.94($M^+$+1, detection time)

.

Example 95

1-[2-(1-Benzyl-6-nitro-1H-indol-3-yl)-3,3,3-trifluoro-2-hydroxypropyl]-N-methyl-N-phenylpiperidine-4-carboxamide

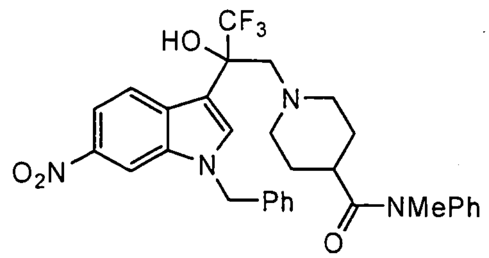

The title compound was obtained in a similar manner to the preparation of the compound of Example 5 (Yield: 33 mg). $^1$H-NMR (400 MHz, $CDCl_3$) δ 8.24 (d, J= 1.9 Hz, 1H), 7.99 (dd, J= 9.0, 1.9 Hz, 1H), 7.81 (d, J= 9.0 Hz, 1H), 7.58 (s, 1H), 7.44-7.29 (m, 6H), 7.13 (d, J= 7.2 Hz, 2H), 7.12-7.05 (m, 2H), 5.38 (s, 2H), 3.23 (s, 3H), 3.08 (d, J= 13.6 Hz, 1H), 3.04 (d, J= 13.6 Hz, 1H), 3.90-2.80 (m, 1H), 2.49-2.38 (m, 1H), 2.30-2.10 (m, 2H), 1.98-1.68 (m, 4H), 1.63-1.51 (m, 1H).
MS (ESI+) 581, 2.10($M^+$+1, detection time)

Example 96

1-[2-(1-Benzyl-6-nitro-1H-indol-3-yl)-3,3,3-trifluoro-2-hydroxypropyl]-N-phenylpiperidine-4-carboxamide

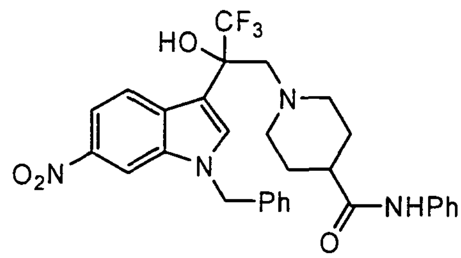

The title compound was obtained in a similar manner to the preparation of the compound of Example 5 (Yield: 39 mg). $^1$H-NMR (400 MHz, $CDCl_3$) δ 8.27 (d, J= 1.9 Hz, 1H), 8.02 (dd, J= 9.0, 1.9 Hz, 1H), 7.88 (d, J= 9.0 Hz, 1H), 7.60 (s, 1H), 7.48 (d, J= 7.9 Hz, 2H), 7.37-7.27 (m, 5H), 7.21-7.13 (m, 1H), 7.13-7.05 (m, 3H), 5.39 (s, 2H), 3.24 (d, J= 13.6 Hz, 1H), 3.16 (d, J= 13.6 Hz, 1H), 3.09-2.98 (m, 1H), 2.70-2.49 (m, 2H), 2.30-2.15 (m, 2H), 1.99-1.88 (m, 2H).
MS (ESI+) 567, 2.07($M^+$+1, detection time)

Example 97

1-[2-(1-Benzyl-6-nitro-1H-indol-3-yl)-3,3,3-trifluoro-2-hydroxypropyl]-N-cyclopropylpiperidine-4-carboxamide

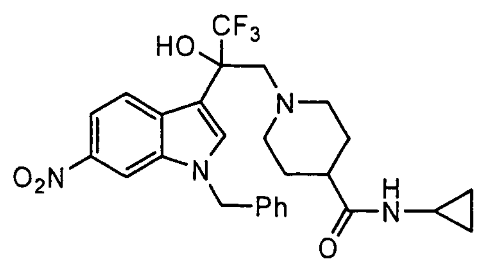

The title compound was obtained in a similar manner to the preparation of the compound of Example 93 (Yield: 33 mg). $^1$H-NMR (400 MHz, $CDCl_3$) δ 8.26 (d, J= 2.0 Hz, 1H), 8.02 (dd, J= 9.0, 2.0 Hz, 1H), 7.87 (d, J= 9.0 Hz, 1H), 7.58 (s, 1H), 7.39-7.29 (m, 3H), 7.09 (d, J= 7.8 Hz, 2H), 5.54 (bs, 1H), 5.39 (s, 2H), 3.21 (d, J= 13.6 Hz, 1H), 3.11 (d, J= 13.6

Hz, 1H), 3.02-2.91 (m, 1H), 2.73-2.62 (m, 1H), 2.62-2.42 (m, 2H), 2.15-2.09 (m, 1H), 2.06-1.91 (m, 1H), 1.84-1.71 (m, 2H), 1.71-1.54 (m, 2H), 0.82-0.73 (m, 2H), 0.49-0.40 (m, 2H),.
MS (ESI+) 531, 1.94($M^{+}$+1, detection time)

Example 98

1-[2-(1-Benzyl-6-nitro-1H-indol-3-yl)-3,3,3-trifluoro-2-hydroxypropyl]-N-methylpiperidine-4-carboxamide

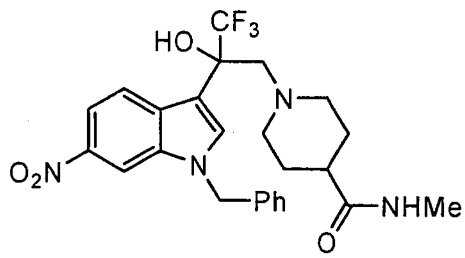

The title compound was obtained in a similar manner to the preparation of the compound of Example 93 (Yield: 32 mg).
$^{1}$H-NMR (400 MHz, $CDCl_3$) δ 8,26 (d, J= 1.8 Hz, 1H), 8.02 (dd, J= 9.0, 1.8 Hz, 1H), 7.87 (d, J= 9.0 Hz, 1H), 7.58 (s, 1H), 7.37-7.28 (m, 3H), 7.09 (d, J= 6.0 Hz, 2H), 5.42 (bs, 1H), 5.39 (s, 2H), 3.21 (d, J= 13.6 Hz, 1H), 3.12 (d, J= 13.6 Hz, 1H), 3.02-2.93 (m, 1H), 2.80 (d, J= 4.8 Hz, 1H), 2.63-2.45 (m, 2H), 2.26-2.12 (m, 1H), 2.09-1.97 (m, 1H), 1.86-1.78 (m, 2H), 1.78-1.57 (m, 2H).
MS (ESI+) 505, 1.92($M^{+}$+1, detection time)

Example 99

1-[2-(1-Benzyl-6-nitro-1H-indol-3-yl)-3,3,3-trifluoro-2-hydroxypropyl]piperidine-4-carboxamide

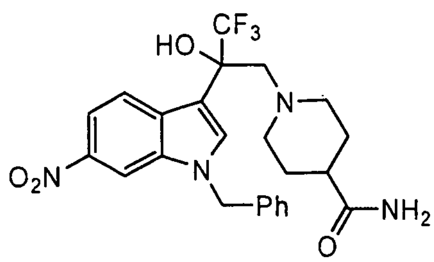

The title compound was obtained in a similar manner to the preparation of the compound of Example 93 (Yield: 37 mg).
$^{1}$H-NMR (400 MHz, $CDCl_3$) δ 8.26 (d, J= 1.9 Hz, 1H), 8.02 (dd, J= 9.0, 1.9 Hz, 1H), 7.88 (d, J= 9.0 Hz, 1H), 7.58 (s, 1H), 7.37-7.28 (m, 3H), 7.10 (d, J= 7.8 Hz, 1H), 5.54-5.41 (m, 2H), 5.39 (s, 2H), 3.32 (d, J= 13.6 Hz, 1H), 3.12 (d, J= 13.6 Hz, 1H), 3.02-2.92 (m, 1H), 2.66-2.45 (m, 2H), 2.28-2.08 (m, 2H), 2.91-2.75 (m, 2H) 2.75-2.60 (m, 2H).
MS (ESI+) 491, 1.88($M^{+}$+1, detection time)

Example 100

tert-Butyl 4-[2-(1-benzyl-6-nitro-1H-indol-3-yl)-3,3,3-trifluoro-2-hydroxypropyl]-piperazine-1-carboxylate

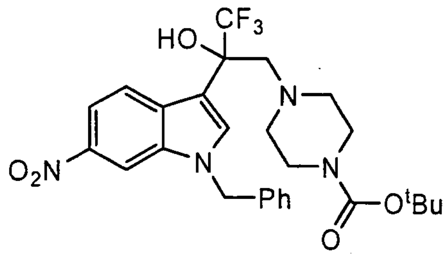

The title compound was obtained in a similar manner to the preparation of the compound of Example 1.
H-NMR (400 MHz, $CDCl_3$) δ 8.28 (d, J= 2.0 Hz, 1H), 8.03 (dd, J= 9.0, 2.0 Hz, 1H), 7.90 (d, J= 9.0 Hz, 1H), 7.56 (s, 1H), 7.36-7.31 (m, 3H), 7.10 (d, J= 7.8 Hz, 2H), 5.50 (bs, 1H), 5.40 (s, 2H), 3.37 (bs, 4H), 3.25 (d, J= 13.7 Hz, 1H), 3.13 (d, J= 13.7 Hz, 1H), 2.50-2.49 (m, 4H), 1.43 (s, 9H).

Example 101

2-(1-Benzyl-6-nitro-1H-indol-3-yl)-1,1,1-trifluoro-3-piperazin-1-ylpropan-2-ol dihydrochloride

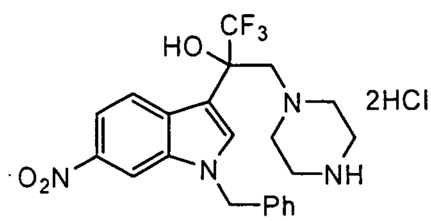

To the compound of Example 100 (1.29 g) was added a solution of 4N hydrochloric acid in 1,4-dioxane (20 ml), and the mixture was stirred at 25˚C for 20 hours. The reaction solution was concentrated under reduced pressure, and thereto was added diethyl ether, and the mixture was filtered. The obtained residue was dried to give the title compound (1.16 g).
H-NMR (400 MHz, DMSO) δ 8.54 (d, J= 2.1 Hz, 1H), 8.23 (bs, 1H), 8.05 (d, J= 9.0 Hz, 1H), 7.95 (dd, J= 9.0,2.1 Hz, 1H), 7.38-7.23 (m, 6H), 5.66 (s, 2H), 3.77 (bs, 1H), 3.50 (bs, 1H), 3.11 (bs, 8H).
MS (ESI+) 449,1.94 ($M^+$+1, detection time)

Example 102

3-Amino-2-(1-benzyl-6-nitro-1H-indol-3-yl)-1,1,1-trifluoropropan-2-ol

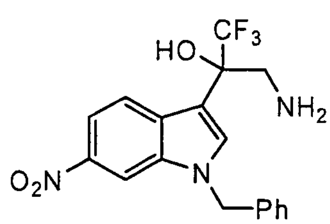

The title compound was obtained in a similar manner to the preparation of the compound of Example 1 (Yield: 855 mg).
H-NMR (400 MHz, $CDCl_3$) δ 8.26 (d, J= 2.0 Hz, 1H), 8.02 (dd, J= 9.0, 2.0 Hz, 1H), 7.94 (d, J= 9.0 Hz, 1H), 7.52 (s, 1H), 7.35-7.31 (m, 3H), 7.13 (d, J= 7.9 Hz, 2H), 5.39 (s, 2H), 3.63 (d, J= 13.3 Hz, 1H), 3.18 (d, J= 13.1 Hz, 1H).
MS (ESI+) 380, 1.87(M++1, detection time)

Example 103

tert-Butyl {1-[2-(1-benzyl-6-nitro-1H-indol-3-yl)-3,3,3-trifluoro-2-hydroxypropyl]piperidin-4-yl}carbamate

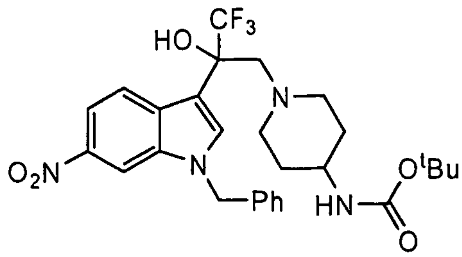

The title compound was obtained in a similar manner to the preparation of the compound of Example 1.
MS (ESI+) 563, 2.17($M^{+}$+1, detection time)

Example 104

3-(4-Aminopiperidin-1-yl)-2-(1-benzyl-6-nitro-1H-indol-3-yl)-1,1,1-trifluoropropan-2-ol dihydrochloride

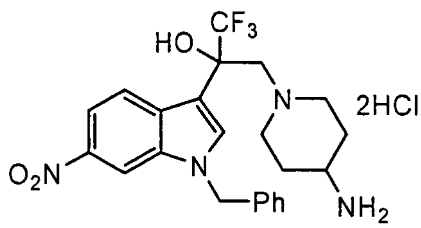

The title compound was obtained from the compound of Example 103 (928 mg) as a starting compound in a similar manner to the preparation of the compound of Example 101 (Yield: 778 mg).
MS (ESI+) 463, 1.86($M^{+}$+1, detection time)

Example 105

3-(1-Benzyl-6-nitro-1H-indol-3-yl)-1-(2-chloro-5-fluorophenyl)-4,4,4-trifluoro-3-hydroxybutan-1-one

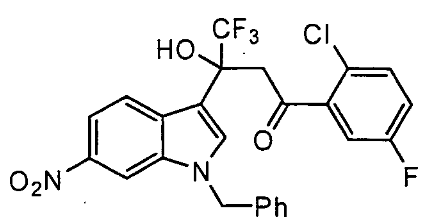

The title compound was obtained in a similar manner to the preparation of the compound of Example 14.
$^{1}$H-NMR (400 MHz, $CDCl_3$) δ 8.26 (d, J= 1.9 Hz, 1H), 7.99 (dd, J= 9.0, 1.9 Hz, 1H), 7.93 (d, J= 9.0 Hz, 1H), 7.43 (s, 1H), 7.40-7.30 (m, 4H), 7.15-7.06 (m, 3H), 6.93-9.89 (m, 1H), 5.37 (s, 2H), 4.04 (d, J= 17.0 Hz, 1H), 3.77 (d, J= 17.0 Hz, 1H).

Example 106

2-(1-Benzyl-6-nitro-1H-indol-3-yl)-1,1,1-trifluoro-3-[4-(2-thienylcarbonyl)-piperazin-1-yl]propan-2-ol

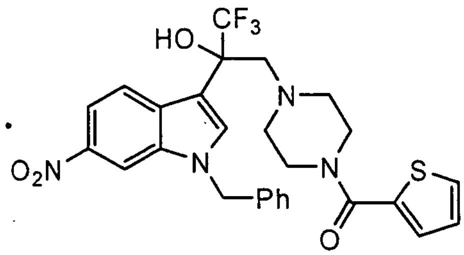

The title compound was obtained from the compound of Example 101 (52 mg) as a starting compound in a similar manner to Example 114 (Yield: 46 mg).
$^1H$-NMR (400 MHz, $CDCl_3$) δ 8.29 (d, J= 1.9 Hz, 1H), 8.03 (dd, J= 9.0, 1.9 Hz, 1H), 7.91 (d, J= 9.0 Hz, 1H), 7.56 (s, 1H), 7.45 (d, J= 5.0 Hz, 1H), 7.34-7.31 (m, 3H), 7.25-7.23 (m, 1H), 7.11-7.09 (m, 2H), 7.05-7.00 (m, 1H), 5.40 (s, 2H), 3.70 (bs, 4H), 3.28 (d, J= 13.7 Hz, 1H), 3.17 (d, J= 13.7 Hz, 1H), 2.68-2.52 (m, 4H). MS (ESI+) 559, 2.39($M^+$+1, detection time)

Example 107

2-(1-Benzyl-6-nitro-1H-indol-3-yl)-3-[4-(cyclopropylcarbonyl)piperazin-1-yl]-1,1,1-trifluoropropan-2-ol

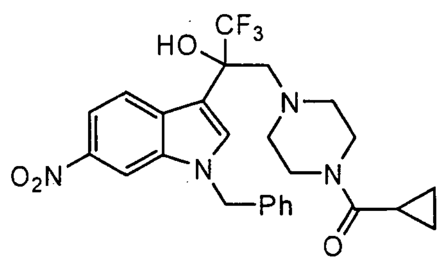

The title compound was obtained from the compound of Example 101 (52mg) as a starting compound in a similar manner to Example 114 (Yield: 48 mg).
$^1H$-NMR (400 MHz, $CDCl_3$) δ 8.29 (d, J= 1.9 Hz, 1H), 8.04 (dd, J= 9.0, 1.9 Hz, 1H), 7.90 (d, J= 9.0 Hz, 1H), 7.57 (s, 1H), 7.37-7.31 (m, 3H), 7.12-7.09 (m, 2H), 5.40 (s, 2H), 3.77-3.49 (m, 4H), 3.28 (d, J= 13.7 Hz, 1H), 3.15 (d, J= 13.7 Hz, 1H), 2.70-2.45 (m, 4H), 1.68-1.62 (m, 1H), 0.98-0.93 (m, 2H), 0.78-0.73 (m, 2H). MS (ESI+) 517, 2.28($M^+$+1, detection time)

Example 108

2-(1-Benzyl-6-nitro-1H-indol-3-yl)-1,1,1-trifluoro-3-[4-(2-furoyl)piperazin-1-yl]propan-2-ol

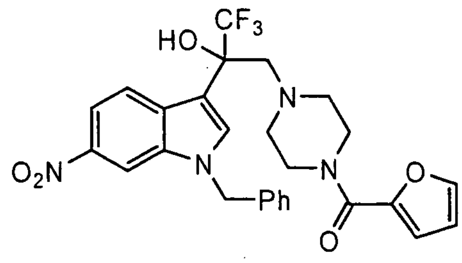

The title compound was obtained from the compound of Example 101 (52 mg) as a starting compound in a similar manner to Example 114 (Yield: 38 mg).
$^1$H-NMR (400 MHz, $CDCl_3$) δ 8.29 (d, J= 1.9 Hz, 1H), 8.04 (dd, J= 9.0, 1.9 Hz, 1H), 7.91 (d, J= 9.0 Hz, 1H), 7.57 (s, 1H), 7.45 (s, 1H), 7.35-7.32 (m, 3H), 7.12-7.09 (m, 2H), 7.00 (d, J= 3.3 Hz, 1H), 6.48-6.46 (m, 1H), 5.41 (s, 2H), 3.77 (bs, 4H), 3.29 (d, J= 13.7 Hz, 1H), 3.17 (d, J=13.7 Hz, 1H), 2.62-2.59 (m, 4H).
MS (ESI+) 543, 2.23($M^+$+1, detection time)

Example 109

2-(1-Benzyl-6-nitro-1H-indol-3-yl)-1,1,1-trifluoro-3-[4-(methoxyacetyl)piperazin-1-yl]propan-2-ol

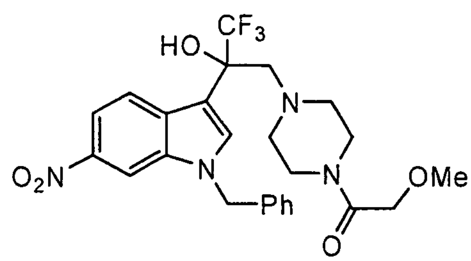

The title compound was obtained from the compound of Example 101 (52 mg) as a starting compound in a similar manner to Example 114 (Yield: 42 mg).
$^1$H-NMR (400 MHz, $CDCl_3$) δ 8.28 (d, J= 2.0 Hz, 1H), 8.03 (dd, J= 9.0, 2.0 Hz, 1H), 7.90 (d, J=9.0 Hz, 1H), 7.56 (s, 1H), 7.36-7.32 (m, 3H), 7.12-7.10 (m, 2H), 5.40 (s, 2H), 4.06 (s, 2H), 3.58 (bs, 2H), 3.49 (bs, 2H), 3.39 (s, 3H), 3.27 (d, J= 13.8 Hz, 1H), 3.14 (d, J= 13.8 Hz, 1H), 2.61-2.51 (m, 4H).
MS (ESI+) 521, 2.21($M^+$+1, detection time)

Example 110

4-[2-(1-Benzyl-6-nitro-1H-indol-3-yl)-3,3,3-trifluoro-2-hydroxypropyl]-N-phenylpiperazine-1-carboxamide

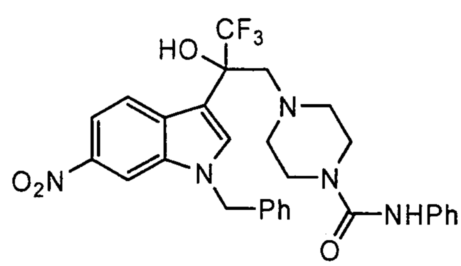

The title compound was obtained from the compound of Example 101 (52 mg) as a starting compound in a similar

manner to Example 114 (Yield: 48 mg).
$^1H$-NMR (400 MHz, $CDCl_3$) δ 8.28 (d, J= 1.9 Hz, 1H), 8.03 (dd, J= 8.9, 1.9 Hz, 1H), 7.91 (d, J= 8.9 Hz, 1H), 7.57 (s, 1H), 7.35-7.26 (m, 7H), 7.10 (d, J= 6.8 Hz, 2H), 7.09-7.00 (m, 1H), 6.30 (s, 1H), 5.40 (s, 2H), 3.46 (bs, 4H), 3.30 (d, J=13.7 Hz, 1H), 3.17 (d, J= 13.7 Hz, 1H), 2.70-2.50 (m, 4H).
MS (ESI+) 568, 2.33($M^+$+1, detection time)

Example 111

4-[2-(1-Benzyl-6-nitro-1H-indol-3-yl)-3,3,3-trifluoro-2-hydroxypropyl]-N-methylpiperazine-1-carboxamide

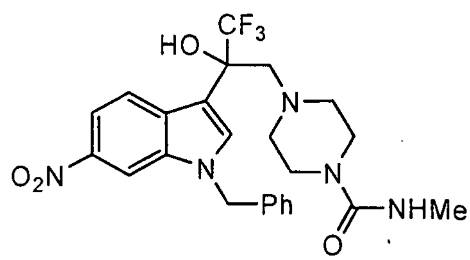

The title compound was obtained from the compound of Example 101 (52 mg) as a starting compound in a similar manner to Example 114 (Yield: 39 mg).
$^1H$-NMR (400 MHz, $CDCl_3$) δ 8.27 (d, J= 1.8 Hz, 1H), 8.02 (dd, J= 9.0, 1.8 Hz, 1H), 7.90 (d, J= 9.0 Hz, 1H), 7.56 (s, 1H), 7.35-7.31 (m, 3H), 7.10 (d, J= 7.6 Hz, 1H), 5.39 (s, 1H), 4.43 (d, J= 4.6 Hz, 1H), 3.31 (bs, 4H), 3.26 (d, J= 13.8 Hz, 1H), 3.13 (d, J= 13.8 Hz, 1H), 2.78 (d, J= 4.6 Hz, 3H), 2.56-2.52 (m, 4H).
MS (ESI+) 506, 2.07($M^+$+1, detection time)

Example 112

2-(1-Benzyl-6-nitro-1H-indol-3-yl)-1,1,1-trifluoro-3-[4-(phenylsulfonyl)piperazin-1-yl]propan-2-ol

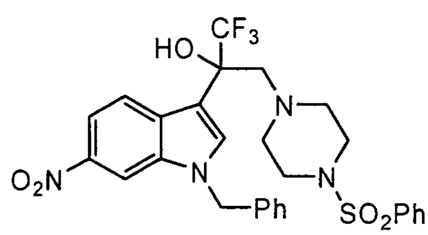

The title compound was obtained from the compound of Example 101 (52 mg) as a starting compound in a similar manner to Example 114 (Yield: 39 mg).
$^1H$-NMR (400 MHz, $CDCl_3$) δ 8.26 (d, J= 1.9 Hz, 1H), 8.02 (dd, J= 9.0, 1.9 Hz, 1H), 7.87 (d, J= 9.0 Hz, 1H), 7.72 (d, J= 7.3 Hz, 2H), 7.65 (dd, J= 7.8, 7.3 Hz, 1H), 7.57 (dd, J= 7.8, 7.8 Hz, 2H), 7.47 (s, 1H), 7.34-7.32 (m, 3H), 7.09-7.07 (m, 2H), 5.38 (s, 2H), 5.20-4.90 (bs, 1H), 3.24 (d, J= 13.9 Hz, 1H), 3.09 (d, J= 13.9 Hz, 1H), 2.98 (bs, 4H), 2.69-2.61 (m, 4H).
MS (ESI+) 589, 2.53($M^+$+1, detection time)

Example 113

2-(1-Benzyl-6-nitro-1H-indol-3-yl)-1,1,1-trifluoro-3-(4-methyl-3,4-dihydro-1'H-spiro[1,4-benzoxazine-2,4'-piperidin]-1'-yl)propan-2-ol

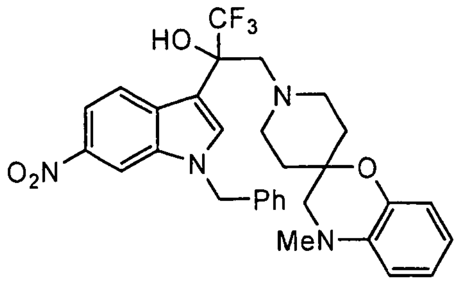

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.0, 2.0 Hz, 1H), 7.93 (d, J= 9.0 Hz, 1H), 7.56 (s, 1H), 7.32-7.30 (m, 3H), 7.09 (d, J= 7.7 Hz, 2H), 6.85 (dd, J= 7.5, 7.5 Hz, 1H), 6.76 (dd, J= 7.5, 7.5 Hz, 1H), 6.66 (d, J= 7.5 Hz, 2H), 5.90 (bs, 1H), 5.39 (s, 2H), 3.29 (d, J= 13.5 Hz, 1H), 3.18 (d, J= 13.5 Hz, 1H), 2.95 (s, 2H), 2.88 (s, 3H), 2.95-2.90 (m, 1H), 2.73-2.61 (m, 2H), 2.40-2.37 (m, 1H), 1.86-1.83 (m, 1H), 1.70-1.56 (m, 3H).
MS (ESI+) 581, 2.27($M^+$+1, detection time)

Example 114

3-(4-Acetylpiperazin-1-yl)-2-(1-benzyl-6-nitro-1H-indol-3-yl)-1,1,1-trifluoropropan-2-ol

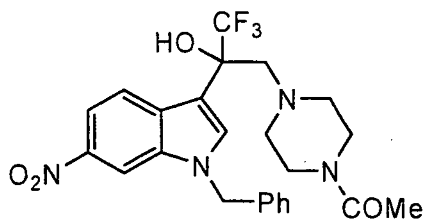

To a solution of the compound of Example 101 (52 mg), triethylamine (41 mg) in tetrahydrofuran (3 ml) was added acetyl chloride (8.6 mg), and the mixture was stirred at 25°C overnight. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (23 mg).
$^1$H-NMR (400 MHz, $CDCl_3$) δ 8.28 (d, J= 2.0 Hz, 1H), 8.04 (dd, J= 9.0, 2.0 Hz, 1H), 7.90 (d, J= 9.0 Hz, 1H), 7.56 (s, 1H), 7.39-7.29 (m, 3H), 7.10 (dd, J= 7.5, 1.9 Hz, 2H), 5.40 (s, 2H), 5.37 (s, 1H), 3.57 (bs, 2H), 3.50-3.34 (m, 2H), 3.28 (d, J= 13.8 Hz, 1H), 3.14 (d, J= 13.8 Hz, 1H), 2.64-2.45 (m, 4H).
MS (ESI+) 491, 2.20($M^+$+1, detection time)

Example 115

3-(4-Benzoylpiperazin-1-yl)-2-(1-benzyl-6-nitro-1H-indol-3-yl)-1,1,1-trifluoropropan-2-ol

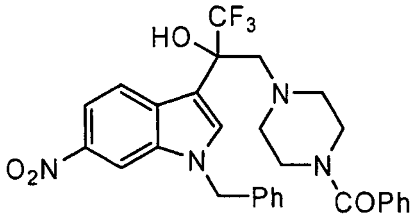

The title compound was obtained from the compound of Example 101 (52 mg) as a starting compound in a similar manner to Example 114 (Yield: 30 mg).
$^1$H-NMR (400 MHz, $CDCl_3$) δ 8.28 (d, J= 1.6 Hz, 1H), 8.03 (dd, J= 9.0, 1.6 Hz, 1H), 7.90 (d, J= 9.0Hz, 1H), 7.54 (s, 1H), 7.40-7.30 (m, 8H), 7.10-7.08 (m, 2H), 5.38 (s, 2H), 3.90-3.30 (m, 4H), 3.27 (d, J= 13.7 Hz, 1H), 3.16 (d, J= 13.7 Hz, 1H), 2.73-2.37 (m, 4H).
MS (ESI+) 553, 2.39($M^+$+1, detection time)

Example 116

2-(1-Benzyl-6-nitro-1H-indol-3-yl)-1,1,1-trifluoro-3-[4-(4-methylphenoxy)-piperidin-1-yl]propan-2-ol

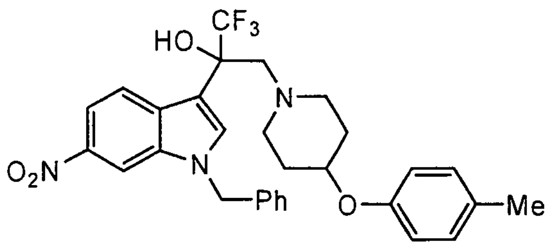

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.1 2.0 Hz, 1H), 7.90 (d, J= 9.1 Hz, 1H), 7.57 (s, 1H), 7.32-7.28 (m, 3H), 7.10-7.07 (m, 2H), 7.05 (d, J= 8.4 Hz, 2H), 6.75 (d, J= 8.4 Hz, 2H), 5.38 (s, 2H), 4.32-4.23 (m, 1H), 3.24 (d, J= 13.6 Hz, 1H), 3.14 (d, J= 13.6 Hz, 1H), 2.82-2.72 (m, 2H), 2.51-2.42 (m, 2H), 2.26 (s, 3H), 1.92-1.82 (m, 2H), 1.82-1.70 (m, 2H).
MS (ESI+) 554, 2.30($M^+$+1, detection time)

Example 117

2-(1-Benzyl-6-nitro-1H-indol-3-yl)-1,1,1-trifluoro-3-[4-(2-methoxyphenoxy)-piperidin-1-yl] propan-2-ol

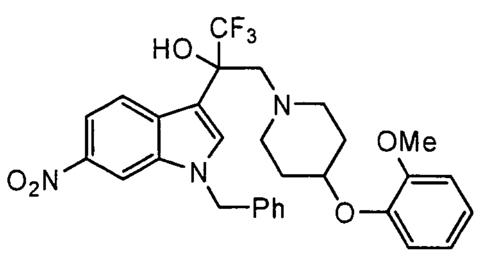

The title compound was obtained in a similar manner to the preparation of the compound of Example 1.
$^1$H-NMR (400 MHz, $CDCl_3$) δ 8.26 (d, J= 1.8 Hz, 1H), 8.02 (dd, J= 9.0, 1.8 Hz, 1H), 7.90 (d, J= 9.0 Hz, 1H), 7.58 (s,

1H), 7.35-7.27 (m, 3H), 7.08 (d, J= 7.5 Hz, 2H), 6.95-6.82 (m, 4H), 5.38 (s, 2H), 4.29-4.20 (m, 1H), 3.82 (s, 3H), 3.25 (d, J= 13.6 Hz, 1H), 3.15 (d, J= 13.6 Hz, 1H), 2.90-2.74 (m, 2H), 2.52-2.40 (m, 2H), 1.96-1.75 (m, 4H).
MS (ESI+) 570, 2.17($M^{+}$+1, detection time)

Example 118

2-(1-Benzyl-6-nitro-1H-indol-3-yl)-1,1,1-trifluoro-3-(4-phenoxypiperidin-1-yl)propan-2-ol

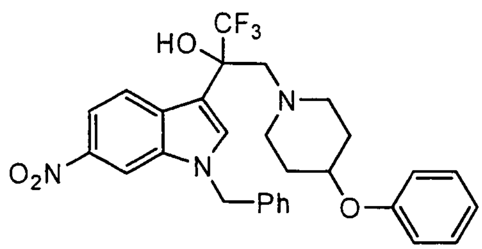

The title compound was obtained in a similar manner to the preparation of the compound of Example 1.
$^{1}$H-NMR (400 MHz, $CDCl_3$) δ 8.26 (d, J= 2.0 Hz, 1H), 8.02 (dd, J= 9.0, 2.0 Hz, 1H), 7.90 (d, J= 9.0 Hz, 1H), 7.58 (s, 1H), 7.35-7.22 (m, 5H), 7.10-7.05 (m, 2H), 6.93 (dd, J= 7.8, 7.8 Hz, 1H), 6.85 (d, J= 7.8 Hz, 1H), 5.38 (s, 2H), 4.38-4.29 (m, 1H), 3.25 (d, J= 13.6 Hz, 1H), 3.15 (d, J= 13.6 Hz, 1H), 2.82-2.73 (m, 2H), 2.54-2.42 (m, 2H), 1.97-1.72 (m, 4H).
MS (ESI+) 540, 2.24($M^{+}$+1, detection time)

Example 119

2-(1-Benzyl-6-nitro-1H-indol-3-yl)-3-[4-(2-chlorophenoxy)piperidin-1-yl]-1,1,1-trifluoropropan-2-ol

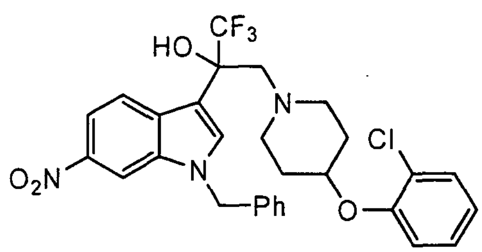

The title compound was obtained in a similar manner to the preparation of the compound of Example 1.
$^{1}$H-NMR (400 MHz, $CDCl_3$) δ 8.26 (d, J= 2.0 Hz, 1H), 8.02 (d, J= 9.0, 2.0 Hz, 1H), 7.92 (d, J= 9.0 Hz, 1H), 7.57 (s, 1H), 7.37-7.27 (m, 4H), 7.19-7.13 (m, 1H), 7.10-7.08 (m, 2H), 6.91-6.88 (m, 2H), 5.39 (s, 2H), 4.44-4.38 (m, 1H), 3.25 (d, J= 13.6 Hz, 1H), 3.15 (d, J= 13.6 Hz, 1H), 2.92-2.78 (m, 2H), 2.60-2,50 (m, 1H), 2.50-2.41 (m, 1H), 1.92-1.78 (m, 4H).
MS (ESI+) 574, 2.32($M^{+}$+1, detection time)

Example 120

2-(1-Benzyl-6-nitro-1H-indol-3-yl)-3-(1,1-dioxidothiomorpholin-4-yl)-1,1,1-trifluoropropan-2-ol

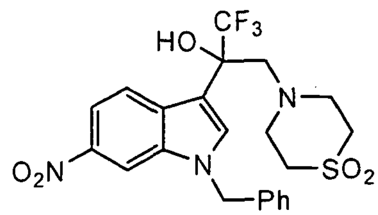

The title compound was obtained in a similar manner to the preparation of the compound of Example 1.
$^1$H-NMR (400 MHz, $CDCl_3$) δ 8.30 (d, J= 2.0 Hz, 1H), 8.04 (dd, J= 9.0, 2.0 Hz, 1H), 7.90 (dd, J= 9.0 Hz, 1H), 7.53 (s, 1H), 7.35-7.33 (m, 3H), 7.12 (d, J= 7.6 Hz, 2H), 5.40 (s, 2H), 4.65 (bs, 1H), 3.38 (d, J= 14.1 Hz, 1H), 3.32 (d, J= 14.1 Hz, 1H), 3.15-3.13 (m, 4H), 3.00-2.98 (m, 4H).
MS (ESI+) 498, 2.28($M^+$+1, detection time)

Example 121

2-(1-Benzyl-6-nitro-1H-indol-3-yl)-1,1,1-trifluoro-3-(1'H-spiro[1,4-benzodioxine-2,4'-piperidin]-1'-yl)propan-2-ol

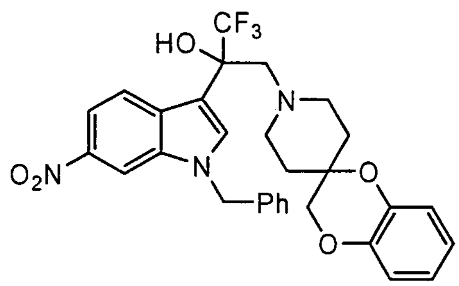

The title compound was obtained in a similar manner to the preparation of the compound of Example 1.
$^1$H-NMR (400 MHz, $CDCl_3$) δ 8,26 (d, J= 2.0 Hz, 1H), 8.02 (d, J= 9.0, 2.0 Hz, 1H), 7.91 (d, J= 9.0 Hz, 1H), 7.57 (s, 1H), 7.32-7.30 (m, 3H), 7.08 (d, J= 7.7 Hz, 2H), 6.84-6.83 (m, 4H), 5.39 (s, 2H), 3.88 (s, 2H), 3.29 (d, J= 13.6 Hz, 1H), 3.20 (d, J= 13.6 Hz, 1H), 2.95-2.92 (m, 1H), 2.76-2.72 (m, 1H), 2.63-2.59 (m, 1H), 2.42-2.40 (m, 1H), 1.87-1.82 (m, 1H), 1.72-1.66 (m, 2H), 1.59-1.55 (m, 1H).
MS (ESI+) 568, 2.31($M^+$+1, detection time)

Example 122

2-(1-Benzyl-6-nitro-1H-indol-3-yl)-1,1,1-trifluoro-3-(1'H-spiro[1,4-benzoxa thiine-2,4'-piperidin]-1'-yl)propan-2-ol

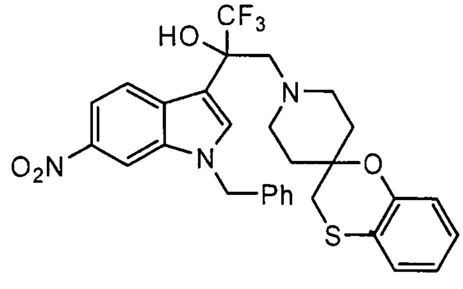

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.0, 2.0 Hz, 1H), 7.92 (d, J= 9.0 Hz, 1H), 7.57 (s, 1H), 7.31-7.30 (m, 3H), 7.09-7.01 (m, 4H), 6.98-6.81 (m, 2H), 5.39 (s, 2H), 3.28 (d, J= 13.5 Hz, 1H), 3.19 (d, J= 13.5 Hz, 1H), 2.94-2.81 (m, 1H), 2.88 (d, J= 13.1 Hz, 1H), 2.82 (d, J= 13.1 Hz, 1H), 2.78-2.76 (m, 1H), 2.60-2.56 (m, 1H), 1.99-1.95 (m, 1H), 1.84-1.73 (m, 2H), 1.60-1.58 (m, 1H).
MS (ESI+) 584, 2.34($M^+$+1, detection time)

Example 123

2-(1-Benzyl-6-nitro-1H-indol-3-yl)-3-(4,4-dioxido-1'H-spiro[1,4-benzoxathiine-2,4'-piperidin]-1'-yl)-1,1,1-trifluoropropan-2-ol

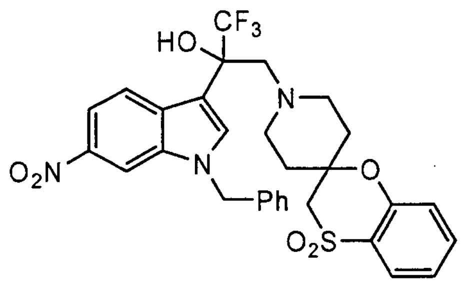

The title compound was obtained in a similar manner to the preparation of the compound of Example 1.
$^1$H-NMR (400 MHz, $CDCl_3$) δ 8.27 (d, J= 2.0 Hz, 1H), 8.02 (dd, J= 9.0, 2.0 Hz, 1H), 7.89 (d, J= 9.0 Hz, 1H), 7.77 (d, J= 7.9 Hz, 1H), 7.58 (s, 1H), 7.46-7.41 (m, 1H), 7.33-7.31 (m, 3H), 7.14 (dd, J= 7.9, 7.9 Hz, 1H), 7.08 (d, J= 7.8 Hz, 2H), 6.99 (d, J= 7.9 Hz, 1H), 5.39 (s, 2H), 3.47 (d, J= 14.3 Hz, 1H), 3.41 (d, J= 14.3 Hz, 1H), 3.27 (d, J= 13.6 Hz, 1H), 3.21 (d, J= 13.6 Hz, 1H), 2.93-2.90 (m, 1H), 2.80-2.76 (m, 1H), 2.56-2.50 (m, 1H), 2.39-2.37 (m, 1H), 2.26-2.22 (m, 1H), 2.14-2.10 (m, 2H), 1.95-1.86 (m, 1H), 1.77-1.72 (m, 1H).
MS (ESI+) 616, 2.28($M^+$+1, detection time)

Example 124

3-(4-Acetyl-3,4-dihydro-1'H-spiro[1,4-benzoxazine-2,4'-piperidin]-1'-yl)-2-(1-benzyl-6-nitro-1H-indol-3-yl)-1,1,1-trifluoropropan-2-ol

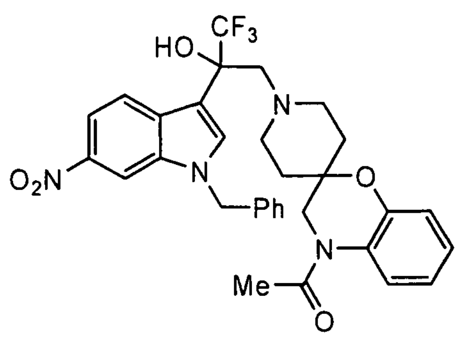

The title compound was obtained in a similar manner to the preparation of the compound of Example 1.
$^1$H-NMR (400 MHz, $CDCl_3$) δ 8.26 (d, J= 2.0 Hz, 1H), 8.03 (dd, J= 9.0, 2.0 Hz, 1H), 7.93 (d, J= 9.0 Hz, 1H), 7.59 (s, 1H), 7.33-7.31 (m, 3H), 7.10-7.09 (m, 4H), 6.89-6.87 (m, 2H), 5.39 (s, 2H), 3.91-3.89 (m, 1H), 3.72-3.70 (m, 1H), 3.29 (d, J= 13.6 Hz, 1H), 3.22 (d, J= 13.6 Hz, 1H), 2.95-2.91 (m, 1H), 2.74-2.61 (m, 2H), 2.45-2.42 (m, 1H), 2.33 (s, 3H), 1.74-1.59 (m, 4H).

Example 125

2-(1-Benzyl-6-nitro-1H-indol-3-yl)-1,1,1-trifluoro-3-[4-(methylsulfonyl)-3,4-dihydro-1'H-spiro[1,4-benzoxazine-2,4'-piperidin]-1'-yl]propan-2-ol

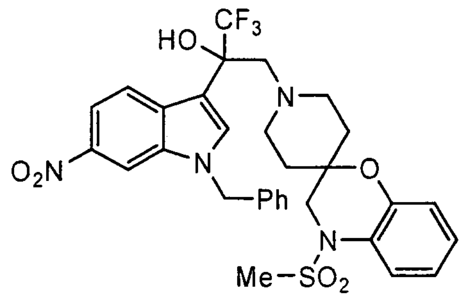

The title compound was obtained in a similar manner to the preparation of the compound of Example 1.
$^1$H-NMR (400 MHz, $CDCl_3$) δ 8.26 (d, J= 2.0 Hz, 1H), 8.03 (dd, J= 9.0, 2.0 Hz, 1H), 7.93 (d, J= 9.0 Hz, 1H), 7.59 (s, 1H), 7.54 (d, J= 8.1 Hz, 1H), 7.33-7.30 (m, 3H), 7.09 (d, J= 7.8 Hz, 2H), 7.03-6.98 (m, 1H), 6.90 (dd, J= 8.1, 8.1 Hz, 2H), 5.39 (s, 2H), 3.65 (d, J= 12.7 Hz, 1H), 3.55 (d, J= 12.7 Hz, 1H), 3.28 (d, J= 13.6 Hz, 1H), 3.21 (d, J= 13.6 Hz, 1H), 3.12 (s, 3H), 2.95-2.89 (m, 1H), 2.77-2.75 (m, 1H), 2.58-2.55 (m, 1H), 2.42-2.39 (m 1H), 1.80-1.60 (m, 4H).

Example 126

1'-[2-(1-Benzyl-6-nitro-1H-indol-3-yl)-3,3,3-trifluoro-2-hydroxypropyl]-N-ethylspiro[1,4-benzoxazine-2,4'-piperidine]-4(3H)-carboxamide

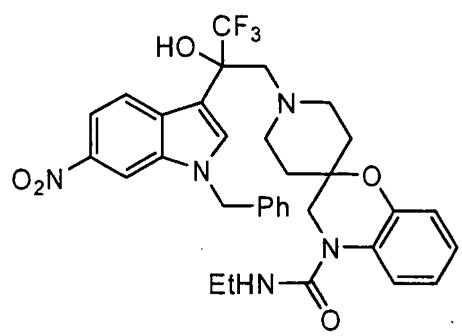

The title compound was obtained in a similar manner to the preparation of the compound of Example 1.
$^1$H-NMR (400 MHz, $CDCl_3$) δ 8.26 (d, J= 1.9 Hz, 1H), 8.03 (dd, J= 9.0, 1.9 Hz, 1H), 7.89 (d, J= 9.0 Hz, 1H), 7.59 (s, 1H), 7.33-7.31 (m, 3H), 7.23 (d, J= 8.1 Hz, 1H), 7.09 (d, J= 7.7 Hz, 1H) 7.05-7.00 (m, 1H), 6.90 (m, 2H), 5.72 (bs, 1H), 5.39 (s, 2H), 3.77 (d, J= 13.5 Hz, 1H), 3.61 (d, J= 13.5 Hz, 1H), 3.33-3.19 (m, 4H), 2.93-2.89 (m, 1H), 2.74-2.65 (m, 2H), 2.45-2.42 (m, 1H), 1.73-1.58 (m, 4), 1.13 (t, J= 7.2 Hz, 3H).
MS (ESI+) 638, 2.17($M^+$+1, detection time)

Example 127

2-(1-Benzyl-6-nitro-1H-indol-3-yl)-1,1,1-trifluoro-3-(1'H-spiro[1,4-dioxino[2,3-b]pyridine-3,4'-piperidin]-1'-yl)propan-2-ol

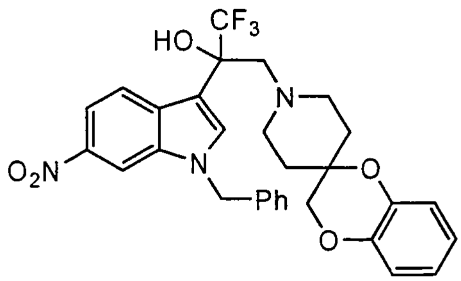

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.0, 2.0 Hz, 1H), 7.94 (d, J= 9.0 Hz, 1H), 7.83 (dd, J= 5.0, 1.5 Hz, 1H), 7.55 (s, 2H), 7.32-7.30 (m, 3H), 7.16 (dd, J= 6.3, 1.5 Hz, 1H), 7.10-7.08 (m, 2H), 6.86-6.85 (m, 2H), 5.39 (s, 2H), 3.90 (s, 2H), 3,29 (d, J= 14.2 Hz, 1H), 3.18 (d, J= 4.2 Hz, 1H), 3.09-3.04 (m, 1H), 2.77-2.74 (m, 2H), 2.44-2.41 (m, 1H), 1.88-1.87 (m, 1H), 1.74-1.70 (m, 2H), 1.62-1.60 (m, 1H).
MS (ESI+) 569, 2.10($M^+$+1, detection time)

Example 128

2-(1-Benzyl-6-nitro-1H-indol-3-yl)-3-(3,4-dihydro-1'H-spiro[1,4-benzoxazine-2,4'-piperidin]-1'-yl)-1,1,1-trifluoropropan-2-ol

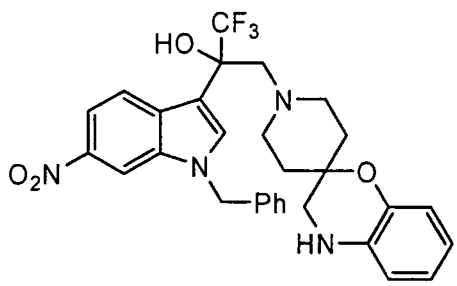

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.0, 2.0 Hz, 1H), 7.94 (d, J= 9.0 Hz, 1H), 7.57 (s, 1H), 7.32-7.30 (m, 3H), 7.09-7.07 (m, 2H), 6.78-6.75 (m, 2H), 6.67-6.65 (m, 1H), 6.58 (d, J= 7.7 Hz, 1H), 5.38 (s, 2H), 3.74 (bs, 1H), 3.29 (d, J= 13.6 Hz, 1H), 3.19 (d, J= 13.6 Hz, 1H), 3.08 (d, J= 1.3 Hz, 2H), 2.97-2.92 (m, 1H), 2.75-2.73 (m, 1H), 2.66-2.61 (m, 1H), 2.40-2.37 (m, 1H), 1.86-1.83 (m, 1H), 1.70-1.65 (m, 2H), 1.53-1.49 (m 1H).
MS (ESI+) 569, 2.14($M^+$+1, detection time)

Example 129.

2-(1-Benzyl-6-nitro-1H-indol-3-yl)-3-(3,4-dihydro-1'H-spiro[chromene-2,4'-piperidin]-1'-yl)-1,1,1-trifluoropropan-2-ol

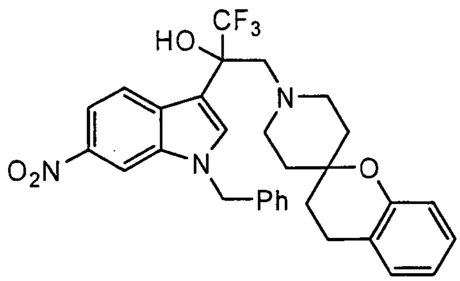

The title compound was obtained in a similar manner to the preparation of the compound of Example 1.
$^1$H-NMR (400 MHz, $CDCl_3$) δ 8.25 (d, J= 2.0 Hz, 1H), 8.03 (dd, J= 9.0, 2.0 Hz, 1H), 7.92 (d, J= 9.0 Hz, 1H), 7.57 (s, 1H), 7.31-7.30 (m, 3H), 7.09-7.02 (m, 4H), 6.82-6.77 (m, 2H), 5.38 (s, 2H), 3.28 (d, J= 13.5 Hz, 1H), 3.18 (d, J= 13.5 Hz, 1H), 2.96-2.93 (m, 1H), 2.76-2.73 (m, 3H), 2.70-2.68 (m, 1H), 2.36-2.33 (m, 1H), 1.87-1.51 (m, 4H), 1.77 (t, J= 6.9 Hz, 2H).
MS (ESI+) 566, 2.24($M^+$+1, detection time)

Example 130

2-(1-Benzyl-6-nitro-1H-indol-3-yl)-1,1,1-trifluoro-3-(1'H-spiro[1,4-dioxino[2,3-b]pyridine-2,4'-piperidin]-1'-yl)propan-2-ol

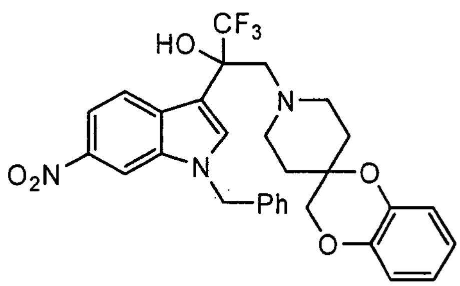

The title compound was obtained in a similar manner to the preparation of the compound of Example 1.
$^1$H-NMR (400 MHz, $CDCl_3$) δ 8.27 (d, J= 2.0 Hz, 1H), 8.02 (dd, J= 9.0, 2.0 Hz, 1H), 7.92 (d, J= 9.0 Hz, 1H), 7.81 (dd, J= 5.0, 1.5 Hz, 1H), 7.58 (s, 1H), 7.32-7.31 (m, 3H), 7.15 (dd, J= 6.3, 1.5 Hz, 1H), 7.10-7.08 (m, 2H), 6.88-6.85 (m, 1H), 5.39 (s, 2), 4.07 (s, 2H), 3.30 (d, J= 13.6 Hz, 1H), 3.21 (d, J= 13.6 Hz, 1H), 2.92-2.89 (m, 1H), 2.79-2.75 (m, 1H), 2.60-2.56 (m, 1H), 2.44-2.41 (m, 1H), 1.82-1.60 (m, 4H).
MS (ESI+) 569, 2.14($M^+$+1, detection time)

Example 131

2-(1-Benzyl-6-nitro-1H-indol-3-yl)-1,1,1-trifluoro-3-[(2-methoxyethyl)amino]-propan-2-ol

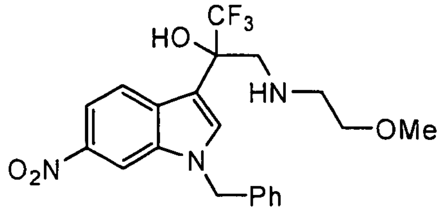

The title compound was obtained in a similar manner to the preparation of the compound of Example 1.
H-NMR (400 MHz, $CDCl_3$) δ 8,26 (d, J= 2.0 Hz, 1H), 8.03 (dd, J= 9.0, 2.0 Hz, 1H), 7.95 (d, J= 9.0 Hz, 1H), 7.53 (s, 1H), 7.35-7.32 (m, 3H), 7.13 (d, J= 7.8 Hz, 2H), 5.39 (s, 2H), 3.54 (d, J= 13.0 Hz, 1H), 3.45 (t, J= 5.0 Hz, 2H), 3.34 (s, 3H), 3.15 (d, J= 13.0 Hz, 1H), 2.88-2.85 (m, 1H), 2.83-2.80 (m, 1H).
MS (ESI+) 452, 1.99($M^+$+1, detection time)

Example 132

2-(1-Benzyl-6-nitro-1H-indol-3-yl)-3-[bis(2-methoxyethyl)amino]-1,1,1-trifluoropropan-2-ol

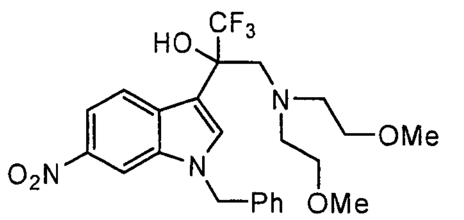

The title compound was obtained in a similar manner to the preparation of the compound of Example 1.
H-NMR (400 MHz, $CDCl_3$) δ 8.26 (d, J= 2.0 Hz, 1H), 8.02 (dd, J= 9.0, 2.0 Hz, 1H), 7.91 (d, J= 9.0 Hz, 1H), 7.60 (s, 1H), 7.33-7.30 (m, 3H), 7.12 (d, J= 7.8 Hz, 2H), 6.07 (bs, 1H), 5.39 (s, 2H), 3.45 (s, 1H), 3.44 (s, 1H), 3.36-3.32 (m, 4H), 3.27 (s, 6H), 2.81-2.71 (m, 4H).
MS (ESI+) 496, 2.29($M^+$+1, detection time)

Example 133

2-(1-Benzyl-6-nitro-1H-indol-3-yl)-1,1,1-trifluoro-3-[(2-phenoxyethyl)amino]-propan-2-ol

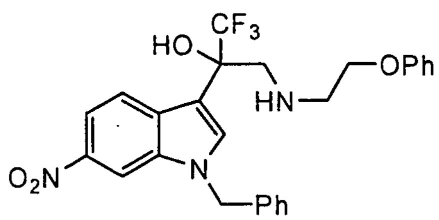

The title compound was obtained in a similar manner to the preparation of the compound of Example 1.
$^1$H-NMR (400 MHz, $CDCl_3$) δ 8.26 (d, J= 2.0 Hz, 1H), 8.01 (dd, J= 9.0, 2.0 Hz, 1H), 7.93 (d, J= 9.0 Hz, 1H), 7.53 (s, 1H), 7.39-7.29 (m, 3H), 7.27 (d, J= 7.8 Hz, 2H), 7.12 (dd, J= 7.8, 2.2 Hz, 2H), 6.98 (dd, J= 7.8, 7.8 Hz, 1H), 6.84 (d, J= 7.8 Hz, 2H), 5.38 (s, 2H), 4.08-4.00 (m, 2H), 3.60 (d, J= 12.9 Hz, 1H), 3.22 (d, J= 12.9 Hz, 1H), 3.18-3.08 (m, 1H), 3.08-2.97 (m, 1H).
MS (ESI+) 500, 2.05($M^+$+1, detection time)

Example 134

2-(1-Benzyl-6-nitro-1H-indol-3-yl)-3-[(1,3-dioxolan-2-ylmethyl)amino]-1,1,1-trifluoropropan-2-ol

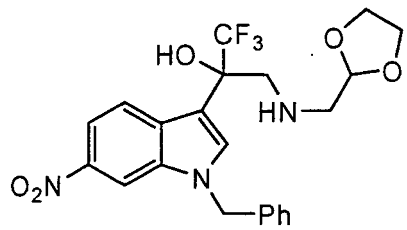

The title compound was obtained in a similar manner to the preparation of the compound of Example 1.
$^1$H-NMR (400 MHz, $CDCl_3$) δ 8.26 (d, J= 2.0 Hz, 1H), 8.02 (dd, J= 9.0, 2.0 Hz, 1H), 7.94 (d, J= 9.0 Hz, 1H), 7.53 (s, 1H), 7.39-7.28 (m, 3H), 7.12 (dd, J= 8.0, 1.8 Hz, 2H), 5.39 (s, 2H), 4.93 (t, J= 3.6 Hz, 1H), 4.00-3.82 (m, 4H), 3.62 (d, J=13.2 Hz, 1H), 3.23 (d, J= 13.2 Hz, 1H), 2.92-2.86 (m, 2H).
MS (ESI+) 466, 1.95($M^+$+1, detection time)

Example 135

2-(1-Benzyl-6-nitro-1H-indol-3-yl)-3-[(1,3-dioxolan-2-ylmethyl)amino]-1,1,1-trifluoropropan-2-ol

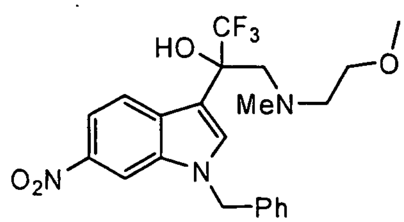

The title compound was obtained in a similar manner to the preparation of the compound of Example 1.
$^1$H-NMR (400 MHz, $CDCl_3$) δ 8.25 (d, J= 2.0 Hz, 1H), 8.02 (dd, J= 9.0, 2.0 Hz, 1H), 7.91 (d, J= 9.0 Hz, 1H), 7.60 (s, 1H), 7.38-7.30 (m, 3H), 7.11 (dd, J= 8.0, 2.0 Hz, 1H), 5.97 (bs, 1H), 5.39 (s, 2H), 3.50-3.35 (m, 2H), 3.38 (d, J= 14.0 Hz, 1H), 3.34 (s, 3H), 3.19 (d, J= 14.0 Hz, 1H), 2.79-2.67 (m, 2H), 2.26 (s, 3H).
MS (ESI+) 452, 1.99($M^+$+1, detection time)

Example 136

2-(1-Benzyl-6-nitro-1H-indol-3-yl)-1,1,1-trifluoro-3-[(3-methoxypropyl)amino]-propan-2-ol

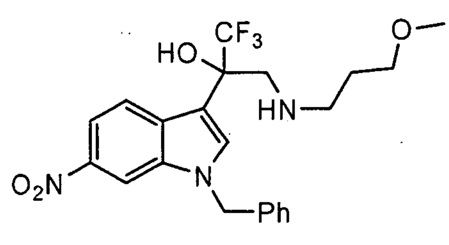

The title compound was obtained in a similar manner to the preparation of the compound of Example 1.
H-NMR (400 MHz, $CDCl_3$) δ 8.26 (d, J= 2.0 Hz, 1H), 8.03 (dd, J= 9.0, 2.0 Hz, 1H), 7.94 (d, J= 9.0 Hz, 1H), 7.53 (s, 1H), 7.35-7.33 (m, 3H), 7.13 (d, J= 7.9 Hz, 1H), 5.39 (s, 2H), 3.55 (d, J= 12.9 Hz, 1H), 3.43 (t, J= 6.0 Hz, 2H), 3.27 (s, 3H), 3.09 (d, J= 12.9 Hz, 1H), 2.83 (dt, J= 12.0, 6.2 Hz, 1H), 2.74 (dt, J= 12.0, 6.2 Hz, 1H), 1.74 (tt, J= 6.2, 6.0 Hz, 2H).
MS (ESI+) 452, 1.93($M^+$+1, detection time)

Example 137

3-(Benzylamino)-2-(1-benzyl-6-nitro-1H-indol-3-yl)-1,1,1-trifluoropropan-2-ol

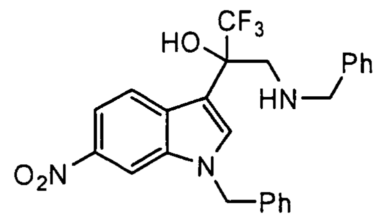

The title compound was obtained in a similar manner to the preparation of the compound of Example 1.
H-NMR (400 MHz, $CDCl_3$) δ 8.24 (d, J= 2.0 Hz, 1H), 7.96 (dd, J= 9.0, 2.0 Hz, 1H), 7.80 (d, J= 9.0 Hz, 1H), 7.49 (s, 1H), 7.34-7.32 (m, 6H), 7.24 (d, J= 8.2 Hz, 2H), 7.11 (d, J= 7.7 Hz, 1H), 5.36 (s, 2H), 3.87 (d, J= 13.3 Hz, 1H), 3.80 (d, J= 13.3 Hz, 1H), 3.50 (d, J= 12.9 Hz, 1H), 3.15 (d, J= 12.9 Hz, 1H).
MS (ESI+) 470, 2.03($M^+$+1, detection time)

Example 138

2-(1-Benzyl-6-nitro-1H-indol-3-yl)-1,1,1-trifluoro-3-[(1-phenylethyl)amino]-propan-2-ol

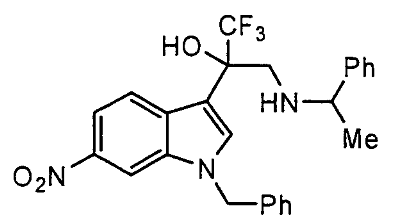

The title compound was obtained in a similar manner to the preparation of the compound of Example 1.
H-NMR (400 MHz, $CDCl_3$) δ 8.25 (d, J= 2.0 Hz, 1/2H), 8.20 (d, J= 2.0 Hz, 1/2H), 7.96 (dd, J= 9.0, 2.0 Hz, 1/2H), 7.86 (dd, J= 9.0, 2.0 Hz, 1H), 7.50-7.48 (m, 1H), 7.39-7.08 (m, 1/2H), 5.37 (s, 1H), 5.33 (s, 1H), 3.83 (q, J= 6.6 Hz, 1/2H), 3.71 (q, J= 6.6 Hz, 1/2H), 3.35 (d, J= 13.0 Hz, 1/2H), 3.33 (d, J= 13.0 Hz, 1/2H), 2.97 (d, J= 13.0 Hz, 1/2H), 1.41 (d, J= 66 Hz, 3/2H), 1.36 (d, J= 6.6 Hz, 3/2H). MS (ESI+) 484, 2.07($M^+$+1, detection time)

Example 139

2-(1-Benzyl-6-nitro-1H-indol-3-yl)-1,1,1-trifluoro-3-[(1-methyl-1-phenylethyl)-amino]propan-2-ol

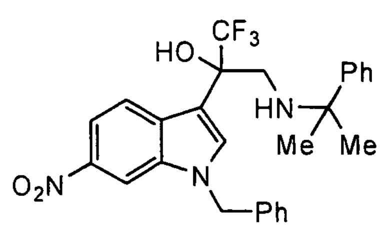

The title compound was obtained in a similar manner to the preparation of the compound of Example 1.

H-NMR (400 MHz, $CDCl_3$) δ 8.21 (d, J= 2.0 Hz, 1H), 7.89 (dd, J= 9.0, 2.0 Hz, 1H), 7.55 (d, J= 9.0 Hz, 1H), 7.41-7.31 (m, 9H), 7.10 (d, J= 7.8 Hz, 2H), 5.36 (s, 2H), 3.17 (d, J= 13.0 Hz, 1H), 2.81 (d, J= 13.0 Hz, 1H), 1.52 (s, 3H), 1.48 (s, 3H).
MS (ESI+) 498, 2.10($M^+$+1, detection time)

Example 140

2-(1-Benzyl-6-nitro-1H-indol-3-yl)-1,1,1-trifluoro-3-[(tetrahydrofuran-2-ylmethyl)amino]propan-2-ol

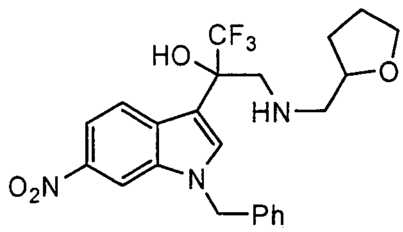

The title compound was obtained in a similar manner to the preparation of the compound of Example 1.
$^1$H-NMR (400 MHz, $CDCl_3$) δ 8.26 (d, J= 2.1 Hz, 1H), 8.02 (d, J= 9.0, 2.1 Hz, 1H), 7.94 (dd, J= 9.0, 3.1 Hz, 1H), 7.53 (s, 1H), 7.39-7.29 (m, 3H), 7.12 (dd, J= 8.0, 1.9 Hz, 2H), 5.39 (s, 2H), 4.02-3.90 (m, 1H), 3.85-3.68 (m, 2H), 3.55 (dd, J= 18.6, 13.0 Hz, 1H), 3.18 (dd, J= 18.6, 13.0 Hz, 1H), 2.82-2.64 (m, 2H), 2.01-1.81 (m, 3H), 1.60-1.42 (m, 1H).
MS (ESI+) 464, 1.95($M^+$+1, detection time)

Example 141

2-(1-Benzyl-6-nitro-1H-indol-3-yl)-1,1,1-trifluoro-3-[(2-furylmethyl)amino]-propan-2-ol

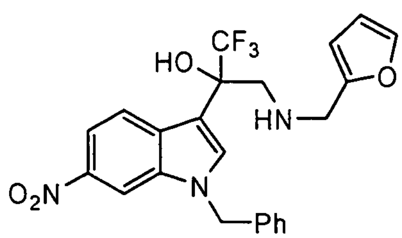

The title compound was obtained in a similar manner to the preparation of the compound of Example 1.
$^1$H-NMR (400 MHz, $CDCl_3$) δ 8.25 (d, J= 2.0 Hz, 1H), 8.00 (dd, J= 9.0, 2.0 Hz, 1H), 7.85 (d, J= 9.0 Hz, 1H), 7.50 (s, 1H), 7.39-7.30 (m, 4H), 7.12 (dd, J= 7.9, 2.0 Hz, 2H), 6.32 (dd, J= 3.0, 1.9 Hz, 1H), 6.13 (d, J= 3.0 Hz, 1H), 5.38 (s, 1H), 3.84 (d, J= 14.8 Hz, 1H), 3.78 (d, J= 14.8 Hz, 1H), 3.47 (d, J= 13.0 Hz, 1H), 3.16 (d, J= 13.0 Hz, 1H).
MS (ESI+) 460, 1.98($M^+$+1, detection time)

Example 142

3-[Benzyl(methyl)amino]-2-(1-benzyl-6-nitro-1H-indol-3-yl)-1,1,1-trifluoropropan-2-ol

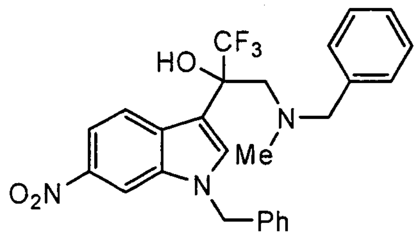

The title compound was obtained in a similar manner to the preparation of the compound of Example 1.
$^1$H-NMR (400 MHz, $CDCl_3$) δ 8.24 (d, J= 1.9 Hz, 1H), 8.02 (dd, J= 9.0, 1.9 Hz, 1H), 7.88 (d, J= 9.0 Hz, 1H), 7.59 (s, 1H), 7.36-7.24 (m, 6H), 7.20 (dd, J= 7.6, 1.8 Hz, 2H), 7.10-7.01 (m, 2H), 5.37 (s, 2H), 3.57 (s, 2H), 3.35 (d, J= 13.4 Hz, 1H), 3.24 (d, J= 13.4 Hz, 1H), 2.20 (s, 3H).
MS (ESI+) 484, 2.17($M^+$+1, detection time)

Example 143

2-(1-Benzyl-6-nitro-1H-indol-3-yl)-1,1,1-trifluoro-3-[4-(2-methoxyphenyl)-piperazin-1-yl]propan-2-ol

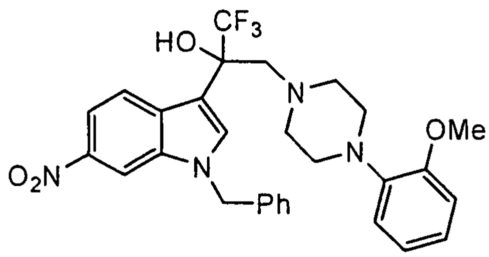

The title compound was obtained in a similar manner to the preparation of the compound of Example 1.
$^1$H-NMR (400 MHz, $CDCl_3$) δ 8.28 (d, J= 2.0 Hz, 1H), 8.05 (dd, J= 9.0, 2.0 Hz, 1H), 7.95 (d, J= 9.0 Hz, 1H), 7.59 (s, 1H), 7.38-7.28 (m, 3H), 7.11 (dd, J= 7.7, 2.0 Hz, 2H), 7.06-6.97 (m, 1H), 6.97-6.82 (m, 1H), 5.41 (s, 2H), 3.83 (s, 3H), 3.35 (d, J= 13.4 Hz, 1H), 3.23 (d, J= 13.4 Hz, 1H), 3.04 (bs, 4H), 2.79 (bs, 4H). MS (ESI+) 555, 2.30($M^+$+1, detection time)

Example 144

2-(1-Benzyl-6-nitro-1H-indol-3-yl)-1,1,1-trifluoro-3-[4-(3-methoxyphenyl)-piperazin-1-yl]propan-2-ol

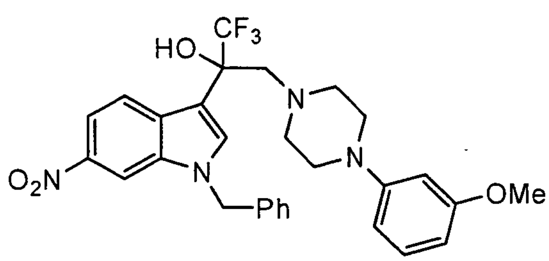

The title compound was obtained in a similar manner to the preparation of the compound of Example 1.
$^1$H-NMR (400 MHz, $CDCl_3$) δ 8.29 (d, J= 2.0 Hz, 1H), 8.04 (dd, J= 9.0, 2.0 Hz, 1H), 7.93 (d, J= 9.0 Hz, 1H), 7.58 (s, 1H), 7.38-7.29 (m, 3H), 7.16 (dd, J= 8.2, 8.2 Hz, 1H), 7.12 (dd, J= 7.7, 2.1 Hz, 2H), 6.47 (d, J= 8.2 Hz, 1H), 6.43 (d, J= 8.2 Hz, 1H), 6.43-6.38 (m, 1H), 5.63 (bs, 1H), 5.41 (s, 2H), 3.78 (s, 3H), 3.30 (d, J= 13.6 Hz, 1H), 3.18 (d, J= 13.6 Hz, 1H), 3.18-3.05 (m, 4H), 2.78-2.60 (m, 4H). MS (ESI+) 555, 2.32($M^+$+1, detection time)

Example 145

2-(1-Benzyl-6-nitro-1H-indol-3-yl)-1,1,1-trifluoro-3-[4-(4-methoxyphenyl)-piperazin-1-yl]propan-2-ol

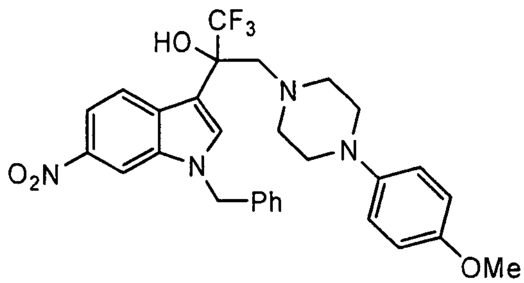

The title compound was obtained in a similar manner to the preparation of the compound of Example 1.
$^1$H-NMR (400 MHz, $CDCl_3$) δ 8.28 (d, J= 1.9 Hz, 1H), 8.04 (dd, J= 9.0, 1.9 Hz, 1H), 7.94 (d, J= 9.0 Hz, 1H), 7.58 (s, 1H), 7.38-7.29 (m, 3H), 7.11 (dd, J= 7.6, 1.9 Hz, 2H), 6.83 (s, 4H), 5.68 (bs, 1H), 5.41 (s, 2H), 3.76 (s, 3H), 3.30 (d, J= 13.6 Hz, 1H), 3.18 (d, J= 13.6 Hz, 1H), 3,08-2.94 (m, 4H), 2.79-2.64 (m, 4H). MS (ESI+) 555, 2.34($M^+$+1, detection time)

Example 146

2-(1-Benzyl-6-nitro-1H-indol-3-yl)-1,1,1-trifluoro-3-(4-pyridin-4-ylpiperazin-1-yl)propan-2-ol)

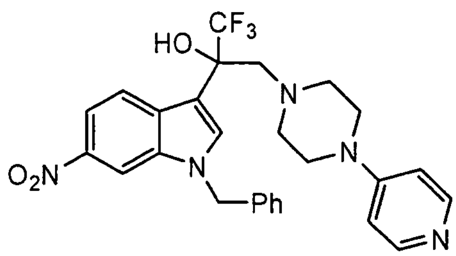

The title compound was obtained in a similar manner to the preparation of the compound of Example 1.
$^1$H-NMR (400 MHz, $CDCl_3$) δ 8.30 (d, J= 2.0 Hz, 1H), 8.26 (d, J= 5.8 Hz, 2H), 8.04 (d, J= 9.0, 2.0 Hz, 1H), 7.92 (d, J= 9.0 Hz, 1H), 7.58 (s, 1H), 7.39-7.30 (m, 3H), 7.12 (dd, J= 7.7, 2.4 Hz, 2H), 6.61 (d, J= 5.8 Hz, 2H), 5.41 (s, 2H), 3.35-3.22 (m, 4H), 3.32 (d, J= 13.7 Hz, 1H), 3.18 (d, J= 13.7 Hz, 1H), 2.79-2.60 (m, 4H).
MS (ESI+) 526, 2.04($M^+$+1, detection time)

Example 147

2-(1-Benzyl-6-nitro-1H-indol-3-yl)-1,1,1-trifluoro-3-(4-pyridin-2-ylpiperazin-1-yl)propan-2-ol

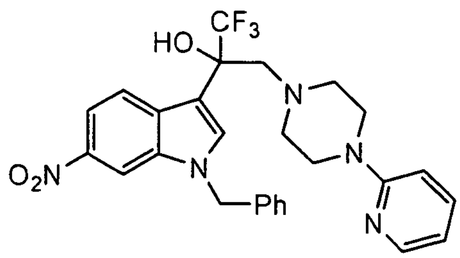

The title compound was obtained in a similar manner to the preparation of the compound of Example 1.
$^1$H-NMR (400 MHz, $CDCl_3$) δ 8.28 (d, J= 2.0 Hz, 1H), 8.20-8.13 (m, 1H), 8.03 (dd, J= 9.0, 2.0 Hz, 1H), 7.92 (d, J= 9.0 Hz, 1H), 7.58 (s, 1H), 7.52-7.42 (m, 1H), 7.48-7.30 (m, 3H), 7.11 (dd, J= 7.8, 1.7 Hz, 2H), 6.64 (dd, J= 7.8, 7.8 Hz, 1H), 6.59 (d, J= 7.8 Hz, 1H), 5.67 (bs, 1H), 5.40 (s, 2H), 3.61-3.38 (m, 4H), 3.31 (d, J= 13.7 Hz, 1H), 3.17 (d, J= 13.7 Hz, 1H), 2.78-2.57 (m, 1H).
MS (ESI+) 526, 2.12($M^+$+1, detection time)

Example 148

2-(1-Benzyl-6-nitro-1H-indol-3-yl)-1,1,1-trifluoro-3-(4-pyrimidin-2-ylpiperazin-1-yl)propan-2-ol

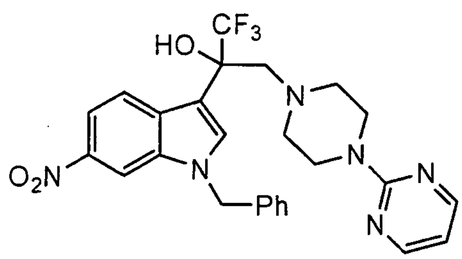

The title compound was obtained in a similar manner to the preparation of the compound of Example 1.
$^1$H-NMR (400 MHz, $CDCl_3$) δ 8.30-8.28 (m, 3H), 8.04 (dd, J= 9.0, 2.0 Hz, 1H), 7.91 (d, J= 9.0 Hz, 1H), 7.59 (s, 1H), 7.40-7.29 (m, 3H), 7.11 (dd, J= 8.0, 1.8 Hz, 2H), 6.51 (dd, J= 4.7, 4.7 Hz, 1H), 5.67 (bs, 1H), 5.41 (s, 2H), 3.90-3.65 (m, 4H), 3.30 (d, J= 13.7 Hz, 1H), 3.17 (d, J= 13.7 Hz, 1H)
MS (ESI+) 527, 2.08($M^+$+1, detection time)

Example 149

2-(1-Benzyl-6-nitro-1H-indol-3-yl)-1,1,1-trifluoro-3-[4-(4-fluorophenyl)piperazin-1-yl]propan-2-ol

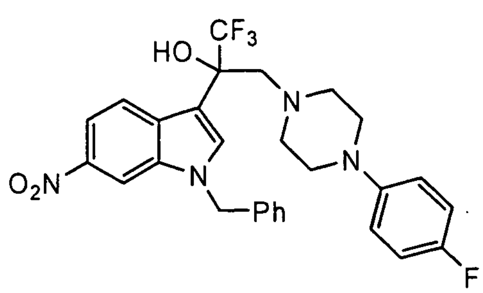

The title compound was obtained in a similar manner to the preparation of the compound of Example 1.

$^1$H-NMR (400 MHz, $CDCl_3$) δ 8.28 (d, J= 2.0 Hz, 1H), 8.04 (dd, J= 9.0, 2.0 Hz, 1H), 7.93 (d, J= 9.0 Hz, 1H), 7.58 (s, 1H), 7.39-7.28 (m, 3H), 7.11 (dd, J= 7.7, 2.2 Hz, 2H), 7.00-6.90 (m, 2H), 6.86-6.78 (m, 2H), 5.61 (bs, 1H), 5.40 (s, 2H), 3.31 (d, J= 13.7 Hz, 1H), 3.18 (d, J= 13.7 Hz, 1H), 3.11-2.97 (m, 4H), 2.80-2.63 (m, 4H).
MS (ESI+) 543, 2.47($M^+$+1, detection time)

Example 150

2-(1-Benzyl-6-nitro-1H-indol-3-yl)-1,1,1-trifluoro-3-(1H-pyrazol-1-yl)propan-2-ol

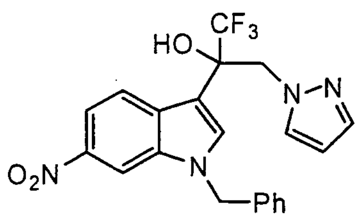

The title compound was obtained in a similar manner to the preparation of the compound of Example 1.
$^1$H-NMR (400 MHz, $CDCl_3$) δ 8.22 (d, J= 2.0 Hz, 1H), 8.06 (dd, J= 9.0, 2.0 Hz, 1H), 7.96 (d, J= 9.0 Hz, 1H), 7.54-7.48 (m, 2H), 7.33-7.28 (m, 3H), 7.17 (d, J= 2.1 Hz, 1H), 6.99-6.89 (m, 2H), 6.56 (bs, 1H), 6.15 (dd, J= 2.1, 2.1 Hz, 1H), 5.36 (s, 2H), 4.83 (d, J= 13.6 Hz, 1H), 4.78 (d, J= 13.6 Hz, 1H).
MS (ESI+) 431, 2.39($M^+$+1, detection time)

Example 151

1'-[2-(1-Benzyl-6-nitro-1H-indol-3-yl)-3,3,3-trifluoro-2-hydroxypropyl]spiro[1-benzofuran-3,4'-piperidin]-2-one

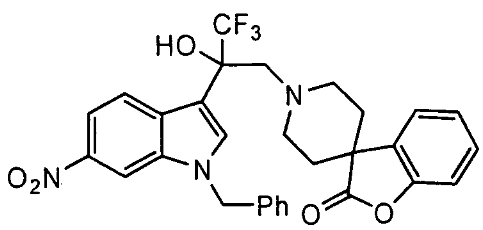

The title compound was obtained in a similar manner to the preparation of the compound of Example 1.
$^1$H-NMR (400 MHz, $CDCl_3$) δ 8.27 (d, J= 2.0 Hz, 1H), 8.00 (dd, J= 9.0, 2.0 Hz, 1H), 7.92 (d, J= 9.0 Hz, 1H), 7.60 (s, 1H), 7.32-7.30 (m, 5H), 7.10 (dd, J= 8.0, 8.0 Hz, 1H), 7.09-7.06 (m, 3H), 5.40 (s, 2H), 4.72 (bs, 1H), 3.37 (d, J= 13.7 Hz, 1H), 3.29 (d, J= 13.7 Hz, 1H), 3.16-3.13 (m, 1H), 3.05-2.99 (m, 1H), 2.86-2.81 (m, 1H), 2.67-2.64 (m, 1H), 1.98-1.85 (m, 4H).

Example 152

2-(1-Benzyl-1H-indol-3-yl)-1,1,1-trifluoro-3-morpholin-4-yl-3-oxopropan-2-ol

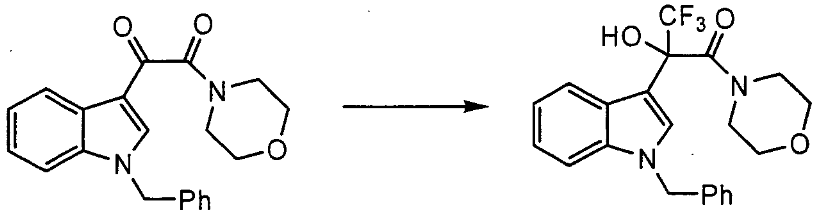

To a solution of 1-(1-benzyl-1H-indol-3-yl)-2-morpholin-4-yl-2-oxoethanone (139 mg), (trifluoromethyl)trimethylsilane (89 μl) in N,N-dimethylformamide (1 ml) was added lithium acetate (2.6 mg) at 0°C, and the mixture was stirred at the same temperature for 30 minutes. Then, the mixture was further stirred at 25°C for one hour, and thereto was added a 1.0M tetrabutylammonium fluoride (1.0ml), and the mixture was further stirred for 3 hours. The reaction solution was concentrated under reduced pressure, and the obtained residue was purified by silica gel column chromatography to give the title compound (84 mg).
$^1$H-NMR (400 MHz, $CDCl_3$) δ 7.45 (d, J= 8.0 Hz, 1H), 7.34 (d, J= 1.6 Hz, 1H), 7.31-7.24 (m, 4H), 7.24-7.18 (m, 1H), 7.14-7.09 (m, 1H), 7.09-7.01 (m, 2H), 6.19 (bs, 1H), 5.37 (d, J= 16.0 Hz, 1H), 5.31 (d, J= 16.0 Hz, 1H), 3.98-3.83 (m, 1H), 3.71-3.49 (m, 3H), 3.37-3.24 (m, 1H), 3.24-3.11 (m, 1H), 3.11-2.99 (m, 1H), 2.71-2.57 (m, 1H).
MS (ESI+) 401, 2.63($M^+$+1, detection time)

Example 153

{1-[2-(1-Benzyl-6-nitro-1H-indol-3-yl)-3,3,3-trifluoro-2-methoxypropyl]piperidin-4-yl}(phenyl)methanone

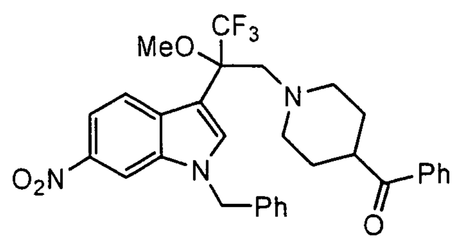

To a solution of the compound of Example 67 (110 mg) in tetrahydrofuran (5 ml) was added sodium hydride (55 %, 9.6 mg) at 0°C. Then, to the mixture was added methyl iodide (13.7 ul), and the mixture was stirred at 70°C for 10 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (25 mg).
MS (ESI+) 566, 2.74($M^+$+1, detection time)

Example 154

2-(1-Benzyl-6-nitro-1H-indol-3-yl)-1,1,1-trifluoro-3-[(2-methoxybenzyl)amino]-propan-2-ol

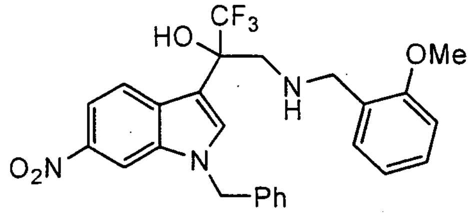

The title compound was obtained in a similar manner to the preparation of the compound of Example 1.
MS (ESI+) 500, 2.05($M^+$+1, detection time)

Example 155

2-(1-Benzyl-6-nitro-1H-indol-3-yl)-1,1,1-trifluoro-3-[(3-methoxybenzyl)amino]-propan-2-ol

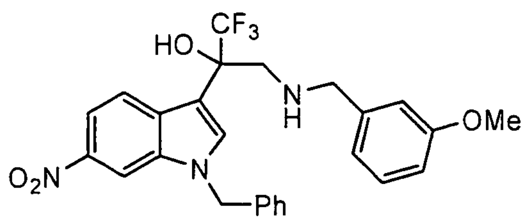

The title compound was obtained in a similar manner to the preparation of the compound of Example 1.
$^1$H-NMR (400 MHz, $CDCl_3$) δ 8.25 (d, J= 2.0 Hz, 1H), 7.98 (d, J= 9.0, 2.0 Hz, 1H), 7.80 (d, J= 9.0 Hz, 1H), 7.49 (s, 1H), 7.38-7.30 (m, 3H), 7.26 (s, 1H), 7.11 (dd, J= 7.8, 2.2 Hz, 2H), 6.87-6.80 (m, 2H), 6.80-6.75 (m, 1H), 5.37 (s, 2H), 3.84 (d, J= 13.5 Hz, 1H), 3.79 (s, 3H), 3.78 (d, J= 13.5 Hz, 1H), 3.50 (d, J= 12.9 Hz, 1H), 3.15 (d, J= 12.9 Hz, 1H).
MS (ESI+) 500, 2.04($M^+$+1, detection time)

Example 156

2-(1-Benzyl-6-nitro-1H-indol-3-yl)-1,1,1-trifluoro-3-[(4-methoxybenzyl)amino]-propan-2-ol

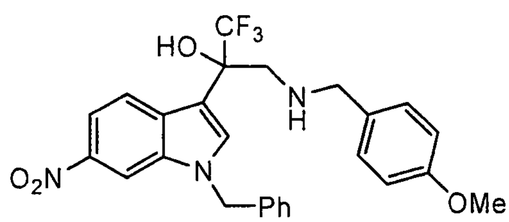

The title compound was obtained in a similar manner to the preparation of the compound of Example 1.
$^1$H-NMR (400 MHz, $CDCl_3$) δ 8.24 (d, J= 1.9 Hz, 1H), 7.97 (dd, J= 9.0, 1.9 Hz, 1H), 7.78 (d, J= 9.0 Hz, 1H), 7.49 (s, 1H), 7.38-7.29 (m, 3H), 7.16 (d, J= 8.6 Hz, 2H), 7.11 (dd, J= 7.7, 2.1 Hz, 2H), 6.86 (d, J= 8.6 Hz, 2H), 5.37 (s, 2H), 3.81 (s, 3H), 3.80 (d, J= 13.1 Hz, 1H), 3.73 (d, J= 13.1 Hz, 1H), 3.48 (d, J= 12.9 Hz, 1H), 3.14 (d, J= 12.9 Hz, 1H). MS (ESI+) 500, 2.04($M^+$+1, detection time)

Example 157

2-(1-Benzyl-6-nitro-1H-indol-3-yl)-1,1,1-trifluoro-3-[(2-phenylethyl)amino]-propan-2-ol

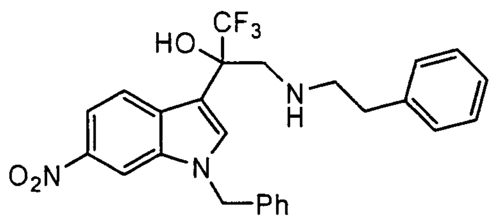

The title compound was obtained in a similar manner to the preparation of the compound of Example 1. $^1$H-NMR (400 MHz, $CDCl_3$) δ 8.25 (d, J= 2.0 Hz, 1H), 8.01 (dd, J= 9.0, 2.0 Hz, 1H), 7.90 (d, J= 9.0 Hz, 1H), 7.47 (s, 1H), 7.39-7.17 (m, 6H), 7.17-7.08 (m, 4H), 5.37 (s, 2H), 3.51 (d, J= 13.0 Hz, 1H), 3.11 (d, J= 13.0 Hz, 1H), 3.04-2.93 (m, 1H), 2.93-2.82 (m, 1H), 2.80-2.72 (m, 2H).
MS (ESI+) 484, 2.06($M^+$+1, detection time)

Example 158

2-(1-Benzyl-6-nitro-1H-indol-3-yl)-1,1,1-trifluoro-3-(1H-imidazol-1-yl)propan-2-ol

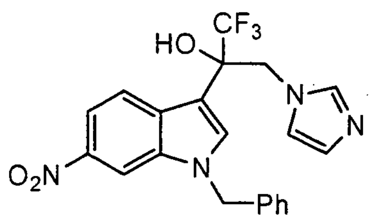

The title compound was obtained in a similar manner to the preparation of the compound of Example 1.
MS (ESI+) 431, 2.38($M^+$+1, detection time)

Example 159

2-(1-Benzyl-6-nitro-1H-indol-3-yl)-1,1,1-trifluoro-3-(4-pyrazin-2-ylpiperazin-1-yl)propan-2-ol

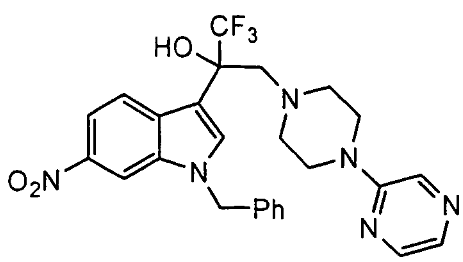

The title compound was obtained in a similar manner to the preparation of the compound of Example 1. $^1$H-NMR (400 MHz, $CDCl_3$) δ 8.29 (d, J= 2.2 Hz, 1H), 8.07 (d, J= 12.0 Hz, 2H), 8.04 (dd, J= 9.0, 2.0 Hz, 1H), 7.92 (d, J= 9.0 Hz, 1H), 7.87 (d, J= 2.2 Hz, 1H), 7.59 (s, 1H), 7.39-7.29 (m, 3H), 7.11 (dd, J= 7.8, 2.0 Hz, 2H), 5.41 (s, 2H), 3.65-3.45 (m, 4H), 3.32 (d, J= 13.7 Hz, 1H), 3.19 (d, J= 13.7 Hz, 1H), 2.89-2.59 (m, 4H).
MS (ESI+) 527, 2.29($M^+$+1, detection time)

Example 160

{1-[2-(1-Benzyl-6-rutro-1H-indol-3-yl)-3,3,3-trifluoro-2-hydroxypropyl]-1H-pyrrol-3-yl}(4-chlorophenyl)methanone

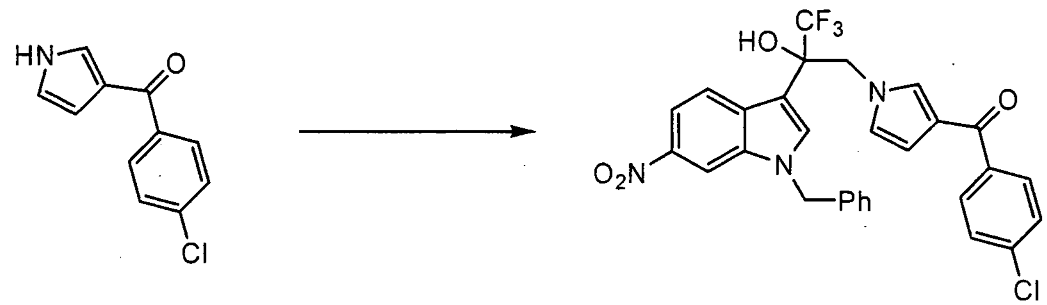

To a solution of (4-chlorophenyl)(1H-pyrrol-3-yl)methanone (46 mg) in tetrahydrofuran (2 ml) was added potassium t-butoxide (26.5 mg), and the mixture was stirred at 25˚C for one hour. To the reaction solution was added 1-benzyl-6-nitro-3-[2-(trifluoromethyl)oxiran-2-yl]-1H-indole (72 mg) obtained in Reference Example 3, and the mixture was stirred at 25˚C for 3 hours. Then, the mixture was further stirred at 60˚C for 8 hours. The reaction solution was concentrated under reduced pressure, and the obtained residue was purified by silica gel column chromatography to give the title compound (52 mg).
$^1$H-NMR (400 MHz, $CDCl_3$) δ 8.26 (d, J= 1.9 Hz, 1H), 8.01 (dd, J= 9.0, 1.9 Hz, 1H), 7.77 (d, J= 9.0 Hz. 1H), 7.49 (s, 1H), 7.37-7.21 (m, 5H), 7.11 (s, 1H), 7.02-6.94 (m, 2H), 6.42 (dd, J= 2.6, 1.6 Hz, 1H), 6.34 (dd, J= 2.6, 2.3 Hz, 1H), 5.36 (s, 1H), 4.64 (d, J= 14.4 Hz, 1H), 4.60 (d, J= 14.4 Hz, 1H), 4.03 (bs, 1H).
MS (ESI+) 568, 2.53($M^+$+1, detection time)

Example 161

{1-[2-(1-Benzyl-6-nitro-1H-indol-3-yl)-3,3,3-trifluoro-2-hydroxypropyl]-1H-pyrrol-2-yl}(4-methylphenyl)methanone

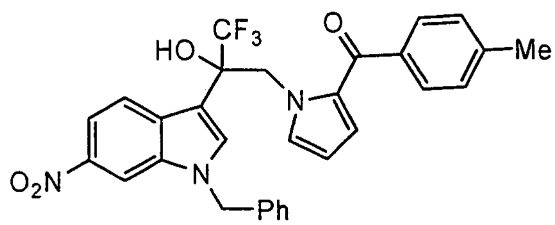

The title compound was obtained in a similar manner to the preparation of the compound of Example 160 (Yield: 45 mg).
$^1$H-NMR (400 MHz, $CDCl_3$) δ 8.27 (d, J= 2.0 Hz, 1H), 8.05 (dd, J= 9.0, 2.0 Hz, 1H), 7.83 (d, J= 9.0 Hz, 1H), 7.75 (d, J= 9.1 Hz, 2H), 7.67 (d, J= 9.1 Hz, 2H), 7.37-7.21 (m, 4H), 7.08-6.98 (m, 2H), 6.74 (dd, J= 4.0, 1.6 Hz, 1H), 6.10-6.05 (m 1H), 5.93 (dd, J= 4.0, 2.6 Hz, 1H), 5.38 (s, 2H), 5.27 (d, J= 14.4 Hz, 1H), 4.74 (d, J= 14.4 Hz, 1H), 2.45 (s, 3H).
MS (ESI+) 548, 2.71($M^+$+1, detection time)

Example 162

1-Benzyl-3-(2,2,2-trifluoro-1-hydroxy-1-{[4-(2-methoxyphenoxy)piperidin-1-yl]-methyl}ethyl)-1H-indole-6-carbonitrile

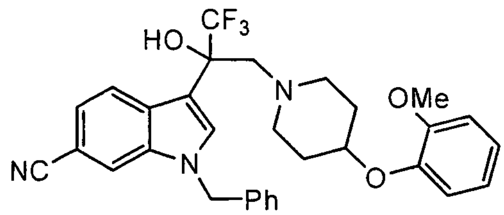

The title compound was obtained in a similar manner to the preparation of the compound of Example 1.
$^1$H-NMR (400 MHz, $CDCl_3$) δ 7.90 (d, J= 8.4 Hz, 1H), 7.58 (s, 1H), 7.25 (s, 1H), 7.35 (d, J= 8.4 Hz, 1H), 7.34-7.28 (m, 3H), 7.10-7.01 (m, 2H), 6.98-6.91 (m, 1H), 6.91-6.80 (m, 3H), 5.33 (s, 2H), 4.25 (bs, 1H), 3.83 (s, 3H), 3.24 (d, J= 13.6 Hz, 1H), 3.15 (d, J= 13.6 Hz, 1H), 2.90-2.72 (m, 2H), 2.52-2.39 (m, 2H), 1.98-1.77 (m, 4H).
MS (ESI+) 550, 2.09 ($M^+$+1, detection time)

Example 163

3-{1-[(4-Benzoylpiperidin-1-yl)methyl]-2,2,2-trifluoro-1-hydroxyethyl}-1-benzyl-1H-indole-6-carbonitrile

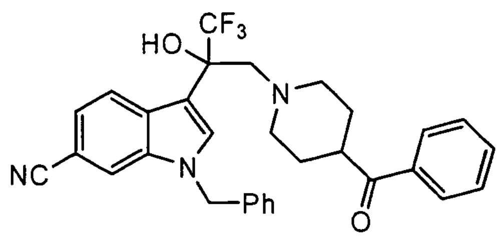

The title compound was obtained in a similar manner to the preparation of the compound of Example 1.
$^1$H-NMR (400 MHz, $CDCl_3$) δ 7.93-7.85 (m, 4H), 7.63-7.42 (m, 6H), 7.35 (dd, J= 9.0, 2.0 Hz, 1H), 7.29 (s, 1H), 7.07 (dd, J= 7.5, 2.1 Hz, 2H), 5.69 (bs, 1H), 5.34 (s, 1H), 3.25 (d, J= 12.6 Hz, 1H), 3.24-3.13 (m, 1H), 3.15 (d, J= 12.6 Hz, 1H), 3.05-2.95 (m, 1H), 2.70-2.57 (m, 2H), 2.38-2.25 (m, 1H), 1.92-1.63 (m, 4H).

Example 164

1-[2-(1-Benzyl-6-nitro-1H-indol-3-yl)-3,3,3-trifluoro-2-hydroxypropyl]pyrrolidin-3-ol

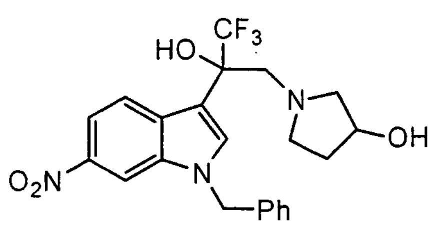

The title compound was obtained in a similar manner to the preparation of the compound of Example 1.
MS (ESI+) 450, 2.29 ($M^+$+1, detection time)

Example 165

1-[2-(1-Benzyl-6-nitro-1H-indol-3-yl)-3,3,3-trifluoro-2-hydroxypropyl]piperidin-3-ol

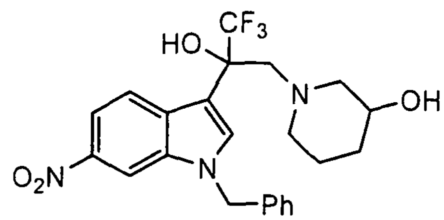

The title compound was obtained in a similar manner to the preparation of the compound of Example 1.
MS (ESI+) 464, 1.98($M^{+}$+1, detection time)

Example 166

2-(1-Benzyl-6-nitro-1H-indol-3-yl)-1,1,1-trifluoro-3-1,3-thiazolidin-3-yl)propan-2-ol

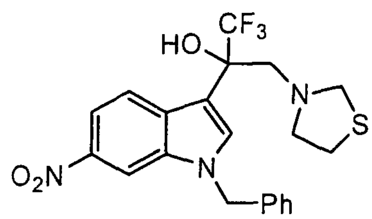

The title compound was obtained in a similar manner to the preparation of the compound of Example 1.
MS (ESI+) 452, 2.49 ($M^{+}$+1, detection time)

Example 167

tert-Butyl (2-{[2-(1-benzyl-6-nitro-1H-indol-3-yl)-3,3,3-trifluoro-2-hydroxypropyl]amino}ethyl)carbamate

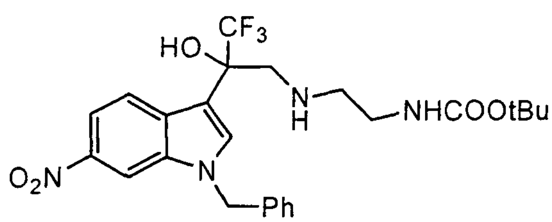

The title compound was obtained in a similar manner to the preparation of the compound of Example 1.
MS (ESI+) 523, 2.10 ($M^{+}$+1, detection time)

Example 168

tert-Butyl (3-{[2-(1-benzyl-6-nitro-1H-indol-3-yl)-3,3,3-trifluoro-2-hydroxypropyl]amino}propyl)carbamate

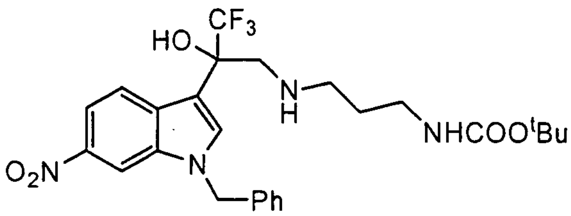

The title compound was obtained in a similar manner to the preparation of the compound of Example 1.
MS (ESI+) 537, 2.08 ($M^+$+1, detection time)

Example 169

Ethyl 4-{[2-(1-benzyl-6-nitro-1H-indol-3-yl)-3,3,3-trifluoro-2-hydroxypropyl]-amino}butanoate
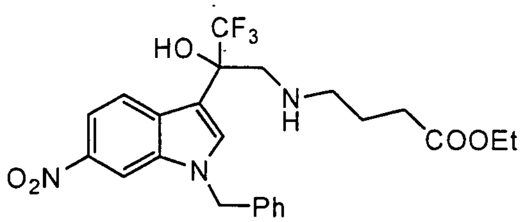

The title compound was obtained in a similar manner to the preparation of the compound of Example 1.
MS (ESI+) 494, 1.99($M^+$+1, detection time)

Example 170

Ethyl 1-[2-(1-benzyl-6-nitro-1H-indol-3-yl)-3,3,3-trifluoro-2-hydroxypropyl]-piperidine-2-carboxylate

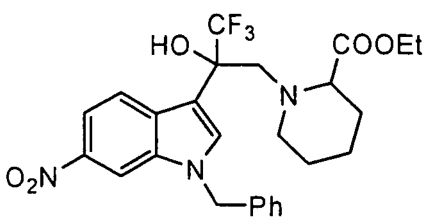

The title compound was obtained in a similar manner to the preparation of the compound of Example 1.
MS (ESI+) 520, 2.71($M^+$+1, detection time)

Example 171

Ethyl N-[2-(1-benzyl-6-nitro-1H-indol-3-yl)-3,3,3-trifluoro-2-hydroxypropyl]-β-alaninate
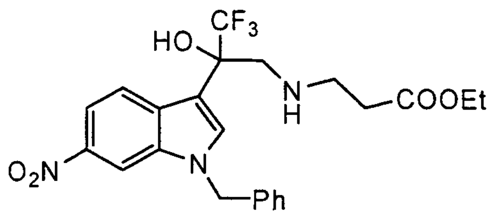

The title compound was obtained in a similar manner to the preparation of the compound of Example 1.
MS (ESI+) 480, 1.99($M^{+}$+1, detection time)

Example 172

Ethyl N-[2-(1-benzyl-6-nitro-1H-indol-3-yl)-3,3,3-trifluoro-2-hydroxypropyl]-glycinate

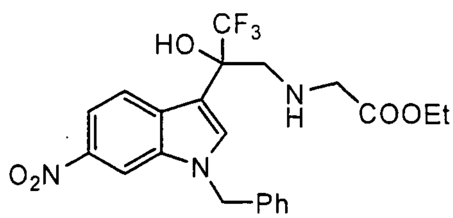

The title compound was obtained in a similar manner to the preparation of the compound of Example 1.
MS (ESI+) 466, 2.13($M^{+}$+1, detection time)

Example 173

Ethyl 5-{[2-(1-benzyl-6-nitro-1H-indol-3-yl)-3,3,3-trifluoro-2-hydroxypropyl]-amino}pentanoate

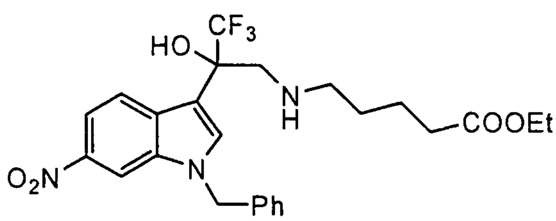

The title compound was obtained in a similar manner to the preparation of the compound of Example 1.
MS (ESI+) 508, 2.01($M^{+}$+1, detection time)

Example 174

1-[2-(1-Benzyl-6-nitro-1H-indol-3-yl)-3,3,3-trifluoro-2-hydroxypropyl]piperidine-2-carboxylic acid

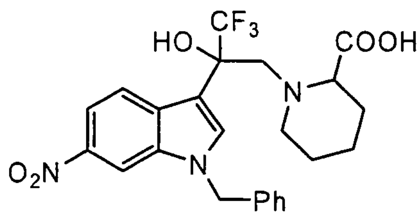

The title compound was obtained from the compound of Example 170 as a starting compound in a similar manner to Example 4.
MS (ESI+) 492, 2.13($M^{+}$+1, detection time)

Example 175

1-[2-(1-Benzyl-6-nitro-1H-indol-3-yl)-3,3,3-trifluoro-2-hydroxypropyl]piperidine-2-carboxylic acid

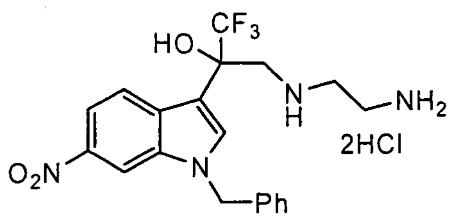

The title compound was obtained from the compound of Example 167 as a starting compound in a similar manner to Example 101.
MS (ESI+) 423, 2.25($M^{+}$+1, detection time)

Example 176

3-[(3-Aminopropyl)amino]-2-(1-benzyl-6-nitro-1H-indol-3-yl)-1,1,1-trifluoropropan-2-ol dihydrochloride

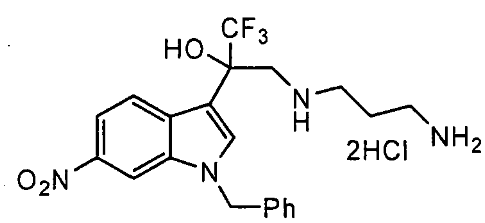

The title compound was obtained from the compound of Example 168 as a starting compound in a similar manner to Example 101.
MS (ESI+) 437, 2.13($M^{+}$+1, detection time)

Example 177

Methyl 4-({1-[2-(1-benzyl-6-nitro-1H-indol-3-yl)-3,3,3-trifluoro-2-hydroxypropyl]-piperidin-4-yl}oxy)benzoate
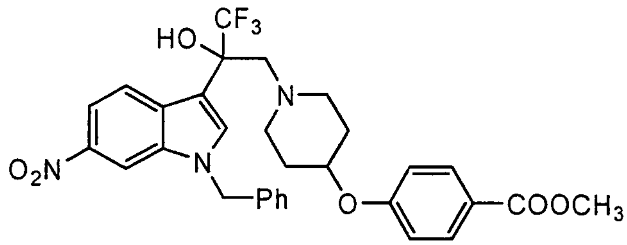

The title compound was obtained in a similar manner to the preparation of the compound of Example 1.
MS (ESI+) 598, 2.25($M^+$+1, detection time)

Example 178

4-({1-[2-(1-Benzyl-6-nitro-1H-indol-3-yl)-3,3,3-trifluoro-2-hydroxypropyl]-piperidin-4-yl}oxy)benzoic acid

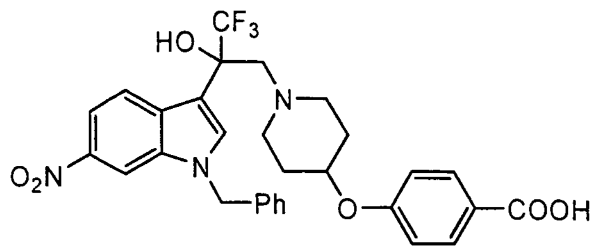

The title compound was obtained from the compound of Example 177 in a similar manner to Example 4.
$^1$H-NMR (400 MHz, DMSO) δ 9.40 (bs, 1H), 8.14-7.91 (m, 4H), 7.87 (d, J= 7.4 Hz, 2H), 7.38-7.13 (m, 4H), 7.13-6.90 (m, 2H), 5.70 (d, J= 16.0 Hz, 1H), 5.63 (d, J= 16.0 Hz, 1H), 4.88-2.88 (m, 9H), 2.30-1.60 (m, 2H).
MS (ESI+) 584, 2.08($M^+$+1, detection time)

Example 179

Ethyl [4-({1-[2-(1-benzyl-6-nitro-1H-indol-3-yl)-3,3,3-trifluoro-2-hydroxypropyl]-piperidin-4-yl}oxy)phenyl]acetate)

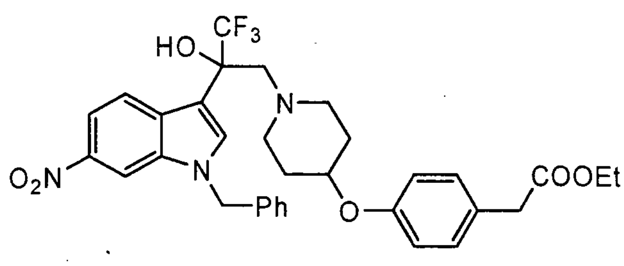

The title compound was obtained in a similar manner to the preparation of the compound of Example 1.
MS (ESI+) 626, 2.27($M^+$+1, detection time)

Example 180

[4-({1-[2-(1-Benzyl-6-nitro-1H-indol-3-yl)-3,3,3-trifluoro-2-hydroxypropyl]-piperidin-4-yl}oxy)phenyl]acetic acid

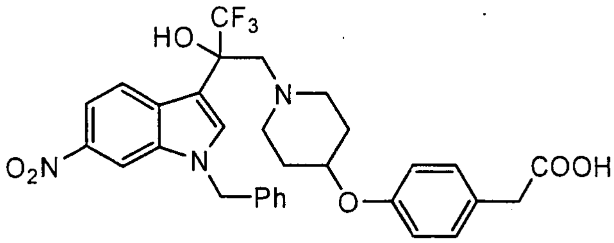

The title compound was obtained from the compound of Example 179 in a similar manner to Example 4.
$^1$H-NMR (400 MHz, DMSO) δ 12.35 (bs, 1H), 8.69 (bs, 1H), 8.16 (bs, 1H), 8.09 (bs, 1H), 7.50-7.16 (m, 6H), 7.08-6.85 (m, 2H), 5.80 (d, J= 15.9 Hz, 1H), 5.73 (d J= 15.9 Hz, 1H), 4.80-4.15 (m, 2H), 4.15-3.85 (m, 1H), 3.70-3.35 (m, 6H), 3.35-3.03 (m, 2H), 2.38-1.70 (m, 4H).
MS (ESI+) 598, 2.07($M^+$+1, detection time)

Example 181

Ethyl 3-[4-({1-[2-(1-benzyl-6-nitro-1H-indol-3-yl)-3,3,3-trifluoro-2-hydroxypropyl]piperidin-4-yl}oxy)phenyl]propanoate

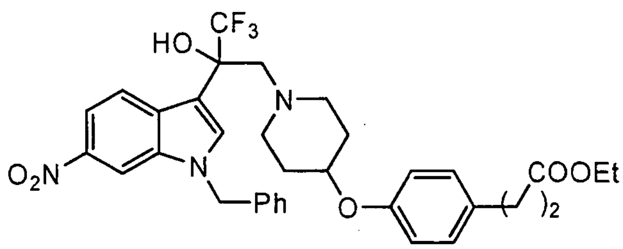

The title compound was obtained in a similar manner to the preparation of the compound of Example 1.
MS (ESI+) 640, 2.30($M^+$+1, detection time)

Example 182

3-[4-({1-[2-(1-Benzyl-6-nitro-1H-indol-3-yl)-3,3,3-trifluoro-2-hydroxypropyl]-piperidin-4-yl}oxy)phenyl]propanoic acid

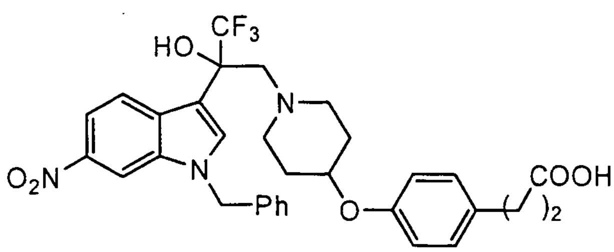

The title compound was obtained from the compound of Example 181 in a similar manner to Example 4.
$^1$H-NMR (400 MHz, DMSO) δ 12.15 (bs, 1H), 8.59 (bs, 1H), 8.08 (d, J= 8.8 Hz, 2H), 7.98 (d, J= 8.8 Hz, 2H), 7.40-7.18 (m, 3H), 7.13 (d, J= 7.9 Hz, 1H), 6.85 (bs, 3H), 5.70 (d, J= 16.0 Hz, 1H), 5.64 (d, J= 16.0 Hz, 1H), 4.70-1.48 (m, 11H), 2.74 (t, J= 7.4 Hz, 2H), 2.51 (t, J= 7.4 Hz, 2H).
MS (ESI+) 612, 2.11($M^+$+1, detection time)

Example 183

2-(1-Benzyl-6-nitro-1H-indol-3-yl)-1,1,1-trifluoro-3-[4-(methylsulfonyl)piperidin-1-yl]propan-2-ol

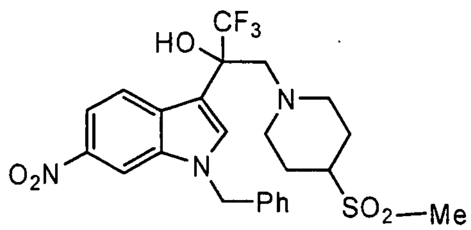

The title compound was obtained in a similar manner to the preparation of the compound of Example 1.
$^1$H-NMR (400 MHz, $CDCl_3$) δ 8.21 (d, J= 2.0 Hz, 1H), 7.96 (dd, J= 9.0, 2.0 Hz, 1H), 7.81 (d, J= 9.0 Hz, 1H), 7.51 (s, 1H), 7.35-7.20 (m, 3H), 7.07-7.00 (m, 2H), 5.33 (s, 2H), 5.06 (bs, 1H), 3.21 (d, J= 13.8 Hz, 1H), 3.10 (d, J= 13.8 Hz, 1H), 3.09-3.00 (m, 1H), 2.76 (s, 3H), 2.58-2.45 (m, 1H), 2.27-2.13 (m, 1H), 2.13-2.02 (m, 2H), 1.95-1.79 (m, 2H), 1.79-1.59 (m, 2H).
MS (ESI+) ($M^+$+1, detection time)

Example 184

2-(1-Benzyl-6-nitro-1H-indol-3-yl)-1,1,1-trifluoro-3-[4-(phenylsulfonyl)piperidin-1-yl]propan-2-ol

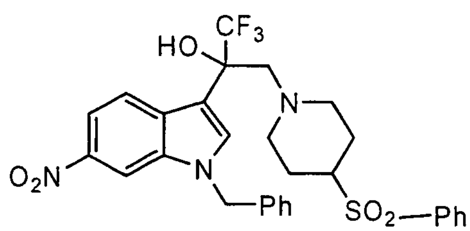

The title compound was obtained in a similar manner to the preparation of the compound of Example 1.
$^1$H-NMR (400 MHz, $CDCl_3$) δ 8.27 (d, J= 2.0 Hz, 1H), 8.02 (dd, J= 9.0, 2.0 Hz, 1H), 7.85 (d, J= 9.0 Hz, 1H), 7.84 (dd, J= 7.5, 1.4 Hz, 2H), 7.70 (dd, J= 7.5, 7.5 Hz, 1H), 7.59 (d, J= 7.5 Hz, 2H), 7.56 (s, 1H), 7.37-7.30 (m, 3H), 7.12-7.05 (m, 2H), 5.39 (s, 2H), 3.29 (d, J= 13.7 Hz, 1H), 3.18 (d, J=13.7 Hz, 1H), 3.14-3.05 (m, 1H), 2.95-2.82 (m, 1H), 2.82-2.68 (m, 1H), 2.62-2.50 (m, 1H), 2.32-2.17 (m, 1H), 2.09-1.99 (m, 1H), 1.93-1.80 (m, 1H), 1.80-1.65 (m, 1H).
MS (ESI+) ($M^+$+1, detection time)

Example 185

3-(4-Anilinopiperidin-1-yl)-2-(1-benzyl-6-nitro-1H-indol-3-yl)-1,1,1-trifluoropropan-2-ol

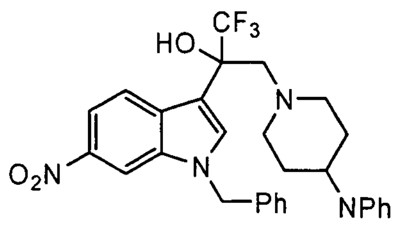

The title compound was obtained in a similar manner to the preparation of the compound of Example 1.
$^1$H-NMR (400 MHz, $CDCl_3$) δ 8.20 (d, J= 2.0 Hz, 1H), 7.96 (dd, J= 9.0, 2.0 Hz, 1H), 7.82 (d, J= 9.0 Hz, 1H), 7.51 (s, 1H), 7.30-7.22 (m, 3H), 7.08 (dd, J= 7.5, 7.5 Hz, 2H), 7.01 (dd, J= 7.7, 2.1 Hz, 2H), 6.62 (dd, J= 7.5, 7.5 Hz, 1H), 6.49 (d, J= 7.5 Hz, 2H), 5.32 (s, 2H), 3.28-3.12 (m, 1H), 3.21 (d, J= 13.6 Hz, 1H), 3.07 (d, J= 13.6 Hz, 1H), 2.90-2.76 (m, 1H), 2.60-2.48 (m, 2H), 2.31-2.18 (m, 1H), 2.05-1.90 (m, 1H), 1.90-1.78 (m, 1H), 1.48-1.24 (m, 2H).
MS (ESI+) 539, 2.13($M^+$+1, detection time)

Example 186

N-{1-[2-(1-Benzyl-6-nitro-1H-indol-3-yl)-3,3,3-trifluoro-2-hydroxypropyl]-piperidin-4-yl}-N-phenylacetamide

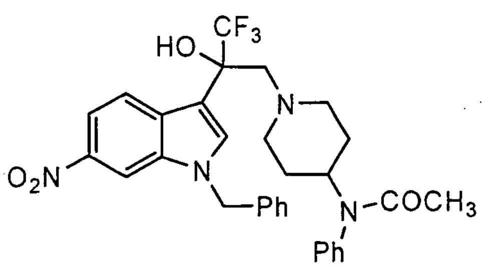

The title compound was obtained in a similar manner to the preparation of the compound of Example 1. MS (ESI+) 581, 2.06($M^+$+1, detection time)

Example 187

N-{1-[2-(1-Benzyl-6-nitro-1H-indol-3-yl)-3,3,3-trifluoro-2-hydroxypropyl]-piperidin-4-yl}-N-phenylmethanesulfonamide

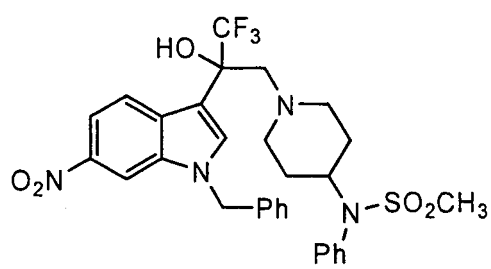

The title compound was obtained in a similar manner to the preparation of the compound of Example 1.
$^1$H-NMR (400 MHz, $CDCl_3$) δ 8.26 (d, J= 1.9 Hz, 1H), 8.01 (dd, J= 9.0, 1.9 Hz, 1H), 7.85 (d, J= 9.0 Hz, 1H), 7.49 (s, 1H), 7.47-7.39 (m, 3H), 7.35-7.30 (m, 3H), 7.24-7.18 (m, 2H), 7.11-7.03 (m, 2H), 5.37 (s, 2H), 4.19-4.06 (m, 1H), 3.26 (d, J= 13.7 Hz, 1H), 3.18-3.01 (m, 1H), 3.11 (d, J= 13.7 Hz, 1H), 2.92 (s, 3H), 2.78-2.60 (m, 2H), 2.40-2.30 (m, 1H), 2.00-1.89 (m, 1H), 1.79-1.69 (m, 1H), 1.69-1.52 (m, 1H), 1.52-1.35 (m, 1H).
MS (ESI+) 617, 2.17($M^+$+1, detection time)

Example 188

Methyl 3-({1-[2-(1-benzyl-6-nitro-1H-indol-3-yl)-3,3,3-trifluoro-2-hydroxypropyl]-piperidin-4-yl}oxy)benzoate
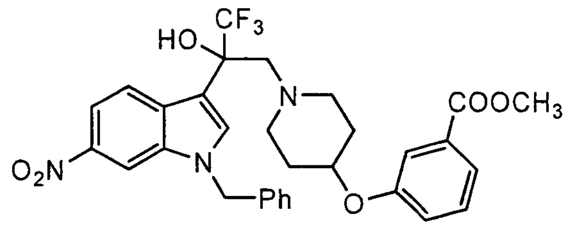

The title compound was obtained in a similar manner to the preparation of the compound of Example 1.
MS (ESI+) 598, 2.23($M^{+}$+1, detection time)

Example 189

Ethyl [3-({1-[2-(1-benzyl-6-nitro-1H-indol-3-yl)-3,3,3-trifluoro-2-hydroxypropyl]-piperidin-4-yl}oxy)phenyl] acetate
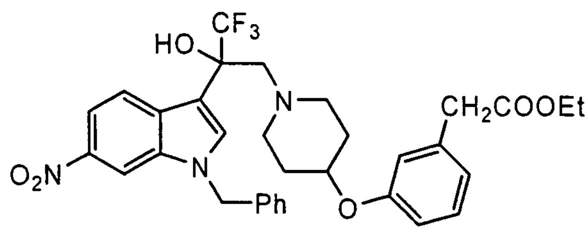

The title compound was obtained in a similar manner to the preparation of the compound of Example 1.
MS (ESI+) 626, 2.27($M^{+}$+1, detection time)

Example 190

Ethyl 3-[3-({1-[2-(1-benzyl-6-nitro-1H-indol-3-yl)-3,3,3-trifluoro-2-hydroxypropyl]piperidin-4-yl}oxy)phenyl]propanoate

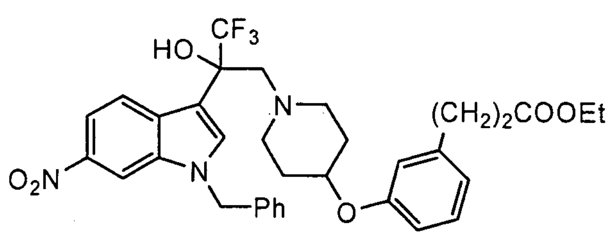

The title compound was obtained in a similar manner to the preparation of the compound of Example 1.
MS (ESI+) 230, 640($M^{+}$+1, detection time)

Example 191

3-({1-[2-(1-Benzyl-6-nitro-1H-indol-3-yl)-3,3,3-trifluoro-2-hydroxypropyl]-piperidin-4-yl}oxy)benzoic acid)

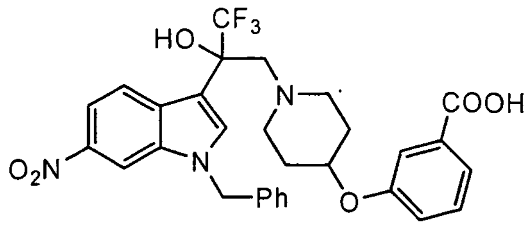

The title compound was obtained from the compound of Example 188 in a similar manner to Example 4.
MS (ESI+) 584, 2.07($M^+$+1, detection time)

Example 192

[3-({1-[2-(1-Benzyl-6-nitro-1H-indol-3-yl)-3,3,3-trifluoro-2-hydroxypropyl]-piperidin-4-yl}oxy)phenyl]acetic acid

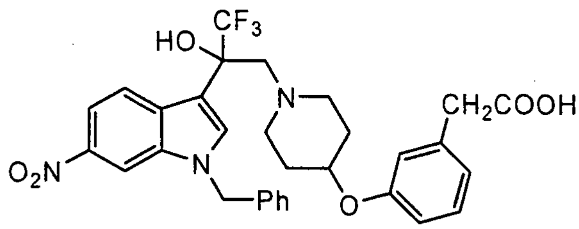

The title compound was obtained from the compound of Example 189 in a similar manner to Example 4.
MS (ESI+) 598, 2.06($M^+$+1, detection time)

Example 193

3-[3-({1-[2-(1-Benzyl-6-nitro-1H-indol-3-yl)-3,3,3-trifluoro-2-hydroxypropyl]-piperidin-4-yl}oxy)phenyl]propanoic acid

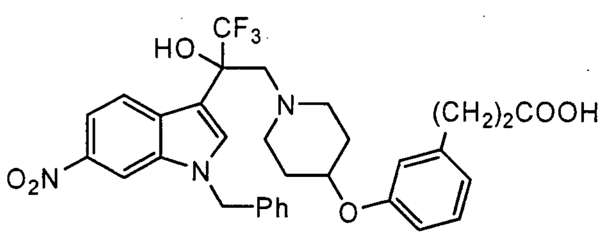

The title compound was obtained from the compound of Example 190 in a similar manner to Example 4.
MS (ESI+) 612, 2.08($M^+$+1, detection time)

Example 194

{1-[2-(1-Benzyl-6-nitro-1H-indol-3-yl)-3,3,3-trifluoro-2-hydroxypropyl]piperidin-4-yl}(2-thienyl)methanone

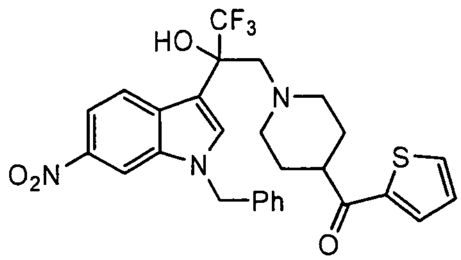

The title compound was obtained in a similar manner to the preparation of the compound of Example 1.
$^1$H-NMR (400 MHz, CDCl3) δ 8.27 (d, J= 2.0 Hz, 1H), 8.03 (dd, J= 9.0, 2.0 Hz, 1H), 7.90 (d, J= 9.0 Hz, 1H), 7.69-7.64 (m, 2H), 7.61 (s, 1H), 7.33-7.26 (m, 3H), 7.14-7.09 (m, 3H), 5.45 (s, 2H), 3.25 (d, J= 13.5 Hz, 1H), 3.18 (d, J= 13.5 Hz, 1H), 3.07-3.00 (m, 2H), 2.64-2,62 (m, 2H), 2.33-2.29 (m, 1H), 1.94-1.61 (m, 4H).

Example 195

{1-[2-(1-Benzyl-6-nitro-1H-indol-3-yl)-3,3,3-trifluoro-2-hydroxypropyl]piperidin-4-yl}(3-thienyl)methanone

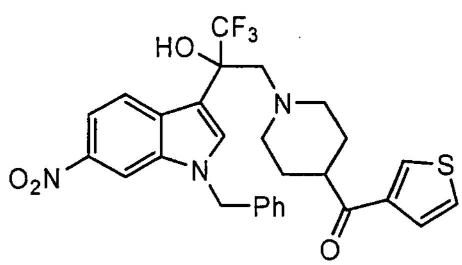

The title compound was obtained in a similar manner to the preparation of the compound of Example 1.
$^1$H-NMR (400 MHz, $CDCl_3$) δ 8.27 (d, J= 2.0 Hz, 1H), 8.04 (dd, J= 9.0, 2.0 Hz, 1H), 7.89 (d, J= 9.0 Hz, 1H), 7.69-7.64 (m, 2H), 7.60 (s, 1H), 7.33-7.30 (m, 3H), 7.14-7.09 (m, 3H), 5.40 (s, 2H), 3.25 (d, J= 13.6 Hz, 1H), 3.18 (d, J= 13.6 Hz, 1H), 3.07-3.01 (m, 2H), 2.64-2,62 (m, 2H), 2.33-2.27 (m, 1H), 1.91-1.70 (m, 4H).

Example 196

{1-[2-(1-Benzyl-6-nitro-1H-indol-3-yl)-3,3,3-trifluoro-2-hydroxypropyl]piperidin-4-yl}(3-furyl)methanone

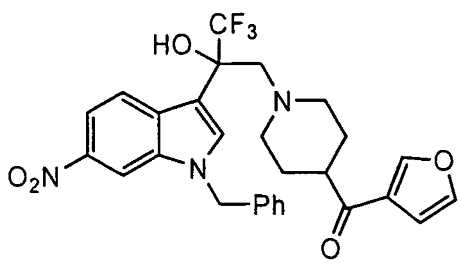

The title compound was obtained in a similar manner to the preparation of the compound of Example 1.
$^1$H-NMR (400 MHz, $CDCl_3$) δ 8.27 (d, J= 2.0 Hz, 1H), 8.03 (dd, J= 9.0, 2.0 Hz, 1H), 8.00 (s, 1H), 7.88 (d, J= 9.0 Hz, 1H), 7.60 (s, 1H), 7.45 (d, J= 1.8 Hz, 1H), 7.33-7.31 (m, 3H), 7.10 (d., J= 9.4 Hz, 1H), 6.74 (d, J= 1.8 Hz, 1H), 5.40 (s, 2H), 3.24 (d, J= 13.6 Hz, 1H), 3.16 (d, J= 13.6 Hz, 1H), 3.02-2.99 (m, 1H), 2.80-2.77 (m, 1H), 2.63-2.59 (m, 2H), 2.30-2.24 (m, 1H), 1.89-1.63 (m, 4H).
MS (ESI+) 542, 2.09($M^+$+1, detection time)

Example 197

{1-[2-(1-Benzyl-6-nitro-1H-indol-3-yl)-3,3,3-trifluoro-2-hydroxypropyl]piperidin-4-yl}(pyridin-3-yl)methanone

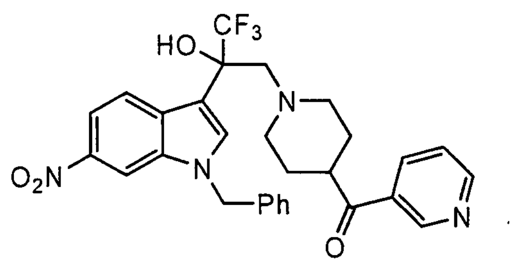

The title compound was obtained in a similar manner to the preparation of the compound of Example 1.
$^1$H-NMR (400 MHz, CDCl3) δ 9.11 (d, J= 1.8 Hz, 1H), 8.78 (dd, J= 4.8, 1.9 Hz, 1H), 8.27 (d, J= 2.0 Hz, 1H), 8.19 (ddd, J= 8.0, 1.9, 1.8 Hz, 1H), 8.04 (dd, J= 9.0, 2.0 Hz, 1H), 7.90 (d, J= 9.0 Hz, 1H), 7.59 (s, 1H), 7.45-7.73 (m, 1H), 7.33-7.30 (m, 3H), 7.11-7.09 (m, 2H), 5.04 (s, 2H), 3.26 (d, J= 13.6 Hz, 1H), 3.18 (d, J= 13.6 Hz, 1H), 3.28-3.16 (m, 1H), 3.04-3.01 (m, 1H), 2.68-2.63 (m, 2H), 2.35-2.34 (m, 1H), 1.90-1.69 (m, 4H).

Example 198

Ethyl 4-({1-[2-(1-benzyl-6-nitro-1H-indol-3-yl)-3,3,3-trifluoro-2-hydroxypropyl]-piperidin-4-yl}oxy)-3-methoxybenzoate
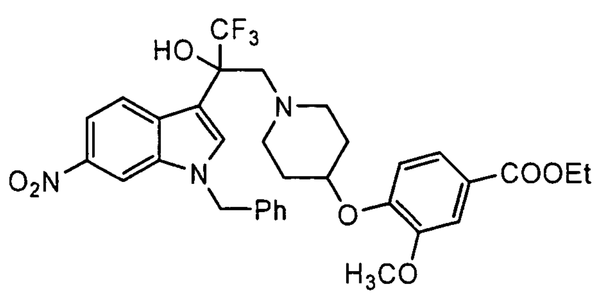

The title compound was obtained in a similar manner to the preparation of the compound of Example 1.
MS (ESI+) 642, 2.25($M^+$+1, detection time)

Example 199

4-({1-[2-(1-Benzyl-6-nitro-1H-indol-3-yl)-3,3,3-trifluoro-2-hydroxypropyl]-piperidin-4-yl}oxy)-3-methoxybenzoic acid

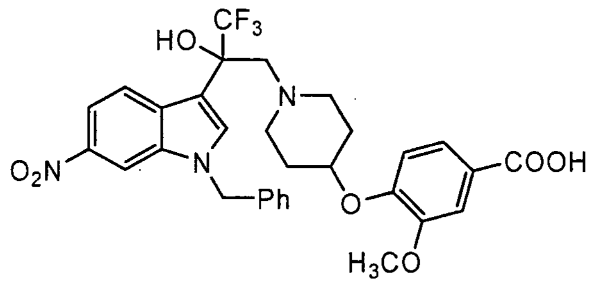

The title compound was obtained from the compound of Example 198 in a similar manner to Example 4.
$^1$H-NMR (400 MHz, $CDCl_3$) δ 8.19 (d, J= 2.0 Hz, 1H), 7.95 (dd, J= 9.0, 2.0 Hz, 1H), 7.82 (d, J= 9.0 Hz, 1H), 7.58 (dd, J= 8.6, 2.0 Hz, 1H), 7.55-7.45 (m, 2H), 7.30-7.20 (m, 3H), 7.05-6.96 (m, 2H), 6.80 (d, J= 8.6 Hz, 1H), 5.32 (s, 2H), 4.34 (bs, 1H), 3.80 (s, 3H), 3.19 (d, J= 13.5 Hz, 1H), 3.10 (d, J= 13.5 Hz, 1H), 2.83-2.64 (m, 2H), 2.50-2.32 (m, 2H), 1.95-1.65 (m, 4H).
MS (ESI+) 614, 2.05($M^+$+1, detection time)

Example 200

Ethyl [4-({1-[2-(1-benzyl-6-nitro-1H-indol-3-yl)-3,3,3-trifluoro-2-hydroxypropyl]-piperidin-4-yl}oxy)-3-methoxyphenyl]acetate

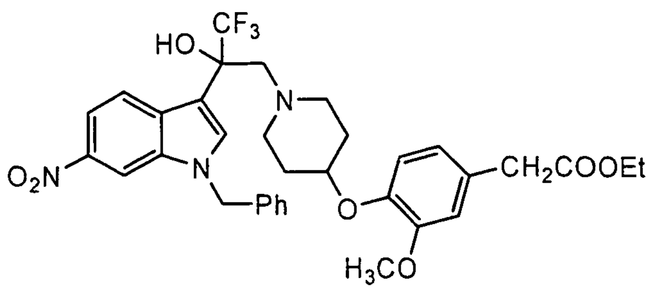

The title compound was obtained in a similar manner to the preparation of the compound of Example 1.
MS (ESI+) 656, 2.21($M^+$+1, detection time)

Example 201

[4-({1-[2-(1-Benzyl-6-nitro-1H-indol-3-yl)-3,3,3-trifluoro-2-hydroxypropyl]-piperidin-4-yl}oxy)-3-methoxyphenyl]acetic acid

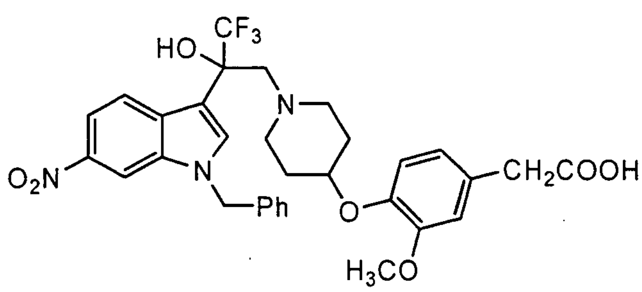

The title compound was obtained from the compound of Example 200 in a similar manner to Example 4.

$^1$H-NMR (400 MHz, $CDCl_3$) δ 9.7 (s, 1H), 8.00 (dd, J= 9.5, 1.8 Hz, 1H), 7.82 (d, J= 8.8 Hz, 1H), 7.63 (d, J= 6.8 Hz, 1H), 7.34 (s, 1H), 7.30-7.13 (m, 3H), 7.05 (d, J= 4.3 Hz, 1H), 6.75-6.68 (m, 1H), 5.33 (s, 2H), 4.45-4.28 (m, 1H), 3.88-3.25 (m, 2H), 3.74 (s, 3H), 3.60 (s, 2H), 3.51 (s, 1H), 3.25-3.01 (m, 1H), 3.01-2.73 (m, 1H), 2.45-2.12 (m, 1H), 2.12-1.93 (m, 2H), 1.93-1.70 (m, 1H).
MS (ESI+) 628, 2.03($M^+$+1, detection time)

Example 202

2-(1-Benzyl-6-nitro-1H-indol-3-yl)-1,1,1-trifluoro-3-(4-{4-[(4-methylpiperazin-1-yl)carbonyl]phenoxy}piperidin-1-yl)propan-2-ol

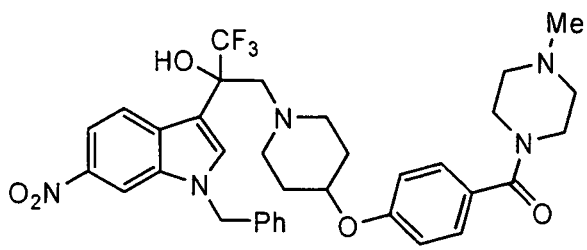

To a solution of the compound of Example 178 (70 mg) in dichloromethane (3 ml) were added 1-ethyl-3-(3-dimethylaminopropyl)carbodiimide hydrochloride (29 mg), triethylamine (30 mg), 1-methylpyperazine (15 mg), 1-hydroxybenzotriazole (20 mg), and the mixture was stirred at 25˚C for 6 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (29 mg).
MS (ESI+) 666, 1.85($M^+$+1, detection time)

Example 203

2-(1-Benzyl-6-nitro-1H-indol-3-yl)-1,1,1-trifluoro-3-(4-{4-[(4-methyl-1,4-diazepan-1-yl)carbonyl]phenoxy}piperidin-1-yl) propan-2-ol

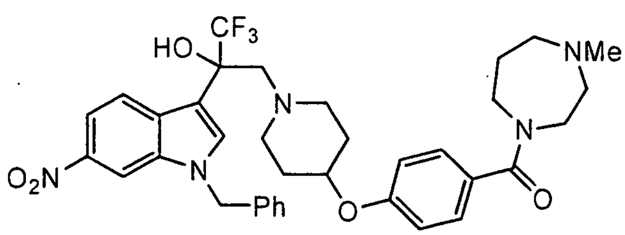

The title compound was obtained in a similar manner to Example 202.
MS (ESI+) 680, 1.85($M^+$+1, detection time)

Example 204

2-(1-Benzyl-6-nitro-1H-indol-3-yl)-3-(4-benzylpiperazin-1-yl)-1,1,1-trifluoropropan-2-ol

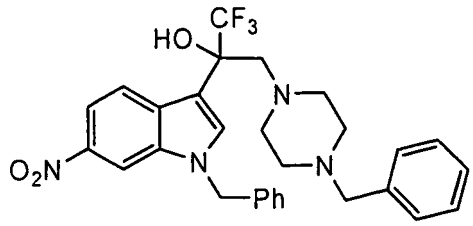

The title compound was obtained in a similar manner to the preparation of the compound of Example 1.
MS (ESI+) 539, 2.06($M^{+}$+1, detection time)

Example 205

2-(1-Benzyl-6-nitro-1H-indol-3-yl)-1,1,1-trifluoro-3-[4-(2-methoxybenzyl)-piperazin-1-yl]propan-2-ol

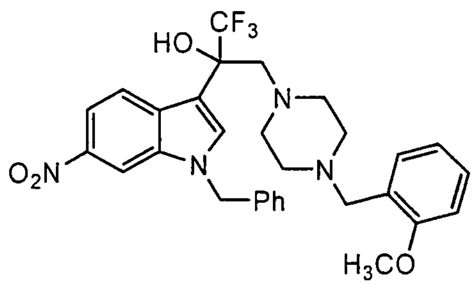

The title compound was obtained in a similar manner to the preparation of the compound of Example 1.
MS (ESI+) 569, 2.09($M^{+}$+1, detection time)

Example 206

{1-[2-(1-Benzyl-6-nitro-1H-indol-3-yl)-3,3,3-trifluoro-2-hydroxypropyl]piperidin-4-yl}(pyridin-2-yl)methanone

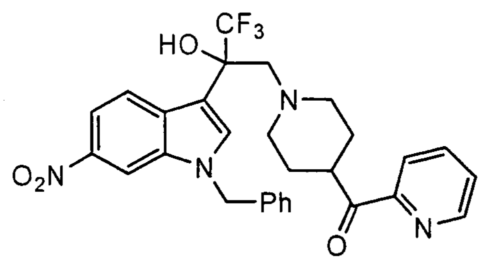

The title compound was obtained in a similar manner to the preparation of the compound of Example 1.
$^{1}$H-NMR (400 MHz, $CDCl_3$) δ 8.65-8.64 (m, 1H), 8.26 (d, J= 2.0 Hz, 1H), 8.05-8.02 (m, 2H), 7.91 (d, J= 9.0 Hz, 1H), 7.84 (ddd, J= 7.7, 7.7, 1.7 Hz, 1H), 7.60 (s, 1H), 7.47 (ddd, J= 7.7, 4.8, 1.2 Hz, 1H), 7.34-7.29 (m, 3H), 7.10 (d, J= 8.0 Hz, 2H), 5.82 (bs, 1H), 5.40 (s, 2H), 3.86-3.80 (m, 1H), 3.25 (d, J= 13.5 Hz, 1H), 3.17 (d, J= 13.5 Hz, 1H), 2.98-2.95 (m 1H), 2.73-2.69 (m, 1H), 2.63-2.60 (m, 1H), 2.37-2.36 (m, 1H), 1.93-1.70(m, 4H).
MS (ESI+) 553, 2.11($M^{+}$+1, detection time)

Example 207

{1-[2-(1-Benzyl-6-nitro-1H-indol-3-yl)-3,3,3-trifluoro-2-hydroxypropyl]piperidin-4-yl}(1H-imidazol-2-yl)methanone

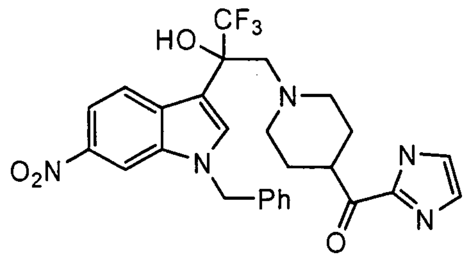

The title compound was obtained in a similar manner to the preparation of the compound of Example 1.
$^1$H-NMR (400 MHz, $CDCl_3$) δ 8.26 (d, J= 2.0 Hz, 1H), 8.03 (dd, J= 9.0, 2.0 Hz, 1H), 7.91 (d, J= 9.0 Hz, 1H), 7.58 (s, 1H), 7.33-7.31 (m, 3H), 7.24-7.21 (m, 2H), 7.10 (d, J= 7.4 Hz, 2H), 5.40 (s, 2H), 3.55-3.54 (m, 1H), 3.26 (d, J= 13.6 Hz, 1H), 3.15 (d, J= 13.6 Hz, 1H), 3.02-3.00 (m, 1H), 2.69-2.62 (m, 2H), 2.40-2.35 (m, 1H), 1.98-1.76 (m, 4H).
MS (ESI+) 542, 1.98($M^+$+1, detection time)

Example 208

{1-[2-(1-Benzyl-6-nitro-1H-indol-3-yl)-3,3,3-trifluoro-2-hydroxypropyl]piperidin-4-yl}(1H-pyrrol-3-yl)methanone

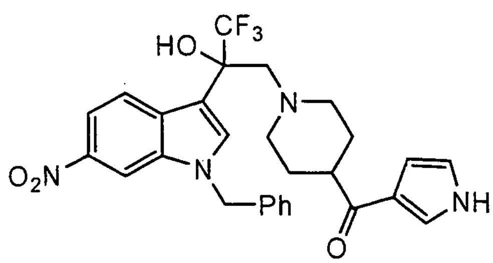

The title compound was obtained in a similar manner to the preparation of the compound of Example 1.
$^1$H-NMR (400 MHz, $CDCl_3$) δ 8.58 (bs, 1H), 8.26 (d, J= 2.0 Hz, 1H), 8.03 (dd, J= 9.0, 2.0 Hz, 1H), 7.88 (d, J= 9.0 Hz, 1H), 7.61 (s, 1H), 7.42-7.41(m, 1H), 7.32-7.29 (m, 3H), 7.10 (d, J= 7.9 Hz, 2H), 6.80-6.78 (m, 1H), 6.64-6.62 (m, 1H), 5.88 (s, 2H), 3.23 (d, J= 13.5 Hz, 1H), 3.17 (d, J= 13.5 Hz, 1H), 3.01-2.98 (m, 1H), 2.92-2.87 (m, 1H), 2.61-2.59 (m, 2H), 2.29-2.27 (m, 1H), 1.91-1.65 (m, 4H).
MS (ESI+) 543, 1.99($M^+$+1, detection time)

Example 209

Ethyl [3-({1-[2-(1-benzyl-6-nitro-1H-indol-3-yl)-3,3,3-trifluoro-2-hydroxypropyl]-piperidin-4-yl}carbonyl)-1H-pyrrol-1-yl]acetate

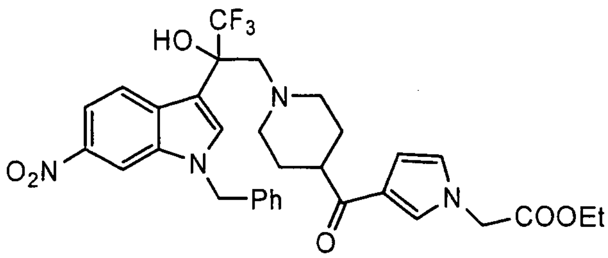

The title compound was obtained in a similar manner to the preparation of the compound of Example 1.
$^1$H-NMR (400 MHz, $CDCl_3$) δ 8,26 (d, J= 2.0 Hz, 1H), 8.03 (dd, J= 9.0, 2.0 Hz, 1H), 7.89 (d, J= 9.0 Hz, 1H), 7.61 (s, 1H), 7.33-7.27 (m, 4H), 7.10 (d, J= 8.0 Hz, 1H), 6.64-6.63 (m, 1H), 6.60-6.59 (m, 1H), 5.76 (bs, 1H), 5.39 (s, 2H), 4.62 (s, 2H), 4.24 (q, J= 7.2 Hz, 2H), 3.22 (d, J= 13.6 Hz, 1H), 3.16 (d, J= 13.6 Hz, 1H), 3.00-2.97 (m, 1H), 2.91-2.83 (m, 1H), 2.61-2.56 (m, 2H), 2.28-2.21 (m, 1H), 1.87-1.64 (m, 4H), 1.29 (t, J= 7.2 Hz, 3H).
MS (ESI+) 627, 2.11($M^+$+1, detection time)

Example 210

Ethyl [4-({(3S)-1-[2-(1-benzyl-6-nitro-1H-indol-3-yl)-3,3,3-trifluoro-2-hydroxypropyl]pyrrolidin-3-yl}oxy)-3-methoxyphenyl]acetate
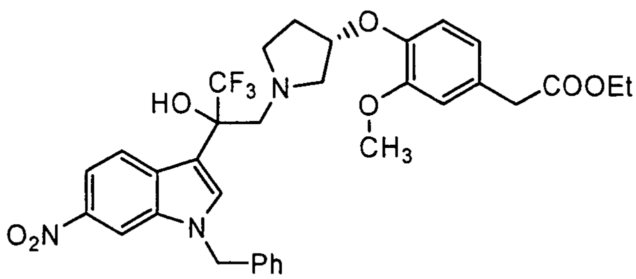

The title compound was obtained in a similar manner to the preparation of the compound of Example 1.
MS (ESI+) 642, 2.37($M^+$+1, detection time)

Example 211

[4-({(3S)-1-[2-(1-Benzyl-6-nitro-1H-indol-3-yl)-3,3,3-trifluoro-2-hydroxypropyl]-pyrrolidin-3-yl}oxy)-3-methoxyphenyl] acetic acid

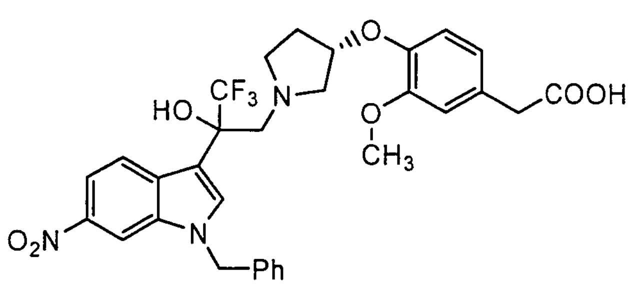

The title compound was obtained from the compound of Example 210 in a similar manner to Example 4.
MS (ESI+) 614, 2.03($M^+$+1, detection time)

Example 212

Ethyl [4-({(3R)-1-[2-(1-benzyl-6-nitro-1H-indol-3-yl)-3,3,3-trifluoro-2-hydroxypropyl]pyrrolidin-3-yl}oxy)-3-methoxyphenyl]acetate

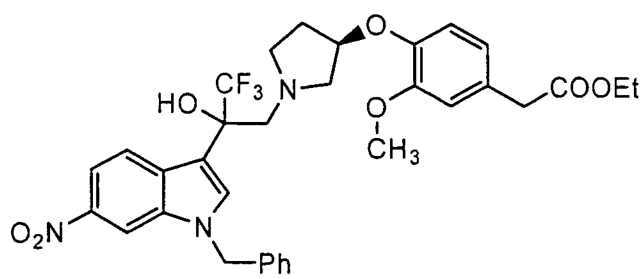

The title compound was obtained in a similar manner to the preparation of the compound of Example 1.
MS (ESI+) 642, 2.37($M^{+}$+1, detection time)

Example 213

[4-({(3R)-1-[2-(1-Benzyl-6-nitro-1H-indol-3-yl)-3,3,3-trifluoro-2-hydroxypropyl]-pyrrolidin-3-yl}oxy)-3-methoxyphenyl] acetic acid

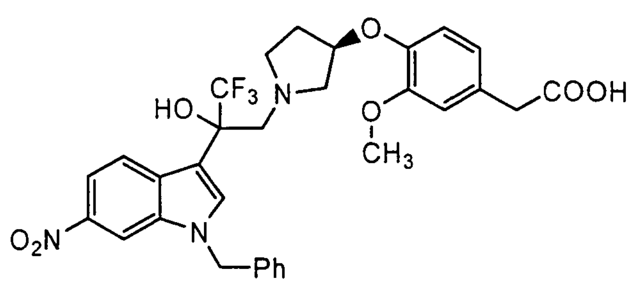

The title compound was obtained from the compound of Example 212 in a similar manner to Example 4.
MS (ESI+) 614, 2.04($M^{+}$+1, detection time)

Example 214

Ethyl [4-({1-[2-(1-benzyl-6-cyano-1H-indol-3-yl)-3,3,3-trifluoro-2-hydroxypropyl]piperidin-4-yl}oxy)-3-methoxyphenyl] acetate

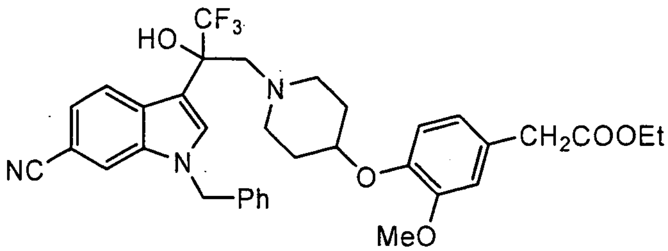

The title compound was obtained in a similar manner to the preparation of the compound of Example 1.
$^1$H-NMR (400 MHz, $CDCl_3$) δ 7.89 (d, J= 8.4 Hz, 1H), 7.58 (s, 1H), 7.51 (s, 1H), 7.35 (dd, J= 8.4, 1.3 Hz, 1H), 7.34-7.28 (m, 3H), 7.06 (dd, J= 7.7, 2.4 Hz, 2H), 6.82 (d, J= 2.0 Hz, 1H), 6.80 (s, 1H), 6.75 (dd, J= 8.1, 1.9 Hz, 1H), 5.82 (bs, 1H), 5.33 (s, 2H), 4.26-4.18 (m, 1H), 4.14 (q, J= 7.1 Hz, 2H), 3.82 (s, 3H), 3.53 (s, 2H), 3.24 (d, J= 13.6 Hz, 1H), 3.15 (d, J= 13.6 Hz, 1H), 2.89-2.75 (m, 2H), 2.52-2.38 (m, 2H), 1.98-1.74 (m, 4H), 1.25 (t, J= 7.1 Hz, 1H). MS (ESI+) 636, 2.15 ($M^+$+1, detection time)

Example 215

[4-({1-[2-(1-Benzyl-6-cyano-1H-indol-3-yl)-3,3,3-trifluoro-2-hydroxypropyl]-piperidin-4-yl}oxy)-3-methoxyphenyl]acetic acid

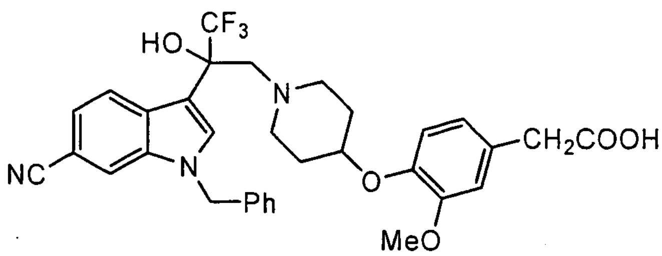

The title compound was obtained from the compound of Example 214 in a similar manner to Example 4.
MS (ESI+) 608, 1.99($M^+$+1, detection time)

Example 216

2-(1-Benzyl-6-nitro-1H-indol-3-yl)-1,1,1-trifluoro-3-{4-[(2-methoxyphenyl)-sulfonyl]piperidin-1-yl}propan-2-ol

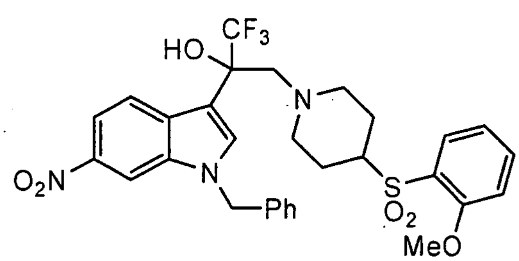

The title compound was obtained in a similar manner to the preparation of the compound of Example 1.
MS (ESI+) 618, 2.31($M^+$+1, detection time)

Example 217

Ethyl [4-({1-[3-(1-benzyl-6-nitro-1H-indol-3-yl)-4,4,4-trifluoro-3-hydroxybutyl]-piperidin-4-yl}oxy)-3-methoxyphenyl]acetate

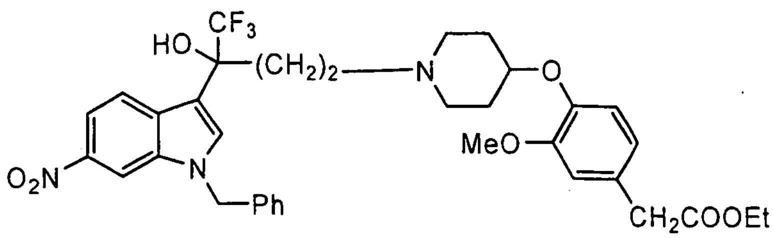

The title compound was obtained in a similar manner to the preparation of the compound of Example 11.
MS (ESI+) 670, 2.14($M^{+}$+1, detection time)

Example 218

[4-({1-[3-(1-Benzyl-6-nitro-1H-indol-3-yl)-4,4,4-trifluoro-3-hydroxybutyl]-piperidin-4-yl}oxy)-3-methoxyphenyl]acetic acid

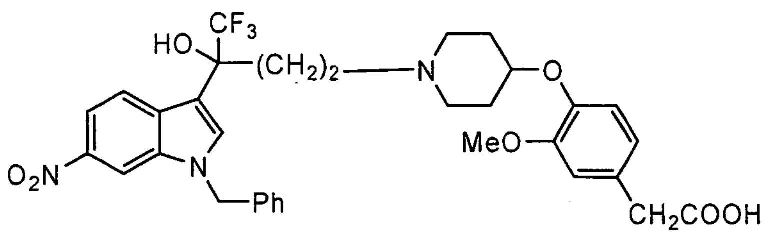

The title compound was obtained from the compound of Example 217 in a similar manner to Example 4.
$^{1}$H-NMR (400 MHz, $CDCl_3$) δ 8.27 (d, J= 2.0 Hz, 1H), 7.99 (dd, J= 9.0, 2.0 Hz, 1H), 7.85 (d, J= 9.0 Hz, 1H), 7.34-7.28 (m, 3H), 7.11 (d, J= 8.0 Hz, 2H), 6.78-6.73 (m, 3H), 5.40 (d, J= 15.9 Hz, 1H), 5.35 (d, J= 15.9 Hz, 1H), 4.13 (bs, 1H), 3.75 (s, 3H), 3.51-3.49 (m, 2H), 2.92-2.42 (m, 6H), 1.83-1.78 (m, 4H).
MS (ESI+) 642, 2.02($M^{+}$+1, detection time)

Example 219

Ethyl {4-[(1-{2-[6-cyano-1-(4-fluorobenzyl)-1H-indol-3-yl]-3,3,3-trifluoro-2-hydroxypropyl}piperidin-4-yl)oxy]-3-methoxyphenyl}acetate

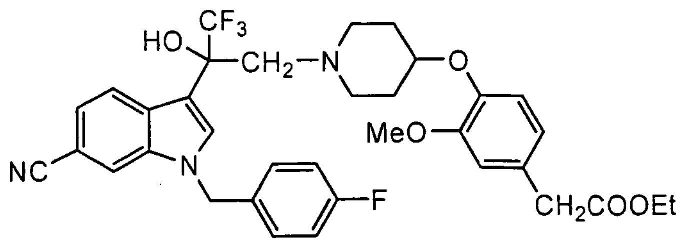

The title compound was obtained in a similar manner to the preparation of the compound of Example 1.
MS (ESI+) 654, 2.13($M^{+}$+1, detection time)

Example 220

Ethyl {4-[(1-{2-[1-(4-chlorobenzyl)-6-cyano-1H-indol-3-yl]-3,3,3-trifluoro-2-hydroxypropyl}piperidin-4-yl)oxy]-3-methoxyphenyl}acetate

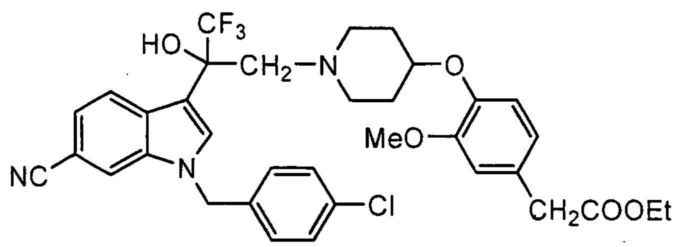

The title compound was obtained in a similar manner to the preparation of the compound of Example 1.
MS (ESI+) 671, 2.19($M^{+}$+1, detection time)

Example 221

Ethyl {4-[(1-{2-[6-cyano-1-(4-methylbenzyl)-1H-indol-3-yl]-3,3,3-trifluoro-2-hydroxypropyl}piperidin-4-yl)oxy]-3-methoxyphenyl}acetate

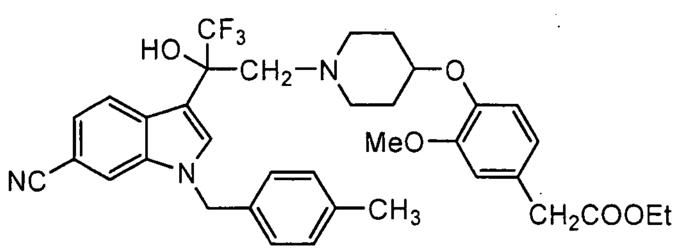

The title compound was obtained in a similar manner to the preparation of the compound of Example 1.
MS (ESI+) 650, 2.07($M^{+}$+1, detection time)

Example 222

Ethyl {4-[(1-{2-(6-cyano-1-(4-methoxybenzyl)-1H-indol-3-y1J-3,3,3-trifluoro-2-hydroxypropyl}piperidin-4-yl)oxy]-3-methoxyphenyl}acetate

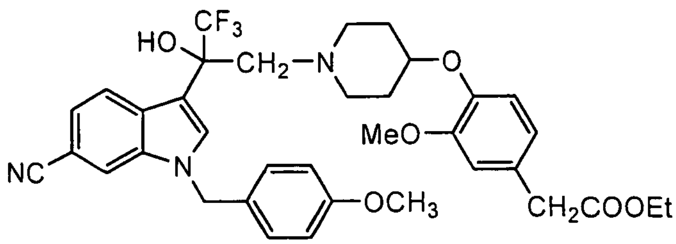

The title compound was obtained in a similar manner to the preparation of the compound of Example 1.
MS (ESI+) 666, 2.00($M^{+}$+1, detection time)

Example 223

{4-[(1-{2-[6-Cyano-1-(4-fluorobenzyl)-1H-indol-3-yl]-3,3,3-trifluoro-2-hydroxypropyl}piperidin-4-yl)oxy]-3-methoxyphenyl}acetic acid

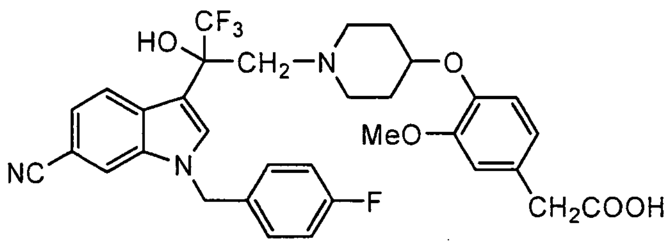

The title compound was obtained from the compound of Example 219 in a similar manner to Example 4.
$^{1}$H-NMR (400 MHz, $CDCl_3$) δ 7.89 (d, J= 8.4 Hz, 1H), 7.61 (s, 1H), 7.60 (s, 1H), 7.39 (dd, J= 8.4, 1.0 Hz, 1H), 7.06 (d, J= 8.6 Hz, 1H), 7.05 (d, J= 8.6 Hz, 1H), 6.99 (d, J= 8.6 Hz, 1H), 6.96 (d, J= 8.6 Hz, 1H), 6.80-6.75 (m, 3H), 5.30 (s, 2H), 4.38 (bs, 1), 3.80-3.60 (m, 2H), 3.70 (s, 3H), 3.55 (s, 2H), 3.45-3.20 (m, 2H), 3.20-2.90 (m, 2H), 2.27-2.11 (m, 1H), 2.11-1.93 (m, 2H), 1.93-1.77 (m, 1H).
MS (ESI+) 626, 2.02($M^{+}$+1, detection time)

Example 224

{4-[(1-{2-[1-(4-Chlorobenzyl)-6-cyano-1H-indol-3-yl]-3,3,3-trifluoro-2-hydroxypropyl}piperidin-4-yl)oxy]-3-methoxyphenyl}acetic acid

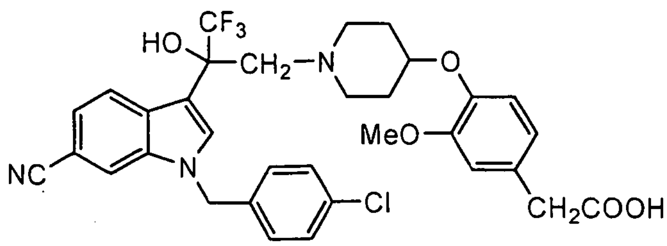

The title compound was obtained from the compound of Example 220 in a similar manner to Example 4.
$^1$H-NMR (400 MHz, $CDCl_3$) δ 7.89 (d, J= 8.4 Hz, 1H), 7.61 (s, 1H), 7.59 (s, 1H), 7.40 (dd, J= 8.4, 1.1 Hz, 1H), 7.29-7.24 (m, 2H), 7.00 (d, J= 8.4 Hz, 2H), 6.81-6.76 (m, 3H), 5.32 (s, 1H), 5.31 (s, 1H), 4.41 (bs, 1H), 3.85-3.61 (m, 2H), 3.70 (s, 3H), 3.57 (s, 2H), 3.61-3.30 (m, 2H), 3.20-3.07 (m, 1H), 3.07-2.90 (m, 1H), 2.35-2.14 (m, 1H), 2.14-1.94 (m, 2H), 1.94-1.77 (m, 1H).
MS (ESI+) 642, 2.07($M^+$+1, detection time)

Example 225

{4-[(1-{2-[6-Cyano-1-(4-methylbenzyl)-1H-indol-3-yl]-3,3,3-trifluoro-2-hydroxypropyl}piperidin-4-yl)oxy]-3-methoxyphenyl}acetic acid

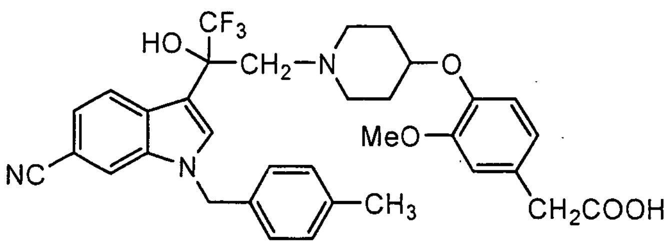

The title compound was obtained from the compound of Example 221 in a similar manner to Example 4.
MS (ESI+) 622, 2.07($M^+$+1, detection time)

Example 226

{4-[(1-{2-[6-Cyano-1-(4-methoxybenzyl)-1H-indol-3-yl]-3,3,3-trifluoro-2-hydroxypropyl}piperidin-4-yl)oxy]-3-methoxyphenyl}acetic acid

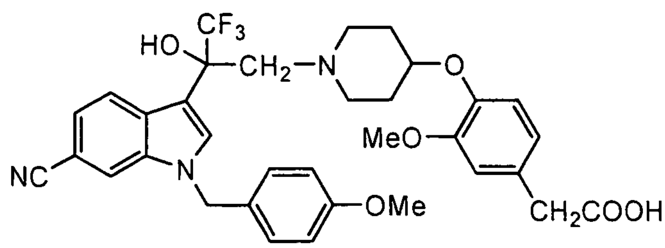

The title compound was obtained from the compound of Example 222 in a similar manner to Example 4.
$^1$H-NMR (400 MHz, $CDCl_3$) δ 7.86 (d, J= 8.4 Hz, 1H), 7.65 (s, 1H), 7.62 (s, 1H), 7.40 (d, J= 8.4 Hz, 1H), 7.04 (d, J= 8.6 Hz, 2H), 6.82 (d, J= 8.6 Hz, 2H), 6.80-6.76 (m, 3H), 5.28 (s, 2H), 4.43 (bs, 1H), 3.87-3.63 (m, 2H), 3.77 (s, 3H), 3.67 (s,

3H), 3.58 (s, 2H), 3.53-3.34 (m, 2H), 3.23-3.10 (m, 1H), 3.04-2.85 (m, 1H), 2.40-2.20 (m, 1H), 2.16-2.00 (m, 2H), 1.93-1.80 (m, 2H).
MS (ESI+) 638, 2.00($M^+$+1, detection time)

Example 227

Ethyl [4-({1-[2-(6-cyano-1-methyl-1H-indol-3-yl)-3,3,3-trifluoro-2-hydroxypropyl]piperidin-4-yl}oxy)-3-methoxyphenyl] acetate

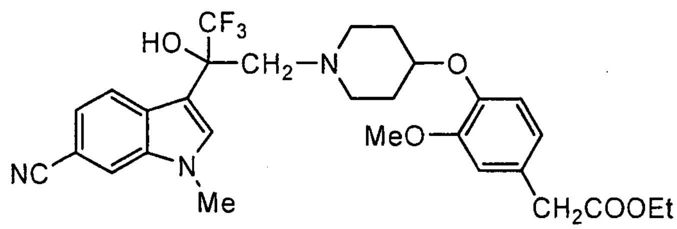

The title compound was obtained in a similar manner to the preparation of the compound of Example 1.
$^1$H-NMR (400 MHz, $CDCl_3$) δ 7.87(d, J= 8.4 Hz, 1H), 7.67 (s, 1H), 7.43 (s, 1H), 7.41 (d, J= 8.4 Hz, 1H), 6.82 (s, 1H), 6.80 (d, J= 8.1 Hz, 1H), 6.74 (d, J= 8.1 Hz, 1H), 4.24-4.21 (m, 1H), 4.13 (q, J= 7.1 Hz, 2H), 3.83 (s, 3H), 3.82 (s, 3H), 3.53 (s, 2H), 3.25 (d, J= 13.6 Hz, 1H), 3.12 (d, J= 13.6 Hz, 1H), 2.84-2.78 (m, 2H), 2.52-2.46 (m, 2H), 1.91-1.77 (m, 4H), 1.26 (t, J= 7.1 Hz, 3H).
MS (ESI+) 560, 1.99($M^+$+1, detection time)

Example 228

Ethyl [4-({1-[2-(6-cyano-1-ethyl-1H-indol-3-yl)-3,3,3-trifluoro-2-hydroxypropyl]-piperidin-4-yl}oxy)-3-methoxyphenyl]acetate

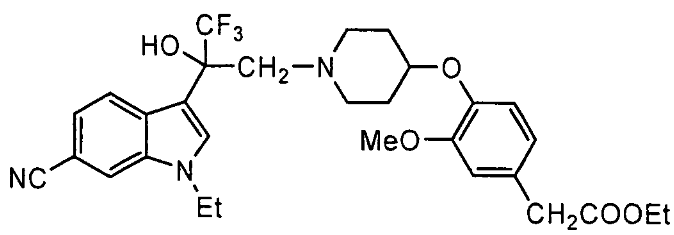

The title compound was obtained in a similar manner to the preparation of the compound of Example 1.
$^1$H-NMR (400 MHz, $CDCl_3$) 87.86 (d, J= 8.4 Hz, 1H), 7.68 (s, 1H), 7.48 (s, 1H), 7.35 (d, J= 8.4 Hz, 1H), 6.82-6.73 (m, 3H), 5.85 (bs, 1H), 4.14 (q, J= 7.2 Hz, 2H), 4.25-4.10 (m, 1H), 3.82 (s, 3H), 3.53 (s, 2H), 3.48 (q, J= 7.0 Hz, 2H), 3.26 (d, J= 13.6 Hz, 1H), 3.12 (d, J= 13.6 Hz, 1H), 2.85-2.78 (m, 2H), 2.50-2.46 (m, 2H), 1.91-1.81 (m, 4H), 1.50 (t, J= 7.2 Hz, 3H), 1.21 (t, J= 7.0 Hz, 3H).
MS (ESI+) 574, 2.05($M^+$+1, detection time)

Example 229

Ethyl 3-[4-({1-[2-(1-benzyl-6-nitro-1H-indol-3-yl)-3,3,3-trifluoro-2-hydroxypropyl]piperidin-4-yl}carbonyl)phenyl]propanoate

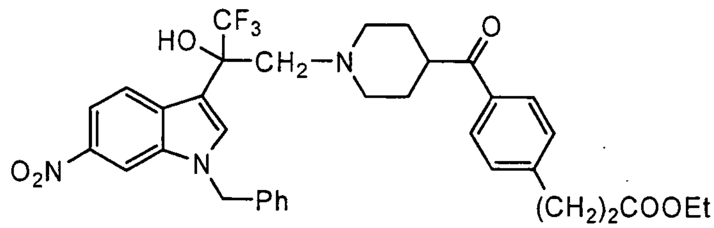

The title compound was obtained in a similar manner to the preparation of the compound of Example 1.
$^1$H-NMR (400 MHz, $CDCl_3$) δ 8.25 (d, J= 2.1 Hz, 1H), 8.02 (dd, J= 6.4, 2.1 Hz, 1H), 7.89 (d, J= 6.4 Hz, 1H), 7.87 (s, 1H), 7.35-7.28 (m, 3H), 7.19-7.04 (m, 6H), 5.39 (s, 1H), 4.12 (q, J= 7.5 Hz, 2H), 3.18 (d, J= 13.5 Hz, 1H), 3.06 (d, J= 13.5 Hz, 1H), 2.96-2.85 (m, 3H), 2.63-2.41 (m, 4H), 2.11-2.00 (m, 1H), 1.79-1.68 (m, 4H), 1.21 (t, J= 7.5 Hz, 3H).
MS (ESI+) 654, 2.14($M^+$+1, detection time)

Example 230

Ethyl {4-[(1-{2-[6-cyano-1-(pyridin-2-ylmethyl)-1H-indol-3-yl]-3,3,3-trifluoro-2-hydroxypropyl}piperidin-4-yl)oxy]-3-methoxyphenyl}acetate
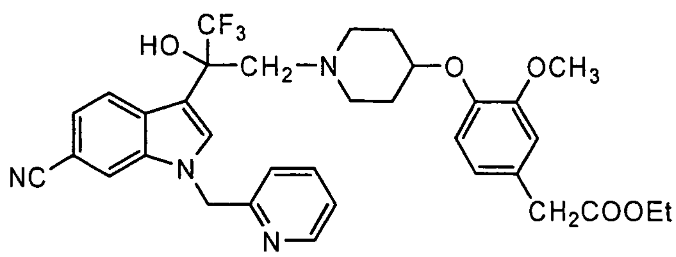

The title compound was obtained in a similar manner to the preparation of the compound of Example 1.
MS (ESI+) 637, 2.09($M^+$+1, detection time)

Example 231

Ethyl {4-[(1-{2-[6-cyano-1-(pyridin-3-ylmethyl)-1H-indol-3-yl]-3,3,3-trifluoro-2-hydroxypropyl}piperidin-4-yl)oxy]-3-methoxyphenyl}acetate

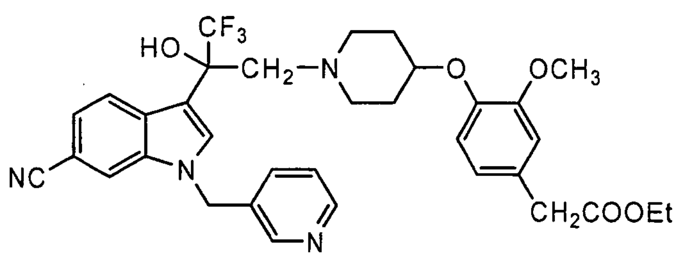

The title compound was obtained in a similar manner to the preparation of the compound of Example 1.
MS (ESI+) 637, 2.08($M^+$+1, detection time)

Example 232

Ethyl {4-[(1-{2-[6-cyano-1-(pyridin-4-ylmethyl)-1H-indol-3-yl]-3,3,3-trifluoro-2-hydroxypropyl}piperidin-4-yl)oxy]-3-methoxyphenyl}acetate

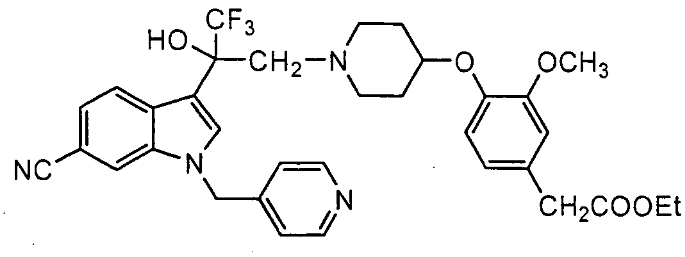

The title compound was obtained in a similar manner to the preparation of the compound of Example 1.
MS (ESI+) 637, 2.04($M^{+}$+1, detection time)

Example 233

4-[(1-{2-[6-Cyano-1-(pyridin-2-ylmethyl)-1H-indol-3-yl]-3,3,3-trifluoro-2-hydroxypropyl}piperidin-4-yl)oxy]-3-methoxyphenyl}acetic acid

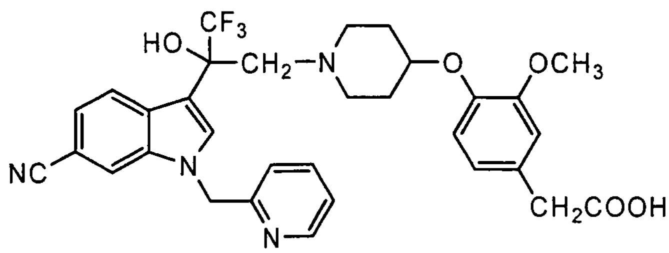

The title compound was obtained from the compound of Example 230 in a similar manner to Example 4.
$^{1}$H-NMR (400 MHz, $CD_3OD$) δ 8.54 (d, J= 4.8 Hz, 1H), 7.98 (d, J= 8.4 Hz, 1H), 7.77 (s, 1H), 7.72 (dd, J= 7.7, 7.6 Hz, 1H), 7.67 (s, 1H), 7.40 (d, J= 8.4 Hz, 1H), 7.32-7.29 (m, 1H), 7.02 (d, J= 7.7 Hz, 1H), 6.80 (d, J= 9.4 Hz, 2H), 6.78 (s, 1H), 5.54 (s, 2H), 4.43 (bs, 1H), 3.81 (d, J= 13.5 Hz, 1H), 3.76 (d, J= 13.5 Hz, 1H), 3.70 (s, 3H), 3.59 (s, 2H), 3.51-3.44 (m, 2H), 3.27-3.25 (m, 1H), 3.03 (bs, 1H), 2.26-1.86 (m, 4H).
MS (ESI+) 609, 1.89($M^{+}$+1, detection time)

Example 234

4-[(1-{2-[6-Cyano-1-(pyridin-3-ylmethyl)-1H-indol-3-yl]-3,3,3-trifluoro-2-hydroxypropyl}piperidin-4-yl)oxy]-3-methoxyphenyl}acetic acid

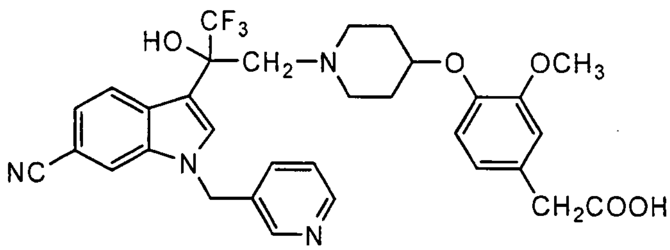

The title compound was obtained from the compound of Example 231 in a similar manner to Example 4.
MS (ESI+) 609, 1.87($M^+$+1, detection time)

Example 235

{4-[(1-{2-[6-Cyano-1-(pyridin-4-ylmethyl)-1H-indol-3-yl]-3,3,3-trifluoro-2-hydroxypropyl}piperidin-4-yl)oxy]-3-methoxyphenyl}acetic acid

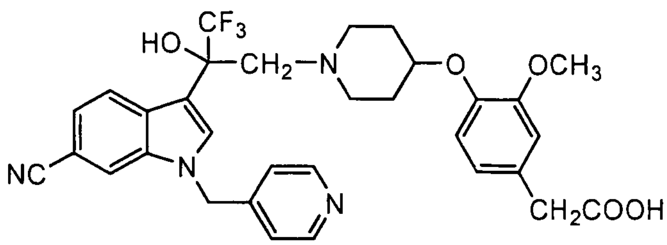

The title compound was obtained from the compound of Example 232 in a similar manner to Example 4.
MS (ESI+) 609, 1.83($M^+$+1, detection time)

Example 236

[4-({1-[2-(6-Cyano-1-methyl-1H-indol-3-yl)-3,3,3-trifluoro-2-hydroxypropyl]-piperidin-4-yl}oxy)-3-methoxyphenyl]acetic acid

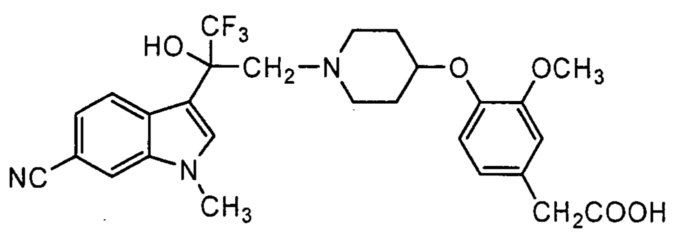

The title compound was obtained from the compound of Example 227 in a similar manner to Example 4.
$^1$H-NMR (400 MHz, CDCl3) δ 7.84 (d, J= 8.3 Hz, 1H), 7.71 (s, 1H), 7.60 (bs, 1H), 7.42 (d, J= 8.3 Hz, 1H), 6.79-6.78 (m, 3H), 4.46 (bs, 1H), 3.38 (s, 3H), 3.88-3.79 (m, 2H), 3.72 (s, 3H), 3.58 (s, 2H), 3.52-3.46 (m, 2H), 3.26-3.25 (m, 1H), 2.94 (bs, 1H), 2.45 (bs, 1H), 2.18-2.11 (m, 2H), 1.91-1.88 (m, 1H).
MS (ESI+) 532, 1.84($M^+$+1, detection time)

Example 237

[4-({1-[2-(6-Cyano-1-ethyl-1H-indol-3-yl)-3,3,3-trifluoro-2-hydroxypropyl]-piperidin-4-yl}oxy)-3-methoxyphenyl]acetic acid

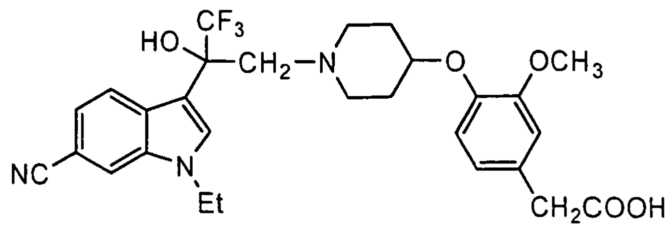

The title compound was obtained from the compound of Example 228 in a similar manner to Example 4.
$^1$H-NMR (400 MHz, $CDCl_3$) δ 7.82 (d, J= 8.2 Hz, 1H), 7.74 (s, 1H), 7.68 (bs, 1H), 7.41 (d, J=8.2 Hz, 1H), 6.78 (d, J= 6.0 Hz, 3H), 4.46 (bs, 1H), 4.26-4.23 (m, 2H), 3.90-3.47 (m, 2H), 3.72 (s, 3H), 3.57 (s, 2H), 3.49 (q, J= 7.0 Hz, 2H), 3.28-3.23 (m, 1H), 2.99-2.91 (m, 1H), 2.43 (bs, 1H), 2.18-2.05 (m, 2H), 1.89 (bs, 1H), 1.53 (t, J= 7.0 Hz, 3H).
MS (ESI+) 546, 1.89($M^+$+1, detection time)

Reference Example 5

1-Benzyl-6-nitro-3-[2-phenyl-l-(trifluoromethyl)vinyl]-1H-indole

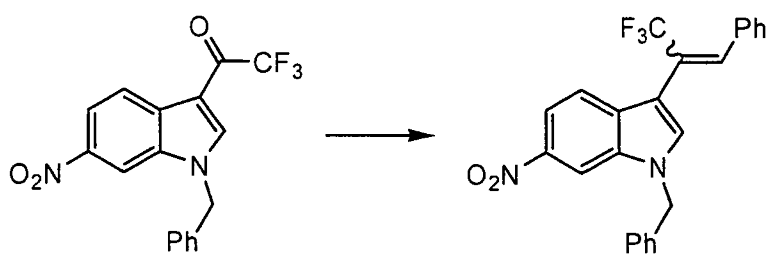

To a suspension of benzyltriphenylphosphonium chloride (727 mg) in tetrahydrofuran (5 ml) was added potassium t-butoxide at 0˚C, and the mixture was stirred at the same temperature for 15 minutes. To the reaction solution was added the compound of Reference Example 2 (500 mg), and the mixture was stirred at 25˚C for 5 hours. To the reaction solution was added a saturated aqueous ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (558 mg).
MS (ESI+) 423, 2.87($M^+$+1, detection time)

Example 238

2-(1-Benzyl-6-nitro-1H-indol-3-yl)-3,3,3-trifluoro-1-phenylpropane-1,2-diol

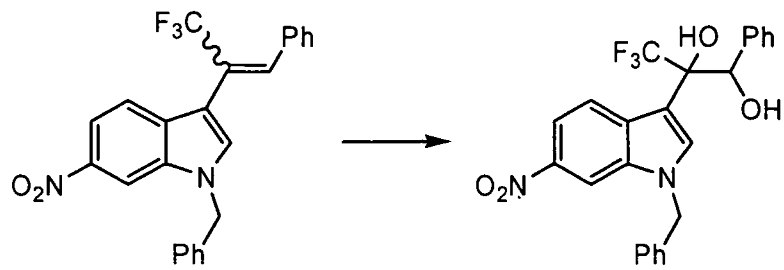

To a solution of potassium permanganate (114 mg) and benzyltriethylammonium chloride (342mg) in methylene chloride (6 ml) was added the compound of Reference Example 5 (200 mg) at 0˚C. The mixture was stirred at 0˚C for one hour, and thereto was added 1N aqueous sodium hydroxide solution, and the mixture was stirred at room temperature overnight. The mixture was filtered through celite, and the filtrate was extracted with ethyl acetate. The organic layer was washed with water and a saturated saline solution, dried over sodium sulfate, and filtered. The filtrate was concentrated under reduced pressure, and the obtained residue was purified by silica gel column chromatography to give the title compound (6 mg).

$^1$H-NMR (400 MHz, $CDCl_3$) δ 8.07 (d, J= 2.0 Hz, 1H), 7.96 (dd, J= 9.0, 2.0 Hz, 1H), 7.85 (d, J= 9.0 Hz, 1H), 7.31 (s, 1H), 7.26-7.22 (m, 3H), 7.15-7.10 (m, 3H), 7.06-7.02 (m, 2H), 6.71-6.69(m, 2H), 5.53 (d, J= 2.7 Hz, 1H), 5.26 (s, 2H), 4.20 (s, 1H), 2.60 (d, J= 2.7 Hz, 1H).

MS (ESI+) 457, 2.50($M^+$+1, detection time)

Example 239

2-Hydroxyethyl 2-(1-benzyl-6-nitro-1H-indol-3-yl)-3,3,3-trifluoro-2-hydroxypropanoate

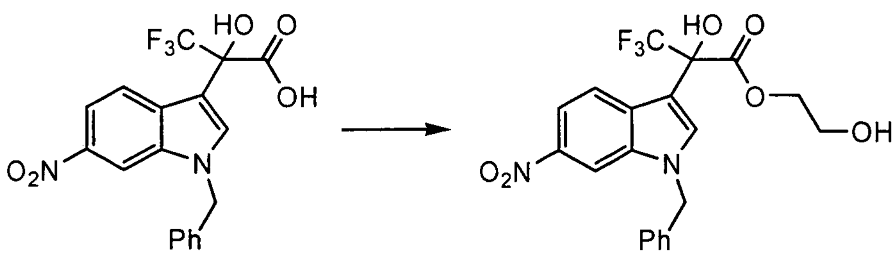

To a solution of the compound of Example 61 (250 mg) in ethylene glycol (1 ml) was added iron (III) sulfate n·hydrate (6.3 mg), and the mixture was stirred at 120˚C for 5 hours. To the reaction solution was added a saturated aqueous ammonium chloride solution, and the mixture was extracted with ethyl acetate, dried over sodium sulfate, and filtered. The filtrate was concentrated under reduced pressure, and the obtained residue was purified by silica gel column chromatography to give the title compound (25 mg).

MS (ESI+) 439, 2.38($M^+$+1, detection time)

Example 240

2-(Benzyloxy)ethyl 2-(1-benzyl-6-nitro-1H-indol-3-yl)-3,3,3-trifluoro-2-hydroxypropanoate

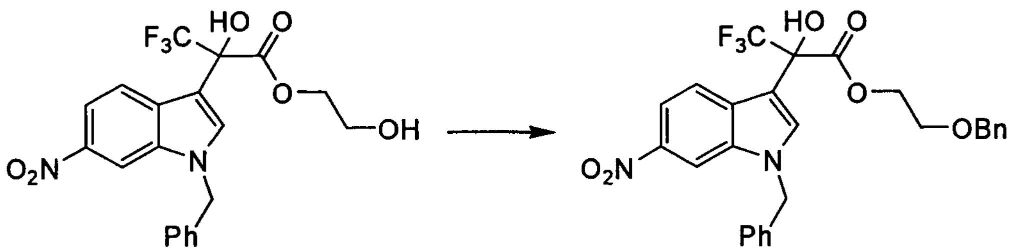

To a solution of the compound of Example 239 (7 mg) in dimethylformamide (0.5 ml) was added sodium hydride (2 mg), and the mixture was stirred at room temperature 5 minutes. Benzyl bromide (10 μl) was added to the reaction solution, and the mixture was stirred at room temperature for 6 hours. To the reaction solution was added a saturated aqueous ammonium chloride solution, and the mixture was extracted with ethyl acetate, dried over sodium sulfate, and filtered. The filtrate was concentrated under reduced pressure, and the obtained residue was purified by silica gel column chromatography to give the title compound (2 mg).
MS (ESI+) 529, 3.01($M^+$+1, detection time)

Example 241

2-(1-Benzyl-6-nitro-1H-indol-3-yl)-1,1,1-trifluorobut-3-yn-2-ol

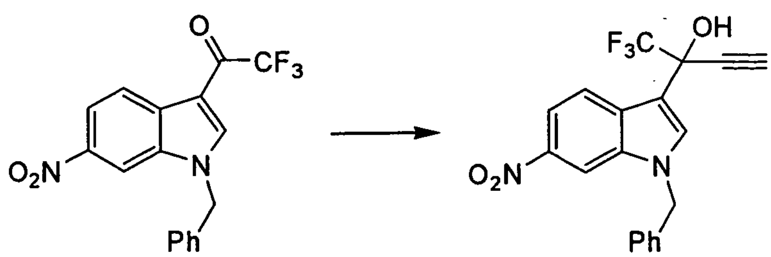

To a solution of the compound of Reference Example 2 (120 mg) in tetrahydrofuran (1.5 ml) was added ethynyl magnesium bromide (0.5 M tetrahydrofuran solution, 1.52 ml) at 0°C, and the mixture was stirred at 0°C for 2 hours. To the reaction solution was added a saturated aqueous ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (56 mg).
$^1$H-NMR (400 MHz, $CDCl_3$) δ 8.27 (d, J= 1.9 Hz, 1H), 8.06 (dd, J= 9.0, 1.9 Hz, 1H), 8.03 (d, J= 9.0 Hz, 1H), 7.69 (s, 1H), 7.39-7.31 (m, 3H), 7.16-7.14 (m, 2H), 5.41 (s, 2H), 3.20 (s, 1H), 2.87 (s, 1H).
MS (ESI+) 375, 2.53($M^+$+1, detection time)

Example 242

1-Benzyl-3-[1-(methoxymethoxy)-1-(trifluoromethyl)prop-2-yn-1-yl]-6-nitro-1H-indol

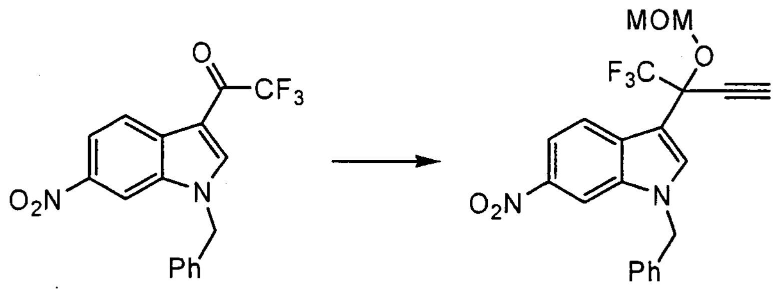

To a solution of the compound of Reference Example 2 (1 g) in tetrahydrofuran (5 ml) was added ethynyl magnesium bromide (0.5 M tetrahydrofuran solution, 24 ml) at 0˚C, and the mixture was stirred at the same temperature for 2 hours. To the reaction solution was added methoxymethyl chloride (0.34 ml), and the mixture was stirred at room temperature for 10 hours. To the reaction solution was added a saturated aqueous ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (248 mg). MS (ESI+) 419, 2.73($M^{+}$+1, detection time)

Example 243

Ethyl 4-[3-(1-benzyl-6-nitro-1H-indol-3-yl)-4,4,4-trifluoro-3-(methoxymethoxy)-but-1-yn-1-yl]benzoate

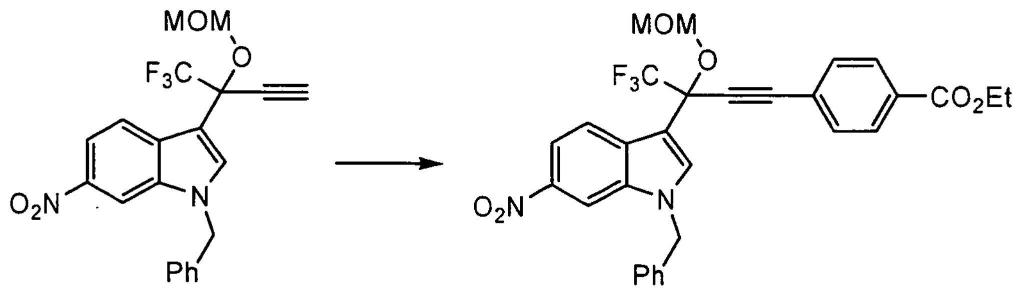

To a solution of the compound of Example 242 (107 mg) in triethylamine (1 ml) were added ethyl 4-bromobenzoate (70 mg), dichlorobistriphenylphosphine palladium (18 mg), copper (II) iodide (2.4 mg), and the mixture was stirred at 100˚C for 8 hours. The insoluble materials were removed by filtration on celite, and concentrated. The obtained residue was purified by silica gel column chromatography to give the title compound (21 mg).
MS (ESI+) 567, 3.01($M^{+}$+1, detection time)

Example 244

1-(1-Benzyl-6-nitro-1H-indol-3-yl)-2,2,2-trifluoro-1-(4-methylphenyl)ethanol

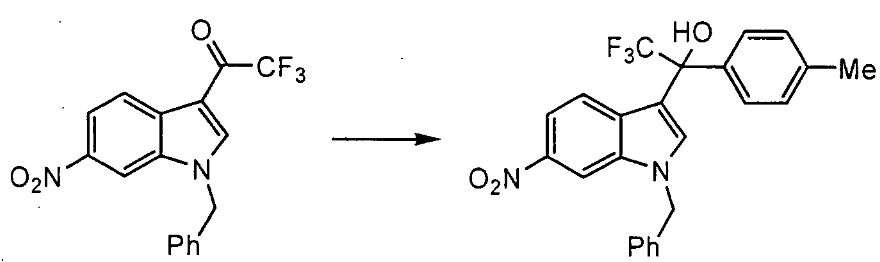

To a solution of the compound of Reference Example 2 (1 ml) was added 4-tolyl magnesium bromide (1.0 M tetrahydrofuran solution, 0.4 ml) at -78˚C, and the mixture was warmed to -78˚C to room temperature over a period of one hour. To the reaction solution was added a saturated aqueous ammonium chloride solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (48 mg).
$^1$H-NMR (400 MHz, $CDCl_3$) δ 8.24 (d, J= 2.0 Hz, 1H), 7.83 (dd, J= 8.9, 2.0 Hz, 1H), 7.64-7.63 (m, 1H), 7.42 (d, J= 8.0 Hz, 1H), 7.40-7.32 (m, 4H), 7.26 (d, J= 8.9 Hz, 1H), 7.17-7.16 (m, 4H), 5.43 (s, 2H), 2.84 (s, 1H), 2.36 (s, 3H).
MS (ESI+) 441, 2.55($M^+$+1, detection time)

Example 245

1-(1-Benzyl-6-nitro-1H-indol-3-yl)-2,2,2-trifluoro-1-(4-methoxyphenyl)ethanol

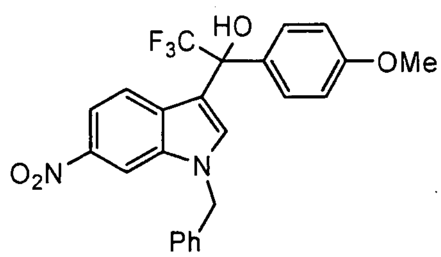

The title compound was obtained in a similar manner to Example 244.
$^1$H-NMR (400 MHz, $CDCl_3$) δ 8.24 (d, J= 2.0 Hz, 1H), 7.83 (dd, J= 9.0, 2.0 Hz, 1H), 7.63 (bs, 1H), 7.44 (d, J= 8.8 Hz, 2H), 7.41-7.34 (m, 3H), 7.29-7.24 (m, 1H), 7.19-7.17 (m, 1H), 6.92 (d, J= 8.8 Hz, 2H), 5.44 (s, 2H), 3.81 (s, 3H), 2.83 (s, 1H). MS (ESI+) 457, 2.56($M^+$+1, detection time)

Example 246

Ethyl 4-[3-(1-benzyl-6-nitro-1H-indol-3-yl)-4,4,4-trifluoro-3-hydroxybut-1-yn-1-yl]benzoate

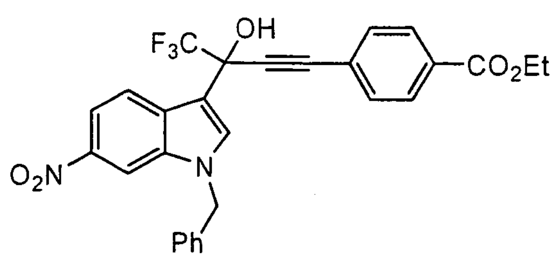

The title compound was obtained from the compound of Example 243 as a starting compound in a similar manner to Example 7.
MS (ESI+) 523, 2.86($M^+$+1, detection time)

Example 247

1-(3-Fluorobenzyl)-3-[(3E)-1-hydroxy-4-(3-thienyl)-1-(trifluoromethyl)but-3-en-1-yl]-1H-indole-6-carbonitrile

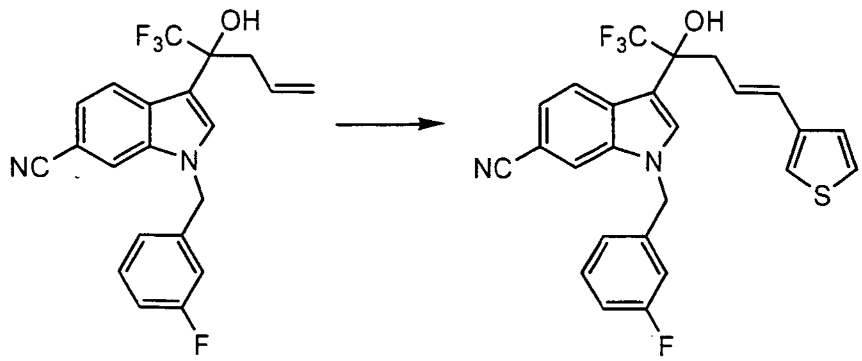

To a solution of 1-(3-fluorobenzyl)-3-[1-hydroxy-1-(trifluoromethyl)but-3-en-1-yl]-1H-indole-6-carbonitrile (58 mg) prepared in a similar manner to Example 8 and 3-bromothiophene (21 μl) in N,N-dimethylformamide (0.5 ml) were added diisopropylethylamine (51 μl) and di-t-butylphosphine palladium (15 mg), and the mixture was stirred at 90°C for 8 hours. To the reaction solution was added a saturated aqueous ammonium chloride solution, and the mixture was extracted with ethyl acetate. The organic layer was dried over sodium sulfate, and filtered. The filtrate was concentrated under reduced pressure, and the obtained residue was purified by silica gel column chromatography to give the title compound (53 mg).

$^1$H-NMR (400 MHz, $CDCl_3$) δ 7.99 (d, J= 8.4 Hz, 1H), 7.57 (bs, 1H), 7.46 (s, 1H), 7.37 (d, J= 8.4 Hz, 1H), 7.24-7.22 (m, 2H), 7.08-6.97 (m, 3H), 6.82 (d, J= 7.7 Hz, 1H), 6.72 (bd, J= 9.2 Hz, 1H), 6.57 (d, J= 15.6 Hz, 1H), 5.83 (dt, J= 15.6, 7.4 Hz, 1H), 5.34 (s, 2H), 3.19 (dd, J= 14.4, 7.4 Hz, 1H), 3.04 (dd, J= 14.4, 7.4 Hz, 1H), 2.83 (s, 1H).

MS (ESI+) 471, 2.62($M^+$+1, detection time)

In a similar manner to the preparation of the compound of Example 247, the compounds of Examples 248 to 253 having a chemical structure as listed in Table 1 were obtained.

[Table 1]

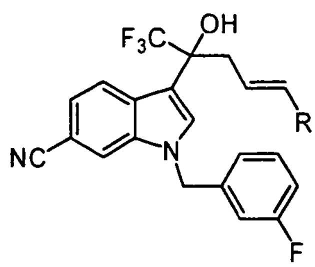

| Example | R | | Example | R | | Example | R |
|---|---|---|---|---|---|---|---|
| 248 | 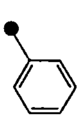 | | 250 | 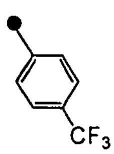 | | 252 | 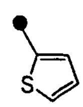 |
| 249 | 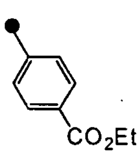 | | 251 | 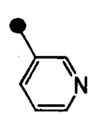 | | 253 | 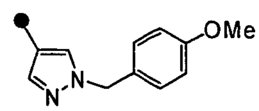 |

Example 248

1-(3-Fluorobenzyl)-3-[(3E)-1-hydroxy-4-phenyl-1-(trifluoromethyl)but-3-en-1-yl]-1H-indole-6-carbonitrile

$^1$H-NMR (400 MHz, $CDCl_3$) δ 8.00 (d, J= 8.4 Hz, 1H), 7.57 (bs, 1H), 7.46 (s, 1H), 7.43 (dd, J= 8.4, 1.4 Hz, 1H), 7.39-7.19 (m, 6H), 7.02-6.97 (m, 1H), 6.82 (d, J= 7.7 Hz, 1H), 6.73 (d, J= 9.0 Hz, 1H), 6.57 (d, J= 15.6 Hz, 1H), 5.99 (dt, J= 15.6, 7.4 Hz, 1H), 5.35 (s, 2H), 3.24 (dd, J= 14.8, 7.4 Hz, 1H), 3.08 (dd, J= 14.8, 7.4 Hz, 1H), 2.74 (s, 1H).
MS (ESI+) 465, 2.51($M^+$+1, detection time)

Example 249

Ethyl 4-{(1E)-4-[6-cyano-1-(3-fluorobenzyl)-1H-indol-3-yl]-5,5,5-trifluoro-4-hydroxypent-1-en-1-yl}benzoate

$^1$H-NMR (400 MHz, $CDCl_3$) δ 7.99 (d, J= 8.5 Hz, 1H), 7.93 (d, J= 8.4 Hz, 2H), 7.58 (bs, 1H), 7.46 (s, 1H), 7.40 (dd, J= 8.5, 1.3 Hz, 1H), 7.28-7.21 (m, 3H), 7.02-6.97 (m, 1H), 6.81 (d, J= 7.6 Hz, 1H), 6.71 (d, J= 9.3 Hz, 1H), 6.57 (d, J= 15.5 Hz, 1H), 6.12 (dt, J= 15.5, 7.5 Hz, 1H), 5.35 (s, 2H), 4.36 (q, J= 7.1 Hz, 2H), 3.25 (dd, J= 14.5,7.5 Hz, 1H), 3.10 (dd, J= 14.5, 7.5 Hz, 1H), 2.77 (s, 1H), 1.38 (t, J= 7.1 Hz, 3H).
MS (ESI+) 537, 2.57($M^+$+1, detection time)

Example 250

1-(3-Fluorobenzyl)-3-{(3E)-1-hydroxy-1-(trifluoromethyl)-4-(4-(trifluoromethyl)-phenyl]but-3-en-1-yl}-1H-indole-6-carbonitrile

$^1$H-NMR (400 MHz, $CDCl_3$) δ 7.99 (d, J= 8.4 Hz, 1H), 7.58 (bs, 1H), 7.51 (d, J= 8.1 Hz, 2H), 7.46 (s, 1H), 7.41 (d, J= 8.4 Hz, 1H), 7.31 (d, J= 8.1 Hz, 2H), 7.25-7.20 (m, 1H), 7.02-6.97 (m, 1H), 6.82 (d, J= 8.2 Hz, 1H), 6.68 (d, J= 8.8 Hz, 1H), 6.56 (d, J= 15.8 Hz, 1H), 6.12 (dt, J= 15.8, 7.4 Hz, 1H), 5.35 (s, 2H), 3.26 (dd, J= 14.7, 7.4 Hz, 1H), 3.10 (dd, J= 14.7, 7.4 Hz, 1H), 2.72 (s, 1H).
MS (ESI+) 533, 2.75($M^+$+1, detection time)

Example 251

1-(3-Fluorobenzyl)-3-[(3E)-1-hydroxy-4-pyridin-3-yl-1-(trifluoromethyl)but-3-en-1-yl]-1H-indole-6-carbonitrile

$^1$H-NMR (400 MHz, $CDCl_3$) δ 8.38 (bs, 2H), 8.05 (d, J= 8.3 Hz, 1H), 7.58 (s, 1H), 7.52-7.48 (m, 1H), 7.48 (s, 1H), 7.40 (d, J= 8.3 Hz, 1H), 7.29-7.23 (m, 1H), 7.19-7.16 (m, 1H), 7.02-6.97 (m, 1H), 6.83 (d, J= 7.7 Hz, 1H), 6.69 (bd, J= 9.4 Hz, 1H), 6.46 (d, J= 15.8 Hz, 1H), 6.07 (dt, J= 14.3, 7.2 Hz, 1H), 3.10 (dd, J= 14.3, 7.2 Hz, 1H).
MS (ESI+) 466, 2.27($M^+$+1, detection time)

Example 252

1-(3-Fluorobenzyl)-3-[(3E)-1-hydroxy-4-(2-thienyl)-1-(trifluoromethyl)but-3-en-1-yl]-1H-indole-6-carbonitrile

$^1$H-NMR (400 MHz, $CDCl_3$) δ 7.99 (d, J= 8.5 Hz, 1H), 7.56 (bs, 1H), 7.45 (s, 1H), 7.40 (dd, J= 8.5, 1.4 Hz, 1H), 7.28-7.23 (m, 1H), 7.13 (d, J= 5.1 Hz, 1H), 7.04-6.97 (m, 1H), 6.93 (dd, J= 5.1, 3.3 Hz, 1H), 6.88 (d, J= 3.3 Hz, 1H), 6.80 (d, J= 7.7 Hz, 1H), 6.76-6.74 (m, 1H), 6.67 (d, J= 15.7 Hz, 1H), 5.81 (dt, J= 15.7, 7.4 Hz, 1H), 5.35 (s, 2H), 3.19 (dd, J= 14.5, 7.4 Hz, 1H), 3.04 (dd, J= 14.5, 7.4 Hz, 1H), 2.78 (bs, 1H).
MS (ESI+) 471, 2.59($M^+$+1, detection time)

Example 253

1-(3-Fluorobenzyl)-3-[(3E)-1-hydroxy-4-[1-(4-methoxybenzyl)-1H-pyrazol-4-yl]-1-(trifluoromethyl)but-3-en-1-yl]-1H-indole-6-carbonitrile

$^1$H-NMR (400 MHz, CDCl3) δ 7.97 (d, J= 8.4 Hz, 1H), 7.55 (bs, 1H), 7.43 (s, 1H), 7.37 (d, J= 8.4 Hz, 1H), 7.27-7.21 (m, 2H), 7.19-6.83 (m, 2H), 7.16 (d, J= 8.7 Hz, 2H), 6.86 (d, J= 8.7 Hz, 2H), 6.82 (d, J= 7.7 Hz, 1H), 6.69 (bd, J= 9.6 Hz, 1H), 6.34 (d, J= 15.9 Hz, 1H), 5.65 (dt, J= 15.9, 7.3 Hz, 1H), 5.33 (s, 2H), 5.14 (s, 2H), 3.80 (s, 3H), 3.07 (dd, J= 14.8, 7.3 Hz, 1H), 2.97 (dd, J= 14.8, 7.3 Hz, 1H).

MS (ESI+) 575, 2.61($M^+$+1, detection time)

Example 254

1-(3-Fluorobenzyl)-3-[1-hydroxy-4-(3-thienyl)-1-(trifluoromethyl)butyl]-1H-indole-6-carbonitrile

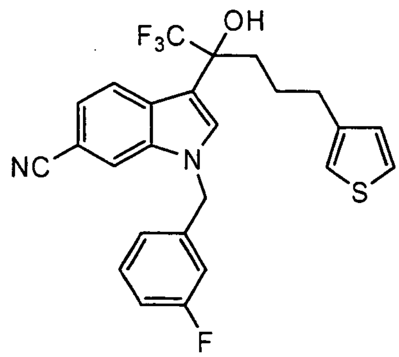

To a solution of the compound of Example 247 (35 mg) in ethanol (3 ml) was added 10% Pd-C (80 mg). Under hydrogen atmosphere, the mixture was stirred at room temperature for 6 hours, and filtered through celite. The filtrate was concentrated under reduced pressure, and purified by silica gel column chromatography to give the title compound (24 mg). $^1$H-NMR (400 MHz, $CDCl_3$) δ 7.60 (d, J= 8.4 Hz, 1H), 7.54 (bs, 1H), 7.31 (d, J= 8.4 Hz, 1H), 7.30-7.28 (m, 3H), 7.20-7.18 (m, 1H), 7.04-6.99 (m, 1H), 6.85-6.83 (m, 2H), 6.78 (dd, J= 4.9, 1.2 Hz, 1H), 6.73-6.70 (m, 1H), 5.31 (s, 2H), 2.73-2.56 (m, 2H), 2.52 (bs, 1H), 2.36-2.28 (m, 1H), 2.11-2.03 (m, 1H), 1.82-1.73 (m, 1H), 1.53-1.42 (m, 1H).
MS (ESI+) 473, 2.51($M^+$+1, detection time)

In a similar manner to the preparation of the compound of Example 254, the compounds of Examples 255 to 258 having a chemical structure as shown in Table 2 were obtained.

[Table 2]

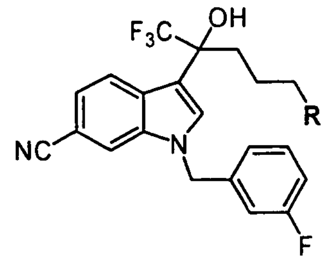

| Exam. | R | | Exam. | R | | Exam. | R | | Exam. | R |
|---|---|---|---|---|---|---|---|---|---|---|
| 255 | 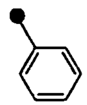 | | 256 | 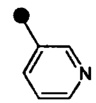 | | 257 | 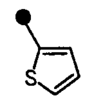 | | 258 | 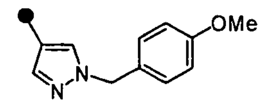 |

Example 255

1-(3-Fluorobenzyl)-3-[1-hydroxy-4-phenyl-1-(trifluoromethyl)butyl]-1H-indole-6-carbonitrile

MS (ESI+) 467, 2.63($M^{+}$+1, detection time)

Example 256

1-(3-Fluorobenzyl)-3-[1-hydroxy-4-pyridin-3-yl-1-(trifluoromethyl)butyl]-1H-indole-6-carbonitrile

$^{1}$H-NMR (400 MHz, $CDCl_3$) δ 8.29 (bs, 1H), 8.23 (bs, 1H), 7.87 (d, J= 8.5 Hz, 1H), 7.55 (bs, 1H), 7.45 (s, 1H), 7.38 (d, J= 7.7 Hz, 1H), 7.35 (d, J= 8.5 Hz, 1H), 7.35-7.26 (m, 1H), 7.14 (dd, J= 7.7, 4.8 Hz, 1H), 7.02-6.98 (m, 1H), 6.86 (d, J= 7.6 Hz, 1H), 6.69 (bd, J= 9.3 Hz, 1H), 5.33 (s, 2H), 2.62-2.53 (m, 2H), 2.38-2.30 (m, 1H), 2.18-2.11 (m, 1H), 1.86-1.75 (m, 1H), 1.53-1.47 (m, 1H).
MS (ESI+) 468, 2.00 ($M^{+}$+1, detection time)

Example 257

1-(3-Fluorobenzyl)-3-[1-hydroxy-4-(2-thienyl)-1-(trifluoromethyl)butyl]-1H-indole-6-carbonitrile

$^{1}$H-NMR (400 MHz, $CDCl_3$) δ 7.86 (d, J= 8.4 Hz, 1H), 7.55 (s, 1H), 7.37 (s, 1H), 7.37-7.28 (m, 2H), 7.08 (bd, J= 4.7 Hz, 1H), 7.00 (dd, J= 8.3, 8.3 Hz, 1H), 6.89-6.85 (m, 1H), 6.86 (d, J= 8.3 Hz, 1H), 6.75-6.70 (m, 2H), 5.33 (s, 2H), 2.91-2.74 (m, 2H), 2.36-2.29 (m, 1H), 2.15-2.07 (m, 1H), 1.89-1.81 (m, 1H), 1.50-1.44 (m, 1H).
MS (ESI+) 473, 2.55($M^{+}$+1, detection time)

Example 258

1-(3-Fluorobenzyl)-3-[1-hydroxy-4-[1-(4-methoxybenzyl)-1H-pyrazol-4-yl]-1-(trifluoromethyl)butyl]-1 H-indole-6-carbonitrile

$^{1}$H-NMR (400 MHz, $CDCl_3$) δ 7.84 (d, J= 8.5 Hz, 1H), 7.55 (s, 1H), 7.41 (s, 1H), 7.30 (d, J= 8.5 Hz, 1H), 7.28-7.19 (m, 1H), 7.13 (d, J= 8.4 Hz, 2H), 7.05 (s, 2H), 7.01-6.97 (m, 1H), 6.86 (d, J= 8.4 Hz, 2H), 6.85-6.80 (m, 1H), 6.70 (d, J= 9.4 Hz, 1H), 5.33 (s, 2H), 5.13 (s, 2H), 3.79 (s, 3H), 2.48-2.37 (m, 2H), 2.35-2.23 (m, 1H), 2.11-2.03 (m, 1H), 1.99 (bs, 1H), 1.73-1.66 (m, 1H), 1.37-1.22 (m, 1H).
MS (ESI+) 577, 2.46($M^{+}$+1, detection time)

Example 259

1-Benzyl-3-[1-hydroxy-1-(trifluoromethyl)prop-2-yn-1-yl]-1H-indole-6-carbonitrile

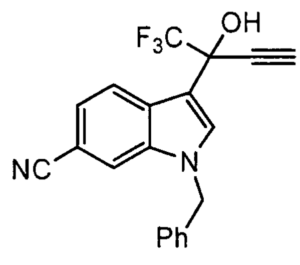

The title compound was obtained in a similar manner to the preparation of the compound of Example 241.
$^{1}$H-NMR (400 MHz, $CDCl_3$) 8 8.06 (d, J= 8.4 Hz, 1H), 7.65 (s, 1H), 7.60 (bs, 1H), 7.38-7.31 (m, 4H), 7.13-7.11 (m, 2H), 5.36 (s, 2H), 2.87 (s, 1H), 2.27 (s, 1H).
MS (ESI+) 355, 2.33($M^{+}$+1, detection time)

Example 260

1-Benzyl-3-[1-(methoxymethoxy)-1-(trifluoromethyl)prop-2-yn-1-yl]-1H-indole-6-carbonitrile

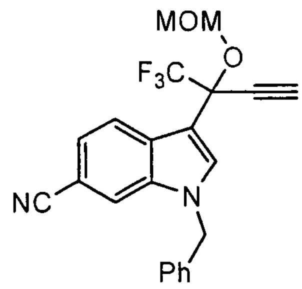

To a solution of the compound of Example 259 (548 mg) in a mixed solvent of tetrahydrofuran/N,N-dimethylformamide (3/2 ml) was added sodium hydride (81 mg) at 0˚C, and the mixture was stirred at the same temperature for 10 minutes. To the reaction solution was added methoxymethyl chloride, and the mixture was stirred at the same temperature for one hour. To the reaction solution was added a saturated aqueous sodium hydrogen carbonate solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (563 mg).
$^1$H-NMR (400 MHz, $CDCl_3$) δ 7.98 (d, J= 8.4 Hz, 1H), 7.68 (s, 1H), 7.59 (bs, 1H), 7.40-7.30 (m, 4H), 7.11 (d, J= 8.4 Hz, 1H), 7.11-7.09 (m, 1H), 5.36 (s, 2H), 4.97 (d, J= 6.6 Hz, 1H), 4.93 (d, J= 6.6 Hz, 1H), 3.40 (s, 3H).

Example 261

Ethyl 4-[3-(1-benzyl-6-cyano-1H-indol-3-yl)-4,4,4-trifluoro-3-(methoxymethoxy)-but-1-yn-1-yl]benzoate
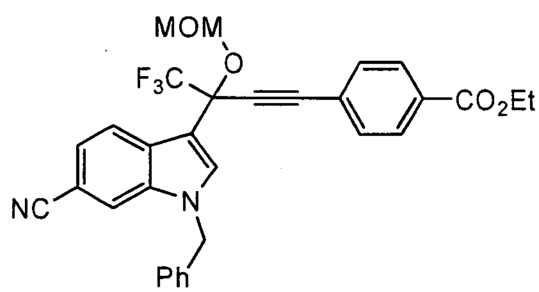

The title compound was obtained in a similar manner to the preparation of the compound of Example 243.
$^1$H-NMR (400 MHz, $CDCl_3$) δ 8.05 (d, J= 10.0 Hz, 2H), 8.04-8.02 (m, 1H), 7.68 (s, 1H), 7.61 (bs, 1H), 7.58 (d, J= 10.0 Hz, 2H), 7.42-7.31 (m, 4H), 7.13 (d, J= 8.0 Hz, 1H), 7.12 (d, J= 7.2 Hz, 1H), 5.37 (s, 2H), 5.03 (d, J= 6.7 Hz, 1H), 5.00 (d, J= 6.7 Hz, 1H), 4.40 (q, J= 7.1 Hz, 2H), 3.44 (s, 3H), 1.41 (t, J= 7.1 Hz, 3H).
MS (ESI+) 547, 2.78($M^+$+1, detection time)

Example 262

3-[4-({1-[2-(1-Benzyl-6-nitro-1H-indol-3-yl)-3,3,3-trifluoro-2-hydroxypropanoyl]-piperidin-4-yl}oxy)-3-methoxyphenyl] propanoic acid

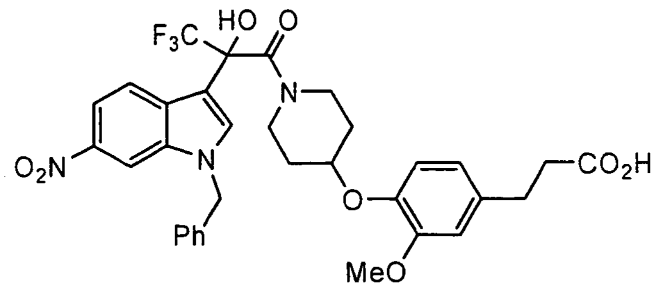

The title compound was obtained in a similar manner to the preparation of the compound of Example 7.
H-NMR (400 MHz, $CDCl_3$) δ 8.29 (d, J= 1.4 Hz, 1H), 8.02 (s, 1H), 7.60 (s, 1H), 7.55 (d, J= 8.9 Hz, 1H), 7.33-7.27 (m, 3H), 7.13-7.05 (m, 2H), 6.78-6.62 (m, 3H), 6.25 (bs, 1H), 5.45 (d, J= 16.0 Hz, 1H), 5.40 (d, J= 16.0 Hz, 1H), 4.30-4.00 (m, 1H), 4.00-3.60 (m, 6H), 3.40-3.20 (m, 1H), 2.86 (t, J= 7.7 Hz, 2H), 2.63 (t, J= 7.7 Hz, 2H), 1.97-1.50 (m, 4H).
MS (ESI+) 656, 2.37($M^{+}$+1, detection time)

Example 263

1-Benzyl-3-[1-({4-[4-(3-ethoxy-3-oxopropyl)-2,6-dimethoxyphenoxy]piperidin-1-yl}methyl)-2,2,2-trifluoro-1-hydroxyethyl]-1H-indole-6-carboxylic acid

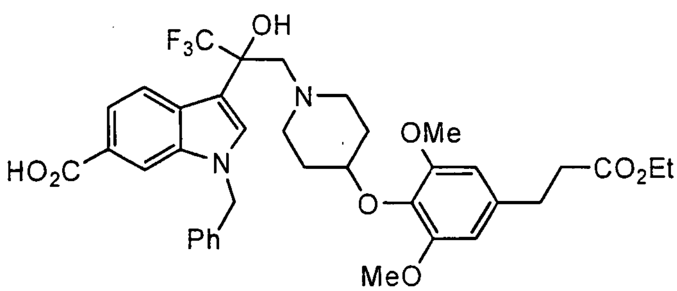

The title compound was obtained from benzyl 1-benzyl-3-[1-({4-[4-(3-ethoxy-3-oxopropyl)-2,6-dimethoxyphenoxy]piperidin-1-yl}methyl)-2,2,2-trifluoro-1-hydroxyethyl]-1H-indole-6-carboxylate (the compound of Example 367 as described hereinbelow) as a starting compound in a similar manner to the preparation of the compound of Example 28.
MS (ESI+) 699, 2.14($M^{+}$+1, detection time)

In a similar manner to the preparation of the compound of Example 93, the compounds of Examples 264 to 269 having a chemical structure as shown in Table 3 were obtained from the compound of Example 263 as a starting compound.

[Table 3]

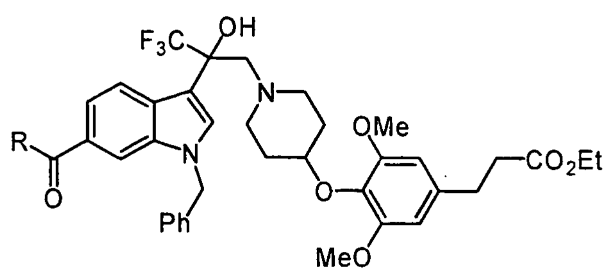

(continued)

| Exam. | R | | Exam. | R | | Exam. | R |
|---|---|---|---|---|---|---|---|
| 264 | 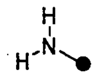 | | 266 | 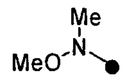 | | 268 | 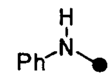 |
| 265 | 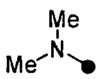 | | 267 | 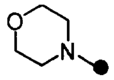 | | 269 | 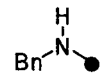 |

Example 264

Ethyl 3-{4-[(1-{2-[6-(aminocarbonyl)-1-benzyl-1H-indol-3-yl]-3,3,3-trifluoro-2-hydroxypropyl}piperidin-4-yl)oxy]-3,5-dimethoxyphenyl}propanoate MS (ESI+) 698, 2.42($M^{+}$+1, detection time)

Example 265

Ethyl 3-(4-{[1-(2-{1-benzyl-6-[(dimethylamino)carbonyl]-1H-indol-3-yl}-3,3,3-trifluoro-2-hydroxypropyl)piperidin-4-yl]oxy}-3,5-dimethoxyphenyl)propanoate MS (ESI+) 726, 2.15($M^{+}$+1, detection time)

Example 266

Ethyl 3-[4-({1-[2-(1-benzyl-6-{[methoxy(methyl)amino]carbonyl}-1H-indol-3-yl)-3,3,3-trifluoro-2-hydroxypropyl]piperidin-4-yl}oxy)-3,5-dimethoxyphenyl]-propanoate

MS (ESI+) 742, 2.19($M^{+}$+1, detection time)

Example 267

Ethyl 3-{4-[(1-{2-[1-benzyl-6-(morpholin-4-ylcarbonyl)-1H-indol-3-yl]-3,3,3-trifluoro-2-hydroxypropyl}piperidin-4-yl)oxy]-3,5-dimethoxyphenyl}propanoate MS (ESI+) 768, 2.15($M^{+}$+1, detection time)

Example 268

Ethyl 3-{4-[(1-{2-[6-(anilinocarbonyl)-1-benzyl-1H-indol-3-yl]-3,3,3-trifluoro-2-hydroxypropyl}piperidin-4-yl)oxy]-3,5-dimethoxyphenyl}propanoate

MS (ESI+) 774, 2.30($M^{+}$+1, detection time)

Example 269

Ethyl 3-(4-{[1-(2-{1-benzyl-6-[(benzylamino)carbonyl]-1H-indol-3-yl}-3,3,3-trifluoro-2-hydroxypropyl)piperidin-4-yl] oxy}-3, 5-dimethoxyphenyl)propanoate

MS (ESI+) 788, 2.29($M^{+}$+1, detection time)
In a similar manner to the preparation of the compound of Example 93 the compounds of Examples 270 to 277 having a chemical structure as shown in Table 4 were obtained from the compound of Example 199 as a starting compound.
[Table 4]

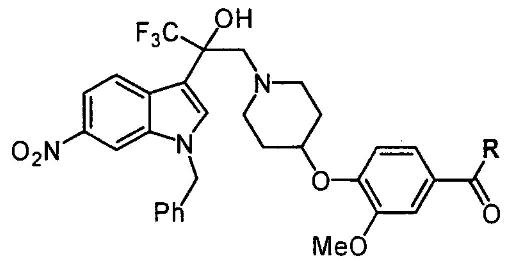

| 実施例 | R | | 実施例 | R | | 実施例 | R |
|---|---|---|---|---|---|---|---|
| 270 | 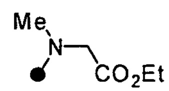 | | 273 | 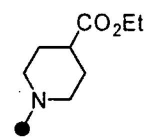 | | 276 | 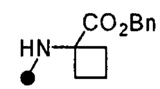 |
| 271 | 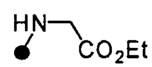 | | 274 | 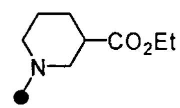 | | 277 | 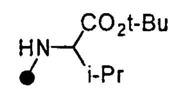 |
| 272 | 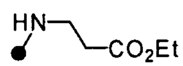 | | 275 | 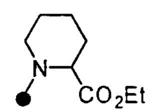 | | | |

Example 270

Ethyl N-[4-({1-[2-(1-benzyl-6-nitro-1H-indol-3-yl)-3,3,3-trifluoro-2-hydroxypropyl]piperidin-4-yl}oxy)-3-methoxybenzoyl]-N-methylglycinate

MS (ESI+) 713, 2.15($M^+$+1, detection time)

Example 271

Ethyl N-[4-({1-[2-(1-benzyl-6-nitro-1H-indol-3-yl)-3,3,3-trifluoro-2-hydroxypropyl]piperidin-4-yl}oxy)-3-methoxybenzoyl] glycinate

MS (ESI+) 699, 2.13($M^+$+1, detection time)

Example 272

Ethyl N-[4-({1-[2-(1-benzyl-6-nitro-1H-indol-3-yl)-3,3,3-trifluoro-2-hydroxypropyl]piperidin-4-yl}oxy)-3-methoxybenzoyl]-β-alaninate

MS (ESI+) 713, 2.14($M^+$+1, detection time)

Example 273

Ethyl 1-[4-({1-[2-(1-benzyl-6-nitro-1H-indol-3-yl)-3,3,3-trifluoro-2-hydroxypropyl]piperidin-4-yl}oxy)-3-methoxybenzoyl] piperidine-4-carboxylate

MS (ESI+) 753, 2.21($M^+$+1, detection time)

Example 274

Ethyl 1-[4-({1-[2-(1-benzyl-6-nitro-1H-indol-3-yl)-3,3,3-trifluoro-2-hydroxypropyl]piperidin-4-yl}oxy)-3-methoxybenzoyl] piperidine-3-carboxylate

MS (ESI+) 753, 2.22($M^+$+1, detection time)

Example 275

Ethyl 1-[4-({1-[2-(1-benzyl-6-nitro-1H-indol-3-yl)-3,3,3-trifluoro-2-hydroxypropyl]piperidin-4-yl}oxy)-3-methoxybenzoyl] piperidine-2-carboxylate

MS (ESI+) 753, 2.27($M^+$+1, detection time)

Example 276

Benzyl 1-{[4-({1-[2-(1-benzyl-6-nitro-1H-indol-3-yl)-3,3,3-trifluoro-2-hydroxypropyl]piperidin-4-yl}oxy)-3-methoxybenzoyl]amino}cyclobutanecarboxylate

MS (ESI+) 801, 2.31($M^+$+1, detection time)

Example 277

tert-Butyl N-[4-({1-[2-(1-benzyl-6-nitro-1H-indol-3-yl)-3,3,3-trifluoro-2-hydroxypropyl]piperidin-4-yl}oxy)-3-methoxybenzoyl]-D-valinate

MS (ESI+) 769, 2.39($M^+$+1, detection time)
Example 278

2-(1-Benzyl-6-nitro-1H-indol-3-yl)-1,1,1-trifluoro-3-[4-(1H-tetrazol-5-yl)-piperidin-1-yl]propan-2-ol

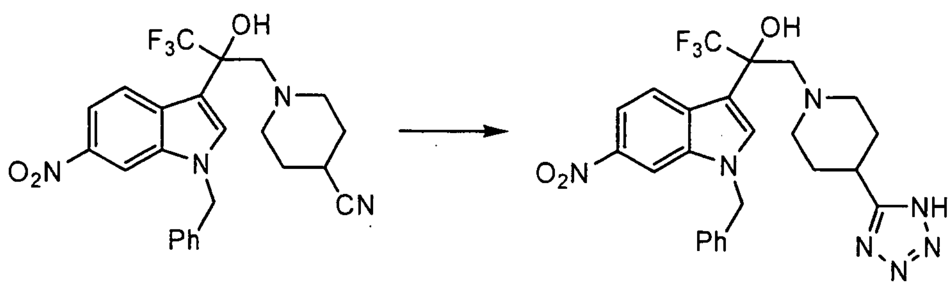

To a solution of 1-[2-(1-benzyl-6-nitro-1H-indol-3-yl)-3,3,3-trifluoro-2-hydroxypropyl]piperidine-4-carbonitrile (the compound of Example 381 as described hereinbelow) (236 mg) in toluene (5 ml) were added sodium azide (97 mg) and triethylamine hydrochloride (207 mg), and the mixture was stirred at 100°C for 30 hours. To the reaction solution was added 1N hydrochloric acid, and the mixture was concentrated under reduced pressure. Ethyl acetate was added to the residue to give the title compound (230 mg).
MS (ESI+) 516, 1.93($M^{+}$+1, detection time)

Example 279

1-(3-Fluorobenzyl)-3-[1-hydroxy-1-(trifluoromethyl)but-3-en-1-yl]-1H-indole-6-carbonitrile

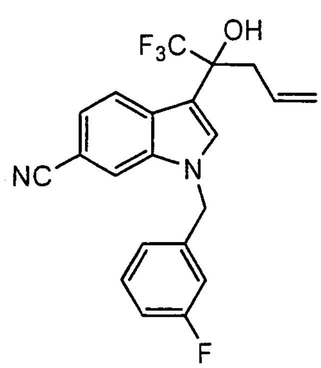

The title compound was obtained in a similar manner to the preparation of the compound of Example 8.
MS (ESI+) 389, 2.89($M^{+}$+1, detection time)

Example 280

2-(1-Benzyl-6-nitro-1H-indol-3-yl)-1,1,1-trifluoro-3-methoxypropan-2-ol

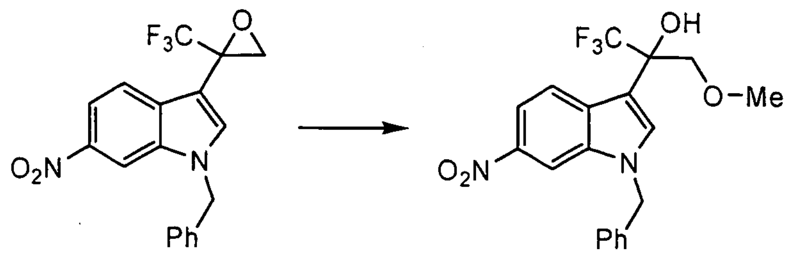

To a solution of the compound of Reference Example 3 (36 mg) in methanol (3 ml) was added lithium bis(trifluoromethanesulfonyl)imide (14.3 mg), and the mixture was stirred at 25°C for one week. The obtained reaction solution was purified by silica gel column chromatography to give the title compound.
MS (ESI+) 395, 2.29($M^{+}$+1, detection time)

Example 281

tert-Butyl 4-[2-(1-benzyl-6-nitro-1H-indol-3-yl)-3,3,3-trifluoro-2-hydroxypropoxy]piperidine-1-carboxylate

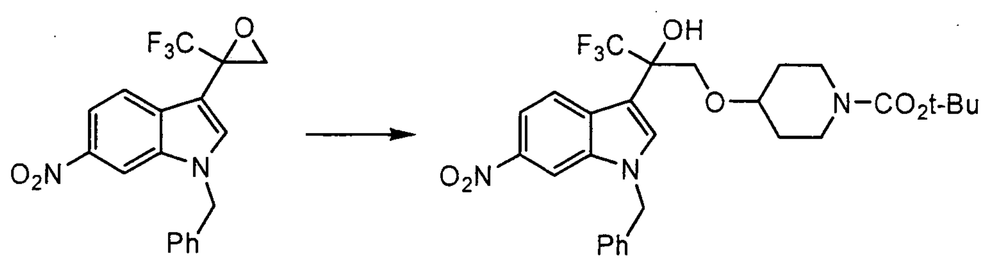

To a solution of t-butyl 4-hydroxy-1-piperidinecarboxylate (60 mg) in tetrahydrofuran (1 ml) was added potassium t-butoxide (33.6 mg), and the mixture was stirred at 25°C for 10 minutes. To the reaction solution was added the compound of Reference Example 3 (54 mg), and the mixture was stirred at 25°C for 10 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (38 mg). $^{1}$H-NMR (400 MHz, $CDCl_3$) δ 8.28 (d, J= 2.0 Hz, 1H), 8.03 (dd, J= 9.0, 2.0 Hz, 1H), 7.96 (d, J= 9.0 Hz, 1H), 7.48 (s, 1H), 7.38-7.28 (m, 3H), 7.16-7.09 (m, 2H), 5.39 (s, 2H), 4.15 (d, J= 9.5 Hz, 1H), 3.87 (d, J= 9.5 Hz, 1H), 3.82 (s, 1H), 3.71-3.53 (m, 3H), 3.22-3.09 (m, 2H), 1.88-1.74 (m, 2H), 1.65-1.50 (m, 2H), 1.45 (s, 9H).
MS (ESI+) 564, 2.61($M^{+}$+1, detection time)

Example 282

2-(1-Benzyl-6-nitro-1H-indol-3-yl)-1,1,1-trifluoro-3-phenoxypropan-2-ol

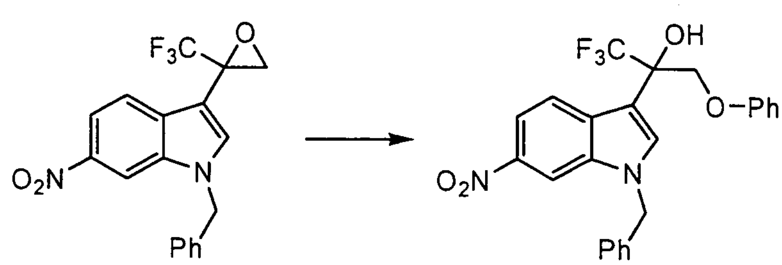

To the compound of Reference Example 3 (54 mg) was added phenol (1 g), and the mixture was stirred at 50°C for 20 hours. Water was added to the reaction solution, and the mixture was extracted with ethyl acetate. The organic layer was washed with 1N aqueous sodium hydroxide solution three times, and further washed with a saturated saline solution, dried over sodium sulfate, and filtered. The filtrate was concentrated under reduced pressure, and the obtained residue was purified by silica gel column chromatography to give the title compound (5.4 mg).
$^1$H-NMR (400 MHz, $CDCl_3$) δ 8.25 (d, J= 2.0 Hz, 1H), 7.76 (dd, J= 9.0, 2.1 Hz, 1H), 7.27 (s, 1H), 7.42-7.30 (m, 4H), 7.25-7.20 (m, 2H), 7.20-7.15 (m, 2H), 6.86-6.72 (m, 3H), 5.45 (s, 2H), 4.49 (d, J= 11.8 Hz, 1H), 4.41 (d, J= 11.8 Hz, 1H). MS (ESI+) 457, 2.35($M^+$+1, detection time)

Example 283

2-(1-Benzyl-6-nitro-1H-indol-3-yl)-1,1,1-trifluoro-3-[2,2,2-trifluoro-1-(trifluoromethyl)ethoxy]propan-2-ol

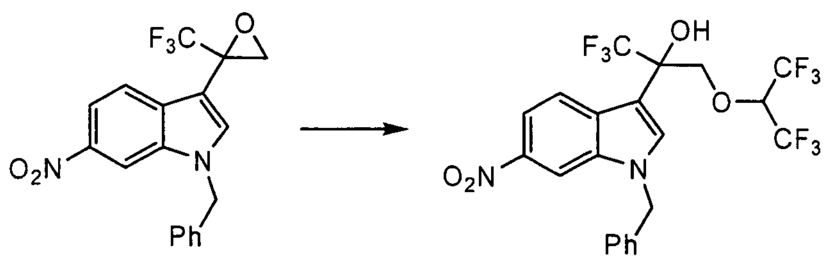

To the compound of Reference Example 3 (362 mg) was added hexafluoroisopropanol (5 ml), and the mixture was stirred at 25°C for one hour. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (397mg).
$^1$H-NMR (400 MHz, $CDCl_3$) δ 8.28 (d, J= 2.0 Hz, 1H), 8.06 (dd, J= 9.0, 2.0 Hz, 1H), 7.88 (d, J= 9.0 Hz, 1H), 7.64 (s, 1H), 7.40-7.28 (m, 3H), 7.26-7.18 (m, 2H), 5.43 (s, 2H), 5.40-5.30 (m, 1H), 4.67-4.51 (m, 2H).
MS (ESI+) 531, 2.66($M^+$+1, detection time)

Example 284

2-(1-Benzyl-6-nitro-1H-indol-3-yl)-3,3,3-trifluoro-2-hydroxypropyl formate

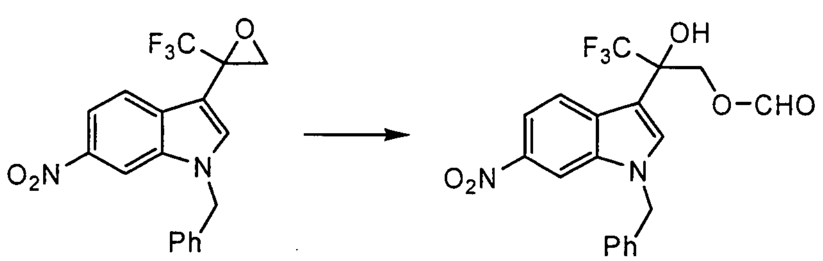

To the compound of Reference Example 3 (362 mg) was added formic acid (5 ml), and the mixture was stirred at 25°C for one hour. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (248 mg).
$^1$H-NMR (400 MHz, $CDCl_3$) δ 8.28 (d, J= 2.0 Hz, 1H), 8.09 (s, 1H), 8.05 (dd, J= 9.0, 2.0 Hz, 1H), 7.98 (d, J= 9.0 Hz, 1H), 7.40-7.30 (m, 3H), 7.16-7.10 (m, 2H), 5.41 (s, 2H), 4.88 (d, J= 12.2 Hz, 1H), 4.70 (d, J= 12.2 Hz, 1H).

MS (ESI+) 409, 2.36($M^{+}$+1, detection time)

Example 285

Ethyl [4-({1-[2-(1-benzyl-6-nitro-1H-indol-3-yl)-3,3,3-trifluoro-2-hydroxypropyl]-piperidin-4-yl}oxy)-3-hydroxyphenyl]acetate

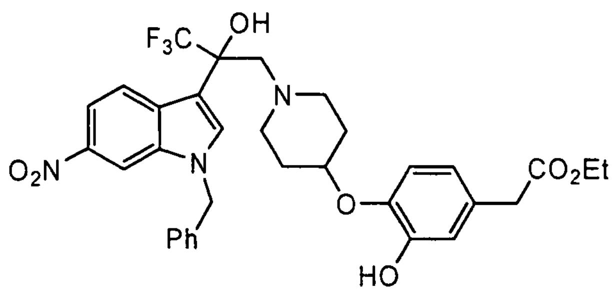

To a solution of the compound of Example 200 (140 mg) in dichloromethane (3 ml) was added dropwise boron tribromide (1.0M $CH_2Cl_2$ solution, 678 μl) at -78°C, and the mixture was warmed to 25°C under stirring over a period of 6 hours. Further, the reaction solution was cooled to -78°C again, and thereto was added dropwise boron tribromide (1.0M $CH_2Cl_2$ solution, 678 μl). The mixture was warmed to 25°C under stirring over a period of 6 hours. To the reaction solution was added a saturated aqueous sodium hydrogen carbonate solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (14.4mg).

$^{1}$H-NMR (400 MHz, $CDCl_3$) δ 8.31 (s, 1H), 8.05 (d, J= 9.0 Hz, 1H), 7.91 (d, J= 9.0 Hz, 1H), 7.67 (s, 1H), 7.38-7.26 (m, 3H), 7.16-7.08 (m, 2H), 6.84 (s, 1H), 6.78-6.66 (m, 2H), 5.44 (d, J= 15.6 Hz, 1H), 5.37 (d, J= 15.6 Hz, 1H), 4.63-4.45 (m, 1H), 4.13 (q, J= 7.1 Hz, 1H), 3.85 (d, J= 13.4 Hz, 1H), 3.76 (d, J= 13.4 Hz, 1H), 3.55-3.35 (m, 2H), 3.49 (s, 2H), 3.28-3.12 (m, 2H), 2.50-2.28 (m, 1H), 2.28-2.07 (m, 2H), 2.07-1.88 (m, 1H), 1.36-1.13 (m, 3H).

Example 286

2-(1-Benzyl-6-nitro-1H-indol-3-yl)-1,1,1-trifluoro-3-(methylthio)propan-2-ol

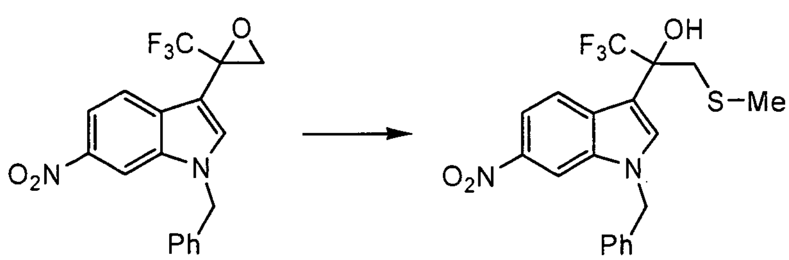

To a solution of the compound of Reference Example 3 (362 mg) in tetrahydrofuran (4 ml) was added sodium methanethiolate (16 mg), and the mixture was stirred at 60°C for 8 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound.

MS (ESI+) 411, 2.38($M^{+}$+1, detection time)

Example 287

2-(1-Benzyl-6-nitro-1H-indol-3-yl)-1,1,1-trifluoro-3-(phenylthio)propan-2-ol

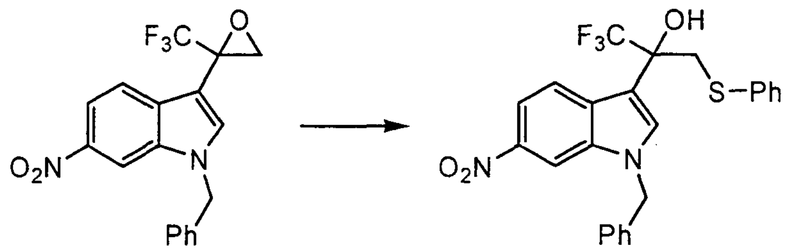

To a solution of the compound of Reference Example 3 (362 mg) in tetrahydrofuran (4 ml) was added sodium thiophenoxide (30 mg), and the mixture was stirred at 60°C for 8 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound.
MS (ESI+) 473, 2.56($M^+$+1, detection time)

Example 288

1-[2-(1-Benzyl-6-nitro-1H-indol-3-yl)-3,3,3-trifluoro-2-hydroxypropyl]pyrrolidin-2-one

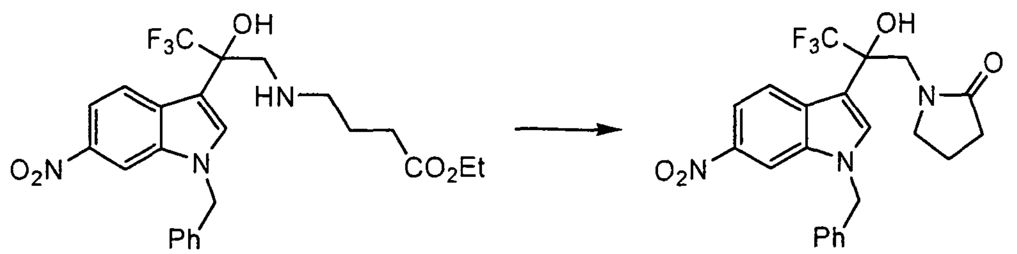

To a solution of the compound of Example 169 (60 mg) in methanol (4 ml) was added potassium carbonate (83 mg), and the mixture was stirred at 25°C for 10 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (32 mg). $^1$H-NMR (400 MHz, $CDCl_3$) δ 8.29 (d, J= 2.0 Hz, 1H), 8.04 (dd, J= 9.0, 2.0 Hz, 1H), 7.89 (d, J= 9.0 Hz, 1H), 7.67 (s, 1H), 7.40-7.28 (m, 3H), 7.11 (dd, J= 7.8, 2.3 Hz, 2H), 6.67 (bs, 1H), 5.41 (s, 2H), 4.13 (d, J= 14.8 Hz, 1H), 3.92 (d, J= 14.8 Hz, 1H), 3.45-3.32 (m, 1H), 3.00-2.88 (m, 1H), 3.48-3.26 (m, 2H), 2.05-1.92 (m, 1H), 1.85-1.70 (m, 1H).
MS (ESI+) 448, 2.27($M^+$+1, detection time)

Example 289

1-[2-(1-Benzyl-6-nitro-1H-indol-3-yl)-3,3,3-trifluoro-2-hydroxypropyl]piperidin-2-one

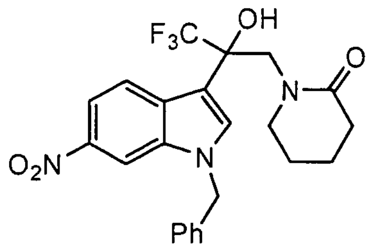

The title compound was obtained from the compound of Example 173 in a similar manner to Example 288.
$^1$H-NMR (400 MHz, $CDCl_3$) δ 8.21 (d, J= 1.9 Hz, 1H), 7.95 (dd, J= 9.0, 1.9 Hz, 1H), 7.83 (d, J= 9.0Hz, 1H), 7.60 (s, 1H), 7.31-7.20 (m, 3H), 7.10-6.98 (m, 2H), 5.33 (s, 2H), 4.24 (d, J= 14.6 Hz, 1H), 3.76 (d, J= 14.6 Hz, 1H), 3.24-3.13 (m, 1H), 2.77-2.68 (m, 1H), 2.44-2.24 (m, 2H), 1.71-1.47 (m, 3H).
MS (ESI+) 462, 2.39($M^+$+1, detection time)

Example 290

2-(1-Benzyl-6-nitro-1H-indol-3-yl)-3-(1,1-dioxidoisothiazolidin-2-yl)-1,1,1-trifluoropropan-2-ol

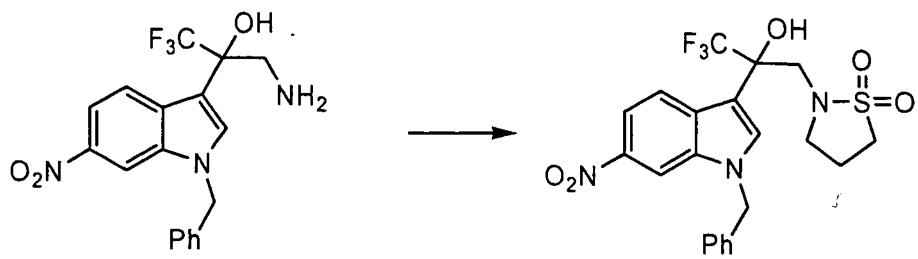

To a solution of the compound of Example 102 (76 mg) in N,N-dimethylformamide (4 ml) were added potassium carbonate (83 mg) and chloropropanesulfonyl chloride (43 mg), and the mixture was stirred at 25°C for 10 hours. Water was added to the reaction solution, and the mixture was extracted with ethyl acetate. The organic layer was washed with a saturated saline solution, dried over sodium sulfate, and filtered. The filtrate was concentrated under reduced pressure, and the obtained residue was purified by silica gel column chromatography to give the title compound (63 mg).
$^1$H-NMR (400 MHz, $CDCl_3$) δ 8.28 (d, J= 2.0 Hz, 1H), 8.03 (dd, J= 9.0, 2.0 Hz, 1H), 7.94 (d, J= 9.0 Hz, 1H), 7.61 (s, 1H), 7.42-7.29 (m, 2H), 5.40 (s, 2H), 4.08 (bs, 1H), 3.90 (d, J= 15.6 Hz, 1H), 3.79 (d, J= 15.6 Hz, 1H), 3.45-3.36 (m, 1H), 3.25-3.09 (m, 2H), 3.09-2.98 (m, 1H), 2.40-2.18 (m, 2H).
MS (ESI+) 484, 2.26($M^+$+1, detection time)

Example 291

1-[2-(1-Benzyl-6-nitro-1H-indol-3-yl)-3,3,3-trifluoro-2-hydroxypropyl]tetrahydropyrimidin-2(1H)-one

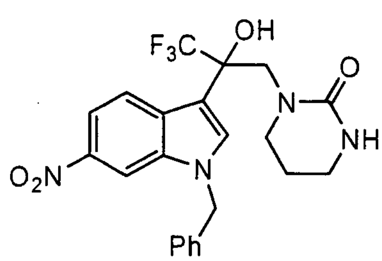

The title compound was obtained from the compound of Example 168 in a similar manner to Example 288.
MS (ESI+) 463, 1.95($M^{+}$+1, detection time)

Example 292

2-(1-Benzyl-6-nitro-1H-indol-3-yl)-1,1,1-trifluoro-3-[4-(piperidin-4-ylcarbonyl)-piperazin-1-yl]propan-2-ol dihydrochloride

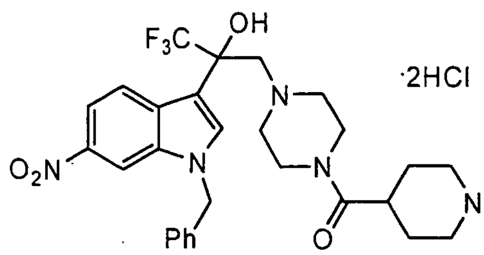

The title compound was obtained from tert-butyl 4-({4-[2-(1-benzyl-6-nitro-1H-indol-3-yl)-3,3,3-trifluoro-2-hydroxypropyl] piperazin-1-yl}carbonyl)-piperidin-1-carboxylate (the compound of Example 648 as described hereinbelow) in a similar manner to the preparation of the compound of Example 101.
MS (ESI+) 560, 1.89($M^{+}$+1, detection time)

Example 293

{6-Cyano-3-[1-({4-[4-(2-ethoxy-2-oxoethyl)-2-methoxyphenoxy]piperidin-1-yl}-methyl)-2,2,2-trifluoro-1-hydroxyethyl]-1H-indol-1-yl}acetic acid

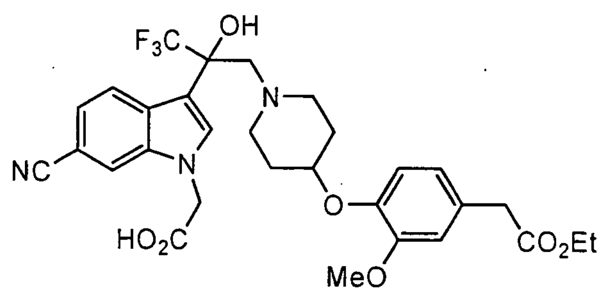

In a similar manner to the preparation of the compound of Example 101, the title compound was obtained from ethyl {4-[(1-{2-[1-(2-tert-butoxy-2-oxoethyl)-6-cyano-1H-indol-3-yl]-3,3,3-trifluoro-2-hydroxypropyl}piperidin-4-yl)oxy]-3-methoxyphenyl}acetate (the compound of Example 328 as described hereinbelow).
$^{1}$H-NMR (400 MHz, $CDCl_3$) δ 8.02 (d, J= 8.5 Hz, 1H), 7.61 (s, 1H), 7.46 (s, 1H), 7.43 (dd, J= 8.5, 1.3 Hz, 1H), 6.78-6.70 (m, 3H), 4.91 (d, J= 18.0 Hz, 1H), 4.84 (d, J= 18.0 Hz, 1H), 4.54 (bs, 1H), 4.23-4.11 (m, 2H), 3.91 (d, J= 13.5 Hz, 1H), 3.65 (d, J= 13.5 Hz, 1H), 3.63 (s, 3H), 3.56 (s, 2H), 3.50-3.40 (m, 1H), 3.25-3.12 (m, 1H), 2.78-2.62 (m, 2H), 2.48-2.32 (m, 1H), 2.21-2.09 (m, 1H), 1.96-1.75 (m, 2H), 1.29 (t, J=7.1 Hz, 3H). MS (ESI+) 604, 1.95($M^{+}$+1, detection time)

Example 294

{1-[2-(6-Amino-1-benzyl-1H-indol-3-yl)-3,3,3-trifluoro-2-hydroxypropyl]-piperidin-4-yl}(phenyl)methanone

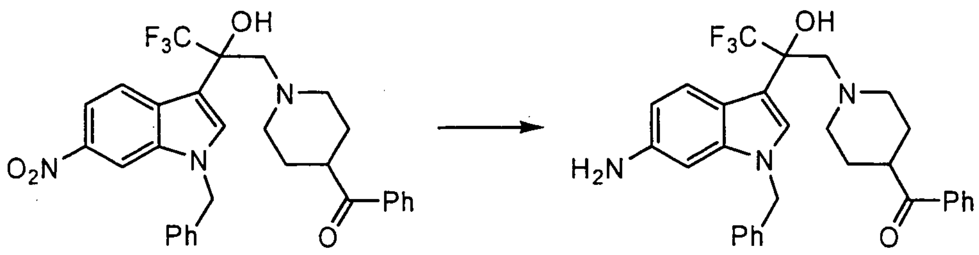

To a solution of the compound of Example 67 (151 mg) in ethanol (2 ml) were added ammonium formate (581 mg), 10 % palladium carbon (29 mg), and the mixture was stirred at 80˚C for 2.5 hours. The insoluble materials were removed by filtration on celite, and a 2N aqueous sodium hydroxide solution was added, and the mixture was extracted with ethyl acetate. The organic layer was washed with water and a saturated saline solution, dried over sodium sulfate, and filtered. The filtrate was concentrated under reduced pressure, and the obtained residue was purified by silica gel column chromatography to give the title compound (65 mg).

$^1$H-NMR (400 MHz, $CDCl_3$) δ 7.53-7.50 (m, 1H), 7.34-7.20 (m, 1H), 7.20-7.08 (m, 3H), 6.59-6.49 (m, 1H), 6.47 (d, J= 9.3 Hz, 1H), 5.18-5.16 (m, 2H), 4.36-4.31 (m, 1H), 3.13 (d, J= 13.6 Hz, 1H), 3.08 (d, J= 13.6 Hz, 1H), 2.49-2.37 (m, 4H), 2.00-1.03 (m, 3H).

MS (ESI+) 522, 1.71($M^+$+1, detection time)

Example 295

N-(3-{1-[(4-Benzoylpiperidin-1-yl)methyl]-2,2,2-trifluoro-1-hydroxyethyl}-1-benzyl-1H-indol-6-yl)methanesulfonamide

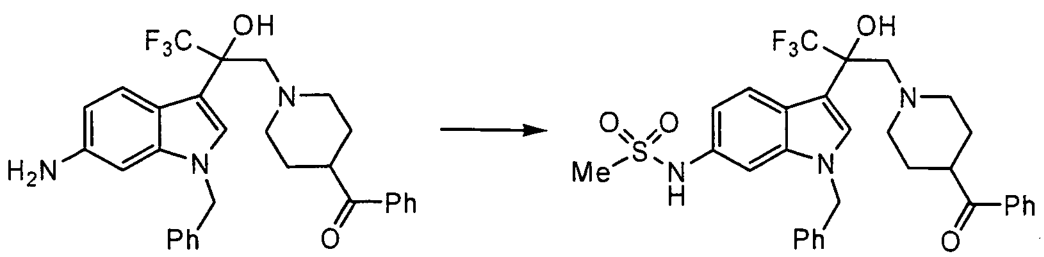

To a solution of the compound of Example 294 (30 mg) in pyridine (1ml) was added methanesulfonyl chloride (13 μl), and the mixture was stirred at 25˚C for 19 hours. To the reaction solution was added a saturated aqueous ammonium chloride solution, and the mixture was extracted with ethyl acetate dried over sodium sulfate, and filtered. The filtrate was concentrated under reduced pressure, and the obtained residue was purified by silica gel column chromatography to give the title compound (21 mg).

$^1$H-NMR (400 MHz, $CDCl_3$) δ8.61 (s, 1H), 7.74-7.68 (m, 2H), 7.39-7.22 (m, 6H), 7.10-7.03 (m, 3H), 6.91-6.88 (m, 2H), 6.44 (bs, 1H), 5.28 (s, 1H), 5.25 (s, 2H), 4.38-4.33 (m, 1H), 3.16-3.10 (m, 2H), 2.89 (s, 3H), 2.58-1.03 (m, 4H).

MS (ESI+) 600, 1.89($M^+$+1, detection time)

In a similar manner to the preparation of the compound of Example 29 the compounds of Examples 296 to 297 having a chemical structure as shown in Table 5 were obtained from the compound of Example 294 as a starting compound.

[Table 5]

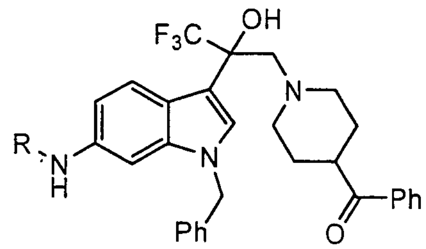

| Exam. | R | | Exam. | R |
|---|---|---|---|---|
| 296 | 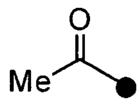 | | 297 | 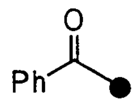 |

Example 296

N-(3-{1-[(4-Benzoylpiperidin-1-yl)methyl)-2,2,2-trifluoro-1-hydroxyethyl}-1-benzyl- 1H-indol-6-yl)acetamide

MS (ESI+) 564, 1.95($M^{+}$+1, detection time)

Example 297

N-(3-{1-[(4-Benzoylpiperidin-1-yl)methyl]-2,2,2-trifluoro-1-hydroxyethyl}-1-benzyl-1H-indol-6-yl)benzamide

$^{1}$H-NMR (400 MHz, $CDCl_3$) δ 8.12 (d, J= 8.1 Hz, 1H), 8.12 (d, J= 8.5 Hz, 1H), 7.90-7.85 (m, 4H), 7.61-7.46 (m, 8H), 7.31 (s, 1H), 7.24-7.20 (m, 3H), 7.10-7.08 (m, 2H), 5.30 (s, 2H), 3.26 (d, J= 13.4 Hz, 1H), 3.19 (d, J= 13.4 Hz, 1H), 3.26-3.19 (m, 1H), 3.03-2.98 (m, 1H), 2.68-2.63 (m, 2H), 2.30-2.27 (m, 1H), 1.88-1.84 (m, 2H), 1.71-1.67 (m, 2H).

Example 298

N-[2-(1-Benzyl-6-nitro-1H-indol-3-yl)-3,3,3-trifluoro-2-hydroxypropyl]-4-bromobenzenesulfonamide

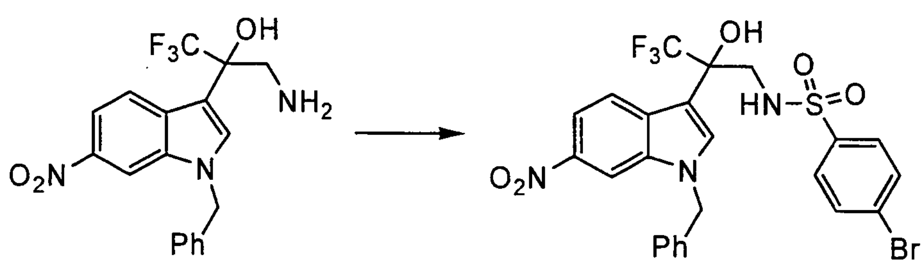

To a solution of the compound of Example 102 (150 mg) in pyridine (2 ml) was added 4-bromobenzenesulfonyl chloride (153 mg), and the mixture was stirred at 25°C for 36 hours. To the reaction solution was added a saturated aqueous ammonium chloride solution, and the mixture was extracted with ethyl acetate. The organic layer was washed with a saturated saline solution, dried over sodium sulfate and filtered. The filtrate was concentrated under reduced pressure, and the obtained residue was purified by silica gel column chromatography to give the title compound (239 mg).
$^{1}$H-NMR (400 MHz, $CDCl_3$) 8 8.52 (bs, 1H), 8.23 (s, 1H), 7.95 (d, J= 9.0 Hz, 1H), 7.73 (d, J= 9.0 Hz, 1H), 7.60 (d, J= 8.8 Hz, 2H), 7.58 (s, 1H), 7.56 (d, J= 8.8 Hz, 2H), 7.39-7.27 (m, 3H), 7.16 (d, J= 8.0 Hz, 2H), 5.37 (s, 2H), 5.02 (bs, 1H), 3.75 (dd, J= 14.3, 6.0 Hz, 1H), 3.65 (dd, J= 14.3, 6.0 Hz, 1H).
MS (ESI+) 598, 2.50($M^{+}$+1, detection time)

Example 299

N-[2-(1-Benzyl-6-nitro-1H-indol-3-yl)-3,3,3-trifluoro-2-hydroxypropyl]benzenesulfonamide

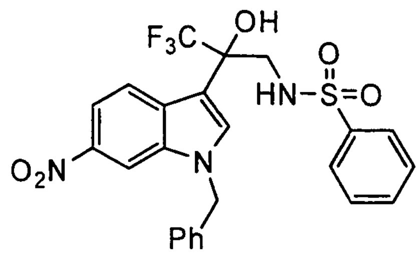

The title compound was obtained in a similar manner to Example 298.
MS (ESI+) 520, 2.38($M^{+}$+1, detection time)

Example 300

N-[2-(1-Benzyl-6-nitro-1H-indol-3-yl)-3,3,3-trifluoro-2-hydroxypropyl]-2-hydroxyacetamide

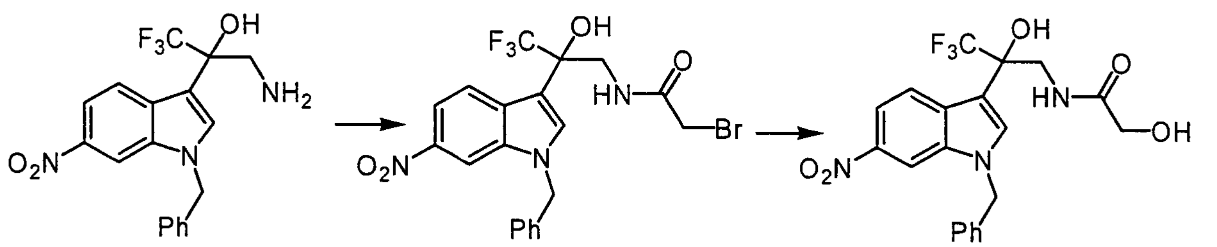

To a solution of the compound of Example 102 (300 mg) in N,N-dimethylformamide (4 ml) were added bromoacetic bromide (72 μl) and potassium carbonate (120 mg), and the mixture was stirred at 25°C for 3 hours. To the reaction solution was added a saturated aqueous ammonium chloride solution, and the mixture was extracted with ethyl acetate. The organic layer was washed with a saturated saline solution, dried over sodium sulfate and filtered. The filtrate was concentrated under reduced pressure, and the obtained residue was purified by silica gel column chromatography to give N-[2-(1-benzyl-6-nitro-1H-indol-3-yl)-3,3,3-trifluoro-2-hydroxypropyl]-2-bromoacetamide (84 mg).
MS (ESI+) 500, 2.30($M^{+}$+1, detection time)
To a solution of methyl4-hydroxyphenylacetate (17 mg) in tetrahydrofuran (2.5 ml) was added sodium hydride (4.5 mg), and the mixture was stirred at 0°C for 5 minutes. To the reaction solution was added N-[2-(1-benzyl-6-nitro-1H-indol-3-yl)-3,3,3-trifluoro-2-hydroxypropyl]-2-bromoacetamide (40 mg), and the mixture was stirred at 25°C for 3 hours. To the reaction solution was added a saturated aqueous ammonium chloride solution, and the mixture was extracted with ethyl acetate. The organic layer was washed with a saturated saline solution, dried over sodium sulfate and filtered. The filtrate was concentrated under reduced pressure, and the obtained residue was purified by silica gel column chromatography to give the title compound (11 mg).
$^{1}$H-NMR (400 MHz, $CDCl_3$) δ 8.30 (d, J= 2.0 Hz, 1H), 7.96 (dd, J= 8.8, 2.0 Hz, 1H), 7.92 (s, 1H), 7.85 (d, J= 8.8 Hz, 1H), 7.26-7.18 (m, 3H), 7.08 (d, J= 8.8 Hz, 1H), 7.26-7.18 (m, 3H), 7.08 (d, J= 8.4 Hz, 2H), 5.48 (s, 2H), 4.50 (s, 1H), 4.25 (d, J= 16.9 Hz, 1H), 4.02 (d, J= 13.2 Hz, 1H), 3.95 (d, J= 13.2 Hz, 1H), 3.80 (d, J= 16.9 Hz, 1H).
MS (ESI+) 438, 2.27($M^{+}$+1, detection time)

Example 301

Methyl 4-(2-{[2-(1-benzyl-6-nitro-1H-indol-3-yl)-3,3, 3-trifluoro-2-hydroxypropyl]-amino}-2-oxoethoxy)benzoate

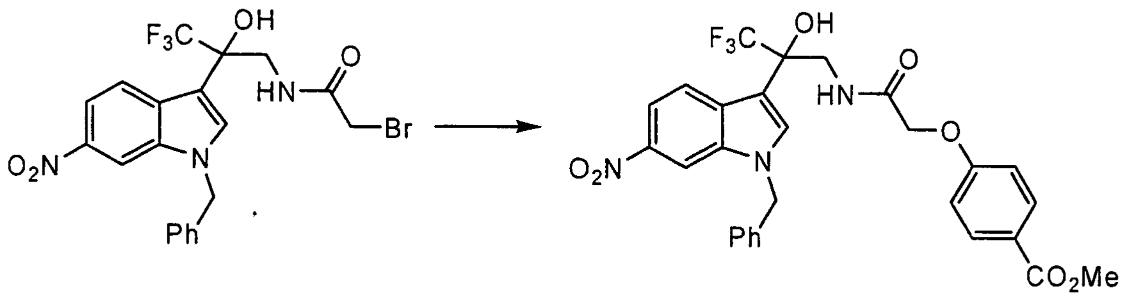

To a solution of the compound of Example 300 (40 mg) in N,N-dimethylformamide (3 ml) were added methyl 4-bromobenzoate (23 mg) and potassium carbonate (28 mg), and the mixture was stirred at 80°C for 8 hours. To the reaction solution was added a saturated aqueous ammonium chloride solution, and the mixture was extracted with ethyl acetate. The organic layer was washed with a saturated saline solution, dried over sodium sulfate and filtered. The filtrate was concentrated under reduced pressure, and the obtained residue was purified by silica gel column chromatography to give the title compound (10 mg).
$^1$H-NMR (400 MHz; $CDCl_3$) δ 8.24 (d, J= 2.0 Hz, 1H), 8.00 (dd, J= 9.0, 2.0 Hz, 1H), 7.83 (d, J= 9.0 Hz, 3H), 7.59 (s, 1H), 7.31-7.30 (m, 3H), 7.12-7.11 (m, 2H), 6.80, (bt, J= 5.8 Hz, 1H), 6.78 (d, J= 9.0 Hz, 1H), 5.40 (d, J= 15.7 Hz, 1H), 5.35 (d, J= 15.7 Hz, 1H), 5.10 (s, 1H), 4.51 (d, J= 15.6 Hz, 1H), 4.46 (d, J= 15.6 Hz, 1H), 4.19-4.07 (m, 2H), 3.90 (s, 3H).
MS (ESI+) 572, 2.42($M^+$+1, detection time)

In a similar manner to the preparation of the compound of Example 301, the compounds of Examples 302 to 303 having a chemical structure as shown in Table 6 were obtained from the compound of Example 300 as a starting compound.

[Table 6]

| 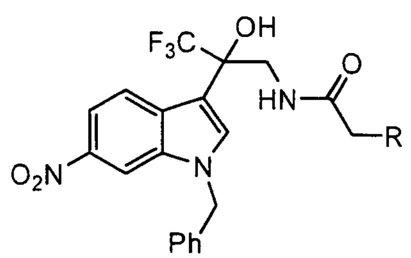 | | | | |
|---|---|---|---|---|
| Exam. | R | | Exam. | R |
| 302 | 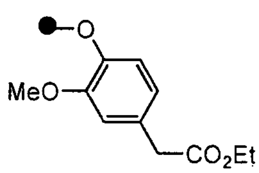 | | 303 | 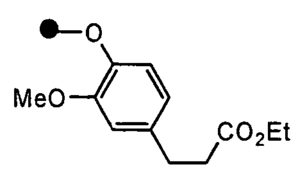 |

Example 302

Ethyl [4-(2-{[2-(1-benzyl-6-nitro-1H-indol-3-yl)-3,3,3-trifluoro-2-hydroxypropyl]-amino}-2-oxoethoxy)-3-methoxyphenyl] acetate

$^1$H-NMR (400 MHz, $CDCl_3$) δ 8.26 (d, J= 2.0 Hz, 1H), 8.02 (dd, J= 9.0, 2.0 Hz, 1H), 7.88 (d, J= 9.0 Hz, 1H),

7.62 (s, 1H), 7.49 (bt, J= 6.1 Hz, 1H), 7.29-7.27 (m, 3H), 7.11-7.08 (m, 2H), 6.74 (d, J= 1.9 Hz, 1H), 6.70 (dd, J= 8.2, 1.9 Hz, 1H), 6.65 (d, J= 8.2 Hz, 1H), 5.48 (s, 1H), 5.37 (s, 2H), 4.47 (d, J= 15.7 Hz, 1H), 4.42 (d, J= 15.7 Hz, 1H), 4.16 (q, J= 7.2 Hz, 2H), 4.17-4.09 (m, 2H), 3.63 (s, 3H), 3.52 (s, 2H), 1.26 (t, J= 7.2 Hz, 3H).
MS (ESI+) 630, 2.46($M^{+}$+1, detection time)

Example 303

Ethyl 3-[4-(2-{[2-(1-benzyl-6-nitro-1H-indol-3-yl)-3,3,3-trifluoro-2-hydroxypropyl]amino}-2-oxoethoxy)-3-methoxyphenyl]propanoate

$^{1}$H-NMR (400 MHz, $CDCl_3$) δ 8.26 (d, J= 2.0 Hz, 1H), 8.03 (dd, J= 9.0, 2.0 Hz, 1H), 7.89 (d, J= 9.0 Hz, 1H), 7.63 (s, 1H), 7.54 (bt, J= 5.6 Hz, 1H), 7.29-7.27 (m, 3H), 7.10-7.07 (m, 2H), 6.66-6.64 (m, 3H), 5.49 (s, 1H), 5.38 (s, 2H), 4.44 (s, 2H), 4.12 (q, J= 7.1 Hz, 2H), 3.62 (s, 3H), 2.86 (t, J= 7.5 Hz, 2H), 2.56 (t, J= 7.5 Hz, 2H), 1.24 (t, J= 7.1 Hz, 3H).
MS (ESI+) 644, 2.51($M^{+}$+1, detection time)

Example 304

N-[2-(1-Benzyl-6-nitro-1H-indol-3-yl)-3,3,3-trifluoro-2-hydroxypropyl]methanesulfonamide

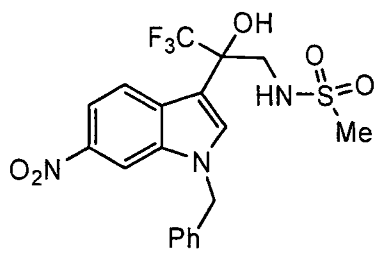

The title compound was obtained in a similar manner to Example 298.
MS (ESI+) 458, 2.22(M++1, detection time)
In a similar manner to the preparation of the compound of Example 1, the compounds of Examples 305 to 465 having a chemical structure as disclosed in Tables 7 to 22 were obtained.
[Table 7]

| 305 | 306 |
|---|---|
| 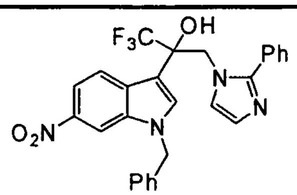 | 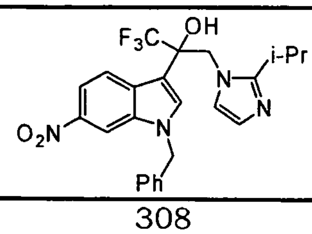 |
| 307 | 308 |
| 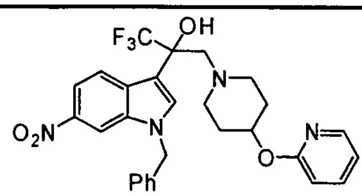 | 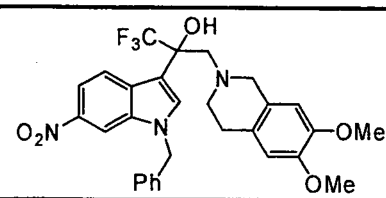 |
| 309 | 310 |
| 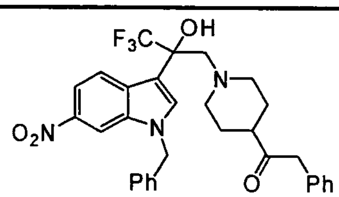 | 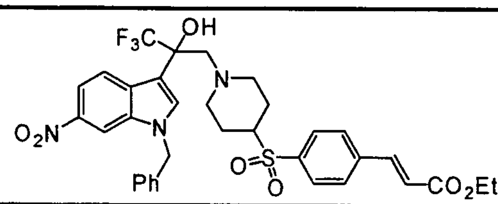 |
| 311 | 312 |
| 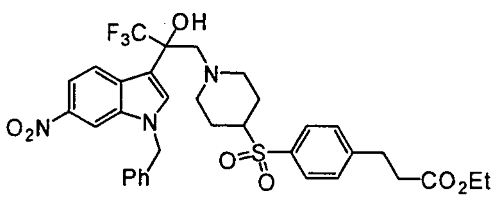 | 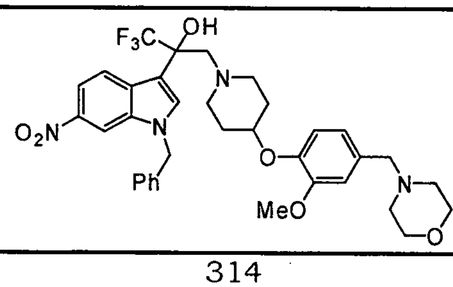 |
| 313 | 314 |
| 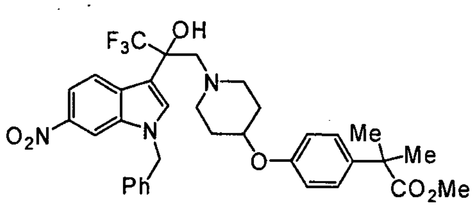 | 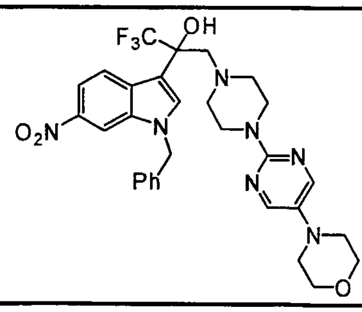 |

[Table 8]

| | |
|---|---|
| 315 | 316 |
| 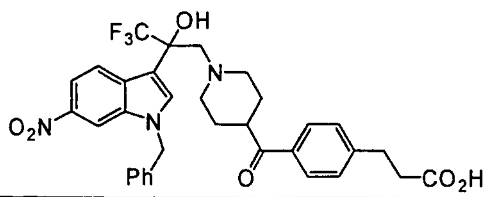 | 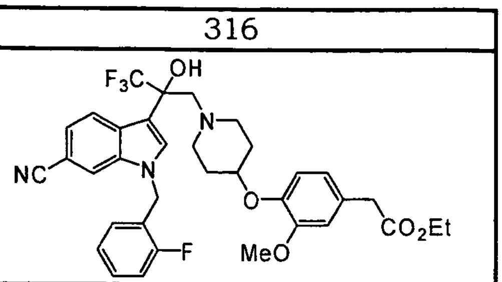 |
| 317 | 318 |
| 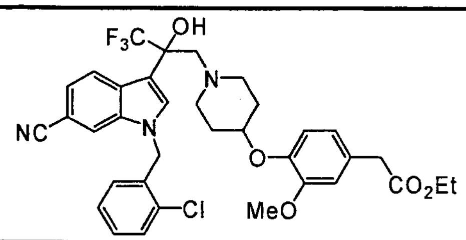 | 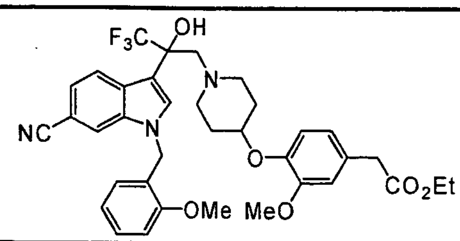 |
| 319 | 320 |
| 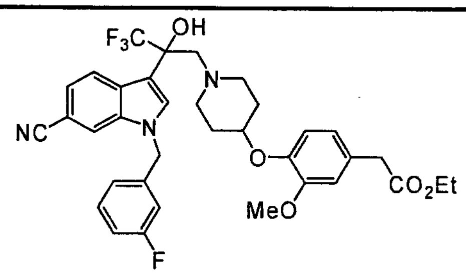 | 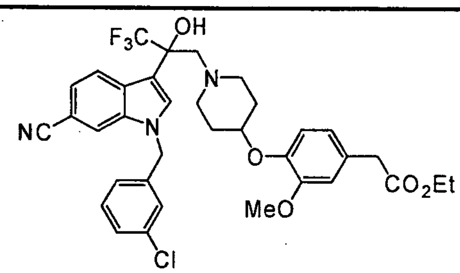 |
| 321 | 322 |
| 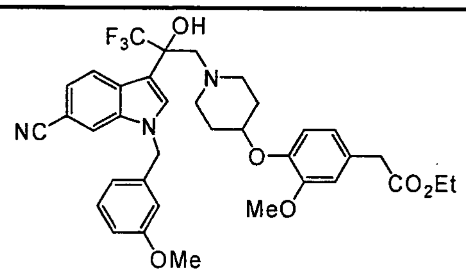 | 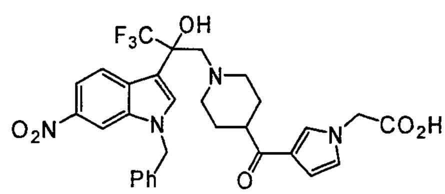 |
| 323 | 324 |
| 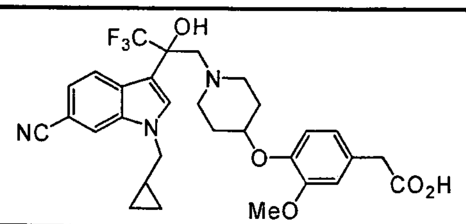 | 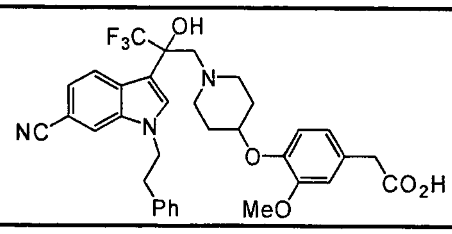 |

[Table 9]

| 325 | 326 |
|---|---|
| 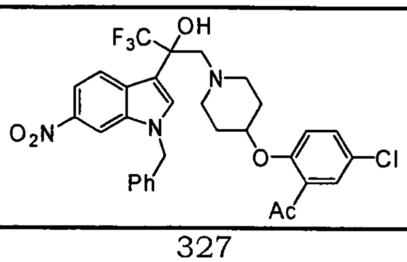 | 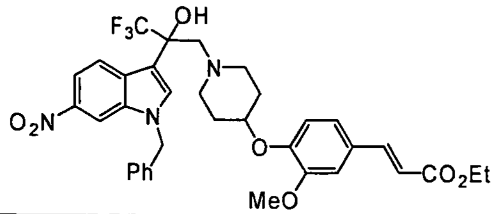 |
| 327 | 328 |
| 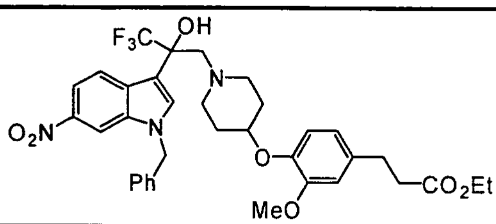 | 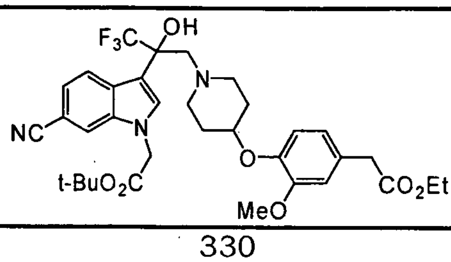 |
| 329 | 330 |
| 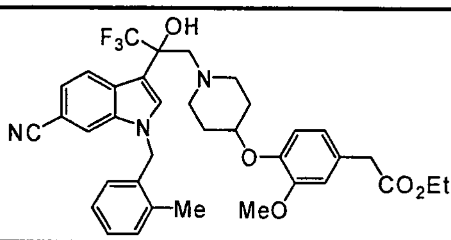 | 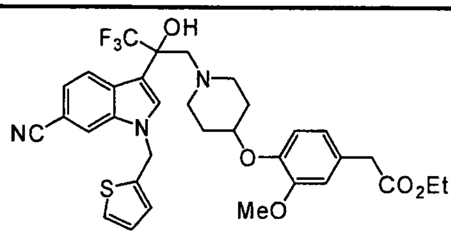 |
| 331 | 332 |
| 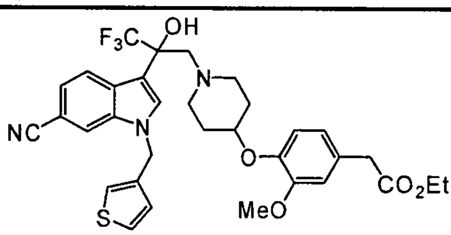 | 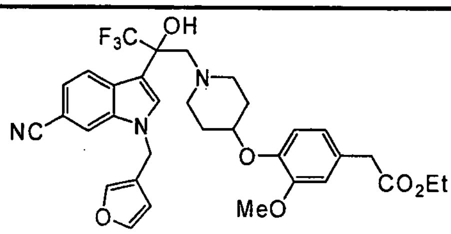 |
| 333 | 334 |
| 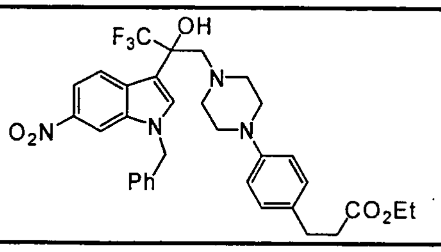 | 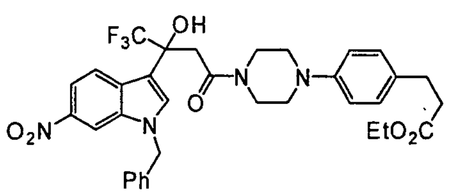 |

[Table 10]

| 335 | 336 |
|---|---|
| 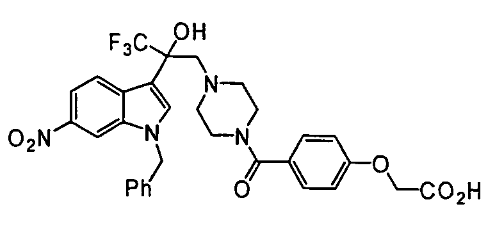 | 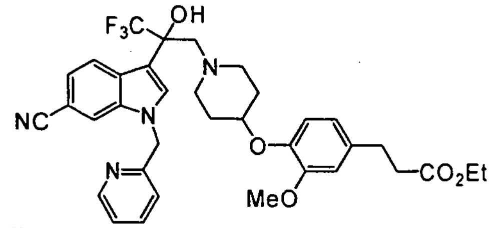 |
| 337 | 338 |
| 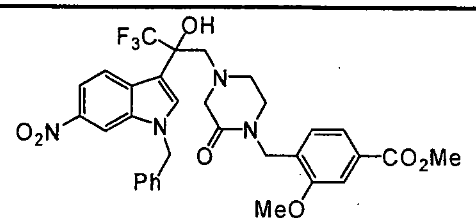 | 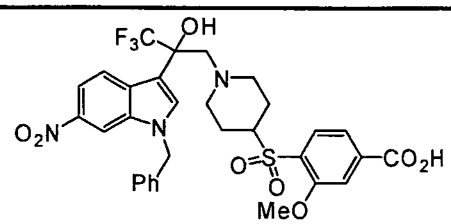 |
| 339 | 340 |
| 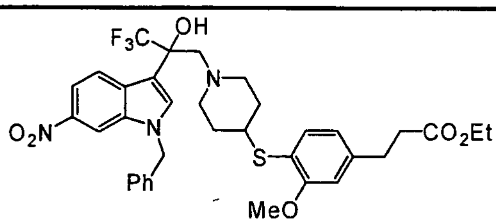 | 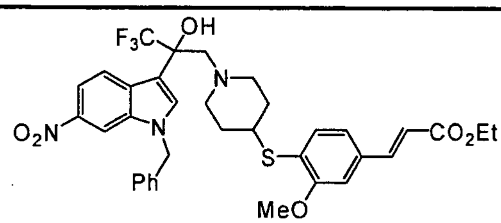 |
| 341 | 342 |
| 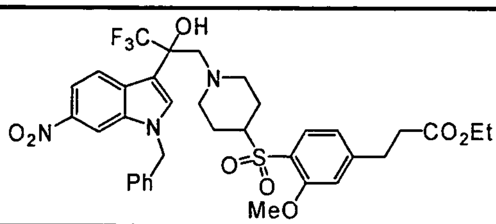 | 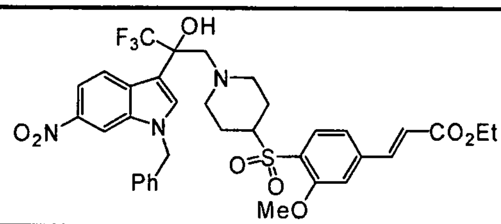 |
| 343 | 344 |
| 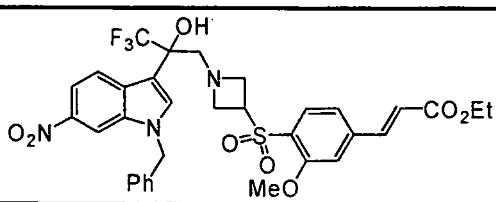 | 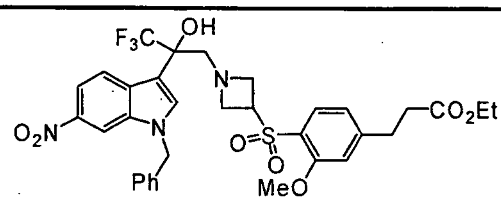 |

[Table 11]

| 345 | 346 |
|---|---|
| 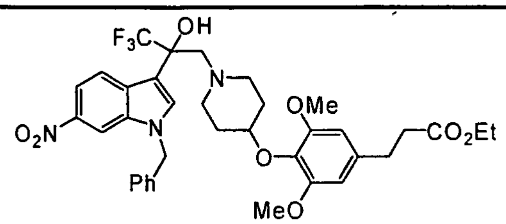 | 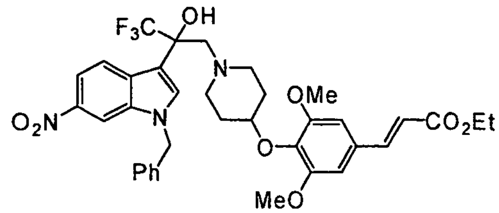 |
| 347 | 348 |
| 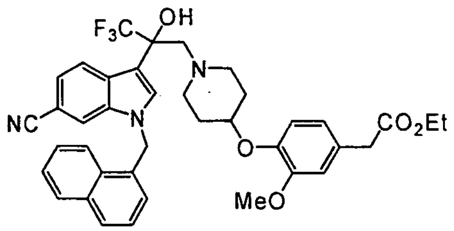 | 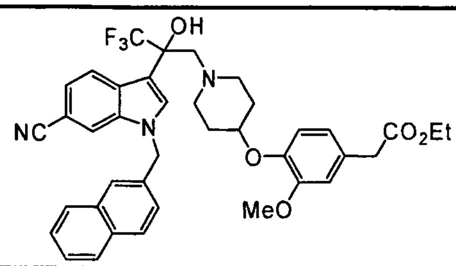 |
| 349 | 350 |
| 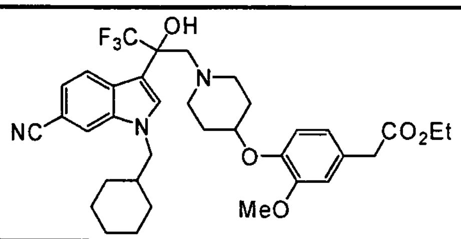 | 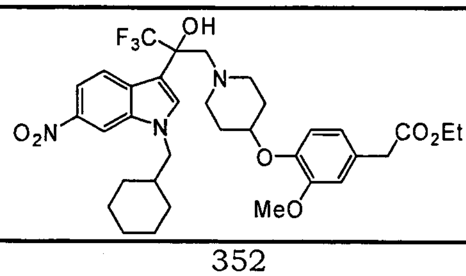 |
| 351 | 352 |
| 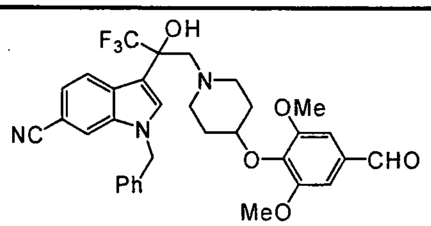 | 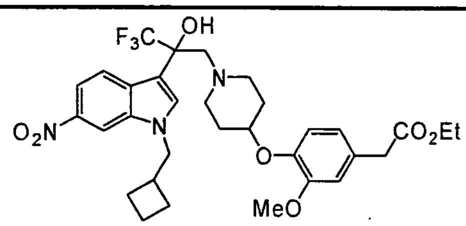 |
| 353 | 354 |
| 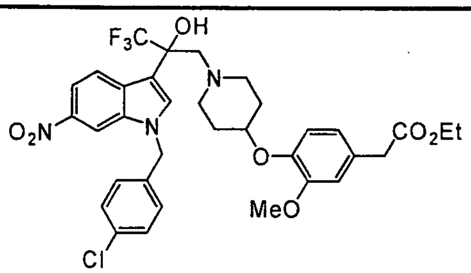 | 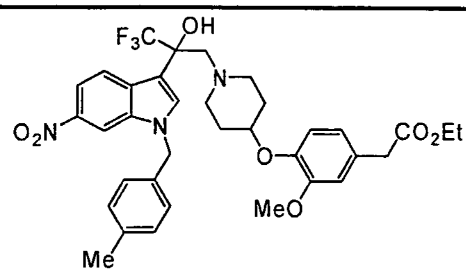 |

[Table 12]

| 355 | 356 |
|---|---|
| 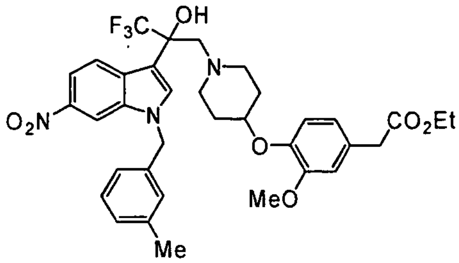 | 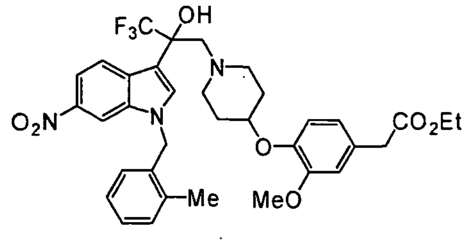 |
| 357 | 358 |
| 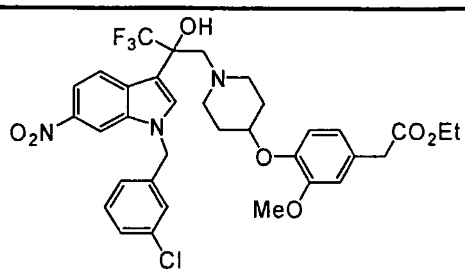 | 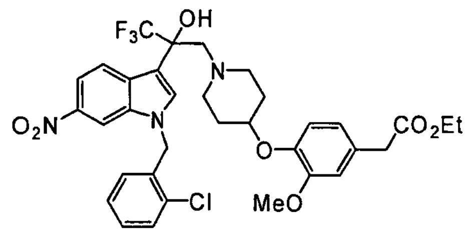 |
| 359 | 360 |
| 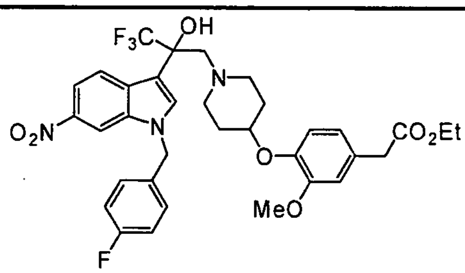 | 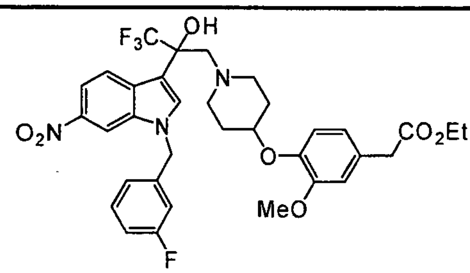 |
| 361 | 362 |
| 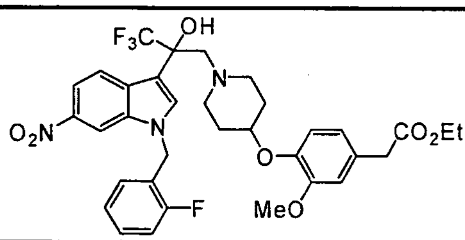 | 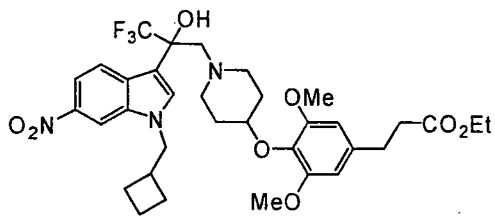 |
| 363 | 364 |
| 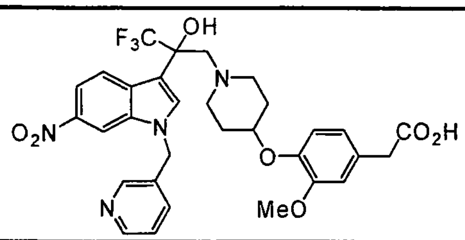 | 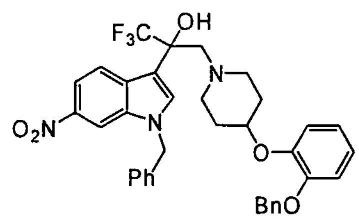 |

[Table 13]

| 365 | 366 |
|---|---|
| 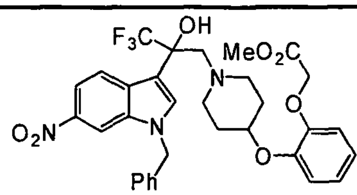 | 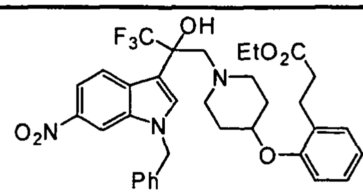 |
| 367 | 368 |
| 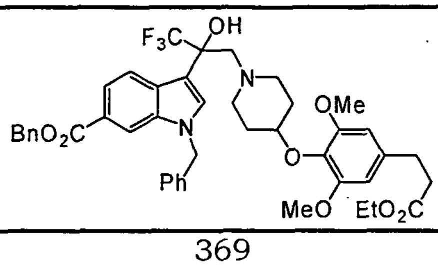 | 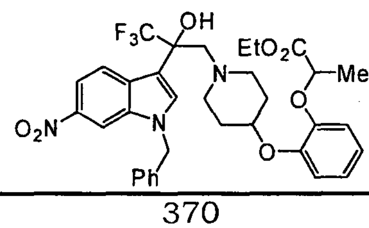 |
| 369 | 370 |
| 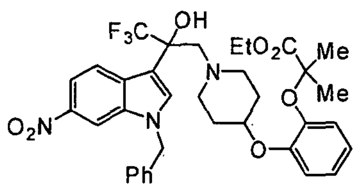 | 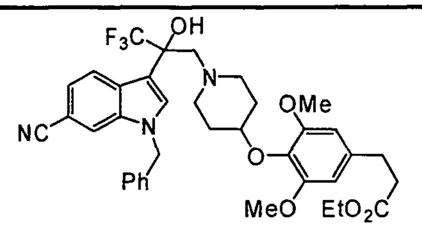 |
| 371 | 372 |
| 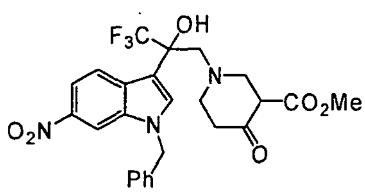 | 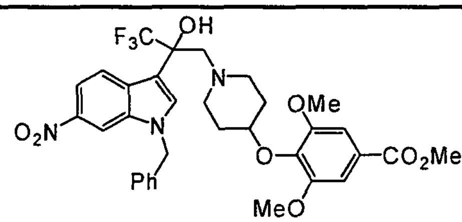 |
| 373 | 374 |
| 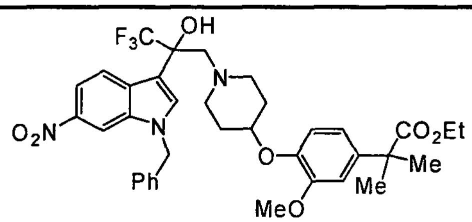 | 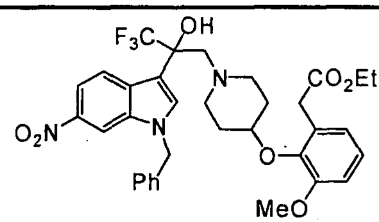 |

[Table 14]

| 375 | 376 |
|---|---|
| 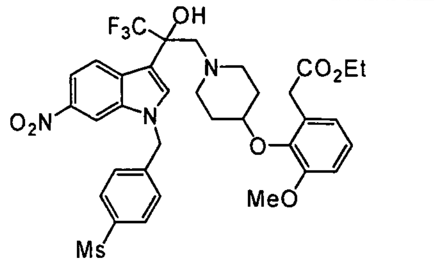 | 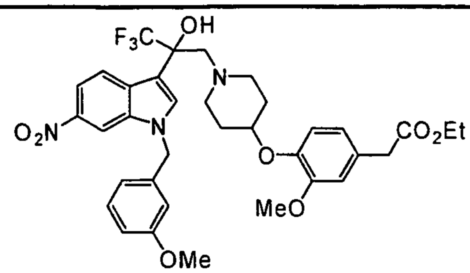 |
| 377 | 378 |
| 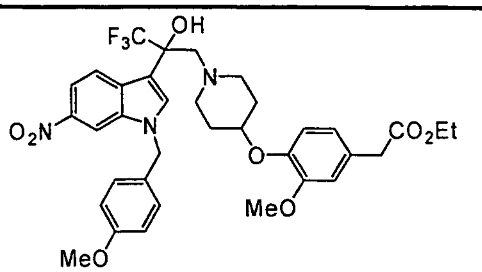 | 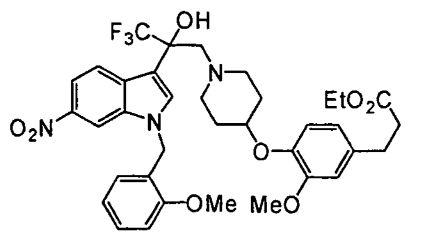 |
| 379 | 380 |
| 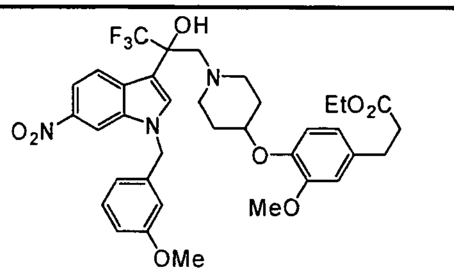 | 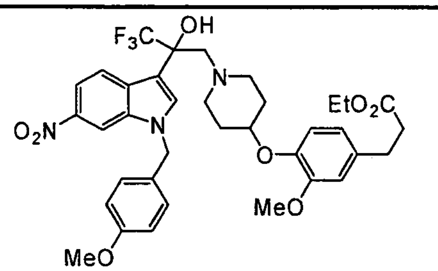 |
| 381 | 382 |
| 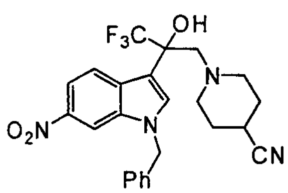 | 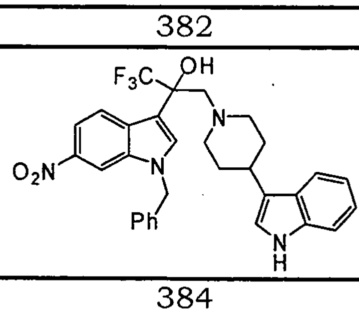 |
| 383 | 384 |
| 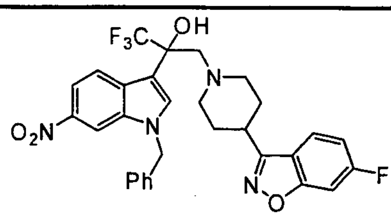 | 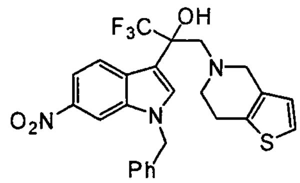 |

[Table 15]

| 385 | 386 |
|---|---|
| 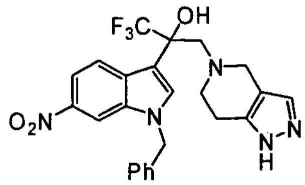 | 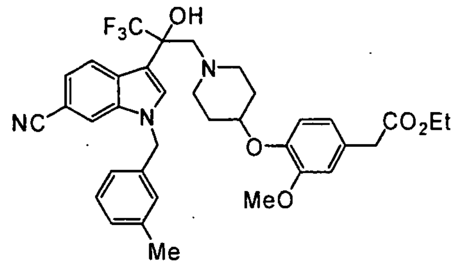 |
| **387** | **388** |
| 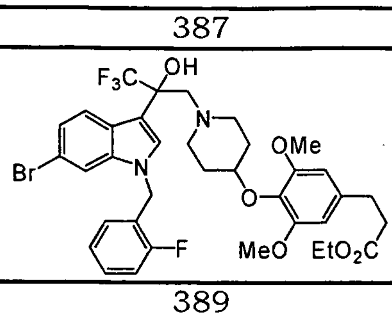 | 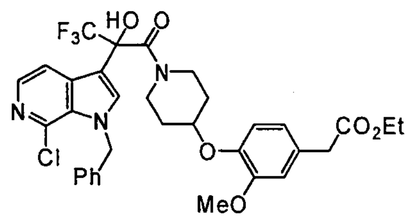 |
| **389** | **390** |
| 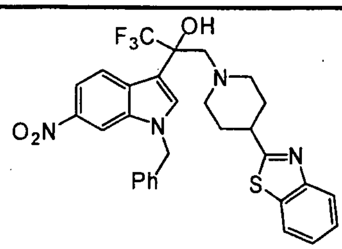 | 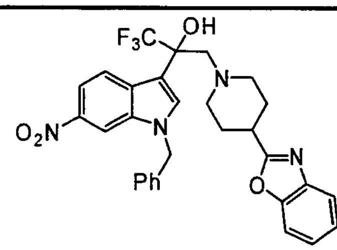 |
| **391** | **392** |
| 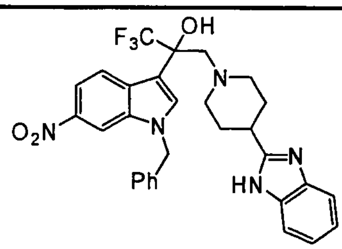 | 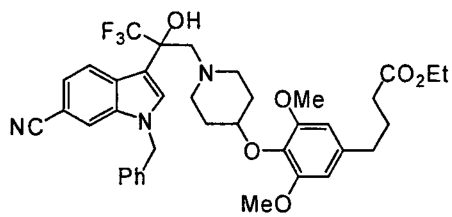 |
| **393** | **394** |
| 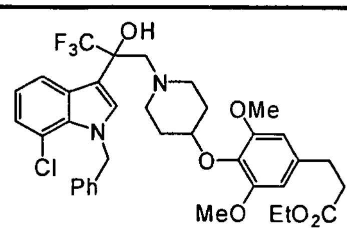 | 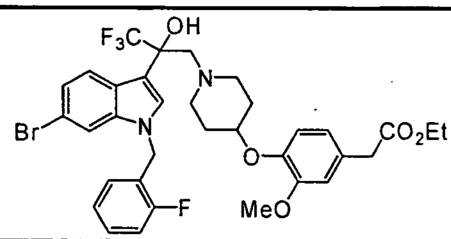 |

[Table 16]

| 395 | 396 |
|---|---|
| 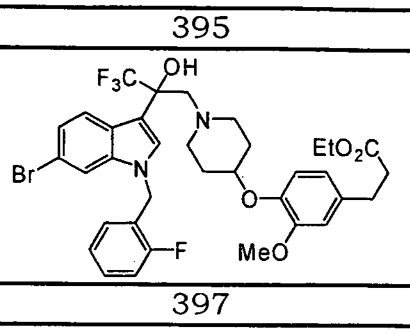 | 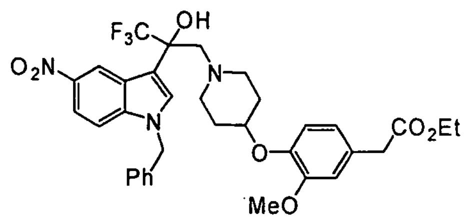 |
| 397 | 398 |
| 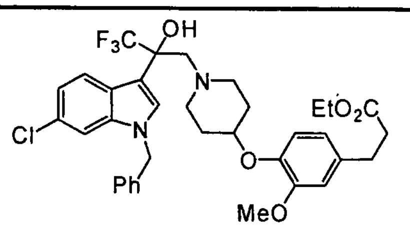 | 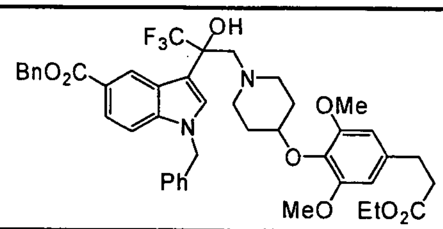 |
| 399 | 400 |
| 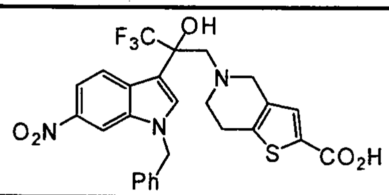 | 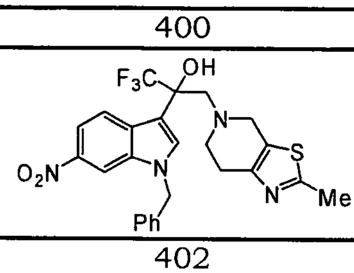 |
| 401 | 402 |
| 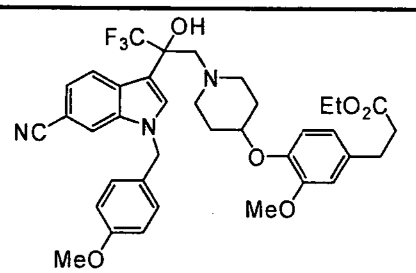 | 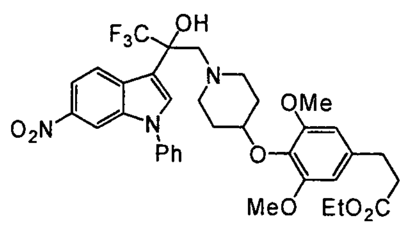 |
| 403 | 404 |
| 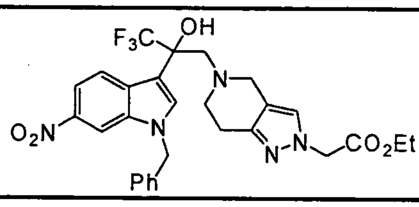 | 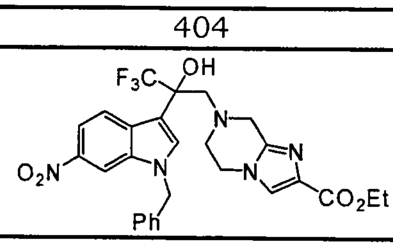 |

[Table 17]

| 405 | 406 |
|---|---|
| 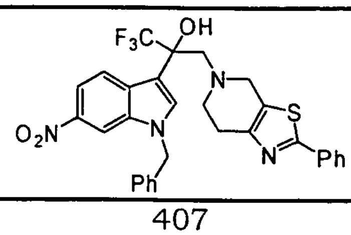 | 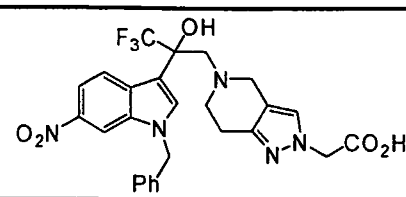 |
| 407 | 408 |
| 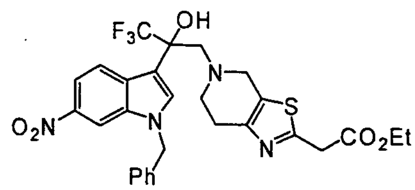 | 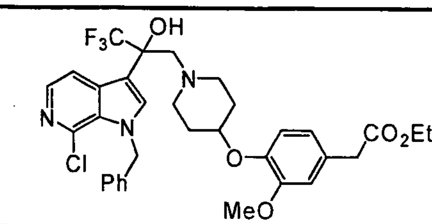 |
| 409 | 410 |
| 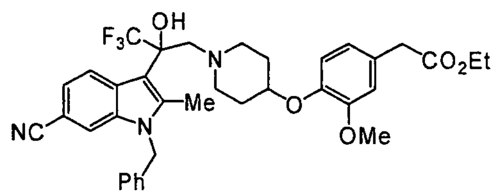 | 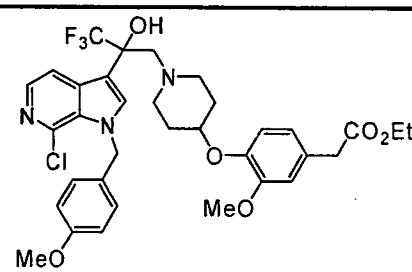 |
| 411 | 412 |
| 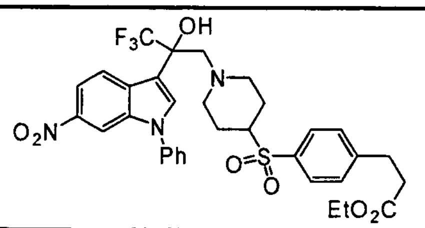 | 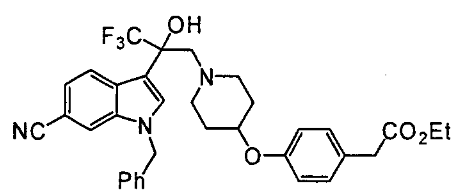 |
| 413 | 414 |
| 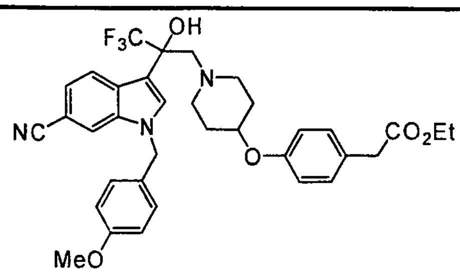 | 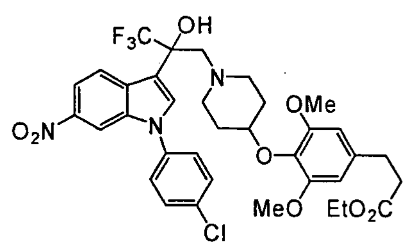 |

[Table 18]

| | |
|---|---|
| 415 | 416 |
| 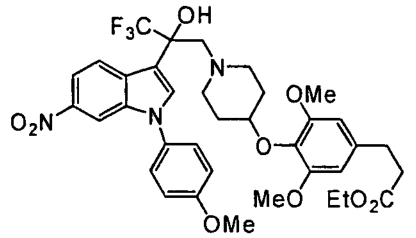 | 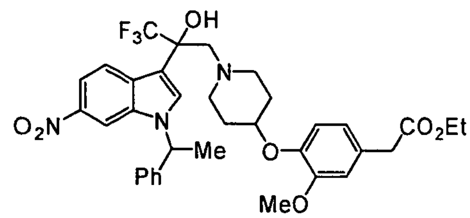 |
| 417 | 418 |
| 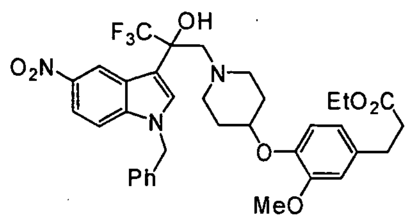 | 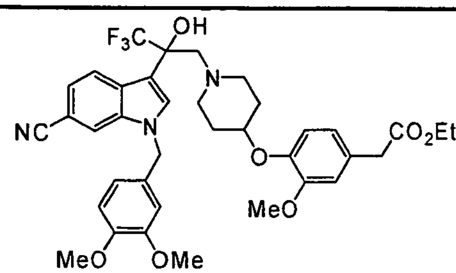 |
| 419 | 420 |
| 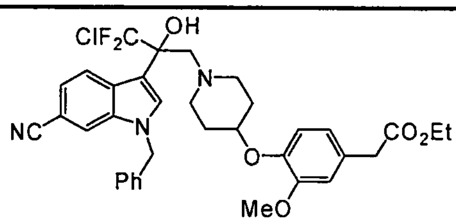 | 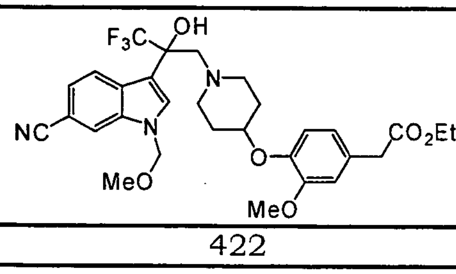 |
| 421 | 422 |
| 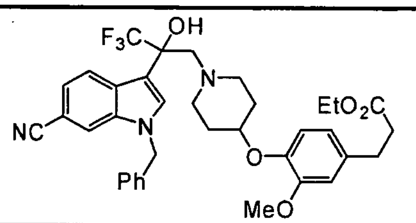 | 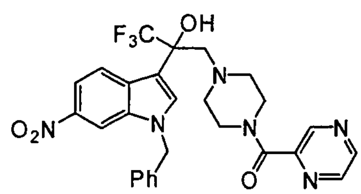 |
| 423 | 424 |
| 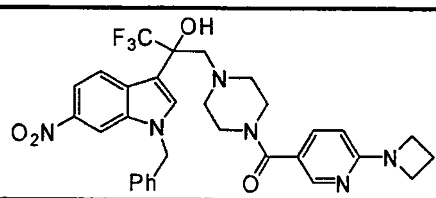 | 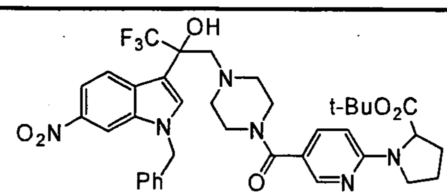 |

[Table 19]

| 425 | 426 |
|---|---|
| 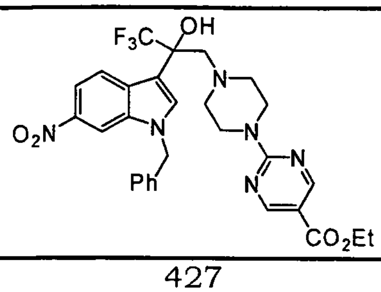 | 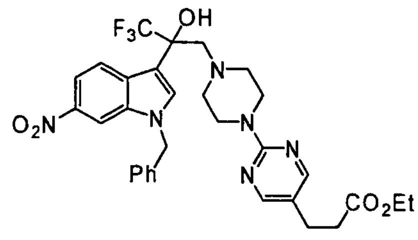 |
| **427** | **428** |
| 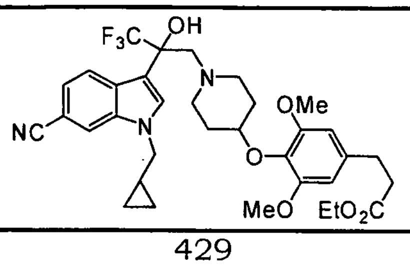 | 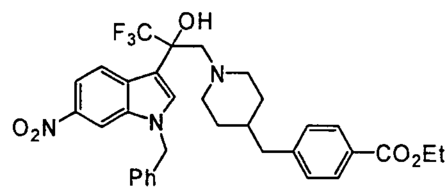 |
| **429** | **430** |
| 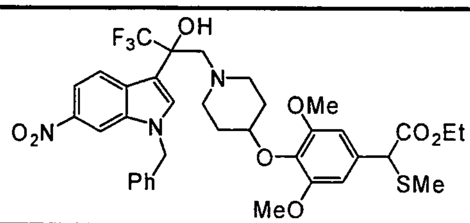 | 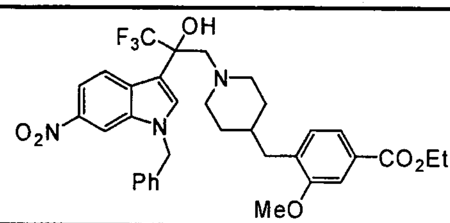 |
| **431** | **432** |
| 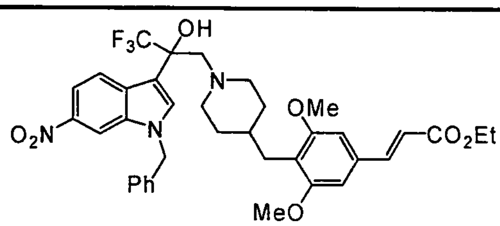 | 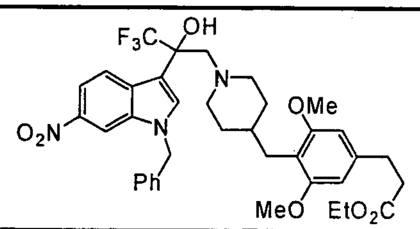 |
| **433** | **434** |
| 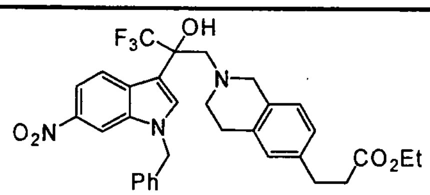 | 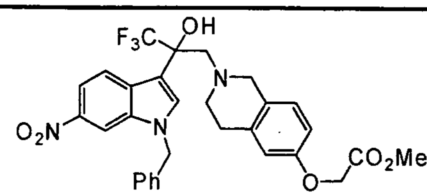 |

[Table 20]

| | |
|---|---|
| 435 | 436 |
| 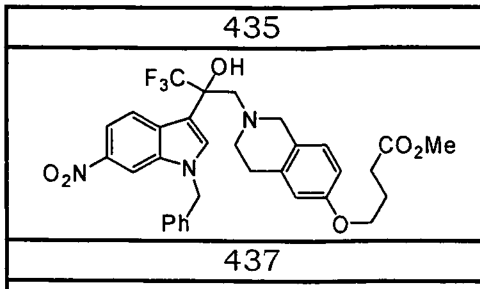 | 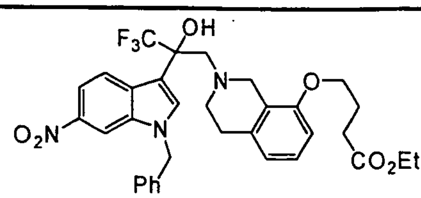 |
| 437 | 438 |
| 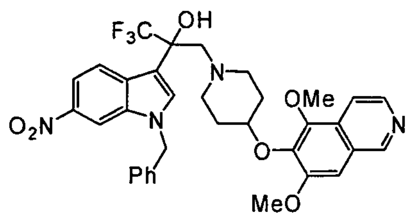 | 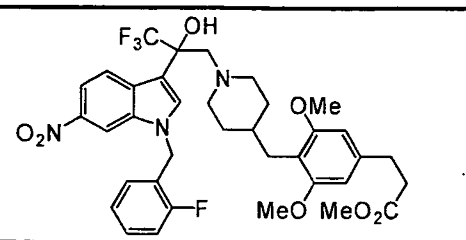 |
| 439 | 440 |
| 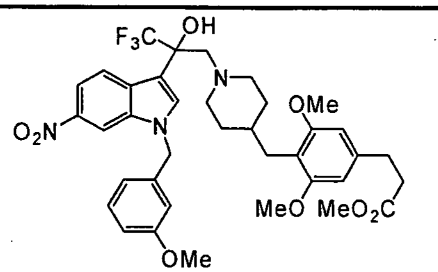 | 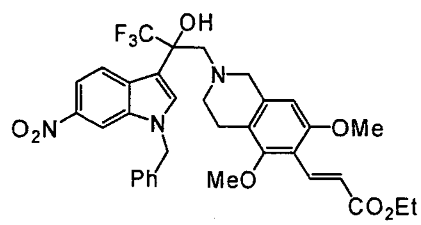 |
| 441 | 442 |
| 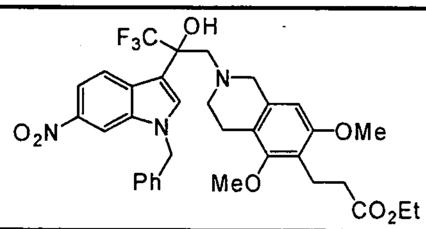 | 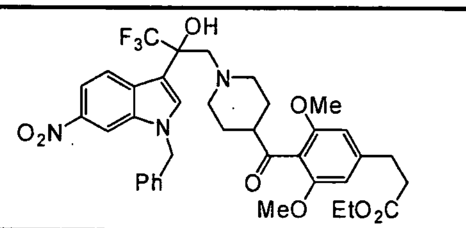 |
| 443 | 444 |
| 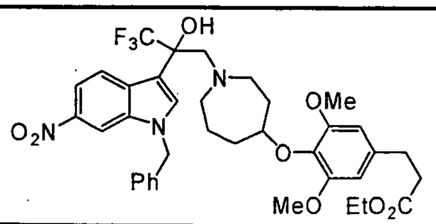 | 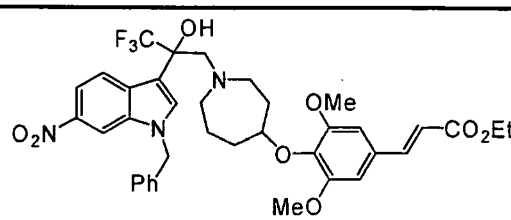 |

[Table 21]

| 445 | 446 |
|---|---|
| 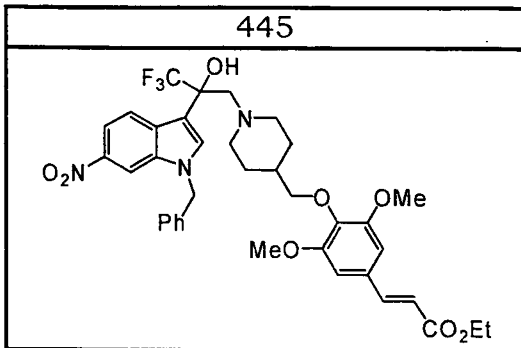 | 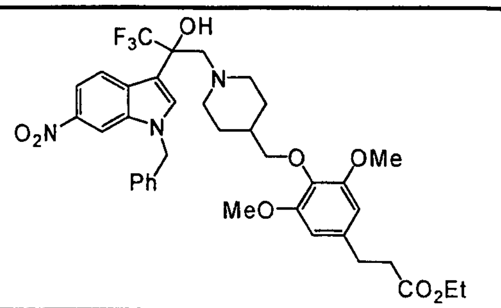 |
| 447 | 448 |
| 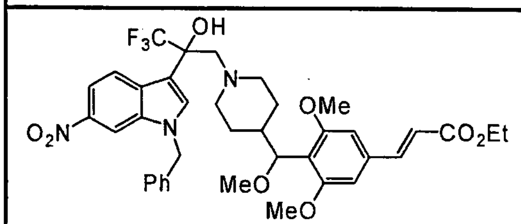 | 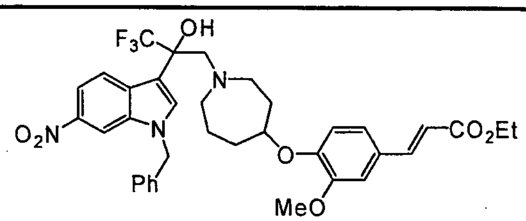 |
| 449 | 450 |
| 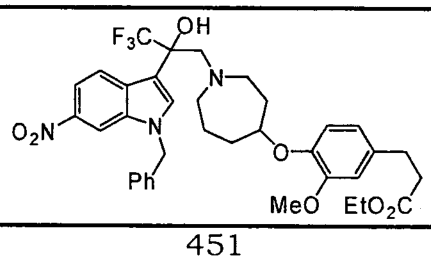 | 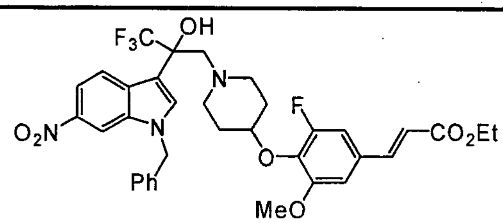 |
| 451 | 452 |
| 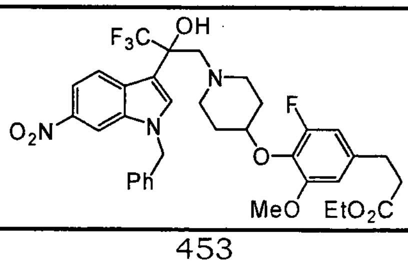 | 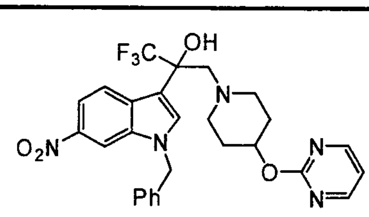 |
| 453 | 454 |
| 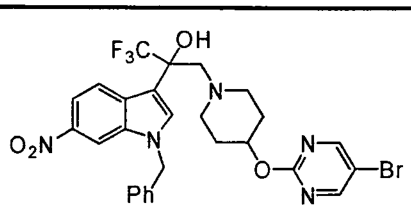 | 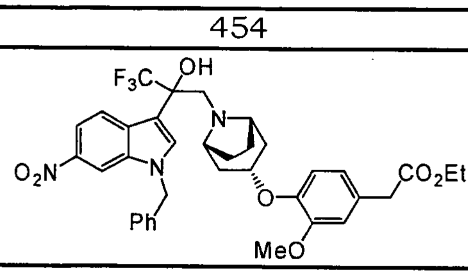 |

[Table 22]

| 455 | 456 |
|---|---|
| 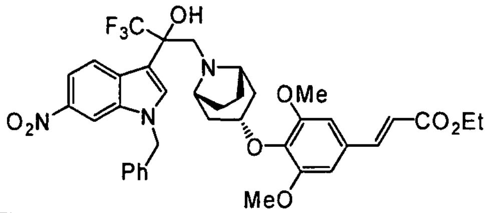 | 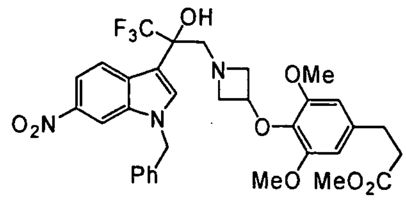 |
| 457 | 458 |
| 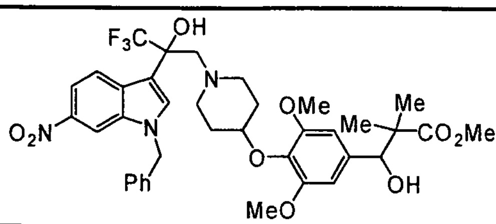 | 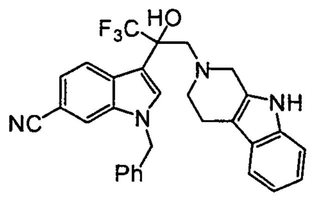 |
| 459 | 460 |
| 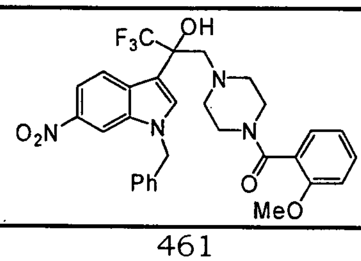 | 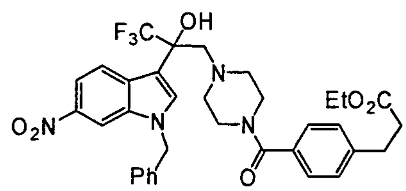 |
| 461 | 462 |
| 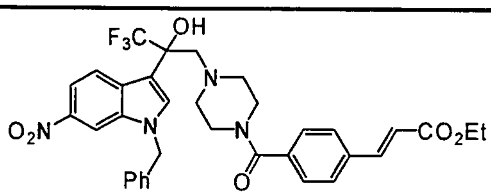 | 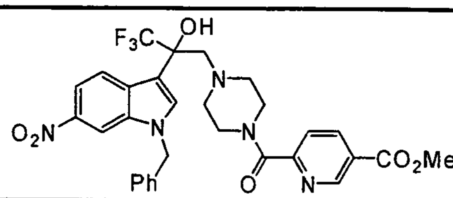 |
| 463 | 464 |
| 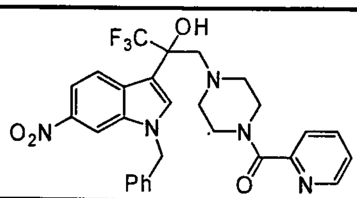 | 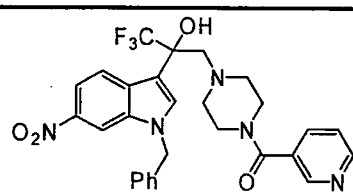 |
| 465 | |
| 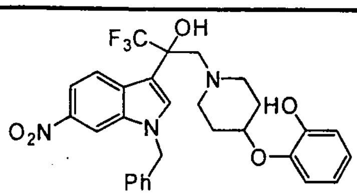 | |

Example 305

2-(1-Benzyl-6-nitro-1H-indol-3-yl)-1,1,1-trifluoro-3-(2-phenyl-1H-imidazol-1-yl)-propan-2-ol

MS (ESI+) 507, 2.78($M^+$+1, detection time)

Example 306

2-(1-Benzyl-6-nitro-1H-indol-3-yl)-1,1,1-trifluoro-3-(2-isopropyl-1H-imidazol-1-yl)propan-2-ol

MS (ESI+) 473, 2.56($M^+$+1, detection time)

Example 307

2-(1-Benzyl-6-nitro-1H-indol-3-yl)-1,1,1-trifluoro-3-[4-(pyridin-2-yloxy)piperidin-1-yl]propan-2-ol

MS (ESI+) 541, 2.13($M^+$+1, detection time)

Example 308

2-(1-Benzyl-6-nitro-1H-indol-3-yl)-3-(6,7-dimethoxy-3,4-dihydroisoquinolin-2(1H)-yl)-1,1,1-trifluoropropan-2-ol

MS (ESI+) 556, 2.09($M^+$+1, detection time)

Example 309

1-{1-[2-(1-Benzyl-6-nitro-1H-indol-3-yl)-3,3,3-trifluoro-2-hydroxypropyl]-piperidin-4-yl}-2-phenylethanone

$^1$H-NMR (400 MHz, $CDCl_3$) δ 8.26 (d, J= 2.0 Hz, 1H), 8.02 (dd, J= 9.0, 2.0 Hz, 1H), 7.87 (d, J= 9.0 Hz, 1H), 7.57 (s, 1H), 7.34-7.08 (m, 10H), 5.63 (bs, 1H), 5.42 (s, 2H), 3.70 (s, 2H), 3.41-3.33 (m, 1H), 3.19 (d, J= 14.0 Hz, 1H), 3.10 (d, J= 14.0 Hz, 1H), 2.91-1.61 (m, 8H).
MS (ESI+) 566, 2.22($M^+$+1, detection time)

Example 310

Ethyl (2E)-3-[4-({1-[2-(1-benzyl-6-nitro-1H-indol-3-yl)-3,3,3-trifluoro-2-hydroxypropyl]piperidin-4-yl}sulfonyl)phenyl]acrylate

MS (ESI+) 686, 2.48($M^+$+1, detection time)

Example 311

Ethyl 3-[4-({1-[2-(1-benzyl-6-nitro-1H-indol-3-yl)-3,3,3-trifluoro-2-hydroxypropyl]piperidin-4-yl}sulfonyl)phenyl]propanoate

MS (ESI+) 688, 2.44($M^+$+1, detection time)

Example 312

2-(1-Benzyl-6-nitro-1H-indol-3-yl)-1,1,1-trifluoro-3-{4-[2-methoxy-4-(morpholin-4-ylmethyl)phenoxy]piperidin-1-yl}propan-2-ol

MS (ESI+) 669, 1.82($M^+$+1, detection time)

Example 313

Methyl 2-[4-({1-[2-(1-benzyl-6-nitro-1H-indol-3-yl)-3,3,3-trifluoro-2-hydroxypropyl]piperidin-4-yl}oxy)phenyl]-2-methylpropanoate

MS (ESI+) 640, 2.29($M^+$+1, detection time)

Example 314

2-(1-Benzyl-6-nitro-1H-indol-3-yl)-1,1,1-trifluoro-3-[4-(5-morpholin-4-yl-pyrimidin-2-yl)piperazin-1-yl] propan-2-ol

$^1$H-NMR (400 MHz, $CDCl_3$) δ 8.28 (d, J= 1.9 Hz, 1H), 8.08 (s, 2H), 8.03 (dd, J= 9.0, 1.9 Hz, 1H), 7.91 (d, J= 9.0 Hz, 1H), 7.58 (s, 1H), 7.40-7.29 (m, 3H), 7.11 (d, J= 6.4 Hz, 1H), 5.41 (s, 2H), 3.85 (t, J= 4.7 Hz, 4H), 3.78-3.59 (m, 4H), 3.31 (d, J= 13.7 Hz, 1H), 3.16 (d, J= 13.7 Hz, 1H), 2.99 (t, J= 4.7 Hz, 1H), 2.70-2.53 (m, 4H).
MS (ESI+) 612, 2.27($M^+$+1, detection time)

Example 315

3-[4-({1-[2-(1-Benzyl-6-nitro-1H-indol-3-yl)-3,3,3-trifluoro-2-hydroxypropyl]-piperidin-4-yl}carbonyl)phenyl]propanoic acid

$^1$H-NMR (400 MHz, $CDCl_3$) δ 8.31-8.27 (m, 1H), 8.08-8.03 (m, 1H), 7.84 (d, J= 9.2 Hz, 1H), 7.66-7.63 (m, 1H), 7.52-7.00 (m, 5H), 5.42 (s, 2H), 4.37-4.29 (m, 1H), 3.76-3.62 (m, 4H), 2.93 (t, J= 7.5 Hz, 2H), 2.67 (t, J= 7.5 Hz, 2H), 1.97-1.43 (m, 5H).
MS (ESI+) 624, 1.99($M^+$+1, detection time)

Example 316

Ethyl {4-[(1-{2-[6-cyano-1-(2-fluorobenzyl)-1H-indol-3-yl]-3,3,3-trifluoro-2-hydroxypropyl}piperidin-4-yl)oxy]-3-methoxyphenyl}acetate

MS (ESI+) 654, 2.15($M^+$+1, detection time)

Example 317

Ethyl {4-[(1-{2-[1-(2-chlorobenzyl)-6-cyano-1H-indol-3-yl]-3,3,3-trifluoro-2-hydroxypropyl}piperidin-4-yl)oxy]-3-methoxyphenyl}acetate

MS (ESI+) 671, 2.20($M^+$+2, detection time)

Example 318

Ethyl {4-[(1-{2-[6-cyano-1-(2-methoxybenzyl)-1H-indol-3-yl]-3,3,3-trifluoro-2-hydroxypropyl}piperidin-4-yl)oxy]-3-methoxyphenyl}acetate

MS (ESI+) 666, 2.17($M^+$+1, detection time)

Example 319

Ethyl {4-[(1-{2-[6-cyano-1-(3-fluorobenzyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.89 (d, J= 8.3 Hz, 1H), 7.60-7.48 (m, 2H), 7.38 (d, J= 8.2 Hz, 1H), 7.33-7.25 (m, 1H), 6.99 (ddd, J= 8.3, 8.3, 2.1 Hz, 1H), 6.89-6.65 (m, 5H), 5.85 (bs, 1H), 5.34 (s, 2H), 4.24 (bs, 1H), 4.13 (q, J= 7.2 Hz, 2H), 3.81 (s, 3H), 3.53 (s, 2H), 3.40-3.05 (m, 2H), 2.95-2.30 (m, 2H), 2.00-1.70 (m, 4H). MS (ESI+) 654, 2.16($M^+$+1, detection time)

Example 320

Ethyl {4-[(1-{2-[6-cyano-1-(3-fluorobenzyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.90 (d, J= 8.4 Hz, 1H), 7.56 (s, 1H), 7.52 (bs, 1H), 7.38 (d, J= 8.4 Hz, 1H), 7.31-7.22 (m, 2H), 7.01 (s, 1H), 6.93 (d, J= 6.7 Hz, 1H), 6.84-6.73 (m, 3H), 5.86 (bs, 1H), 5.32 (s, 2H), 4.35 (bs, 1H), 4.14 (q, J= 7.1 Hz, 2H), 3.81 (s, 3H), 3.53 (s, 2H), 3.36-3.08 (m, 2H), 2.95-2.70 (m, 2H), 2.60-2.40 (m, 2H), 2.02-1.75

(m, 4H), 1.25 (t, J= 7.1 Hz, 3H).
MS (ESI+) 671, 2.35($M^{+}$+2, detection time)

Example 321

Ethyl {4-[(1-{2-[6-cyano-1-(3-fluorobenzyl)-1H-indol-3-yl]-3,3,3-trifluoro-2-hydroxypropyl}piperidin-4-yl)oxy]-3-methoxyphenyl}acetate

MS (ESI+) 666, 2.17($M^{+}$+1, detection time)

Example 322

[3-({1-[2-(1-Benzyl-6-nitro-1H-indol-3-yl)-3,3,3-trifluoro-2-hydroxypropyl]-piperidin-4-yl}carbonyl)-1H-pyrrol-1-yl]acetic acid

$^{1}$H-NMR (400 MHz, $CD_3OD$) δ 8.50 (bs, 1H), 8.05 (d, J= 9.0 Hz, 1H), 8.02 (d, J= 9.0 Hz, 1H), 7.53-7.52 (bs, 1H), 7.36-7.30 (m, 3H), 7.23-7.21 (m 2H), 6.76 (bs, 1H), 6.58 (bs, 1H), 5.55 (s, 2H), 4.76 (s, 2H), 4.14 (d, J= 14.0 Hz, 1H), 3.83 (d, J= 14.0 Hz, 1H), 3.49-3.29 (m, 5H), 2.15-1.80 (m, 4H).

Example 323

{4-[(1-{2-[6-Cyano-1-(cyclopropylmethyl)-1H-indol-3-yl]-3,3,3-trifluoro-2-hydroxypropyl}piperidin-4-yl)oxy] -3-methoxyphenyl}acetic acid

MS (ESI+) 572, 1.96($M^{+}$+1, detection time)

Example 324

{4-[(1-{2-[6-Cyano-1-(2-phenylethyl)-1H-indol-3-yl]-3,3,3-trifluoro-2-hydroxypropyl}piperidin-4-yl)oxy]-3-methoxyphenyl}acetic acid

MS (ESI+) 622, 2.02($M^{+}$+1, detection time)

Example 325

1-[2-({1-[2-(1-Benzyl-6-nitro-1H-indol-3-yl)-3,3,3-trifluoro-2-hydroxypropyl]-piperidin-4-yl}oxy)-5-chlorophenyl]ethanone

$^{1}$H-NMR (400 MHz, $CDCl_3$) δ 8.21 (d, J= 2.0 Hz, 1H), 8.08 (d, J= 9.0 Hz, 1H), 8.03 (dd, J= 9.0, 2.0 Hz, 1H), 7.77 (s, 1H), 7.49 (d, J= 2.5 Hz, 1H), 7.39 (dd, J= 8.8, 2.5 Hz, 1H), 7.28-7.20 (m, 3H), 7.04 (d, J= 6.9 Hz, 1H), 6.85 (d, J= 8.8 Hz, 1H), 5.43 (d, J= 14.0 Hz, 1H), 5.39 (d, J= 14.0 Hz, 1H), 4.75 (bs, 1H), 3.94 (d, J= 13.7 Hz, 1H), 3.89 (d, J= 13.7 Hz, 1H), 3.65-3.59 (m, 1H), 3.40 (bs, 1H), 3.21-3.20 (m, 1H), 2.45 (s, 3H), 2.45-2.40 (m, 1H), 2.19-1.86 (m, 3H).

Example 326

Ethyl (2E)-3-[4-({1-[2-(1-benzyl-6-nitro-1H-indol-3-yl)-3,3,3-trifluoro-2-hydroxypropyl]piperidin-4-yl}oxy)-3-methoxyphenyl]acrylate

MS (ESI+) 668, 2.31($M^{+}$+1, detection time)

Example 327

Ethyl 3-[4-({1-[2-(1-benzyl-6-nitro-1H-indol-3-yl)-3,3,3-trifluoro-2-hydroxypropyl]piperidin-4-yl}oxy)-3-methoxyphenyl] propanoate

MS (ESI+) 670, 2.24($M^{+}$+1, detection time)

Example 328

Ethyl {4-[(1-{2-[1-(2-tert-butoxy-2-oxoethyl)-6-cyano-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.89 (d, J= 8.4 Hz, 1H), 7.56 (s, 1H), 7.45 (s, 1H), 7.38 (dd, J= 8.4, 1.3 Hz, 1H), 6.81 (d, J= 1.9 Hz, 1H), 6.80 (d, J= 8.1 Hz, 1H), 6.74 (dd, J= 8.1, 1.9 Hz, 1H), 5.88 (bs, 1H), 4.75 (s, 2H), 4.21 (bs, 1H), 4.14 (t, J= 7.1 Hz, 2H), 3.82 (s, 3H), 3.53 (s, 2H), 3.24 (d, J= 13.6 Hz, 1H), 3.14 (d, J=, 13.6 Hz, 1H), 2.90-2.74 (m, 2H), 2.53-2.39 (m, 2H), 1.97-1.75 (m, 4H), 1.43 (s, 9H), 1.25 (t, J=7.1 Hz, 3H).

Example 329

Ethyl {4-[(1-{2-[6-cyano-1-(2-methylbenzyl)-1H-indol-3-yl]-3,3,3-trifluoro-2-hydroxypropyl}piperidin-4-yl)oxy]-3-methoxyphenyl}acetate

MS (ESI+) 650, 2.20($M^+$+1, detection time)

Example 330

Ethyl {4-[(1-{2-[6-cyano-1-(2-thienylmethyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.89 (d, J= 8.4 Hz, 1H), 7.69 (s, 1H), 7.52 (s, 1H), 7.36 (dd, J= 8.4, 1.4 Hz, 1H), 7.23 (dd, J= 3.1, 3.1 Hz, 1H), 6.95 (d, J= 3.1 Hz, 1H), 6.95 (d, J= 3.1 Hz, 1H), 6.82 (d, J= 2.0 Hz, 1H), 6.80 (d, J= 8.2 Hz, 1H), 6.75 (dd, J= 8.2, 2.0 Hz, 1H), 5.82 (bs, 1H), 5.48 (s, 2H), 4.25-4.16 (m, 1H), 4.14 (q, J= 7.2 Hz, 2H), 3.82 (s, 3H), 3.53 (s, 2H), 3.22 (d, H= 13.5 Hz, 1H), 3.14 (d, J= 13.5 Hz, 1H), 2.87-2.70 (m, 2H), 2.52-2.34 (m, 2H), 1.98-1.70 (m, 4H), 1.26 (t, J= 7.2 Hz, 3H).

Example 331

Ethyl {4-[(1-{2-[6-cyano-1-(3-thienylmethyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.89 (d, J= 8.2 Hz, 1H), 7.63 (s, 1H), 7.50 (s, 1H), 7.36 (dd, J= 8.2, 1.3 Hz, 1H), 7.30 (dd, J= 5.0, 3.0 Hz, 1H), 7.03-6.98 (m, 1H), 6.87-6.81 (m, 2H), 6.81 (d, J= 8.5 Hz, 1H), 6.75 (dd, J= 8.5, 1.9 Hz, 1H), 5.32 (s, 2H), 4.28-4.18 (m, 1H), 4.14 (q, J= 7.1 Hz, 2H), 3.82 (s, 3H), 3.53 (s, 2H), 3.24 (d, J= 13.6 Hz, 1H), 3.13 (d, J= 13.6 Hz, 1H), 2.88-2.72 (m, 2H), 2.52-2.38 (m, 2H), 1.98-1.72 (m, 4H), 1.25 (t, J= 7.1 Hz, 3H).

Example 332

Ethyl {4-[(1-{2-[6-cyano-1-(3-furylmethyl)-1H-indol-3-yl]-3,3,3-trifluoro-2-hydroxypropyl}piperidin-4-yl)oxy]-3-methoxyphenyl}acetate

MS (ESI+) 626, 2.10($M^+$+1, detection time)

Example 333

Ethyl 3-(4-{4-[2-(1-benzyl-6-nitro-1H-indol-3-yl)-3,3,3-trifluoro-2-hydroxypropyl]piperazin-1-yl}phenyl)propanoate

$^1$H-NMR (400 MHz, $CDCl_3$) δ 8.28 (d, J= 2.0 Hz, 1H), 8.06 (dd, J= 9.1, 2.0 Hz, 1H), 7.96 (d, J= 9.0 Hz, 1H), 7.52 (s, 1H), 7.38-7.31 (m, 5H), 7.13-7.08 (m, 4H), 5.42 (s, 2H), 4.11 (q, J= 7.1 Hz, 2H), 3.30 (d, J= 13.7 Hz, 1H), 3.18 (d, J= 13.7 Hz, 1H), 3.12-3.06 (m, 4H), 2.86 (t, J= 7.5 Hz, 2H), 2.73-2.69 (m, 4H), 2.57 (t, J= 7.5 Hz, 2H), 1.21 (t, J= 7.1 Hz, 3H).
MS (ESI+) 625, 2.53($M^+$+1, detection time)

Example 334

Ethyl 3-(4-{4-[3-(1-benzyl-6-nitro-1H-indol-3-yl)-4,4,4-trifluoro-3-hydroxybutanoyl]piperazin-1-yl}phenyl)propanoate

$^1$H-NMR (400 MHz, $CDCl_3$) δ 8.27 (d, J= 2.0 Hz, 1H), 8.05 (dd, J= 9.0, 2.0 Hz, 1H), 7.97 (d, J= 9.0 Hz, 1H), 7.48 (s, 1H), 7.32-7.25 (m, 3H), 7.12-7.09 (m, 2H), 6.76 (d, J= 8.6 Hz, 1H), 5.40 (d, J= 15.6 Hz, 1H), 5.35 (d, J= 15.6 Hz, 1H), 4.13 (q, J= 7.2 Hz, 2H), 3.71-3.54 (m, 4H), 3.29 (d, J= 15.5 Hz, 1H), 3.21 (d, J= 15.5 Hz, 1H), 3.08-3.03 (m, 2H), 2.88 (t, J= 7.5 Hz, 2H), 2.85-2.80 (m, 2H), 2.58 (t, J= 7.5 Hz, 2H), 1.23 (t, J= 7.2 Hz, 3H).
MS (ESI+) 653, 2.67($M^+$+1, detection time)

Example 335

[4-({4-[2-(1-Benzyl-6-nitro-1H-indol-3-yl)-3,3,3-trifluoro-2-hydroxypropyl]-piperazin-1-yl}carbonyl)phenoxy]acetic acid

$^1$H-NMR (400 MHz, $CDCl_3$) δ 8.28 (d, J= 2.0 Hz, 1H), 8.02 (dd, J= 9.0, 2.0 Hz, 1H), 7.99 (d, J= 9.0 Hz, 1H), 7.56 (s, 1H), 7.34-7.30 (m, 5H), 7.12-7.08 (m, 2H), 6.86 (d, J= 8.7 Hz, 2H), 5.39 (s, 2H), 4.64 (s, 2H), 3.61 (bs, 4H), 3.38 (d, J= 13.7 Hz, 1H), 3.26 (d, J= 13.7 Hz, 1H), 2.77-2.55 (m, 4H).

Example 336

Ethyl 3-{4-[(1-{2-[6-cyano-1-(pyridin-2-ylmethyl)-1H-indol-3-yl]-3,3,3-trifluoro-2-hydroxypropyl}piperidin-4-yl)oxy]-3-methoxyphenyl}propanoate

$^1$H-NMR (400 MHz, $CDCl_3$) δ 8.59 (d, J= 4.8 Hz, 1H), 7.91 (d, J= 8.4 Hz, 1H), 7.63 (d, J= 1.1 Hz, 1H), 7.60 (s, 1H), 7.58 (ddd, J= 8.0, 7.6, 1.8 Hz, 1H), 7.36 (dd, J= 8.4, 1.1 Hz, 1H), 7.22 (dd, J= 7.6, 4.8 Hz, 1H), 6.78 (d, J= 8.0 Hz, 1H), 6.76 (d, J= 8.6 Hz, 1H), 6.72 (d, J= 1.9 Hz, 1H), 6.67 (dd, J= 8.6, 1.9 Hz, 1H), 5.84 (bs, 1H), 5.47 (s, 2H), 4.20 (bs, 1H), 4.12 (q, J= 7.1 Hz, 2H), 3.81 (s, 3H), 3.25 (d, J= 13.6 Hz, 1H), 3.15 (d, J= 13.6 Hz, 1H), 2.88 (t, J= 7.5 Hz, 2H), 2.79-2.78 (m, 2H), 2.62 (t, J= 7.5 Hz, 2H), 2.52-2.44 (m, 2H), 1.90-1.61 (m, 4H), 1.23 (t, J= 7.1 Hz, 3H).
MS (ESI+) 651, 2.10($M^+$+1, detection time)

Example 337

Methyl 4-({4-[2-(1-benzyl-6-nitro-1H-indol-3-yl)-3,3,3-trifluoro-2-hydroxypropyl]-2-oxopiperazin-1-yl}methyl)-3-methoxybenzoate

MS (ESI+) 641, 2.45($M^+$+1, detection time)

Example 338

4-({1-[2-(1-Benzyl-6-nitro-1H-indol-3-yl)-3,3,3-trifluoro-2-hydroxypropyl]-piperidin-4-yl}sulfonyl)-3-methoxybenzoic acid

MS (ESI+) 662, 2.23($M^+$+1, detection time)

Example 339

Ethyl 3-[4-({1-[2-(1-benzyl-6-nitro-1H-indol-3-yl)-3,3,3-trifluoro-2-hydroxypropyl]piperidin-4-yl}thio)-3-methoxyphenyl]propanoate

MS (ESI+) 686, 2.42($M^+$+1, detection time)

Example 340

Ethyl (2E)-3-[4-({1-[2-(1-benzyl-6-nitro-1H-indol-3-yl)-3,3,3-trifluoro-2-hydroxypropyl]piperidin-4-yl}thio)-3-methoxyphenyl]acrylate

MS (ESI+) 684, 2.47($M^+$+1, detection time)

Example 341

Ethyl 3-(4-({1-[2-(1-benzyl-6-nitro-1H-indol-3-yl)-3,3,3-trifluoro-2-hydroxypropyl]piperidin-4-yl}sulfonyl)-3-methoxyphenyl]propanoate

MS (ESI+) 718, 2.42($M^+$+1, detection time)

Example 342

Ethyl (2E)-3-[4-({1-[2-(1-benzyl-6-nitro-1H-indol-3-yl)-3,3,3-trifluoro-2-hydroxypropyl]piperidin-4-yl} sulfonyl)-3-methoxyphenyl]acrylate

MS (ESI+) 716, 2.49($M^+$+1, detection time)

Example 343

Ethyl (2E)-3-[4-({1-[2-(1-benzyl-6-nitro-1H-indol-3-yl)-3,3,3-trifluoro-2-hydroxypropyl]azetidin-3-yl}sulfonyl)-3-methoxyphenyl]acrylate

MS (ESI+) 688, 2.51($M^+$+1, detection time)

Example 344

Ethyl 3-[4-({1-[2-(1-benzyl-6-nitro-1H-indol-3-yl)-3,3,3-trifluoro-2-hydroxypropyl]azetidin-3-yl}sulfonyl)-3-methoxyphenyl]propanoate

MS (ESI+) 690, 2.41($M^+$+1, detection time)

Example 345

Ethyl 3-[4-({1-(2-(1-benzyl-6-nitro-1H-indol-3-yl)-3,3,3-trifluoro-2-hydroxypropyl]piperidin-4-yl}oxy)-3,5-dimethoxyphenyl]propanoate

MS (ESI+) 700, 2.27($M^+$+1, detection time)

Example 346

Ethyl (2E)-3-[4-({1-[2-(1-benzyl-6-nitro-1H-indol-3-yl)-3,3,3-trifluoro-2-hydroxypropyl]piperidin-4-yl}oxy)-3,5-dimethoxyphenyl]acrylate

MS (ESI+) 698, 2.29($M^+$+1, detection time)

Example 347

Ethyl {4-[(1-{2-[6-cyano-1-(1-naphthylmethyl)-1H-indol-3-yl]-3,3,3-trifluoro-2-hydroxypropyl}piperidin-4-yl)oxy]-3-methoxyphenyl}acetate

MS (ESI+) 686, 2.27($M^+$+1, detection time)

Example 348

Ethyl {4-[(1-{2-[6-cyano-1-(2-naphthylmethyl)-1H-indol-3-yl]-3,3,3-trifluoro-2-hydroxypropyl}piperidin-4-yl)oxy]-3-methoxyphenyl}acetate

MS (ESI+) 686, 2.30($M^+$+1, detection time)

Example 349

Ethyl {4-[(1-{2-[6-cyano-1-(cyclohexylmethyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.85 (d, J= 8.4 Hz, 1H), 7.66 (s, 1H), 7.43 (bs, 1H), 7.34 (d, J= 8.4 Hz, 1H), 6.84-6.70 (m, 3H), 5.80 (bs, 1H), 4.22 (bs, 1H), 4.13 (q, J= 7.2 Hz, 2H), 3.95 (d, J= 7.3 Hz, 2H), 3.81 (s, 3H), 3.53 (s, 2H), 3.35-3.06 (m, 1H), 2.90-2.65 (m, 2H), 2.58-2.30 (m, 1H), 2.00-1.45 (m, 10H), 1.32-1.08 (m, 6H), 1.07-0.90 (m, 2H). MS (ESI+) 642, 2.41($M^+$+1, detection time)

Example 350

Ethyl {4-[(1-{2-[1-(cyclohexylmethyl)-6-nitro-1H-indol-3-yl]-3,3,3-trifluoro-2-hydroxypropyl}piperidin-4-yl)oxy]-3-methoxyphenyl}acetate

MS (ESI+) 662, 2.38($M^+$+1, detection time)

Example 351

1-Benzyl-3-(2,2,2-trifluoro-1-{[4-(4-formyl-2,6-dimethoxyphenoxy)piperidin-1-yl]methyl}-1-hydroxyethyl)-1H-indole-6-carbonitrile $^1$H-NMR (400 MHz, $CDCl_3$) δ 9.85 (s, 1H), 7.90 (d, J= 8.4 Hz, 1H), 7.58 (s, 1H), 7.52 (s, 1H), 7.39-7.27 (m, 4H), 7.11 (s, 2H), 7.09-7.03 (m, 2H), 5.33 (s, 2H), 4.32-4.20 (m, 1H), 3.88 (s, 6H), 3.25 (d, J= 13.6 Hz, 1H), 3.14 (d, J= 13.6 Hz, 1H), 2.95-2.75 (m, 2H), 2.52-2.33 (m, 2H), 1.92-1.70 (m, 4H). MS (ESI+) 608, 2.11($M^+$+1, detection time)

Example 352

Ethyl {4-((1-{2-[1-(cyclobutylmethyl)-6-nitro-1H-indol-3-yl]-3,3,3-trifluoro-2-hydroxypropyl}piperidin-4-yl)oxy]-3-methoxyphenyl}acetate

MS (ESI+) 634, 2.27($M^+$+1, detection time)

Example 353

Ethyl {4-[(1-{2-[1-(4-chlorobenzyl)-6-nitro-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 8.22 (d, J= 2.0 Hz, 1H), 8.04 (dd, J= 9.0, 2.0 Hz, 1H), 7.90 (d, J= 9.0 Hz, 1H), 7.55 (s, 1H), 7.30 (d, J= 8.5 Hz, 2H), 7.30 (d, J= 8.5 Hz, 2H), 7.03 (d,, J= 8.5 Hz, 2H), 6.84-6.72 (m, 3H), 5.36 (s, 2H), 4.36 (bs, 1H), 4.14 (q, J= 7.1 Hz, 2H), 3.82 (s, 3H), 3.53 (s, 2H), 3.26 (d, J= 13.6 HZ, 1H), 3.14 (d, J= 13.6 Hz, 1H), 2.90-2.70 (m, 2H), 2.55-2.36 (m, 2H), 1.98-1.72 (m, 4H), 1.25 (t, J= 7.1 Hz, 3H),
MS (ESI+) 690, 2.43($M^+$+1, detection time)

Example 354

Ethyl {3-methoxy-4-[(1-{3,3,3-trifluoro-2-hydroxy-2-[1-(4-methylbenzyl)-6-nitro-1 H-indol-3-yl]propyl}piperidin-4-yl)oxy] phenyl}acetate

$^1$H-NMR (400 MHz, $CDCl_3$) δ 8.26 (d, J= 2.0 Hz, 1H), 8.02 (dd, J= 9.0, 2.0 Hz, 1H), 7.88 (d, J= 9.0 Hz, 1H), 7.56 (s, 1H), 7.12 (d, J= 7.9 Hz, 2H), 7.00 (d, J= 7.9 Hz, 2H), 6.83-6.72 (m, 3H), 5.34 (s, 2H), 4.22 (bs, 1H), 4.14 (q, J= 7.1 Hz, 2H), 3.82 (s, 3H), 3.53 (s, 2H), 3.23 (d, J= 13.6 Hz, 1H), 3.13 (d, J= 13.6 HZ, 1H), 2.88-2.70 (m, 2H), 2.52-2.47 (m, 2H), 2.22 (s, 3H), 1.97-1.72 (m, 4H), 1.25 (t, J= 7.1 Hz, 3H).
MS (ESI+) 670, 2.38($M^+$+1, detection time)

Example 355

Ethyl {3-methoxy-4-[(1-{3,3,3-trifluoro-2-hydroxy-2-[1-(3-methylbenzyl)-6-nitro-1H-indol-3-yl]propyl}piperidin-4-yl)oxy] phenyl}acetate

$^1$H-NMR (400 MHz, $CDCl_3$) δ 8.27 (d, J= 2.0 Hz, 1H), 8.03 (dd, J= 9.0, 2.0 Hz, 1H), 7.90 (d, J= 9.0 Hz, 1H), 7.57 (s, 1H), 7.20 (dd, J= 7.5, 7.5 Hz, 1H), 7.10 (d, J= 7.5 Hz, 1H), 6.92 (s, 1H), 6.87 (d, J= 7.5 Hz, 1H), 6.83-6.72 (m, 3H), 5.35 (s, 1H), 4.22 (bs, 1H), 4.14 (q, J= 7.1 Hz, 2H), 3.82 (s, 3H), 3.53 (s, 2H), 3.25 (d, J= 13.6 Hz, 1H), 3.15 (d, J= 13.6 Hz, 1H), 2.90-2.70 (m, 2H), 2.54-2.36 (m, 2H), 2.28 (s, 3H), 1.97-1.72 (m, 4H), 1.24 (t, J= 7.1 Hz, 3H).
MS (ESI+) 670, 2.39($M^+$+1, detection time)

Example 356

Ethyl {3-methoxy-4-[(1-{3,3,3-trifluoro-2-hydroxy-2-[1-(2-methylbenzyl)-6-nitro-1H-indol-3-yl]propyl}piperidin-4-yl)oxy] phenyl}acetate

$^1$H-NMR (400 MHz, $CDCl_3$) δ 8.27 (d, J= 2.0 Hz, 1H), 8.04 (dd, J= 9.0, 2.0 Hz, 1H), 7.92 (d, J= 9.0 Hz, 1H), 7.42 (s, 1H), 7.29-7.21 (m, 2H), 7.16-7.09 (m, 1H), 6.83-6.70 (m, 4H), 5.35 (s, 2H), 4.22 (bs, 1H), 4.14 (q, J= 7.1 Hz, 2H), 3.82 (s, 3H), 3.53 (s, 2H), 3.22 (d, J= 13.6 Hz, 1H), 3.13 (d, J= 13.6 Hz, 1H), 2.88-2.70 (m, 2H), 2.50-2.36 (m, 2H), 2.27 (s, 3H), 1.95-1.70 (m, 4H), 1.25 (t, J= 7.1 Hz, 3H).
MS (ESI+) 670, 2.40($M^+$+1, detection time)

Example 357

Ethyl {4-[(1-{2-[1-(3-chlorobenzyl)-6-nitro-1H-indol-3-yl]-3,3,3-trifluoro-2-hydroxypropyl}piperidin-4-yl) oxy] -3-methoxy-phenyl}acetate

$^1$H-NMR (400 MHz, $CDCl_3$) δ 8.22 (d, J= 1.9 Hz, 1H), 8.05 (dd, J= 9.0, 1.9 Hz, 1H), 7.92 (d, J= 9.0 Hz, 1H), 7.57 (s, 1H), 7.30-7.24 (m, 2H), 7.04-7.00 (m, 1H), 6.98-6.93 (m, 1H), 6.83-6.72 (m, 3H), 5.37 (s, 2H), 4.23 (bs, 1H), 4.14 (q, J= 7.2 Hz, 2H), 3.82 (s, 3H), 3.53 (s, 2H), 3.26 (d, J= 13.6 Hz, 1H), 3.16 (d, J= 13.6 Hz, 1H), 2.90-2.73 (m, 2H), 2.53-2.40 (m, 2H), 1.97-1.75 (m, 4H), 1.25 (t, J= 7.2 Hz, 3H).
MS (ESI+) 690, 2.17($M^+$+1, detection time)

Example 358

Ethyl {4-[(1-{2-[1-(2-chlorobenzyl)-6-nitro-1H-indol-3-yl]-3,3,3-trifluoro-2-hydroxypropyl}piperidin-4-yl)oxy]-3-methoxy-phenyl}acetate

$^1$H-NMR (400 MHz, $CDCl_3$) δ 8.27 (d, J= 2.0 Hz, 1H), 8.05 (dd, J= 9.0, 2.0 Hz, 1H), 7.92 (d, J= 9.0 Hz, 1H), 7.55 (s, 1H), 7.44 (dd, J= 8.0, 1.2 Hz, 1H), 7.31-7.24 (m, 1H), 7.15 (ddd, J= 8.0, 8.0, 1.2 Hz, 1H), 6.83-6.67 (m, 4H), 5.48 (s, 2H), 4.22 (bs, 1H), 3.24 (d, J= 13.5 Hz, 1H), 3.15 (d, J= 13.5 Hz, 1H), 2.88-2.73 (m, 2H), 2.54-2.36 (m, 2H), 1.97-1.74 (m, 4H), 1.25 (t, J= 7.1 Hz, 1H). MS (ESI+) 690, 2.42($M^+$+1, detection time)

Example 359

Ethyl {3-methoxy-4-[(1-{3,3,3-trifluoro-2-[1-(4-fluorobenzyl)-6-nitro-1H-indol-3-yl]-2-hydroxypropyl}piperidin-4-yl)oxy] phenyl}acetate

$^1$H-NMR (400 MHz, $CDCl_3$) δ 8.24 (d, J= 2.0 HZ, 1H), 8.03 (dd, J= 9.0, 2.0 Hz, 1H), 7.90 (d, J= 9.0 Hz, 1H), 7.55 (s, 1H), 7.12-6.98 (m, 4H), 6.84-6.73 (m, 3H), 5.36 (s, 2H), 4.23 (bs, 1H), 4.13 (q, J= 7.2 Hz, 3H), 3.82 (s, 1H), 3.53 (s, 2H), 3.26 (d, J= 13.7 Hz, 1H), 3.14 (d, J= 13.7 Hz, 1H), 2.90-2.72 (m, 2H), 2.54-2.39 (m, 2H), 1.95-1.74 (m, 4H), 1.25 (t, J= 7.2 Hz, 3H).
MS (ESI+) 674, 2.29($M^+$+1, detection time)

Example 360

Ethyl {3-methoxy-4-[(1-{3,3,3-trifluoro-2-[1-(3-fluorobenzyl)-6-nitro-1H-indol-3-yl]-2-hydroxypropyl}piperidin-4-yl)oxy] phenyl}acetate

$^1$H-NMR (400 MHz, $CDCl_3$) δ 8.22 (d, J= 2.0 Hz, 1H), 8.04 (dd, J= 9.0, 2.0 Hz, 1H), 7.91 (d, J= 9.0 Hz, 1H), 7.58 (s, 1H), 7.34-7.26 (m, 1H), 6.99 (ddd, J= 8.4, 8.4, 2.4 Hz, 1H), 6.88 (d, J= 8.4 Hz, 1H), 6.85-6.78 (m, 2H), 6.78-6.68 (m, 2H), 5.39 (s, 2H), 4.28-4.18 (m, 1H), 4.13 (q, J= 7.1 Hz, 2H), 3.82 (s, 3H), 3.53 (s, 2H), 3.26 (d, J= 13.6 Hz, 1H), 3.16 (d, J= 13.6 Hz, 1H), 2.90-2.73 (m, 2H), 2.55-2.40 (m, 2H), 1.98-1.75 (m, 4H), 1.24 (t, J= 7.1 Hz, 3H).
MS (ESI+) 674, 2.29($M^+$+1, detection time)

Example 361

Ethyl {3-methoxy-4-[(1-{3,3,3-trifluoro-2-[1-(2-fluorobenzyl)-6-nitro-1H-indol-3-yl]-2-hydroxypropyl}piperidin-4-yl)oxy] phenyl}acetate

$^1$H-NMR (400 MHz, $CDCl_3$) δ 8.32 (d, J= 2.0 Hz, 1H), 8.03 (dd, J= 9.0, 2.0 Hz, 1H), 7.90 (d, J= 9.0 Hz, 1H), 7.60 (s, 1H), 7.35-7.27 (m, 1H), 7.14-7.03 (m, 2H), 6.95 (ddd, J= 7.6, 7.6, 1.6 Hz, 1H), 6.83-6.72 (m, 3H), 5.43 (s, 2H), 4.28-4.18 (m, 1H), 4.14 (q, J= 7.1 Hz, 2H), 3.82 (s, 3H), 3.53 (s, 2H), 3.24 (d, J= 13.6 Hz, 1H), 3.13 (d, J= 13.6 Hz, 1H), 2.90-2.70 (m, 2H), 2.53-2.37 (m, 2H), 1.97-1.72 (m, 4H).
MS (ESI+) 674, 2.27($M^+$+1, detection time)

Example 362

Ethyl 3-{4-[(1-{2-[1-(cyclobutylmethyl)-6-nitro-1H-indol-3-yl]-3,3,3-trifluoro-2-hydroxypropyl}piperidin-4-yl)oxy]-3,5-dimethoxyphenyl}propanoate

$^1$H-NMR (400 MHz, $CDCl_3$) δ 8.31 (d, J= 2.0 Hz, 1H), 8.00 (dd, J= 8.9, 2.0 Hz, 1H), 7.86 (d, J= 8.9 Hz, 1H), 7.52 (s, 1H), 6.38 (s, 2H), 5.99 (bs, 1H), 4.19 (d, J= 7.4 Hz, 2H), 4.13 (q, J= 7.1 Hz, 2H), 4.07-3.94 (m, 1H), 3.77 (s, 6H), 3.22 (d, J= 13.6 Hz, 1H), 3.12 (d, J= 13.6 Hz, 1H), 2.94-2.80 (m, 4H), 2.80-2.70 (m, 1H), 2.64-2.55 (m, 2H), 2.48-2.38 (m, 1H), 2.38-2.28 (m, 1H), 2.13-2.00 (m, 2H), 2.00-1.72 (m, 8H), 1.22 (t, J= 7.1 Hz, 3H).
MS (ESI+) 678, 2.29($M^+$+1, detection time)

Example 363

{3-Methoxy-4-[(1-{3,3,3-trifluoro-2-hydroxy-2-[6-nitro-1-(pyridin-3-ylmethyl)-1H-indol-3-yl]propyl}piperidin-4-yl)oxy] phenyl}acetic acid

$^1$H-NMR (400 MHz, $CD_3OD$) δ 8.58-8.45 (m, 3H), 8.14-8.03 (m, 3H), 7.78-7.70 (m, 1H), 7.48-7.40 (m, 1H), 6.96-6.90 (m, 2H), 6.83-6.75 (m, 1H), 5.70 (s, 2H), 4.52-4.42 (m, 1H), 4.22 (d, J= 14.7 Hz, 1H), 3.93 (d, J= 14.7 Hz, 1H), 3.77 (s, 3H), 3.68-3.45 (m, 1H), 3.55 (s, 2H), 3.00-2.65 (m, 1H), 2.20-1.90 (m, 4H), 1.40-1.20 (m, 2H).
MS (ESI+) 629, 1.87($M^+$+1, detection time)

Example 364

2-(1-Benzyl-6-nitro-1H-indol-3-yl)-3-{4-[2-(benzyloxy)phenoxy]piperidin-1-yl}-1, 1, 1-trifluoropropan-2-ol

$^1$H-NMR (400 MHz, $CDCl_3$) δ 8.26 (d, J= 2.0 Hz, 1H), 8.03 (dd, J= 9.0, 2.0 Hz, 1H), 7.93-7.80 (m, 1H), 7.56 (s, 1H), 7.45-7.28 (m, 8H), 7.11-7.06 (m, 2H), 7.00-6.85 (m, 4H), 5.88 (bs, 1H), 5.38 (s, 2H), 5.06 (s, 2H), 4.33 (bs, 1H), 3.21 (d, J= 13.3 Hz, 1H), 3.10 (d, J= 13.3 Hz, 1H), 2.89-2.72 (m, 2H), 2.53-2.30 (m, 2H), 1.96-1.70 (m, 4H).
MS (ESI+) 646, 2.49($M^+$+1, detection time)

Example 365

Methyl [2-({1-[2-(1-benzyl-6-nitro-1H-indol-3-yl)-3,3,3-trifluoro-2-hydroxypropyl] piperidin-4-yl}oxy)phenoxy] acetate

MS (ESI+) 628, 2.27($M^+$+1, detection time)

Example 366

Ethyl 3-[2-({1-[2-(1-benzyl-6-nitro-1H-indol-3-yl)-3,3,3-trifluoro-2-hydroxypropyl]piperidin-4-yl}oxy)phenyl]propanoate

MS (ESI+) 640, 2.47($M^+$+1, detection time)

Example 367

Benzyl 1-benzyl-3-[1-({4-[4-(3-ethoxy-3-oxopropyl)-2,6-dimethoxyphenoxy]-piperidin-1-yl}methyl)-2,2,2-trifluoro-1-hydroxyethyl]-1H-indole-6-carboxylate

MS (ESI+) 789, 2.42($M^+$+1, detection time)

Example 368

Ethyl 2-[2-({1-[2-(1-benzyl-6-nitro-1H-indol-3-yl)-3,3,3-trifluoro-2-hydroxypropyl]piperidin-4-yl}oxy)phenoxy]propanoate

MS (ESI+) 656, 2.38($M^+$+1, detection time)

Example 369

Ethyl 2-[2-({1-[2-(1-benzyl-6-nitro-1H-indol-3-yl)-3,3,3-trifluoro-2-hydroxypropyl]piperidin-4-yl}oxy)phenoxy]-2-methylpropanoate

$^1$H-NMR (400 MHz, $CDCl_3$) δ 8.26 (d, J= 2.0 Hz, 1H), 8.03 (dd, J= 9.0, 2.0 Hz, 1H), 7.91 (d, J= 9.0 Hz, 1H), 7.36-7.27 (m, 3H), 7.12-7.06 (m, 2H), 6.98-6.83 (m, 4H), 5.40 (s, 2H), 4.28 (bs, 1H), 4.22 (q, J= 7.1 Hz, 2H), 3.25 (d, J= 13.3 Hz, 1H), 3.13 (d, J= 13.3 Hz, 1H), 2.90-2.72 (m, 2H), 2.52-2.37 (m, 2H), 1.95-1.68 (m, 4H), 1.54 (s, 6H), 1.24 (t, J= 7.1 Hz, 3H).
MS (ESI+) 670, 2.45($M^+$+1, detection time)

Example 370

Ethyl 3-[4-({1-[2-(1-benzyl-6-cyano-1H-indol-3-yl)-3,3,3-trifluoro-2-hydroxypropyl]piperidin-4-yl}oxy)-3,5-dimethoxyphenyl]propanoate

$^1$H-NMR (400 MHz, $CDCl_3$) δ 7.89 (d, J= 8.4 Hz, 1H), 7.58 (s, 1H), 7.53 (s, 1H), 7.39-7.28 (m, 4H), 7.10-7.03 (m, 2H), 6.39 (s, 2H), 5.93 (bs, 1H), 5.34 (s, 2H), 4.13 (q, J= 7.1 Hz, 2H), 4.02 (bs, 1H), 3.77 (s, 6H), 3.23 (d, J= 13.5 Hz, 1H), 2.95-2.70 (m, 2H), 2.88 (t, J= 7.4 Hz, 2H), 2.60 (t, J= 7.4 Hz, 2H), 2.51-2.37 (m, 2H), 1.93-1.70 (m, 4H), 1.24 (t, J= 7.4 Hz, 3H).
MS (ESI+) 680, 2.15($M^+$+1, detection time)

Example 371

Methyl 1-[2-(1-benzyl-6-nitro-1H-indol-3-yl)-3,3,3-trifluoro-2-hydroxypropyl]-4-oxopiperidine-3-carboxylate

MS (ESI+) 520, 2.55($M^+$+1, detection time)

Example 372

Methyl 4-({1-[2-(1-benzyl-6-nitro-1H-indol-3-yl)-3,3,3-trifluoro-2-hydroxypropyl]-piperidin-4-yl}oxy)-3, 5-dimethoxybenzoate

$^1$H-NMR (400 MHz, $CDCl_3$) δ 8.26 (d, J= 1.7 Hz, 1H), 8.03 (dd, J= 9.0, 1.7 Hz, 1H), 7.90 (d, J= 9.0 Hz, 1H), 7.59 (s, 1H), 7.39-7.24 (m, 5H), 7.13-7.06 (m, 2H), 5.90 (bs, 1H), 5.39 (s, 2H), 4.25-4.15 (m, 1H), 3.90 (s, 3H), 3.85 (s, 6H), 3.25 (d, J= 13.5 Hz, 1H), 3.13 (d, J= 13.5 Hz, 1H), 2.95-2.74 (m, 2H), 2.50-2.32 (m, 2H), 1.89-1.75 (m, 4H).
MS (ESI+) 658, 2.30($M^+$+1, detection time)

Example 373

Ethyl 2-[4-({1-[2-(1-benzyl-6-nitro-1H-indol-3-yl)-3,3,3-trifluoro-2-hydroxypropyl]piperidin-4-yl}oxy)-3-methoxyphenyl]-2-methylpropanoate

$^1$H-NMR (400 MHz, $CDCl_3$) δ 8.26 (d, J= 2.0 Hz, 1H), 8.03 (dd, J= 9.0, 2.0 Hz, 1H), 7.90 (d, J= 9.0 Hz, 1H), 7.58 (s, 1H), 7.38-7.28 (m, 3H), 7.12-7.06 (m, 2H), 6.88-6.76 (m, 3H), 5.84 (bs, 1H), 5.39 (s, 2H), 4.23 (bs, 1H), 4.11 (q, J= 7.2 Hz, 2H), 3.84 (s, 3H), 3.25 (d, J= 13.6 Hz, 1H), 3.15 (d, J= 13.6 Hz, 1H), 2.90-2.70 (m, 2H), 2.55-2.35 (m, 2H), 2.00-1.70 (m, 4H), 1.53 (s, 6H), 1.18 (t, J= 7.2 Hz, 3H).
MS (ESI+) 684, 2.45($M^+$+1, detection time)

Example 374

Ethyl [3-({1-[2-(1-benzyl-6-nitro-1H-indol-3-yl)-3,3,3-trifluoro-2-hydroxypropyl]-piperidin-4-yl}oxy)-4-methoxyphenyl]acetate

$^1$H-NMR (400 MHz, $CDCl_3$) 8 8.26 (d, J= 2.0 Hz, 1H), 8.03 (dd, J= 9.0, 2.0 Hz, 1H), 7.90 (d, J= 9.0 Hz, 1H), 7.58 (s, 1H), 7.37-7.28 (m, 3H), 7.13-7.07 (m, 2H), 6.88-6.79 (m, 3H), 5.85 (bs, 1H), 5.40 (s, 2H), 4.26 (bs, 1H), 4.12 (q, J= 7.2 Hz, 2H), 3.81 (s, 3H), 3.50 (s, 2H), 3.25 (d, J= 13.6 Hz, 1H), 3.16 (d, J= 13.6 Hz, 1H), 2.90-2.72 (m, 2H), 2.55-2.38 (m, 2H), 2.00-1.74 (m, 4H), 1.23 (t, J= 7.2 Hz, 3H). MS (ESI+) 656, 2.37($M^+$+1, detection time)

Example 375

Ethyl (3-methoxy-4-{[1-(3,3,3-trifluoro-2-hydroxy-2-{1-[4-(methylsulfonyl)-benzyl]-6-nitro-1H-indol-3-yl}propyl)piperidin-4-yl]oxy}phenyl)acetate

$^1$H-NMR (400 MHz, $CDCl_3$) δ 8.19 (s, 1H), 8.07 (d, J= 8.6 Hz, 1H), 7.94 (s, 1H), 7.91 (d, J= 8.6 Hz, 1H), 7.61 (s, 1H), 7.30-7.20 (m, 2H), 6.85-6.72 (m, 3H), 5.91 (bs, 1H), 5.51 (s, 2H), 4.25 (bs, 1H), 4.14 (q, J= 7.2 Hz, 2H), 3.82 (s, 3H), 3.54 (s, 2H), 3.29 (d, J= 12.9 Hz, 1H), 3.16 (d, J= 12.9 Hz, 1H), 3.04 (s, 3H), 2.95-2.75 (m, 2H), 2.58-2.40 (m, 2H), 2.00-1.75 (m, 4H), 1.26 (t, J= 7.2 Hz, 3H).
MS (ESI+) 734, 2.25($M^+$+1, detection time)

Example 376

Ethyl {3-methoxy-4-[(1-{3,3,3-trifluoro-2-hydroxy-2-[l-(3-methoxybenzyl)-6-nitro-1 H-indol-3-yl]propyl}piperidin-4-yl)oxy]phenyl}acetate

$^1$H-NMR (400 MHz, $CDCl_3$) δ 8.26 (d, J= 1.7 Hz, 1H), 8.03 (dd, J= 9.0, 1.7 Hz, 1H), 7.89 (d, J= 9.0 Hz, 1H), 7.59 (s, 1H), 7.23 (dd, J= 7.7, 7.7 Hz, 1H), 6.85-6.77 (m, 3H), 6.75 (dd, J= 7.7, 1.9 Hz, 1H), 6.68 (d, J= 7.5 Hz, 1H), 6.57 (s, 1H), 5.85 (bs, 1H), 5.37 (s, 2H), 4.23 (bs, 1H), 4.14 (q, J= 7.2 Hz, 2H), 3.82 (s, 3H), 3.70 (s, 3H), 3.53 (s, 2H), 3.25 (d, J= 13.2 Hz, 1H), 3.16 (d, J= 13.2 Hz, 1H), 2.90-2.70 (m, 2H), 2.56-2.37 (m, 2H), 2.00-1.70 (m, 4H), 1.25 (t, J= 7.2 Hz, 3H). MS (ESI+) 686, 2.35($M^+$+1, detection time)

Example 377

Ethyl {3-methoxy-4-[(1-{3,3,3-trifluoro-2-hydroxy-2-[1-(4-methoxybenzyl)-6-nitro-1 H-indol-3-yl]propyl}piperidin-4-yl)oxy]phenyl}acetate

$^1$H-NMR (400 MHz, $CDCl_3$) δ 8.28 (d, J= 1.9 Hz, 1H), 8.02 (dd, J= 9.0, 1.9 Hz, 1H), 7.88 (d, J= 9.0 Hz, 1H), 7.55 (bs, 1H), 7.07 (d, J= 8.5 Hz, 2H), 6.90-6.70 (m, 5H), 5.83 (bs, 1H), 5.32 (s, 2H), 4.23 (bs, 1H), 4.14 (q, J= 7.7 Hz, 2H), 3.82 (s, 3H), 3.77 (s, 3H), 3.53 (s, 2H), 3.25 (d, J= 12.6 Hz, 1H), 3.14 (d, J= 12.6 Hz, 1H), 2.91-2.68 (m, 2H), 2.55-2.35 (m, 2H), 2.00-1.70 (m, 4H), 1.25 (t, J= 7.7 Hz, 3H). MS (ESI+) 686, 2.35($M^+$+1, detection time)

Example 378

Ethyl 3-{3-methoxy-4-[(1-{3,3,3-trifluoro-2-hydroxy-2-[1-(2-methoxybenzyl)-6-nitro-1H-indol-3-yl]propyl}piperidin-4-yl) oxy]phenyl}propanoate

$^1$H-NMR (400 MHz, $CDCl_3$) δ 8.40 (d, J= 1.9 Hz, 1H), 8.00 (dd, J= 8.9, 1.9 Hz, 1H), 7.86 (d, J= 8.9 Hz, 1H), 7.62 (s, 1H), 7.32-7.24 (m, 1H), 6.95-6.82 (m, 3H), 6.77 (d, J= 8.1 Hz, 1H), 6.72 (d, J= 2.0 Hz, 1H), 6.67 (dd, J= 8.1, 2.1 Hz, 1H), 5.84 (bs, 1H), 5.37 (s, 2H), 4.20 (bs, 1H), 4.12 (q, J= 7.2 Hz, 2H), 3.88 (s, 3H), 3.80 (s, 3H), 3.30-3.10 (m, 2H), 2.92-2.68 (m, 2H), 2.88 (t, J= 7.5 Hz, 2H), 2.59 (t, J= 7.5 Hz, 2H), 2.54-2.33 (m, 2H), 1.98-1.70 (m, 4H), 1.23 (t, J= 7.2 Hz, 2H). MS (ESI+) 700, 2.43($M^+$+1, detection time)

Example 379

Ethyl 3-{3-methoxy-4-[(1-{3,3,3-trifluoro-2-hydroxy-2-[1-(3-methoxybenzyl)-6-nitro-1H-indol-3-yl]propyl}piperidin-4-yl) oxy]phenyl}propanoate

$^1$H-NMR (400 MHz, $CDCl_3$) δ 8.26 (s, 1H), 8.03 (d, J= 9.0 Hz, 1H), 7.89 (d, J= 9.0 Hz, 1H), 7.59 (bs, 1H), 7.23 (t, J= 8.0 Hz, 1H), 6.81 (dd, J= 8.0, 2.0 Hz, 1H), 6.77 (d, J= 8.0 Hz, 1H), 6.72 (d, J= 2.0 Hz, 1H), 6.70-6.64 (m, 2H), 6.58-6.54 (m, 1H), 5.86 (bs, 1H), 5.37 (s, 2H), 4.20 (bs, 1H), 4.12 (q, J= 7.1 Hz, 2H), 3.80 (s, 3H), 3.71 (s, 3H), 3.24 (d, J= 12.8 Hz, 1H), 3.16 (d, J= 12.8 Hz, 1H), 2.92-2.70 (m, 2H), 2.88 (t, J= 7.5 Hz, 2H), 2.58 (t, J= 7.5 Hz, 2H), 2.54-2.35 (m, 2H), 2.00-1.70 (m, 4H), 1.23 (t, J= 7.1Hz, 3H).
MS (ESI+) 700, 2.42($M^+$+1, detection time)

Example 380

Ethyl 3-{3-methoxy-4-[(1-{3,3,3-trifluoro-2-hydroxy-2-[1-(4-methoxybenzyl)-6-nitro-1H-indol-3-yl]propyl}piperidin-4-yl) oxy]phenyl}propanoate

$^1$H-NMR (400 MHz, $CDCl_3$) δ 8.28 (d, J= 1.7 Hz, 1H), 8.02 (dd, J= 9.0, 1.7 Hz, 1H), 7.88 (d, J= 9.0 Hz, 1H), 7.55 (s, 1H), 7.07 (d, J= 8.5 Hz, 2H), 6.85 (d, J= 8.5 Hz, 2H), 6.78 (d, J= 8.1 Hz, 1H), 6.72 (d, J= 1.9 Hz, 1H), 6.67 (dd, J= 8.1, 1.9 Hz, 1H), 5.84 (bs, 1H), 5.32 (s, 2H), 4.20 (bs, 1H), 4.12 (q, J= 7.2 Hz, 2H), 3.80 (s, 3H), 3.78 (s, 3H), 3.25 (d, J= 13.2 Hz, 1H), 3.14 (d, J= 13.2 Hz, 1H), 2.92-2.68 (m, 2H), 2.88 (t, J= 7.5 Hz, 2H), 2.58 (t, J= 7.5 Hz, 2H), 2.53-2.34 (m, 2H), 2.00-1.70 (m, 4H), 1.23 (t, J= 7.2 Hz, 2H).
MS (ESI+) 700, 2.39($M^+$+1, detection time)

Example 381

1-[2-(1-Benzyl-6-nitro-1H-indol-3-yl)-3,3,3-trifluoro-2-hydroxypropyl]piperidine-4-carbonitrile

MS (ESI+) 473, 2.31($M^+$+1, detection time)

Example 382

2-(1-benzyl-6-nitro-1H-indol-3-yl)-1,1,1-trifluoro-3-[4-(1H-indol-3-yl)piperidin-1-yl]propan-2-ol

$^1$H-NMR (400 MHz, $CDCl_3$) δ 8.28 (d, J= 2.0 Hz, 1H), 8.05 (dd, J= 9.0, 2.0 Hz, 1H), 7.99 (bs, 1H), 7.94 (d, J= 9.0 Hz, 1H), 7.62 (s, 1H), 7.59 (d, J= 8.0 Hz, 1H), 7.37 (d, J= 8.0 Hz, 1H), 7.35-7.28 (m, 3H), 7.23-7.16 (m, 1H), 7.14-7.07 (m, 3H), 6.95 (d, J= 2.1 Hz, 1H), 6.05 (bs, 1H), 5.39 (s, 2H), 3.31 (d, J= 13.1 Hz, 1H), 3.20 (d, J= 13.1 Hz, 1H), 3.13-3.00 (m, 1H), 2.89-2.58 (m, 3H), 2.44-2.32 (m, 1H), 2.15-2.04 (m, 1H), 1.95-1.79 (m, 2H), 1.79-1.63 (m, 1H).
MS (ESI+) 563, 2.25($M^+$+1, detection time)

Example 383

2-(1-Benzyl-6-nitro-1H-indol-3-yl)-1,1,1-trifluoro-3-[4-(6-fluoro-1,2-benzisoxazol-3-yl)piperidin-1-yl]propan-2-ol

MS (ESI+) 583, 2.38($M^+$+1, detection time)

Example 384

2-(1-Benzyl-6-nitro-1H-indol-3-yl)-3-(6,7-dihydrothieno[3,2-c]pyridin-5(4H)-yl)-1,1,1-trifluoropropan-2-ol

MS (ESI+) 502, 2.46($M^+$+1, detection time)

Example 385

2-(1-Benzyl-6-nitro-1H-indol-3-yl)-1,1,1-trifluoro-3-(1,4,6,7-tetrahydro-5H-pyrazolo[4,3-c]pyridin-5-yl)propan-2-ol

$^1$H-NMR (400 MHz, $CDCl_3$) δ 8.29 (d, J= 2.0 Hz, 1H), 8.03 (dd, J= 9.0, 2.0 Hz, 1H), 7.90 (d, J= 9.0 Hz, 1H), 7.60 (s, 1H), 7.38-7.28 (m, 3H), 7.18 (s, 1H), 7.15-7.09 (m, 2H), 5.41 (s, 2H), 3.68 (d, J= 13.8 Hz, 1H), 3.62 (d, J= 13.8Hz, 1H), 3.42 (d, J= 13.8 Hz, 1H), 3.33 (d, J= 13.8 Hz, 1H), 2.99-2.86 (m, 2H), 2.82-2.67 (m, 2H).
MS (ESI+) 486, 2.10($M^+$+1, detection time)

Example 386

Ethyl {4-[(1-{2-[6-cyano-1-(3-methylbenzyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.88 (d, J= 8.3 Hz, 1H), 7.61 (s, 1H), 7.53 (bs, 1H), 7.37 (d, J=8.3 Hz, 1H), 7.19 (dd, J= 7.6, 7.6 Hz, 1H), 7.10 (d, J= 7.6 Hz, 1H), 6.91 (s, 1H), 6.84 (d, J= 7.6 Hz, 1H), 6.82-6.72 (m, 3H), 5.30 (s, 2H), 4.24 (bs, 1H), 4.14 (q, J= 7.1 Hz, 2H), 3.80 (bs, 3H), 3.53 (s, 2H), 3.39-3.03 (m, 2H), 2.95-2.65 (m, 2H), 2.60-2.34 (m, 2H), 2.29 (s, 3H), 2.00-1.72 (m, 4H), 1.25 (t, J= 7.1 Hz, 3H).
MS (ESI+) 650, 2.47($M^+$+1, detection time)

Example 387

Ethyl 3-{4-[(1-{2-[6-bromo-1-(2-fluorobenzyl)-1H-indol-3-yl]-3,3,3-trifluoro-2-hydroxypropyl}piperidin-4-yl)oxy]-3, 5-dimethoxyphenyl}propanoate

MS (ESI+) 752, 2.50($M^+$+1, detection time)

Example 388

Ethyl [4-({1-[2-(1-benzyl-7-chloro-1H-pyrrolo[2,3-c]pyridin-3-yl)-3,3,3-trifluoro-2-hydroxypropanoyl]piperidin-4-yl}oxy)-3-methoxyphenyl]acetate

MS (ESI+) 660, 2.62($M^+$+1, detection time)

Example 389

3-[4-(1,3-Benzothiazol-2-yl)piperidin-1-yl]-2-(1-benzyl-6-nitro-1H-indol-3-yl)-1,1,1-trifluoropropan-2-ol

MS (ESI+) 581, 2.25($M^+$+1, detection time)

Example 390

3-[4-(1,3-Benzoxazol-2-yl)piperidin-1-yl]-2-(1-benzyl-6-nitro-1H-indol-3-yl)-1,1,1-trifluoropropan-2-ol

MS (ESI+) 565, 2.21($M^+$+1, detection time)

Example 391

3-[4-(1H-Benzimidazol-2-yl)piperidin-1-yl]-2-(1-benzyl-6-nitro-1H-indol-3-yl)-1, 1, 1-trifluoropropan-2-ol

MS (ESI+) 564, 1.93($M^+$+1, detection time)

Example 392

Ethyl 4-[4-({1-[2-(1-benzyl-6-cyano-1H-indol-3-yl)-3,3,3-trifluoro-2-hydroxypropyl]piperidin-4-yl}oxy)-3,5-dimethoxyphenyl]butanoate

MS (ESI+) 694, 2.19($M^+$+1, detection time)

Example 393

Ethyl 3-[4-({1-[2-(1-benzyl-7-chloro-1H-indol-3-yl)-3,3,3-trifluoro-2-hydroxypropyl]piperidin-4-yl}oxy)-3, 5-dimethoxyphenyl]propanoate

$^1$H-NMR (400 MHz, $CDCl_3$) δ 7.74 (d, J= 7.3 Hz, 1H), 7.35-7.17 (m, 4H), 7.17-7.10 (m, 1H), 7.09-6.99 (m, 1H), 6.96 (d, J= 6.7 Hz, 2H), 6.38 (s, 2H), 6.00-5.65 (m, 3H), 4.13 (q, J= 7.1 Hz, 2H), 4.00 (bs, 1H), 3.77 (s, 6H), 3.18 (s, 2H), 2.94-2.70 (m, 2H), 2.88 (t, J= 7.9 Hz, 2H), 2.60 (t, J= 7.9 Hz, 2H), 2.53-2.23 (m, 2H), 1.89-1.70 (m, 4H), 1.23 (t, J= 7.1 Hz, 3H).
MS (ESI+) 689, 2.46($M^+$+1, detection time)

Example 394

Ethyl {4-[(1-{2-[6-bromo-1-(2-fluorobenzyl)-1H-indol-3-yl]-3,3,3-trifluoro-2-hydroxypropyl}piperidin-4-yl)oxy]-3-methoxyphenyl}acetate

$^1$H-NMR (400 MHz, $CDCl_3$) δ 7.67 (d, J= 8.6 Hz, 1H), 7.46 (d, J= 1.5 Hz, 1H), 7.31 (s, 1H), 7.29-7.20 (m, 3H), 7.08 (dd, J= 7.5, 7.5 Hz, 1H), 7.01 (ddd, J= 7.5, 7.5, 1.1 Hz, 1H), 6.85-6.72 (m, 4H), 5.73 (bs, 1H), 5.30 (s, 2H), 4.20 (bs, 1H), 4.14 (q, J= 7.1 Hz, 2H), 3.82 (s, 3H), 3.53 (s, 2H), 3.17 (s, 2H), 2.89-2.70 (m, 2H), 2.53-2.30 (m, 2H), 1.98-1.70 (m, 4H), 1.25 (t, J= 7.1 Hz, 3H).
MS (ESI+) 707, 2.46($M^+$+1, detection time)

Example 395

Ethyl 3-{4-[(1-{2-[6-bromo-1-(2-fluorobenzyl)-1H-indol-3-yl]-3,3,3-trifluoro-2-hydroxypropyl}piperidin-4-yl)oxy]-3-methoxyphenyl}propanoate

$^1$H-NMR (400 MHz, $CDCl_3$) δ 7.67 (d, J= 8.7 Hz, 1H), 7.46 (d, J= 1.3 Hz, 1H), 7.30 (s, 1H), 7.29-7.20 (m, 3H), 7.12-7.05 (m, 1H), 7.01 (ddd, J= 7.6, 7.6, 1.3 Hz, 1H), 6.85-6.75 (m, 2H), 6.74-6.65 (m, 2H), 5.75 (bs, 1H), 5.30 (s, 2H), 4.17 (bs, 1H), 4.12 (q, J= 7.1 Hz, 2H), 3.81 (s, 3H), 3.17 (s, 2H), 2.88 (t, J= 8.1 Hz, 2H), 2.88-2.70 (m, 2H), 2.58 (t, J= 8.1 Hz, 2H), 2.52-2.31 (m, 2H), 1.97-1.70 (m, 4H), 1.23 (t, J= 7.1 Hz, 2H).
MS (ESI+) 721, 2.53($M^+$+1, detection time)

Example 396

Ethyl [4-({1-[2-(1-benzyl-5-nitro-1H-indol-3-yl)-3,3,3-trifluoro-2-hydroxypropyl]-piperidin-4-yl}oxy)-3-methoxyphenyl]acetate

$^1$H-NMR (400 MHz, $CDCl_3$) δ 8.80 (d, J= 2.2 Hz, 1H), 8.09 (dd, J= 9.1, 2.2 Hz, 1H), 7.46 (s, 1H), 7.37-7.28 (m, 4H), 7.46 (s, 1H), 7.37-7.28 (m, 4H), 7.10-7.02 (m, 2H), 6.85-6.72 (m, 3H), 5.85 (bs, 1H), 5.36 (s, 2H), 4.24 (bs, 1H), 4.14 (q, J= 7.2 Hz, 2H), 3.82 (s, 3H), 3.53 (s, 2H), 3.28 (d, J= 13.6 Hz, 1H), 3.15 (d, J= 13.6 Hz, 1H), 2.92-2.72 (m, 2H), 2.55-2.39 (m, 2H), 1.98-1.72 (m, 4H), 1.25 (t, J= 7.2 Hz, 3H).
MS (ESI+) 656, 2.43($M^+$+1, detection time)

Example 397

Ethyl 3-[4-({1-[2-(1-benzyl-6-chloro-1H-indol-3-yl)-3,3,3-trifluoro-2-hydroxypropyl]piperidin-4-yl}oxy)-3-methoxyphenyl]propanoate

$^1$H-NMR (400 MHz, $CDCl_3$) δ 7.72 (d, J= 8.6 Hz, 1H), 7.34-7.22 (m, 5H), 7.09 (dd, J= 8.6, 1.8 Hz, 1H), 7.07-7.02 (m, 2H), 6.78 (d, J= 8.2 Hz, 1H), 6.72 (d, J= 2.0 Hz, 1H), 6.67 (dd, J= 8.2, 2.0 Hz, 1H), 5.26 (bs, 1H), 5.26 (s, 2H), 4.17

(bs, 1H), 4.12 (q, J= 7.1 Hz, 2H), 3.81 (s, 3H), 3.18 (s, 2H), 2.88 (t, J= 8.0 Hz, 2H), 2.88-2.70 (m, 2H), 2.59 (t, J= 8.0 Hz, 2H), 2.53-2.30 (m, 2H), 1.96-1.70 (m, 4H), 1.23 (t, J= 7.1 Hz, 3H).
MS (ESI+) 659, 2.50($M^+$+1, detection time)

Example 398

Benzyl 1-benzyl-3-[1-({4-[4-(3-ethoxy-3-oxopropyl)-2,6-dimethoxyphenoxy]-piperidin-1-yl}methyl)-2,2,2-trifluoro-1-hydroxyethyl]-1H-indole-5-carboxylate

$^1$H-NMR (400 MHz, $CDCl_3$) δ 8.59 (s, 1H), 7.91 (dd, J= 8.7, 1.4 Hz, 1H), 7.50-7.20 (m, 10H), 7.08-7.00 (m, 2H), 6.72+6.38 (s, 2H), 5.90 (bs, 1H), 5.45-5.30 (m, 2H), 5.32 (s, 2H), 4.13 (q, J= 7.2 Hz, 2H), 4.00 (bs, 1H), 3.82+3.77 (s, 6H), 3.29-3.15 (m, 2H), 2.95-2.83 (m, 1H), 2.88 (t, J= 8.2 Hz, 2H), 2.60 (t, J= 8.2 Hz, 2H), 2.50-2.23 (m, 2H), 1.90-1.70 (m, 4H), 1.23 (t, J= 7.2 Hz, 2H).
MS (ESI+) 789, 2.58($M^+$+1, detection time)

Example 399

5-[2-(1-Benzyl-6-nitro-1H-indol-3-yl)-3,3,3-trifluoro-2-hydroxypropyl]-4,5,6,7-tetrahydrothieno[3,2-c]pyridine-2-carboxylic acid

MS (ESI+) 546, 2.20($M^+$+1, detection time)

Example 400

2-(1-Benzyl-6-nitro-1H-indol-3-yl)-1,1,1-trifluoro-3-(2-methyl-6,7-dihydro[1,3]-thiazolo[5,4-c]pyridin-5(4H)-yl)propan-2-ol

$^1$H-NMR (400 MHz, $CDCl_3$) δ 8.29 (d, J= 2.0 Hz, 1H), 8.02 (dd, J= 9.0, 2.0 Hz, 1H), 7.90 (d, J= 9.0 Hz, 1H), 7.58 (s, 1H), 7.39-7.28 (m, 3H), 7.14-7.08 (m, 2H), 5.40 (s, 2H), 3.77 (d, J= 15.0 Hz, 1H), 3.70 (d, J= 15.0 Hz, 1H), 3.39 (d, J= 13.8 Hz, 1H), 3.34 (d, J= 13.8 Hz, 1H), 2.99-2.92 (m, 2H), 2.82-2.76 (m, 2H), 2.64 (s, 3H).
MS (ESI+) 517, 2.45($M^+$+1, detection time)

Example 401

Ethyl 3-{4-[(1-{2-[6-cyano-1-(4-methoxybenzyl)-1H-indol-3-yl]-3,3,3-trifluoro-2-hydroxypropyl}piperidin-4-yl)oxy]-3-methoxyphenyl}propanoate

$^1$H-NMR (400 MHz, $CDCl_3$) δ 7.88 (d, J= 8.4 Hz, 1H), 7.60 (s, 1H), 7.48 (s, 1H), 7.34 (d, J= 8.4 Hz, 1H), 7.02 (d, J= 8.5 Hz, 2H), 6.84 (d, J= 8.5 Hz, 2H), 6.78 (d, J= 8.5 Hz, 1H), 6.72 (d, J= 1.8 Hz, 1H), 6.68 (dd, J= 8.5, 1.8 Hz, 1H), 5.25 (s, 2H), 4.19 (bs, 1H), 4.12 (q, J= 7.1 Hz, 2H), 3.81 (s, 3H), 3.78 (s, 3H), 3.23 (d, J= 13.6 Hz, 1H), 3.13 (d, J= 13.6 Hz, 1H), 2.90-2.70 (m, 2H), 2.88 (t, J= 7.5 Hz, 2H), 2.58 (t, J= 7.5 Hz, 2H), 2.52-2.35 (m, 2H), 1.95-1.73 (m, 4H), 1.23 (t, J= 7.1 Hz, 3H).
MS (ESI+) 680, 2.28($M^+$+1, detection time)

Example 402

Ethyl 3-[3,5-dimethoxy-4-({1-[3,3,3-trifluoro-2-hydroxy-2-(6-nitro-1-phenyl-1H-indol-3-yl)propyl]piperidin-4-yl}oxy)phenyl]propanoate

$^1$H-NMR (400 MHz, $CDCl_3$) δ 8.42 (d, J= 2.0 Hz, 1H), 8.08 (dd, J= 9.0, 2.0 Hz, 1H), 7.98 (d, J= 9.0 Hz, 1H), 7.75 (s, 1H), 7.64-7.55 (m, 2H), 7.53-7.45 (m, 3H), 6.73+6.39 (s, 2H), 4.19-4.09 (m, 2H), 4.09-4.00 (m, 1H), 3.83+3.78 (s, 6H), 3.32 (d, J= 13.5 Hz, 1H), 3.16 (d, J= 13.5 Hz, 1H), 3.00-2.90 (m, 2H), 2.88 (t, J= 8.1 Hz, 2H), 2.62 (t, J= 8.1 Hz, 2H), 2.55-2.40 (m, 2H), 1.91-1.79 (m, 4H), 1.39-1.29 (m, 3H).
MS (ESI+) 686, 2.84($M^+$+1, detection time)

Example 403

Ethyl {5-[2-(1-benzyl-6-nitro-1H-indol-3-yl)-3,3,3-trifluoro-2-hydroxypropyl]-4,5,6,7-tetrahydro-2H-pyrazolo[4,3-c]pyridin-2-yl}acetate

MS (ESI+) 572, 2.25($M^{+}$+1, detection time)

Example 404

Ethyl 7-[2-(1-benzyl-6-nitro-1H-indol-3-yl)-3,3,3-trifluoro-2-hydroxypropyl]-5,6,7,8-tetrahydroimidazo[1,2-a]pyrazine-2-carboxylate

MS (ESI+) 558, 2.39($M^{+}$+1, detection time)

Example 405

2-(1-Benzyl-6-nitro-1H-indol-3-yl)-1,1,1-trifluoro-3-(2-phenyl-6,7-dihydro[1,3]-thiazolo[5,4-c]pyridin-5(4H)-yl)propan-2-ol

MS (ESI+) 579, 2.72($M^{+}$+1, detection time)

Example 406

{5-[2-(1-Benzyl-6-nitro-1H-indol-3-yl)-3,3,3-trifluoro-2-hydroxypropyl]-4,5,6,7-tetrahydro-2H-pyrazolo[4,3-c]pyridin-2-yl}acetic acid

MS (ESI+) 544, 2.18($M^{+}$+1, detection time)

Example 407

Ethyl {5-[2-(1-benzyl-6-nitro-1H-indol-3-yl)-3,3,3-trifluoro-2-hydroxypropyl]-4,5,6,7-tetrahydro[1,3]thiazolo[5,4-c]pyridin-2-yl}acetate

MS (ESI+) 589, 2.59($M^{+}$+1, detection time)

Example 408

Ethyl [4-({1-[2-(1-benzyl-7-chloro-1H-pyrrolo[2,3-c]pyridin-3-yl)-3,3,3-trifluoro-2-hydroxypropyl]piperidin-4-yl}oxy)-3-methoxyphenyl]acetate

$^{1}H$-NMR (400 MHz, $CDCl_3$) δ 8.02 (d, J= 5.6 Hz, 1H), 7.67 (d, J= 5.6 Hz, 1H), 7.45 (s, 1H), 7.33-7.22 (m, 3H), 7.04-6.97 (m, 2H), 6.85-6.72 (m, 3H), 5.81 (d, J= 16.2 Hz, 1H), 5.75 (d, J= 16.2 Hz, 1H), 4.21 (bs, 1H), 4.14 (q, J= 7.2 Hz, 2H), 3.82 (s, 3H), 3.53 (s, 2H), 3.21 (d, J= 13.6 Hz, 1H), 3.11 (d, J= 13.6 Hz, 1H), 2.90-2.70 (m, 2H), 2.50-2.34 (m, 2H), 1.95-1.70 (m, 4H), 1.25 (t, J= 7.2 Hz, 3H). MS (ESI+) 646, 2.34($M^{+}$+1, detection time)

Example 409

Ethyl [4-({1-[2-(1-benzyl-6-cyano-2-methyl-1H-indol-3-yl)-3,3,3-trifluoro-2-hydroxypropyl]piperidin-4-yl}oxy)-3-methoxyphenyl] acetate

$^{1}H$-NMR (400 MHz, $CDCl_3$) δ 8.09 (s, 1H), 7.52 (s, 1H), 7.37-7.25 (m, 4H), 6.94-6.72 (m, 5H), 5.40-5.30 (m, 2H), 4.30-4.00 (m, 3H), 3.83 (s, 3H), 3.55 (s, 2H), 3.46-3.20 (m, 1H), 3.00-2.80 (m, 2H), 2.68-2.50 (m, 1H), 2.58 (s, 3H), 2.00-1.80 (m, 4H), 1.29-1.22 (m, 3H).
MS (ESI+) 650, 2.44($M^{+}$+1, detection time)

Example 410

Ethyl {4-[(1-{2-[7-chloro-1-(4-methoxybenzyl)-1H-pyrrolo[2,3-c]pyridin-3-yl]-3,3,3-trifluoro-2-hydroxypropyl}piperidin-4-yl)oxy]-3-methoxyphenyl}acetate

$^1$H-NMR (400 MHz, $CDCl_3$) δ 8.01 (d, J= 5.5 Hz, 1H), 7.65 (d, J= 5.5 Hz, 1H), 7.42 (s, 1H), 6.99 (d, J= 8.8 Hz, 2H), 6.86-6.73 (m, 5H), 5.74 (d, J= 15.8 Hz, 1H), 5.68 (d, J= 15.8 Hz, 1H), 4.21 (bs, 1H), 4.19-4.09 (m, 2H), 3.82 (s, 3H), 3.76 (s, 3H), 3.53 (s, 2H), 3.20 (d, J= 13.6 Hz, 1H), 3.10 (d, J= 13.6 Hz, 1H), 2.88-2.69 (m, 2H), 2.50-2.35 (m, 2H), 1.95-1.72 (m, 4H), 1.30-1.20 (m, 3H).
MS (ESI+) 676, 2.31($M^+$+1, detection time)

Example 411

Ethyl 3-[4-({1-[3,3,3-trifluoro-2-hydroxy-2-(6-nitro-1-phenyl-1H-indol-3-yl)propyl]piperidin-4-yl}sulfonyl)phenyl]propanoate

$^1$H-NMR (400 MHz, $CDCl_3$) δ 8.40 (d, J= 2.0 Hz, 1H), 8.06 (dd, J= 9.0, 2.0 Hz, 1H), 7.92 (d, J= 9.0 Hz, 1H), 7.76 (d, J= 8.4 Hz, 2H), 7.72 (s, 1H), 7.64-7.56 (m, 2H), 7.53-7.45 (m, 3H), 7.43-7.37 (m, 2H), 5.42 (bs, 1H), 4.19-4.08 (m, 2H), 3.30 (d, J= 13.9 Hz, 1H), 3.12 (d, J= 13.9 Hz, 1H), 3.12-3.00 (m, 1H), 3.04 (t, J= 7.6 Hz, 2H), 2.92-2.72 (m, 2H), 2.65 (t, J= 7.6 Hz, 2H), 2.55-2.45 (m, 1H), 2.28-2.16 (m, 1H), 2.07-1.96 (m, 1H), 1.91-1.64 (m, 3H).
MS (ESI+) 674, 2.51($M^+$+1, detection time)

Example 412

Ethyl [4-({1-[2-(1-benzyl-6-cyano-1H-indol-3-yl)-3,3,3-trifluoro-2-hydroxypropyl]piperidin-4-yl}oxy)phenyl]acetate

$^1$H-NMR (400 MHz, $CDCl_3$) δ 7.90 (d, J= 8.4 Hz, 1H), 7.58 (s, 1H), 7.51 (s, 1H), 7.35 (dd, J= 8.4, 1.4 Hz, 1H), 7.33-7.28 (m, 3H), 7.16 (d, J= 8.8 Hz, 2H), 7.08-7.03 (m, 2H), 6.81 (d, J= 8.8 Hz, 2H), 5.78 (bs, 1H), 5.33 (s, 2H), 4.31 (bs, 1H), 4.13 (q, J= 7.1 Hz, 2H), 3.53 (s, 2H), 3.23 (d, J= 13.5 Hz, 1H), 3.14 (d, J= 13.5 Hz, 1H), 2.82-2.70 (m, 2H), 2.55-2.40 (m, 2H), 1.96-1.69 (m, 4H), 1.24 (t, J= 7.1 Hz, 3H).
MS (ESI+) 606, 2.21($M^+$+1, detection time)

Example 413

Ethyl {4-[(1-{2-[6-cyano-1-(4-methoxybenzyl)-1H-indol-3-yl]-3,3,3-trifluoro-2-hydroxypropyl}piperidin-4-yl)oxy]phenyl}acetate

$^1$H-NMR (400 MHz, $CDCl_3$) δ 7.88 (d, J= 8.4 Hz, 1H), 7.60 (s, 1H), 7.48 (s, 1H), 7.35 (dd, J= 8.4, 1.4 Hz, 1H), 7.16 (d, J= 8.7 Hz, 2H), 7.03 (d, J= 8.7 Hz, 2H), 6.84 (d, J= 8.7 Hz, 2H), 6.81 (d, J= 8.7 Hz, 2H), 5.78 (bs, 1H), 5.26 (s, 1H), 4.30 (bs, 1H), 4.13 (q, J= 7.1 Hz, 2H), 3.77 (s, 3H), 3.53 (s, 2H), 3.23 (d, J= 13.6 Hz, 1H), 3.13 (d, J= 13.6 Hz, 1H), 2.82-2.70 (m, 2H), 2.55-2.40 (m, 2H), 1.95-1.70 (m, 4H), 1.24 (t, J= 7.1 Hz, 3H).
MS (ESI+) 636, 2.23($M^+$+1, detection time)

Example 414

Ethyl 3-{4-[(1-{2-[1-(4-chlorophenyl)-6-nitro-1H-indol-3-yl]-3,3,3-trifluoro-2-hydroxypropyl}piperidin-4-yl)oxy]-3,5-dimethoxyphenyl}propanoate

$^1$H-NMR (400 MHz, $CDCl_3$) δ 8.36 (d, J= 2.0 Hz, 1H), 8.09 (dd, J= 9.0, 2.0 Hz, 1H), 7.98 (d, J= 9.0 Hz, 1H), 7.71 (s, 1H), 7.57 (d, J= 8.7 Hz, 2H), 7.44 (d, J= 8.7 Hz, 2H), 6.72+6.39 (s, 2H), 4.18-4.09 (m, 2H), 4.09-4.00 (m, 1H), 3.83+3.78 (s, 6H), 3.32 (d, J= 13.7 Hz, 1H), 3.13 (d, J= 13.7 Hz, 1H), 3.00-2.90 (m, 2H), 2.87 (t, J= 8.0 Hz, 2H), 2.59 (t, J= 8.0 Hz, 2H), 2.55-2.40 (m, 2H), 1.92-1.78 (m, 4H), 1.30-1.18 (m, 3H).
MS (ESI+) 720, 2.35($M^+$+1, detection time)

Example 415

Ethyl 3-{3,5-dimethoxy-4-[(1-{3,3,3-trifluoro-2-hydroxy-2-[1-(4-methoxyphenyl)-6-nitro-1 H-indol-3-yl]propyl}piperidin-4-yl)oxy]phenyl}propanoate

$^1$H-NMR (400 MHz, $CDCl_3$) δ 8.13 (d, J= 2.0 Hz, 1H), 8.06 (dd, J= 9.0, 2.0 Hz, 1H), 7.96 (d, J= 9.0 Hz, 1H), 7.68 (s, 1H), 7.39 (d, J= 8.9 Hz, 1H), 7.08 (d, J= 8.9 Hz, 1H), 6.73+6.39 (s, 2H), 4.18-4.08 (m, 2H), 4.05-4.00 (m, 1H), 3.91 (s, 3H), 3.89+3.78 (s, 6H), 3.31 (d, J= 13.8 Hz, 1H), 3.14 (d, J= 13.8 Hz, 1H), 3.00-2.82 (m, 4H), 2.55-2.38 (m, 4H), 1.92-1.75 (m, 4H).
MS (ESI+) 716, 2.28($M^+$+1, detection time)

Example 416

Ethyl {3-methoxy-4-[(1-{3,3,3-trifluoro-2-hydroxy-2-[6-nitro-1-(1-phenylethyl)-1H-indol-3-yl]propyl}piperidin-4-yl)oxy]phenyl}acetate

$^1$H-NMR (400 MHz, $CDCl_3$) δ 8.23-8.19 (m, 1H), 7.99 (dd, J= 9.0, 2.0 Hz, 1H), 7.88+7.83 (d, J= 9.0 Hz, 1H), 7.77+7.71 (s, 1H), 7.35-7.22 (m, 3H), 7.14-7.05 (m, 2H), 6.85-6.72 (m, 3H), 5.74 (q, J= 7.0 Hz, 1H), 4.22 (bs, 1H), 4.19-4.08 (m, 2H), 3.82 (s, 3H), 3.53 (s, 2H), 3.30-3.10 (m, 2H), 2.90-2.65 (m, 4H), 2.53-2.32 (m, 4H), 2.00-1.75 (m, 7H), 1.30-1.20 (m, 3H).
MS (ESI+) 670, 2.23($M^+$+1, detection time)

Example 417

Ethyl 3-[4-({1-[2-(1-benzyl-5-nitro-1H-indol-3-yl)-3,3,3-trifluoro-2-hydroxypropyl]piperidin-4-yl}oxy)-3-methoxyphenyl]propanoate

MS (ESI+) 670, 2.22($M^+$+1, detection time)

Example 418

Ethyl {4-[(1-{2-[6-cyano-1-(3,4-dimethoxybenzyl)-1H-indol-3-yl]-3,3,3-trifluoro-2-hydroxypropyl}piperidin-4-yl)oxy]-3-methoxyphenyl}acetate

MS (ESI+) 696, 2.12($M^+$+1, detection time)

Example 419

Ethyl [4-({1-[2-(1-benzyl-6-cyano-1H-indol-3-yl)-3-chloro-3,3-difluoro-2-hydroxypropyl]piperidin-4-yl}oxy)-3-methoxyphenyl]acetate

$^1$H-NMR (400 MHz, $CDCl_3$) δ 7.92 (d, J= 8.4 Hz, 1H), 7.57 (s, 1H), 7.53 (s, 1H), 7.35 (dd, J= 8.4, 1.4 Hz, 1H), 7.33-7.27 (m, 3H), 7.08-7.01 (m, 2H), 6.84-6.72 (m, 3H), 5.34 (s, 2H), 4.21 (bs, 1H), 4.14 (q, J= 7.2 Hz, 1H), 3.82 (s, 3H), 3.53 (s, 2H), 3.31 (d, J= 13.5 Hz, 1H), 3.20 (d, J= 13.5 Hz, 1H), 2.88-2.70 (m, 2H), 2.52-2.35 (m, 2H), 1.95-1.72 (m, 4H), 1.25 (t, J= 7.2 Hz, 3H).
MS (ESI+) 652, 2.18($M^+$+1, detection time)

Example 420

Ethyl {4-[(1-{2-[6-cyano-1-(methoxymethyl)-1H-indol-3-yl]-3,3,3-trifluoro-2-hydroxypropyl}piperidin-4-yl)oxy]-3-methoxyphenyl}acetate

MS (ESI+) 590, 2.01($M^+$+1, detection time)

Example 421

Ethyl 3-[4-({1-[2-(1-benzyl-6-cyano-1H-indol-3-yl)-3,3,3-trifluoro-2-hydroxypropyl]piperidin-4-yl}oxy)-3-methoxyphenyl] propanoate

$^1$H-NMR (400 MHz, $CDCl_3$) δ 7.90 (d, J= 8.4 Hz, 1H), 7.58 (s, 1H), 7.51 (s, 1), 7.35 (dd, J= 8.4, 1.4 Hz, 1H), 7.33-7.29 (m, 3H), 7.07-7.05 (m, 2H), 6.78 (d, J= 8.1 Hz, 1H), 6.73-6.72 (m, 1H), 6.67 (d, J= 8.1 Hz, 1H), 5.82 (bs, 1H), 5.33 (s, 2H), 4.22-4.18 (m, 1H), 4.12 (q, J= 7.2 Hz, 1H), 3.81 (s, 3H), 3.23 (d, J= 13.6 Hz, 1H), 3.14 (d, J= 13.6 Hz, 1H), 2.88 (t, J= 7.8 Hz, 2H), 2.90-2.73 (m, 2H), 2.61 (t, J= 7.8 Hz, 1H), 2.60 (t, J= 7.8 Hz, 1H), 2.50-2.41 (m, 2H), 1.89-1.76 (m, 4H), 1.25 (t, J= 7.2 Hz, 3H).
MS (ESI+) 650, 2.23($M^+$+1, detection time)

Example 422

2-(1-Benzyl-6-nitro-1H-indol-3-yl)-1,1,1-trifluoro-3-[4-(pyrazin-2-ylcarbonyl)-piperazin-1-yl]propan-2-ol

$^1$H-NMR (400 MHz, $CDCl_3$) δ 8.94 (d, J= 1.5 Hz, 1H), 8.63 (d, J= 2.5 Hz, 1H), 8.51 (dd, J= 2.5, 1.5 Hz, 1H), 8.28 (d, J= 2.0 Hz, 1H), 8.04 (dd, J= 9.0, 2.0 Hz, 1H), 7.91 (d, J= 9.0 Hz, 1H), 7.56 (s, 1H), 7.36-7.29 (m, 3H), 7.11-7.09 (m, 2H), 5.40 (s, 2H), 5.33 (bs, 1H), 3.79 (bs, 2H), 3.62-3.57 (m, 2H), 3.30 (d, J= 13.8 Hz, 1H), 3.18 (d, J= 13.8 Hz, 1H), 2.74-2.66 (m, 2H), 2.60-2.58 (m, 2H).
MS (ESI+) 555, 2.31($M^+$+1, detection time)

Example 423

3-{4-[(6-Azetidin-1-ylpyridin-3-yl)carbonyl]piperazin-1-yl}-2-(1-benzyl-6-nitro-1H-indol-3-yl)-1,1,1-trifluoropropan-2-ol

$^1$H-NMR (400 MHz, $CDCl_3$) δ 8.28 (d, J= 2.0 Hz, 1H), 8.17 (d, J= 2.3 Hz, 1H), 8.04 (dd, J= 9.0, 2.0 Hz, 1H), 7.90 (d, J= 9.0 Hz, 1H), 7.55 (s, 1H), 7.53 (dd, J= 8.7,2.3 Hz, 1H9, 7.36-7.31 (m, 3H), 7.12-7.09 (m, 2H), 6.21 (d, J= 8.7 Hz, 1H), 5.39 (s, 2H), 4.08 (t, J= 7.4 Hz, 4H), 3.66-3.58 (m, 4H), 3.27 (d, J= 13.8 Hz, 1H), 3.16 (d, J= 13.8 Hz, 1H), 2.63-2.51 (m, 4H), 2.44 (t, J= 7.4 Hz, 1H), 2.41 (t, J= 7.4 Hz, 1H).
MS (ESI+) 609, 2.15($M^+$+1, detection time)

Example 424

tert-Butyl 1-[5-({4-[2-(1-benzyl-6-nitro-1H-indol-3-yl)-3,3,3-trifluoro-2-hydroxypropyl]piperazin-1-yl}carbonyl)pyridin-2-yl]prolinate

MS (ESI+) 723, 2.23($M^+$+1, detection time)

Example 425

Ethyl 2-{4-[2-(1-benzyl-6-nitro-1H-indol-3-yl)-3,3,3-trifluoro-2-hydroxypropyl]-piperazin-1-yl}pyrimidine-5-carboxylate

MS (ESI+) 599, 2.66($M^+$+1, detection time)

Example 426

Ethyl 3-(2-{4-[2-(1-benzyl-6-nitro-1H-indol-3-yl)-3,3,3-trifluoro-2-hydroxypropyl]piperazin-1-yl}pyrimidin-5-yl)propanoate

MS (ESI+) 627, 2.60($M^+$+1, detection time)

Example 427

Ethyl 3-{4-[(1-{2-[6-cyano-1-(cyclopropylmethyl)-1H-indol-3-yl]-3,3,3-trifluoro-2-hydroxypropyl}piperidin-4-yl)oxy]-3,5-dimethoxyphenyl}propanoate

MS (ESI+) 644, 2.17($M^+$+1, detection time)

Example 428

Ethyl 4-({1-[2-(1-benzyl-6-nitro-1H-indol-3-yl)-3,3,3-trifluoro-2-hydroxypropyl]-piperidin-4-yl}methyl)benzoate

MS (ESI+) 610, 2.28($M^+$+1, detection time)

Example 429

Ethyl [4-({1-[2-(1-benzyl-6-nitro-1H-indol-3-yl)-3,3,3-trifluoro-2-hydroxypropyl]-piperidin-4-yl}oxy)-3,5-dimethoxyphenyl](methylthio)acetate

$^1$H-NMR (400 MHz, $CDCl_3$) δ 8.26 (d, J= 2.0 Hz, 1H), 8.02 (dd, J= 9.0, 2.0 Hz, 1H), 7.90 (d, J= 9.0 Hz, 1H), 7.59 (s, 1H), 7.35-7.28 (m, 3H), 7.10-7.08 (m, 2H), 6.68 (s, 2H), 5.95 (bs, 1H), 5.40 (s, 2H), 4.41 (s, 1H), 4.27-4.15 (m, 2H), 4.10-4.03 (m, 1H), 3.80 (s, 6H), 3.24 (d, J= 13.6 Hz, 1H), 3.13 (d, J= 13.6 Hz, 1H), 2.93-2.74 (m, 2H), 2.46-2.37 (m, 2H), 2.10 (s, 3H), 1.83-1.78 (m, 4H), 1.27 (t, J= 7.1 Hz, 3H). MS (ESI+) 732, 2.31 ($M^+$+1, detection time)

Example 430

Ethyl 4-({1-[2-(1-benzyl-6-nitro-1H-indol-3-yl)-3,3,3-trifluoro-2-hydroxypropyl]-piperidin-4-yl}methyl)-3-methoxybenzoate

$^1$H-NMR (400 MHz, $CDCl_3$) δ 8.24 (d, J= 2.0 Hz, 1H), 8.01 (dd, J= 9.0, 2.0 Hz, 1H), 7.88 (d, J= 9.0 Hz, 1H), 7.57-7.54 (m, 2H), 7.49 (d, J= 1.5 Hz, 1H), 7.34-7.29 (m, 3H), 7.09-7.06 (m, 3H), 5.40 (s, 2H), 4.36 (q, J= 7.1 Hz, 1H), 3.84 (s, 3H), 3.19 (d, J= 13.6 Hz, 1H), 3.06 (d, J= 13.6 Hz, 1H), 2.89-2.87 (m, 1H), 2.57-2.41 (m, 4H), 2.08-2.05 (m, 1H), 1.63-1.19 (m, 5H), 1.38 (t, J= 7.1 Hz, 3H).
MS (ESI+) 640, 2.31($M^+$+1, detection time)

Example 431

Ethyl (2E)-3-[4-({1-[2-(1-benzyl-6-nitro-1H-indol-3-yl)-3,3,3-trifluoro-2-hydroxypropyl]piperidin-4-yl}methyl)-3,5-dimethoxyphenyl]acrylate

$^1$H-NMR (400 MHz, $CDCl_3$) δ 8.24 (d, J= 2.0 Hz, 1H), 8.01 (dd, J= 9.0, 2.0 Hz, 1H), 7.89 (d, J= 9.0 Hz, 1H), 7.62 (d, J= 15.9 Hz, 1H), 7.57 (s, 1H), 7.34-7.29 (m, 3H), 7.09-7.06 (m, 2H), 6.67 (s, 2H), 6.39 (d, J= 15.9 Hz, 1H), 5.39 (s, 2H), 4.26 (q, J= 7.1 Hz, 2H), 3.80 (s, 6H), 3.19 (d, J= 13.5 Hz, 1H), 3.05 (d, J= 13.5 Hz, 1H), 2.90-2.83 (m, 1H), 2.56 (d, J= 6.9 Hz, 2H), 2.50-2.39 (m, 2H), 2.11-2.05 (m, 1H), 1.59-1.23 (m, 5H), 1.34 (t, J= 7.1 Hz, 3H).
MS (ESI+) 696, 2.41($M^+$+1, detection time)

Example 432

Ethyl 3-[4-({1-[2-(1-benzyl-6-nitro-1H-indol-3-yl)-3,3,3-trifluoro-2-hydroxypropyl]piperidin-4-yl}methyl)-3,5-dimethoxyphenyl]propanoate

$^1$H-NMR (400 MHz, $CDCl_3$) δ 8.24 (d, J= 2.0 Hz, 1H), 8.01 (dd, J= 9.0, 2.0 Hz, 1H), 7.89 (d, J= 9.0 Hz, 1H), 7.57 (s, 1H), 7.34-7.29 (m, 3H), 7.09-7.06 (m, 2H), 6.36 (s, 2H), 5.39 (s, 2H), 4.13 (q, J= 7.2 Hz, 2H), 3.75 (s, 6H), 3.18 (d, J= 13.5 Hz, 1H), 3.05 (d, J= 13.5 Hz, 1H), 2.90 (t, J= 7.9 Hz, 2H), 2.88-2.82 (m, 1H), 2.61 (t, J= 7.9 Hz, 2H), 2.50 (d, J= 6.9 Hz, 2H), 2.42-2.38 (m, 2H), 2.09-2.06 (m, 1H), 1.60-1.32 (m, 5H), 1.26 (t, J= 7.2 Hz, 3H).
MS (ESI+) 698, 2.35($M^+$+1, detection time)

Example 433

Ethyl 3-{2-[2-(1-benzyl-6-nitro-1H-indol-3-yl)-3,3,3-trifluoro-2-hydroxypropyl]-1,2,3,4-tetrahydroisoquinolin-6-yl}propanoate

$^1$H-NMR (400 MHz, $CDCl_3$) δ 8.30 (d, J= 2.0 Hz, 1H), 8.02 (dd, J= 9.0, 2.0 Hz, 1H), 7.89 (d, J= 9.0 Hz, 1H), 7.60 (s, 1H), 7.38-7.29 (m, 3H), 7.16-7.12 (m, 2H), 6.94 (s, 1H), 6.94-6.93 (m, 1H), 6.70 (bs, 1H), 5.41 (s, 2H), 4.12 (q, J= 7.1 Hz, 2H), 3.77 (d, J= 15.3 Hz, 1H), 3.71 (d, J= 15.3 Hz, 1H), 3.45 (d, J= 13.5 Hz, 1H), 3.37 (d, J= 13.5 Hz, 1H), 2.95-2.79 (m, 4H), 2.88 (t, J= 7.8 Hz, 2H), 2.58 (t, J= 7.8 Hz, 2H), 1.24 (t, J= 7.1 Hz, 3H).

MS (ESI+) 596, 2.50($M^+$+1, detection time)

Example 434

Methyl ({2-[2-(1-benzyl-6-nitro-1H-indol-3-yl)-3,3,3=trifluoro-2-hydroxypropyl]-1,2,3,4-tetrahydroisoquinolin-6-yl}oxy) acetate

$^1$H-NMR (400 MHz, $CDCl_3$) δ 8.30 (d, J= 2.0 Hz, 1H), 8.04 (dd, J= 9.0, 2.0 Hz, 1H), 7.92 (d, J= 9.0 Hz, 1H), 7.59 (s, 1H), 7.36-7.29 (m, 3H), 7.13-7.11 (m, 2H), 6.74 (d, J= 8.4 Hz, 1H), 6.68 (dd, J= 8.4, 2.6 Hz, 1H), 6.62 (d, J= 2.6 Hz, 1H), 5.41 (s, 2H), 4.60 (s, 2H), 3.80 (s, 3H), 3.65 (s, 2H), 3.38 (d, J= 13.6 Hz, 1H), 3.29 (d, J= 13.6 Hz, 1H), 2.86-2.79 (m, 4H).
MS (ESI+) 584, 2.35($M^+$+1, detection time)

Example 435

({2-[2-(1-Benzyl-6-nitro-1H-indol-3-yl)-3,3,3-trifluoro-2-hydroxypropyl]-1,2,3,4-tetrahydroisoquinolin-6-yl}oxy)acetic acid

MS (ESI+) 612, 2.47($M^+$+1, detection time)

Example 436

Ethyl 4-({2-[2-(1-benzyl-6-nitro-1H-indol-3-yl)-3,3,3-trifluoro-2-hydroxypropyl]-1,2,3,4-tetrahydroisoquinolin-8-yl}oxy) butanoate

$^1$H-NMR (400 MHz, $CDCl_3$) δ 8.29 (d, J= 2.0 Hz, 1H), 8.05 (dd, J= 9.0, 2.0 Hz, 1H), 7.95 (d, J= 9.0 Hz, 1H), 7.61 (s, 1H), 7.36-7.30 (m, 3H), 7.13-7.08 (m, 3H), 6.70 (d, J= 7.8 Hz, 1H), 6.61 (d, J= 7.8 Hz, 1H), 5.71 (bs, 1H), 5.42 (s, 2H), 4.12 (q, J= 7.2 Hz, 2H), 3.88 (t, J= 6.1 Hz, 2H), 3.72-3.61 (m, 2H), 3.45 (d, J= 13.6 Hz, 1H), 3.34 (d, J= 13.6 Hz, 1H), 2.81-2.74 (m, 4H), 2.30 (t, J= 7.2 Hz, 2H), 1.94-1.87 (m, 2H), 1.25 (t, J= 7.2 Hz, 3H).
MS (ESI+) 626, 2.53($M^+$+1, detection time)

Example 437

2-(1-Benzyl-6-nitro-1H-indol-3-yl)-3-{4-[(5,7-dimethoxyisoquinolin-6-yl)oxy]-piperidin-1-yl}-1,1,1-trifluoropropan-2-ol

$^1$H-NMR (400 MHz, $CDCl_3$) δ 9.07 (s, 1H), 8.41 (d, J= 5.3 Hz, 1H), 8.26 (d, J= 2.0 Hz, 1H), 8.03 (dd, J= 9.0, 2.0 Hz, 1H), 7.91 (d, J= 9.0 Hz, 1H), 7.77 (d, J= 5.3 Hz, 1H), 7.59 (s, 1H), 7.34-7.28 (m, 3H), 7.10-7.08 (m, 2H), 7.04 (s, 1H), 5.40 (s, 2H), 4.35 (bs, 1H), 3.98 (s, 3H), 3.97 (s, 3H), 3.27 (d, J= 13.6 Hz, 1H), 3.15 (d, J= 13.6 Hz, 1H), 2.94-2.77 (m, 2H), 2.52-2.43 (m, 2H), 1.91-1.84 (m, 4H).
MS (ESI+) 651, 2.23($M^+$+1, detection time)

Example 438

Methyl 3-{3,5-dimethoxy-4-[(1-{3,3,3-trifluoro-2-[1-(2-fluorobenzyl)-6-nitro-1H-indol-3-yl]-2-hydroxypropyl}piperidin-4-yl)methyl]phenyl}propanoate

$^1$H-NMR (400 MHz, $CDCl_3$) δ 8.30 (d, J= 2.0 Hz, 1H), 8.02 (dd, J= 9.0, 2.0 Hz, 1H), 7.89 (d, J= 9.0 Hz, 1H), 7.59 (s, 1H), 7.33-7.28 (m, 1H), 7.13-7.09 (m, 1H), 7.07-7.05 (m, 1H), 6.92-6.89 (m, 1H), 6.35 (s, 2H), 6.16 (bs, 1H), 5.42 (s, 2H), 3.75 (s, 6H), 3.68 (s, 3H), 3.18 (d, J= 13.6 Hz, 1H), 3.04 (d, J= 13.6 nHz, 1H), 2.90 (t, J= 7.9 Hz, 2H), 2.86-2.84 (m, 1H), 2.63 (t, J= 7.9 Hz, 2H), 2.49 (d, J= 6.9 Hz, 2H), 2.44-2.39 (m, 2H), 2.10-2.04 (m, 1H), 1.50-1.16 (m, 5H).
MS (ESI+) 702, 2.37($M^+$+1, detection time)

Example 439

Methyl 3-{3,5-dimethoxy-4-[(1-{3,3,3-trifluoro-2-hydroxy-2-[1-(3-methoxybenzyl)-6-nitro-1H-indol-3-yl]propyl}piperidin-4-yl)methyl]phenyl}propanoate

$^1$H-NMR (400 MHz, $CDCl_3$) δ 8.24 (d, J= 2.0 Hz, 1H), 8.01 (dd, J= 9.0, 2.0 Hz, 1H), 7.89 (d, J= 9.0 Hz, 1H),

7.56 (s, 1H), 7.23 (dd, J= 8.0, 7.8 Hz, 1H), 6.82 (dd, J= 8.0, 2.1 Hz, 1H), 6.66 (d, J= 7.8 Hz, 1H), 6.58 (d, J= 2.1 Hz, 1H), 6.35 (s, 2H), 6.17 (bs, 1H), 5.36 (s, 2H), 3.77 (s, 6H), 3.71 (s, 3H), 3.69 (s, 3H), 3.19 (d, J= 13.6 Hz, 1H), 3.05 (d, J= 13.6 Hz, 1H), 2.90 (t, J= 7.9 Hz, 2H), 2.88-2.84 (m, 1H), 2.62 (t, J= 7.9 Hz, 2H), 2.53-2.46 (m, 1H), 2.50 (d, J= 6.9 Hz, 2H), 2.45-2.39 (m, 1H), 2.10-2.05 (m, 1H), 1.50-1.24 (m, 5H).
MS (ESI+) 714, 2.37($M^+$+1, detection time)

Example 440

Ethyl (2E)-3-{2-[2-(1-benzyl-6-nitro-1H-indol-3-yl)-3,3,3-trifluoro-2-hydroxypropyl]-5,7-dimethoxy-1,2,3,4-tetrahydroisoquinolin-6-yl}acrylate

$^1$H-NMR (400 MHz, $CDCl_3$) δ 8.30 (d, J= 2.0 Hz, 1H), 8.05 (dd, J= 9.1, 2.0 Hz, 1H), 7.93 (d, J= 16.3 Hz, 1H), 7.92 (d, J= 9.1 Hz, 1H), 7.61 (s, 1H), 7.37-7.32 (m, 3H), 7.14-7.12 (m, 2H), 6.87 (d, J= 16.3 Hz, 1H), 6.20 (s, 1H), 5.62 (s, 1H), 5.42 (s, 2H), 4.26 (q, J= 7.1 Hz, 2H), 3.78 (s, 2H), 3.71 (s, 6H), 3.41 (d, J= 13.5 Hz, 1H), 3.30 (d, J= 13.5 Hz, 1H), 2.83-2.74 (m, 4H), 1.34 (t, J= 7.1 Hz, 3H).
MS (ESI+) 654, 2.65($M^+$+1, detection time)

Example 441

Ethyl 3-{2-[2-(1-benzyl-6-nitro-1H-indol-3-yl)-3,3,3-trifluoro-2-hydroxypropyl]-5,7-dimethoxy-1,2,3,4-tetrahydroisoquinolin-6-yl}propanoate

MS (ESI+) 656, 2.50($M^+$+1, detection time)

Example 442

Ethyl 3-[4-({1-[2-(1-benzyl-6-nitro-1H-indol-3-yl)-3,3,3-trifluoro-2-hydroxypropyl]piperidin-4-yl}carbonyl)-3, 5-dimethoxyphenyl] propanoate

$^1$H-NMR (400 MHz, $CDCl_3$) δ 8.25 (d, J= 2.0 Hz, 1H), 8.02 (dd, J= 9.0, 2.0 Hz, 1H), 7.87 (d, J= 9.0 Hz, 1H), 7.59 (s, 1H), 7.35-7.29 (m, 3H), 7.10-7.08 (m, 2H), 6.38 (s, 2H), 5.81 (bs, 1H), 5.39 (s, 2H), 4.13 (q, J= 7.1 Hz, 2H), 3.74 (s, 6H), 3.20 (d, J= 13.5 Hz, 1H), 3.11 (d, J= 13.5 Hz, 1H), 2.92 (t, J= 7.7 Hz, 2H), 2.69-2.47 (m, 3H), 2.61 (t, J= 7.7 Hz, 2H), 2.22-2.18 (m, 1H), 1.88-1.56 (m, 4H), 1.25 (t, J= 7.1 Hz, 3H).
MS (ESI+) 712, 2.43($M^+$+1, detection time)

Example 443

Ethyl 3-[4-({1-[2-(1-benzyl-6-nitro-1H-indol-3-yl)-3,3,3-trifluoro-2-hydroxypropyl] azepan-4-yl}oxy)-3,5-dimethoxyphenyl] propanoate

$^1$H-NMR (400 MHz, $CDCl_3$) δ 8.25-8.24 (m, 1H), 8.00 (m, 1H), 7.89 (d, J= 9.0 Hz, 1H), 7.59+7.58 (s, 1H), 7.33-7.27 (m, 3H), 7.10-7.07 (m, 2H), 6.39 (s, 1H), 6.38 (s, 1H), 6.01 (bs, 1H), 5.39 (s, 2H), 4.24-4.20 (m, 1H), 4.13 (q, J= 7.1 Hz, 2H), 3.78 (s, 3H), 3.75 (s, 3H), 3.32 (s, 2H), 2.94-2.56 (m, 4H), 2.89 (t, J= 7.8 Hz, 2H), 2.61 (t, J= 7.8 Hz, 1H), 2.60 (t, J= 7.8 Hz, 1H), 1.94-1.81 (m, 5H), 1.47-1.43 (m, 1H), 1.24 (t, J= 7.1 Hz, 3H).
MS (ESI+) 714, 2.37($M^+$+1, detection time)

Example 444

Ethyl (2E)-3-[4-({1-[2-(1-benzyl-6-nitro-1H-indol-3-yl)-3,3,3-trifluoro-2-hydroxypropyl]azepan-4-yl}oxy)-3,5-dimethoxyphenyl]acrylate

$^1$H-NMR (400 MHz, $CDCl_3$) δ 8.25 (m, 1H), 8.04-8.00 (m, 1H), 7.89 (d, J= 9.0 Hz, 1H), 7.59 (d, J= 15.9 Hz, 1H), 7.59 (s, 1H), 7.33-7.28 (m, 3H), 7.10-7.07 (m, 2H), 6.73+6.72 (s, 2H), 6.34 (d, J= 15.9 Hz, 1H), 5.97 (bs, 1H), 5.39 (s, 2H), 4.38-4.32 (m, 1H), 4.27 (q, J= 7.1 Hz, 2H), 3.83+3.80 (s, 3H), 3.34+3.30 (d, J= 13.9 Hz, 2H), 2.96-2.52 (m, 4H), 1.96-1.82 (m, 2H), 1.34 (t, J= 7.1 Hz, 3H).
MS (ESI+) 712, 2.35(M++1, detection time)

Example 445

Ethyl (2E)-3-[4-({1-[2-(1-benzyl-6-nitro-1H-indol-3-yl)-3,3,3-trifluoro-2-hydroxypropyl]azepan-4-yl}oxy)-3,5-dimethoxyphenyl]acrylate

$^1$H-NMR (400 MHz, $CDCl_3$) δ 8.25 (m, 1H), 8.04-8.00 (m, 1H), 7.89 (d, J= 9.0 Hz, 1H), 7.59 (d, J= 15.9 Hz, 1H), 7.59 (s, 1H), 7.33-7.28 (m, 3H), 7.10-7.07 (m, 2H), 6.73+6.72 (s, 2H), 6.34 (d, J= 15.9 Hz, 1H), 5.97 (bs, 1H), 5.39 (s, 2H), 4.38-4.32 (m, 1H), 4.27 (q, J= 7.1 Hz, 2H), 3.83+3.80 (s, 3H), 3.34+3.30 (d, J= 13.9 Hz, 2H), 2.96-2.52 (m, 4H), 1.96-1.82 (m, 2H), 1.34 (t, J= 7.1 Hz, 3H).
MS (ESI+) 712, 2.35($M^+$+1, detection time)

Example 446

Ethyl 3-[4-({1-[2-(1-benzyl-6-nitro-1H-indol-3-yl)-3,3,3-trifluoro-2-hydroxypropyl]piperidin-4-yl}methoxy)-3,5-dimethoxyphenyl]propanoate

$^1$H-NMR (400 MHz, $CDCl_3$) δ 8.26 (d, J= 2.0 Hz, 1H), 8.03 (dd, J= 9.0, 2.0 Hz, 1H), 7.91 (d, J= 9.0 Hz, 1H), 7.90 (s, 1H), 7.35-7.30 (m, 3H), 7.10-7.08 (m, 2H), 6.40 (s, 2H), 6.03 (bs, 1H), 5.40 (s, 2H), 4.13 (q, J= 7.1 Hz, 2H), 3.79 (s, 6H), 3.75 (d, J= 6.4 Hz, 1H), 3.23 (d, J= 13.6 Hz, 1H), 3.10 (d, J= 13.6 Hz, 1H), 2.97-2.94 (m, 1H), 2.88 (t, J= 7.8 Hz, 2H), 2.60 (t, J= 7.8 Hz, 2H), 2.62-2.51 (m, 2H), 2.23-2.18 (m, 1H), 1.93-1.88 (m, 1H), 1.75-1.72 (m, 2H), 1.46-1.28 (m, 2H), 1.24 (t, J= 7.1 Hz, 3H).
MS (ESI+) 714, 2.21($M^+$+1, detection time)

Example 447

Ethyl (2E)-3-{4-[{1-[2-(1-benzyl-6-nitro-1H-indol-3-yl)-3,3,3-trifluoro-2-hydroxypropyl]piperidin-4-yl}(methoxy)methyl]-3,5-dimethoxyphenyl} acrylate

MS (ESI+) 726, 2.38(M++1, detection time)

Example 448

Ethyl (2E)-3-[4-({1-[2-(1-benzyl-6-nitro-1H-indol-3-yl)-3,3,3-trifluoro-2-hydroxypropyl]azepan-4-yl}oxy)-3-methoxyphenyl]acrylate

$^1$H-NMR (400 MHz, $CDCl_3$) δ 8.26 (bs, 1H), 8.04-8.00 (m, 1H), 7.91-7.87 (m, 1H), 7.61 (d, J= 15.9 Hz, 1H), 7.59 (s, 0.5H), 7.58 (s, 0.5H), 7.30-7.27 (m, 3H), 7.10-7.03 (m, 4H), 6.79-6.76 (m, 1H), 6.29 (d, J= 15.9 Hz, 1H), 5.83 (bs, 1H), 5.34 (s, 2H), 4.55-4.52 (m, 1H), 4.26 (q, J= 7.1 Hz, 2H), 3.87 (s, 1.5H), 3.85 (s, 1.5H), 3.38-3.29 (m, 2H), 2.86-2.68 (m, 4H), 2.00-1.89 (m, 5H), 1.62-1.45 (m, 1H).
MS (ESI+) 682, 2.27($M^+$+1, detection time)

Example 449

Ethyl 3-[4-({1-[2-(1-benzyl-6-nitro-1H-indol-3-yl)-3,3,3-trifluoro-2-hydroxypropyl]azepan-4-yl}oxy)-3-methoxyphenyl] propanoate

$^1$H-NMR (400 MHz, CDCl3) δ 8.26 (d, J= 2.0 Hz, 1H), 8.04-8.00 (m, 1H), 7.89 (d, J= 9.0 Hz, 1H), 7.59+7.58 (s, 1H), 7.32-7.27 (m, 3H), 7.10-7.07 (m, 2H), 6.75-6.67 (m, 3H), 5.90 (bs, 1H), 5.38 (s, 2H), 4.40-4.37 (m, 1H), 4.13 (q, J= 7.1 Hz, 2H), 3.81+3.80 (s, 3H), 3.39-3.28 (m, 2H), 2.89 (t, J= 7.5 Hz, 2H), 2.85-2.67 (m, 4H), 2.59(t, J= 7.5 Hz, 2H), 1.97-1.79 (m, 5H), 1.49-1.47 (m, 1H), 1.26+1.22 (t, J=7.1Hz, 3H).
MS (ESI+) 684, 2.22($M^+$+1, detection time)

Example 450

Ethyl (2E)-3-[4-({1-[2-(1-benzyl-6-nitro-1H-indol-3-yl)-3,3,3-trifluoro-2-hydroxypropyl]piperidin-4-yl}oxy)-3-fluoro-5-methoxyphenyl]acrylate

$^1$H-NMR (400 MHz, $CDCl_3$) δ 8.26 (d, J= 2.0 Hz, 1H), 8.03 (dd, J= 9.0, 2.0 Hz, 1H), 7.91 (d, J= 9.0 Hz, 1H),

7.58 (s, 1H), 7.54 (d, J= 15.9 Hz, 1H), 7.36-7.28 (m, 3H), 7.12-7.07 (m, 2H), 6.91-6.88 (m, 1H), 6.82 (s, 1H), 6.32 (d, J= 15.9 Hz, 1H), 5.83 (bs, 1H), 5.40 (s, 2H), 4.29-4.23 (m, 1H), 4.26 (q, J= 7.1 Hz, 2H), 3.85 (s, 3H), 3.25 (d, J= 13.6 Hz, 1H), 3.14 (d, J= 13.6 Hz, 1H), 2.88-2.84 (m, 2H), 2.51-2.50 (m, 2H), 1.88-1.82 (m, 4H), 1.33 (t, J= 7.1 Hz, 3H).
MS (ESI+) 686, 2.36($M^+$+1, detection time)

Example 451

Ethyl 3-[4-({1-[2-(1-benzyl-6-nitro-1H-indol-3-yl)-3,3,3-trifluoro-2-hydroxypropyl]piperidin-4-yl}oxy)-3-fluoro-5-methoxyphenyl]propanoate

$^1$H-NMR (400 MHz, $CDCl_3$) δ 8.26 (d, J= 2.0 Hz, 1H), 8.03 (dd, J= 9.0, 2.0 Hz, 1H), 7.91 (d, J= 9.0 Hz, 1H), 7.58 (s, 1H), 7.35-7.30 (m, 3H), 7.11-7.08 (m, 2H), 6.56-6.51 (m, 2H), 5.40 (s, 2H), 4.15-4.10 (m, 1H), 4.12 (q, J= 7.2 Hz, 2H), 3.79 (s, 3H), 3.26-3.12 (m, 2H), 2.86 (t, J= 7.7 Hz, 2H), 2.85-2.80 (m, 2H), 2.58 (t, J= 7.7 Hz, 2H), 2.50-2.40 (m, 2H), 1.90-1.74 (m, 4H), 1.26 (t, J= 7.2 Hz, 3H).
MS (ESI+) 688, 2.29($M^+$+1, detection time)

Example 452

2-(1-Benzyl-6-nitro-1H-indol-3-yl)-1,1,1-trifluoro-3-[4-(pyrazin-2-yloxy)-piperidin-1-yl]propan-2-ol

MS (ESI+) 542, 2.19($M^+$+1, detection time)

Example 453

2-(1-Benzyl-6-nitro-1H-indol-3-yl)-3-{4-[(5-bromopyrimidin-2-yl)oxy]piperidin-1-yl}-1,1,1-trifluoropropan-2-ol

MS (ESI+) 621, 2.28($M^+$+1, detection time)

Example 454

Ethyl [4-({(3-exo)-8-[2-(1-benzyl-6-nitro-1H-indol-3-yl)-3,3,3-trifluoro-2-hydroxypropyl]-8-azabicyclo[3.2.1]oct-3-yl}oxy)-3-methoxyphenyl] acetate

$^1$H-NMR (400 MHz, $CDCl_3$) δ 8.27 (d, J= 2.0 Hz, 1H), 8.03 (dd, J= 9.0, 2.0 Hz, 1H), 7.91-7.89 (m, 1H), 7.58 (s, 1H), 7.33-7.27 (m, 3H), 7.12-7.09 (m, 2H), 6.82-6.74 (m, 3H), 5.39 (s, 2H), 4.37-4.34 (m, 1H), 4.15 (q, J= 7.2 Hz, 2H), 3.83 (s, 3H), 3.54 (s, 2H), 3.40-2.80 (m, 2H), 1.94-1.48 (m, 10H), 1.26 (t, J= 7.2 Hz, 3H). MS (ESI+) 682, 2.33 ($M^+$+1, detection time)

Example 455

Ethyl (2E)-3-[4-({(3-exo)-8-[2-(1-benzyl-6-nitro-1H-indol-3-yl)-3,3,3-trifluoro-2-hydroxypropyl]-8-azabicyclo [3.2.1]oct-3-yl}oxy)-3, 5-dimethoxyphenyl] acrylate

$^1$H-NMR (400 MHz, $CDCl_3$) δ 8.26 (d, J= 2.0 Hz, 1H), 8.02 (dd, J= 9.0, 2.0 Hz, 1H), 7.91 (bs, 1H), 7.74 (d, J= 15.8 Hz, 1H), 7.61 (s, 1H), 7.34-7.27 (m, 3H), 7.11-7.09 (m, 2H), 6.73 (s, 2H), 6.34 (d, J= 15.8 Hz, 1H), 6.08 (bs, 1H), 5.40 (s, 2H), 4.29-4.21 (m, 1H), 4.26 (q, J= 7.1 Hz, 2H), 3.84 (s, 6H), 3.40-2.80 (m, 4H), 2.04-1.46 (m, 8H), 1.34 (t, J= 7.1 Hz, 3H).
MS (ESI+) 724, 2.37($M^+$+1, detection time)

Example 456

Methyl 3-[4-({1-[2-(1-benzyl-6-nitro-1H-indol-3-yl)-3,3,3-trifluoro-2-hydroxypropyl]azetidin-3-yl}oxy)-3,5-dimethoxyphenyl]propanoate

$^1$H-NMR (400 MHz, $CDCl_3$) δ 8.25 (d, J= 2.0 Hz, 1H), 8.03 (dd, J= 9.0, 2.0 Hz, 1H), 7.92 (d, J= 9.0 Hz, 1H), 7.54 (s, 1H), 7.38-7.28 (m, 3H), 7.15-7.07 (m, 2H), 6.37 (s, 2H), 5.38 (s, 2H), 4.60-4.50 (m, 1H), 3.85-3.70 (m, 1H), 3.74 (s, 6H), 3.67 (s, 3H), 3.60-3.30 (m, 3H), 3.42 (d, J= 13.1 Hz, 1H), 3.23 (d, J= 13.1 Hz, 1H), 2.88 (t, J= 7.9 Hz, 2H), 2.61 (t, J= 7.9 Hz, 2H).

MS (ESI+) 658, 2.23($M^+$+1, detection time)

Example 457

Methyl 3-[4-({1-[2-(1-benzyl-6-nitro-1H-indol-3-yl)-3,3,3-trifluoro-2-hydroxypropyl]piperidin-4-yl}oxy)-3,5-dimethoxyphenyl]-3-hydroxy-2,2-dimethylpropanoate

MS (ESI+) 730, 2.30($M^+$+1, detection time)

Example 458

1-Benzyl-3-[2,2,2-trifluoro-1-hydroxy-1-(1,3,4,9-tetrahydro-2H-β-carbolin-2-ylmethyl)ethyl]-1 H-indole-6-carbonitrile

$^1$H-NMR (400 MHz, $CDCl_3$) δ 7.93 (s, J= 8.4 Hz, 1H), 7.63 (s, 1H), 7.50 (s, 1H), 7.45 (d, J= 7.6 Hz, 1H), 7.40-7.30 (m, 4H), 7.20-7.05 (m, 4H), 5.73 (br, 1H), 5.33 (s, 2H), 3.76 (d, J= 15.1 Hz, 1H), 3.70 (d, J= 15.1 Hz, 1H), 3.40 (s, 2H), 3.05-2.95 (m, 2H), 2.82-2.70 (m, 2H).
MS (ESI+) 515, 2.17($M^+$+1, detection time)

Example 459

2-(1-Benzyl-6-nitro-1H-indol-3-yl)-1,1,1-trifluoro-3-[4-(2-methoxybenzoyl)-piperazin-1-yl]propan-2-ol

$^1$H-NMR (400 MHz, $CDCl_3$) δ 8.27 (s, 1H), 8.03 (d, J= 9.1 Hz, 1H), 7.90 (d, J= 9.1 Hz, 1H), 7.54 (s, 1H), 7.37-7.27 (m, 4H), 7.20 (d, J= 7.8 Hz, 1H), 7.12-7.05 (m, 2H), 7.00-6.96 (m, 1H), 6.89 (d, J= 7.8 Hz, 1H), 5.42 (bs, 1H), 5.38 (s, 2H), 3.84 (s, 3H), 3.84-3.79 (m, 2H), 3.27 (d, J= 13.9Hz, 1H), 3.29-3.12 (m, 2H), 3.15 (d, J= 13.9 Hz, 1H), 2.75-2.37 (m, 4H).
MS (ESI+) 583, 2.45($M^+$+1, detection time)

Example 460

Ethyl 3-[4-({4-[2-(1-benzyl-6-nitro-1H-indol-3-yl)-3,3,3-trifluoro-2-hydroxypropyl]piperazin-1-yl}carbonyl)phenyl]propanoate

MS (ESI+) 653, 2.55($M^+$+1, detection time)

Example 461

Ethyl (2E)-3-[4-({4-[2-(1-benzyl-6-nitro-1H-indol-3-yl)-3,3,3-trifluoro-2-hydroxypropyl]piperazin-1-yl}carbonyl)phenyl] acrylate

MS (ESI+) 651, 2.58($M^+$+1, detection time)

Example 462

Methyl 6-({4-[2-(1-benzyl-6-nitro-1H-indol-3-yl)-3,3,3-trifluoro-2-hydroxypropyl]piperazin-1-yl}carbonyl)nicotinate

MS (ESI+) 612, 2.42($M^+$+1, detection time)

Example 463

2-(1-Benzyl-6-nitro-1H-indol-3-yl)-1,1,1-trifluoro-3-[4-(pyridin-2-ylcarbonyl)-piperazin-1-yl]propan-2-ol

$^1$H-NMR (400 MHz, $CDCl_3$) δ 8.55 (d, J= 4.3 Hz, 1H), 8.27 (d, J= 1.5 Hz, 1H), 8.03 (dd, J= 9.0, 1.5 Hz, 1H), 7.91 (d, J= 9.0 Hz, 1H), 7.79 (dd, J= 7.7, 6.5 Hz, 1H), 7.64 (d, J= 7.7 Hz, 1H), 7.56 (s, 1H), 7.40-7.31 (m, 4H), 7.10-7.09 (m, 2H), 5.39 (s, 2H), 3.77 (bs, 2H), 3.58 (bs, 2H), 3.28 (d, J= 13.7 Hz, 1H), 3.17 (d, J= 13.7 Hz, 1H), 2.74-2.63 (m, 2H), 2.56 (bs, 2H), 1.67 (bs, 2H).
MS (ESI+) 554, 2.31($M^+$+1, detection time)

Example 464

2-(1-Benzyl-6-nitro-1H-indol-3-yl)-1,1,1-trifluoro-3-[4-(pyridin-3-ylcarbonyl)-piperazin-1-yl]propan-2-ol

$^1$H-NMR (400 MHz, $CDCl_3$) δ 8.65 (dd, J= 4.8, 1.5 Hz, 1H), 8.60 (d, J= 1.5 Hz, 1H), 8.28 (d, J= 2.0 Hz, 1H), 8.02 (dd, J= 9.0, 2.0 Hz, 1H), 7.90 (d, J= 9.0 Hz, 1H), 7.73-7.70 (m, 1H), 7.55 (s, 1H), 7.41-7.28 (m, 4H), 7.16-7.08 (m, 2H), 5.39 (s, 2H), 3.85-3.61 (m, 2H), 3.49-3.30 (m, 2H), 3.29 (d, J= 13.8 Hz, 1H), 3.17 (d, J= 13.8 Hz, 1H), 2.76-2.42 (m, 4H).
MS (ESI+) 554, 2.26($M^+$+1, detection time)

Example 465

2-({1-[2-(1-Benzyl-6-nitro-1H-indol-3-yl)-3,3,3-trifluoro-2-hydroxypropyl]-piperidin-4-yl}oxy)phenol

$^1$H-NMR (400 MHz, $CDCl_3$) δ 8.27 (d, J= 2.0 Hz, 1H), 8.03 (dd, J= 9.0, 2.0 Hz, 1H), 7.91 (d, J= 9.0 Hz, 1H), 7.59 (bs, 1H), 7.37-7.27 (m, 3H), 7.15-7.06 (m, 2H), 6.98-6.77 (m, 4H), 5.69 (bs, 1H), 5.59 (bs, 1H), 5.40 (s, 2H), 4.36 (bs, 1H), 3.40-3.04 (m, 2H), 2.89-2.60 (m, 2H), 2.60-2.40 (m, 2H), 2.07-1.50 (m, 4H).
MS (ESI+) 556, 2.19($M^+$+1, detection time)

In a similar manner to the preparation of the compound of Example 2, the compounds of Examples 466 to 474 having a chemical structure as disclosed in Table 23 were obtained

[Table 23]

| | | |
|---|---|---|
| 466 | 467 | 468 |
| 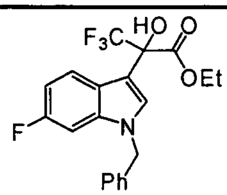 | 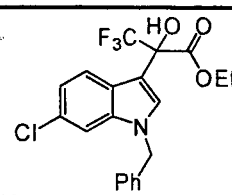 | 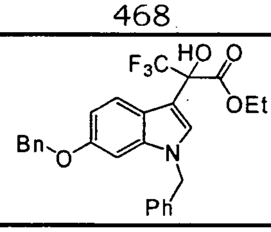 |
| 469 | 470 | 471 |
| 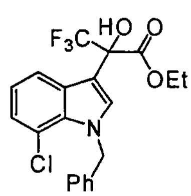 | 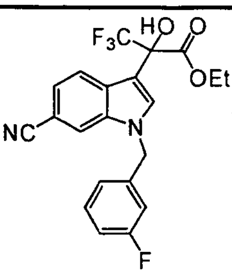 | 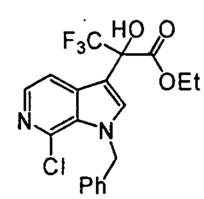 |
| 472 | 473 | 474 |
| 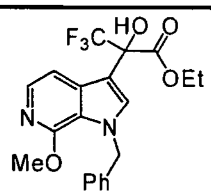 | 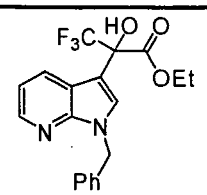 | 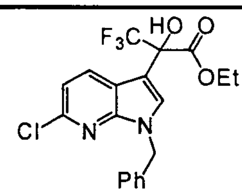 |

Example 466

Ethyl 2-(1-benzyl-6-fluoro-1H-indol-3-yl)-3,3,3-trifluoro-2-hydroxypropanoate MS (ESI+) 396, 2.43($M^+$+1, detection time)

Example 467

Ethyl 2-(1-benzyl-6-chloro-1H-indol-3-yl)-3,3,3-trifluoro-2-hydroxypropanoate

MS (ESI+) 412, 2.50($M^+$+1, detection time)

Example 468

Ethyl 2-[1-benzyl-6-(benzyloxy)-1H-indol-3-yl]-3,3,3-trifluoro-2-hydroxypropanoate

MS (ESI+) 484, 2.55($M^+$+1, detection time)

Example 469

Ethyl 2-(1-benzyl-7-chloro-1H-indol-3-yl)-3,3,3-trifluoro-2-hydroxypropanoate

MS (ESI+) 412, 2.71($M^+$+1, detection time)

Example 470

Ethyl 2-[6-cyano-1-(3-fluorobenzyl)-1 H-indol-3-yl]-3,3,3-trifluoro-2-hydroxypropanoate

MS (ESI+) 421, 2.36($M^+$+1, detection time)

Example 471

Ethyl 2-(1-benzyl-7-chloro-1H-pyrrolo[2,3-c]pyridin-3-yl)-3,3,3-trifluoro-2-hydroxypropanoate

$^1$H-NMR (400 MHz, $CDCl_3$) $\delta$ 8.05 (d, J= 5.6 Hz, 1H), 7.86 (d, J= 5.6 Hz, 1H), 7.59 (s, 1H), 7.38-7.27 (m, 3H), 7.09-7.02 (m, 2H), 5.83 (d, J= 16.1 Hz, 1H), 5.77 (d, J= 16.1 Hz, 1H), 4.53 (bs, 1H), 4.50-4.33 (m, 2H), 1.31 (t, J= 8.0 Hz, 3H).
MS (ESI+) 413, 2.46($M^+$+1, detection time)

Example 472

Ethyl 2-(1-benzyl-7-methoxy-1H-pyrrolo[2,3-c]pyridin-3-yl)-3,3,3-trifluoro-2-hydroxypropanoate

$^1$H-NMR (400 MHz, $CDCl_3$) $\delta$ 7.74 (d, J= 5.8 Hz, 1H), 7.43-7.37 (m, 2H), 7.33-7.22 (m, 3H), 7.12 (d, J= 6.7 Hz, 2H), 5.60 (s, 2H), 4.50-4.27 (m, 2H), 4.01 (s, 3H), 1.30 (t, J= 7.1 Hz, 3H).
MS (ESI+) 409, 2.28($M^+$+1, detection time)

Example 473

Ethyl 2-(1-benzyl-1H-pyrrolo[2,3-b]pyridin-3-yl)-3,3,3-trifluoro-2-hydroxypropanoate

$^1$H-NMR (400 MHz, $CDCl_3$) $\delta$ 8.37 (dd, J= 4.7, 1.5 Hz, 1H), 8.26 (d, J= 8.0 Hz, 1H), 7.48 (s, 1H), 7.35-7.20 (m, 5H), 7.13 (dd, J= 6.4, 4.7 Hz, 1H), 5.52 (d, J= 15.2 Hz, 1H), 5.48 (d, J= 15.2 Hz, 1H), 4.45 (bs, 1H), 4.44-4.30 (m, 2H), 1.30 (t, J= 7.2 Hz, 3H).
MS (ESI+) 379, 2.42($M^+$+1, detection time)

Example 474

Ethyl 2-(1-benzyl-6-chloro-1H-pyrrolo[2,3-b]pyridin-3-yl)-3,3,3-trifluoro-2-hydroxypropanoate

$^1$H-NMR (400 MHz, $CDCl_3$) $\delta$ 8.19 (d, J= 8.3 Hz, 1H), 7.42 (s, 1H), 7.38-7.20 (m, 5H), 7.14 (d, J= 8.4 Hz, 1H), 5.47 (d, J= 15.1 Hz, 1H), 5.41 (d, J= 15.1 Hz, 1H), 4.50-4.30 (m, 2H), 1.29 (t, J= 8.0 Hz, 3H).
MS (ESI+) 413, 2.65($M^+$+1, detection time)
In a similar manner to the preparation of the compound of Example 4, the compounds of Examples 475 to 627

having a chemical structure as disclosed in Tables 24 to 39 were obtained.

[Table 24]

| 475 | 476 |
|---|---|
| 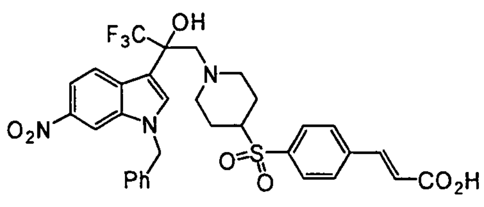 | 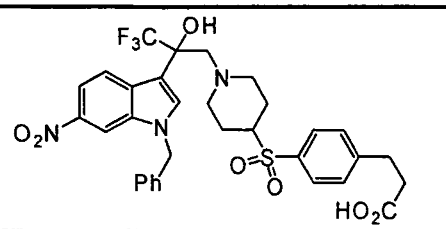 |
| 477 | 478 |
| 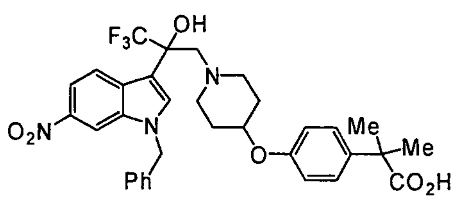 | 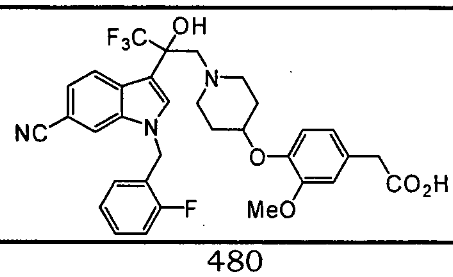 |
| 479 | 480 |
| 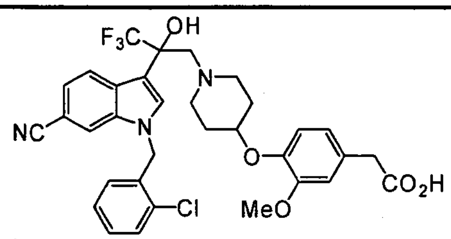 | 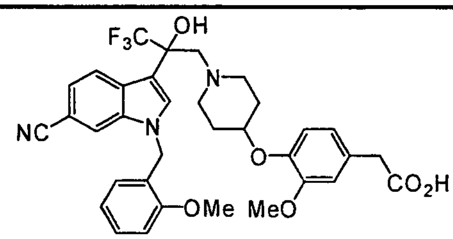 |
| 481 | 482 |
| 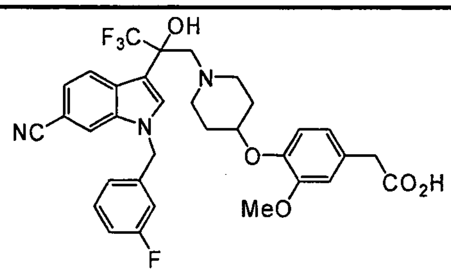 | 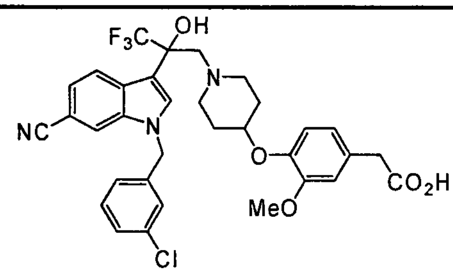 |
| 483 | 484 |
| 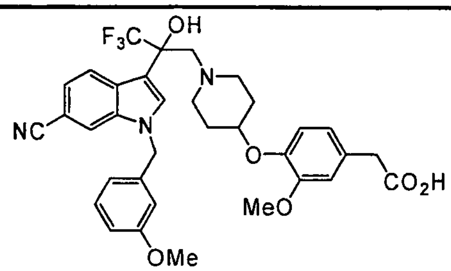 | 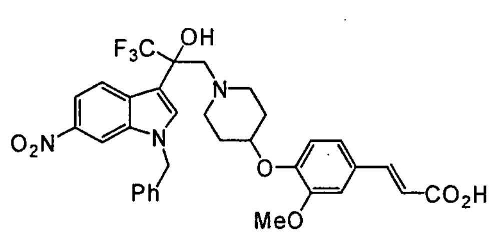 |

[Table 25]

| 485 | 486 |
|---|---|
| 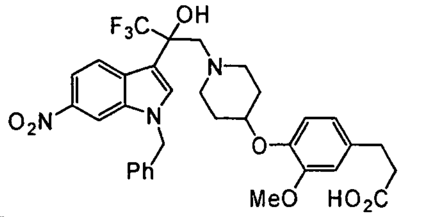 | 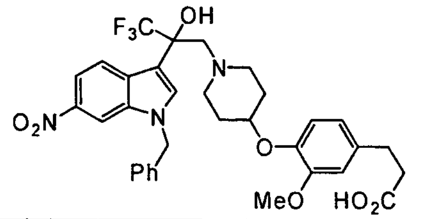 |
| 487 | 488 |
| 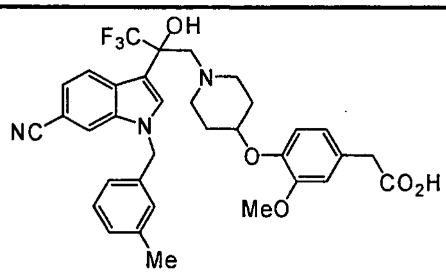 | 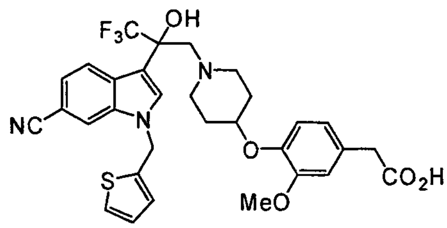 |
| 489 | 490 |
| 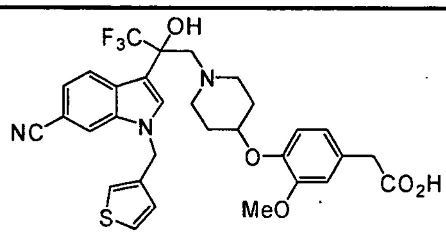 | 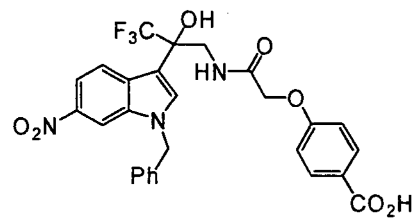 |
| 491 | 492 |
| 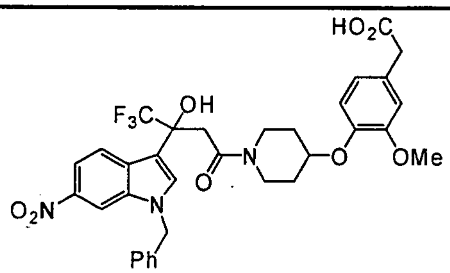 | 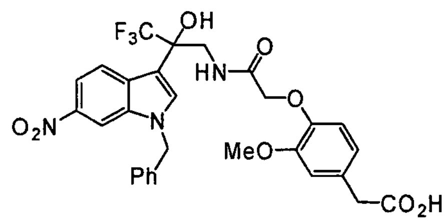 |
| 493 | 494 |
| 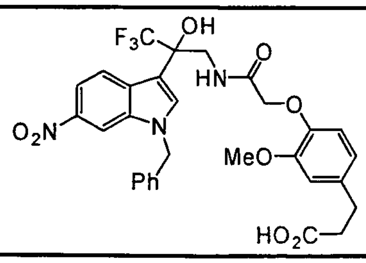 | 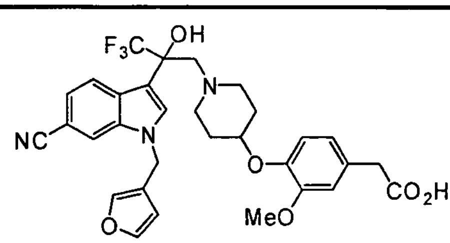 |

[Table 26]

| 495 | 496 |
|---|---|
| 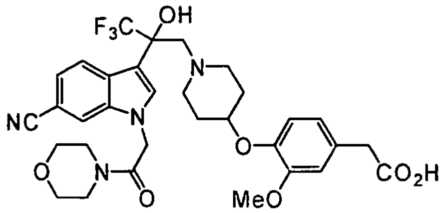 | 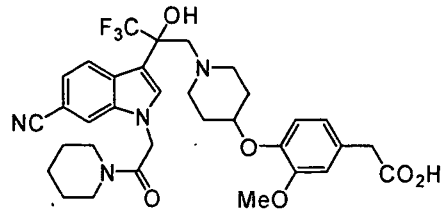 |
| 497 | 498 |
| 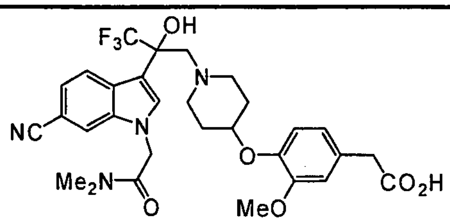 | 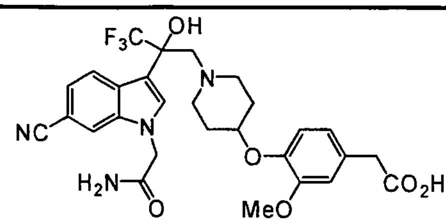 |
| 499 | 500 |
| 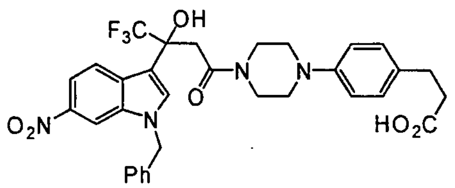 | 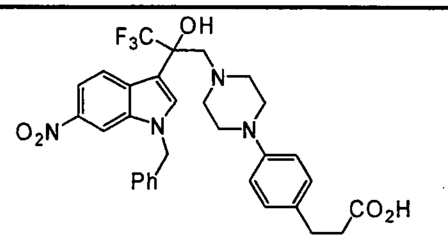 |
| 501 | 502 |
| 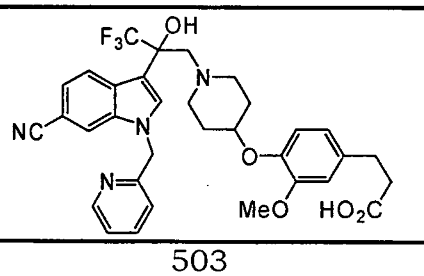 | 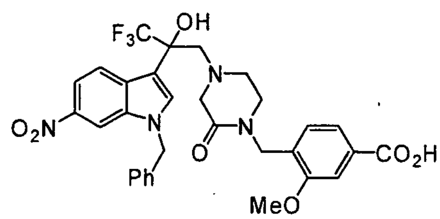 |
| 503 | 504 |
| 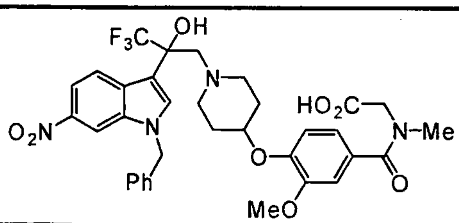 | 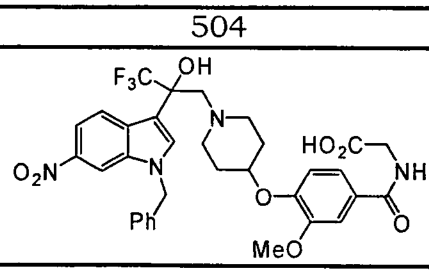 |

[Table 27]

| | |
|---|---|
| 505 | 506 |
| 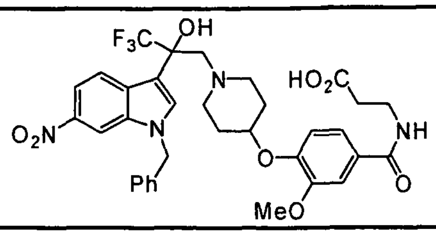 | 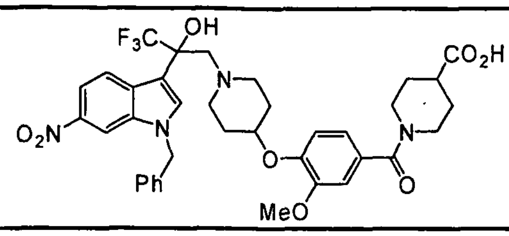 |
| 507 | 508 |
| 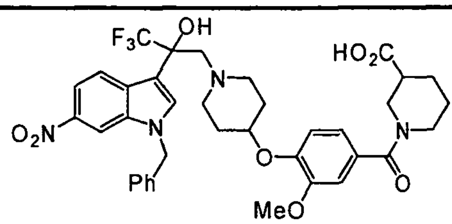 | 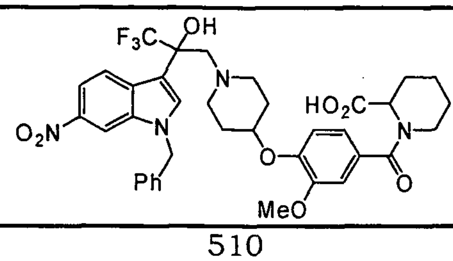 |
| 509 | 510 |
| 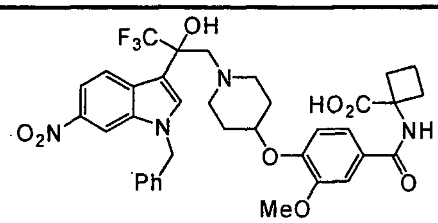 | 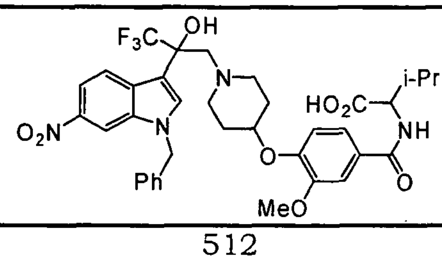 |
| 511 | 512 |
| 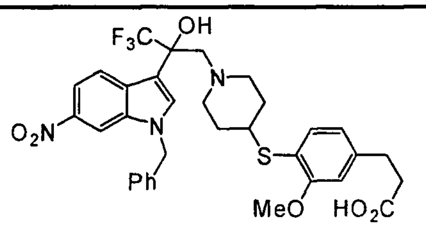 | 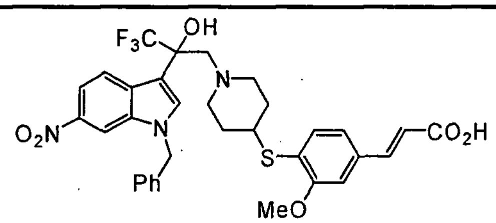 |
| 513 | 514 |
| 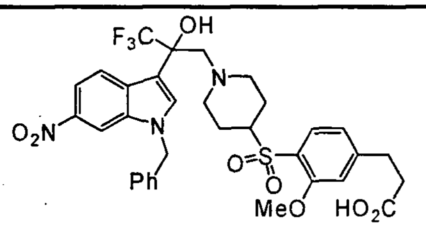 | 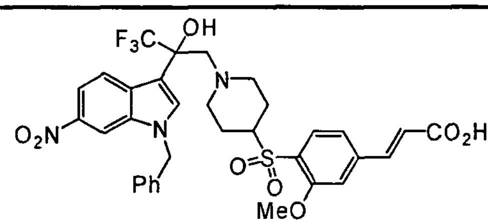 |

[Table 28]

| 515 | 516 |
|---|---|
| 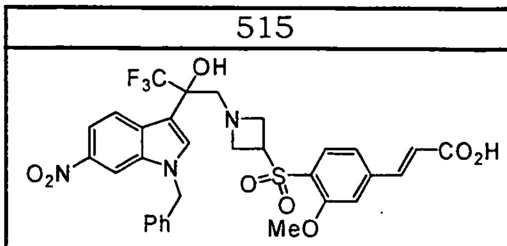 | 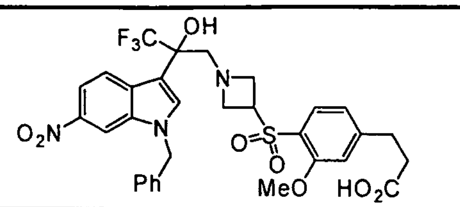 |
| 517 | 518 |
| 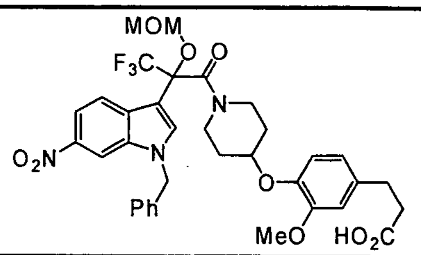 | 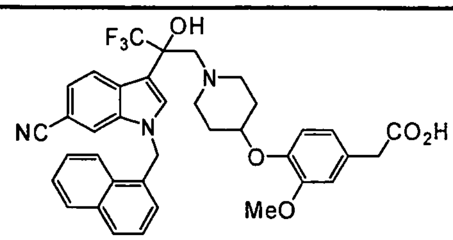 |
| 519 | 520 |
| 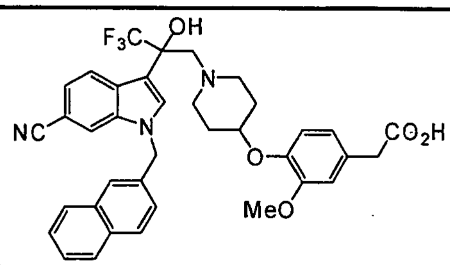 | 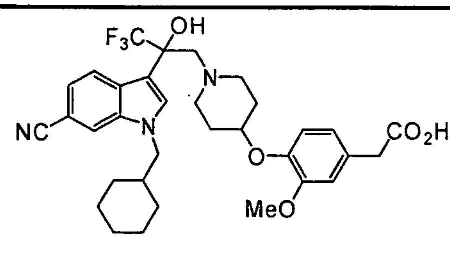 |
| 521 | 522 |
| 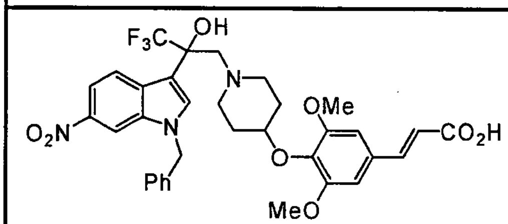 | 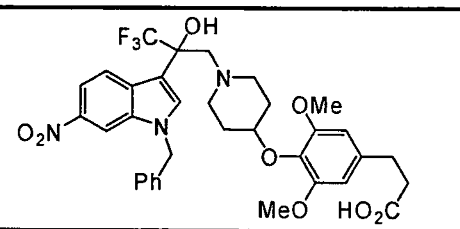 |
| 523 | 524 |
| 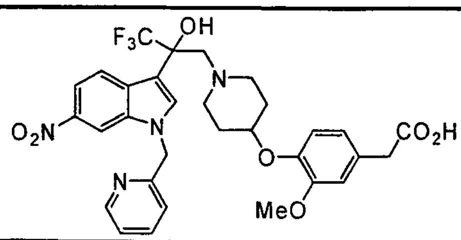 | 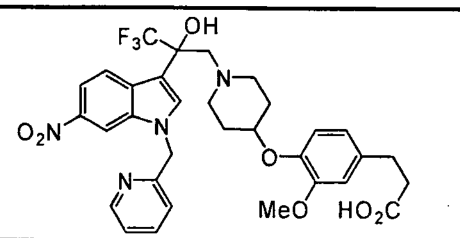 |

[Table 29]

| 525 | 526 |
|---|---|
| 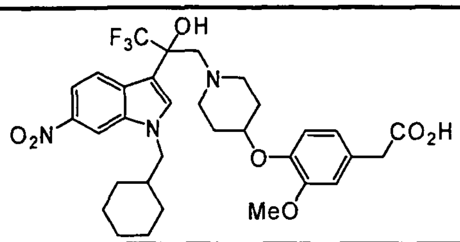 | 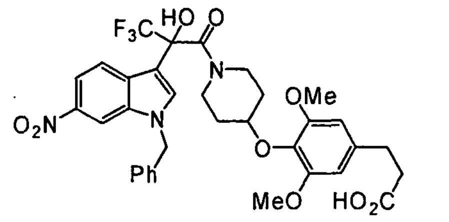 |
| 527 | 528 |
| 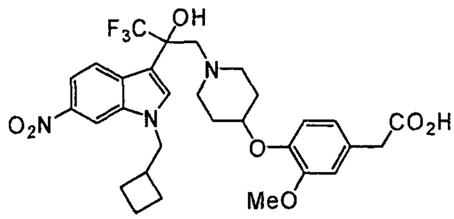 | 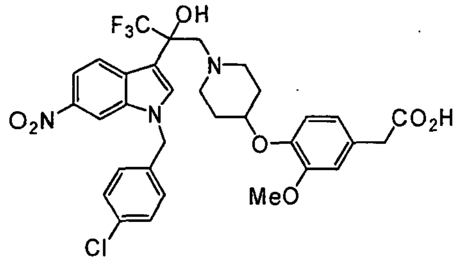 |
| 529 | 530 |
| 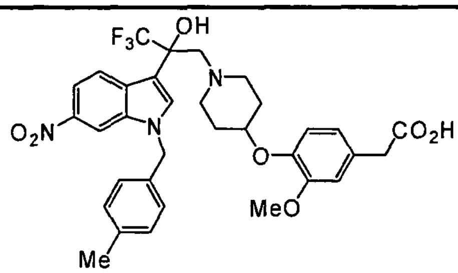 | 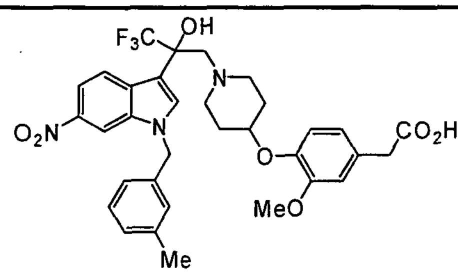 |
| 531 | 532 |
| 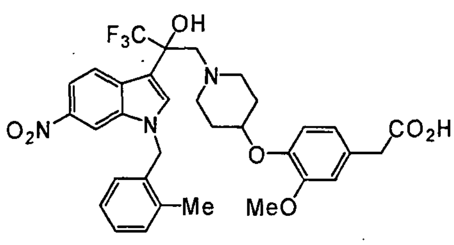 | 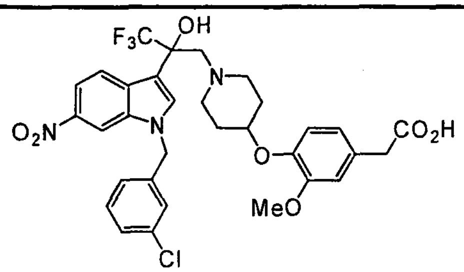 |
| 533 | 534 |
| 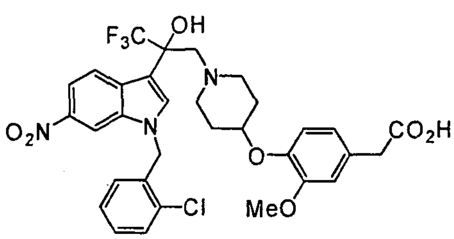 | 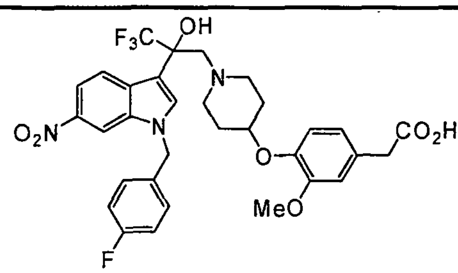 |

[Table 30]

| | |
|---|---|
| 535 | 536 |
| 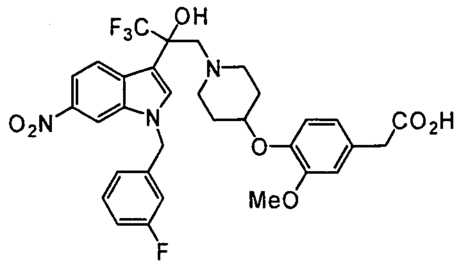 | 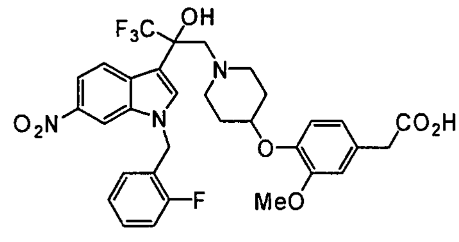 |
| 537 | 538 |
| 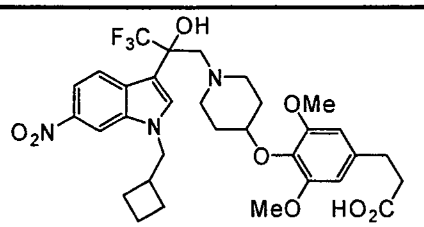 | 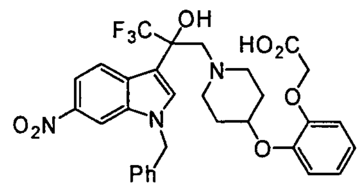 |
| 539 | 540 |
| 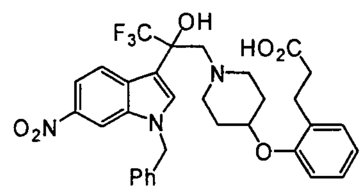 | 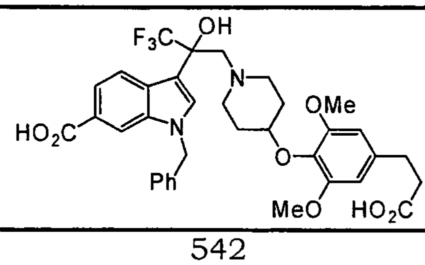 |
| 541 | 542 |
| 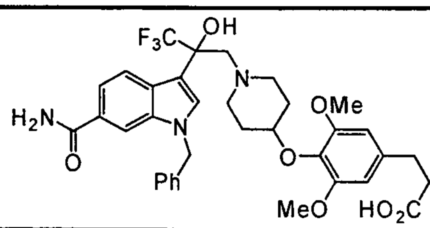 | 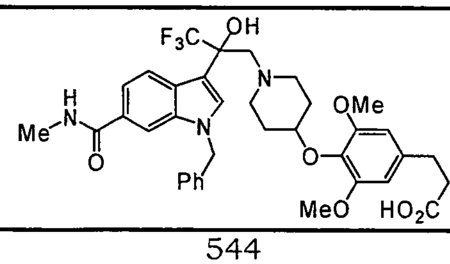 |
| 543 | 544 |
| 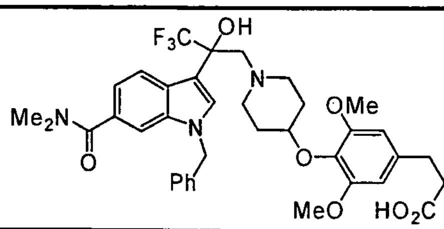 | 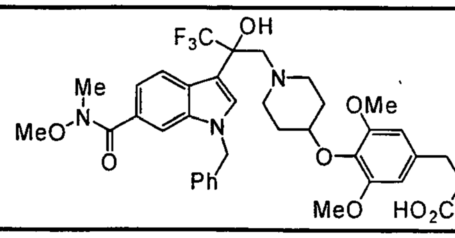 |

[Table 31]

| 545 | 546 |
|---|---|
| 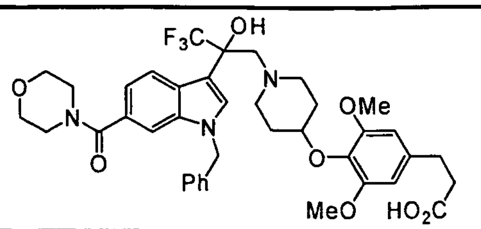 | 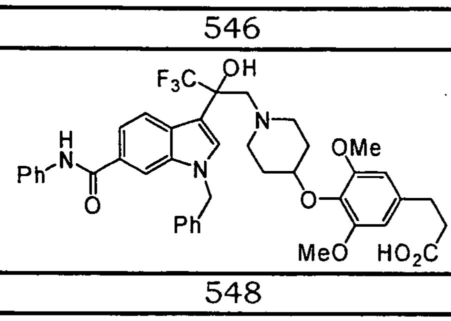 |
| **547** | **548** |
| 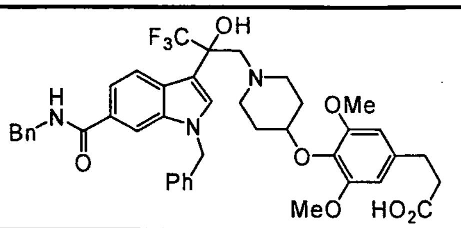 | 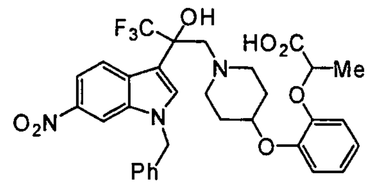 |
| **549** | **550** |
| 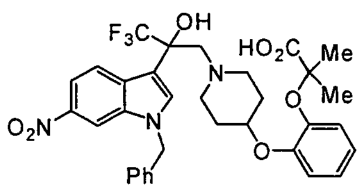 | 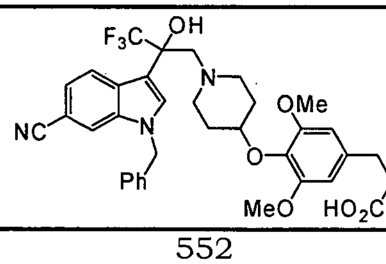 |
| **551** | **552** |
| 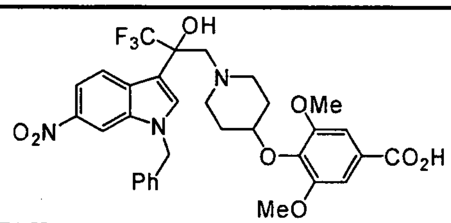 | 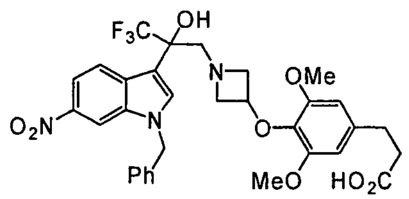 |
| **553** | **554** |
| 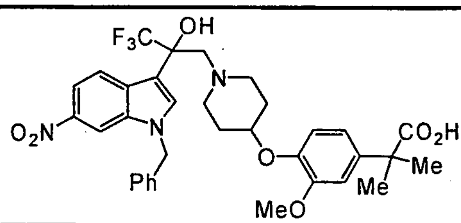 | 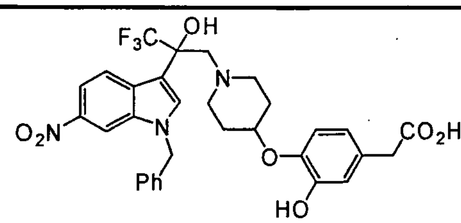 |

[Table 32]

| 555 | 556 |
|---|---|
| 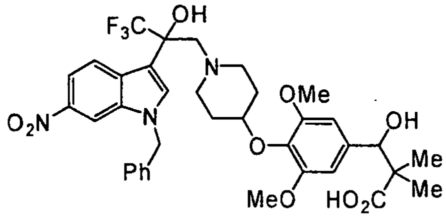 | 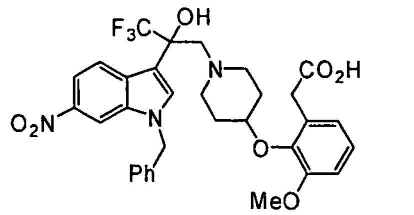 |
| 557 | 558 |
| 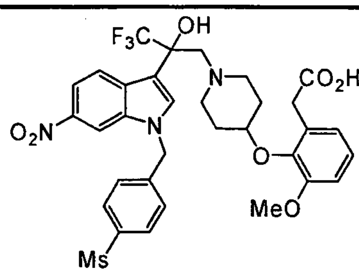 | 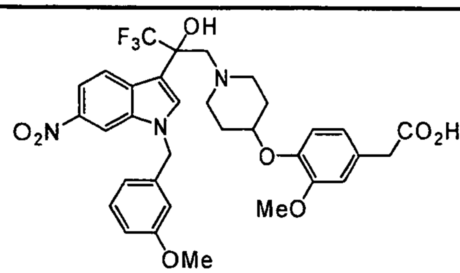 |
| 559 | 560 |
| 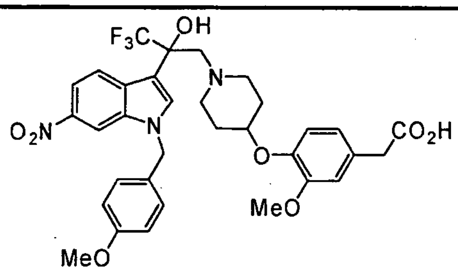 | 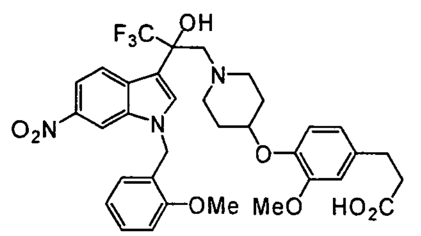 |
| 561 | 562 |
| 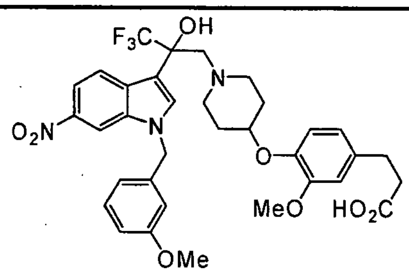 | 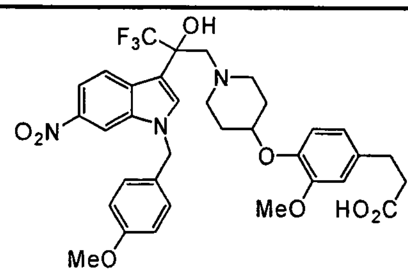 |
| 563 | 564 |
| 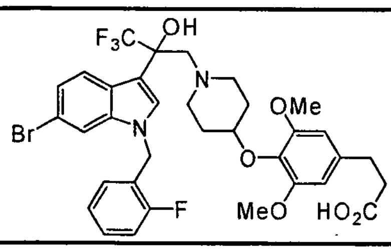 | 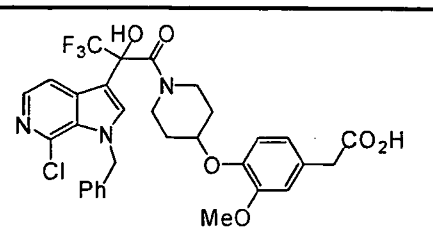 |

[Table 33]

| 565 | 566 |
|---|---|
| 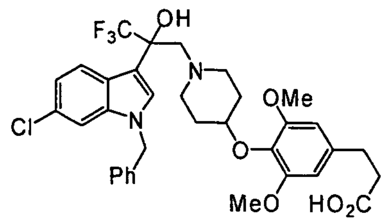 | 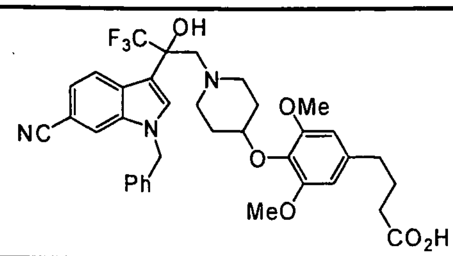 |
| 567 | 568 |
| 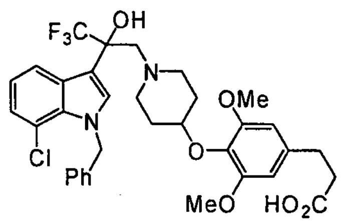 | 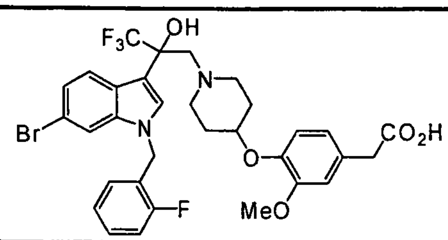 |
| 569 | 570 |
| 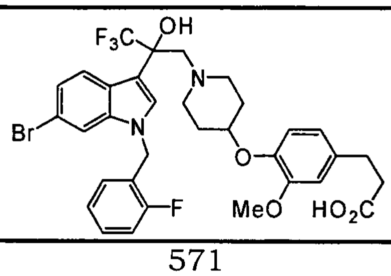 | 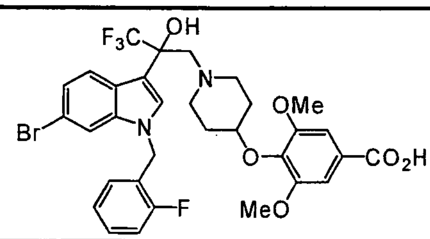 |
| 571 | 572 |
| 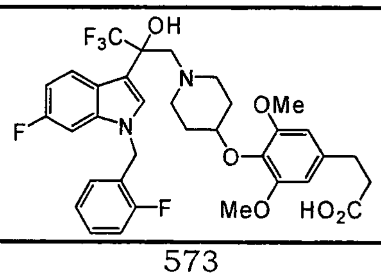 | 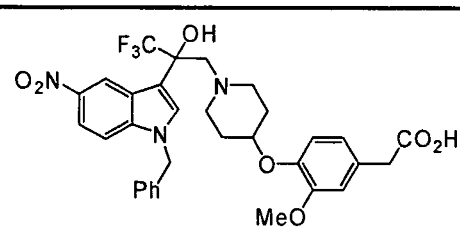 |
| 573 | 574 |
| 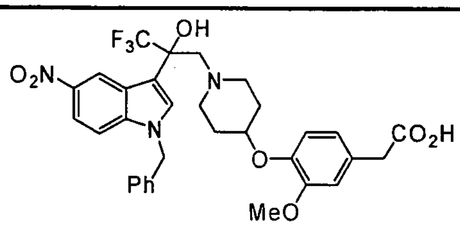 | 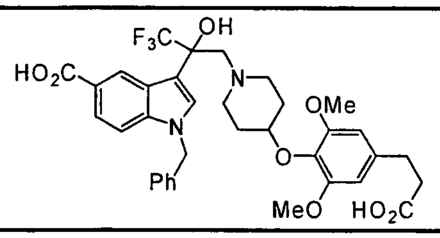 |

[Table 34]

| 575 | 576 |
|---|---|
| 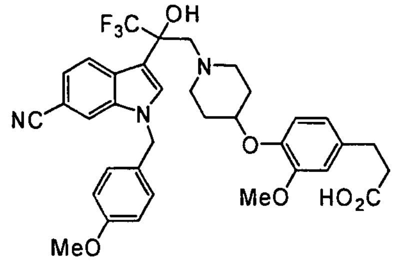 | 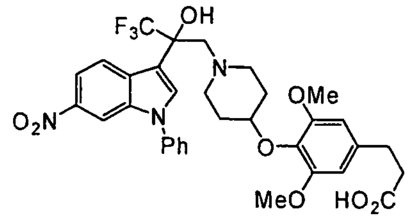 |
| 577 | 578 |
| 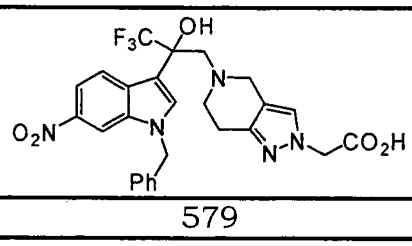 | 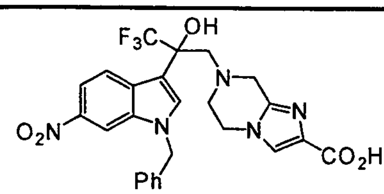 |
| 579 | 580 |
| 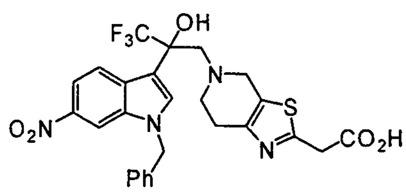 | 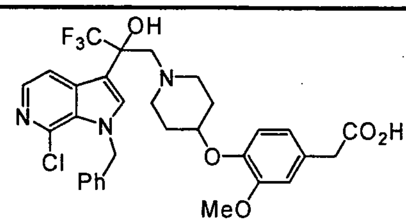 |
| 581 | 582 |
| 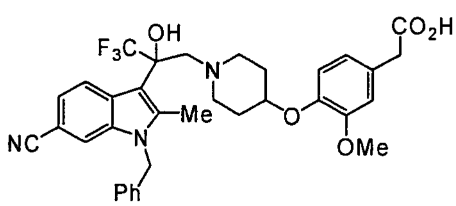 | 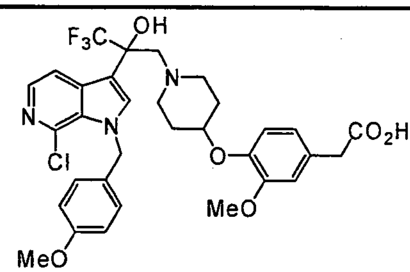 |
| 583 | 584 |
| 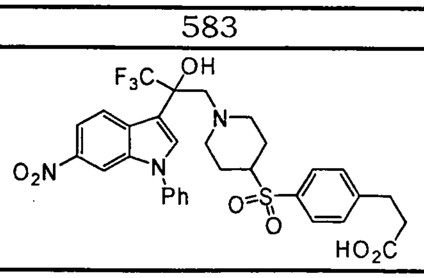 | 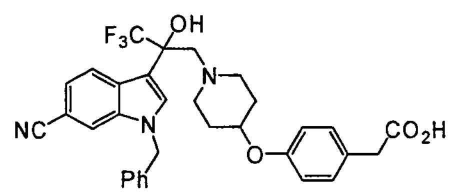 |

[Table 35]

| 585 | 586 |
|---|---|
| 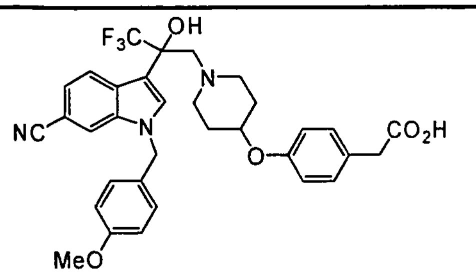 | 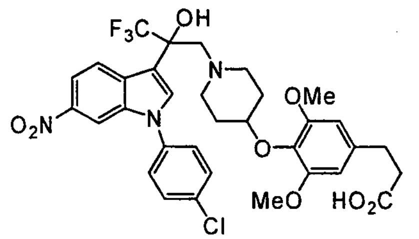 |
| 587 | 588 |
| 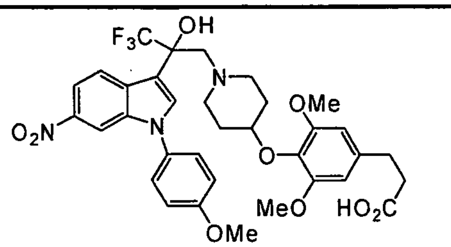 | 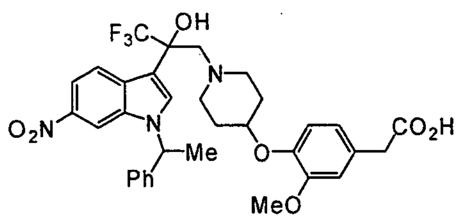 |
| 589 | 590 |
| 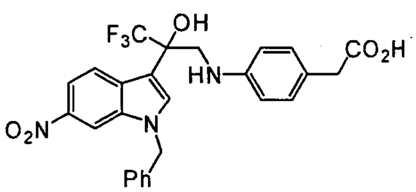 | 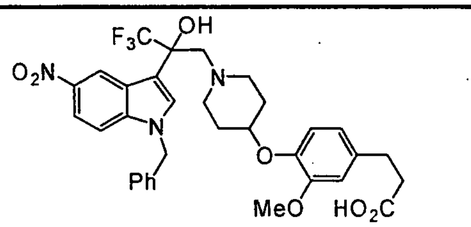 |
| 591 | 592 |
| 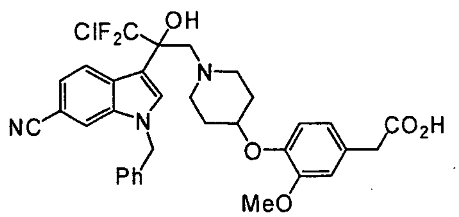 | 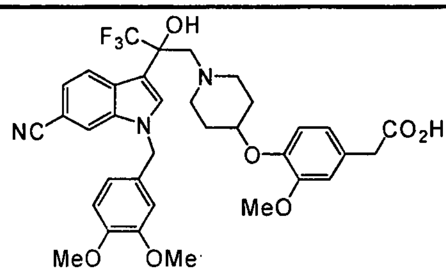 |
| 593 | 594 |
| 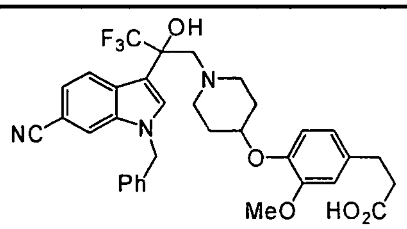 | 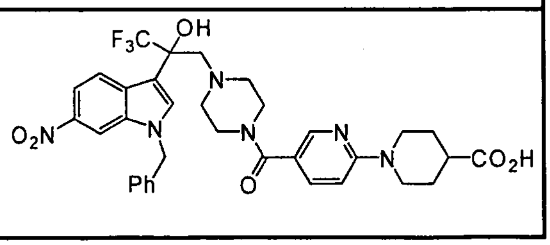 |

[Table 36]

| 595 | 596 |
|---|---|
| 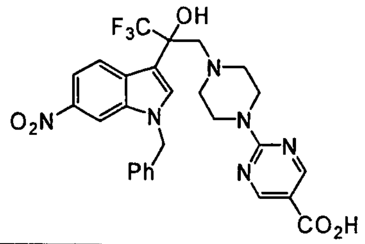 | 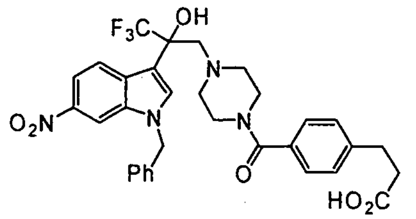 |
| 597 | 598 |
| 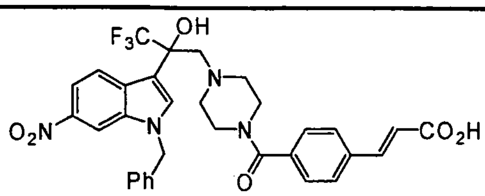 | 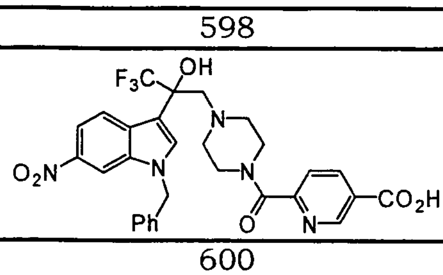 |
| 599 | 600 |
| 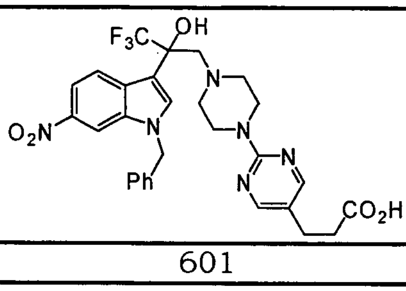 | 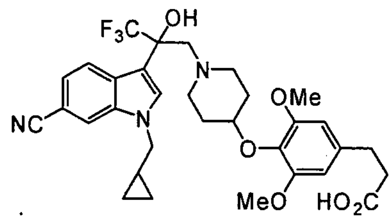 |
| 601 | 602 |
| 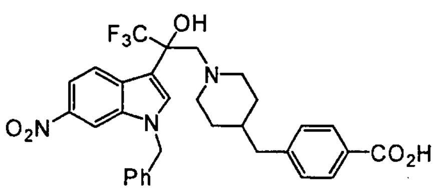 | 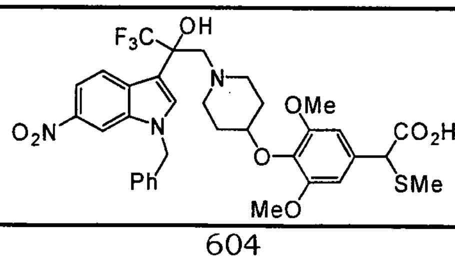 |
| 603 | 604 |
| 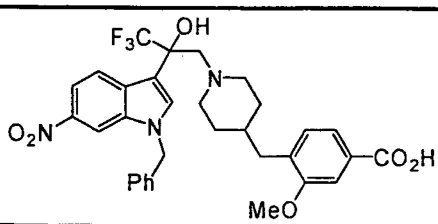 | 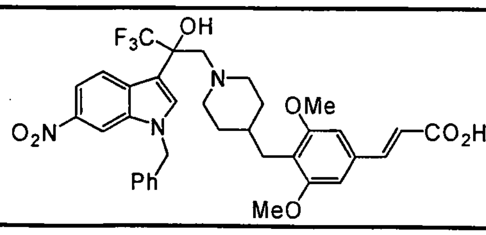 |

[Table 37]

| 605 | 606 |
|---|---|
| 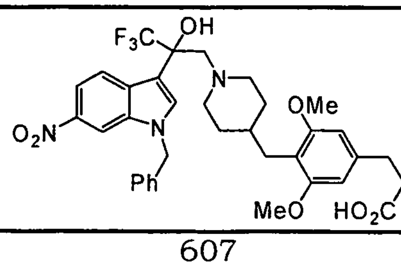 | 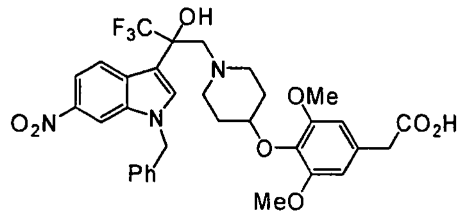 |
| 607 | 608 |
| 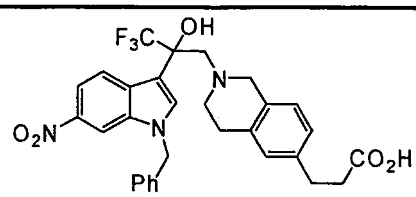 | 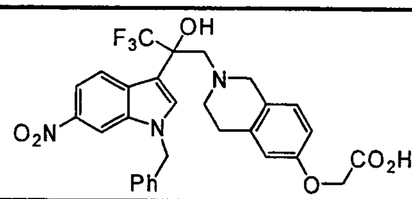 |
| 609 | 610 |
| 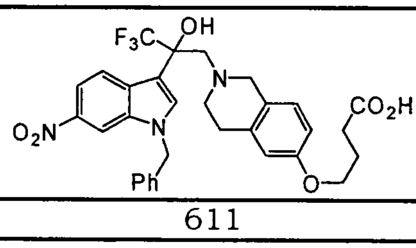 | 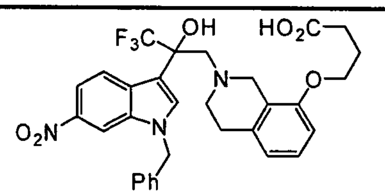 |
| 611 | 612 |
| 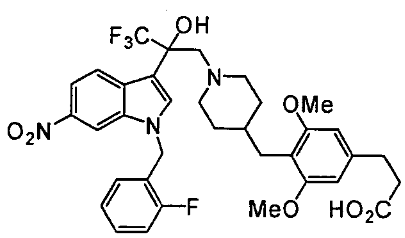 | 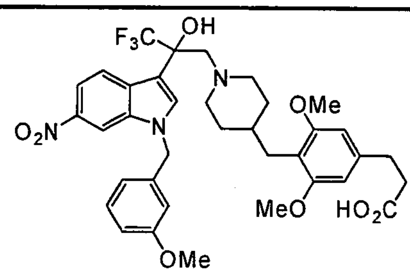 |
| 613 | 614 |
| 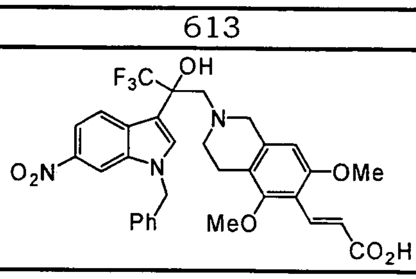 | 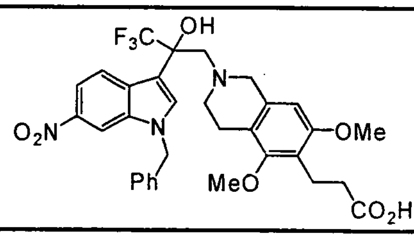 |

[Table 38]

| 615 | 616 |
|---|---|
| 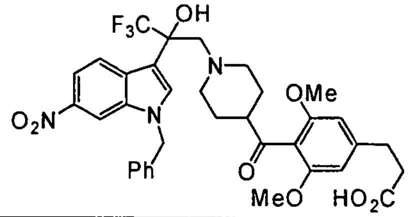 | 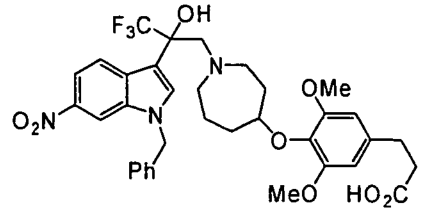 |
| 617 | 618 |
| 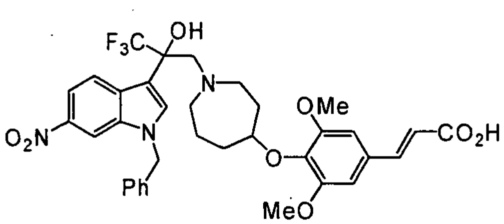 | 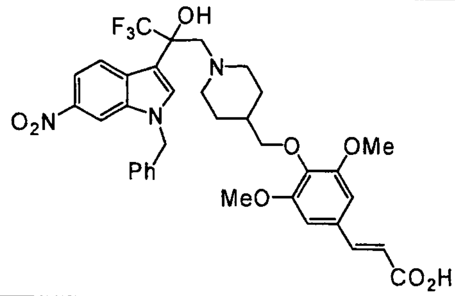 |
| 619 | 620 |
| 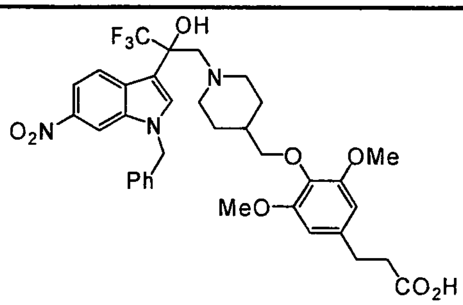 | 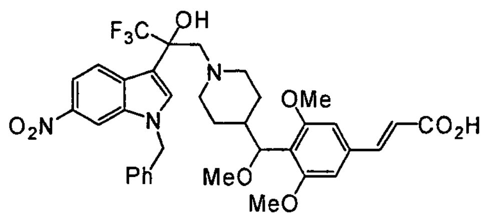 |
| 621 | 622 |
| 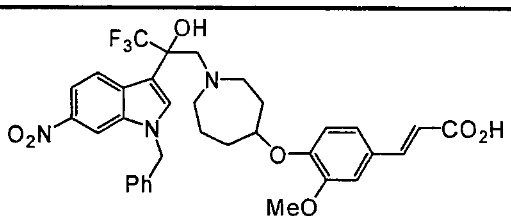 | 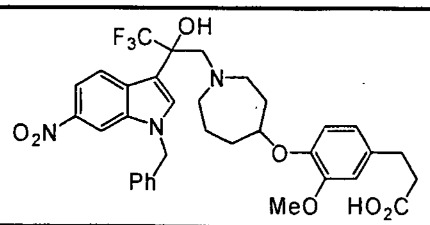 |
| 623 | 624 |
| 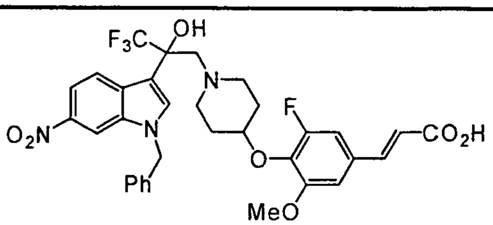 | 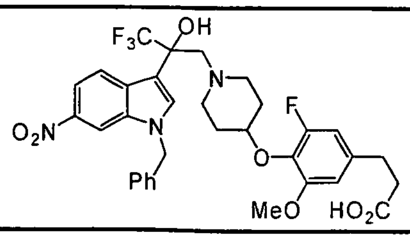 |

[Table 39]

| 625 | 626 |
|---|---|
| 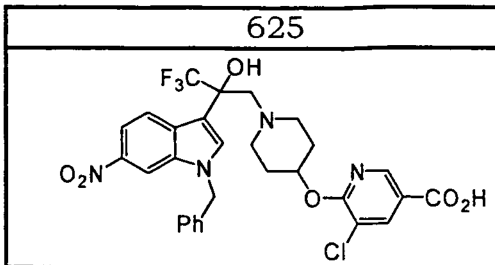 | 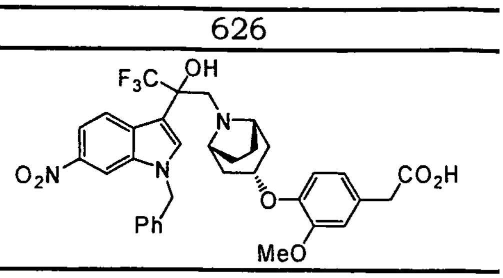 |
| 627 | |
| 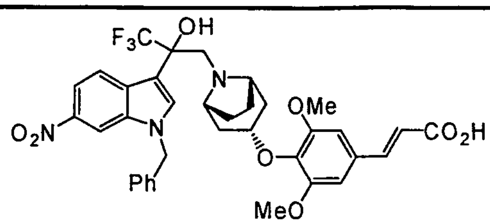 | |

Example 475

(2E)-3-[4-({1-[2-(1-Benzyl-6-nitro-1H-indol-3-yl)-3,3,3-trifluoro-2-hydroxypropyl]piperidin-4-yl}sulfonyl)phenyl]acrylic acid

MS (ESI+) 658, 2.53($M^+$+1, detection time)

Example 476

3-[4-({1-[2-(1-Benzyl-6-nitro-1H-indol-3-yl)-3,3,3-trifluoro-2-hydroxypropyl]-piperidin-4-yl}sulfonyl)phenyl]propanoic acid

MS (ESI+) 660, 2.25($M^+$+1, detection time)

Example 477

2-[4-({1-[2-(1-Benzyl-6-nitro-1H-indol-3-yl)-3,3,3-trifluoro-2-hydroxypropyl]-piperidin-4-yl}oxy)phenyl]-2-methylpropanoic acid

MS (ESI+) 626, 2.16($M^+$+1, detection time)

Example 478

{4-[(1-{2-[6-Cyano-1-(2-fluorobenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.94 (d, J= 8.4 Hz, 1H), 7.64, (bs, 1H), 7.59 (s, 1H), 7.42 (d, J= 8.4 Hz, 1H), 7.41 (d, J= 7.6 Hz, 1H), 7.26 (dd, J= 7.6, 7.6 Hz, 1H), 7.13 (dd, J= 7.6, 7.6 Hz, 1H), 6.79-6.74 (m, 4H), 5.44 (s, 2H), 4.43 (bs, 1H), 3.85 (d, J= 13.4 Hz, 1H), 3.75 (d, J= 13.4 Hz, 1H), 3.66 (s, 3H), 3.57 (s, 2H), 3.50-2.97 (m, 4H), 2.27-1.86 (m, 4H). MS (ESI+) 626, 1.94($M^+$+1, detection time)

Example 479

{4-[(1-{2-[1-(2-Chlorobenzyl)-6-cyano-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.91 (d, J= 8.3 Hz, 1H), 7.63 (s, 1H), 7.62 (s, 1H), 7.63-7.60 (m, 1H), 7.44-6.78 (m, 5H), 6.64 (d, J= 7.5 Hz, 1H), 6.53 (bs, 1H), 5.32 (s, 2H), 4.42 (s, 1H), 3.82 (d, J= 13.4 Hz, 1H), 3.73 (d, J= 13.4 Hz,

1H), 3.68 (s, 3H), 3.58 (s, 2H), 3.51-2.99 (m, 4H), 2.27-1.90 (m, 4H).
MS (ESI+) 643, 2.03($M^+$+2, detection time)

Example 480

{4-[(1-{2-[6-Cyano-1-(2-methoxybenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.88 (d, J= 8.4 Hz, 1H), 7.74 (s, 1H), 7.65 (s, 1H), 7.39 (d, J= 8.4 Hz, 1H), 7.29-7.25 (m, 1H), 6.90-6.77 (m, 6H), 5.33 (s, 2H), 4.42 (s, 1H), 3.87-3.80 (m, 4H), 3.75 (d, J= 13.3 Hz, 1H), 3.65 (s, 3H), 3.57 (s, 2H), 3.51-2.89 (m, 4H), 2.32-1.86 (m, 4H).
MS (ESI+) 638, 2.00($M^+$+1, detection time)

Example 481

{4-[(1-{2-[6-Cyano-1-(3-fluorobenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.87 (d, J= 8.0 Hz, 1H), 7.73 (s, 1H), 7.61 (s, 1H), 7.43 (d, J= 8.0 Hz, 1H), 7.33-7.23 (m, 1H), 7.03-6.94 (m, 1H), 6.88 (d, J= 8.0 Hz, 1H), 6.78 (s, 3H), 6.72 (d, J= 8.0 Hz, 1H), 5.38 (s, 2H), 4.45 (bs, 1H), 3.96-3.74 (m, 2H), 3.68 (s, 3H), 3.62-3.40 (m, 2H), 3.58 (s, 2H), 3.30-3.12 (m, 1H), 3.05-2.88 (m, 1H), 2.62-2.35 (m, 1H), 2.26-2.02 (m, 2H), 1.97-1.78 (m, 1H).
MS (ESI+) 626, 2.19($M^+$+1, detection time)

Example 482

{4-[(1-{2-[1-(3-Chlorobenzyl)-6-cyano-1H-indol-3-yl]-3,3,3-trifluoro-2-hydroxypropyl}piperidin-4-yl)oxy]-3-methoxyphenyl}acetic acid

$^1$H-NMR (400 MHz, $CD_3OD$) δ 7.88 (d, J= 8.4 Hz, 1H), 7.72 (s, 1H), 7.60 (s, 1H), 7.43 (d, J= 8.4 Hz, 1H), 7.29-7.20 (m, 2H), 7.01 (s, 1H), 6.96 (d, J= 7.3 Hz, 1H), 6.82-6.73 (m, 3H), 5.34 (s, 2H), 4.44 (bs, 1H), 3.89 (d, J= 13.4 Hz, 1H), 3.82 (d, J= 13.4 Hz, 1H), 3.66 (s, 3H), 3.62-3.41 (m, 2H), 3.57 (s, 2H), 3.30-3.16 (m, 1H), 3.05-2.87 (m, 1H), 2.53-2.32 (m, 1H), 2.22-2.00 (m, 2H), 1.95-1.80 (m, 1H).
MS (ESI+) 642, 2.21($M^+$+1, detection time)

Example 483

{4-[(1-{2-[6-Cyano-1-(3-methoxybenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.88 (d, J= 8.4 Hz, 1H), 7.63 (s, 1H), 7.62 (s, 1H), 7.41 (d, J= 8.4 Hz, 1H), 7.20 (dd, J= 8.2, 7.8 Hz, 1H), 6.80 (d, J= 8.2 Hz, 1H), 6.79-6.75 (m, 3H), 6.64 (d, J= 7.8 Hz, 1H), 6.53 (s, 1H), 5.34 (d, J= 16.3 Hz, 1H), 5.29 (d, J= 16.3 Hz, 1H), 4.42 (bs, 1H), 3.84 (d, J= 13.9 Hz, 1H), 3.76 (d, J= 13.9 Hz, 1H), 3.67 (s, 3H), 3.63 (s, 3H), 3.57 (s, 2H), 3.52-2.98 (m, 4H), 2.29-1.86 (m, 4H).
MS (ESI+) 638, 1.95($M^+$+1, detection time)

Example 484

(2E)-3-[4-({1-[2-(1-Benzyl-6-nitro-1H-indol-3-yl)-3,3,3-trifluoro-2-hydroxypropyl]piperidin-4-yl}oxy)-3-methoxyphenyl]acrylic acid

$^1$H-NMR (400 MHz, $CDCl_3$) δ 8.29 (d, J= 2.0 Hz, 1H), 8.06 (dd, J= 9.0, 2.0 Hz, 1H), 7.67 (d, J= 9.0 Hz, 1H), 7.67 (d, J= 15.2 Hz, 1H), 7.65 (s, 1H), 7.33-7.28 (m, 3H), 7.12-7.03 (m, 4H), 6.84 (d, J= 8.3 Hz, 1H), 6.31 (d, J= 15.2 Hz, 1H), 5.43 (d, J= 16.0 Hz, 1H), 5.38 (d, J= 16.0 Hz, 1H), 4.52 (bs, 1H), 3.79 (s, 3H), 3.75-3.48 (m, 3H), 3.24 (bs, 1H), 2.99-2.97 (m, 2H), 2.27-1.89 (m, 4H).
MS (ESI+) 640, 2.10($M^+$+1, detection time)

Example 485

3-[4-({1-[2-(1-Benzyl-6-nitro-1H-indol-3-yl)-3,3,3-trifluoro-2-hydroxypropyl]-piperidin-4-yl}oxy)-3-methoxyphenyl]propanoic acid

$^1$H-NMR (400 MHz, $CDCl_3$) δ 8.30 (d, J= 2.0 Hz, 1H), 8.75 (dd, J= 9.0, 2.0 Hz, 1H), 7.90 (d, J= 9.0 Hz, 1H), 7.68 (s, 1H), 7.32-7.28 (m, 3H), 7.11-7.09 (m, 2H), 6.75 (d, J= 7.9 Hz, 1H), 6.69 (s, 1H), 6.69 (d, J= 7.9 Hz, 1H), 5.44 (d, J= 16.0 Hz, 1H), 5.39 (d, J= 6.0 Hz, 1H), 4.40 (bs, 1H), 3.80 (d, J= 13.1 Hz, 1H), 3.71 (d, J= 13.1 Hz, 1H), 3.68 (s, 3H), 3.41-3.38 (m, 1H), 3.18-3.16 (m, 1H), 3.01 (bs, 1H), 2.89 (t, J= 7.6 Hz, 2H), 2.64 (t, J= 7.6 Hz, 2H), 2.27-2.24 (m, 1H), 2.11-2.05 (m, 2H), 1.89-1.85 (m, 1H).
MS (ESI+) 642, 2.10($M^+$+1, detection time)

Example 486

{4-[(1-{2-[6-Cyano-1-(2-methylbenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.91 (d, J= 8.4 Hz, 1H), 7.62 (s, 1H), 7.45 (s, 1H), 7.40 (d, J= 8.4 Hz, 1H), 7.25-7.18 (m, 2H), 7.14-7.05 (m, 1H), 6.84-6.73 (m, 3H), 6.69 (d, J= 7.6 Hz, 1H), 5.32 (s, 2H), 4.33 (bs, 1H), 3.73 (s, 3H), 3.65-3.32 (m, 2H), 3.57 (s, 2H), 3.32-3.00 (m, 2H), 3.00-2.80 (m, 2H), 2.25 (s, 3H), 2.15-1.90 (m, 2H), 1.90-1.77 (m, 2H).
MS (ESI+) 622, 2.05($M^+$+1, detection time)

Example 487

{4-[(1-{2-[6-Cyano-1-(3-methylbenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.88 (d, J= 8.3 Hz, 1H), 7.69 (s, 1H), 7.64 (s, 1H), 7.39 (d, J= 8.3 Hz, 1H), 7.15 (dd, J= 7.5, 7.5 Hz, 1H), 7.08 (d, J= 7.5 Hz, 1H), 6.91 (s, 1H), 6.84 (d, J= 7.5 Hz, 1H), 6.77 (s, 3H), 5.31 (s, 2H), 4.43 (bs, 1H), 3.89 (d, J= 13.1 Hz, 1H), 3.81 (d, J= 13.1 Hz, 1H), 3.65 (s, 3H), 3.64-3.40 (m, 2H), 3.56 (s, 2H), 3.31-3.15 (m, 1H), 3.03-2.85 (m, 1H), 2.50-2.31 (m, 1H), 2.27 (s, 3H), 2.20-2.00 (m, 2H), 1.94-1.77 (m, 1H).
MS (ESI+) 622, 2.17($M^+$+1, detection time)

Example 488

{4-[(1-{2-[6-Cyano-1-(2-thienylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.89 (d, J= 8.2 Hz, 1H), 7.74 (s, 1H), 7.69 (s, 1H), 7.42 (d, J= 8.2 Hz, 1H), 7.12 (d, J= 4.8 Hz, 1H), 6.99 (d, J= 2.6 Hz, 1H), 6.94-6.88 (m, 1H), 6.77 (s, 3H), 5.51 (s, 2H), 4.45 (bs, 1H), 3.90 (d, J= 12.6 Hz, 1H), 3.78 (d, J= 12.6 Hz, 1H), 3.63 (s, 3H), 3.57 (s, 2H), 3.55-3.40 (m, 2H), 3.32-3.18 (m, 1H), 2.95-2.79 (m, 1H), 2.50-2.30 (m, 1H), 2.19-2.00 (m, 2H), 1.90-1.75 (m, 1H).
MS (ESI+) 614, 1.98($M^+$+1, detection time)

Example 489

{4-[(1-{2-[6-Cyano-1-(3-thienylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.87 (d, J= 8.3 Hz, 1H), 7.69 (s, 2H), 7.41 (d, J= 8.3 Hz, 1H), 7.29-7.24 (m, 1H), 7.08 (bs, 1H), 6.86 (d, J= 4.9 Hz, 1H), 6.82-6.75 (m, 3H), 5.36 (s, 2H), 4.45 (bs, 1H), 3.87 (d, J= 13.3 Hz, 1H), 3.79 (d, J= 13.3 Hz, 1H), 3.68 (s, 3H), 3.57 (s, 2H), 3.55-3.41 (m, 2H), 3.25-3.13 (m, 1H), 3.04-2.85 (m, 1H), 2.54-2.32 (m, 1H), 2.21-2.02 (m, 2H), 1.94-1.78 (m, 1H).
MS (ESI+) 614, 1.99($M^+$+ 1, detection time)

Example 490

4-(2-{[2-(1-Benzyl-6-nitro-1H-indol-3-yl)-3,3,3-trifluoro-2-hydroxypropyl]amino}-2-oxoethoxy)benzoic acid

$^1$H-NMR (400 MHz, $CDCl_3$) δ 8.23 (d, J= 2.0 Hz, 1H), 7.92 (d, J= 9.0 Hz, 1H), 7.82 (dd, J= 9.0, 2.0 Hz, 1H), 7.66 (d, J= 8.9 Hz, 2H), 7.64 (s, 1H), 7.19-7.07 (m, 5H), 6.61 (d, J= 8.9 Hz, 2H), 5.37 (s, 2H), 4.37 (d, J= 15.2 Hz, 1H), 4.26 (d, J= 15.2 Hz, 1H), 4.23 (d, J= 14.1 Hz, 1H), 3.76 (d, J= 14.1 Hz, 1H).
MS (ESI+) 558, 2,25 ($M^+$+1, detection time)

Example 491

[4-({1-(3-(1-Benzyl-6-nitro-1H-indol-3-yl)-4,4,4-trifluoro-3-hydroxybutanoyl]-piperidin-4-yl}oxy)-3-methoxyphenyl]acetic acid

$^1$H-NMR (400 MHz, $CDCl_3$) δ 8.28 (s, 1H), 8.00 (d, J= 9.0 Hz, 1H), 7.88 (dd, J= 9.0, 2.0 Hz, 1H), 7.70 (s, 1H), 7.24-7.09 (m, 5H), 6.86-6.70 (m, 3H), 5.46 (d, J= 14.9 Hz, 1H), 5.42 (d, J= 14.9 Hz, 1H), 4.73-4.67 (m, 1H), 3.72 (s, 3H), 3.67 (s, 2H), 3.59-3.26 (m, 6H), 1.75-1.26 (m, 4H).
MS (ESI+) 656, 2.45($M^+$+1, detection time)

Example 492

[4-(2-{[2-(1-Benzyl-6-nitro-1H-indol-3-yl)-3,3,3-trifluoro-2-hydroxypropyl]-amino}-2-oxoethoxy)-3-methoxyphenyl]acetic acid

$^1$H-NMR (400 MHz, $CDCl_3$) δ 8.31 (s, 1H), 8.05 (d, J= 9.1 Hz, 1H), 7.93 (d, J= 9.1 Hz, 1H), 7.72 (s, 1H), 7.22-7.20 (m, 3H), 7.14 (s, 2H), 6.87 (s, 1H), 6.66 (s, 2H), 5.47 (s, 2H), 4.38 (d, J= 15.5 Hz, 1H), 4.33 (d, J= 14.2 Hz, 1H), 4.22 (d, J= 15.5 Hz, 1H), 3.85 (d, J= 14.2 Hz, 1H), 3.64 (s, 3H), 3.42 (s, 2H).
MS (ESI+) 602, 2.26($M^+$+1, detection time)

Example 493

3-[4-(2-{[2-(1-Benzyl-6-nitro-1H-indol-3-yl)-3,3,3-trifluoro-2-hydroxypropyl]-amino}-2-oxoethoxy)-3-methoxyphenyl] propanoic acid

$^1$H-NMR (400 MHz, $CDCl_3$) δ 8.32 (d, J= 2.0 Hz, 1H), 8.05 (d, J= 9.0 Hz, 1H), 7.89 (dd, J= 9.0, 2.0 Hz, 1H), 7.21-7.11 (m, 5H), 6.76 (d, J= 1.8 Hz, 1H), 6.48 (d, J= 8.1 Hz, 1H), 6.55 (dd, J= 8.1, 1.8 Hz, 1H), 5.43 (s, 2H), 4.37 (d, J= 15.6 Hz, 1H), 4.33 (d, J= 15.0 Hz, 1H), 4.19 (d, J= 15.6 Hz, 1H), 3.85 (d, J= 15.0 Hz, 1H), 3.61 (s, 3H), 2.79 (t, J= 7.6 Hz, 2H), 2.50 (t, J= 7.6 Hz, 2H).
MS (ESI+) 616, 2.33($M^+$+1, detection time)

Example 494

{4-[(1-{2-[6-Cyano-1-(3-furylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.27 (d, J= 8.4 Hz, 1H), 8.05 (s, 1H), 7.62 (s, 1H), 7.53 (dd, J= 8.4, 1.2 Hz, 1H), 7.43-7.33 (m, 5H), 6.29 (s, 1H), 5.23 (s, 2H), 4.42 (bs, 1H), 3.83-3.65 (m, 2H), 3.72 (s, 3H), 3.59 (s, 2H), 3.45-3.30 (m, 2H), 3.15-2.87 (m, 2H), 2.40-2.18 (m, 1H), 2.18-1.97 (m, 2H), 1.95-1.78 (m, 1H).
MS (ESI+) 598, 1.94($M^+$+1, detection time)

Example 495

{4-[(1-{2-[6-Cyano-1-(2-morpholin-4-yl-2-oxoethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CD_3OD$) δ 7.98 (dd, J= 8.2, 4.4 Hz, 1H), 7.86 (d, J= 14.0, 1H), 7.68 (d, J= 14.0 Hz, 1H), 7.37-7.10 (m, 1H), 6.87-6.80 (m, 2H), 6.69 (d, J= 8.1 Hz, 1H), 5.27 (s, 1H), 4.89 (s, 2H), 4.38-4.29 (m, 1H), 3.98 (d, J= 14.0 Hz, 1H), 3.75-3.63 (m, 2H), 3.71 (s, 3H), 3.63-3.53 (m, 2H), 3.53-3.45 (m, 1H), 3.45-3.30 (m, 2H), 3.44 (s, 2H),

3.30-3.05 (m, 4H), 2.04-1.78 (m, 4H).
MS (ESI+) 645, 1.82($M^+$+1, detection time)

Example 496

{4-[(1-{2-[6-Cyano-1-(2-oxo-2-piperidin-1-ylethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.92 (d, J= 8.2 Hz, 1H), 7.58 (s, 1H), 7.56 (s, 1H), 7.39 (d, J= 8.2 Hz, 1H), 6.82-6.70 (m, 3H), 4.95 (s, 2H), 4.34-4.22 (m, 1H), 3.74 (s, 3H), 3.55 (s, 2H), 3.52-3.40 (m, 2H), 3.30-3.05 (m, 2H), 3.05-2.84 (m, 4H), 2.03-1.78 (m, 6H), 1.73-1.50 (m, 6H).
MS (ESI+) 643, 1.92($M^+$+1, detection time)

Example 497

(4-{[1-(2-{6-Cyano-1-[2-(dimethylamino)-2-oxoethyl]-1H-indol-3-yl}-3,3,3-trifluoro-2-hydroxypropyl)piperidin-4-yl]oxy}-3-methoxyphenyl)acetic acid

$^1$H-NMR (400 MHz, $CDCl_3$) δ 7.91 (d, J= 8.9 Hz, 1H), 7.61 (s, 1H), 7.59 (s, 1H), 7.42 (d, J= 8.9 Hz, 1H), 6.83-6.70 (m, 3H), 4.97 (d, J= 3.6 Hz, 2H), 4.41-4.32 (m, 1H), 3.81 (d, J= 14.0 Hz, 1H), 3.77-3.35 (m, 5H), 3.72 (s, 3H), 3.58 (s, 2H), 3.17 (s, 3H), 2.99 (s, 3H), 2.13-1.95 (m, 4H).
MS (ESI+) 603, 1.82($M^+$+1, detection time)

Example 498

{4-[(1-{2-[1-(2-Amino-2-oxoethyl)-6-cyano-1H-indol-3-yl]-3,3,3-trifluoro-2-hydroxypropyl}piperidin-4-yl)oxy]-3-methoxyphenyl}acetic acid

$^1$H-NMR (400 MHz, $CD_3OD$) δ 7.97 (dd, J= 8.4, 3.5 Hz, 1H), 7.85 (d, J= 14.0 Hz, 1H), 7.73 (d, J= 3.5 Hz, 1H), 7.40-7.33 (m, 1H), 6.87-6.80 (m, 2H), 6.69 (d, J= 8.1 Hz, 1H), 4.99 (d, J= 6.7 Hz, 2H), 4.40-4.30 (m, 1H), 4.06 (d, J= 14.0 Hz, 1H), 3.76 (d, J= 14.0 Hz, 1H), 3.69 (s, 3H), 3.55-3.35 (m, 2H), 3.45 (s, 2H), 3.30-3.10 (m, 2H), 2.05-1.80 (m, 4H).
MS (ESI+) 575, 1.75($M^+$+1, detection time)

Example 499

3-(4-{4-[3-(1-Benzyl-6-nitro-1H-indol-3-yl)-4,4, 4-trifluoro-3-hydroxybutanoyl]-piperazin-1-yl}phenyl)propanoic acid

$^1$H-NMR (400 MHz, $CDCl_3$) δ 8.35 (d, J= 1.4 Hz, 1H), 8.10 (d, J= 9.1 Hz, 1H), 7.97 (dd, J= 9.1, 1.4 Hz, 1H), 7.33-7.15 (m, 5H), 7.09 (d, J= 8.4 Hz, 2H), 6.76 (d, J= 8.4 Hz, 2H), 5.52 (d, J= 15.8 Hz, 1H), 5.45 (d, J= 15.8 Hz, 1H), 3.65-3.59 (m, 2H), 3.56 (d, J= 15.4 Hz, 2H), 3.49-3.45 (m, 2H), 3.10 (d, J= 15.4 Hz, 2H), 3.03-2.96 (m, 2H), 2.82 (t, J= 7.6 Hz, 2H), 2.65-2.56 (m, 2H), 2.53 (t, J= 7.6 Hz, 2H). MS (ESI+) 625, 2.46($M^+$+1, detection time)

Example 500

3-(4-{4-[2-(1-Benzyl-6-nitro-1H-indol-3-yl)-3,3,3-trifluoro-2-hydroxypropyl]-piperazin-1-yl}phenyl)propanoic acid

$^1$H-NMR (400 MHz, $CDCl_3$) δ 8.16 (d, J= 2.0 Hz, 1H), 7.84 (d, J= 9.0 Hz, 1H), 7.76 (dd, J= 9.2, 2.0 Hz, 1H), 7.63 (s, 1H), 7.11-7.01 (m, 3H), 6.99 (d, J= 8.2 Hz, 2H), 6.87 (d, J= 8.6 Hz, 2H), 6.60 (d, J= 8.6 Hz, 2H), 5.35 (d, J= 15.1 Hz, 1H), 5.31 (d, J= 15.1 Hz, 1H), 3.12-3.05 (m, 1H), 2.90 (d, J= 13.8 Hz, 1H), 2.72 (bt, J= 4.9 Hz, 4H), 2.59 (t, J= 7.7 Hz, 2H), 2.50-2.45 (m, 2H), 2.41-2.36 (m, 2H), 2.30 (t, J= 7.7 Hz, 2H).
MS (ESI+) 597, 2.29($M^+$+1, detection time)

Example 501

3-{4-[(1-{2-[6-Cyano-1-(pyridin-2-ylmethyl)-1H-indol-3-yl]-3,3,3-trifluoro-2-hydroxypropyl}piperidin-4-yl)oxy]-3-methoxyphenyl}propanoic acid

$^1$H-NMR (400 MHz, $CD_3OD$) δ 8.53 (d, J= 4.7 Hz, 1H), 8.02 (d, J= 8.4 Hz, 1H), 7.80 (s, 1H), 7.76 (s, 1H), 7.70

(ddd, J= 7.8, 7.3, 1.7 Hz, 1H), 7.35 (dd, J= 8.4, 1.3 Hz, 1H), 7.29 (dd, J= 7.3, 4.7 Hz, 1H), 6.97 (d, J= 7.8 Hz, 1H), 6.81 (d, J= 7.9 Hz, 1H), 6.81 (bs, 1H), 6.70 (dd, J= 7.9, 1.8 Hz, 1H), 5.55 (s, 2H), 4.18-4.16 (m, 1H), 3.89 (s, 3H), 3.27 (d, J= 13.8 Hz, 1H), 3.18 (d, J= 13.8 Hz, 1H), 2.85 (t, J= 7.6 Hz, 2H), 2.83-2.76 (m, 2H), 2.56 (t, J= 7.6 Hz, 2H), 2.48-2.44 (m, 2H), 1.82 (bs, 2H), 1.72-1.66 (m, 2H).
MS (ESI+) 623, 1.95($M^+$+1, detection time)

Example 502

4-({4-[2-(1-Benzyl-6-nitro-1H-indol-3-yl)-3,3,3-trifluoro-2-hydroxypropyl]-2-oxopiperazin-1-yl}methyl)-3-methoxybenzoic acid

MS (ESI+) 627, 2.29($M^+$+1, detection time)

Example 503

N-[4-({1-[2-(1-Benzyl-6-nitro-1H-indol-3-yl)-3,3,3-trifluoro-2-hydroxypropyl]-piperidin-4-yl}oxy)-3-methoxybenzoyl]-N-methylglycine .

$^1$H-NMR (400 MHz, $CDCl_3$) δ 8.28 (s, 1H), 8.10-8.00 (m, 1H), 7.89 (d, J= 9.0 Hz, 1H), 7.37-7.27 (m, 4H), 7.16-7.08 (m, 2H), 7.05-6.93 (m, 1H), 6.88-6.76-(m, 1H), 5.41 (s, 2H), 4.23 (bs, 1H), 3.78-3.50 (m, 4H), 3.40-3.12 (m, 2H), 3.09 (s, 3H), 3.06-2.90 (m, 2H), 2.38-1.80 (m, 7H).
MS (ESI+) 685, 2.02($M^+$+1, detection time)

Example 504

N-[4-({1-[2-(1-Benzyl-6-nitro-1H-indol-3-yl)-3,3,3-trifluoro-2-hydroxypropyl]-piperidin-4-yl}oxy)-3-methoxybenzoyl]glycine

$^1$H-NMR (400 MHz, $CDCl_3$) δ 8.27 (d, J= 1.9 Hz, 1H), 8.40 (dd, J= 9.0, 1.9 Hz, 1H), 7.90 (d, J= 9.0 Hz, 1H), 7.61 (s, 1H), 7.42 (d, J= 1.8 Hz, 1H), 7.36-7.28 (m, 3H), 7.14-7.06 (m, 2H), 6.88-6.81 (m, 1H), 6.66-6.60 (m, 1H), 5.40 (s, 2H), 4.43 (bs, 1H), 4.21 (d, J= 4.9 Hz, 2H), 3.85 (s, 3H), 3.53-3.25 (m, 2H), 3.14-2.82 (m, 2H), 2.82-2.60 (m, 2H), 2.20-1.40 (m, 4H).
MS (ESI+) 671, 2.05($M^+$+1, detection time)

Example 505

N-[4-({1-[2-(1-Benzyl-6-nitro-1H-indol-3-yl)-3,3,3-trifluoro-2-hydroxypropyl]-piperidin-4-yl}oxy)-3-methoxybenzoyl]-β-alanine

$^1$H-NMR (400 MHz, $CDCl_3$) δ 8.27 (d, J= 1.6 Hz, 1H), 8.04 (dd, J= 9.1, 1.6 Hz, 1H), 7.89 (d, J= 9.1 Hz, 1H), 7.63 (s, 1H), 7.40 (d, J= 1.6 Hz, 1H), 7.37-7.28 (m, 2H), 7.18 (dd, J= 8.2, 1.6 Hz, 1H), 7.15-7.06 (m, 2H), 6.85-6.72 (m, 2H), 5.40 (s, 2H), 4.43 (bs, 1H), 3.83 (s, 3H), 3.75-3.65 (m, 2H), 3.58-3.30 (m, 2H), 3.13-2.60 (m, 6H), 2.40-1.50 (m, 4H).
MS (ESI+) 685, 2.03($M^+$+1, detection time)

Example 506

1-[4-({1-[2-(1-Benzyl-6-nitro-1H-indol-3-yl)-3,3,3-trifluoro-2-hydroxypropyl]-piperidin-4-yl}oxy)-3-methoxybenzoyl]piperidine-4-carboxylic acid

$^1$H-NMR (400 MHz, $CDCl_3$) δ 8.29 (d, J= 1.9 Hz, 1H), 8.05 (dd, J= 9.0, 1.9 Hz, 1H), 7.89 (d, J= 9.0 Hz, 1H), 7.68 (s, 1H), 7.36-7.27 (m, 3H), 7.15-7.06 (m, 2H), 6.96 (d, J= 1.9 Hz, 1H), 6.89 (dd, J= 8.2, 1.9 Hz, 1H), 6.83 (d, J= 8.2 Hz, 1H), 5.41 (s, 2H), 4.53 (bs, 1H), 3.77 (s, 3H), 3.70-3.40 (m, 2H), 3.30-2.80 (m, 6H), 2.68-2.55 (m, 2H), 2.30-2.15 (m, 1H), 2.15-1.80 (m, 6H), 1.80-1.60 (m, 2H).
MS (ESI+) 725, 2.02($M^+$+1, detection time)

Example 507

1-[4-({1-[2-(1-Benzyl-6-nitro-1H-indol-3-yl)-3,3,3-trifluoro-2-hydroxypropyl]-piperidin-4-yl}oxy)-3-methoxybenzoyl]piperidine-3-carboxylic acid

$^1$H-NMR (400 MHz, $CDCl_3$) δ 8.28 (d, J= 1.8 Hz, 1H), 8.05 (dd, J= 9.0, 1.8 Hz, 1H), 7.89 (d, J= 9.0 Hz, 1H), 7.68 (s, 1H), 7.36-7.27 (m, 3H), 7.15-7.07 (m, 2H), 6.97 (d, J= 1.7 Hz, 1H), 6.90 (dd, J= 8.1, 1.7 Hz, 1H), 6.82 (d, J= 8.1 Hz, 1H), 5.41 (s, 2H), 4.43 (bs, 1H), 3.78 (s, 3H), 3.70-3.40 (m, 2H), 3.35-3.22 (m, 2H), 3.22-3.00 (m, 2H), 3.00-2.80 (m, 2H), 2.80-1.40 (m, 11H).
MS (ESI+) 725, 2.09($M^+$+1, detection time)

Example 508

1-[4-({1-[2-(1-Benzyl-6-nitro-1H-indol-3-yl)-3,3,3-trifluoro-2-hydroxypropyl]-piperidin-4-yl}oxy)-3-methoxybenzoyl]piperidine-2-carboxylic acid

$^1$H-NMR (400 MHz, $CDCl_3$) δ 8.28 (d, J= 1.9 Hz, 1H), 8.04 (d, J= 8.9, 1.9 Hz, 1H), 7.90 (d, J= 8.9 Hz, 1H), 7.73 (s, 1H), 7.35-7.27 (m, 3H), 7.12 (dd, J= 7.7, 2.3 Hz, 2H), 7.08-6.75 (m, 3H), 5.41 (s, 2H), 5.35 (bs, 1H), 3.85-3.55 (m, 5H), 3.35-3.09 (m, 2H), 3.09-2.88 (m, 2H), 2.42-1.35 (m, 13H).
MS (ESI+) 725, 2.13($M^+$+1, detection time)

Example 509

1-{[4-({1-[2-(1-Benzyl-6-nitro-1H-indol-3-yl)-3,3,3-trifluoro-2-hydroxypropyl]-piperidin-4-yl}oxy)-3-methoxybenzoyl]amino}cyclobutanecarboxylic acid

$^1$H-NMR (400 MHz, $CDCl_3$) δ 8.27 (d, J= 2.0 Hz, 1H), 8.04 (dd, J= 9.0, 2.0 1H), 7.90 (d, J= 9.0 Hz, 1H), 7.62 (s, 1H), 7.41 (d, J= 2.0 Hz, 1), 7.37-7.27 (m, 3H), 7.14-7.06 (m, 2H), 6.88-6.78 (m, 2H), 5.40 (s, 2H), 4.44 (bs, 1H), 3.86 (s, 3H), 3.55-3.20 (m, 2H), 3.10-1.50 (m, 14H).
MS (ESI+) 711, 2.10($M^+$+1, detection time)

Example 510.

N-[4-({1-[2-(1-Benzyl-6-nitro-1H-indol-3-yl)-3,3,3-trifluoro-2-hydroxypropyl]-piperidin-4-yl}oxy)-3-methoxybenzoyl]valine

MS (ESI+) 713, 2.13($M^+$+1, detection time)

Example 511

3-[4-({1-[2-(1-Benzyl-6-nitro-1H-indol-3-yl)-3,3,3-trifluoro-2-hydroxypropyl]-piperidin-4-yl}thio)-3-methoxyphenyl]propanoic acid

MS (ESI+) 658, 2.18($M^+$+1, detection time)

Example 512

(2E)-3-[4-({1-[2-(1-Benzyl-6-nitro-1H-indol-3-yl)-3,3,3-trifluoro-2-hydroxypropyl]piperidin-4-yl}thio)-3-methoxyphenyl] acrylic acid

H-NMR (400 MHz, $CD_3OD$) δ 8.36 (s, 1H), 7.94 (s, 2H), 7.87 (s, 1H), 7.53 (d, J= 15.9 Hz, 1H), 7.32-7.00 (m, 8H), 6.40 (d, J= 15.9 Hz, 1H), 5.46 (s, 2H), 4.90-4.70 (m, 1H), 4.09-3.97 (m, 1H), 3.85-.3.70 (m, 1H), 3.77 (s, 3H), 3.50-3.23 (m, 3H), 3.17-3.02 (m, 1H), 2.08-1.82 (m, 2H), 1.80-1.55 (m, 2H).
MS (ESI+) 656, 2.25($M^+$+1, detection time)

Example 513

3-[4-({1-[2-(1-Benzyl-6-nitro-1H-indol-3-yl)-3,3,3-trifluoro-2-hydroxypropyl]-piperidin-4-yl}sulfonyl)-3-methoxyphenyl] propanoic acid

H-NMR (400 MHz, $CDCl_3$) δ 8.26 (d, J= 2.0 Hz, 1H), 8.01 (dd, J= 9.0, 2.0 Hz, 1H), 7.85 (d, J= 9.0 Hz, 1H), 7.80 (d, J= 8.0 Hz, 1H), 7.58 (s, 1H), 7.36-7.28 (m, 3H), 7.11-7.06 (m, 2H), 6.95 (d, J= 8.0 Hz, 1H), 6.88 (s, 1H), 5.38 (s, 2H), 3.92 (s, 3H), 3.44-3.32 (m, 1H), 3.31-3.14 (m, 1H), 3.09-2.96 (m, 3H), 2.75-2.65 (m, 3H), 2.64-2.52 (m, 1H), 2.34-2.20 (m, 1H), 2.00-1.67 (m, 4H).
MS (ESI+) 690, 2.25($M^+$+1, detection time)

Example 514

(2E)-3-[4-({1-[2-(1-Benzyl-6-nitro-1H-indol-3-yl)-3,3,3-trifluoro-2-hydroxypropyl]piperidin-4-yl}sulfonyl)-3-methoxyphenyl]acrylic acid

H-NMR (400 MHz, $CDCl_3$) δ 8.28 (d, J= 1.8 Hz, 1H), 8.04 (dd, J= 9.0, 1.8 Hz, 1H), 7.94-7.85 (m, 2H), 7.71 (d, J= 16.0 Hz, 1H), 7.59 (s, 1H), 7.38-7.20 (m, 4H), 7.15-7.07 (m, 3H), 6.53 (d, J= 16.0 Hz, 1H), 5.40 (s, 2H), 3.99 (s, 3H), 3.58-3.34 (m, 3H), 3.19-3.10 (m, 2H), 2.89-2.76 (m, 2H), 2.30-1.60 (m, 2H).
MS (ESI+) 688, 2.27($M^+$+1, detection time)

Example 515

(2E)-3-[4-({1-[2-(1-Benzyl-6-nitro-1H-indol-3-yl)-3,3,3-trifluoro-2-hydroxypropyl]azetidin-3-yl}sulfonyl)-3-methoxyphenyl]acrylic acid

MS (ESI+) 660, 2.27($M^+$+1, detection time)

Example 516

3-[4-({1-[2-(1-Benzyl-6-nitro-1H-indol-3-yl)-3,3,3-trifluoro-2-hydroxypropyl]-azetidin-3-yl}sulfonyl)-3-methoxyphenyl] propanoic acid

H-NMR (400 MHz, $CDCl_3$) δ 8.26 (d, J= 1.6 Hz, 1H), 8.06 (dd, J= 9.0, 1.6 Hz, 1H), 7.95 (d, J= 8.5 Hz, 1H), 7.75 (d, J= 8.5 Hz, 1H), 7.61 (s, 1H), 7.44-7.10 (m, 5H), 6.92 (dd, J= 8.5, 1.0 Hz, 1H), 6.84 (s, 1H), 5.38 (s, 2H), 4.40-4.30 (m, 1H), 4.25-4.15 (m, 1H), 4.11-4.00 (m, 1H), 3.87-3.60 (m, 7H), 2.99 (t, J= 7.3 Hz, 2H), 2.69 (t, J= 7.3 Hz, 2H).
MS (ESI+) 662, 2.19($M^+$+1, detection time)

Example 517

3-[4-({1-[2-(1-Benzyl-6-nitro-1H-indol-3-yl)-3,3,3-trifluoro-2-(methoxymethoxy)-propanoyl]piperidin-4-yl}oxy)-3-methoxyphenyl]propanoic acid

MS (ESI+) 700, 1.75($M^+$+1, detection time)

Example 518

{4-[(1-{2-[6-Cyano-1-(1-naphthylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.95 (d, J= 8.4 Hz, 1H), 7.92-7.87 (m, 1H), 7.84 (d, J= 8.4 Hz, 1H), 7.80-7.74 (m, 1H), 7.70 (s, 1H), 7.52-7.44 (m, 2H), 7.44-7.32 (m, 3H), 6.92 (d, J= 7.0 Hz, 1H), 6.80 (s, 1H), 6.77 (s, 2H), 5.77 (s, 2H), 4.24 (bs, 1H), 3.75 (s, 3H), 3.56 (s, 2H), 3.50-2.40 (m, 6H), 2.00-1.62 (m, 4H).
MS (ESI+) 658, 2.13($M^+$+1, detection time)

Example 519

{4-[(1-{2-[6-Cyano-1-(2-naphthylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.90 (d, J= 8.4 Hz, 1H), 7.85-7.70 (m, 3H), 7.66 (s, 1H), 7.60 (s, 1H), 7.54 (s, 1H), 7.52-7.43 (m, 2H), 7.38 (d, J= 8.4 Hz, 1H), 7.18 (d, J= 8.4 Hz, 1H), 6.85-6.73 (m, 2H), 5.49 (s, 2H), 4.27 (bs, 1H), 3.76 (s, 3H), 3.58 (s, 2H), 3.52-3.17 (m, 2H), 3.00-2.78 (m, 2H), 2.78-1.40 (m, 6H).
MS (ESI+) 658, 2.14($M^+$+1, detection time)

Example 520

{4-[(1-{2-[6-Cyano-1-(cyclohexylmethyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.85 (d, J= 8.4 Hz, 1H), 7.66 (s, 1H), 7.42 (s, 1H), 7.34 (dd, J= 8.4, 1.2 Hz, 1H), 6.84-6.70 (m, 3H), 4.44 (bs, 1H), 4.22-4.10 (m, 1H), 3.94 (d, J= 7.3 Hz, 1H), 3.79 (s, 3H), 3.53 (s, 2H), 3.27 (d, J= 13.5 Hz, 1H), 3.20 (d, J= 13.5 Hz, 1H), 2.89-2.72 (m, 2H), 2.59-2.42 (m, 2H), 1.97-1.60 (m, 8H), 1.60-1.48 (m, 2H), 1.28-1.05 (m, 3H), 1.05-0.88 (m, 2H).
MS (ESI+) 614, 2.13($M^+$+1, detection time)

Example 521

(2E)-3-(4-({1-[2-(1-Benzyl-6-nitro-1H-indol-3-yl)-3,3,3-trifluoro-2-hydroxypropyl]piperidin-4-yl}oxy)-3,5-dimethoxyphenyl]acrylic acid

H-NMR (400 MHz, $CD_3OD$) δ 8.32 (d, J= 1.8 Hz, 1H), 7.99-7.89 (m, 2H), 7.85 (s, 1H), 7.47 (d, J= 15.9 Hz, 1H), 7.26-7.13 (m, 3H), 7.13-7.08 (m, 2H), 6.78 (s, 2H), 6.33 (d, J= 15.9 Hz, 1H), 4.17 (bs, 1H), 3.80-3.60 (m, 1H), 3.68 (s, 6H), 3.54-3.40 (m, 1H), 3.29-3.10 (m, 2H), 2.88-2.70 (m, 2H), 1.90-1.64 (m, 4H).
MS (ESI+) 670, 2.11($M^+$+1, detection time)

Example 522

3-[4-({1-[2-(1-Benzyl-6-nitro-1H-indol-3-yl)-3,3,3-trifluoro-2-hydroxypropyl]-piperidin-4-yl}oxy)-3,5-dimethoxyphenyl] propanoic acid

$^1$H-NMR (400 MHz, $CDCl_3$) δ 8.29 (d, J= 1.8 Hz, 1H), 8.06 (dd, J= 9.0, 1.8 Hz, 1H), 7.87 (d, J= 9.0 Hz, 1H), 7.84 (s, 1H), 7.36-7.23 (m, 3H), 7.18-7.10 (m, 2H), 6.36 (s, 2H), 5.43 (s, 2H), 4.28 (bs, 1H), 3.90 (d, J= 11.1 Hz, 1H), 3.79 (d, J= 11.1 Hz, 1H), 3.70-3.40 (m, 2H), 3.64 (s, 6H), 3.24-3.00 (m, 2H), 2.86 (t, J= 7.6 Hz, 2H), 2.63 (t, J= 7.6 Hz, 2H), 2.50-2.20 (m, 1H), 2.15-1.95 (m, 2H), 1.90-1.70 (m, 1H).
MS (ESI+) 672, 2.10($M^+$+1, detection time)

Example 523

{3-Methoxy-4-[(1-{3,3,3-trifluoro-2-hydroxy-2-[6-nitro-1-(pyridin-2-ylmethyl)-1H-indol-3-yl]propyl}piperidin-4-yl)oxy] phenyl}acetic acid

$^1$H-NMR (400 MHz, $CDCl_3$) δ 8.50 (d, J= 7.7 Hz, 1H), 8.35-8.30 (m, 1H), 8.07 (dd, J= 9.0, 2.0 Hz, 1H), 7.98 (d, J= 9.0 Hz, 1H), 7.85 (s, 1H), 7.69 (ddd, J= 7.7, 7.7, 1.6 Hz, 1H), 7.29-7.18 (m, 1H), 7.14-7.05 (m, 1H), 6.84-6.75 (m, 3H), 5.56 (s, 2H), 4.45 (bs, 1H), 3.91-3.63 (m, 2H), 3.71 (s, 3H), 3.59 (s, 2H), 3.55-3.40 (m, 2H), 3.32-3.18 (m, 1H), 3.18-2.95 (m, 1H), 2.95-1.65 (m, 4H). MS (ESI+) 629, 1.95($M^+$+1, detection time)

Example 524

3-{3-Methoxy-4-[(1-{3,3,3-trifluoro-2-hydroxy-2-[6-nitro-1-(pyridin-2-ylmethyl)-1 H-indol-3-yl]propyl}piperidin-4-yl)oxy] phenyl}propanoic acid

$^1$H-NMR (400 MHz, $CDCl_3$) δ 8.51 (d, J= 7.7 Hz, 1H), 8.32 (d, J= 2.0 Hz, 1H), 8.07 (dd, J= 9.0, 2.0 Hz, 1H),

7.98 (d, J= 9.0 Hz, 1H), 7.85 (s, 1H), 7.70 (ddd, J= 7.7, 7.7, 1.7 Hz, 1H), 7.30-.7.22 (m, 1H), 7.10 (d, J= 7.7 Hz, 1H), 6.78-6.68 (m, 3H), 5.56 (s, 2H), 4.43 (bs, 1H), 3.86 (d, J= 13.6 Hz, 1H), 3.79 (d, J= 13.6 Hz, 1H), 3.71 (s, 3H), 3.60-3.40 (m, 1H), 3.32-3.20 (m, 1H), 3.20-3.00 (m, 1H), 2.90 (t, J= 7.5 Hz, 2H), 2.82-2.15 (m, 3H), 2.65 (t, J= 7.5 Hz, 2H), 2.15-2.00 (m, 2H), 1.95-1.80 (m, 1H).
MS (ESI+) 643, 1.95($M^+$+1, detection time)

Example 525

{4-[(1-{2-[1-(Cyclohexylmethyl)-6-nitro-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.29 (d, J= 1.8 Hz, 1H), 8.01 (dd, J= 8.9, 1.8 Hz, 1H), 7.85 (d, J= 8.9 Hz, 1H), 7.51 (s, 1H), 6.88-6.66 (m, 3H), 4.25-4.10 (m, 1H), 4.00 (d, J= 7.3 Hz, 2H), 3.80 (s, 3), 3.54 (s, 2H), 3.26 (d, J= 13.6 Hz, 1H), 3.18 (d, J= 13.6 Hz, 1H), 3.14-2.60 (m, 2H), 2.60-2.35 (m, 2H), 2.00-1.47 (m, 10H), 1.36-0.91 (m, 5H).
MS (ESI+) 634, 2.19($M^+$+1, detection time)

Example 526

3-[4-({1-[2-(1-Benzyl-6-nitro-1H-indol-3-yl)-3,3,3-trifluoro-2-hydroxypropanoyl]-piperidin-4-yl}oxy)-3,5-dimethoxyphenyl]propanoic acid

$^1$H-NMR (400 MHz, $CDCl_3$) δ 8.27 (s, 1H), 8.00 (bs, 1H), 7.60 (s, 1H), 7.54 (d, J= 8.9 Hz, 1H), 7.37-7.28 (m, 3H), 7.12-7.04 (m, 2H), 6.36 (s, 2H), 5.46 (d, J= 16.0 Hz, 1H), 5.40 (d, J= 16.0 Hz, 1H), 4.28-3.90 (m, 2H), 3.90-3.50 (m, 8H), 3.45-3.20 (m, 1H), 2.87 (t, J= 7.8 Hz, 2H), 2.64 (t, J= 7.8 Hz, 2H), 2.10-1.32 (m, 4H). MS (ESI+) 686, 2.35 ($M^+$+1, detection time)

Example 527

{4-[(1-{2-[1-(Cyclobutylmethyl)-6-nitro-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.34 (d, J= 2.0 Hz, 1H), 8.05 (dd, J= 9.0, 2.0 Hz, 1H), 7.82 (d, J= 9.0 Hz, 1H), 7.67 (s, 1H), 6.83-6.73 (m, 3H), 4.37 (bs, 1H), 4.22 (d, J= 7.4 Hz, 1H), 3.75 (s, 3H), 3.75-3.59 (m, 2H), 3.56 (s, 2H), 3.42-3.10 (m, 2H), 3.10-2.77 (m, 3H), 2.16-1.98 (m, 4H), 1.98-1.73 (m, 6H).
MS (ESI+) 606, 2.11($M^+$+1, detection time)

Example 528

{4-[(1-{2-[1-(4-Chlorobenzyl)-6-nitro-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.26 (d, J= 1.9 Hz, 1H), 8.08 (dd, J= 9.0, 1.9 Hz, 1H), 7.89 (d, J= 9.0 Hz, 1H), 7.69 (s, 1H), 7.28 (d, J= 8.5 Hz, 2H), 7.06 (d, J= 8.5 Hz, 2H), 6.82-6.75 (m, 3H), 5.41 (d, J= 16.1 Hz, 1H), 5.36 (d, J= 16.1 Hz, 1H), 4.46 (bs, 1H), 3.86 (d, J= 13.6 Hz, 1H), 3.78 (d, J= 13.6 Hz, 1H), 3.70 (s, 3H), 3.58 (s, 2H), 3.55-3.40 (m, 2H), 3.26-3.15 (m, 1H), 3.10-2.90 (m, 1H), 2.40-2.20 (m, 1H), 2.19-2.00 (m, 2H), 1.97-1.81 (m, 1H).
MS (ESI+) 662, 2.14($M^+$+1, detection time)

Example 529

{3-Methoxy-4-[(1-{3,3,3-trifluoro-2-hydroxy-2-[1-(4-methylbenzyl)-6-nitro-1H-indol-3-yl]propyl}piperidin-4-yl)oxy]phenyl}acetic acid

$^1$H-NMR (400 MHz, $CDCl_3$) δ 8.30 (d, J= 2.0 Hz, 1H), 8.06 (d, J= 8.9, 2.0 Hz, 1H), 7.88 (d, J= 8.9 Hz, 1H), 7.68 (s, 1H), 7.10 (d, J= 7.9 Hz, 2H), 7.02 (d, J= 7.9 Hz, 2H), 6.82-6.75 (m, 3H), 5.36 (s, 2H), 4.44 (bs, 1H), 3.82 (d, J= 13.6 Hz, 1H), 3.74 (d, J= 13.6 Hz, 1H), 3.69 (s, 3H), 3.57 (s, 2H), 3.49-3.37 (m, 2H), 3.23-3.11 (m, 1H), 3.06-2.88 (m, 1H), 2.35-2.20 (m, 1H), 2.29 (s, 3H), 2.28-2.00 (m, 2H), 1.95-1.80 (m, 1H).
MS (ESI+) 642, 2.15($M^+$+1, detection time)

Example 530

{3-Methoxy-4-[(1-{3,3,3-trifluoro-2-hydroxy-2-[1-(3-methylbenzyl)-6-nitro-1H-indol-3-yl]propyl}piperidin-4-yl)oxy]phenyl}acetic acid

$^1$H-NMR (400 MHz, $CDCl_3$) δ 8.31 (d, J= 2.0 Hz, 1H), 8.08 (dd, J= 8.9, 2.0 Hz, 1H), 7.90 (d, J= 9.0 Hz, 1H), 7.68 (s, 1H), 7.17 (dd, J= 7.6, 7.6 Hz, 1H), 7.09 (d, J= 7.6 Hz, 1H), 6.94 (s, 1H), 6.88 (d, J= 7.6 Hz, 1H), 6.81-6.74 (m, 3H), 5.40 (d, J= 16.0 Hz, 1H), 5.34 (d, J= 16.0 Hz, 1H), 4.45 (bs, 1H), 3.85 (d, J= 13.5 Hz, 1H), 3.77 (d, J= 13.5 Hz, 1H), 3.68 (s, 3H), 3.58 (s, 2H), 3.53-3.40 (m, 2H), 3.30-3.18 (m, 1H), 3.10-2.85 (m, 1H), 2.40-1.80 (m, 4H), 2.28 (s, 3H).
MS (ESI+) 642, 2.23($M^+$+1, detection time)

Example 531

{3-Methoxy-4-[(1-{3,3,3-trifluoro-2-hydroxy-2-[1-(2-methylbenzyl)-6-nitro-1H-indol-3-yl]propyl}piperidin-4-yl)oxy]phenyl}acetic acid

$^1$H-NMR (400 MHz, $CDCl_3$) δ 8.31 (s, 1H), 8.13-8.05 (m, 1H), 7.92 (d, J= 9.1 Hz, 1H), 7.54 (s, 1H), 7.30-7.18 (m, 3H), 7.12-7.04 (m, 1H), 6.83-6.70 (m, 3H), 5.40 (s, 2H), 4.45 (bs, 1H), 3.95-3.63 (m, 2H), 3.68 (s, 3H), 3.57 (s, 2H), 3.56-3.35 (m, 2H), 3.30-3.17 (m, 1H), 3.08-2.85 (m, 1H), 2.45-1.80 (m, 4H), 2.25 (s, 3H).
MS (ESI+) 642, 2.23($M^+$+1, detection time)

Example 532

{4-[(1-{2-[1-(3-Chlorobenzyl)-6-nitro-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.25 (d, J= 2.0 Hz, 1H), 8.08 (dd, J= 9.0, 2.0 Hz, 1H), 7.91 (d, J= 9.0 Hz, 1H), 7.73 (s, 1H), 7.28-7.20 (m, 3H), 7.02 (s, 1H), 6.98 (d, J= 6.7 Hz, 1H), 6.80-6.73 (m, 2H), 5.40 (s, 2H), 4.44 (bs, 1H), 3.92-3.73 (m, 2H), 3.67 (s, 3H), 3.56 (s, 2H), 3.55-3.42 (m, 2H), 3.30-3.17 (m, 1H), 3.09-2.90 (m, 1H), 2.46-1.82 (m, 4H).
MS (ESI+) 662, 2.17($M^+$+1, detection time)

Example 533

{4-[(1-{2-[1-(2-Chlorobenzyl)-6-nitro-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 8.31 (d, J= 1.8 Hz, 1H), 8.09 (dd, J= 9.0, 1.8 Hz, 1H), 7.93 (d, J= 9.0 Hz, 1H), 7.67 (s, 1H), 7.42 (d, J= 7.5 Hz, 1H), 7.32-7.24 (m, 2H), 7.20-7.13 (m, 1H), 6.83 (d, J= 7.5 Hz, 1H), 6.80-6.74 (m, 2H), 5.50 (s, 1H), 4.44 (bs, 1H), 3.90-3.60 (m, 2H), 3.67 (s, 3H), 3.57 (s, 2H), 3.53-3.38 (m, 2H), 3.29-2.86 (m, 2H), 2.45-2.20 (m, 1H), 2.20-2.00 (m, 2H), 1.95-1.80 (m, 1H).
MS (ESI+) 662, 2.14($M^+$+1, detection time)

Example 534

{3-Methoxy-4-[(1-{3,3,3-trifluoro-2-[1-(4-fluorobenzyl)-6-nitro-1H-indol-3-yl]-2-hydroxypropyl}piperidin-4-yl)oxy]phenyl} acetic acid

$^1$H-NMR (400 MHz, $CDCl_3$) δ 8.28 (d, J= 2.0 Hz, 1H), 8.07 (dd, J= 9.0, 2.0 Hz, 1H), 7.87 (d, J= 9.0 Hz, 1H), 7.72 (s, 1H), 7.16-7.08 (m, 2H), 7.05-6.95 (m, 2H), 6.82-6.74 (m, 3H), 5.43 (d, J= 16.4 Hz, 1H), 5.36 (d, J= 16.4 Hz, 1H), 4.46 (bs, 1H), 3.87 (d, J= 13.4 Hz, 1H), 3.81 (d, J= 13.4 Hz, 1H), 3.70 (s, 3H), 3.57 (s, 2H), 3.26-3.13 (m, 2H), 3.08-2.88 (m, 2H), 2.48-2.26 (m, 1H), 2.21-2.03 (m, 2H), 1.96-1.78 (m, 1H).
MS (ESI+) 646, 2.11($M^+$+1, detection time)

Example 535

{3-Methoxy-4-[(1-{3,3,3-trifluoro-2-[1-(3-fluorobenzyl)-6-nitro-1H-indol-3-yl]-2-hydroxypropyl}piperidin-4-yl)oxy]phenyl} acetic acid

$^1$H-NMR (400 MHz, $CDCl_3$) δ 8.26 (d, J= 1.9 Hz, 1H), 8.09 (dd, J= 9.0, 1.9 Hz, 1H), 7.89 (d, J= 9.0 Hz, 1H), 7.75 (s, 1H), 7.33-7.24 (m, 1H), 6.98 (ddd, J= 7.5, 7.5, 2.0 Hz, 1H), 6.93 (d, J= 7.5 Hz, 1H), 6.82-6.71 (m, 4H), 5.42 (s, 2H), 4.44 (bs, 1H), 3.87 (d, J= 13.5 Hz, 1H), 3.80 (d, J= 13.5 Hz, 1H), 3.69 (s, 3H), 3.57 (s, 2H), 3.30-3.12 (m, 2H), 3.12-2.88 (m, 2H), 2.47-2.23 (m, 1H), 2.23-2.00 (m, 4H), 2.00-1.82 (m, 1H).
MS (ESI+) 646, 2.11($M^+$+1, detection time)

Example 536

{3-Methoxy-4-[(1-{3,3,3-trifluoro-2-[1-(2-fluorobenzyl)-6-nitro-1H-indol-3-yl]-2-hydroxypropyl}piperidin-4-yl)oxy]phenyl} acetic acid

$^1$H-NMR (400 MHz, $CDCl_3$) δ 8.35 (s, 1H), 8.06 (d, J= 8.3 Hz, 1H), 7.91 (d, J= 8.3 Hz, 1H), 7.34-7.24 (m, 1H), 7.13-6.98 (m, 3H), 6.84-6.68 (m, 3H), 5.45 (s, 2H), 4.44 (bs, 1H), 3.95-3.72 (m, 2H), 3.68 (s, 3H), 3.56 (s, 2H), 3.34-3.10 (m, 2H), 3.10-2.80 (m, 2H), 2.48-2.20 (m, 1H), 2.20-1.96 (m, 2H), 1.96-1.77 (m, 1H).
MS (ESI+) 646, 2.10($M^+$+1, detection time)

Example 537

3-{4-[(1-{2-[1-(Cyclobutylmethyl)-6-nitro-1H-indol-3-yl]-3,3,3-trifluoro-2-hydroxypropyl}piperidin-4-yl)oxy]-3,5-dimethoxyphenyl}propanoic acid

$^1$H-NMR (400 MHz, $CDCl_3$) δ 8.36 (d, J= 1.8 Hz, 1H), 8.07 (d, J= 7.8 Hz, 1H), 7.83 (d, J= 7.8 Hz, 1H), 7.72 (s, 1H), 6.50-6.10 (bs, 1H), 6.37 (s, 2H), 4.38-4.27 (m, 1H), 4.27-4.15 (m, 1H), 3.90 (d, J= 13.3 Hz, 1H), 3.84 (d, J= 13.3 Hz, 1H), 3.78-3.60 (m, 1H), 3.66 (s, 6H), 3.60-3.45 (m, 1H), 3.30-3.16 (m, 1H), 3.16-3.04 (m, 1H), 2.93-2.78 (m, 3H), 2.61-2.59 (m, 2H), 2.39-2.21 (m, 1H), 2.15-1.97 (m, 4H), 1.97-1.72 (m, 5H).
MS (ESI+) 650, 2.17($M^+$+1, detection time)

Example 538

[2-({1-[2-(1-Benzyl-6-nitro-1H-indol-3-yl)-3,3,3-trifluoro-2-hydroxypropyl]-piperidin-4-yl}oxy)phenoxy]acetic acid

$^1$H-NMR (400 MHz, $CDCl_3$) δ 8.25 (s, 1H), 8.10-7.99 (m, 2H), 7.85 (s, 1H), 7.30-7.20 (m, 3H), 7.10-7.04 (m, 2H), 7.04-6.88 (m, 3H), 6.86-6.78 (m, 1H), 5.43 (d, J= 16.0 Hz, 1H), 5.38 (d, J= 16.0 Hz, 1H), 4.60 (bs, 1H), 4.53 (d, J= 15.9 Hz, 1H), 4.40 (d, J= 15.9 Hz, 1H), 4.07 (d, J= 13.5 Hz, 1H), 3.94 (d, J= 13.5 Hz, 1H), 3.89-3.77 (m, 1H), 3.45-3.35 (m, 2H), 2.40-2.24 (m, 1H), 2.20-2.00 (m, 2H), 1.95-1.80 (m, 1H).
MS (ESI+) 614, 2.18($M^+$+1, detection time)

Example 539

3-[2-({1-[2-(1-Benzyl-6-nitro-1H-indol-3-yl)-3,3,3-trifluoro-2-hydroxypropyl]-piperidin-4-yl}oxy)phenyl]propanoic acid

$^1$H-NMR (400 MHz, $CDCl_3$) δ 8.25 (s, 1H), 8.02 (s, 2H), 7.76 (s, 1H), 7.30-7.10 (m, 5H), 7.05-6.98 (m, 2H), 6.95-6.90 (m, 1H), 6.74-6.68 (m, 1H), 5.44 (d, J= 16.0 Hz, 1H), 5.37 (d, J= 16.0 Hz, 1H), 4.70 (bs, 1H), 4.03 (bs, 2H), 3.62-3.47 (m, 2H), 3.34-3.22 (m, 1H), 2.97-2.80 (m, 2H), 2.58-1.84 (m, 5H).
MS (ESI+) 612, 2.23($M^+$+1, detection time)

Example 540

1-Benzyl-3-[1-({4-[4-(2-carboxyethyl)-2,6-dimethoxyphenoxy]piperidin-1-yl}-methyl)-2,2,2-trifluoro-1-hydroxyethyl]-1H-indole-6-carboxylic acid

MS (ESI+) 671, 2.02($M^+$+1, detection time)

Example 541

3-{4-[(1-{2-[6-(Aminocarbonyl)-1-benzyl-1H-indol-3-yl]-3,3,3-trifluoro-2-hydroxypropyl}piperidin-4-yl)oxy]-3,5-dimethoxyphenyl}propanoic acid

$^1$H-NMR (400 MHz, $CDCl_3$) δ 7.99 (s, 1H), 7.82 (d, J= 8.5 Hz, 1H), 7.64 (s, 1H), 7.53 (d, J= 8.5 Hz, 1H), 7.29-7.20 (m, 3H), 7.08-7.01 (m, 2H), 6.35 (s, 2H), 5.41 (s, 1H), 5.40 (s, 1H), 4.40 (bs, 1H), 3.89 (d, J= 13.6 Hz, 1H), 3.81 (d, J= 13.6 Hz, 1H), 3.58 (s, 6H), 3.53-3.41 (m, 1H), 3.27-3.12 (m, 1H), 3.03-2.22 (m, 5H), 2.13-1.88 (m, 2H), 1.72-1.58 (m, 1H), 1.40-1.13 (m, 2H).
MS (ESI+) 670, 1.95($M^+$+1, detection time)

Example 542

3-(4-{[1-(2-{1-Benzyl-6-[(methylmnino)carbonyl]-1H-indol-3-yl}-3,3,3-trifluoro-2-hydroxypropyl)piperidin-4-yl]oxy}-3,5-dimethoxyphenyl)propanoic acid

MS (ESI+) 684, 1.99($M^+$+1, detection time)

Example 543

3-(4-([1-(2-(1-Benzyl-6-[(dimethylamino)carbonyl]-1H-indol-3-yl)-3,3,3-trifluoro-2-hydroxypropyl)piperidin-4-yl]oxy}-3,5-dimethoxyphenyl)propanoic acid

MS (ESI+) 698, 2.03($M^+$+1, detection time)

Example 544

3-[4-({1-[2-(1-Benzyl-6-{[methoxy(methyl)amino]carbonyl}-1H-indol-3-yl)-3,3,3-trifluoro-2-hydroxypropyl]piperidin-4-yl}oxy)-3,5-dimethoxyphenyl]propanoic acid

$^1$H-NMR (400 MHz, $CDCl_3$) δ 7.82-7.69 (m, 2H), 7.62 (s, 1H), 7.55 (d, J= 8.5 Hz, 1H), 7.29-7.20 (m, 3H), 7.19-7.00 (m, 2H), 6.34 (s, 2H), 5.70 (bs, 1H), 5.47-5.28 (m, 2H), 3.98-3.80 (m, 1H), 3.70-3.40 (m, 2H), 3.58 (s, 6H), 3.48 (s, 3H), 3.38 (s, 3H), 3.30-2.98 (m, 2H), 2.98-2.78 (m, 2H), 2.72-2.52 (m, 2H), 2.40-2.23 (m, 1H), 2.16-2.02 (m, 1H), 1.98-1.82 (m, 1H), 1.72-1.52 (m, 1H), 1.37-1.18 (m, 2H).
MS (ESI+) 714, 2.06($M^+$+1, detection time)

Example 545

3-{4-[(1-{2-[1-Benzyl-6-(morpholin-4-ylcarbonyl)-1H-indol-3-yl]-3,3,3-trifluoro-2-hydroxypropyl}piperidin-4-yl)oxy]-3,5-dimethoxyphenyl}propanoic acid

MS (ESI+) 740, 2.00($M^+$+1, detection time)

Example 546

3-{4-[(1-{2-[6-(Anilinocarbonyl)-1-benzyl-1H-indol-3-yl]-3,3,3-trifluoro-2-hydroxypropyl}piperidin-4-yl)oxy]-3,5-dimethoxyphenyl}propanoic acid

$^1$H-NMR (400 MHz, $CDCl_3$) δ 8.02 (s, 1H), 7.84 (d, J= 8.0 Hz, 1H), 7.67-7.56 (m, 4H), 7.37 (dd, J= 8.0, 8.0 Hz, 2H), 7.26-7.21 (m, 3H), 7.16 (dd, J= 8.0, 8.0 Hz, 2H), 7.09-7.02 (m, 2H), 6.34 (s, 2H), 5.50-5.30 (m, 2H), 4.88 (bs, 1H), 4.39 (bs, 1H), 3.89 (d, J= 13.9 Hz, 1H), 3.81 (d, J= 13.9 Hz, 1H), 3.69-3.40 (m, 2H), 3.58 (s, 6H), 3.29-3.15 (m, 1H), 3.05-2.75 (m, 3H), 2.70-2.50 (m, 2H), 2.40-2.20 (m, 1H), 2.14-2.00 (m, 1H), 2.00-1.83 (m, 1H), 1.73-1.60 (m, 1H).
MS (ESI+) 746, 2.18($M^+$+1, detection time)

Example 547

3-(4-{[1-(2-{1-Benzyl-6-[(benzylamino)carbonyl]-1H-indol-3-yl}-3,3,3-trifluoro-2-hydroxypropyl)piperidin-4-yl]oxy}-3,5-dimethoxyphenyl)propanoic acid

$^1$H-NMR (400 MHz, $CDCl_3$) δ 7.99 (s, 1H), 7.77 (d, J= 8.6 Hz, 1H), 7.59 (s, 1H), 7.48 (d, J= 8.6 Hz, 1H), 7.40-7.29 (m, 5H), 7.25-7.19 (m, 3H), 7.08-6.98 (m, 2H), 6.34 (s, 1H), 5.50-5.30 (m, 2H), 4.44 (bs, 1H), 3.85 (d, J= 13.6 Hz, 1H), 3.79 (d, J= 13.6 Hz, 1H), 3.58 (s, 6H), 3.50-3.10 (m, 2H), 2.95-2.75 (m, 2H), 2.70-2.50 (m, 2H), 2.39-2.24 (m, 1H), 2.13-2.00 (m, 1H), 1.98-1.80 (m, 1H), 1.70-1.53 (m, 1H), 1.35-1.10 (m, 2H).
MS (ESI+) 760, 2.15($M^+$+1, detection time)

Example 548

2-[2-({1-[2-(1-Benzyl-6-nitro-1H-indol-3-yl)-3,3,3-trifluoro-2-hydroxypropyl]-piperidin-4-yl}oxy)phenoxy]propanoic acid

$^1$H-NMR (400 MHz, $CDCl_3$) δ 8.27+8.26 (d, J= 2.2 Hz, 1H), 8.03+8.02 (dd, J= 9.0, 2.2 Hz, 1H), 7.91 (d, J= 9.0 Hz, 1H), 7.59 (s, 1H), 7.35-7.27 (m, 3H), 7.13-7.06 (m, 2H), 6.97-6.89 (m, 4H), 5.87 (bs, 1H), 5.40 (s, 2H), 4.78-4.67 (m, 1H), 4.33 (bs, 1H), 3.32-3.08 (m, 2H), 2.90-2.70 (m, 2H), 2.55-2.35 (m, 2H), 2.00-1.70 (m, 4H), 1.65-1.50 (m, 3H).
MS (ESI+) 628, 2.21($M^+$+1, detection time)

Example 549

2-[2-({1-[2-(1-Benzyl-6-nitro-1H-indol-3-yl)-3,3,3-trifluoro-2-hydroxypropyl]-piperidin-4-yl}oxy)phenoxy]-2-methylpropanoic acid

$^1$H-NMR (400 MHz, $CDCl_3$) 68.26 (d, J= 2.0 Hz, 1H), 8.03 (dd, J= 9.0, 2.0 Hz, 1H), 7.91 (d, J= 9.0 Hz, 1H), 7.60 (s, 1H), 7.37-7.28 (m, 3H), 7.14-6.98 (m, 4H), 6.94-6.85 (m, 2H), 5.40 (s, 2H), 4.37 (bs, 1H), 3.27 (d, J= 13.7 Hz, 1H), 3.17 (d, J= 13.7 Hz, 1H), 2.93-2.80 (m, 1H), 2.78-2.68 (m, 1H), 2.61-2.49 (m, 2H), 2.09-1.89 (m, 2H), 1.89-1.73 (m, 2H), 1.51 (s, 6H).

Example 550

3-[4-({1-[2-(1-Benzyl-6-cyano-1H-indol-3-yl)-3,3,3-trifluoro-2-hydroxypropyl]-piperidin-4-yl}oxy)-3,5-dimethoxyphenyl] propanoic acid

$^1$H-NMR (400 MHz, $CDCl_3$) δ 7.89 (d, J= 8.3 Hz, 1H), 7.67 (s, 1H), 7.64 (s, 1H), 7.41 (d, J= 8.3 Hz, 1H), 7.32-7.27 (m, 3H), 7.10-7.03 (m, 2H), 6.37 (s, 2H), 5.35 (s, 2H), 5.10 (bs, 1H), 4.33 (bs, 1H), 3.86 (d, J= 13.5 Hz, 1H), 3.79 (d, J= 13.5 Hz, 1H), 3.70-3.40 (m, 2H), 3.61 (s, 6H), 3.30-3.02 (m, 2H), 2.88 (t, J= 7.6 Hz, 2H), 2.65 (t, J= 7.6 Hz, 2H), 2.34-2.12 (m, 1H), 2.10-1.92 (m, 2H), 1.84-1.70 (m, 1H). MS (ESI+) 652, 2.10($M^+$+1, detection time)

Example 551

4-({1-[2-(1-Benzyl-6-nitro-1H-indol-3-yl)-3,3,3-trifluoro-2-hydroxypropyl]-piperidin-4-yl}oxy)-3,5-dimethoxybenzoic acid

$^1$H-NMR (400 MHz, $CDCl_3$) δ 8.31 (d, J= 2.0 Hz, 1H), 8.09 (dd, J= 9.0, 2.0 Hz, 1H), 7.89 (d, J= 9.0 Hz, 1H), 7.72 (s, 1H), 7.35-7.28 (m, 5H), 7.15-7.08 (m, 2H), 5.45 (d, J= 15.8 Hz, 1H), 5.40 (d, J= 15.8 Hz, 1H), 4.52 (bs, 1H), 3.89 (d, J= 13.4 Hz, 1H), 3.80 (d, J= 13.4 Hz, 1H), 3.73 (s, 6H), 3.68-3.50 (m, 2H), 3.32-3.05 (m, 2H), 2.40-2.00 (m, 1H), 2.15-2.00 (m, 2H), 1.90-1.75 (m, 1H).
MS (ESI+) 644, 2.22($M^+$+1, detection time)

Example 552

3-[4-({(1-[2-(1-Benzyl-6-nitro-1H-indol-3-yl)-3,3,3-trifluoro-2-hydroxypropyl]-azetidin-3-yl}oxy)-3,5-dimethoxyphenyl] propanoic acid

$^1$H-NMR (400 MHz, $CDCl_3$) δ 8.29 (s, 1H), 8.06 (d, J= 1.8 Hz, 1H), 7.87 (d, J= 1.8 Hz, 1H), 7.80 (s, 1H), 7.39-7.27 (m, 3H), 7.22-7.14 (m, 2H), 6.36 (s, 2H), 5.40 (s, 2H), 4.80-4.60 (m, 1H), 4.25-4.04 (m, 4H), 3.83-3.54 (m, 2H), 3.67 (s, 6H), 2.85 (t, J= 7.1 Hz, 2H), 2.63 (t, J= 7.1 Hz, 2H).

MS (ESI+) 644, 2.14($M^+$+1, detection time)

Example 553

2-[4-({1-[2-(1-Benzyl-6-nitro-1H-indol-3-yl)-3,3,3-trifluoro-2-hydroxypropyl]-piperidin-4-yl}oxy)-3-methoxyphenyl]-2-methylpropanoic acid

$^1$H-NMR (400 MHz, $CDCl_3$) δ 8.30 (d, J= 1.9 Hz, 1H), 8.11-8.04 (m, 1H), 7.88 (d, J= 8.9 Hz, 1H), 7.77-7.72 (m, 1H), 7.34-7.27 (m, 3H), 7.15-7.08 (m, 2H), 6.92-6.84 (m, 2H), 6.80-6.74 (m, 1H), 5.42 (s, 2H), 4.46 (m, 1H), 3.93-3.75 (m, 2H), 3.74-3.40 (m, 2H), 3.66 (s, 3H), 3.28-3.16 (m, 1H), 3.03-2.87 (m, 1H), 2.52-2.31 (m, 1H), 2.21-2.03 (m, 4H), 1.94-1.77 (m, 1H), 1.57 (s, 6H).
MS (ESI+) 656, 2.31($M^+$+1, detection time)

Example 554

[4-({1-[2-(1-Benzyl-6-nitro-1H-indol-3-yl)-3,3,3-trifluoro-2-hydroxypropyl]-piperidin-4-yl}oxy)-3-hydroxyphenyl]acetic acid

$^1$H-NMR (400 MHz, $CD_3OD$) δ 8.38 (m, 1H), 7.95 (s, 1H), 7.95 (s, 1H), 7.88 (s, 1H), 7.25-7.18 (m, 5H), 6.76 (d, J= 8.2 Hz, 1H), 6.68 (d, J= 2.1 Hz, 1H), 6.57 (dd, J= 8.2, 2.1 Hz, 1H), 5.47 (s, 1H), 5.46 (s, 1H), 4.48-4.35 (m, 1H), 4.06 (d, J= 13.5 Hz, 1H), 3.76 (d, J= 13.5 Hz, 1H), 3.61-3.22 (m, 3H), 3.35 (s, 2H), 3.16-3.02 (m, 1H), 2.08-1.78 (m, 4H).
MS (ESI+) 614, 2.17($M^+$+1, detection time)

Example 555

3-[4-({1-[2-(1-Benzyl-6-nitro-1H-indol-3-yl)-3,3,3-trifluoro-2-hydroxypropyl]-piperidin-4-yl}oxy)-3,5-dimethoxyphenyl]-3-hydroxy-2,2-dimethylpropanoic acid MS (ESI+) 716, 2.21($M^+$+1, detection time)

Example 556

[3-({1-[2-(1-Benzyl-6-nitro-1H-indol-3-yl)-3,3,3-trifluoro-2-hydroxypropyl]-piperidin-4-yl}oxy)-4-methoxyphenyl]acetic acid

$^1$H-NMR (400 MHz, $CDCl_3$) δ 8.29 (d, J= 1.7 Hz, 1H), 8.05 (dd, J= 8.9, 1.7 Hz, 1H), 7.89 (d, J= 8.9 Hz, 1H), 7.72 (s, 1H), 7.33-7.27 (m, 3H), 7.15-7.08 (m, 2H), 6.92-6.77 (m, 3H), 5.41 (s, 2H), 4.46 (s, 1H), 3.88 (d, J= 13.5 Hz, 1H), 3.80 (d, J= 13.5 Hz, 1H), 3.68 (s, 3H), 3.60-3.40 (m, 2H), 3.51 (s, 2H), 3.30-3.17 (m, 1H), 3.10-2.85 (m, 1H), 2.45-2.23 (m, 1H), 2.20-2.00 (m, 2H), 1.95-1.80 (m, 1H).
MS (ESI+) 628, 2.18($M^+$+1, detection time)

Example 557

(3-Methoxy-4-{[1-(3,3,3-trifluoro-2-hydroxy-2-{1-[4-(methylsulfonyl)benzyl]-6-nitro-1H-indol-3-yl}propyl)piperidin-4-yl]oxy}phenyl)acetic acid

$^1$H-NMR (400 MHz, $CDCl_3$) δ 8.19 (d, J= 1.9 Hz, 1H), 8.09 (dd, J= 9.0, 1.9 Hz, 1H), 7.95-7.75 (m, 4H), 7.30-7.20 (m, 2H), 6.83-6.75 (m, 3H), 5.53 (s, 2H), 4.48 (bs, 1H), 3.86 (s, 3H), 3.78-3.52 (m, 2H), 3.58 (s, 2H), 3.40-2.80 (m, 4H), 3.03 (s, 3H), 2.45-2.23 (m, 1H), 2.23-2.06 (m, 2H), 1.99-1.80 (m, 1H).
MS (ESI+) 706, 2.06($M^+$+1, detection time)

Example 558

{3-Methoxy-4-[(1-{3,3,3-trifluoro-2-hydroxy-2-[1-(3-methoxybenzyl)-6-nitro-1H-indol-3-yl]propyl}piperidin-4-yl)oxy]phenyl}acetic acid

$^1$H-NMR (400 MHz, $CDCl_3$) δ 8.28 (d, J= 1.9 Hz, 1H), 8.06 (dd, J= 9.0, 1.9 Hz, 1H), 7.89 (d, J= 9.0 Hz, 1H), 7.72 (s, 1H), 7.21 (dd, J= 7.8, 7.8 Hz, 1H), 6.85-6.72 (m, 4H), 6.68 (d, J= 7.8 Hz, 1H), 6.56 (s, 1H), 5.38 (s, 2H), 4.40 (s, 1H), 3.90-3.40 (m, 2H), 3.68 (s, 3H), 3.67 (s, 3H), 3.56 (s, 2H), 3.45-3.30 (m, 2H), 3.20-2.90 (m, 2H), 2.40-2.18 (m, 1H), 2.18-1.95 (m, 2H), 1.95-1.80 (m, 1H).

MS (ESI+) 658, 2.16($M^+$+1, detection time)

Example 559

{3-Methoxy-4-[(1-{3,3,3-trifluoro-2-hydroxy-2-[1-(4-methoxybenzyl)-6-nitro-1H-indol-3-yl]propyl}piperidin-4-yl)oxy]phenyl}acetic acid

$^1$H-NMR (400 MHz, $CDCl_3$) δ 8.31 (d, J= 1.8 Hz, 1H), 8.05 (dd, J= 9.0, 1.8 Hz, 1H), 7.87 (d, J= 9.0 Hz, 1H), 7.71 (s, 1H), 7.09 (d, J= 8.6 Hz, 1H), 6.83 (d, J= 8.6 Hz, 1H), 6.80-6.72 (m, 3H), 5.34 (s, 2H), 4.42 (bs, 1H), 3.84 (d, J= 13.9 Hz, 1H), 3.76 (d, J= 13.9 Hz, 1H), 3.75 (s, 3H), 3.68 (s, 3H), 3.56 (s, 2H), 3.52-3.36 (m, 2H), 3.22-3.09 (m, 1H), 3.09-2.87 (m, 1H), 2.45-2.20 (m, 1H), 2.20-1.98 (m, 2H), 1.98-1.78 (m, 2H).
MS (ESI+) 658, 2.15($M^+$+1, detection time)

Example 560

3-{3-Methoxy-4-[(1-{3,3,3-trifluoro-2-hydroxy-2-[1-(2-methoxybenzyl)-6-nitro-1H-indol-3-yl]propyl}piperidin-4-yl)oxy]phenyl}propanoic acid

$^1$H-NMR (400 MHz, $CDCl_3$) δ 8.46 (d, J= 1.9 Hz, 1H), 8.05 (dd, J= 8.9, 1.9 Hz, 1H), 7.84 (d, J= 8.9 Hz, 1H), 7.80 (s, 1H), 7.29 (d, J= 8.2 Hz, 1H), 6.99 (d, J= 8.2 Hz, 1H), 6.89 (d, J= 8.2 Hz, 1H), 6.85 (dd, J= 8.2, 8.2 Hz, 1H), 6.79-6.65 (m, 3H), 5.40 (s, 2H), 4.41 (s. H), 3.91 (d, J= 13.0 Hz, 1H), 3.88 (s, 3H), 3.79 (d, J= 13.0 Hz, 1H), 3.66 (s, 3H), 3.59-3.40 (m, 2H), 3.32-3.18 (m, 1H), 3.00-2.80 (m, 3H), 2.64 (t, J= 7.5 Hz, 2H), 2.55-2.08 (m, 1H), 2.22-2.00 (m, 2H), 1.90-1.78 (m, 1H). MS (ESI+) 672, 2.25($M^+$+1, detection time)

Example 561

3-{3-Methoxy-4-[(1-{3,3,3-trifluoro-2-hydroxy-2-[1-(3-methoxybenzyl)-6-nitro-1H-indol-3-yl]propyl}piperidin-4-yl)oxy]phenyl}propanoic acid

$^1$H-NMR (400 MHz, $CDCl_3$) δ 8.29 (d, J= 1.9 Hz, 1H), 8.06 (dd, J= 9.0, 1.9 Hz, 1H), 7.89 (d, J= 9.0 Hz, 1H), 7.75 (s, 1H), 7.20 (dd, J= 7.9, 7.9 Hz, 1H), 6.84-6.65 (m, 5H), 6.56 (s, 1H), 5.39 (s, 2H), 4.40 (s, 1H), 3.89 (d, J= 13.5 Hz, 1H), 3.81 (d, J= 13.5 Hz, 1H), 3.68 (s, 3H), 3.65 (s, 3H), 3.60-3.42 (m, 2H), 3.33-3.18 (m, 1H), 3.08-2.92 (m, 1H), 2.92-2.80 (m, 2H), 2.68-2.57 (m, 2H), 2.49-2.28 (m, 1H), 2.20-2.01 (m, 2H), 1.98-1.78 (m, 1H).
MS (ESI+) 672, 2.23($M^+$+1, detection time)

Example 562

3-{3-Methoxy-4-[(1-{3,3,3-trifluoro-2-hydroxy-2-[1-(4-methoxybenzyl)-6-nitro-1H-indol-3-yl]propyl}piperidin-4-yl)oxy]phenyl}propanoic acid

$^1$H-NMR (400 MHz, $CDCl_3$) δ 8.31 (d, J= 1.7 Hz, 1H), 8.07 (d, J= 8.9 Hz, 1H), 7.86 (d, J= 8.9 Hz, 1H), 7.74 (s, 1H), 7.09 (d, J= 8.5 Hz, 1H), 6.82 (d, J= 8.5 Hz, 1H), 6.79-6.65 (m, 3H), 5.35 (s, 2H), 4.42 (bs, 1H), 3.89 (d, J= 13.4 Hz, 1H), 3.79 (d, J= 13.4 Hz, 1H), 3.76 (s, 3H), 3.66 (s, 3H), 3.61-3.42 (m, 2H), 3.30-3.15 (m, 1H), 3.06-2.90 (m, 1H), 2.88 (t, J= 7.8 Hz, 2H), 2.64 (t, J= 7.8 Hz, 2H), 2.50-2.32 (m, 1H), 2.21-2.01 (m, 2H), 1.93-1.73 (m, 1H).
MS (ESI+) 672, 2.19($M^+$+1, detection time)

Example 563

3-{4-[(1-{2-[6-Bromo-1-(2-fluorobenzyl)-1H-indol-3-yl]-3,3,3-trifluoro-2-hydroxypropyl}piperidin-4-yl)oxy]-3,5-dimethoxyphenyl}propanoic acid

$^1$H-NMR (400 MHz, $CDCl_3$) δ 7.63 (d, J= 8.6 Hz, 1H), 7.52 (d, J= 1.6 Hz, 1H), 7.50 (s, 1H), 7.34-7.23 (m, 2H), 7.10-6.97 (m, 2H), 6.92-6.84 (m, 1H), 6.35 (s, 2H), 5.34 (s, 2H), 4.33 (bs, 1H), 3.85 (d, J= 13.7 Hz, 1H), 3.80 (d, J= 13.7 Hz, 1H), 3.68-3.51 (m, 1H), 3.58 (s, 6H), 3.51-3.40 (m, 1H), 3.32-3.21 (m, 1H), 3.12-2.98 (m, 1H), 2.88 (t, J= 7.7 Hz, 2H), 2.66 (t, J= 7.7 Hz, 2H), 2.42-2.27 (m, 1H), 2.15-1.88 (m, 2H), 1.78-1.65 (m, 1H). MS (ESI+) 724, 2.33(M++2, detection time)

Example 564

[4-({1-[2-(1-Benzyl-7-chloro-1H-pyrrolo[2,3-c]pyridin-3-yl)-3,3,3-trifluoro-2-hydroxypropanoyl]piperidin-4-yl}oxy)-3-methoxyphenyl]acetic acid

$^1$H-NMR (400 MHz, $CDCl_3$) δ 8.04 (bs, 1H), 7.46 (s, 1H), 7.32 (d, J= 5.5 Hz, 1H), 7.29-7.22 (m, 3H), 7.04-6.97 (m, 2H), 6.82-6.69 (m, 3H), 5.90 (d, J= 16.3 Hz, 1H), 5.71 (d, J= 16.3 Hz, 1H), 4.40-3.14 (m, 10H), 3.58 (s, 2H), 2.00-1.60 (m, 4H).
MS (ESI+) 632, 2.39($M^+$+1, detection time)

Example 565

3-[4-({1-[2-(1-Benzyl-6-chloro-1H-indol-3-yl)-3,3,3-trifluoro-2-hydroxypropyl]-piperidin-4-yl}oxy)-3,5-dimethoxyphenyl] propanoic acid

$^1$H-NMR (400 MHz, $CDCl_3$) δ 7.68 (d, J= 9.2 Hz, 1H), 7.51 (s, 1H), 7.30 (d, J= 1.7 Hz, 1H), 7.29-7.23 (m, 3H), 7.19-7.14 (m, 1H), 7.08-7.02 (m, 2H), 6.36 (s, 2H), 5.31 (s, 2H), 4.33 (bs, 1H), 3.87-3.78 (m, 2H), 3.67-3.40 (m, 2H), 3.59 (s, 6H), 3.33-3.21 (m, 1H), 3.13-3.00 (m, 1H), 2.89 (t, J= 7.9 Hz, 2H), 2.66 (t, J= 7.9 Hz, 2H), 2.48-2.27 (m, 1H), 2.15-1.35 (m, 3H).
MS (ESI+) 661, 2.30($M^+$+1, detection time)

Example 566

4-[4-({1-[2-(1-Benzyl-6-cyano-1H-indol-3-yl)-3,3,3-trifluoro-2-hydroxypropyl] - piperidin-4-yl}oxy)-3,5-dimethoxyphenyl] butanoic acid

MS (ESI+) 666, 2.26($M^+$+1, detection time)

Example 567

3-[4-({1-[2-(1-Benzyl-7-chloro-1H-indol-3-yl)-3,3,3-trifluoro-2-hydroxypropyl]-piperidin-4-yl}oxy)-3,5-dimethoxyphenyl] propanoic acid

$^1$H-NMR (400 MHz, $CDCl_3$) δ 7.68 (d, J= 7.9 Hz, 1H), 7.50 (s, 1H), 7.25-7.16 (m, 4H), 7.11 (dd, J= 7.9, 7.9 Hz, 1H), 7.01-6.92 (m, 2H), 6.35 (s, 2H), 5.90 (d, J= 6.4 Hz, 1H), 5.69 (d, J= 6.4 Hz, 1H), 4.35 (bs, 1H), 3.86 (s, 2H), 3.65-3.50 (m, 1H), 3.57 (s, 6H), 3.50-3.40 (m, 1H), 3.35-3.25 (m, 1H), 3.12-3.00 (m, 1H), 2.88 (t, J= 7.7 Hz, 2H), 2.65 (t, J= 7.7 Hz, 2H), 2.45-2.30 (m, 1H), 2.15-1.88 (m, 2H), 1.78-1.65 (m, 1H). MS (ESI+) 661, 2.33($M^+$+1, detection time)

Example 568

{4-[(1-{2-[6-Bromo-1-(2-fluorobenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.63 (d, J= 8.5 Hz, 1H), 7.51 (d, J= 1.4 Hz, 1H), 7.49 (s, 1H), 7.33-7.21 (m, 2H), 7.07-6.96 (m, 2H), 6.95-6.88 (m, 1H), 6.77 (s, 3H), 5.33 (s, 2H), 4.42 (s, 1H), 3.88 (d, J= 13.0 Hz, 1H), 3.78 (d, J= 13.0 Hz, 1H), 3.62 (s, 3H), 3.57 (s, 2H), 3.55-3.38 (m, 2H), 3.33-3.20 (m, 1H), 2.97-2.78 (m, 1H), 2.55-2.38 (m, 1H), 2.19-2.00 (m, 2H), 1.88-1.70 (m, 1H).
MS (ESI+) 679, 2.33($M^+$+1, detection time)

Example 569

3-{4-[(1-{2-[6-Bromo-1-(2-fluorobenzyl)-1H-indol-3-yl]-3,3,3-trifluoro-2-hydroxypropyl}piperidin-4-yl)oxy]-3-methoxyphenyl}propanoic acid

$^1$H-NMR (400 MHz, $CDCl_3$) δ 7.64 (d, J= 8.6 Hz, 1H), 7.51 (d, J= 1.6 Hz, 1H), 7.48 (s, 1H), 7.29 (dd, J= 8.6, 1.6 Hz, 1H), 7.28-7.22 (m, 1H), 7.08-6.96 (m, 2H), 6.94-6.86 (m, 1H), 6.78-6.67 (m, 3H), 5.33 (s, 2H), 4.40 (bs, 1H), 3.86 (d, J= 13.3 Hz, 1H), 3.79 (d, J= 13.3 Hz, 1H), 3.62 (s, 3H), 3.55-3.38 (m, 2H), 3.34-3.20 (m, 1H), 2.97-2.80 (m, 1H), 2.89 (t, J= 7.7 Hz, 2H), 2.64 (t, J= 7.7 Hz, 2H), 2.52-2.30 (m, 1H), 2.18-2.00 (m, 2H), 1.88-1.70 (m, 1H).

MS (ESI+) 693, 2.34($M^+$+1, detection time)

Example 570

4-[(1-{2-[6-Bromo-1-(2-fluorobenzyl)-1H-indol-3-yl]-3,3,3-trifluoro-2-hydroxypropyl}piperidin-4-yl)oxy]-3,5-dimethoxybenzoic acid

$^1$H-NMR (400 MHz, $CDCl_3$) δ 7.62 (d, J= 8.6 Hz, 1H), 7.53 (d, J= 1.5 Hz, 1H), 7.52 (s, 1H), 7.31 (d, J= 8.6 Hz, 1H), 7.30-7.23 (m, 3H), 7.09-6.98 (m, 2H), 6.95-6.88 (m, 1H), 5.34 (s, 2H), 4.53 (bs, 1H), 3.88 (d, J= 13.7 Hz, 1H), 3.82 (d, J= 13.7 Hz, 1H), 3.66 (s, 6H), 3.63-3.43 (m, 2H), 3.37-3.25 (m, 1H), 3.09-2.94 (m, 1H), 2.50-2.34 (m, 1H), 2.16-1.97 (m, 2H), 1.78-1.65 (m, 1H).
MS (ESI+) 695, 2.33($M^+$+1, detection time)

Example 571

3-{3,5-Dimethoxy-4-[(1-{3,3,3-trifluoro-2-[6-fluoro-1-(2-fluorobenzyl)-1H-indol-3-yl]-2-hydroxypropyl}piperidin-4-yl)oxy]phenyl}propanoic acid

$^1$H-NMR (400 MHz, $CDCl_3$) δ 7.74-7.64 (m, 1H), 7.50 (s, 1H), 7.32-7.20 (m, 1H), 7.10-6.85 (m, 5H), 6.69+6.35 (s, 2H), 5.32 (s, 2H), 4.46+4.33 (bs, 1H), 3.90-3.75 (m, 2H), 3.70-3.40 (m, 2H), 3.64+3.59 (s, 6H), 3.40-3.23 (m, 1H), 3.14-2.97 (m, 1H), 2.88 (t, J= 7.7 Hz, 2H), 2.65 (t, J= 7.7 Hz, 2H), 2.45-2.22 (m, 1H), 2.15-1.90 (m, 2H), 1.80-1.65 (m, 1H).
MS (ESI+) 663, 2.29($M^+$+1, detection time)

Example 572

[4-({1-[2-(1-Benzyl-5-nitro-1H-indol-3-yl)-3,3,3-trifluoro-2-hydroxypropyl]-piperidin-4-yl}oxy)-3-methoxyphenyl]acetic acid

$^1$H-NMR (400 MHz, $CDCl_3$) δ 8.73 (s, 1H), 8.12 (dd, J= 9.2, 2.0 Hz, 1H), 7.67 (s, 1H), 7.37 (d, J= 9.2 Hz, 1H), 7.32-7.26 (m, 3H), 7.12-7.04 (m, 2H), 6.77 (s, 3H), 5.38 (s, 2H), 4.45 (bs, 1H), 3.90 (d, J= 13.5 Hz, 1H), 3.84 (d, J= 13.5 Hz, 1H), 3.67 (s, 3H), 3.57 (s, 2H), 3.57-3.42 (m, 2H), 3.30-3.15 (m, 1H), 3.10-2.87 (m, 1H), 2.45-2.20 (m, 1H), 2.20-2.00 (m, 2H), 195-1.80 (m, 1H).
MS (ESI+) 628, 2.29($M^+$+1, detection time)

Example 573

3-[4-({1-[2-(1-Benzyl-6-chloro-1H-indol-3-yl)-3,3,3-trifluoro-2-hydroxypropyl]-piperidin-4-yl}oxy)-3-methoxyphenyl]propanoic acid

H-NMR (400 MHz, $CDCl_3$) δ 7.68 (d, J= 8.6 Hz, 1H), 7.48 (s, 1H), 7.30 (d, J= 1.6 Hz, 1H), 7.28-7.20 (m, 3H), 7.16 (d, J= 8.6, 1.6 Hz, 1H), 7.10-7.00 (m, 2H), 6.80-6.65 (m, 3H), 5.29 (s, 2H), 4.40 (bs, 1H), 3.84 (d, J= 13.8 Hz, 1H), 3.80 (d, J= 13.8 Hz, 1H), 3.62 (s, 3H), 3.55-3.36 (m, 2H), 3.33-3.20 (m, 1H), 3.00-2.80 (m, 1H), 2.88 (t, J= 7.6 Hz, 2H), 2.64 (t, J= 7.6 Hz, 2H), 2.53-2.30 (m, 1H), 2.18-2.00 (m, 2H), 1.90-1.70 (m, 1H).
MS (ESI+) 632, 2.35(M++2, detection time)

Example 574

1-Benzyl-3-[1-({4-[4-(2-carboxyethyl)-2,6-dimethoxyphenoxy]piperidin-1-yl}methyl)-2,2,2-trifluoro-1-hydroxyethyl]-1H-indole-5-carboxylic acid

$^1$H-NMR (400 MHz, $CDCl_3$) δ 8.51 (s, 1H), 7.95 (d, J= 8.0 Hz, 1H), 7.67 (s, 1H), 7.40-7.20 (m, 4H), 7.11-7.00 (m, 2H), 6.68+6.35 (s, 2H), 5.37 (s, 2H), 4.58+4.40 (bs, 1H), 3.98 (d, J= 13.2 Hz, 1H), 3.90 (d, J= 13.2 Hz, 1H), 3.74-3.40 (m, 2H), 3.66+3.59 (s, 6H), 3.32-3.20 (m, 1H), 3.16-3.00 (m, 1H), 2.87 (t, J= 7.2 Hz, 2H), 2.66 (t, J= 7.2 Hz, 2H), 2.42-2.24 (m, 1H), 2.12-1.91 (m, 2H), 1.78-1.62 (m, 1H). MS (ESI+) 671, 2.23($M^+$+1, detection time)

Example 575

3-{4-[(1-{2-[6-Cyano-1-(4-methoxybenzyl)-1H-indol-3-yl]-3,3,3-trifluoro-2-hydroxypropyl}piperidin-4-yl)oxy]-3-methoxyphenyl}propanoic acid

$^1$H-NMR (400 MHz, $CDCl_3$) δ 7.87 (d, J= 8.0 Hz, 1H), 7.65 (s, 1H), 7.64 (s, 1H), 7.40 (d, J= 8.0 Hz, 1H), 7.04 (d, J= 8.6 Hz, 2H), 6.82 (d, J= 8.6 Hz, 2H), 6.78-6.66 (m, 3H), 5.28 (s, 2H), 4.40 (bs, 1H), 3.84 (d, J= 13.5 Hz, 1H), 3.77 (s, 3H), 3.75 (d, J= 13.5 Hz, 1H), 3.64 (s, 3H), 3.53-3.46 (m, 2H), 3.27-3.12 (m, 1H), 3.10-2.91 (m, 1H), 2.89 (t, J= 7.5 Hz, 2H), 2.64 (t, J= 7.5 Hz, 2H), 2.43-2.21 (m, 1H), 2.18-2.00 (m, 2H), 1.93-1.78 (m, 1H).
MS (ESI+) 652, 2.10($M^+$+1, detection time)

Example 576

3-[3,5-Dimethoxy-4-({1-[3,3,3-trifluoro-2-hydroxy-2-(6-nitro-1-phenyl-1H-indol-3-yl)propyl]piperidin-4-yl}oxy)phenyl] propanoic acid

$^1$H-NMR (400 MHz, $CDCl_3$) δ 8.44 (d, J= 1.8 Hz, 1H), 8.13 (dd, J= 8.9, 1.8 Hz, 1H), 8.01 (d, J= 8.9 Hz, 1H), 7.88 (s, 1H), 7.67-7.57 (m, 2H), 7.57-7.47 (m, 3H), 6.71+6.37 (s, 2H), 4.45+4.33 (bs, 1H), 3.93 (d, J= 13.6 Hz, 1H), 3.81 (d, J= 13.6 Hz, 1H), 3.75+3.68 (s, 6H), 3.72-3.50 (m, 2H), 3.40-3.10 (m, 2H), 2.88 (t, J= 7.6 Hz, 2H), 2.65 (t, J= 7.6 Hz, 2H), 2.42-2.20 (m, 1H), 2.15-2.00 (m, 2H), 2.00-1.80 (m, 1H).
MS (ESI+) 658, 2.15($M^+$+1, detection time)

Example 577

{5-[2-(1-Benzyl-6-nitro-1H-indol-3-yl)-3,3,3-trifluoro-2-hydroxypropyl]-4,5,6,7-tetrahydro-2H-pyrazolo[4,3-c]pyridin-2-yl}acetic acid

MS (ESI+) 544, 2.18($M^+$+1, detection time)

Example 578

7-[2-(1-Benzyl-6-nitro-1H-indol-3-yl)-3,3,3-trifluoro-2-hydroxypropyl]-5,6,7,8-tetrahydroimidazo[1,2-a]pyrazine-2-carboxylic acid

MS (ESI+) 530, 2.29($M^+$+1, detection time)

Example 579

{5-[2-(1-Benzyl-6-nitro-1H-indol-3-yl)-3,3,3-trifluoro-2-hydroxypropyl]-4,5,6,7-tetrahydro[1,3]thiazolo[5,4-c]pyridin-2-yl} acetic acid

MS (ESI+) 561, 2.32($M^+$+1, detection time)

Example 580

[4-({1-[2-(1-Benzyl-7-chloro-1H-pyrrolo[2,3-c]pyridin-3-yl)-3,3,3-trifluoro-2-hydroxypropyl]piperidin-4-yl}oxy)-3-methoxyphenyl]acetic acid

$^1$H-NMR (400 MHz, $CDCl_3$) δ 8.09 (d, J= 5.6 Hz, 1H), 7.69 (d, J= 5.6 Hz, 1H), 7.65 (s, 1H), 7.25-7.20 (m, 3H), 7.03-6.96 (m, 2H), 6.82-6.73 (m, 3H), 5.80 (s, 2H), 4.43 (bs, 1H), 3.86 (d, J= 13.5 Hz, 1H), 3.75 (d, J= 13.5 Hz, 1H), 3.65 (s, 3H), 3.57 (s, 2H), 3.52-3.39 (m, 2H), 3.24-3.10 (m, 1H), 3.05-2.80 (m, 1H), 2.45-2.22 (m, 1H), 2.15-2.00 (m, 2H), 1.94-1.77 (m, 1H).
MS (ESI+) 618, 2.16($M^+$+1, detection time)

Example 581

[4-({1-[2-(1-Benzyl-6-cyano-2-methyl-1H-indol-3-yl)-3,3,3-trifluoro-2-hydroxypropyl]piperidin-4-yl}oxy)-3-methoxyphenyl]acetic acid

$^1$H-NMR (400 MHz, $CDCl_3$) δ 7.72 (bs, 1H), 7.59 (s, 1H), 7.40 (dd, J= 8.5, 1.3 Hz, 1H), 7.26-7.18 (m, 3H), 6.86-6.80 (m, 2H), 6.79-6.75 (m, 3H), 5.38 (s, 2H), 4.46 (bs, 1H), 4.11 (d, J= 13.0 Hz, 1H), 3.87 (d, J= 13.0 Hz, 1H), 3.60 (s, 3H), 3.58 (s, 2H), 3.50-3.20 (m, 3H), 3.00-2.82 (m, 1H), 2.69 (s, 3H), 2.44-2.22 (m, 1H), 2.15-2.00 (m, 2H), 1.93-1.80 (m, 1H).
MS (ESI+) 622, 2.23($M^+$+1, detection time)

Example 582

{4-[(1-{2-[7-Chloro-1-(4-methoxybenzyl)-1H-pyrrolo[2,3-c]pyridin-3-yl]-3,3,3-trifluoro-2-hydroxypropyl}piperidin-4-yl) oxy]-3-methoxyphenyl}acetic acid

$^1$H-NMR (400 MHz, $CDCl_3$) δ 8.09 (d, J= 5.6 Hz, 1H), 7.67 (d, J= 5.5 Hz, 1H), 7.62 (s, 1H), 7.00 (d, J= 8.6 Hz, 2H), 6.89-6.75 (m, 5H), 5.74 (s, 2H), 4.44 (bs, 1H), 3.85 (d, J= 13.6 Hz, 1H), 3.80-3.62 (m, 1H), 3.74 (s, 3H), 3.67 (s, 3H), 3.58 (s, 2H), 3.55-3.40 (m, 2H), 3.22-3.10 (m, 1H), 3.02-2.82 (m, 1H), 2.50-2.25 (m, 1H), 2.20-2.00 (m, 2H), 1.97-1.75 (m, 1H).
MS (ESI+) 648, 2.15($M^+$+1, detection time)

Example 583

3-[4-({1-[3,3,3-Trifluoro-2-hydroxy-2-(6-nitro-1-phenyl-1H-indol-3-yl)propyl]-piperidin-4-yl}sulfonyl)phenyl]propanoic acid

$^1$H-NMR (400 MHz, $CDCl_3$) δ 8.42 (d, J= 2.0 Hz, 1H), 8.09 (dd, J= 8.9, 2.0 Hz, 1H), 7.94 (d, J= 9.0 Hz, 1H), 7.78 (s, 1H), 7.73 (d, J= 8.3 Hz, 2H), 7.65-7.58 (m, 2H), 7.55-7.46 (m, 3H), 7.43 (d, J= 8.3 Hz, 2H), 3.64 (d, J= 14.0 Hz, 1H), 3.50 (d, J= 14.0 Hz, 1H), 3.42-3.23 (m, 2H), 3.10-2.65 (m, 3H), 3.05 (t, J= 7.2 Hz, 2H), 2.72 (t, J= 7.2 Hz, 2H), 2.40-1.40 (m, 4H).
MS (ESI+) 646, 2.27($M^+$+1, detection time)

Example 584

[4-({1-[2-(1-Benzyl-6-cyano-1H-indol-3-yl)-3,3,3-trifluoro-2-hydroxypropyl]-piperidin-4-yl}oxy)phenyl]acetic acid

$^1$H-NMR (400 MHz, $CDCl_3$) δ 7.90 (d, J= 8.5 Hz, 1H), 7.63 (s, 1), 7.61 (s, 1H), 7.39 (dd, J= 8.5, 1.2 Hz, 1H), 7.34-7.26 (m, 3H), 7.18 (d, J= 8.6 Hz, 2H), 7.12-7.04 (m, 2H), 6.77 (d, J= 8.6 Hz, 2H), 5.38 (d, J= 15.8 Hz, 1H), 5.32 (d, J= 15.8 Hz, 1H), 4.54 (bs, 1H), 3.81 (d, J= 13.4 Hz, 1H), 3.69 (d, J= 13.4 Hz, 1H), 3.58 (s, 2H), 3.52-3.35 (m, 1H), 3.35-3.23 (m, 1H), 3.23-3.10 (m, 1H), 2.95-2.73 (m, 1H), 2.48-2.27 (m, 1H), 2.27-2.00 (m, 2H), 1.97-1.82 (m, 1H).
MS (ESI+) 578, 2.00($M^+$+1, detection time)

Example 585

{4-[(1-{2-[6-Cyano-1-(4-methoxybenzyl)-1H-indol-3-yl]-3,3,3-trifluoro-2-hydroxypropyl}piperidin-4-yl)oxy]phenyl}acetic acid

$^1$H-NMR (400 MHz, $CDCl_3$) δ 7.87 (d, J= 8.4 Hz, 1H), 7.65 (s, 1H), 7.60 (s, 1H), 7.39 (dd, J= 8.4, 1.3 Hz, 1H), 7.18 (d, J= 8.6 Hz, 2H), 7.06 (d, J= 8.6 Hz, 2H), 6.83 (d, J= 8.6 Hz, 2H), 6.77 (d, J= 8.6 Hz, 2H), 5.30 (d, J= 15.5 Hz, 1H), 5.25 (d, J= 15.5 Hz, 1H), 4.54 (bs, 1H), 3.81 (d, J= 13.5 Hz, 1H), 3.77 (s, 3H), 3.70 (d, J= 13.5 Hz, 1H), 3.57 (s, 2H), 3.54-3.36 (m, 1H), 3.36-3.23 (m, 1H), 3.23-3.10 (m, 1H), 2.95-2.72 (m, 1H), 2.50-2.30 (m, 1H), 2.25-2.02 (m, 2H), 1.97-1.84 (m, 1H). MS (ESI+) 608, 2.01($M^+$+1, detection time)

Example 586

3-{4-[(1-{2-[1-(4-Chlorophenyl)-6-nitro-1H-indol-3-yl]-3,3,3-trifluoro-2-hydroxypropyl}piperidin-4-yl)oxy]-3,5-dimethoxy-phenyl}propanoic acid

$^1$H-NMR (400 MHz, $CDCl_3$) δ 8.38 (d, J= 2.0 Hz, 1H), 8.13 (dd, J= 9.0, 2.0 Hz, 1H), 7.99 (d, J= 9.0 Hz, 1H), 7.85 (s, 1H), 7.59 (d, J= 8.7 Hz, 2H), 7.47 (d, J= 8.7 Hz, 2H), 6.71+6.38 (s, 2H), 4.44+4.32 (bs, 1H), 3.91 (d, J= 13.5 Hz, 1H), 3.82 (d, J= 13.5 Hz, 1H), 3.77+3.70 (s, 6H), 3.72-3.52 (m, 2H), 3.40-3.18 (m, 2H), 2.88 (t, J= 8.5 Hz, 2H), 2.64 (t, J= 8.5 Hz, 2H), 2.40-1.80 (m, 4H).
MS (ESI+) 692, 2.17($M^+$+1, detection time)

Example 587

3-{3,5-Dimethoxy-4-[(1-{3,3,3-trifluoro-2-hydroxy-2-[1-(4-methoxyphenyl)-6-nitro-1H-indol-3-yl]propyl}piperidin-4-yl)oxy]phenyl}propanoic acid

$^1$H-NMR (400 MHz, $CDCl_3$) δ 8.34 (d, J= 2.0 Hz, 1H), 8.11 (dd, J= 8.9, 2.0 Hz, 1H), 7.98 (d, J= 8.9 Hz, 1H), 7.83 (s, 1H), 7.41 (d, J= 8.8 Hz, 2H), 7.09 (d, J= 8.8 Hz, 2H), 6.70+6.38 (s, 2H), 4.45+4.32 (bs, 1H), 4.00-3.78 (m, 2H), 3.91 (s, 3H), 3.78-3.50 (m, 10H), 3.42-3.11 (m, 2H), 2.87 (t, J= 7.6 Hz, 2H), 2.64 (t, J= 7.6 Hz, 2H), 2.45-1.80 (m, 4H).
MS (ESI+) 688, 2.09($M^+$+1, detection time)

Example 588

{3-Methoxy-4-[(1-{3,3,3-trifluoro-2-hydroxy-2-[6-nitro-1-(1-phenylethyl)-1H-indol-3-yl]propyl}piperidin-4-yl)oxy]phenyl}acetic acid

$^1$H-NMR (400 MHz, $CDCl_3$) δ 8.29-8.23 (m, 1H), 8.08-8.02 (m, 1H), 7.98+7.82 (s, 1H), 7.86+7.79 (d, J= 9.0 Hz, 1H), 7.35-7.20 (m, 5H), 7.16-7.08 (m, 2H), 6.82-6.72 (m, 3H), 5.81-5.69 (m, 1H), 4.50-4.40 (m, 1H), 3.93-3.75 (m, 2H), 3.72+3.58 (s, 3H), 3.57 (s, 2H), 3.52-3.40 (m, 2H), 3.24-3.10 (m, 2H), 2.46-1.70 (m, 7H).
MS (ESI+) 642, 2.07($M^+$+1, detection time)

Example 589

(4-{[2-(1-Benzyl-6-nitro-1H-indol-3-yl)-3,3,3-trifluoro-2-hydroxypropyl]amino}-phenyl)acetic acid

$^1$H-NMR (400 MHz, $CDCl_3$) δ 8.24 (d, J= 2.0 Hz, 1H), 7.99 (d, J= 9.0 Hz, 1H), 7.89 (dd, J= 9.0, 2.0 Hz, 1H), 7.52 (s, 1H), 7.40-7.29 (m, 3H), 7.14-7.09 (m, 2H), 6.86 (d, J= 8.6 Hz, 2H), 6.48 (d, J= 8.6 Hz, 2H), 5.44 (s, 2H), 4.25 (d, J= 12.2 Hz, 1H), 4.15 (d, J= 12.2 Hz, 1H), 3.41 (s, 2H).
MS (ESI+) 514, 2.20($M^+$+1, detection time)

Example 590

3-[4-({1-[2-(1-Benzyl-5-nitro-1H-indol-3-yl)-3,3,3-trifluoro-2-hydroxypropyl]-piperidin-4-yl}oxy)-3-methoxyphenyl]propanoic acid

$^1$H-NMR (400 MHz, $CDCl_3$) δ 8.72 (s, 1H), 8.13 (dd, J= 9.2, 2.2 Hz, 1H), 7.66 (s, 1H), 7.37 (d, J= 9.2 Hz, 1H), 7.35-7.26 (m, 3H), 7.13-7.04 (m, 2H), 6.80-6.66 (m, 3H), 5.39 (s, 2H), 4.41 (bs, 1H), 3.84 (d, J= 13.1 Hz, 1H), 3.78 (d, J= 13.1 Hz, 1H), 3.69 (s, 3H), 3.52-3.32 (m, 2H), 3.22-2.95 (m, 2H), 2.89 (t, J= 7.5 Hz, 2H), 2.64 (t, J= 7.5 Hz, 2H), 2.40-1.80 (m, 4H).
MS (ESI+) 642, 2.05($M^+$+1, detection time)

Example 591

[4-({1-[2-(1-Benzyl-6-cyano-1H-indol-3-yl)-3-chloro-3,3-difluoro-2-hydroxypropyl]piperidin-4-yl}oxy)-3-methoxyphenyl]acetic acid

$^1$H-NMR (400 MHz, $CDCl_3$) δ 7.93 (d, J= 8.3 Hz, 1H), 7.67 (s, 1H), 7.63 (s, 1H), 7.40 (dd, J= 8.3, 1.3 Hz, 1H), 7.32-7.23 (m, 3H), 7.09-7.03 (m, 2H), 6.80-6.75 (m, 3H), 5.37 (s, 2H), 4.43 (bs, 1H), 3.94 (d, J= 14.7 Hz, 1H), 3.79 (d,

J= 14.7 Hz, 1H), 3.68 (s, 3H), 3.58 (s, 2H), 3.53-3.39 (m, 2H), 3.27-3.15 (m, 1H), 3.02-2.80 (m, 1H), 2.48-2.28 (m, 1H), 2.19-2.00 (m, 2H), 1.95-1.80 (m, 1H).
MS (ESI+) 624, 1.99($M^+$+1, detection time)

Example 592

{4-[(1-{2-[6-Cyano-1-(3,4-dimethoxybenzyl)-1H-indol-3-yl]-3,3,3-trifluoro-2-hydroxypropyl}piperidin-4-yl)oxy]-3-methoxyphenyl}acetic acid

$^1$H-NMR (400 MHz, $CDCl_3$) δ 7.88 (d, J= 8.4 Hz, 1H), 7.67 (s, 1H), 7.60 (s, 1H), 7.41 (dd, J= 8.4, 1.2 Hz, 1H), 6.83-6.76 (m, 4H), 6.68 (dd, J= 8.2, 2.0 Hz, 1H), 6.60 (d, J= 2.0 Hz, 1H), 5.31 (d, J= 15.4 Hz, 1H), 5.25 (d, J= 15.4 Hz, 1H), 4.44 (bs, 1H), 3.88-3.67 (s, 3H), 3.83 (s, 3H), 3.74 (s, 3H), 3.70 (s, 3H), 3.58 (s, 2H), 3.53-3.40 (m, 2H), 3.28-3.15 (m, 1H), 3.10-2.90 (m, 1H), 2.40-2.20 (m, 1H), 2.18-2.00 (m, 2H), 1.95-1.80 (m, 1H).
MS (ESI+) 668, 1.92($M^+$+1, detection time)

Example 593

3-[4-({1-[2-(1-Benzyl-6-cyano-1H-indol-3-yl)-3,3,3-trifluoro-2-hydroxypropyl]-piperidin-4-yl}oxy)-3-methoxyphenyl]propanoic acid

$^1$H-NMR (400 MHz, $CD_3OD$) δ 7.97 (d, J= 8.4 Hz, 1H), 7.88 (s, 1H), 7.84 (s, 1H), 7.34 (d, J= 8.4 Hz, 1H), 7.22-7.11 (m, 5H), 6.81-6.77 (m, 3H), 6.65 (d, J= 8.1 Hz, 1H), 5.43 (s, 2H), 4.33 (bs, 1H), 4.12 (d, J= 14.1 Hz, 1H), 3.80 (d, J= 14.1 Hz, 1H), 3.64 (s, 3H), 3.50-3.46 (m, 2H), 3.22-3.17 (m, 2H), 2.76 (t, J= 7.5 Hz, 2H), 2.48 (t, J= 7.5 Hz, 2H), 2.00-1.85 (m, 4H).
MS (ESI+) 622, 2.02($M^+$+1, detection time)

Example 594

1-[5-({4-[2-(1-Benzyl-6-nitro-1H-indol-3-yl)-3,3,3-trifluoro-2-hydroxypropyl]-piperazin-1-yl}carbonyl)pyridin-2-yl]piperidine-4-carboxylic acid

$^1$H-NMR (400 MHz, $CDCl_3$) δ 8.29 (d, J= 2.0 Hz, 1H), 8.16 (d, J= 2.3 Hz, 1H), 8.03 (dd, J= 9.0, 2.0 Hz, 1H), 7.92 (d, J= 9.0 Hz, 1H), 7.58 (s, 1H), 7.53 (dd, J= 8.9, 2.3 Hz, 1H), 7.36-7.29 (m, 3H), 7.12-7.10 (m, 2H), 6.64 (d, J= 8.9 Hz, 1H), 5.41 (s, 2H), 4.28-4.24 (m, 2H), 3.58 (bs, 4H), 3.26 (d, J= 13.7 Hz, 1H), 3.17 (d, J= 13.7 Hz, 1H), 3.07-3.00 (m, 2H), 2.56-2.50 (m, 5H), 2.01-1.98 (m, 2H), 1.75-1.65 (m, 2H).
MS (ESI+) 681, 2.20($M^+$+1, detection time)

Example 595

2-{4-[2-(1-Benzyl-6-nitro-1H-indol-3-yl)-3,3,3-trifluoro-2-hydroxypropyl]-piperazin-1-yl}pyrimidine-5-carboxylic acid

$^1$H-NMR (400 MHz, $CD_3OD$) δ 8.67 (s, 2H), 8.28 (d, J= 2.0 Hz, 1H), 7.97 (d, J= 9.0 Hz, 1H), 7.88 (dd, J= 9.0, 2.0 Hz, 1H), 7.25-7.17 (m, 3H), 7.17-7.10 (m, 2H), 5.46 (s, 3H), 3.62 (t, J= 5.4 Hz, 4H), 3.25-3.17 (m, 1H), 3.02 (d, J= 14.1 Hz, 1H), 2.50 (dt, J= 11.4, 5.4 Hz, 2H), 2.41 (dt, J= 11.4, 5.4 Hz, 2H).
MS (ESI+) 571, 2.33($M^+$+1, detection time)

Example 596

3-[4-({4-[2-(1-Benzyl-6-nitro-1H-indol-3-yl)-3,3,3-trifluoro-2-hydroxypropyl]-piperazin-1-yl}carbonyl)phenyl]propanoic acid

$^1$H-NMR (400 MHz, $CD_3OD$) δ 8.38 (d, J= 2.0 Hz, 1H), 8.04 (d, J= 9.0 Hz, 1H), 7.96 (dd, J= 9.0, 2.0 Hz, 1H), 7.82 (s, 1H), 7.37-7.17 (m, 9H), 5.55 (d, J= 15.7 Hz, 1H), 5.49 (d, J= 15.7 Hz, 1H), 3.54 (bs, 2H), 3.26 (d, J= 14.0 Hz, 1H), 3.20 (bs, 2H), 3.08 (d, J= 14.0 Hz, 1H), 2.93 (t, J= 7.7 Hz, 2H), 2.61-2.38 (m, 4H), 2.55 (t, J= 7.7 Hz, 2H).
MS (ESI+) 625, 2.31($M^+$+1, detection time)

Example 597

(2E)-3-[4-({4-(2-(1-Benzyl-6-nitro-1H-indol-3-yl)-3,3,3-trifluoro-2-hydroxypropyl]piperazin-1-yl}carbonyl)phenyl]acrylic acid

$^1$H-NMR (400 MHz, $CD_3OD$) δ 8.38 (d, J= 2.0 Hz, 1H), 8.05 (d, J= 9.0 Hz, 1H), 7.96 (dd, J= 9.0, 2.0 Hz, 1H), 7.82 (s, 1H), 7.62 (d, J= 8.2 Hz, 2H), 7.50 (d, J= 16.0 Hz, 1H), 7.35 (d, J= 8.2 Hz, 2H), 7.31-7.17 (m, 5H), 6.56 (d, J= 16.0 Hz, 1H), 5.55 (d, J= 15.7 Hz, 1H), 5.50 (d, J= 15.7 Hz, 1H), 3.59-3.55 (m, 2H), 3.27 (d, J= 14.0 Hz, 1H), 3.25-3.16 b(m, 2H), 3.08 (d, J= 14.0 Hz, 1H), 2.63-2.39 (m, 4H).
MS (ESI+) 623, 2.31($M^+$+1, detection time)

Example 598

6-({4-[2-(1-Benzyl-6-nitro-1H-indol-3-yl)-3,3,3-trifluoro-2-hydroxypropyl]-piperazin-1-yl}carbonyl)nicotinic acid

$^1$H-NMR (400 MHz, $CD_3OD$) δ 8.95 (s, 1H), 8.38-8.36 (m, 1H), 8.28 (s, 1H), 7.95 (d, J= 9.0 Hz, 1H), 7.86 (d, J= 9.0 Hz, 1H), 7.72 (s, 1H), 7.42 (d, J= 8.0 Hz, 1H), 7.30-7.08 (m, 5H), 5.55 (s, 2H), 3.50-3.48 (m, 2H), 3.16-3.10 (m, 3H), 2.99 (d, J= 14.0 Hz, 1H), 2.83-2.28 (m, 4H).
MS (ESI+) 598, 2.26($M^+$+1, detection time)

Example 599

3-(2-{4-[2-(1-Benzyl-6-nitro-1H-indol-3-yl)-3,3,3-trifluoro-2-hydroxypropyl]-piperazin-1-yl}pyrimidin-5-yl)propanoic acid

$^1$H-NMR (400 MHz, $CD_3OD$) δ 8.27 (d, J= 2.0 Hz, 1H), 8.10 (s, 1H), 7.96 (d, J= 9.0 Hz, 1H), 7.87 (dd, J= 9.0, 2.0 Hz, 1H), 7.24-7.09 (m, 5H), 5.45 (s, 2H), 3.47 (t, J= 5.1 Hz, 4H), 3.20-3.16 (m, 1H), 3.00 (d, J= 13.9 Hz, 1H), 2.62 (t, J= 7.2 Hz, 2H), 2.48 (dt, J= 11.5, 5.1 Hz, 2H), 2.41-2.36 (m, 2H), 2.38 (t, J= 7.2 Hz, 2H). MS (ESI+) 599, 2.23($M^+$+1, detection time)

Example 600

3-{4-[(1-{2-[6-Cyano-1-(cyclopropylmethyl)-1H-indol-3-yl]-3,3,3-trifluoro-2-hydroxypropyl}piperidin-4-yl)oxy]-3,5-dimethoxyphenyl}propanoic acid

$^1$H-NMR (400 MHz, $CD_3OD$) 6 8.01 (d, J= 8.4 Hz, 1H), 7.95 (s, 1H), 7.75 (s, 1H), 7.33 (d, J= 8.4 Hz, 1H), 6.50 (s, 2H), 4.14-4.12 (m, 2H), 3.97-3.95 (m, 1H), 3.75 (s, 6H), 3.30-3.28 (m, 1H), 3.17 (d, J= 13.8 Hz, 1H), 2.90-2.84 (m, 1H), 2.83 (t, J= 7.6 Hz, 2H), 2.51 (t, J= 7.6 Hz, 2H), 2.43-2.38 (m, 2H), 1.91-1.63 (m, 4H), 1.32-1.28 (m, 1H), 0.63-0.60 (m, 2H), 0.42-0.40 (m, 2H).
MS (ESI+) 616, 2.06($M^+$+1, detection time)

Example 601

4-({1-[2-(1-Benzyl-6-nitro-1H-indol-3-yl)-3,3,3-trifluoro-2-hydroxypropyl]-piperidin-4-yl}methyl)benzoic acid

$^1$H-NMR (400 MHz, $CD_3OD$) δ 8.47 (d, J= 2.0 Hz, 1H), 8.00 (d, J= 9.0 Hz, 1H), 7.995 (dd, J= 9.0, 2.0 Hz, 1H), 7.88 (d, J= 8.1 Hz, 2H), 7.82 (s, 1H), 7.38-7.15 (m, 7H), 5.52 (s, 2H), 3.25 (d, J= 13.8 Hz, 1H), 3.11 (d, J= 13.8 Hz, 1H), 2.93-2.88 (m, 1H), 2.67-2.52 (m, 3H), 2.39-2.34 (m, 1H), 2.17-2.11 (m, 1H), 1.87-1.83 (m, 1H), 1.53-1.11 (m, 4H).
MS (ESI+) 582, 2.11 ($M^+$+1, detection time)

Example 602

[4-({1-[2-(1-Benzyl-6-nitro-1H-indol-3-yl)-3,3,3-trifluoro-2-hydroxypropyl]-piperidin-4-yl}oxy)-3,5-dimethoxyphenyl](methylthio)acetic acid

MS (ESI+) 704, 2.16($M^+$+1, detection time)

Example 603

4-({1-[2-(1-Benzyl-6-nitro-1H-indol-3-yl)-3,3,3-triiluoro-2-hydroxypropyl]-piperidin-4-yl}methyl)-3-methoxybenzoic acid

MS (ESI+) 612, 2.13($M^+$+1, detection time)

Example 604

(2E)-3-[4-({1-[2-(1-Benzyl-6-nitro-1H-indol-3-yl)-3,3,3-trifluoro-2-hydroxypropyl]piperidin-4-yl}methyl)-3,5-dimethoxyphenyl]acrylic acid

$^1$H-NMR (400 MHz, $CD_3OD$) δ 8.33 (d, J= 2.0 Hz, 1H), 8.01 (d, J= 9.0 Hz, 1H), 7.95 (dd, J= 9.0, 2.0 Hz, 1H), 7.84 (s, 1H), 7.51 (d, J= 15.9 Hz, 1H), 7.39-7.23 (m, 3H), 7.15 (m, 2H), 6.78 (s, 2H), 6.44 (d, J= 15.9 Hz, 1H), 5.54 (s, 2H), 3.77 (s, 6H), 3.33-3.27 (m, 1H), 3.15 (d, J= 14.1 Hz, 1H), 2.92-2.89 (m, 1H), 2.66-2.60 (m, 1H), 2.53 (d, J= 7.0 Hz, 1H), 2.40-2.34 (m, 1H), 2.17-2.12 (m, 1H), 1.52-1.18 (m, 5H).
MS (ESI+) 668, 2.22($M^+$+1, detection time)

Example 605

3-[4-({1-[2-(1-Benzyl-6-nitro-1H-indol-3-yl)-3,3,3-trifluoro-2-hydroxypropyl]-piperidin-4-yl}methyl)-3,5-dimethoxyphenyl]propanoic acid

$^1$H-NMR (400 MHz, $CD_3OD$) δ 8.32 (d, J= 2.0 Hz, 1H), 8.01 (d, J= 9.0 Hz, 1H), 7.94 (dd, J= 9.0, 2.0 Hz, 1H), 7.84 (s, 1H), 7.30-7.25 (m, 3H), 7.16-7.14 (m, 2H), 6.43 (s, 2H), 5.50 (s, 2H), 3.32-3.28 (m, 1H), 3.16 (d, J= 13.8 Hz, 1H), 2.94-2.87 (m, 1H), 2.83 (t, J= 7.8 Hz, 2H), 2.64-2.60 (m, 1H), 2.49 (t, J= 7.8 Hz, 2H), 2.45 (d, J= 6.8 Hz, 2H), 2.42-2.33 (m, 1H), 2.17-2.08 (m, 1H), 1.50-1.43 (m, 2H), 1.34-1.28 (m, 2H), 1.19-1.16 (m, 1H).
MS (ESI+) 670, 2.19($M^+$+1, detection time)

Example 606

[4-({1-[2-(1-Benzyl-6-nitro-1H-indol-3-yl)-3,3,3-trifluoro-2-hydroxypropyl]-piperidin-4-yl}oxy)-3,5-dimethoxyphenyl]acetic acid

$^1$H-NMR (400 MHz, $CD_3OD$) δ 8.35 (d, J= 1.9 Hz, 1H), 8.04 (d, J= 8.7 Hz, 1H), 7.98 (dd, J= 8.7, 1.9 Hz, 1H), 7.86 (s, 1H), 7.32-7.16 (m, 5H), 6.57 (s, 2H), 5.54 (s, 2H), 3.96-3.85 (m, 1H), 3.74 (s, 6H), 3.59 (s, 2H), 3.31-3.25 (m, 1H), 3.18-3.13 (m, 1H), 2.97-2.71 (m, 2H), 2.43-2.30 (m, 2H), 1.79-1.54 (m, 4H).
MS (ESI+) 658, 2.12($M^+$+1, detection time)

Example 607

3-{2-[2-(1-Benzyl-6-nitro-1H-indol-3-yl)-3,3,3-trifluoro-2-hydroxypropyl]-1,2,3,4-tetrahydroisoquinolin-6-yl}propanoic acid

$^1$H-NMR (400 MHz, $CD_3OD$) δ 8.24 (d, J= 2.0 Hz, 1H), 7.93 (d, J= 9.0 Hz, 1H), 7.84 (dd, J= 9.0, 2.0 Hz, 1H), 7.75 (s, 1H), 7.19-7.03 (m, 5H), 6.79 (d, J= 7.7 Hz, 1H), 6.78 (s, 1H), 6.58 (d, J= 7.7 Hz, 1H), 5.41 (s, 2H), 3.55 (d, J= 15.2 Hz, 1H), 3.49 (d, J= 15.2 Hz, 1H), 3.33 (d, J= 13.9 Hz, 1H), 3.14 (d, J= 13.4 Hz, 1H), 2.73-2.47 (m, 4H), 2.70 (t, J= 7.7 Hz, 2H), 2.41 (t, J= 7.7 Hz, 2H).
MS (ESI+) 568, 2.22($M^+$+1, detection time)

Example 608

({2-[2-(1-Benzyl-6-nitro-1H-indol-3-yl)-3,3,3-trifluoro-2-hydroxypropyl]-1,2,3,4-tetrahydroisoquinolin-6-yl}oxy)acetic acid

$^1$H-NMR (400 MHz, $CDCl_3$) δ 8.33 (bs, 1H), 8.03 (bs, 2H), 7.75 (bs, 1H), 7.30-7.23 (m, 3H), 7.15 (bs, 2H), 6.70-6.64 (m, 3H), 5.49 (s, 2H), 4.50-4.41 (m, 3H), 4.12-3.97 (m, 1H), 3.41-3.29 (m, 2H), 2.90-2.74 (m, 4H).
MS (ESI+) 570, 2.19($M^+$+1, detection time)

Example 609

4-({2-[2-(1-Benzyl-6-nitro-1H-indol-3-yl)-3,3,3-trifluoro-2-hydroxypropyl]-1,2,3,4-tetrahydroisoquinolin-6-yl}oxy)butanoic acid

$^1$H-NMR (400 MHz, $CD_3OD$) δ 8.32 (d, J= 2.0 Hz, 1H), 8.02 (dd, J= 9.0, 2.0 Hz, 1H), 7.96 (d, J= 9.0 Hz, 1H), 7.67 (s, 2H), 7.45-7.32 (m, 3H), 7.16-7.13 (m, 2H), 6.72 (d, J= 8.4 Hz, 1H), 6.66 (dd, J= 8.4, 2.3 Hz, 1H), 6.62 (d, J= 2.3 Hz, 1H), 5.46 (s, 2H), 3.98 (t, J= 6.5 Hz, 2H), 3.64 (bs, 2H), 3.35 (s, 2H), 2.87-2.76 (m, 4H), 2.48 (t, J= 7.0 Hz, 2H), 2.07 (t, J= 7.0, 6.5 Hz, 2H).
MS (ESI+) 598, 2.26($M^+$+1, detection time)

Example 610

4-({2-[2-(1-Benzyl-6-nitro-1H-indol-3-yl)-3,3,3-trifluoro-2-hydroxypropyl]-1,2,3,4-tetrahydroisoquinolin-8-yl}oxy)butanoic acid

MS (ESI+) 598, 2.31($M^+$+1, detection time)

Example 611

3-{3,5-Dimethoxy-4-[(1-{3,3,3-trifluoro-2-[1-(2-fluorobenzyl)-6-nitro-1H-indol-3-yl]-2-hydroxypropyl}piperidin-4-yl)methyl]phenyl}propanoic acid

$^1$H-NMR (400 MHz, $CD_3OD$) δ 8.42 (d, J= 1.9 Hz, 1H), 8.01 (d, J= 9.0 Hz, 1H), 7.97 (dd, J= 9.0, 1.9 Hz, 1H), 7.94 (s, 1H), 7.35-7.30 (m, 1H), 7.17-7.08 (m, 3H), 6.44 (s, 2H), 5.58 (s, 2H), 3.71 (s, 6H), 3.31-3.27 (m, 1H), 3.15 (d, J= 13.7 Hz, 1H), 2.90-2.83 (m, 1H), 2.85 (t, J= 7.7 Hz, 2H), 2.61-2.55 (m, 1H), 2.53 (t, J= 7.7 Hz, 2H), 2.46 (d, J= 6.8 Hz, 2H), 2.40-2.34 (m, 1H), 2.16-2.11 (m, 1H), 1.49-1.14 (m, 5H).
MS (ESI+) 688, 2.09($M^+$+1, detection time)

Example 612

3-{3,5-Dimethoxy-4-[(1-{3,3,3-trifluoro-2-hydroxy-2-[1-(3-methoxybenzyl)-6-nitro-1H-indol-3-yl]propyl}piperidin-4-yl)methyl]phenyl}propanoic acid

$^1$H-NMR (400 MHz, $CDCl_3$) δ 8.33 (d, J= 2.0 Hz, 1H), 8.02 (d, J= 9.0 Hz, 1H), 7.96 (dd, J= 9.0, 2.0 Hz, 1H), 7.84 (s, 1H), 7.20 (dd, J= 8.1, 7.8 Hz, 1H), 6.81 (d, J= 8.1 Hz, 1H), 6.72 (d, J= 7.8 Hz, 1H), 6.67 (s, 1H), 6.44 (s, 2H), 5.50 (s, 2H), 3.72 (s, 6H), 3.67 (s, 3H), 3.30-3.25 (m, 1H), 3.13 (d, J= 13.7 Hz, 1H), 2.89-2.83 (m, 1H), 2.85 (t, J= 7.7 Hz, 2H), 2.61-2.56 (m, 1H), 2.52 (t, J= 7.7 Hz, 2H), 2.45 (d, J= 6.6 Hz, 2H), 2.39-2.34 (m, 1H), 2.14-2.08 (m, 1H), 1.49-1.28 (m, 5H)..
MS (ESI+) 700, 2.10($M^+$+1, detection time)

Example 613

(2E)-3-{2-[2-(1-Benzyl-6-nitro-1H-indol-3-yl)-3,3,3-trifluoro-2-hydroxypropyl]-5,7-dimethoxy-1,2,3,4-tetrahydroisoquinolin-6-yl}acrylic acid

MS (ESI+) 626, 2.41($M^+$+1, detection time)

Example 614

3-{2-[2-(1-Benzyl-6-nitro-1H-indol-3-yl)-3,3,3-trifluoro-2-hydroxypropyl]-5,7-dimethoxy-1,2,3,4-tetrahydroisoquinolin-6-yl}propanoic acid

$^1$H-NMR (400 MHz, $CD_3OD$) δ 8.33 (d, J= 2.0 Hz, 1H), 8.02 (d, J= 9.0 Hz, 1H), 7.92 (dd, J= 9.0, 2.0 Hz, 1H), 7.87 (s, 1H), 7.28-7.10 (m, 5H), 6.16 (s, 1H), 5.68 (s, 1H), 5.52 (s, 2H), 3.66 (s, 8H), 3.43 (d, J= 13.8 Hz, 1H), 3.23 (d, J= 13.8 Hz, 1H), 2.85 (t, J= 8.3 Hz, 2H), 2.79-2.59 (m, 4H), 2.40 (t, J= 8.3 Hz, 2H).
MS (ESI+) 628, 2.33($M^+$+1, detection time)

Example 615

3-[4-({1-[2-(1-Benzyl-6-nitro-1H-indol-3-yl)-3,3,3-trifluoro-2-hydroxypropyl]-piperidin-4-yl}carbonyl)-3,5-dimethoxyphenyl]propanoic acid

$^1$H-NMR (400 MHz, $CD_3OD$) δ 8.39 (d, J= 2.0 Hz, 1H), 8.06 (d, J= 9.1 Hz, 1H), 7.95 (dd, J= 9.1, 2.0 Hz, 1H), 7.82 (s, 1H), 7.38-7.24 (m, 3H), 7.17-7.15(m, 2H), 6.55 (s, 2H), 5.54 (s, 2H), 3.75 (s, 6H), 3.22 (d, J= 13.8 Hz, 1H), 3.06 (d, J= 13.8 Hz, 1H), 2.92-2.85 (m, 1H), 2.90 (t, J= 7.6 Hz, 2H), 2.65-2.54 (m, 2H), 2.56 (t, J= 7.6 Hz, 2H), 2.34-2.29 (m, 1H), 2.17-2.14 (m, 1H), 1.75-1.66 (m, 1H), 1.63-1.55 (m, 2H), 1.47-1.44 (m, 1H).
MS (ESI+) 684, 2.06($M^+$+1, detection time)

Example 616

3-[4-({1-[2-(1-Benzyl-6-nitro-1H-indol-3-yl)-3,3,3-trifluoro-2-hydroxypropyl]-azepan-4-yl}oxy)-3,5-dimethoxyphenyl]propanoic acid

$^1$H-NMR (400 MHz, $CD_3OD$) δ 8.34-8.33 (m, 1H), 8.02-7.94 (m, 2H), 7.84 (s, 1H), 7.33-7.15 (m, 5H), 6.50-6.48 (m, 2H), 5.50-5.49 (m, 2H), 4.15-4.07 (m, 1H), 3.71 (s, 3H), 3.69 (s, 3H), 3.54 (d, J= 13.9 Hz, 1H), 3.34-3.27 (m, 1H), 3.00-2.51 (m, 4H), 2.84 (t, J= 7.4 Hz, 2H), 2.53 (t, J= 7.4 Hz, 2H), 1.83-1.72 (m, 5H), 1.41-1.36 (m, 1H).
MS (ESI+) 686, 2.27($M^+$+1, detection time)

Example 617

(2E)-3-[4-({1-[2-(1-Benzyl-6-nitro-1H-indol-3-yl)-3,3,3-trifluoro-2-hydroxypropyl]azepan-4-yl}oxy)-3,5-dimethoxyphenyl]acrylic acid

$^1$H-NMR (400 MHz, $CDCl_3$) δ 8.34-8.33 (m, 1H), 8.00 (dd, J= 9.1, 2.8 Hz, 1H), 7.97-7.94 (m, 1H), 7.84 (d, J= 2.8 Hz, 1H), 7.50 (d, J= 15.9 Hz, 1H), 7.28-7.15 (m, 5H), 6.85-6.84 (m, 1H), 6.85 (d, J= 15.9 Hz, 1H), 7.28-7.15 (m, 5H), 6.85-6.84 (m, 1H), 6.85 (d, J= 15.9 Hz, 1H), 5.51 (s, 2H), 4.28-4.24 (m, 1H), 3.77 (s, 3H), 3.74 (s, 3H), 3.53 (d, J= 14.4 Hz, 1H), 3.28 (d, J= 14.4 Hz, 1H), 2.96-2.52 (m, 4H), 1.85-1.71 (m, 5H), 1.42-1.37 (m, 1H).
MS (ESI+) 684, 2.03($M^+$+1, detection time)

Example 618

(2E)-3-[4-({1-[2-(1-Benzyl-6-nitro-1H-indol-3-yl)-3,3,3-trifluoro-2-hydroxypropyl]piperidin-4-yl}methoxy)-3,5-dimethoxyphenyl]acrylic acid

$^1$H-NMR (400 MHz, $CD_3OD$) δ 8.34 (d, J= 2.0 Hz, 1H), 8.03 (d, J= 9.0 Hz, 1H), 7.96 (dd, J= 9.0, 2.0 Hz, 1H), 7.84 (s, 1H), 7.42 (d, J= 15.9 Hz, 1H), 7.32-7.16 (m, 5H), 6.84 (s, 2H), 6.40 (d, J= 15.9 Hz, 1H), 5.53 (s, 2H), 3.80 (s, 6H), 3.73 (d, J= 2.9 Hz, 2H), 3.32-3.30 (m, 1H), 3.12 (d, J= 13.6 Hz, 1H), 2.93-2.91 (m, 1H), 2.64-2.62 (m, 1H), 2.44-2.38 (m, 1H), 2.23-2.17 (m, 1H), 1.77-1.62 (m, 3H), 1.38-1.19 (m, 3H).
MS (ESI+) 684, 2.07($M^+$+1, detection time)

Example 619

3-[4-({1-[2-(1-Benzyl-6-nitro-1H-indol-3-yl)-3,3,3-trifluoro-2-hydroxypropyl]-piperidin-4-yl}methoxy)-3,5-dimethoxyphenyl]propanoic acid

$^1$H-NMR (400 MHz, $CD_3OD$) δ 8.34 (bs, 1H), 8.03 (d, J= 8.9 Hz, 1H), 7.96 (d, J= 8.9 Hz, 1H), 7.84 (s, 1H), 7.33-7.16 (m, 5H), 6.51 (s, 2H), 5.54 (s, 2H), 3.76 (s, 6H), 3.68-3.59 (m, 2H), 3.31-3.25 (m, 1H), 3.12 (d, J= 13.8 Hz, 1H), 2.93-2.90 (m, 1H), 2.84 (t, J= 7.5 Hz, 2H), 2.64-2.62 (m, 2H), 2.52 (t, J= 7.5 Hz, 2H), 2.44-2.39 (m, 1H), 2.23-2.17 (m, 1H), 1.77-1.62 (m, 3H), 1.36-1.14 (m, 3H).
MS (ESI+) 686, 2.29($M^+$+1, detection time)

Example 620

(2E)-3-{4-[{1-(2-(1-Benzyl-6-nitro-1H-indol-3-yl)-3,3,3-trifluoro-2-hydroxypropyl]piperidin-4-yl}(methoxy)methyl]-3,5-dimethoxyphenyl}acrylic acid $^1$H-NMR (400 MHz, $CD_3OD$) δ 8.34+8.31 (d, J= 2.0 Hz, 1H), 8.03-7.93 (m, 2H), 7.84+7.83 (s, 1H), 7.52 (d, J= 15.9 Hz, 1H), 7.33-7.09 (m, 5H), 6.82 (s, 2H), 6.50 (d, J= 15.9 Hz, 1H), 5.54 (s, 1H), 5.51 (s, 1H), 4.70 (d, J= 9.6 Hz, 1H), 3.80 (s, 3H), 3.79 (s, 3H), 3.41-3.26 (m, 5.5H), 3.15-3.09 (m, 1H), 3.01-2.98 (m, 0.5H), 2.82-2.79 (m, 0.5H), 2.72-2.68 (m, 0.5H), 2.47-2.44 (m, 1H), 2.30-1.95 (m, 4H), 1.16-0.83 (m, 1H).

Example 621

(2E)-3-[4-({1-[2-(1-Benzyl-6-nitro-1H-indol-3-yl)-3,3,3-trifluoro-2-hydroxypropyl]azepan-4-yl}oxy)-3-methoxyphenyl] acrylic acid

$^1$H-NMR (400 MHz, $CD_3OD$) δ 8.37-8.36 (m, 1H), 8.00 (d, J= 9.0 Hz, 1H), 7.97-7.95 (m, 1H), 7.82+7.81 (s, 1H), 7.52+7.51 (d, J= 15.9 Hz, 1H), 7.26-7.15 (m, 5H), 7.04+6.98 (d, J= 8.4 Hz, 1H), 6.73+6.58 (d, J= 8.4 Hz, 1H), 6.35 (d, J= 15.9 Hz, 1H), 5.50 (s, 1H), 5.49 (s, 1H), 4.42-4.37 (m, 0.5H), 4.36-4.29 (m, 0.5H), 3.83+3.81 (s, 3H), 3.59-3.48 (m, 1H), 3.27-3.22 (m, 1H), 2.81-2.70 (m, 4H), 1.82-1.65 (m, 5H), 1.42-1.37 (m, 1H).
MS (ESI+) 654, 2.36($M^+$+1, detection time)

Example 622

3-[4-({1-[2-(1-Benzyl-6-nitro-1H-indol-3-yl)-3,3,3-trifluoro-2-hydroxypropyl]-azepan-4-yl}oxy)-3-methoxyphenyl]propanoic acid

$^1$H-NMR (400 MHz, $CD_3OD$) δ 8.36-8.35 (m, 1H), 8.01-7.94 (m, 2H), 7.81 (s, 2H), 7.27-7.16 (m, 5H), 6.83-6.82 (m, 1H), 6.68-6.54 (m, 2H), 5.49 (s, 2H), 4.31-4.09 (m, 1H), 3.82 (s, 3H), 3.50+3.48 (d, J= 14.0 Hz, 1H), 3.25+3.23 (d, J= 14.0 Hz, 1H), 2.85-2.66 (m, 6H), 2.51 (t, J= 7.7 Hz, 2H), 1.80-1.64 (m, 5H), 1.41-1.30 (m, 1H).
MS (ESI+) 656, 2.05($M^+$+1, detection time)

Example 623

(2E)-3-[4-({1-[2-(1-Benzyl-6-nitro-1H-indol-3-yl)-3,3,3-trifluoro-2-hydroxypropyl]piperidin-4-yl}oxy)-3-fluoro-5-methoxyphenyl]acrylic acid

$^1$H-NMR (400 MHz, $CD_3OD$) δ 8.34 (d, J= 2.0 Hz, 1H), 8.04 (d, J= 9.0 Hz, 1H), 7.97 (dd, J= 9.0, 2.0 Hz, 1H), 7.84 (s, 1H), 7.35 (d, J= 15.9 Hz, 1H), 7.32-7.23 (m, 3H), 7.17 (d, J= 6.8 Hz, 1H), 6.98-6.93 (m, 2H), 6.41 (d, J= 15.9 Hz, 1H), 5.53 (s, 2H), 4.14-4.07 (m, 1H), 3.90 (s, 3H), 3.25 (d, J= 13.8 Hz, 1H), 3.10 (d, J= 13.8 Hz, 1H), 2.84-2.75 (m, 2H), 2.39-2.32 (m, 2H), 1.74-1.62 (m, 4H).
MS (ESI+) 658, 2.13($M^+$+1, detection time)

Example 624

3-[4-({1-[2-(1-Benzyl-6-nitro-1H-indol-3-yl)-3,3,3-trifluoro-2-hydroxypropyl]-piperidin-4-yl}oxy)-3-fluoro-5-methoxyphenyl]propanoic acid

MS (ESI+) 660, 2.09($M^+$+1, detection time)

Example 625

6-({1-[2-(1-Benzyl-6-nitro-1H-indol-3-yl)-3,3,3-trifluoro-2-hydroxypropyl]-piperidin-4-yl}oxy)-5-chloronicotinic acid

$^1$H-NMR (400 MHz, $CD_3OD$) δ 8.56 (d, J= 2.0 Hz, 1H), 8.36 (d, J= 2.0 Hz, 1H), 8.17 (d, J= 2.0 Hz, 1H), 8.06 (d, J= 9.0 Hz, 1H), 7.97 (dd, J= 9.0, 2.0 Hz, 1H), 7.85 (s, 1H), 7.32-7.18 (m, 5H), 5.54 (s, 2H), 5.15-5.13 (m, 1H), 3.31-3.27 (m, 1H), 3.13 (d, J= 13.8 Hz, 1H), 2.80-2.73 (m, 2H), 2.55-2.44 (m, 2H), 1.91-1.87 (m, 2H), 1.28-1.22 (m, 2H).
MS (ESI+) 619, 2.31($M^+$+1, detection time)

Example 626

[4-({(3-Exo)-8-[2-(1-benzyl-6-nitro-1H-indol-3-yl)-3,3,3-trifluoro-2-hydroxypropyl]-8-azabicyclo[3.2.1]oct-3-yl}oxy)-3-methoxyphenyl]acetic acid

$^1$H-NMR (400 MHz, $CD_3OD$) δ 8.26 (d, J= 1.9 Hz, 1H), 7.94 (d, J= 9.0 Hz, 1H), 7.87 (dd, J= 9.0, 1.9 Hz, 1H), 7.76 (s, 1H), 7.19-7.06 (m, 5H), 6.81 (d, J= 1.8 Hz, 1H), 6.74 (d, J= 8.2 Hz, 1H), 6.66 (dd, J= 8.2, 1.8 Hz, 1H), 5.43 (s, 2H), 4.28 (dddd, J= 10.6, 10.6, 4.9, 4.9 Hz, 1H), 3.69 (s, 3H), 3.38 (s, 2H), 3.26-3.21 (m, 1H), 3.05 (d, J= 13.5 Hz, 1H), 3.00 (bs, 1H), 2.86 (bs, 1H), 1.91-1.41 (m, 9H).

Example 627

(2E)-3-[4-({(3-Exo)-8-[2-(1-benzyl-6-nitro-1H-indol-3-yl)-3,3,3-trifluoro-2-hydroxypropyl]-8-azabicyclo[3.2.1]oct-3-yl}oxy)-3,5-dimethoxyphenyl]acrylic acid

$^1$H-NMR (400 MHz, $CD_3OD$) δ 8.25 (d, J= 1.9 Hz, 1H), 7.94 (d, J= 9.0 Hz, 1H), 7.88 (dd, J= 9.0, 1.9 Hz, 1H), 7.78 (s, 1H), 7.42 (d, J= 15.9 Hz, 1H), 7.21-7.06 (m, 3H), 7.07 (d, J= 8.1Hz, 2H), 6.77 (s, 2H), 6.31 (d, J= 15.9 Hz, 1H), 5.45 (s, 2H), 4.23-4.15 (m, 1H), 3.26-3.20 (m, 1H), 3.20 (s, 6H), 3.05 (d, J= 13.6 Hz, 1H), 3.00 (bs, 1H), 2.86 (bs, 1H), 1.77-1.30 (m, 9H).
MS (ESI+) 696, 2.14($M^+$+1, detection time)

Reference Example 6

6-Nitro-3-[1-(trifluoromethyl)vinyl]-1H-indole

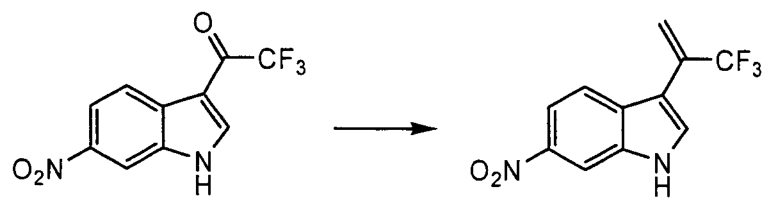

The title compound was obtained from the compound of Reference Example 1 as a starting compound in a similar manner to Example 15.

Reference Example 7

6-Nitro-1-phenyl-3-[1-(trifluoromethyl)vinyl]-1H-indole

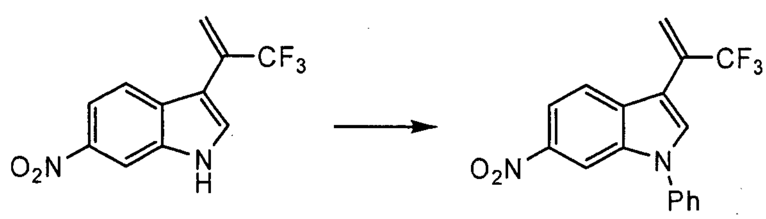

A solution of the compound of Reference Example 6 (256 mg), iodobenzene (245 mg), copper (I) iodide (9.52 mg), rac-trans-N,N'-dimethylcyclohexane-1,2-diamine (14.2 mg), potassium phosphate tribasic (446 mg) in toluene (5 ml) was stirred at 110˚C for 8 hours. The reaction solution was filtered through celite, and the filtrate was concentrated under reduced pressure. Water was added to the filtrate, and the mixture was extracted with ethyl acetate. The organic layer was washed with a saturated saline solution, dried over sodium sulfate and filtered. The filtrate was concentrated under

reduced pressure, and the obtained residue was purified by silica gel column chromatography to give the title compound. $^1$H-NMR (400 MHz, $CDCl_3$) δ 8.43 (d, J= 2.0 Hz, 1H), 8.15 (dd, J= 8.9, 2.0 Hz, 1H), 7.89 (d, J= 8.9 Hz, 1H), 7.76-7.73 (m, 1H), 7.65-7.59 (m, 2H), 7.55-7.49 (m 3H), 6.16-6.12 (m, 1H), 6.03-5.99 (m, 1H).

Reference Example 8

6-Nitro-1-phenyl-3-[2-(trifluoromethyl)oxiran-2-yl]-1H-indole

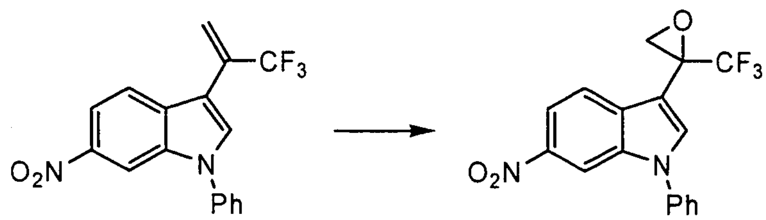

To a solution of the compound of Reference Example 7 (2.02 g), dimethylaminopyridine (118 mg), (s, s)-Jacobsene reagent (193 mg) in a mixed solvent of dichloromethane (18 ml) - N,N-dimethylformamide (54 ml) was added dropwise aqueous hydrogen peroxide solution (31%, 39.96 g) at 0˚C. After the addition, the mixture was stirred for 30 minutes, and warmed to 25˚C. To the reaction solution was added aqueous sodium thiosulfate solution, and the mixture was stirred at 25˚C for one hour. The organic layer was extracted with ethyl acetate, and the organic layer was washed with a saturated saline solution, dried over sodium sulfate and filtered. The filtrate was concentrated under reduced pressure, and the obtained residue was purified by silica gel column chromatography to give the title compound (754 mg).
$^1$H-NMR (400 MHz, $CDCl_3$) δ 8.41 (d, J= 2.0 Hz, 1H), 8.12 (dd, J= 8.9, 2.0 Hz, 1H), 7.88 (d, J= 8.9 Hz, 1H), 7.80 (s, 1H), 7.65-7.58 (m, 2H), 7.55-7.47 (m, 3H), 3.55 (d, J= 5.2 Hz, 1H), 3.24-3.18 (m, 1H).
MS (ESI+) 349, 2.75($M^+$+1, detection time)

Example 628

Ethyl [3-methoxy-4-({1-[3,3,3-trifluoro-2-hydroxy-2-(6-nitro-1-phenyl-1H-indol-3-yl)propyl]piperidin-4-yl}oxy)phenyl]acetate

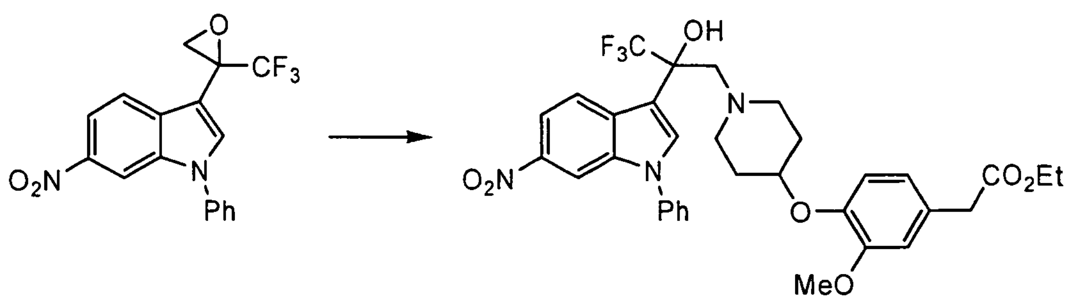

The title compound was obtained from the compound of Reference Example 8 as a starting compound in a similar manner to Example 1.
$^1$H-NMR (400 MHz, $CDCl_3$) δ 8.42 (d, J= 1.9 Hz, 1H), 8.08 (dd, J= 9.0, 1.9 Hz, 1H), 7.98 (d, J= 9.0 Hz, 1H), 7.75 (s, 1H), 7.64-7.55 (m, 2H), 7.53-7.45 (m, 3H), 6.85-6.72 (m, 3H), 5.96 (bs, 1H), 4.25 (bs, 1H), 4.14 (q, J= 7.1 Hz, 2H), 3.83 (s, 3H), 3.53 (s, 2H), 3.33 (d, J= 13.8 Hz, 1H), 3.17 (d, J= 13.8 Hz, 1H), 2.95-2.80 (m, 2H), 2.60-2.45 (m, 2H), 2.00-1.75 (m, 4H), 1.25 (t, J= 7.1 Hz, 3H).
MS (ESI+) 642, 2.50($M^+$+1, detection time)

Example 629

[3-Methoxy-4-({1-[3,3,3-trifluoro-2-hydroxy-2-(6-nitro-1-phenyl-1H-indol-3-yl)-propyl]piperidin-4-yl}oxy)phenyl]acetic acid

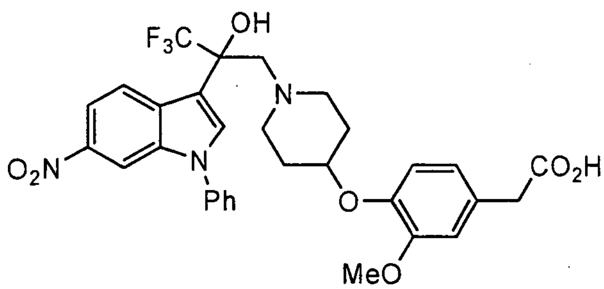

The title compound was obtained from the compound of Example 628 as a starting compound in a similar manner to Example 4.
$^1$H-NMR (400 MHz, $CDCl_3$) δ 8.43 (s, 1H), 8.12 (d, J= 8.7 Hz, 1H), 7.99 (d, J= 8.7 Hz, 1H), 7.87 (s, 1H), 7.68-7.58 (m, 2H), 7.58-7.46 (m, 3H), 6.78 (s, 3H), 4.49 (bs, 1H), 3.95 (d, J= 12.6 Hz, 1H), 3.84 (d, J= 12.6 Hz, 1H), 3.78-3.45 (m, 2H), 3.71 (s, 3H), 3.57 (s, 2H), 3.45-3.30 (m, 1H), 3.15-2.95 (m, 1H), 2.50-1.80 (m, 4H).
MS (ESI+) 614, 2.37($M^+$+1, detection time)

Example 630

3-Anilino-2-(1-benzyl-6-nitro-1H-indol-3-yl)-1,1,1-trifluoropropan-2-ol

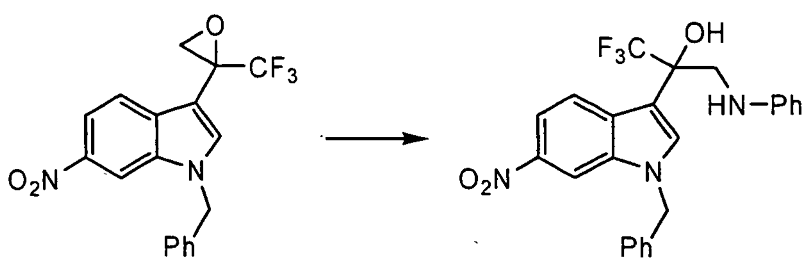

To a solution of the compound of Reference Example 3 (36 mg) in chloroform (1 ml) were added aniline (18.6 mg) and lithium bis(trifluoromethanesulfonyl)imide (14.3 mg), and the mixture was stirred at 25°C for 3 days. The obtained reaction solution was purified by silica gel column chromatography to give the title compound (38 mg).
$^1$H-NMR (400 MHz, $CDCl_3$) δ 8.24 (d, J= 2.0 Hz, 1H), 8.01 (d, J= 9.0 Hz, 1H), 7.90 (dd, J= 9.0, 2.0 Hz, 1H), 7.55-7.51 (m, 1H), 7.40-7.30 (m, 3H), 7.14-7.09 (m, 2H), 7.00-6.93 (m, 2H), 6.73-6.67 (m, 1H), 6.57-6.51 (m, 2H), 5.44 (s, 2H), 4.96 (bs, 1H), 4.28 (dd, J= 12.3, 3.4 Hz, 1H), 4.21-4.11 (m, 1H), 2.13-2.04 (m, 1H).
MS (ESI+) 456, 2.43($M^+$+1, detection time)

In a similar manner to the preparation of the compound of Example 630, the compounds of Examples 631 to 645 having a chemical structure as disclosed in Tables 40 to 41 were obtained.

[Table 40]

| | |
|---|---|
| 631 | 632 |
| 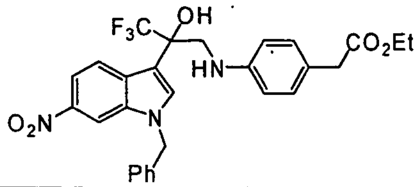 | 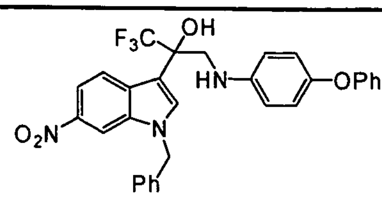 |
| 633 | 634 |
| 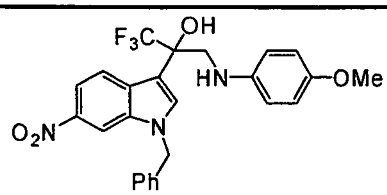 | 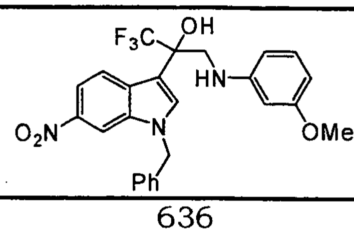 |
| 635 | 636 |
| 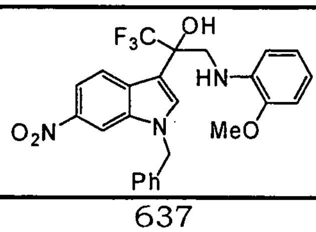 | 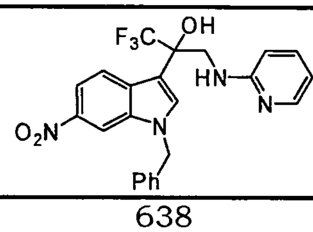 |
| 637 | 638 |
| 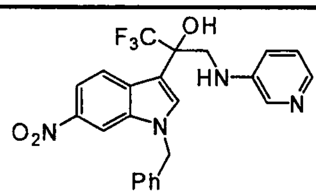 | 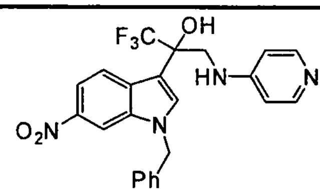 |
| 639 | 640 |
| 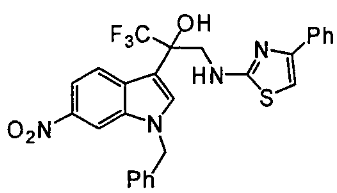 | 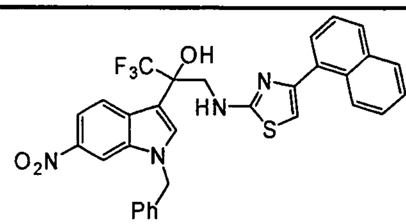 |

[Table 41]

| 641 | 642 |
|---|---|
| 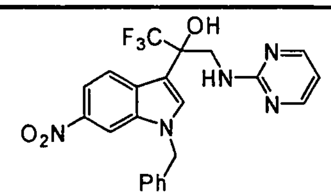 | 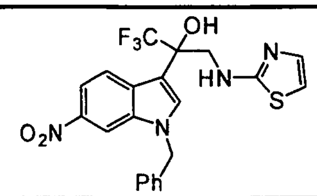 |
| 643 | 644 |
| 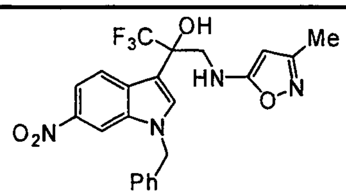 | 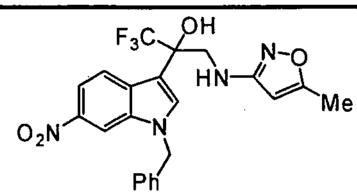 |
| 645 | |
| 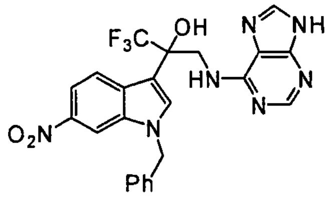 | |

Example 631

Ethyl (4-{[2-(1-benzyl-6-nitro-1H-indol-3-yl)-3,3,3-trifluoro-2-hydroxypropyl]-amino}phenyl) acetate

MS (ESI+) 542, 2.46($M^+$+1, detection time)

Example 632

2-(1-Benzyl-6-nitro-1H-indol-3-yl)-1,1,1-trifluoro-3-[(4-phenoxyphenyl)amino]-propan-2-ol

MS (ESI+) 548, 2.67($M^+$+1, detection time)

Example 633

2-(1-Benzyl-6-nitro-1H-indol-3-yl)-1,1,1-trifluoro-3-[(4-methoxyphenyl)amino]-propan-2-ol

$^1$H-NMR (400 MHz, $CDCl_3$) δ 8.23 (d, J= 1.9 Hz, 1H), 8.09 (d, J= 9.0 Hz, 1H), 7.91 (dd, J= 9.0, 1.9 Hz, 1H), 7.49 (s, 1H), 7.39-7.26 (m, 3H), 7.14-7.05 (m, 2H), 6.55 (d, J= 9.1 Hz, 2H), 6.50 (d, J= 9.1 Hz, 2H), 5.40 (s, 2H), 4.69 (bs, 1H), 4.22 (d, J= 12.0 Hz, 1H), 4.13 (d, J= 12.0 Hz, 1H), 3.64 (s, 1H).
MS (ESI+) 486, 2.40($M^+$+1, detection time)

Example 634

2-(1-Benzyl-6-nitro-1H-indol-3-yl)-1,1,1-trifluoro-3-[(3-methoxyphenyl)amino]-propan-2-ol

$^1$H-NMR (400 MHz, $CDCl_3$) δ 8.23 (d, J= 1.9 Hz, 1H), 8.00 (d, J= 9.0 Hz, 1H), 7.89 (d, J= 9.0 Hz, 1H), 7.53 (s, 1H), 7.40-7.28 (m, 3H), 7.12-7.10 (m, 2H), 6.88 (dd, J= 8.1, 8.1 Hz, 1H), 6.24 (dd, J= 8.1, 2.0 Hz, 1H), 6.17 (dd, J= 8.1, 2.0 Hz, 1H), 6.08 (s, 1H), 5.43 (s, 2H), 4.98 (bs, 1H), 4.27 (d, J= 12.0 Hz, 1H), 4.17 (d, J= 12.0 Hz, 1H), 3.52 (s, 3H).
MS (ESI+) 486, 2.40($M^+$+1, detection time)

Example 635

2-(1-Benzyl-6-nitro-1H-indol-3-yl)-1,1,1-trifluoro-3-[(2-methoxyphenyl)amino]-propan-2-ol

$^1$H-NMR (400 MHz, $CDCl_3$) δ 8.23 (d, J= 2.0 Hz, 1H), 7.95 (d, J= 9.0 Hz, 1H), 7.88 (dd, J= 9.0, 2.0 Hz, 1H), 7.53 (s, 1H), 7.40-7.29 (m, 3H), 7.14-7.08 (m, 2H), 6.80 (dd, J= 7.9, 1.3 Hz, 1H), 6.66 (ddd, J= 7.9, 7.9, 1.3 Hz, 1H), 6.42 (ddd, J= 7.9, 7.9, 1.3 Hz, 1H), 6.25 (d, J= 7.9 Hz, 1H), 5.61 (s, 1H), 5.44 (s, 2H), 4.35-4.25 (m, 1H), 4.25-4.15 (m, 1H), 3.94 (s, 3H), 2.20-2.10 (m, 1H).
MS (ESI+) 486, 2.41($M^+$+1, detection time)

Example 636

2-(1-Benzyl-6-nitro-1H-indol-3-yl)-1,1,1-trifluoro-3-(pyridin-2-ylamino)propan-2-ol

MS (ESI+) 457, 1.86($M^+$+1, detection time)

Example 637

2-(1-Benzyl-6-nitro-1H-indol-3-yl)-1,1,1-trifluoro-3-(pyridin-3-ylamino)propan-2-ol

MS (ESI+) 457, 1.88($M^+$+1, detection time)

Example 638

2-(1-Benzyl-6-nitro-1H-indol-3-yl)-1,1,1-trifluoro-3-(pyridin-4-ylamino)propan-2-ol

MS (ESI+) 457, 1.89($M^+$+1, detection time)

Example 639

2-(1-Benzyl-6-nitro-1H-indol-3-yl)-1,1,1-trifluoro-3-[(4-phenyl-1,3-thiazol-2-yl)aminolpropan-2-ol

MS (ESI+) 539, 2.50($M^+$+1, detection time)

Example 640

2-(1-Benzyl-6-nitro-1H-indol-3-yl)-1,1,1-trifluoro-3-{[4-(1-naphthyl)-1,3-thiazol-2-yl]amino}propan-2-ol

MS (ESI+) 589, 2.6 1 ($M^+$+1, detection time)

Example 641

2-(1-Benzyl-6-nitro-1H-indol-3-yl)-1,1,1-trifluoro-3-(pyrimidin-2-ylamino)propan-2-ol

MS (ESI+) 458, 2.25($M^+$+1, detection time)

Example 642

2-(1-Benzyl-6-nitro-1H-indol-3-yl)-1,1,1-trifluoro-3-(1,3-thiazol-2-ylamino)-propan-2-ol

MS (ESI+) 463, 2.18($M^+$+1, detection time)

Example 643

2-(1-Benzyl-6-nitro-1H-indol-3-yl)-1,1,1-trifluoro-3-[(3-methylisoxazol-5-yl)-amino]propan-2-ol

MS (ESI+) 461, 2.23($M^+$+1, detection time)

Example 644

2-(1-Benzyl-6-nitro-1H-indol-3-yl)-1,1,1-trifluoro-3-[(5-methylisoxazol-3-yl)-amino]propan-2-ol

MS (ESI+) 461, 2.26($M^+$+1, detection time)

Example 645

2-(1-Benzyl-6-nitro-1H-indol-3-yl)-1,1,1-trifluoro-3-(9H-purin-6-ylamino)-propan-2-ol

MS (ESI+) 498, 2.12($M^+$+1, detection time)

Example 646

2-{4-[2-(1-Benzyl-6-nitro-1H-indol-3-yl)-3,3,3-trifluoro-2-hydroxypropyl]-piperazin-1-yl}-1-phenylethanone

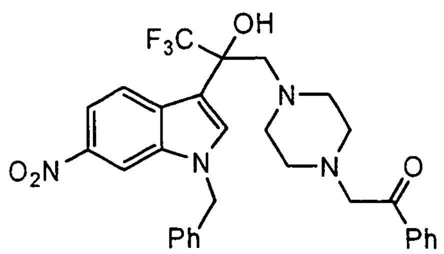

To a solution of the compound of Example 101 (65 mg) in N,N-dimethylformamide (3 ml) were added potassium carbonate (138 mg) and 2-bromoacetophenone (30 mg), and the mixture was stirred at 60°C for 8 hours. Water was added to the reaction solution, and the mixture was extracted with ethyl acetate. The organic layer was washed with a saturated saline solution , dried over sodium sulfate and filtered. The filtrate was concentrated under reduced pressure, and the obtained residue was purified by silica gel column chromatography to give the title compound.
MS (ESI+) 567, 2.06($M^+$+1, detection time)

In a similar manner to the preparation of the compound of Example 646, the compounds of Examples 647 to 648 having a chemical structure as disclosed in Table 42 were obtained.

[Table 42]

| 647 | 648 |
|---|---|
| 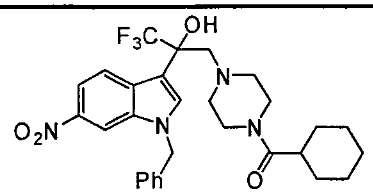 | 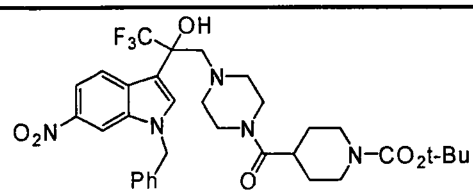 |

Example 647

2-(1-Benzyl-6-nitro-1H-indol-3-yl)-3-[4-(cyclohexylcarbonyl)piperazin-1-yl]-1,1,1-trifluoropropan-2-ol

$^1$H-NMR (400 MHz, $CDCl_3$) δ 8.29 (d, J= 2.0 Hz, 1H), 8.04 (dd, J= 9.0, 2.0 Hz, 1H), 7.90 (d, J= 9.0 Hz, 1H), 7.56 (s, 1H), 7.39-7.29 (m, 3H), 7.10 (dd, =7.8, 1.9 Hz, 2H), 5.40 (s, 2H), 3.68-3.50 (m, 2H), 3.50-3.34 (m, 2H), 3.26 (d, J= 13.7 Hz, 1H), 2.63-2.43 (m, 4H), 2.42-2.31 (m, 1H), 1.90-1.12 (m, 10H).
MS (ESI+) 559, 2.49($M^+$+1, detection time)

Example 648

tert-Butyl 4-({4-[2-(1-benzyl-6-nitro-1H-indol-3-yl)-3,3,3-trifluoro-2-hydroxypropyl]piperazin-1-yl}carbonyl)piperidine-1-carboxylate

MS (ESI+) 660, 2.50($M^+$+1, detection time)

In a similar manner to the preparation of the compound of Example 1, the compounds of Examples 649 to 656 having a chemical structure as disclosed in Table 43 were obtained.

[Table 43]

| 649 | 650 |
|---|---|
| 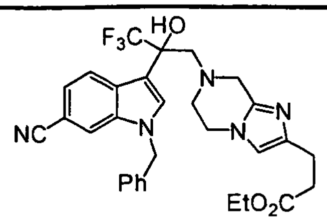 | 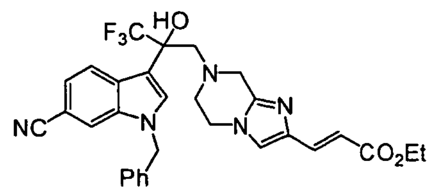 |
| 651 | 652 |
| 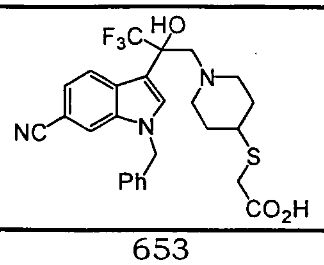 | 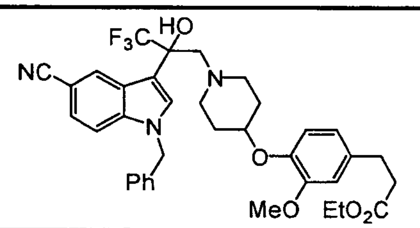 |
| 653 | 654 |
| 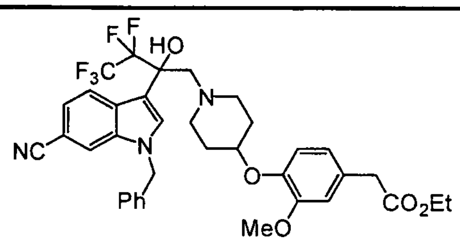 | 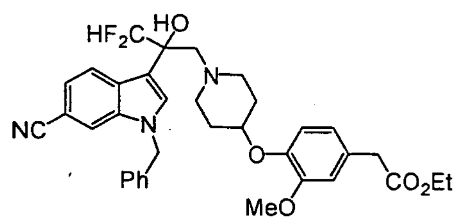 |
| 655 | 656 |
| 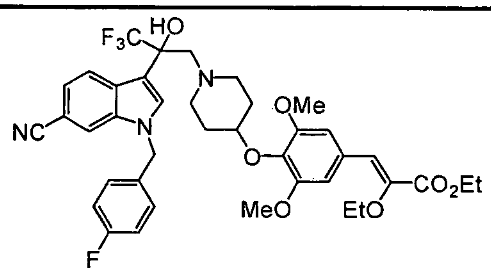 | 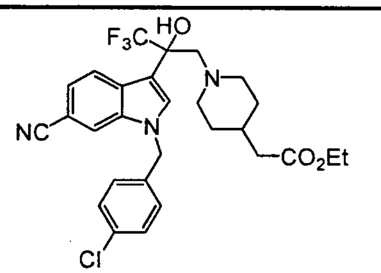 |

Example 649

Ethyl 3-{7-[2-(1-benzyl-6-cyano-1H-indol-3-yl)-3,3,3-trifluoro-2-hydroxypropyl]-5,6,7,8-tetrahydroimidazo[1,2-a]pyrazin-2-yl}propanoate

MS (ESI+) 566, 1.98($M^+$+1, detection time)

Example 650

Ethyl (2E)-3-{7-[2-(1-benzyl-6-cyano-1H-indol-3-yl)-3,3,3-trifluoro-2-hydroxypropyl]-5,6,7,8-tetrahydroimidazo [1,2-a] pyrazin-2-yl}acrylate

MS (ESI+) 564, 2.15($M^+$+1, detection time)

Example 651

({1-[2-(1-Benzyl-6-cyano-1 1H-indol-3-yl)-3,3,3-trifluoro-2-hydroxypropyl]-piperidin-4-yl}thio)acetic acid

MS (ESI+) 518, 1.91($M^+$+1, detection time)

Example 652

Ethyl 3-[4-({1-[2-(1-benzyl-5-cyano-1H-indol-3-yl)-3,3,3-trifluoro-2-hydroxypropyl]piperidin-4-yl}oxy)-3-methoxyphenyl] propanoate

MS (ESI+) 650, 2.16($M^+$+1, detection time)

Example 653

Ethyl [4-({1-[2-(1-benzyl-6-cyano-1H-indol-3-yl)-3,3,4,4,4-pentafluoro-2-hydroxybutyl]piperidin-4-yl}oxy)-3-methoxyphenyl]acetate

MS (ESI+) 686, 2.26($M^+$+1, detection time)

Example 654

Ethyl [4-({1-[2-(1-benzyl-6-cyano-1H-indol-3-yl)-3,3-difluoro-2-hydroxypropyl]-piperidin-4-yl}oxy)-3-methoxyphenyl]acetate

MS (ESI+) 618, 2.05($M^+$+1, detection time)

Example 655

Ethyl (2Z)-3-{4-[(1-{2-[6-cyano-1-(4-fluorobenzyl)-1H-indol-3-yl]-3,3,3-trifluoro-2-hydroxypropyl}piperidin-4-yl)oxy]-3,5-dimethoxyphenyl}-2-ethoxyacrylate

MS (ESI+) 740, 2.26($M^+$+1, detection time)

Example 656

Ethyl (1-{2-[1-(4-chlorobenzyl)-6-cyano-1H-indol-3-yl]-3,3,3-trifluoro-2-hydroxypropyl}piperidin-4-yl)acetate

MS (ESI+) 548, 2.03($M^+$+1, detection time)
In a similar manner to the preparation of the compound of Example 4, the compounds of Examples 657 to 663 having a chemical structure as disclosed in Table 44 were obtained.
[Table 44]

| 657 | 658 |
|---|---|
| 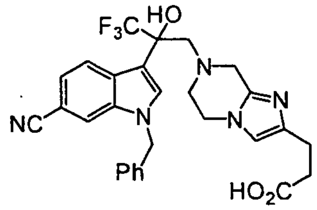 | 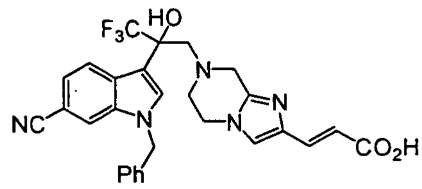 |
| 659 | 660 |
| 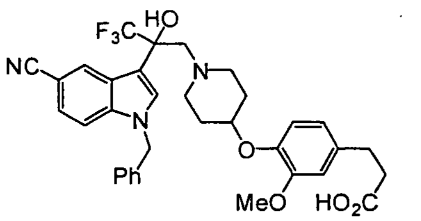 | 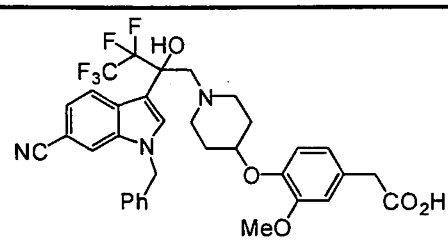 |
| 661 | 662 |
| 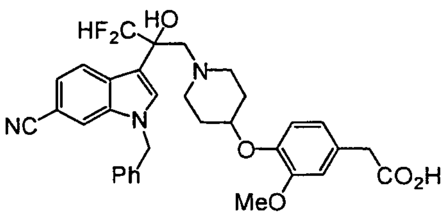 | 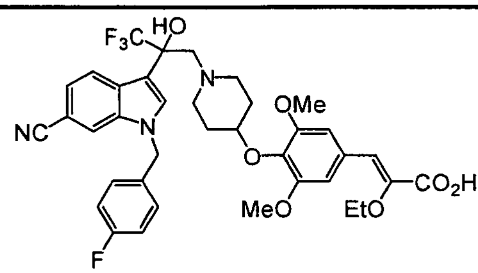 |
| 663 | |
| 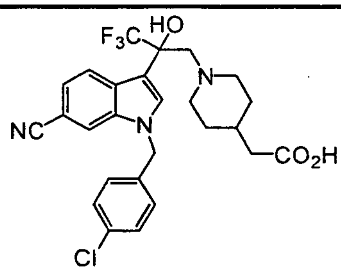 | |

Example 657

3-{7-[2-(1-Benzyl-6-cyano-1H-indol-3-yl)-3,3,3-trifluoro-2-hydroxypropyl]-5,6,7,8-tetrahydroimidazo[1,2-a]pyrazin-2-yl} propanoic acid

MS (ESI+) 538, 1.86($M^{+}$+1, detection time)

Example 658

(2E)-3-{7-[2-(1-Benzyl-6-cyano-1H-indol-3-yl)-3,3,3-trifluoro-2-hydroxypropyl]-5,6,7,8-tetrahydroimidazo[1,2-a] pyrazin-2-yl}acrylic acid

MS (ESI+) 536, 2.05($M^{+}$+1, detection time)

Example 659

3-[4-({1-[2-(1-Benzyl-5-cyano-1H-indol-3-yl)-3,3,3-trifluoro-2-hydroxypropyl]-piperidin-4-yl}oxy)-3-methoxyphenyl]propanoic acid

1H-NMR (400 MHz, $CDCl_3$) δ 8.23 (s, 1H), 7.67 (s, 1H), 7.42 (d, J= 8.7 Hz, 1H), 7.36 (d, J= 8.7 Hz, 1H), 7.31-7.20 (m, 3H), 7.12-7.00 (m, 2H), 6.81-6.61 (m, 3H), 5.36 (s, 2H), 4.41 (br, 1H), 3.98-3.79 (m, 2H), 3.68 (s, 3H), 3.61-3.41 (m, 2H), 3.30-3.18 (m, 1H), 3.10-2.92 (m, 1H), 2.86 (t, J= 7.5 Hz, 2H), 2.61 (t, J= 7.5 Hz, 2H), 2.48-2.25 (m, 1H), 2.20-1.98 (m, 2H), 1.92-1.74 (m, 1H).
MS (ESI+) 622, 1.99($M^+$+1, detection time)

Example 660

[4-({1-[2-(1-Benzyl-6-cyano-1H-indol-3-yl)-3,3,4,4,4-pentafluoro-2-hydroxybutyl]piperidin-4-yl}oxy)-3-methoxyphenyl]acetic acid

$^1$H-NMR (400 MHz, $CDCl_3$) δ 7.95-7.69 (m, 2H), 7.63 (s, 1H), 7.41 (d, J= 8.5 Hz, 1H), 7.37-7.21 (m, 3H), 7.11-7.00 (m, 2H), 6.77 (s, 3H), 5.38 (s, 2H), 4.44 (br, 1H), 3.99 (d, J= 13.2 Hz, 1H), 3.90-3.75 (m, 1H), 3.66 (s, 3H), 3.60-3.40 (m, 2H), 3.57 (s, 2H), 3.20-3.05 (m, 1H), 2.98-2.80 (m, 1H), 2.66-2.49 (m, 1H), 2.29-2.03 (m, 2H), 1.91-1.72 (m, 1H).
MS (ESI+) 658, 2.12($M^+$+1, detection time)

Example 661

[4-({1-[2-(1-Benzyl-6-cyano-1H-indol-3-yl)-3,3-difluoro-2-hydroxypropyl]-piperidin-4-yl}oxy)-3-methoxyphenyl]acetic acid

MS (ESI+) 590, 1.92($M^+$+1, detection time)

Example 662

(2Z)-3-{4-[(1-{2-[6-Cyano-1-(4-fluorobenzyl)-1H-indol-3-yl]-3,3,3-trifluoro-2-hydroxypropyl}piperidin-4-yl)oxy]-3,5-dimethoxyphenyl}-2-ethoxyacrylic acid

$^1$H-NMR (400 MHz, $CDCl_3$) δ 7.86 (d, J= 8.4 Hz, 1H), 7.71 (s, 1H), 7.63 (s, 1H), 7.43 (dd, J= 8.4, 1.1 Hz, 1H), 7.17-7.04 (m, 4H), 7.04-6.93 (m, 3H), 5.35 (S, 2H), 4.43 (br, 1H), 4.00-3.48 (m, 2H), 3.88 (d, J= 13.4 Hz, 1H), 3.81 (d, J= 13.4 Hz, 1H), 3.68-3.55 (m, 3H), 3,28-3.03 (m, 2H), 2.48-2.25 (m, 1H), 2.19-2.00 (m, 2H), 1.93-1,77 (m, 1H).
MS (ESI+) 712, 2.06($M^+$+1, detection time)

Example 663

(1-{2-[1-(4-Chlorobenzyl)-6-cyano-1H-indol-3-yl]-3,3,3-trifluoro-2-hydroxypropyl}piperidin-4-yl)acetic acid

MS (ESI+) 520, 1.90($M^+$+1, detection time)

Example 664

2-(1-Benzyl-6-nitro-1H-indol-3-yl)-1,1,1-trifluoro-3-[(4-methyl-1,3-oxazol-2-yl) amino]propan-2-ol

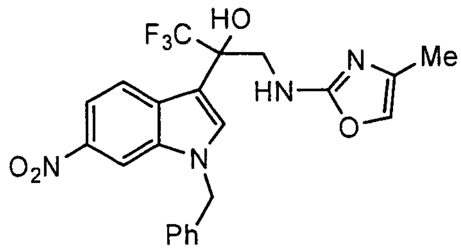

The title compound was obtained in a similar manner to the preparation of the compound of Example 630.
MS (ESI+) 461, 2.18($M^{+}$+1, detection time)

Experiment 1

Binding Inhibitory Assay

Insect cells, which had been infected with baculovirus for expressing human GRa protein, were suspended in an approximately equal volume of a binding buffer (10 mM Tris-Cl, 1.5 mM EDTA, 10% glycerol, 5 mM DTT, 20 mM sodium molybdate, pH 7.6). The mixture was rapidly frozen with liquid nitrogen, and then melted at room temperature. This cycle was repeated totally twice in order to break the cell membrane. The mixture was ultracentrifuged under 100,000g at 4°C for one hour to give the supernatant, which was the cytoplasm fraction containing human GRα protein.
A polypropylene 96-well plate (manufactured by Costar Corporation, etc.) was placed on ice, and DMSO or a test compound (each 1 μl) was put into each well. A 10 nM $^{3}$H-Dex solution (50 μl; tritiated dexamethasone; manufactured by Amersham) in the binding buffer, and a GR protein solution, i.e., a solution by diluting the above cytoplasm fraction with the binding buffer (50 μl) were added to each well, and the plate was lightly shaken with a plate mixer, and then left to stand at 4°C for 16 to 20 hours (the final concentration of DMSO: 1 %).
A 5 % solution of dextran-coated active carbon (manufactured by Sigma) suspended in the binding buffer (50 μl) was put into each well, and the plate was lightly shaken with a plate mixer for 30 seconds. Then, the plate was centrifuged at 2500 rpm at 4°C for 5 minutes, and the $^{3}$H count in the supernatant (50 μl) was measured by a TopCount (manufactured by Perkin-Elmer Corporation). Each test compound was tested twice.
The binding activity was calculated as follows. The mean $^{3}$H count in the presence of dexamethason (Dex) 10 μM was considered as NSB (non-specific binding), and the binding inhibitory ratio in each well was calculated by the following equation.

$$\text{Binding Inhibitory Ratio (\%)} = 100 \times \{1-(\text{cpm value of each well} - \text{NSB})/(\text{mean cpm value of DMSO-well} - \text{NSB})\}$$

(cpm means count per minute)

The mean binding inhibitory ratio of each well was calculated by the above equation to give a binding inhibitory ratio of each test compound.

Experiment 2

TAT (tyrosine aminotransferase) Assay

H4-III-E cells, which had been continuously cultured in α-MEM medium A (supplemented with 10 % inactivated FBS, 50 μM 2-mercapto-ethanol), were suspended in α-MEM medium A or α-MEM medium B (supplemented with 10 % inactivated FBS, 50 μM 2-mercapto-ethanol, but without phenol red), and the cell suspension was seeded to a 96-well plate in an amount of 2 X $10^{4}$ cells/100 μl/well, and the cells were cultured overnight. The supernatant in the medium was removed, and a test compound (100 μl, diluted with α-MEM medium A or α-MEM medium B) was added, and the cells were further cultured overnight.
In order to measure the agonistic activity, a test compound was added alone, while in order to measure the antagonistic activity, a test compound was added simultaneously together with 0.005 μM dexamethasone and evaluated the extent of the inhibitory effect of said test compound against the TAT activity-inducing activity by dexamethasone.

The culture supernatant in the plate was removed by suction, and a cell lysate (20 μL; 1 % NP-40, 0.2% Triton X-100, 0.25 % DOC, 0.1 % SDS, 1 mM EGTA, 150 mM NaCl, Tris (pH7.4), Protease inhibitor cocktail [2.5 mg/ml aprotinin, 2.5 mg/ml leupeptin, 2.5 mg/ml soybean trypsin inhibitor]) was added, and further thereto was added a TAT reaction reagent (150 μL; 10 N KOH, 0.125 M $KH_2PO_4$, 0.5 M α-ketoglutarate, 5 mM Pyridoxal-5'phosphate, L-tyrosine), and the mixture was reacted at 37°C for 15 minutes. Further, a 10N KOH (20 μL) was added thereto, and the mixture was reacted at 37°C for 30 minutes, and the absorbance at 331 nm was measured by a microplate reader (SPECTRA MAX 250).
From the mean absorbance in the wells to which a test compound was added, the mean absorbance in the wells to which a compound and a TAT reaction solution were not added was subtracted. Among the Dex-treated groups (5-steps concentration, 0.5 nM to 5 μM, common ratio: 10), the absorbance of the group, which showed the maximum activity, was considered as 100 %, and the agonistic activity (%) was calculated according to the following equation.

Agonistic activity (%) =
100 x [(absorbance of the drug-treated group) - (absorbance of the group without TAT reaction reagent)]/[(absorbance of Dex-treated group) - (absorbance of the group without TAT reaction reagent)]

On the other hand, the antagonistic activity was calculated as follows.

Antagonistic activity (%) =
Inhibitory ratio (%) against TAT activity increase by Dex =
100 - 100 x [(absorbance of the drug-treated group) - (absorbance of the group without a drug)]/[(absorbance of Dex-treated group) - (absorbance of the group without a drug)]

The results are shown in Table 45 and Table 46. With regard to the agonistic activity and the antagonistic activity, the results of 10 % or more were expressed as O, while the results of less than 10 % were expressed as X.
From the results in Table 45 and Table 46, the compounds of the present invention exhibit the effects of GR function regulating agent.

[Table 45]

| Example No.of test compound | Binding Inhibitory ratio (%) of 100nM of test compound | Agonistic activity of 10 μM test compound | Antagonic activity of 10μM test compound |
|---|---|---|---|
| Example 1 | 92 % | X | ○ |
| Example 2 | 87 % | X | ○ |
| Example 8 | 98 % | X | ○ |
| Example 68 | 79 % | X | ○ |
| Example 21 | 79% (1μM) | X | ○ |
| Example 73 | 95 % | O | ○ |
| Example 75 | 96 % | ○ | ○ |
| Example 76 | 60 % | X | ○ |
| Example 77 | 92 % | ○ | ○ |
| Example 78 | 81 % | ○ | ○ |
| Example 95 | 59 % | X | ○ |
| Example 100 | 101 % | ○ | ○ |

(continued)

| Example No.of test compound | Binding Inhibitory ratio (%) of 100nM of test compound | Agonistic activity of 10 μM test compound | Antagonic activity of 10μM test compound |
|---|---|---|---|
| Example 106 | 101 % | ○ | ○ |
| Example 112 | 98% | X | ○ |
| Example 119 | 78 % | ○ | ○ |
| Example 120 | 97 % | ○ | ○ |
| Example 123 | 72 % | X | O |
| Example 128 | 64 % | X | O |
| Example 131 | 84 % | X | O |
| Example 133 | 51 % | X | O |
| Example 137 | 92 % | O | O |
| Example 140 | 80 % | X | O |

[Table 46]

| Example No. of test compound | Binding Inhibitory ratio (%) of 100 nM test compound | Agonistic activity of 10 μM test compound | Antagonistic activity of 10 μM test compound |
|---|---|---|---|
| Example 144 | 97 % | ○ | ○ |
| Example 150 | 98 % | ○ | ○ |
| Example 152 | 50 % | X | ○ |
| Example 153 | 91 % | ○ | ○ |
| Example 157 | 50 %. | - | - |
| Example 158 | 96 % | X | ○ |
| Example 165 | 95 % | ○ | ○ |
| Example 169 | 99 % | O | O |
| Example 173 | 88 % | X | O |
| Example 178 | 95 % | O | O |
| Example 179 | 81 % | O | O |
| Example 180 | 98 % | O | O |
| Example 181 | 87 % | O | O |
| Example 182 | 99 % | O | O |
| Example 183 | 88 % | O | O |
| Example 184 | 97 % | O | X |
| Example 185 | 82 % | X | O |
| Example 194 | 94 % | ○ | ○ |
| Example 197 | 94 % | ○ | ○ |
| Example 200 | 88 % | ○ | ○ |
| Example 201 | 99 % | ○ | ○ |
| Example 203 | 94 % | ○ | ○ |

(continued)

| Example No. of test compound | Binding Inhibitory ratio (%) of 100 nM test compound | Agonistic activity of 10 μM test compound | Antagonistic activity of 10 μM test compound |
|---|---|---|---|
| Example 229 | 74 % | ○ | ○ |
| Example 226 | 96 % | ○ | ○ |
| Example 235 | 79 % | X | ○ |

INDUSTRIAL APPLICABILITY

The fused pyrrole derivative of the present invention and a pharmaceutically acceptable salt thereof can be used as a non-steroidal anti-inflammatory agent having fewer side effects than steroidal anti-inflammatory agents, or as an antidiabetic agent.

**Claims**

**1.** A compound of the formula (1):

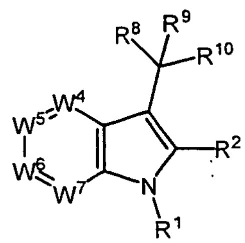

(1)

wherein $R^1$ is a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkoxy group, an optionally substituted alkanoyl group, an optionally substituted alkoxycarbonyl group, an optionally substituted alkylsulfonyl group, an optionally substituted cycloalkyloxy group, an optionally substituted cycloalkylcarbonyl group, an optionally substituted cycloalkyloxycarbonyl group, an optionally substituted cycloalkylsulfonyl group, an optionally substituted aryloxy group, an optionally substituted aroyl group, an optionally substituted aryloxycarbonyl group, an optionally substituted arylsulfonyl group, an optionally substituted heteroaryloxy group, an optionally substituted heteroarylcarbonyl group, an optionally substituted heteroaryloxycarbonyl group, an optionally substituted heteroarylsulfonyl group, an optionally substituted aralkyloxy group, an optionally substituted aralkylcarbonyl group, an optionally substituted aralkyloxycarbonyl group, an optionally substituted aralkylsulfonyl group, an optionally substituted heteroaralkyloxy group, an optionally substituted heteroaralkylcarbonyl group, an optionally substituted heteroaralkyloxycarbonyl group, an optionally substituted heteroaralkylsulfonyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, an optionally substituted carbamoyl group, or an optionally substituted sulfamoyl group;
R2 is a hydrogen atom, a halogen atom, a carboxyl group, an optionally substituted alkyl group, an optionally substituted cycloalkyl group, an optionally substituted aryl group, an optionally substituted heteroaryl group, an optionally substituted aralkyl group, an optionally substituted heteroaralkyl group, an optionally substituted alkanoyl group, an optionally substituted cycloalkylcarbonyl group, an optionally substituted alkoxycarbonyl group, an optionally substituted, saturated or unsaturated aliphatic heterocyclic group, or a carbamoyl group optionally substituted with an optionally substituted alkyl group;
$-W^4=W^5-W^6=W^7-$ is a group selected from the following formulae (a) to (h):

(a) $-CR^4=CR^5-CR^6=CR^7-$;
(b) $-N=CR^5-CR^6=CR^7-$;
(c) $-CR^4=N-CR^6=CR^7-$;

(d) $-CR^4=CR^5-N=CR^7-$;
(e) $-CR^4=CR^5-CR^6=N-$;
(f) $-N=CR^5-N=CR^7-$;
(g) $-CR^4=N-CR^6=N-$;
(h) $-CR^4=N-N=CR^7-$

[in which $R^4$, $R^5$, $R^6$ and $R^7$ are independently the same or different, and each is a group of the formula: -E-A, and in the formula, E is a single bond, or a group selected from the following formulae 1) to 14):

1) $-C(R^{16})R^{17}-$,
2) -O-,
3) $-S(=O)_m-$,
4) $-S(=O)_2NR^{16}-$,
5) -C(=O)-,
6) -C(=O)O-,
7) $-C(=O)NR^{16}-$,
8) $-C(=NR^{16})NR^{17}-$,
9) $-NR^{16}-$,
10) $-N(R^{16})C(=O)-$,
11) $-N(R^{16})S(=O)_2-$,
12) $-N(R^{16})C(=O)N(R^{17})-$,
13) $-N(R^{16})S(=O)_{2N}(R^{17})-$,
14) $-P(=O)(OR^{16})_2-$

(in which $R^{16}$ and $R^{17}$ are independently a hydrogen atom, a $C_{1-3}$ alkyl group, or a $C_{1-3}$ alkoxy group, or in the formulae 8), 12) and 13), $R^{16}$ and $R^{17}$ may combine each other to form a $C_{2-4}$ alkylene group, and m is 0, 1 or 2),

when E is a single bond, then A is a hydrogen atom, a halogen atom, a cyano group, a nitro group, a hydroxy group, a carboxyl group, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, or an optionally substituted, saturated or unsaturated aliphatic heterocyclic group,

When E is a group selected from the above formulae 1) to 14), then A is a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, or an optionally substituted, saturated or unsaturated aliphatic heterocyclic group];

$R^8$ is a group of the formula $-OR^{11}$, $-SR^{11}$, or $-N(R^{11})R^{12}$ (in which $R^{11}$ and $R^{12}$ are independently a hydrogen atom, or an optionally substituted $C_{1-5}$ alkyl group);

R9 is an alkyl group substituted by one or more halogen atoms, or a cycloalkyl group substituted by one or more halogen atoms;

$R^{10}$ is a group of the formula: $-[C(R^{13})R^{14}]_n-R^{15}$ (in which $R^{13}$ and $R^{14}$ are independently a hydrogen atom, an alkyl group or a halogen atom, or $R^{13}$ and $R^{14}$ may combine each other to form an oxo group, or $R^{13}$ and $R^{14}$ may combine each other together with a carbon atom to which they are bonded to form a cycloalkane (one or two $-CH_2-$ groups in said cycloalkane may be replaced by the same or different group(s) selected from -NH-, -S-, -S(=O)-, $-S(=O)_2-$, -C(=O)- and -O-); n is an integer of 0 to 10, and when n is an integer of 2 to 10, then $C(R^{13})R^{14}$ may be either the same or different, $R^{15}$ is a hydroxy group, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted alkoxy group, an optionally substituted alkylthio group, an optionally substituted alkylsulfinyl group, an optionally substituted alkylsulfonyl group, an optionally substituted cycloalkyloxy group, an optionally substituted cycloalkylthio group, an optionally substituted cycloalkylsulfinyl group, an optionally substituted cycloalkylsulfonyl group, an optionally substituted aryloxy group, an optionally substituted arylthio group, an optionally substituted arylsulfinyl group, an optionally substituted arylsulfonyl group, an optionally substituted heteroaryloxy group, an optionally substituted heteroarylthio group, an optionally substituted heteroarylsulfinyl group, an optionally substituted heteroarylsulfonyl group, an optionally substituted carbamoyl group, an optionally substituted sulfamoyl group, an optionally substituted thiocarbamoyl group, an optionally substituted, saturated or unsaturated aliphatic heterocyclic group, an optionally substituted, saturated or unsaturated aliphatic heterocyclic oxy group, an optionally

substituted, saturated or unsaturated aliphatic heterocyclic thio group, an optionally substituted, saturated or unsaturated aliphatic heterocyclic sulfinyl group, an optionally substituted, saturated or unsaturated aliphatic heterocyclic sulfonyl group, or a group of the formula: $N(R^{18})R^{19}$ (in which $R^{18}$ and $R^{19}$ are independently a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkanoyl group, an optionally substituted alkoxycarbonyl group, an optionally substituted alkylsulfonyl group, an optionally substituted cycloalkylcarbonyl group, an optionally substituted cycloalkyloxycarbonyl group, an optionally substituted cycloalkylsulfonyl group, an optionally substituted aroyl group, an optionally substituted aryloxycarbonyl group, an optionally substituted arylsulfonyl group, an optionally substituted heteroarylcarbonyl group, an optionally substituted heteroaryloxycarbonyl group, an optionally substituted heteroarylsulfonyl group, an optionally substituted aralkylcarbonyl group, an optionally substituted aralkyloxycarbonyl group, an optionally substituted aralkylsulfonyl group, an optionally substituted heteroaralkylcarbonyl group, an optionally substituted heteroaralkyloxycarbonyl group, an optionally substituted heteroaralkylsulfonyl group, an optionally substituted carbamoyl group, an optionally substituted sulfamoyl group, an optionally substituted thiocarbamoyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, or $R^{18}$ and $R^{19}$ may combine each other together with a nitrogen atom to which they are bonded to form an optionally substituted, saturated or unsaturated monocyclic, bicyclic or tricyclic nitrogen-containing heterocyclic group containing 1 to 4 heteroatoms selected from 0 to 2 oxygen atoms, 0 to 2 sulfur atoms, and 1to 4 nitrogen atoms);
provided that when $R^{10}$ is methyl group, trifluoromethyl group, hydroxymethyl group, acetoxymethyl group, ethoxycarbonylmethyl group, methoxycarbonyl group, ethoxycarbonyl group, N-butylcarbamoyl group, N-(4-methylbenzenesulfonylmethyl)carbamoyl group, piperidinocarbonyl group, 1-allyl-1H-imidazol-2-yl group, 1-methyl-1H-1,2,4-triazol-5-yl group or 1,3-benzoxazol-2-yl group, then $R^1$ is not a hydrogen atom nor methyl group, or a prodrug thereof, or a pharmaceutically acceptable salt thereof.

**2.** The compound according to claim 1, or a prodrug thereof, or a pharmaceutically acceptable salt thereof, wherein $R^1$ is an optionally substituted alkyl group, an optionally substituted alkenyl group, an optionally substituted alkynyl group, an optionally substituted cycloalkyl group, an optionally substituted cycloalkenyl group, an optionally substituted aryl group, an optionally substituted heteroaryl group, an optionally substituted aralkyl group, an optionally substituted heteroaralkyl group, an optionally substituted alkoxy group, an optionally substituted alkanoyl group, an optionally substituted alkoxycarbonyl group, an optionally substituted alkylsulfonyl group, an optionally substituted cycloalkyloxy group, an optionally substituted cycloalkylcarbonyl group, an optionally substituted cycloalkyloxycarbonyl group, an optionally substituted cycloalkylsulfonyl group, an optionally substituted aryloxy group, an optionally substituted aroyl group, an optionally substituted aryloxycarbonyl group, an optionally substituted arylsulfonyl group, an optionally substituted heteroaryloxy group, an optionally substituted heteroarylcarbonyl group, an optionally substituted heteroaryloxycarbonyl group, an optionally substituted heteroarylsulfonyl group, an optionally substituted aralkyloxy group, an optionally substituted aralkylcarbonyl group, an optionally substituted aralkyloxycarbonyl group, an optionally substituted aralkylsulfonyl group, an optionally substituted heteroaralkyloxy group, an optionally substituted heteroaralkylcarbonyl group, an optionally substituted heteroaralkyloxycarbonyl group, an optionally substituted heteroaralkylsulfonyl group, an optionally substituted, saturated or unsaturated aliphatic heterocyclic group, an optionally substituted, saturated or unsaturated aliphatic heterocyclic carbonyl group, an optionally substituted, saturated or unsaturated aliphatic heterocyclic sulfonyl group, an optionally substituted carbamoyl group, or an optionally substituted sulfamoyl group.

**3.** The compound according to claim 1 or 2, or a prodrug thereof, or a pharmaceutically acceptable salt thereof, wherein $-W^4=W^5-W^6=W^7-$ is a group of the formula (a): $-CR^4=CR^5-CR^6=CR^7-$ (in which $R^4$, $R^5$, $R^6$ and $R^7$ is the same as defined in claim 1).

**4.** The compound according to claim 1 or 2, or a prodrug thereof, or a pharmaceutically acceptable salt thereof, wherein $-W^4=W^5-W^6=W^7-$ is a group of the formula (d): $-CR^4=CR^5-N=CR^7-$ (in which $R^4$, $R^5$ and $R^7$ are the same as defined in claim 1).

**5.** The compound according to any one of claims 1 to 4, or a prodrug thereof, or a pharmaceutically acceptable salt thereof, wherein $R^1$ is an optionally substituted aralkyl group, an optionally substituted heteroaralkyl group, an optionally substituted aryl group, an optionally substituted heteroaryl group, an optionally substituted cycloalkyl group, an alkyl group substituted by an optionally substituted cycloalkyl group, or an alkyl group substituted by an

optionally substituted, saturated or unsaturated aliphatic heterocyclic group.

**6.** The compound according to any one of claims 1 to 5, or a prodrug thereof, or a pharmaceutically acceptable salt thereof, wherein $R^2$ is a hydrogen atom, or an optionally substituted $C_{1-6}$ alkyl group.

**7.** The compound according to any one of claims 1 to 6, or a prodrug thereof, or a pharmaceutically acceptable salt thereof, wherein $R^8$ is a group of the formula: $-OR^{11}$ (in which $R^{11}$ is the same as defined in claim 1).

**8.** The compound according to claim 7, or a prodrug thereof, or a pharmaceutically acceptable salt thereof, wherein $R^{11}$ is a hydrogen atom.

**9.** The compound according to any one of claims 1 to 6, or a prodrug thereof, or a pharmaceutically acceptable salt thereof, wherein $R^8$ is a group of the formula: $-N(R^{11})R^{12}$ (in which $R^{11}$ and $R^{12}$ are the same as defined in claim 1).

**10.** The compound according to claim 9, or a prodrug thereof, or a pharmaceutically acceptable salt thereof, wherein $R^{11}$ and $R^{12}$ are a hydrogen atom.

**11.** The compound according to any one of claims 1 to 10, or a prodrug thereof, or a pharmaceutically acceptable salt thereof, wherein $R^9$ is a $C_{1-6}$ alkyl group substituted by 1 to 7 fluorine or chlorine atoms.

**12.** The compound according to claim 11, or a prodrug thereof, or a pharmaceutically acceptable salt thereof, wherein $R^9$ is a trifluoromethyl group.

**13.** The compound according to any one of claims 1 to 12, or a prodrug thereof, or a pharmaceutically acceptable salt thereof, wherein in the formula: $-[C(R^{13})R^{14}]_n-R^{15}$ for $R^{10}$, $R^{13}$ and $R^{14}$ are a hydrogen atom, n is 1 or 2, and $R^{15}$ is a group of the formula: $N(R^{18})R^{19}$ (in which $R^{18}$ and $R^{19}$ are the same as defined in claim 1).

**14.** The compound according to claim 13, or a prodrug thereof, or a pharmaceutically acceptable salt thereof, wherein $R^{18}$ and $R^{19}$ may combine each other together with a nitrogen atom to which they are bonded to form a substituted saturated or unsaturated nitrogen-containing heteromonocyclic or heterobicyclic group.

**15.** A glucocorticoid receptor function regulating agent, which comprises as the active ingredient the compound as set forth in any one of claims 1-14, or a prodrug thereof, or a pharmaceutically acceptable salt thereof.

**16.** An agent for treatment or prophylaxis of inflammatory diseases or diabetes mellitus, which comprises as the active ingredient the compound as set forth in any one of claims 1-14, or a prodrug thereof, or a pharmaceutically acceptable salt thereof.

**INTERNATIONAL SEARCH REPORT**

International application No.
PCT/JP2006/319426

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y<br><br>A | WO 2004/067529 A1 (ELI LILLY AND CO.), 12 August, 2004 (12.08.04), (Cited in the description of the present application as "patent literature 3") Full text; particularly, Claims 1, 37 to 39, 42; page 7, line 13 to page 11, line 13<br>& EP 1597254 A1 & US 2006/235222 A1<br>& CN 1742007 A & CA 2511806 A1<br>& AU 2004207740 A1 & BR 200406883 A | 1-3,5-7,9, 11,15-16<br>4,8,10,12, 15-16<br>13-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 December, 2006 (26.12.06) | 09 January, 2007 (09.01.07) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No.
PCT/JP2006/319426

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2005-527555 A (BOEHRINGER INGELHEIM PHARMACEUTICALS, INC.), 15 September, 2005 (15.09.05), Full text; particularly, abstract; Claims 1 to 3, 5, 10 to 14; pages 52 to 86, 105 to 108 & WO 2003/082280 A1 & EP 1490062 A1 & US 2004/023999 A1 & CA 2478156 A1 & AU 2003218342 A1 & BR 2003008784 A & CN 1633296 A & NZ 535889 A & NO 2004004031 A & MX PA04009329 A & HR 20040887 A & PL 373043 A | 4,8,12,15-16 |
| Y | JP 2005-529165 A (BOEHRINGER INGELHEIM PHARMACEUTICALS, INC.), 29 September, 2005 (29.09.05), Full text; particularly, abstract; Claims 1 to 4, 10 to 15; pages 25 to 30 & WO 03/090703 A1 & EP 1496851 A1 & US 2005/226822 A1 & CA 2483000 A1 & AU 2003218853 A1 & HR 20041100 A & NO 20044992 A & PL 371950 A & IL 164694 D | 10,12,15-16 |
| Y | WO 2004/058733 A1 (SCHERING AG.), 15 July, 2004 (15.07.04), (Cited in the description of the present application as "patent literature 1") Abstract; Claims 1, 7; pages 5 to 6, Par. Nos. [0001] to [0006]; page 10, Par. Nos. [0016] to [0018] & JP 2006-512382 A & EP 1572671 A1 & US 2004/0209875 A1 & DE 10261874 A1 & AU 2003293843 A1 | 4,8,12,15-16 |
| A | US 2004/0235810 A1 (ABBOTT LABORATORIES), 25 November, 2004 (25.11.04), (Cited in the description of the present application as "patent literature 2") Abstract; Claims & JP 2005-533807 A & WO 2004-000869 A1 & EP 1551860 A1 & CN 1662549 A & CA 2488535 A1 & AU 2003243622 A1 | 1-16 |
| X<br>A | JP 11-349869 A (AGENCY OF INDUSTRIAL SCIENCES AND TECHNOLOGY), 21 December, 1999 (21.12.99), Full text; particularly, Claim 2 (Family: none) | 1,3,6-8, 11-12<br>2,4-5,9-10, 13-16 |

Form PCT/ISA/210 (continuation of second sheet) (April 2005)

**INTERNATIONAL SEARCH REPORT**

International application No.
PCT/JP2006/319426

C (Continuation). DOCUMENTS CONSIDERED TO BE RELEVANT

| Category* | Citation of document, with indication, where appropriate, of the relevant passages | Relevant to claim No. |
|---|---|---|
| X<br><br>A | S. N. OSIPOV ET AL.; "C-Alkylation of some heteroaromatic compounds by methyl trifluoropyruvate (trifluoroacetyl) imine", Izvestiya Akademii Nauk SSSR, Seriya Khimicheskaya, (1989), (9), pp.2131-2134, (abstract) CAplus [online], [retrieved on 2006.12.25], AN 1990:216562 CAPLUS, DN 112: 216562, RN 126954-08-3, CN 1H-Indole-3-acetic acid, α-[(trifluoroacetyl)amino]-α-(trifluoromethyl)-, RN 126954-05-0, CN 1H-Indole-3-acetic acid, α-[(trifluoroacetyl) amino]-α-(trifluoromethyl)-, methyl ester, RN 126954-10-7, CN 1H-Indole-3-acetic acid, α-amino-α-(trifluoromethyl)-, methyl ester, RN 126954-06-1, CN 1H-Indole-3-acetic acid, 2-methyl-α-[(trifluoroacetyl)amino]-α-(trifluoromethyl)-, methyl ester | 1,3,6,9,<br>11-12<br>2,4-5,7-8,<br>10,13-16 |
| A | HEIDI L. HASSINGER ET AL.; "Synthesis of indole-3-carboxamides via a haloform cleavage reaction of 3-trifluoroacetylindole with lithium dialkylamides", Tetrahedron Letters, (1998), Vol.39, Issue 20, pp.3095-3098, Compound 11 | 1-16 |
| A | EVERETT E.GILBERT ET AL.; "Perhaloketones. XIX. Derivatives of benzo-N-heterocycles", Journal of Heterocyclic Chemistry (1969), Vol.6, No.4, pp.483-90, (abstract) CAplus [online], [retrieved on 2006.12.25], AN 1969:491366 CAPLUS, DN 71:91366, RN 23814-16-6, CN 1H-Indole-3-methanol, α,α-bis(trifluoromethyl)- | 1-16 |
| A | KATSUYA KATO ET AL.; "Regio- and enantio-selective acetylation of 3,3,3-trifluoro-2-arylpropane-1,2-diols using Candida antarctica lipase", Biotechnology Letters, (2001), Vol.23, No.21, pp.1729-1734, (abstract) CAplus [online], [retrieved on 2006.12.25], AN 2001:879854 CAPLUS, DN 136:278908, RN 406484-78-4, CN 1,2-Propanediol, 3,3,3-trifluoro-2-(1H-indol-3-yl)-, (-)-, RN 406484-79-5, CN 1,2-Propanediol, 3,3,3-trifluoro-2-(1H-indol-3-yl)-, (+)-, RN 406484-77-3P, CN 1,2-Propanediol, 3,3,3-trifluoro-2-(1H-indol-3-yl)-, 1-acetate, (2S)-, RN 304458-63-7, CN 1H-Indole-3-acetic acid, α-hydroxy-α-(trifluoromethyl)-, ethyl ester RN 406484-72-8, CN 1,2-Propanediol, 3,3,3-trifluoro-2-(1H-indol-3-yl)- | 1-16 |

Form PCT/ISA/210 (continuation of second sheet) (April 2005)

**INTERNATIONAL SEARCH REPORT**

International application No.
PCT/JP2006/319426

C (Continuation). DOCUMENTS CONSIDERED TO BE RELEVANT

| Category* | Citation of document, with indication, where appropriate, of the relevant passages | Relevant to claim No. |
|---|---|---|
| A | MICHAEL P. A. LYLE ET AL.; "Enantioselective Friedel-Crafts Alkylation Reactions Catalyzed by a Chiral Nonracemic C2-Symmetric 2,2'-Bipyridyl Copper(II) Complex", Organic Letters, Vol.7, No.5, (2005.03.03), pp.901-904, Compounds 5a-5g | 1-16 |
| A | MOHAMMED ABID, ET AL.; "Cinchona alkaloid induced chiral discrimination for the determination of the enantiomeric composition of $\alpha$-trifluoromethylated-hydroxyl compounds by $^{19}F$ NMR spectroscopy", Tetrahedron: Asymmetry, Vol.16, Issue 8, (2005.04.18), pp.1547-1555, Compounds 1a-1j, 2a-2j, | 1-16 |
| A | LAURENT EL KAIM ET AL.; "New access to fluorinated glycolic acid derivatives from trifluoropyruvamides", Tetrahedron Letters, Vol.37, Issue 3, (1996), pp.375-378, Compounds 2a, 2b | 1-16 |

Form PCT/ISA/210 (continuation of second sheet) (April 2005)

INTERNATIONAL SEARCH REPORT

International application No.
PCT/JP2006/319426

Continuation of A. CLASSIFICATION OF SUBJECT MATTER
(International Patent Classification (IPC))

*C07D209/12*(2006.01)i, *A61K31/404*(2006.01)i, *A61K31/4045*(2006.01)i, *A61K31/405*(2006.01)i, *A61K31/4155*(2006.01)i, *A61K31/427*(2006.01)i, *A61K31/454*(2006.01)i, *A61K31/496*(2006.01)i, *A61K31/5377*(2006.01)i, *A61K31/5386*(2006.01)i, *A61K31/541*(2006.01)i, *A61K31/551*(2006.01)i, *A61P3/10*(2006.01)i, *A61P5/44*(2006.01)i, *A61P5/46*(2006.01)i, *A61P29/00*(2006.01)i, *C07D209/14*(2006.01)i, *C07D209/22*(2006.01)i, *C07D209/24*(2006.01)i, *C07D401/06*(2006.01)i, *C07D401/14*(2006.01)i, *C07D403/06*(2006.01)i, *C07D403/12*(2006.01)i, *C07D405/12*(2006.01)i, *C07D405/14*(2006.01)i, *C07D409/12*(2006.01)i, *C07D409/14*(2006.01)i, *C07D417/06*(2006.01)i, *C07D491/107*(2006.01)i, *C07D491/113*(2006.01)i, *C07D497/10*(2006.01)i, *C07D498/10*(2006.01)i

(According to International Patent Classification (IPC) or to both national classification and IPC)

Continuation of B. FIELDS SEARCHED
Minimum documentation searched (International Patent Classification (IPC))

C07D209/12, A61K31/404, A61K31/4045, A61K31/405, A61K31/4155, A61K31/427, A61K31/454, A61K31/496, A61K31/5377, A61K31/5386, A61K31/541, A61K31/551, A61P3/10, A61P5/44, A61P5/46, A61P29/00, C07D209/14, C07D209/22, C07D209/24, C07D401/06, C07D401/14, C07D403/06, C07D403/12, C07D405/12, C07D405/14, C07D409/12, C07D409/14, C07D417/06, C07D491/107, C07D491/113, C07D497/10, C07D498/10

Minimum documentation searched (classification system followed by classification symbols)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004058733 A
- US 20040235810 A
- WO 2004067529 A
- WO 0140180 A


### Non-patent literature cited in the description


- *Bull. Chem. Soc. Jpn.,* 1995, vol. 68, 3591
- *J. Am. Chem. Soc.,* 1964, vol. 86, 1899
- **R. C. LAROCK.** Jikken-Kagaku-Koza. VCH publisher Inc, 1989
- **R. C. LAROCK.** Comprehensive Organic transformation. VCH publisher Inc, 1989
- *J. Am. Chem. Soc.,* 1990, vol. 112, 2801
- *J. Am. Chem. Soc.,* 1997, vol. 119, 6189
- *J. Am. Chem. Soc.,* 2000, vol. 122, 3220
- *J. Am. Chem. Soc.,* 2001, vol. 123, 2933
- *Heterocycles,* 2001, vol. 55, 569
- *Heterocycles,* 1996, vol. 42, 83
- Protective Groups in Organic Synthesis. John Wiley & Sons, Inc,
- *J. Org. Chem.,* 1991, vol. 56, 4585
- *Tetrahedron: Asymmetry,* 2003, vol. 14, 503
- *Tetrahedron Lett.,* 1999, vol. 40, 7879
- *Tetrahedron,* 1996, vol. 52, 12761
- *Synthesis,* 1998, vol. 11, 1584
- *Synthesis,* 2003, vol. 1, 45
- *Tetrahedron,* 2000, vol. 56, 7613
- *Chemistry Letters,* 2005, vol. 34, 88
- Jikken-Kagaku-Koza. MARUZEN
- *Tetrahedron Lett.,* 1999, vol. 40, 9333
- **JIRO TSUJI.** Palladium Reagents and Catalysts. John Wiley & Sons Ltd, 2004
- *J. Am. Chem. Soc.,* 2001, vol. 123, 6989
- **RAINER MAHRWALD.** Tetrahedron. John Wiley & Sons Iinc, 2002, vol. 58, 8269
- *Synth Commun.,* 1999, vol. 24, 2419
- *Tetrahedron Lett.,* 1987, vol. 28, 6513
- *J. Org. Chem.,* 1978, vol. 43, 4271
- *Tetrahedron: Asymmetry,* 1997, vol. 8, 903
- *J. Heterocycl. Chem.,* 1991, vol. 28, 473
- *Tetrahedron,* 1995, vol. 51, 11515
- *Synthesis,* 2002, vol. 15, 2254
- *Protective Groups in Organic Synthesis 3rd Edition*
- *J. Org. Chem.,* 1996, vol. 61, 3849
- *J. Am. Chem. Soc.,* 1997, vol. 119, 12386
- **T. W. GREENE ; P. G. M. WUTS.** Protective Groups in Organic Synthesis. John Wiley & Sons, Inc, 1999
- *J. Med. Chem.,* 2004, vol. 47, 2393
- *J. Med. Chem.,* 1992, vol. 35, 4727